(19) Europäisches Patentamt / European Patent Office / Office européen des brevets

(11) EP 4 768 484 A1

(12) # EUROPEAN PATENT APPLICATION

published in accordance with Art. 153(4) EPC

(43) Date of publication:
**01.07.2026 Bulletin 2026/27**

(21) Application number: **24870994.1**

(22) Date of filing: **27.09.2024**

(51) International Patent Classification (IPC):
***C07D 471/04*** (2006.01) ***C07D 401/14*** (2006.01)
***C07D 403/10*** (2006.01) ***C07D 413/14*** (2006.01)
***A61K 31/505*** (2006.01) ***A61K 31/444*** (2006.01)
***A61P 35/00*** (2006.01)

(52) Cooperative Patent Classification (CPC):
**A61K 31/444; A61K 31/505; A61P 35/00; C07D 401/14; C07D 403/10; C07D 413/14; C07D 471/04**

(86) International application number:
**PCT/CN2024/121848**

(87) International publication number:
**WO 2025/067457 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311282731**
**24.06.2024 CN 202410823918**
**20.09.2024 CN 202411323945**

(71) Applicant: **Shenzhen TargetRx, Inc.**
**Shenzhen, Guangdong 518057 (CN)**

(72) Inventors:
- **WANG, Yihan**
  **Shenzhen, Guangdong 518057 (CN)**
- **ZHAO, Jiuyang**
  **Shenzhen, Guangdong 518057 (CN)**
- **AI, Yixin**
  **Shenzhen, Guangdong 518057 (CN)**

(74) Representative: **Novitas Patent AB**
**P.O. Box 55557**
**102 04 Stockholm (SE)**

(54) **HETEROCYCLIC FUSED RING COMPOUND, AND COMPOSITION AND USE THEREOF**

(57) The present invention relates to a compound of formula (I), or a tautomer, stereoisomer, prodrug, crystal form, pharmaceutically acceptable salt, hydrate or solvate thereof, a pharmaceutical composition thereof, and a use thereof in the treatment and/or prevention of diseases modulated by wild-type and/or mutant Bcr-Ab11 kinase.

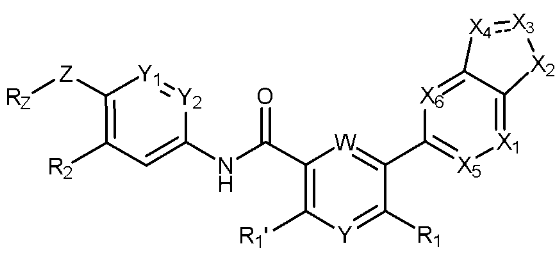

(I)

EP 4 768 484 A1

## Description

### TECHNICAL FIELD

The present disclosure belongs to the field of medicine, and particularly relates to a compound which has an inhibitory effect on the tyrosine kinase activity of Abelson protein (Abl1), Abelson-related protein (Abl2) and related chimeric proteins, especially Ber-Abl1 and its mutations, as well as a pharmaceutical composition comprising the same, and a preparation method and use thereof.

### BACKGROUND

The pathogenesis of chronic myelogenous leukemia (CML) and acute lymphoblastic leukemia (ALL) of some adults is caused by a chromosomal translocation (9;22)(q34;q11), resulting in the fusion of Bcr and Abl1 genes on the Philadelphia chromosome to form a Ber-Abl1 fusion gene. The Ber-Abl1 fusion gene is translated into a Ber-Abl1 fusion protein, thereby abnormally increasing the activity of Ber-Abl1 tyrosine kinase, which has an effect of promoting cell proliferation, inhibiting apoptosis, and reducing cell adhesion. The advent of Ber-Abl1 tyrosine kinase inhibitors has revolutionized the treatment of CML. They inhibit the Ber-Abl1 pathway and significantly reduce the proliferation of CML cells expressing Bcr-Abl1. Currently, the most effective drugs for treating CML that target ATP binding sites include type II tyrosine kinase inhibitors imatinib (Gleevec®) and nilotinib (Tasigna®) which bind to the DFG-out conformation, and type I inhibitors dasatinib (Sprycel®) and bosutinib (Bosulif®) which bind to the DFG-in conformation. However, continuous drug therapy fails due to the occurrence of Ber-$Abl1^{T315I}$ mutation. Although the type II tyrosine kinase inhibitor ponatinib (Iclusing®) can significantly inhibit the T315M mutation, it has adverse effects due to the off-target to other kinases.
In 2003, Kuriyan and SupertiFurga's research group reported that the myristoyl group participates in the autoregulation of Abl1 kinase, inducing the cross-linking of SH3-SH2-Kianse domain and playing a key role in negatively regulating Abl1 kinase activity, thus enabling normal proliferation of cells. However, when the Ber-Abl1 fusion protein is formed, Ber occupies the myristoyl binding site in the N-terminal cap region of Abl1, which prevents the induction of the cross-linking of SH3-SH2-Kinase domain, leading to the structural activation of Bcr-Abl1 and causing cells to undergo continuous differentiation and proliferation and malignant transformation. Asciminib (Scemblix®) is an allosteric inhibitor that binds to the myristoyl pocket of Abl1. Asiciminib is active against all mutations at ATP-catalyzing sites in Bcr-Abl1 in a low nanomolar range, including the T315I mutation. Some mutations at the myristoyl binding site (e.g., P465S, V468F, I502L, A337V, A424T) induce resistance to asiciminib.
Therefore, it is necessary to further develop a new allosteric inhibitor of Bcr-Abl1.

### SUMMARY

The present disclosure provides a novel fused heterocyclic compound, a composition comprising the compound, and use thereof. The compound has high inhibitory activity against Bcr-Abl1 and its ATP-catalyzing site mutations and/or allosteric site mutations, low side effects, and/or excellent pharmacodynamic/pharmacokinetic properties, and can be used to treat a wild-type and/or mutated Ber-Abl1 kinase-mediated disease or condition.
In this regard, the present disclosure adopts the following technical solutions:
In one aspect, the present disclosure provides a compound of formula (I), or a tautomer, a stereoisomer, a prodrug, a crystal form, a pharmaceutically acceptable salt, a hydrate or a solvate thereof:

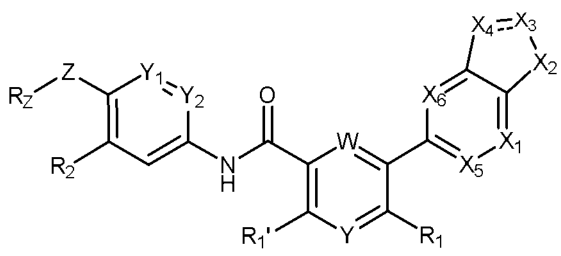

(I)

wherein

$X_1$ is N or CRxi;
$X_2$ is $CR_{X2}R_{X2'}$ or C(O);
$X_3$ is $CR_{X3}R_{X3'}$, $CR_{X3}$ or $NR_{X3}$;
$X_4$ is $CR_{X4}Rx_{4'}$, $CR_{X4}$, $NR_{X4}$ or C(O);

$X_5$ is N or $CR_{X5}$;

$X_6$ is N or $CR_{X6}$;

- - - - is single or double bonds;

$R_{X1}$ is H, D, halogen, $C_{1-6}$ alkyl or $C_{1-6}$ haloalkyl; wherein the above groups are optionally substituted with one or more D, up to fully deuterated;

$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;

$R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with one or more $R_a$;

$R_{X4}$ and $R_{X4'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more $R_a$;

or, $R_{X3}$, $R_{X4}$ and the atoms to which they are attached are taken together to form $C_{3-8}$ cycloalkyl, 4- to 10-membered heterocyclyl, $C_{6-10}$ aryl or 5- to 10-membered heteroaryl; wherein the above groups are optionally substituted with one or more R;

$R_{X5}$ is H, D, halogen, $C_{1-6}$ alkyl or $C_{1-6}$ haloalkyl; wherein the above groups are optionally substituted with one or more D, up to fully deuterated;

$R_{X6}$ is H, D or halogen;

Y is CRy or N;

$R_1$ is 3- to 10-membered heterocyclyl, which is optionally substituted with one or more $R_b$;

Ry is H, D or halogen;

or, $R_1$, $R_Y$ and the carbon atoms to which they are attached are taken together to form $C_{3-8}$ cycloalkyl, 4- to 10-membered heterocyclyl, $C_{6-10}$ aryl or 5- to 10-membered heteroaryl, which is optionally substituted with one or more R*;

$R_1$' is H, D, halogen or $-NH_2$;

W is $CR_W$ or N;

$R_W$ is selected from H, D, halogen and $-NH_2$;

$Y_1$ is $CR_{Y1}$ or N;

$Y_2$ is $CR_{Y2}$ or N;

$R_{Y1}$, $R_{Y2}$ and $R_2$ are independently H, D, halogen, -CN, $-NO_2$, $C_{1-6}$ alkyl or $C_{1-6}$ haloalkyl; wherein the above groups are optionally substituted with one or more $R_c$;

Z is a chemical bond, O or $S(O)_{0-2}$;

$R_Z$ is H, D, $C_{1-6}$ alkyl or $C_{1-6}$ haloalkyl; wherein the above groups are optionally substituted with one or more D, up to fully deuterated;

or $-Z-R_Z$ together represents $-SF_5$;

each of $R_a$, $R_b$ and $R_c$ is independently H, D, halogen, -CN, $-OR_A$, $-NR_BR_C$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;

each R is independently H, D, halogen, -CN, $-OR_A$, $-C(O)R_A$, $-NR_BR_C$, $-NR_AC(O)R_B$, $-S(O)_2R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with one or more R';

each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4-to 10-membered heterocyclyl; wherein the above groups are optionally substituted with one or more R';

wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with one or more R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more R";

or two adjacent R' and the atoms to which they are attached are taken together to form $C_{6-10}$ aryl or 5-to 10-membered heteroaryl; wherein the above groups are optionally substituted with one or more -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;

each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more

D, up to fully deuterated;
with the proviso that, when $X_1$ is N, $X_2$ is $CH_2$, $X_3$ is $NR_{X3}$, $X_4$ is C(O), and $X_5$ is CH, Y is not N.

In another aspect, the present disclosure provides a pharmaceutical composition, comprising the compound of the present disclosure, or a tautomer, a stereoisomer, a prodrug, a crystal form, a pharmaceutically acceptable salt, a hydrate or a solvate thereof, and a pharmaceutically acceptable excipient, and optionally, other therapeutic agent(s).

In another aspect, the present disclosure provides a unit dosage form, comprising the pharmaceutical composition of the compound of the present disclosure.

In another aspect, the present disclosure provides use of the compound of the present disclosure, or a tautomer, a stereoisomer, a prodrug, a crystal form, a pharmaceutically acceptable salt, a hydrate or a solvate thereof, or the pharmaceutical composition of the present disclosure, or the unit dosage form of the present disclosure in the manufacture of a medicament for the treatment and/or prevention of a wild-type and/or mutated Ber-Abl1 kinase-mediated disease. Alternatively, the mutation of the mutated Ber-Abl1 kinase is selected from one or more of the following: T315I, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V and A424T. Alternatively, the mutation of the mutated Ber-Abl1 kinase is selected from T315I and T315M. Alternatively, the mutation of the mutated Ber-Abl1 kinase is T315I. Alternatively, the mutation of the mutated Ber-Abl1 kinase is selected from one or more of the following: P465S, V468F, I502L, A337V and A424T.

In another aspect, the present disclosure provides a method of treating and/or preventing a wild-type and/or mutated Ber-Abl1 kinase-mediated disease in a subject, comprising administering to the subject the compound of the present disclosure or a pharmaceutically acceptable salt, a stereoisomer, a solvate, a hydrate, a polymorph, a prodrug or an isotopic variant thereof, or the pharmaceutical composition of the present disclosure, or the unit dosage form of the present disclosure. Alternatively, the mutation of the mutated Ber-Abl1 kinase is selected from one or more of the following: T3151, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V and A424T. Alternatively, the mutation of the mutated Ber-Abl1 kinase is selected from T315I and T315M. Alternatively, the mutation of the mutated Ber-Abl1 kinase is T315I. Alternatively, the mutation of the mutated Ber-Abl1 kinase is selected from one or more of the following: P465S, V468F, I502L, A337V and A424T.

In another aspect, the present disclosure provides the compound of the present disclosure, or a tautomer, a stereoisomer, a prodrug, a crystal form, a pharmaceutically acceptable salt, a hydrate or a solvate thereof, or the pharmaceutical composition of the present disclosure, or the unit dosage form of the present disclosure, for use in the treatment and/or prevention of a wild-type and/or mutated Ber-Abl1 kinase-mediated disease. Alternatively, the mutation of the mutated Ber-Abl1 kinase is selected from one or more of the following: T315I, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V and A424T. Alternatively, the mutation of the mutated Ber-Abl1 kinase is selected from T315I and T315M. Alternatively, the mutation of the mutated Ber-Abl1 kinase is T3151. Alternatively, the mutation of the mutated Ber-Abl1 kinase is selected from one or more of the following: P465S, V468F, I502L, A337V and A424T.

In a more specific aspect, the wild-type and/or mutated Ber-Abl1 kinase-mediated disease of the present disclosure is selected from: leukemia, non-Hodgkin's lymphoma, Hodgkin's lymphoma, myeloma, neurodegenerative diseases, solid tumors, immune diseases and fibrosis. In a more specific aspect, the wild-type and/or mutated Bcr-Abl1 kinase-mediated disease of the present disclosure is selected from acute lymphoblastic leukemia, acute myeloid leukemia, acute promyelocytic leukemia, chronic lymphocytic lymphoma, chronic myeloid leukemia, chronic myelomonocytic leukemia, chronic neutrophilic leukemia, acute undifferentiated leukemia, anaplastic large cell lymphoma, prolymphocytic leukemia, juvenile myelomonocytic leukemia, adult T-cell leukemia, acute myeloid leukemia with trilineage myelodysplasia, mixed lineage leukemia, myelodysplastic syndrome, amyotrophic lateral sclerosis, Parkinson's disease and Alzheimer's disease.

In a more specific aspect, the wild-type and/or mutated Ber-Abl1 kinase-mediated disease of the present disclosure is acute lymphoblastic leukemia or chronic myeloid leukemia.

Other objects and advantages of the present disclosure will be apparent to those skilled in the art from the specific embodiments, examples and claims that follow.

Definitions

Chemical definitions

The definitions of specific functional groups and chemical terms are described in more detail below.

When a range of values is listed, it is intended to encompass each value and sub-range within the range. For example, "$C_{1-6}$ alkyl" is intended to encompass $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$,

$C_{3-6}$, $C_{3-5}$, $C_{3-4}$, $C_{4-6}$, $C_{4-5}$, and $C_{5-6}$ alkyl.

"$C_{1-6}$ alkyl" refers to a radical of a straight or branched, saturated hydrocarbon group having 1 to 6 carbon atoms, and is also referred to herein as "lower alkyl". In some embodiments, $C_{1-4}$ alkyl and $C_{1-3}$ alkyl are yet alternative. Examples of the alkyl include, but are not limited to, methyl ($C_1$), ethyl ($C_2$), n-propyl ($C_3$), isopropyl ($C_3$), n-butyl ($C_4$), t-butyl ($C_4$), sec-butyl ($C_4$), iso-butyl ($C_4$), n-pentyl (Cs), 3-pentyl (Cs), pentyl (Cs), neo-pentyl ($C_5$), 3-methyl-2-butyl ($C_5$), t-pentyl ($C_5$) and n-hexyl ($C_6$). Regardless of whether or not the alkyl group is modified with "substituted", each alkyl group is independently optionally substituted, for example, with 1 to 5 substituents, 1 to 3 substituents or 1 substituent. Appropriate substituents are defined as follows.

"$C_{1-6}$ alkylidene" refers to =CRR, wherein R is H or $C_{1-5}$ alkyl.

"$C_{2-6}$ alkenyl" refers to a radical of a straight or branched hydrocarbon group having 2 to 6 carbon atoms and one or more carbon-carbon double bonds (e.g., 1, 2 or 3 carbon-carbon double bonds). The one or more carbon-carbon double bonds can be internal (e.g., in 2-butenyl) or terminal (e.g., in 1-butenyl). In some embodiments, $C_{2-4}$ alkenyl is yet alternative. Examples of the alkenyl include, but are not limited to, vinyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl ($C_5$), hexenyl ($C_6$), etc. Regardless of whether or not the alkenyl group is modified with "substituted", each alkenyl group is independently optionally substituted, for example, with 1 to 5 substituents, 1 to 3 substituents or 1 substituent. Appropriate substituents are defined as follows.

"$C_{2-6}$ alkynyl" refers to a radical of a straight or branched hydrocarbon group having 2 to 6 carbon atoms, one or more carbon-carbon triple bonds (e.g., 1, 2 or 3 carbon-carbon triple bonds), and optionally one or more carbon-carbon double bonds (e.g., 1, 2 or 3 carbon-carbon double bonds). In some embodiments, $C_{2-4}$ alkynyl is yet alternative. In some embodiments, the alkynyl group does not contain any double bonds. One or more carbon-carbon triple bonds may be internal (e.g., in 2-butynyl) or terminal (e.g., in 1-butynyl). Examples of the alkynyl include, but are not limited to, ethynyl ($C_2$), 1-propynyl ($C_3$), 2-propynyl ($C_3$), 1-butynyl ($C_4$), 2-butynyl ($C_4$), pentynyl ($C_5$), hexynyl ($C_6$), etc. Regardless of whether or not the alkynyl group is modified with "substituted", each alkynyl group is independently optionally substituted, for example, with 1 to 5 substituents, 1 to 3 substituents or 1 substituent. Appropriate substituents are defined as follows.

"$C_{1-6}$ alkoxy" refers to a group -OR, wherein R is substituted or unsubstituted $C_{1-6}$ alkyl. In some embodiments, $C_{1-4}$ alkoxy and $C_{1-3}$ alkoxy are yet alternative. The specific alkoxy groups include, but are not limited to: methoxy, ethoxy, n-propoxy, isopropoxy, n-butoxy, tert-butoxy, sec-butoxy, n-pentoxy, n-hexyloxy, and 1,2-dimethylbutoxy.

"Halo" or "halogen" refers to fluoro (F), chloro (Cl), bromo (Br) and iodo (I). In some embodiments, the halo group is F, Cl or Br. In some embodiments, the halo group is F or Cl. In some embodiments, the halo group is F.

Therefore, "$C_{1-6}$ haloalkyl" and "$C_{1-6}$ haloalkoxy" refer to the above "$C_{1-6}$ alkyl" and "$C_{1-6}$ alkoxy" substituted with one or more halo groups. In some embodiments, $C_{1-4}$ haloalkyl is yet alternative, $C_{1-3}$ haloalkyl and $C_{1-2}$ haloalkyl are still alternative. In some embodiments, $C_{1-4}$ haloalkoxy is yet alternative, $C_{1-3}$ haloalkoxy and $C_{1-2}$ haloalkoxy are still alternative. Examples of the haloalkyl include, but are not limited to, $-CF_3$, $-CH_2F$, $-CHF_2$, $-CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, etc. Examples of the haloalkoxy include, but are not limited to, $-OCH_2F$, $-OCHF_2$, $-OCF_3$, etc.

"$C_{1-6}$ alkylene" and "$C_{1-6}$ haloalkylene" refer to divalent groups formed by removing another hydrogen of $C_{1-6}$ alkyl and $C_{1-6}$ haloalkyl, and can be substituted or unsubstituted. In some embodiments, $C_{1-4}$ alkylene, $C_{2-4}$ alkylene, and $C_{1-2}$ alkylene are alternative. In some embodiments, $C_{1-4}$ haloalkylene, $C_{2-4}$ haloalkylene, and $C_{1-2}$ haloalkylene are alternative. The unsubstituted alkylene groups include, but are not limited to: methylene ($-CH_2-$), ethylene ($-CH_2CH_2-$), propylene ($-CH_2CH_2CH_2-$), butylene ($-CH_2CH_2CH_2CH_2-$), pentylene ($-CH_2CH_2CH_2CH_2CH_2-$), hexylene ($-CH_2CH_2CH_2CH_2CH_2CH_2-$), etc. Examples of the substituted alkylene, for example, the alkylene substituted with one or more alkyl (methyl), include, but are not limited to: substituted methylene ($-CH(CH_3)-$, $-C(CH_3)_2-$), substituted ethylene ($-CH(CH_3)CH_2-$, $-CH_2CH(CH_3)-$, $-C(CH_3)_2CH_2-$, - $CH_2C(CH_3)_2-$), substituted propylene ($-CH(CH_3)CH_2CH_2-$, $-CH_2CH(CH_3)CH_2-$, $-CH_2CH_2CH(CH_3)-$, - $C(CH_3)_2CH_2CH_2-$, $-CH_2C(CH_3)_2CH_2-$, $-CH_2CH_2C(CH_3)_2-$), etc.

"$C_{3-10}$ cycloalkyl" refers to a radical of a non-aromatic cyclic hydrocarbon group having from 3 to 10 ring carbon atoms and zero heteroatoms. In some embodiments, $C_{3-8}$ cycloalkyl is alternative, $C_{3-6}$ cycloalkyl is yet alternative, and $C_{5-6}$ cycloalkyl is still alternative. The cycloalkyl also includes a ring system in which the cycloalkyl described above is fused with one or more aryl or heteroaryl groups, wherein the point of attachment is on the cycloalkyl ring, and in such case, the number of carbon atoms continues to represent the number of carbon atoms in the cycloalkyl system. Examples of the cycloalkyl include, but are not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), cyclooctyl ($C_8$), cyclooctenyl ($C_8$), bicyclo[2.2.1]heptyl ($C_7$), bicyclo[2.2.2]octyl ($C_8$), cyclononyl ($C_9$), cyclononenyl ($C_9$), cyclodecyl ($C_{10}$), cyclodecenyl ($C_{10}$), octahydro-1H-indenyl ($C_9$), decahydronaphthyl ($C_{10}$), spiro[4.5]decyl ($C_{10}$), etc. Regardless of whether or not the cycloalkyl group is modified with "substituted", each cycloalkyl group is independently optionally substituted, for example, with 1 to 5 substituents, 1 to 3 substituents or 1 substituent. Appropriate substituents are defined as follows.

"3- to 10-membered heterocyclyl" refers to a radical of a 3- to 10-membered non-aromatic ring system having ring carbon atoms and 1 to 4 ring heteroatoms, wherein each heteroatom is independently selected from nitrogen, oxygen,

sulfur, boron, phosphorus, and silicon. In heterocyclyl groups that contain one or more nitrogen atoms, the point of attachment can be a carbon or nitrogen atom, as valency permits. In some embodiments, 4- to 10-membered heterocyclyl is alternative, and it is a 4- to 10-membered non-aromatic ring system having ring carbon atoms and 1 to 4 ring heteroatoms; in some embodiments, 3- to 7-membered heterocyclyl is alternative, and it is a 3- to 7-membered non-aromatic ring system having ring carbon atoms and 1 to 3 ring heteroatoms; in some embodiments, 3- to 6-membered heterocyclyl is yet alternative, and it is a 3- to 6-membered non-aromatic ring system having ring carbon atoms and 1 to 3 ring heteroatoms; in some embodiments, 5- to 6-membered heterocyclyl is still alternative, and it is a 5- to 6-membered non-aromatic ring system having ring carbon atoms and 1 to 3 ring heteroatoms. The heterocyclyl also includes a ring system wherein the heterocyclyl ring described above is fused with one or more cycloalkyl, aryl or heteroaryl groups, wherein the point of attachment is on the heterocyclyl ring, and in such cases, the number of ring members continues to represent the number of ring members in the heterocyclyl ring system. Regardless of whether or not the heterocyclyl group is modified with "substituted", each heterocyclyl group is independently optionally substituted, for example, with 1 to 5 substituents, 1 to 3 substituents or 1 substituent. Appropriate substituents are defined as follows.

Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocyclyl groups containing one heteroatom include, but are not limited to, azepanyl, oxepanyl and thiepanyl. Exemplary 8-membered heterocyclyl groups containing one heteroatom include, but are not limited to, azocanyl, oxecanyl and thiocanyl. Exemplary 5-membered heterocyclyl groups fused with a $C_6$ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a $C_6$ aryl (also referred as 6,6-bicyclic heterocyclyl herein) include, but are not limited to, tetrahydroquinolinyl, tetrahydroisoquinolinyl, etc.

"$C_{6-14}$ aryl" refers to a radical of monocyclic or polycyclic (e.g., bicyclic or tricyclic) 4n+2 aromatic ring system having 6 to 14 ring carbon atoms and zero heteroatoms provided in the aromatic ring system (e.g., having 6, 10 or 14 shared $\pi$ electrons in a cyclic array). In some embodiments, the aryl group has six ring carbon atoms ("$C_6$ aryl"; for example, phenyl). In some embodiments, the aryl group has ten ring carbon atoms ("$C_{10}$ aryl"; for example, naphthyl, e.g., 1-naphthyl and 2-naphthyl). In some embodiments, the aryl group has fourteen ring carbon atoms ("$C_{14}$ aryl"; for example, anthryl). In some embodiments, $C_{6-10}$ aryl is yet alternative, and $C_6$ aryl is still alternative. The aryl also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. Regardless of whether or not the aryl group is modified with "substituted", each aryl group is independently optionally substituted, for example, with 1 to 5 substituents, 1 to 3 substituents or 1 substituent. Appropriate substituents are defined as follows.

"5- to 10-membered heteroaryl" refers to a radical of 5- to 10-membered monocyclic or bicyclic 4n+2 aromatic ring system (e.g., having 6 or 10 shared $\pi$ electrons in a cyclic array) having ring carbon atoms and 1 to 4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more cycloalkyl or heterocyclyl groups, and the point of attachment is on the heteroaryl ring. In such case, the number of the carbon atoms continues to represent the number of carbon atoms in the heteroaryl ring system. In some embodiments, 5- to 6-membered heteroaryl is yet alternative, which is a radical of 5- to 6-membered monocyclic or bicyclic 4n+2 aromatic ring system having ring carbon atoms and 1 to 4 ring heteroatoms. In some embodiments, 5-membered heteroaryl is yet alternative, which is a radical of 5-membered monocyclic or bicyclic 4n+2 aromatic ring system having ring carbon atoms and 1 to 4 ring heteroatoms. Regardless of whether or not the heteroaryl group is modified with "substituted", each heteroaryl group is independently optionally substituted, for example, with 1 to 5 substituents, 1 to 3 substituents or 1 substituent. Appropriate substituents are defined as follows.

Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl, and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl.

Exemplary substituents on carbon atoms include, but are not limited to, halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3^+X^-$, $-N(OR^{cc})R^{bb}$, -SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, $-C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, $-C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, $-OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, $-C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-S_C(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, $-OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, $-P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, carbocyclyl , heterocyclyl, aryl and heteroaryl, wherein each of the alkyl, alkenyl, alkynyl, carbocyclyl , heterocyclyl, aryl and heteroaryl is independently substituted with 0, 1, 2, 3, 4 or 5 $R^{dd}$ groups;

or two geminal hydrogens on a carbon atom are substituted with =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$ or $=NOR^{cc}$ groups;

each of the $R^{aa}$ is independently selected from alkyl, haloalkyl, alkenyl, alkynyl, carbocyclyl, heterocyclyl, aryl and heteroaryl, or two $R^{aa}$ groups are combined to form a heterocyclyl or heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups;

each of the $R^{bb}$ is independently selected from hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, carbocyclyl, heterocyclyl, aryl and heteroaryl, or two $R^{bb}$ groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups;

each of the $R^{cc}$ is independently selected from hydrogen, alkyl, haloalkyl, alkenyl, alkynyl, carbocyclyl, heterocyclyl, aryl and heteroaryl, or two $R^{cc}$ groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{dd}$ groups;

each of the $R^{dd}$ is independently selected from halogen, -CN, $-NO_2$, $-N_3$, $-SO_2H$, $-SO_3H$, -OH, $-OR^{ee}$, $-ON(R^{ff})_2$, $-N(R^{ff})_2$, $-N(R^{ff})_3^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, $-OCO_2R^{ee}$, $-C(=O)N(R^{ff})_2$, $-OC(=O)N(R^{ff})_2$, $-NR^{ff}C(=O)R^{ee}$, $-NR^{ff}CO_2R^{ee}$, $-NR^{ff}C(=O)N(R^{ff})_2$, $-C(=NR^{ff})OR^{ee}$, $-OC(=NR^{ff})R^{ee}$, $-OC(=NR^{ff})OR^{ee}$, $-C(=NR^{ff})N(R^{ff})_2$, $-OC(=NR^{ff})N(R^{ff})_2$, $-NR^{ff}C(=NR^{f})N(R^{ff})_2$, $-NR^{ff}SO_2R^{ee}$, $-SO_2N(R^{ff})_2$, $-SO_2R^{ee}$, $-SO_2OR^{ee}$, $-OSO_2R^{ee}$, $-S(=O)R^{ee}$, $-Si(R^{ee})_3$, $-OSi(R^{ee})_3$, $-C(=S)N(R^{ff})_2$, $-C(=O)SR^{ee}$, $-C(=S)SR^{ee}$, $-SC(=S)SR^{ee}$, $-P(=O)_2R^{ee}$, $-P(=O)(R^{ee})_2$, $-OP(=O)(R^{ee})_2$, $-OP(=O)(OR^{ee})_2$, alkyl, haloalkyl, alkenyl, alkynyl, carbocyclyl, heterocyclyl, aryl, and heteroaryl, wherein each of the alkyl, alkenyl, alkynyl, carbocyclyl, heterocyclyl, aryl and heteroaryl is independently substituted with 0, 1, 2, 3, 4 or 5 $R^{gg}$ groups, or two geminal $R^{dd}$ substituents can be combined to form=O or =S;

each of the $R^{ee}$ is independently selected from alkyl, haloalkyl, alkenyl, alkynyl, carbocyclyl, aryl, heterocyclyl, and heteroaryl, wherein each of the alkyl, alkenyl, alkynyl, carbocyclyl, heterocyclyl, aryl and heteroaryl is independently substituted with 0, 1, 2, 3, 4 or 5 $R^{gg}$ groups;

each of the $R^{ff}$ is independently selected from hydrogen, alkyl, haloalkyl, alkenyl, alkynyl, carbocyclyl, heterocyclyl, aryl and heteroaryl, or two $R^{ff}$ groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 $R^{gg}$ groups;

each of the $R^{gg}$ is independently halogen, -CN, $-NO_2$, $-N_3$, $-SO_2H$, $-SO_3H$, -OH, $-OC_{1-6}$ alkyl, $-ON(C_{1-6}$ alkyl$)_2$, $-N(C_{1-6}$ alkyl$)_2$, $-N(C_{1-6}$ alkyl$)_3^+X^-$, $-NH(C_{1-6}$ alkyl$)_2^+X^-$, $-NH_2(C_{1-6}$ alkyl$)^+X^-$, $-NH_3^+X^-$, $-N(OC_{1-6}$ alkyl$)(C_{1-6}$ alkyl), $-N(OH)(C_{1-6}$ alkyl), -NH(OH), -SH, $-SC_{1-6}$ alkyl, $-SS(C_{1-6}$ alkyl), $-C(=O)(C_{1-6}$ alkyl), $-CO_2H$, $-CO_2(C_{1-6}$ alkyl), $-OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl)

C(=O)($C_{1-6}$ alkyl), -NHCO$_2$($C_{1-6}$ alkyl), -NHC(=O)N($C_{1-6}$ alkyl)$_2$, - NHC(=O)NH($C_{1-6}$ alkyl), -NHC(=O)NH$_2$, -C(=NH)O($C_{1-6}$ alkyl), -OC(=NH)($C_{1-6}$ alkyl), -OC(=NH)OC$_{1-6}$ alkyl, -C(=NH)N($C_{1-6}$ alkyl)$_2$, -C(=NH)NH($C_{1-6}$ alkyl), -C(=NH)NH$_2$, -OC(=NH)N($C_{1-6}$ alkyl)$_2$, -OC(NH)NH($C_{1-6}$ alkyl), -OC(NH)NH$_2$, -NHC(NH)N($C_{1-6}$ alkyl)$_2$, -NHC(=NH)NH$_2$, -NHSO$_2$($C_{1-6}$ alkyl), -SO$_2$N($C_{1-6}$ alkyl)$_2$, - SO$_2$NH($C_{1-6}$ alkyl), -SO$_2$NH$_2$, -SO$_2$C$_{1-6}$ alkyl, -SO$_2$OC$_{1-6}$ alkyl, -OSO$_2$C$_{1-6}$ alkyl, -SOC$_{1-6}$ alkyl, -Si($C_{1-6}$ alkyl)$_3$, - OSi($C_{1-6}$ alkyl)$_3$, -C(=S)N($C_{1-6}$ alkyl)$_2$, C(=S)NH($C_{1-6}$ alkyl), C(=S)NH$_2$, -C(=O)S($C_{1-6}$ alkyl), -C(=S)SC$_{1-6}$ alkyl, -SC(=S)SC$_{1-6}$ alkyl, -P(=O)$_2$($C_{1-6}$ alkyl), -P(=O)($C_{1-6}$ alkyl)$_2$, -OP(=O)($C_{1-6}$ alkyl)$_2$, -OP(=O)(OC$_{1-6}$ alkyl)$_2$, $C_{1-6}$ alkyl, $C_{1-6}$ haloalkyl, $C_2$-$C_6$ alkenyl, $C_2$-$C_6$ alkynyl, $C_3$-$C_7$ carbocyclyl, $C_6$-$C_{10}$ aryl, $C_3$-$C_7$ heterocyclyl, or $C_5$-$C_{10}$ heteroaryl; or two geminal $R^{gg}$ substituents may combine to form =O or =S; wherein $X^-$ is a counter-ion.

Exemplary substituents on nitrogen atoms include, but are not limited to, hydrogen, -OH, -OR$^{aa}$, - N(R$^{cc}$)$_2$, -CN, -C(=O)R$^{aa}$, -C(=O)N(R$^{cc}$)$_2$, -CO$_2$R$^{aa}$, -SO$_2$R$^{aa}$, -C(=NR$^{bb}$)R$^{aa}$, -C(=NR$^{cc}$)OR$^{aa}$, -C(=NR$^{cc}$)N(R$^{cc}$)$_2$, -SO$_2$N(R$^{cc}$)$_2$, -SO$_2$R$^{cc}$, -SO$_2$OR$^{cc}$, -SOR$^{aa}$, -C(=S)N(R$^{cc}$)$_2$, -C(=O)SR$^{cc}$, -C(=S)SR$^{cc}$, -P(=O)$_2$R$^{aa}$, -P(=O)(R$^{aa}$)$_2$, - P(=O)$_2$N(R$^{cc}$)$_2$, -P(=O)(NR$^{cc}$)$_2$, alkyl, haloalkyl, alkenyl, alkynyl, carbocyclyl, heterocyclyl, aryl and heteroaryl, or two R$^{cc}$ groups attached to a nitrogen atom combine to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 R$^{dd}$ groups, and wherein R$^{aa}$, R$^{bb}$, R$^{cc}$ and R$^{dd}$ are as described herein.

"Deuteration", "deuterated" or "D" means that one or more hydrogens in the compound or in the group are substituted with deuterium; Deuteration can be monosubstituted, disubstituted, polysubstituted or fully substituted. The terms "substituted with one or more deuteriums" and "deuterated one or more times" are used interchangeably.

"Non-deuterated compound" refers to a compound with deuterium atom content not higher than the natural deuterium isotope content (0.015%).

The deuterium isotope content of deuterium at the deuterated position is at least greater than the natural deuterium isotope content (0.015%), alternatively greater than 30%, yet alternatively greater than 50%, yet alternatively greater than 75%, yet alternatively greater than 95%, and yet alternatively greater than 99%.

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describe pharmaceutically acceptable salts in detail in J. Pharmaceutical Sciences (1977) 66:1-19. Pharmaceutically acceptable salts of the compounds disclosed herein include those derived from suitable inorganic and organic acids and bases. Examples of pharmaceutically acceptable, nontoxic acid addition salts are salts formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid. Salts formed using conventional methods in the art such as ion exchange are also included. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and $N^+(C_{1-4}$ alkyl$)_4$ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

A "subject" to which administration is contemplated includes, but is not limited to, humans (i.e., a male or female of any age group, e.g., a pediatric subject (e.g., infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In some embodiments, the subject is a human. In some embodiments, the subject is a non-human animal. The terms "human," "patient," and "subject" are used interchangeably herein.

"Disease", "disorder" and "condition" are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplate an action that occurs before a subject begins to suffer from the specified disease, disorder or condition ("prophylactic treatment").

"Combination", "combined", and related terms refer to the simultaneous or sequential administration of the

therapeutic agents of the present disclosure. For example, the compound disclosed herein may be administered with another therapeutic agent simultaneously or sequentially in separate unit dosage forms, or together in a single unit dosage form.

## DETAILED DESCRIPTION

Compounds

As used herein, "compound of the present disclosure" refers to a compound of formula (I) below (including subsets of each formula), or a tautomer, a stereoisomer, a prodrug, a crystal form, a pharmaceutically acceptable salt, a hydrate or a solvate thereof.

In one embodiment, the present disclosure relates to a compound of formula (I), or a tautomer, a stereoisomer, a prodrug, a crystal form, a pharmaceutically acceptable salt, a hydrate or a solvate thereof:

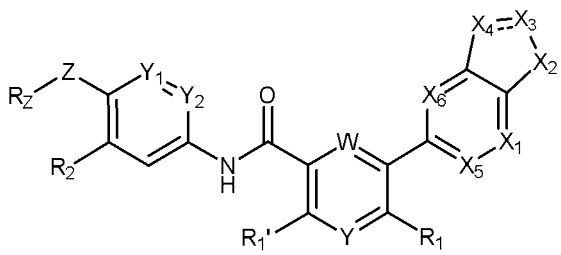

(I)

wherein

$X_1$ is N or $CR_{X1}$;
$X_2$ is $CR_{X2}R_{X2'}$ or C(O);
$X_3$ is $CR_{X3}R_{X3'}$, $CR_{X3}$ or $NR_{X3}$;
$X_4$ is $CR_{X4}R_{X4'}$, $CR_{X4}$, $NR_{X4}$ or C(O);
$X_5$ is N or $CR_{X5}$;
$X_6$ is N or $CR_{X6}$;
- - - - is single or double bonds;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with one or more $R_a$;
$R_{X4}$ and $R_{X4'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more $R_a$;
or, $R_{X3}$, $R_{X4}$ and the atoms to which they are attached are taken together to form $C_{3-8}$ cycloalkyl, 4- to 10-membered heterocyclyl, $C_{6-10}$ aryl or 5- to 10-membered heteroaryl; wherein the above groups are optionally substituted with one or more R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen;
Y is $CR_Y$ or N;
$R_1$ is 3- to 10-membered heterocyclyl, which is optionally substituted with one or more $R_b$;
$R_Y$ is H, D or halogen;
or, $R_1$, $R_Y$ and the carbon atoms to which they are attached are taken together to form $C_{3-8}$ cycloalkyl, 4- to 10-membered heterocyclyl, $C_{6-10}$ aryl or 5- to 10-membered heteroaryl, which is optionally substituted with one or more R*;
$R_1$' is H, D, halogen or $-NH_2$;
W is $CR_W$ or N;
$R_W$ is selected from H, D, halogen and $-NH_2$;
$Y_1$ is $CR_{Y1}$ or N;

$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl; wherein the above groups are optionally substituted with one or more $R_c$;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
each of $R_a$, $R_b$ and $R_c$ is independently H, D, halogen, -CN, $-OR_A$, $-NR_BR_C$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
each R is independently H, D, halogen, -CN, $-OR_A$, $-C(O)R_A$, $-NR_BR_C$, $-NR_AC(O)R_B$, $-S(O)_2R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with one or more R';
each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4-to 10-membered heterocyclyl; wherein the above groups are optionally substituted with one or more R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with one or more R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more R";
or two adjacent R' and the atoms to which they are attached are taken together to form $C_{6-10}$ aryl or 5-to 10-membered heteroaryl; wherein the above groups are optionally substituted with one or more -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated;
with the proviso that, when $X_1$ is N, $X_2$ is $CH_2$, $X_3$ is $NR_{X3}$, $X_4$ is C(O), and $X_5$ is CH, Y is not N.
$X_1$-$X_6$, $R_a$, R, $R_A$-$R_C$ and $P_1$-$P_4$

In one embodiment, $X_1$ is N; in another embodiment, $X_1$ is $CR_{X1}$.
In one specific embodiment, $X_1$ is $CR_{X1}$, and wherein $R_{X1}$ is H, D, halogen, $C_{1-6}$ alkyl or $C_{1-6}$ haloalkyl; wherein the above groups are optionally substituted with one or more D, up to fully deuterated. In another specific embodiment, $X_1$ is $CR_{X1}$, and wherein $R_{X1}$ is H, D or halogen. In one specific embodiment, $X_1$ is $CR_{X1}$, and wherein $R_{X1}$ is H, D or F. In one specific embodiment, $X_1$ is $CR_{X1}$, and wherein $R_{X1}$ is H or D.
In one embodiment, $X_2$ is $CR_{X2}R_{X2'}$; in another embodiment, $X_2$ is C(O).
In one specific embodiment, $X_2$ is $CR_{X2}R_{X2'}$, and wherein $R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated. In another specific embodiment, $X_2$ is $CR_{X2}R_{X2'}$, and wherein $R_{X2}$ and $R_{X2'}$ are independently H, D, halogen, -OH, $C_{1-6}$ alkyl or $C_{1-6}$ haloalkyl; wherein the above groups are optionally substituted with one or more D, up to fully deuterated. In another specific embodiment, $X_2$ is $CR_{X2}R_{X2'}$, and wherein $R_{X2}$ and $R_{X2'}$ are independently H, D, halogen, -OH, $C_{1-3}$ alkyl or $C_{1-3}$ haloalkyl; wherein the above groups are optionally substituted with one or more D, up to fully deuterated. In another specific embodiment, $X_2$ is $CR_{X2}R_{X2'}$, and wherein $R_{X2}$ and $R_{X2'}$ are independently H, D, halogen or -OH. In another specific embodiment, $X_2$ is $CR_{X2}R_{X2'}$, and wherein $R_{X2}$ and $R_{X2'}$ are independently H, D, -F or -OH.
In one embodiment, $X_3$ is $CR_{X3}R_{X3'}$; in another embodiment, $X_3$ is $CR_{X3}$; in another embodiment, $X_3$ is $NR_{X3}$.
In one specific embodiment, $X_3$ is $CR_{X3}R_{X3'}$, and wherein $R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with 1 to 6 (e.g., 1, 2, 3, 4, 5 or 6) $R_a$. In another specific embodiment, $X_3$ is $CR_{X3}R_{X3'}$, and wherein $R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with 1 to 6 (e.g., 1, 2, 3, 4, 5 or 6) $R_a$. In another specific embodiment, $X_3$ is $CR_{X3}R_{X3'}$, and wherein $R_{X3}$ and $R_{X3'}$ are independently H, D, halogen, $C_{1-6}$ alkyl, $C_{1-6}$ haloalkyl or $C_{3-6}$ cycloalkyl; wherein the above groups are optionally substituted with 1 to 6 (e.g., 1, 2, 3, 4, 5 or 6) $R_a$. In another specific embodiment, $X_3$ is $CR_{X3}R_{X3'}$, and wherein

$R_{X3}$ and $R_{X3'}$ are independently H, D, halogen, $C_{1-3}$ alkyl, $C_{1-3}$ haloalkyl or $C_{3-6}$ cycloalkyl; wherein the above groups are optionally substituted with 1 to 6 (e.g., 1, 2, 3, 4, 5 or 6) $R_a$. In another specific embodiment, $X_3$ is $CR_{X3}R_{X3'}$, and wherein $R_{X3}$ and $R_{X3'}$ are independently H, D, halogen, $C_{1-3}$ alkyl or $C_{1-3}$ haloalkyl; wherein the above groups are optionally substituted with 1 to 6 (e.g., 1, 2, 3, 4, 5 or 6) $R_a$. In another specific embodiment, $X_3$ is $CR_{X3}R_{X3'}$, and wherein $R_{X3}$ and $R_{X3'}$ are independently H, D or F. In another specific embodiment, $X_3$ is $CR_{X3}R_{X3'}$, and wherein $R_{X3}$ and $R_{X3'}$ are independently H or D.

In one specific embodiment, $X_3$ is $CR_{X3}$, and wherein $R_{X3}$ is H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with 1 to 6 (e.g., 1, 2, 3, 4, 5 or 6) $R_a$. In another specific embodiment, $X_3$ is $CR_{X3}$, and wherein $R_{X3}$ is H, D, halogen, $C_{1-6}$ alkyl, $C_{1-6}$ haloalkyl or $C_{3-6}$ cycloalkyl; wherein the above groups are optionally substituted with 1 to 6 (e.g., 1, 2, 3, 4, 5 or 6) $R_a$. In another specific embodiment, $X_3$ is $CR_{X3}$, and wherein $R_{X3}$ is H, D, halogen, $C_{1-6}$ alkyl or $C_{1-6}$ haloalkyl; wherein the above groups are optionally substituted with 1 to 6 (e.g., 1, 2, 3, 4, 5 or 6) $R_a$. In another specific embodiment, $X_3$ is $CR_{X3}$, and wherein $R_{X3}$ is H, D, halogen, $C_{1-3}$ alkyl or $C_{1-3}$ haloalkyl; wherein the above groups are optionally substituted with 1 to 6 (e.g., 1, 2, 3, 4, 5 or 6) $R_a$. In another specific embodiment, $X_3$ is $CR_{X3}$, and wherein $R_{X3}$ is H, D or halogen. In another specific embodiment, $X_3$ is $CR_{X3}$, and wherein $R_{X3}$ is H, D or F. In another specific embodiment, $X_3$ is $CR_{X3}$, and wherein $R_{X3}$ is H or D.

In one specific embodiment, $X_3$ is $NR_{X3}$, and wherein $R_{X3}$ is H, $C_{1-6}$ alkyl, $C_{1-6}$ haloalkyl, $C_{3-6}$ cycloalkyl or 3- to 7-membered heterocyclyl; wherein the above groups are optionally substituted with 1 to 6 (e.g., 1, 2, 3, 4, 5 or 6) $R_a$. In another specific embodiment, $X_3$ is $NR_{X3}$, and wherein $R_{X3}$ is H, $C_{1-6}$ alkyl, $C_{1-6}$ haloalkyl or $C_{3-6}$ cycloalkyl; wherein the above groups are optionally substituted with 1 to 6 (e.g., 1, 2, 3, 4, 5 or 6) $R_a$. In another specific embodiment, $X_3$ is $NR_{X3}$, and wherein $R_{X3}$ is $C_{1-6}$ alkyl, $C_{1-6}$ haloalkyl or $C_{3-6}$ cycloalkyl; wherein the above groups are optionally substituted with 1 to 6 (e.g., 1, 2, 3, 4, 5 or 6) $R_a$. In another specific embodiment, $X_3$ is $NR_{X3}$, and wherein $R_{X3}$ is $C_{1-6}$ alkyl, $C_{1-6}$ haloalkyl or $C_{3-6}$ cycloalkyl; wherein the above groups are optionally substituted with one or more D, up to fully deuterated. In another specific embodiment, $X_3$ is $NR_{X3}$, and wherein $R_{X3}$ is $C_{1-3}$ alkyl, $C_{1-3}$ haloalkyl or $C_{3-6}$ cycloalkyl; wherein the above groups are optionally substituted with one or more D, up to fully deuterated. In another specific embodiment, $X_3$ is $NR_{X3}$, and wherein $R_{X3}$ is H, $CH_3$, $CD_3$ or cyclopropane. In another specific embodiment, $X_3$ is $NR_{X3}$, and wherein $R_{X3}$ is $CH_3$, $CD_3$ or cyclopropane.

In one embodiment, $X_4$ is $CR_{X4}R_{X4'}$; in another embodiment, $X_4$ is $CR_{X4}$; in another embodiment, $X_4$ is $NR_{X4}$; in another embodiment, $X_4$ is C(O).

In one specific embodiment, $X_4$ is $CR_{X4}R_{X4'}$, and wherein $R_{X4}$ and $R_{X4'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with 1 to 6 (e.g., 1, 2, 3, 4, 5 or 6) $R_a$. In another specific embodiment, $X_4$ is $CR_{X4}R_{X4'}$, and wherein $R_{X4}$ and $R_{X4'}$ are independently H, D, halogen, $C_{1-6}$ alkyl or $C_{1-6}$ haloalkyl; wherein the above groups are optionally substituted with 1 to 6 (e.g., 1, 2, 3, 4, 5 or 6) $R_a$. In another specific embodiment, $X_4$ is $CR_{X4}R_{X4'}$, and wherein $R_{X4}$ and $R_{X4'}$ are independently H, D, halogen, $C_{1-3}$ alkyl or $C_{1-3}$ haloalkyl; wherein the above groups are optionally substituted with 1 to 6 (e.g., 1, 2, 3, 4, 5 or 6) $R_a$. In another specific embodiment, $X_4$ is $CR_{X4}R_{X4'}$, and wherein $R_{X4}$ and $R_{X4'}$ are independently H or D.

In one specific embodiment, $X_4$ is $CR_{X4}$, and wherein $R_{X4}$ is H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with 1 to 6 (e.g., 1, 2, 3, 4, 5 or 6) $R_a$. In another specific embodiment, $X_4$ is $CR_{X4}$, and wherein $R_{X4}$ is H, D, halogen, $C_{1-6}$ alkyl or $C_{1-6}$ haloalkyl; wherein the above groups are optionally substituted with 1 to 6 (e.g., 1, 2, 3, 4, 5 or 6) $R_a$. In another specific embodiment, $X_4$ is $CR_{X4}$, and wherein $R_{X4}$ is H or D.

In one specific embodiment, $X_4$ is $NR_{X4}$, and wherein $R_{X4}$ is H, $C_{1-6}$ alkyl, $C_{1-6}$ haloalkyl, $C_{3-6}$ cycloalkyl or 3- to 7-membered heterocyclyl; wherein the above groups are optionally substituted with 1 to 6 (e.g., 1, 2, 3, 4, 5 or 6) $R_a$. In another specific embodiment, $X_4$ is $NR_{X4}$, and wherein $R_{X4}$ is H, $C_{1-6}$ alkyl or $C_{1-6}$ haloalkyl; wherein the above groups are optionally substituted with 1 to 6 (e.g., 1, 2, 3, 4, 5 or 6) $R_a$. In another specific embodiment, $X_4$ is $NR_{X4}$, and wherein $R_{X4}$ is $C_{1-6}$ alkyl or $C_{1-6}$ haloalkyl; wherein the above groups are optionally substituted with 1 to 6 (e.g., 1, 2, 3, 4, 5 or 6) $R_a$. In another specific embodiment, $X_4$ is $NR_{X4}$, and wherein $R_{X4}$ is $C_{1-3}$ alkyl or $C_{1-3}$ haloalkyl; wherein the above groups are optionally substituted with 1 to 6 (e.g., 1, 2, 3, 4, 5 or 6) $R_a$.

In one specific embodiment, $R_{X3}$, $R_{X4}$ and the atoms to which they are attached are taken together to form $C_{3-8}$ cycloalkyl, 4- to 10-membered heterocyclyl, $C_{6-10}$ aryl or 5- to 10-membered heteroaryl; wherein the above groups are optionally substituted with 1 to 6 (e.g., 1, 2, 3, 4, 5 or 6) R. In another specific embodiment, $R_{X3}$, $R_{X4}$ and the atoms to which they are attached are taken together to form $C_{3-8}$ cycloalkyl, which is optionally substituted with 1 to 6 (e.g., 1, 2, 3, 4, 5 or 6) R. In another specific embodiment, $R_{X3}$, $R_{X4}$ and the atoms to which they are attached are taken together to form 4- to 10-membered heterocyclyl, which is optionally substituted with 1 to 6 (e.g., 1, 2, 3, 4, 5 or 6) R. In another specific embodiment, $R_{X3}$, $R_{X4}$ and the atoms to which they are attached are taken together to form $C_{6-10}$ aryl, which is optionally substituted with 1 to 6 (e.g., 1, 2, 3, 4, 5 or 6) R. In another specific embodiment, $R_{X3}$, $R_{X4}$ and the atoms to which they are attached are taken together to form 5- to 10-membered heteroaryl, which is optionally substituted with 1 to 6 (e.g., 1, 2, 3, 4, 5 or 6) R.

In another specific embodiment, $R_{X3}$, $R_{X4}$ and the atoms to which they are attached are taken together to form $C_{3-8}$ cycloalkyl, 4- to 10-membered heterocyclyl, phenyl or 5- to 10-membered heteroaryl; wherein the above groups are

optionally substituted with 1 to 6 (e.g., 1, 2, 3, 4, 5 or 6) R. In another specific embodiment, $R_{X3}$, $R_{X4}$ and the atoms to which they are attached are taken together to form phenyl or 5- to 10-membered heteroaryl; wherein the above groups are optionally substituted with 1 to 6 (e.g., 1, 2, 3, 4, 5 or 6) R. In another specific embodiment, $R_{X3}$, $R_{X4}$ and the atoms to which they are attached are taken together to form phenyl or 5-membered heteroaryl; wherein the above groups are optionally substituted with 1 to 3 (e.g., 1, 2 or 3). In another specific embodiment, $R_{X3}$, $R_{X4}$ and the atoms to which they are attached are taken together to form 5-membered heteroaryl, which is optionally substituted with 1 to 3 (e.g., 1, 2 or 3) R. In another specific embodiment, $R_{X3}$, $R_{X4}$ and the atoms to which they are attached are taken together to form

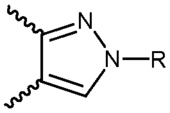 , 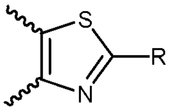 ,

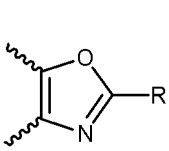 , 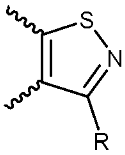 , 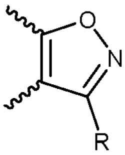 , 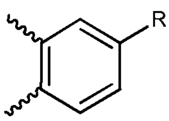 , 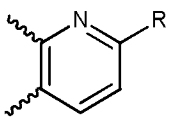 , 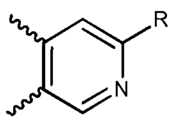

or

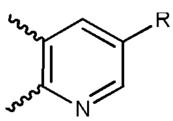

. $R_{X3}$, $R_{X4}$ and the atoms to which they are attached are taken together to form

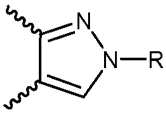 ,

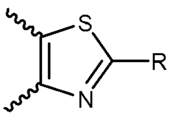 , 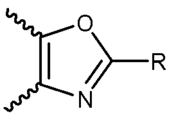 , 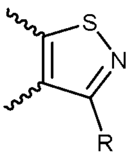 , 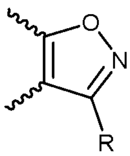 , 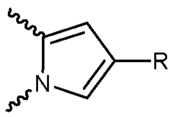 , 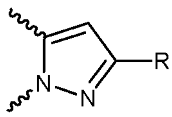

or

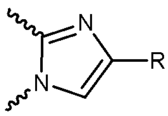 .

In another specific embodiment, $R_{X3}$, $R_{X4}$ and the atoms to which they are attached are taken together to form

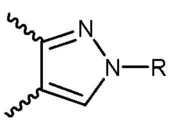 , 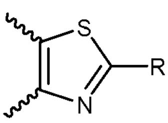 , 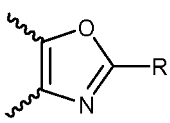 , 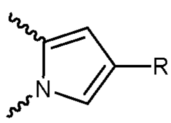 , 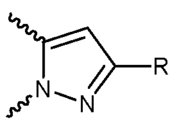

or

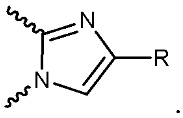

.

In another specific embodiment, $R_{X3}$, $R_{X4}$ and the atoms to which they are attached are taken together to form

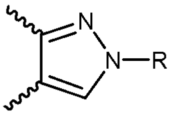

,

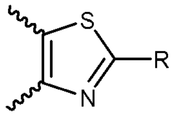

or

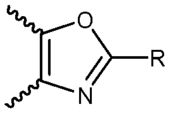

.

In another specific embodiment, $R_{X3}$, $R_{X4}$ and the atoms to which they are attached are taken together to form

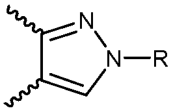

.

In one embodiment, $X_5$ is N; in another embodiment, $X_5$ is $CR_{X5}$.

In one specific embodiment, $X_5$ is $CR_{X5}$, and wherein $R_{X5}$ is H, D, halogen, $C_{1-6}$ alkyl or $C_{1-6}$ haloalkyl; wherein the above groups are optionally substituted with one or more D, up to fully deuterated. In another specific embodiment, $X_5$ is $CR_{X5}$, and wherein $R_{X5}$ is H, D or halogen. In another specific embodiment, $X_5$ is $CR_{X5}$, and wherein $R_{X5}$ is H, D or F. In another specific embodiment, $X_5$ is $CR_{X5}$, and wherein $R_{X5}$ is H or D.

In one embodiment, $X_6$ is N; in another embodiment, $X_6$ is $CR_{X6}$.

In one specific embodiment, $X_6$ is $CR_{X6}$, and wherein $R_{X6}$ is H, D or halogen. In another specific embodiment, $X_6$ is $CR_{X6}$, and wherein $R_{X6}$ is H, D or F. In another specific embodiment, $X_6$ is $CR_{X6}$, and wherein $R_{X6}$ is H or D.

In one embodiment, each $R_a$ is H, D, halogen, -CN, $-OR_A$, $-NR_BR_C$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated. In another embodiment, each $R_a$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated. In another embodiment, each $R_a$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated. In another embodiment, each $R_a$ is H, D, halogen, -CN, -OH, $-NH_2$, $-NHC_{1-6}$ alkyl, - $N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl or 3- to 7-membered heterocyclyl; wherein the above groups are optionally substituted with one or more D, up to fully deuterated. In another embodiment, each $R_a$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated. In another embodiment, each $R_a$ is H, D, halogen, -CN, -OH, $-NH_2$, $-NHC_{1-6}$ alkyl, $-N(C_{1-6}$ alkyl$)_2$, $C_{1-6}$ alkyl or $C_{1-6}$ haloalkyl; wherein the above groups are optionally substituted with one or more D, up to fully deuterated. In another embodiment, each $R_a$ is H, D, halogen, -CN, -OH, $-NH_2$, $-NHC_{1-6}$ alkyl or $-N(C_{1-6}$ alkyl$)_2$; wherein the above groups are optionally substituted with one or more D, up to fully deuterated.

In one embodiment, each R is independently H, D, halogen, -CN, -OH, $-C(O)R_a$, $-NR_BR_C$, - $NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g. 1, 2, 3, 4, 5 or 6) R'. In another embodiment, each R is independently H, D, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g. 1, 2, 3, 4, 5 or 6) R'. In another embodiment, each R is independently H, D, $-C(O)R_A$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl or 5- to 10-membered heteroaryl; wherein the above groups are optionally substituted with 1 to 6 (e.g. 1, 2, 3, 4, 5 or 6) R'. In another embodiment, each R is independently H, D, $C_{1-6}$ alkyl, $C_{1-6}$ haloalkyl, $C_{3-6}$ cycloalkyl, 3- to 7-membered heterocyclyl or 5- to 10-membered heteroaryl; wherein the above groups are optionally substituted with 1 to 6 (e.g. 1, 2, 3, 4, 5 or 6) R'. In another embodiment, each R is independently H, D, $C_{1-3}$ alkyl, $C_{1-3}$ haloalkyl, $C_{3-6}$ cycloalkyl, 3- to 7-membered heterocyclyl or 5- to 10-membered heteroaryl; wherein the above groups are optionally substituted with 1 to 6 (e.g. 1, 2, 3, 4, 5 or 6) R'. In another embodiment, each R is independently $C_{1-3}$ alkyl, $C_{1-3}$ haloalkyl, $C_{3-6}$ cycloalkyl, 3- to 7-membered heterocyclyl or 5- to 10-membered heteroaryl; wherein the above groups are optionally substituted with 1 to 6 (e.g. 1, 2, 3, 4, 5 or 6) R'. In another embodiment, each R is independently 5- to 10-membered heteroaryl; wherein the above group is optionally substituted with 1 to 6 (e.g. 1, 2, 3, 4, 5 or 6) R'.

In one specific embodiment, R is

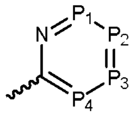
,

wherein

$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g. 1, 2, 3, 4, 5 or 6) R";
or $R_{P1}$ and $R_{P2}$, $R_{P2}$ and $R_{P3}$, or $R_{P3}$ and $R_{P4}$, and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

In another specific embodiment, R is

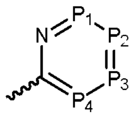
,

wherein

$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
or $R_{P1}$ and $R_{P2}$, $R_{P2}$ and $R_{P3}$, or $R_{P3}$ and $R_{P4}$, and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

In another specific embodiment, R is

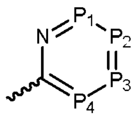
,

wherein

$P_1$ is $CR_{P1}$;
$P_2$ is N or $CR_{P2}$;
$P_3$ is N or $CR_{P3}$;
$P_4$ is N or $CR_{P4}$;
and alternatively, at least one of $P_3$ and $P_4$ is N;
$R_{P1}$, $R_{P2}$, $R_{P3}$ and $R_{P4}$ are independently H, D, halogen, -CN, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, halogen, -CN, $C_{1-6}$ alkyl or $C_{1-6}$ haloalkyl; wherein the above groups are optionally substituted with 1 to 6 (e.g. 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
or $R_{P1}$ and $R_{P2}$, $R_{P2}$ and $R_{P3}$, or $R_{P3}$ and $R_{P4}$, and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

In another specific embodiment, R is

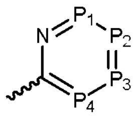

,

wherein

$P_1$ is $CR_{P1}$;
$P_2$ is $CR_{P2}$;
$P_3$ is N or $CR_{P3}$;
$P_4$ is N or $CR_{P4}$;
and at least one of $P_3$ and $P_4$ is N;
$R_{P1}$ is H, D, halogen, -CN, $C_{1-6}$ alkyl or $C_{1-6}$ haloalkyl; wherein the above groups are optionally substituted with one or more D, up to fully deuterated;
$R_{P2}$ is H, D, halogen or -CN;
$R_{P3}$ is H, D, $C_{1-6}$ alkyl or $C_{1-6}$ haloalkyl; wherein the above groups are optionally substituted with one or more D, up to fully deuterated;
$R_{P4}$ is H or D.

In another specific embodiment, R is

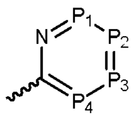

,

wherein

$P_1$ is $CR_{P1}$;
$P_2$ is $CR_{P2}$;
$P_3$ is N or $CR_{P3}$;
$P_4$ is N or $CR_{P4}$;
and at least one of $P_3$ and $P_4$ is N;
$R_{P1}$ is H, D, -CN or Me;
$R_{P2}$ is H, D, F or -CN;
$R_{P3}$ is H, D or Me;
$R_{P4}$ is H or D.

In another specific embodiment, R is

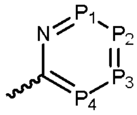

wherein

$P_1$ is $CR_{P1}$;
$P_2$ is N or $CR_{P2}$;
$P_3$ is N or $CR_{P3}$;
$P_4$ is $CR_{P4}$;
$R_{P1}$, $R_{P2}$, $R_{P3}$ and $R_{P4}$ are independently H, D, halogen, -CN, $C_{1-6}$ alkyl or $C_{1-6}$ haloalkyl; wherein the above groups are optionally substituted with one or more D, up to fully deuterated.

In another specific embodiment, R is

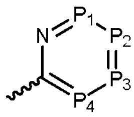

wherein

$P_1$ is $CR_{P1}$;
$P_2$ is N or $CR_{P2}$;
$P_3$ is N or $CR_{P3}$;
$P_4$ is $CR_{P4}$;
$R_{P1}$ is H or D;
$R_{P2}$ is H or D;
$R_{P3}$ is H or D;
$R_{P4}$ is H or D.

In another specific embodiment, R is

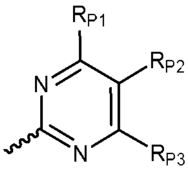

wherein

$R_{P1}$, $R_{P2}$ and $R_{P3}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g. 1, 2, 3, 4, 5 or 6) R";
or $R_{P1}$ and $R_{P2}$, or $R_{P2}$ and $R_{P3}$, and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

In another specific embodiment, R is

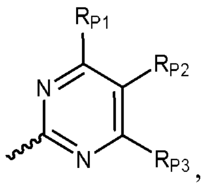

wherein

$R_{P1}$, $R_{P2}$ and $R_{P3}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
or $R_{P1}$ and $R_{P2}$, or $R_{P2}$ and $R_{P3}$, and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

In another specific embodiment, R is

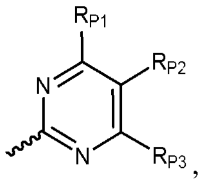

wherein

$R_{P1}$ is H, D, halogen, -CN, $C_{1-3}$ alkyl, $C_{1-3}$ haloalkyl, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
$R_{P2}$ is H;
$R_{P3}$ is H, $C_{1-3}$ alkyl or $C_{1-3}$ haloalkyl; wherein the above groups are optionally substituted with 1 to 6 (e.g. 1, 2, 3, 4, 5 or 6) R".

In another specific embodiment, R is

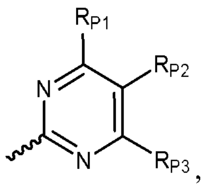

wherein

$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3-to 7-membered heterocyclyl, phenyl or 5- or 6-membered heteroaryl; wherein the above groups are optionally substituted with 1 to 6 (e.g. 1, 2, 3, 4, 5 or 6) R".

In another specific embodiment, R is,

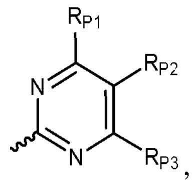

wherein

$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, 3- to 7-membered heterocyclyl or phenyl; wherein the above groups are optionally substituted with 1 to 6 (e.g. 1, 2, 3, 4, 5 or 6) R".

In another specific embodiment, R is

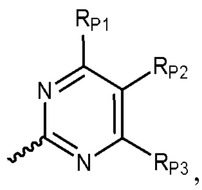

wherein

$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy or phenyl; wherein the above groups are optionally substituted with one or more D, up to fully deuterated.

In one embodiment, each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R'. In another embodiment, each of $R_A$, $R_B$ and $R_C$ is independently H, D, $C_{1-6}$ alkyl, $C_{1-6}$ haloalkyl, $C_{3-6}$ cycloalkyl or 3- to 7-membered heterocyclyl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R'. In another embodiment, each of $R_A$, $R_B$ and $R_C$ is independently H, D, $C_{1-3}$ alkyl, $C_{1-3}$ haloalkyl, $C_{3-6}$ cycloalkyl or 3- to 7-membered heterocyclyl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R'. In another embodiment, each of $R_A$, $R_B$ and $R_C$ is independently H, D, $C_{1-3}$ alkyl, $C_{1-3}$ haloalkyl or $C_{3-6}$ cycloalkyl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R'.

Y, W, $R_Y$, $R_1$, $R_1$', $R_b$, R* and $Q_1$-$Q_3$

In one embodiment, Y is CRy; in one embodiment, Y is N.

In one specific embodiment, Y is CRy, and wherein Ry is H, D or halogen. In another specific embodiment, Y is CRy, and wherein $R_Y$ is H, D or F. In another specific embodiment, Y is CRy, and wherein Ry is H or D.

In one embodiment, W is $CR_W$; in one embodiment, W is N.

In one specific embodiment, W is $CR_W$, and wherein $R_W$ is H, D, halogen or $-NH_2$. In another specific embodiment, W is $CR_W$, and wherein $R_W$ is H, D or halogen. In another specific embodiment, W is $CR_W$, and wherein $R_W$ is H, D or F. In another specific embodiment, W is $CR_W$, and wherein $R_W$ is H or D.

In one embodiment, $R_1$ is 3- to 10-membered heterocyclyl, which is optionally substituted with 1 to 6 (e.g., 1, 2, 3, 4, 5 or 6) $R_b$. In another embodiment, $R_1$ is 4- to 10-membered heterocyclyl, which is optionally substituted with 1 to 6 (e.g., 1, 2, 3, 4, 5 or 6) $R_b$. In another embodiment, $R_1$ is 4- to 7-membered heterocyclyl, which is optionally substituted with 1 to 6 (e.g., 1, 2, 3, 4, 5 or 6) $R_b$. In another embodiment, $R_1$ is 5-membered heterocyclyl, which is optionally substituted with 1 to 6 (e.g., 1, 2, 3, 4, 5 or 6) $R_b$. In another embodiment, $R_1$ is tetrahydropyrrole ring, which is optionally substituted with 1 to 4 (e.g., 1, 2, 3 or 4) $R_b$. In another specific embodiment, $R_1$ is

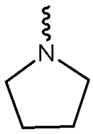, 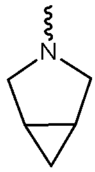, 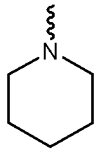, 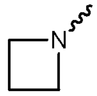, 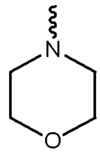, 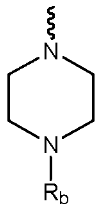, 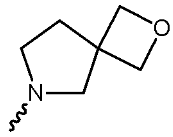,

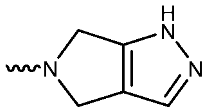, 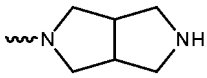, 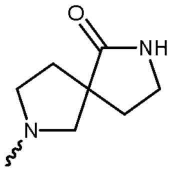

or

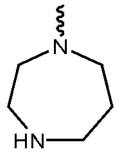,

wherein the above groups are optionally substituted with 1 to 6 (e.g., 1, 2, 3, 4, 5 or 6) $R_b$. In another specific embodiment, $R_1$ is

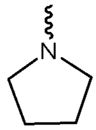,

wherein the above group is optionally substituted with 1 to 4 (e.g., 1, 2, 3 or 4) $R_b$.

In one embodiment, $R_1$' is H, D, halogen or $-NH_2$. In another embodiment, $R_1$' is H, D or $-NH_2$. In another embodiment, $R_1$' is H or $-NH_2$. In another embodiment, $R_1$' is H. In another embodiment, $R_1$' is $-NH_2$. In another embodiment, $R_1$' is -F.

In another embodiment, each $R_b$ is H, D, halogen, -CN, $-OR_A$, $-NR_BR_C$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated. In another embodiment, each $R_b$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated. In another embodiment, each $R_b$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl or 3- to 7-membered heterocyclyl; wherein the above groups are optionally substituted with one or more D, up to fully deuterated. In another embodiment, each $R_b$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated. In another embodiment, each $R_b$ is H, D, halogen, -CN, -OH, $-NH_2$, $-NHC_{1-6}$ alkyl, $-N(C_{1-6}$ alkyl$)_2$, $C_{1-6}$ alkyl or $C_{1-6}$ haloalkyl; wherein the above groups are optionally substituted with one or more D, up to fully deuterated. In another embodiment, each $R_b$ is H, D, halogen, -CN, -OH, $-NH_2$, $-NHC_{1-6}$ alkyl or $-N(C_{1-6}$ alkyl$)_2$; wherein the above groups are optionally substituted with one or more D, up to fully deuterated. In another embodiment, each $R_b$ is halogen, -CN, -OH, $-NH_2$, $-NHC_{1-6}$ alkyl or $-N(C_{1-6}$ alkyl$)_2$; wherein the above groups are optionally substituted with one or more D, up to fully deuterated. In another embodiment, each $R_b$ is halogen, -OH, $-NH_2$, $-NHC_{1-6}$ alkyl or $-N(C_{1-6}$ alkyl$)_2$; wherein the above groups are optionally substituted with one or more D, up to fully deuterated. In another embodiment, each $R_b$ is halogen, - OH, $-NH_2$, $-NHC_{1-3}$ alkyl or $-N(C_{1-3}$ alkyl$)_2$; wherein the above groups are optionally substituted with one or more D, up to fully deuterated. In another embodiment, each $R_b$ is -F or -OH. In another embodiment, each $R_b$ is -F. In another embodiment, each $R_b$ is -OH.

In one embodiment, $R_1$, $R_Y$ and the carbon atoms to which they are attached are taken together to form $C_{3-8}$ cycloalkyl, 4- to 10-membered heterocyclyl, $C_{6-10}$ aryl or 5- to 10-membered heteroaryl, which is optionally substituted with

1 to 6 (e.g., 1, 2, 3, 4, 5 or 6) R*. In another embodiment, $R_1$, $R_Y$ and the carbon atoms to which they are attached are taken together to form $C_{3-8}$ cycloalkyl, which is optionally substituted with 1 to 6 (e.g., 1, 2, 3, 4, 5 or 6) R*. In another embodiment, $R_1$, $R_Y$ and the carbon atoms to which they are attached are taken together to form 4- to 10-membered heterocyclyl, which is optionally substituted with 1 to 6 (e.g., 1, 2, 3, 4, 5 or 6) R*. In another embodiment, $R_1$, $R_Y$ and the carbon atoms to which they are attached are taken together to form $C_{6-10}$ aryl, which is optionally substituted with 1 to 6 (e.g., 1, 2, 3, 4, 5 or 6) R*. In another embodiment, $R_1$, $R_Y$ and the carbon atoms to which they are attached are taken together to form 5- to 10-membered heteroaryl, which is optionally substituted with 1 to 6 (e.g., 1, 2, 3, 4, 5 or 6) R*.

In another embodiment, $R_1$, $R_Y$ and the carbon atoms to which they are attached are taken together to form 4- to 10-membered heterocyclyl or 5- to 10-membered heteroaryl, which is optionally substituted with 1 to 6 (e.g., 1, 2, 3, 4, 5 or 6) R*. In another embodiment, $R_1$, $R_Y$ and the carbon atoms to which they are attached are taken together to form 5-membered heteroaryl, which is optionally substituted with 1 to 3 (e.g., 1, 2 or 3) R*. In another embodiment, $R_1$, $R_Y$ and the carbon atoms to which they are attached are taken together to form

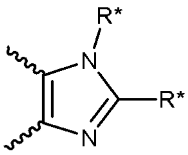

,

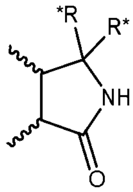

or

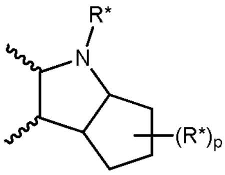

,

wherein p is 0, 1, 2, 3, 4, 5 or 6.

In one embodiment, each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1 to 6 (e.g. 1, 2, 3, 4, 5 or 6) R'. In another embodiment, each R* is independently $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1 to 6 (e.g. 1, 2, 3, 4, 5 or 6) R'. In another embodiment, each R* is independently $C_{1-6}$ alkyl, $C_{1-6}$ haloalkyl, $C_{3-6}$ cycloalkyl or 3- to 7-membered heterocyclyl, or two R* and the atom(s) to which they are attached are taken together to form $C_{3-8}$ cycloalkyl or 4- to 10-membered heterocyclyl; wherein the above groups are optionally substituted with 1 to 6 (e.g. 1, 2, 3, 4, 5 or 6) R'. In another embodiment, each R* is independently $C_{1-6}$ alkyl or $C_{1-6}$ haloalkyl, or two R* and the atom(s) to which they are attached are taken together to form $C_{3-8}$ cycloalkyl or 4- to 10-membered heterocyclyl; wherein the above groups are optionally substituted with 1 to 6 (e.g. 1, 2, 3, 4, 5 or 6) R'. In another embodiment, two R* and the atom(s) to which they are attached are taken together to form $C_{3-8}$ cycloalkyl or 4-to 10-membered heterocyclyl; wherein the above groups are optionally substituted with 1 to 6 (e.g. 1, 2, 3, 4, 5 or 6) R'.

In another specific embodiment, two R* and the atoms to which they are attached are taken together to form

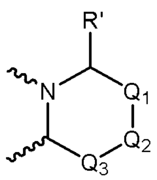

,

wherein $Q_1$ is $C(R')_2$ or $C(R')_2C(R')_2$; $Q_2$ is $C(R')_2$, O, S, S(O) or $S(O)_2$; $Q_3$ is $C(R')_2$, $C(R')_2C(R')_2$, O, S, S(O) or $S(O)_2$. In another specific embodiment, two R* and the atoms to which they are attached are taken together to form

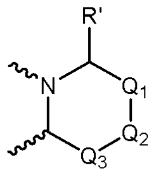

, wherein R' is $CH_3$, $Q_1$ is $(CH_2)_2$, CHD, $(CHD)_2$, $CD_2$ or $(CD_2)_2$; $Q_2$ is $CH_2$, CHD, $CD_2$, O, S, S(O) or $S(O)_2$; $Q_3$ is $C(R')_2$, S or $S(O)_2$;

In another specific embodiment, two R* and the atoms to which they are attached are taken together to form

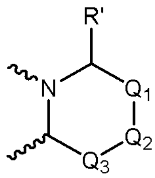

,

wherein R' is $CH_3$, $Q_1$ is $CH_2$, CHD or $CD_2$; $Q_2$ is O, S, $CH_2$, CHD or $CD_2$; $Q_3$ is O, S, $CH_2$, CHD or $CD_2$. In another specific embodiment, two R* and the atoms to which they are attached are taken together to form

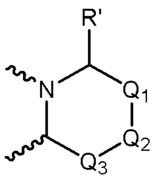

,

wherein R' is $CH_3$, $Q_1$ is $CH_2$, CHD or $CD_2$; $Q_2$ is O, S, $CH_2$, CHD or $CD_2$; $Q_3$ is O, S, $CH_2$, CHD or $CD_2$.

$Y_1$, $Y_2$ and $R_2$

In one embodiment, $Y_1$ is $CR_{Y1}$; in another embodiment, $Y_1$ is N.

In one embodiment, $Y_2$ is $CR_{Y2}$; in another embodiment, $Y_2$ is N.

In one embodiment, $R_{Y1}$, $R_{Y2}$ and $R_2$ are independently H, D, halogen, -CN, $-NO_2$, $C_{1-6}$ alkyl or $C_{1-6}$ haloalkyl; wherein the above groups are optionally substituted with 1 to 6 (e.g., 1, 2, 3, 4, 5 or 6) $R_c$. In another embodiment, $R_{Y1}$, $R_{Y2}$ and $R_2$ are independently H, D or halogen. In another embodiment, $R_{Y1}$, $R_{Y2}$ and $R_2$ are independently H or D.

In another embodiment, each $R_c$ is H, D, halogen, -CN, $-OR_A$, $-NR_BR_C$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated. In another embodiment, each $R_c$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated. In another embodiment, each $R_c$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl or 3- to 7-membered heterocyclyl; wherein the above groups are optionally substituted with one or more D, up to fully deuterated. In another embodiment, each $R_c$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated. In another embodiment, each $R_c$ is H, D, halogen, -CN, -OH, $-NH_2$, $-NHC_{1-6}$ alkyl, $-N(C_{1-6}$ alkyl$)_2$, $C_{1-6}$ alkyl or $C_{1-6}$ haloalkyl; wherein the above groups are optionally substituted with one or more D, up to fully deuterated. In another embodiment, each $R_c$ is H, D, halogen, -CN, -OH, $-NH_2$, $-NHC_{1-6}$ alkyl or $-N(C_{1-6}$ alkyl$)_2$; wherein the above groups are optionally substituted with one or more D, up to fully deuterated.

Z and $R_z$

In one embodiment, Z is a chemical bond; in one embodiment, Z is O; in one embodiment, Z is $S(O)_{0-2}$, such as S(O), $S(O)_1$,or $S(O)_2$.

In one embodiment, $R_Z$ is H, D, $C_{1-6}$ alkyl or $C_{1-6}$ haloalkyl; wherein the above groups are optionally substituted with one or more D. In one embodiment, $R_Z$ is H, D, $C_{1-3}$ alkyl or $C_{1-3}$ haloalkyl; wherein the above groups are optionally

substituted with one or more D. In one embodiment, $R_Z$ is $C_{1-3}$ haloalkyl; wherein the above group is optionally substituted with one or more D. In one embodiment, $R_Z$ is $CF_2Cl$.

In one embodiment, -Z-$R_Z$ together represents -$SF_5$.

R' and R"

In one embodiment, each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R". In another embodiment, each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1 to 6 (e.g., 1, 2, 3, 4, 5 or 6) R". In one embodiment, each R' is independently H, D, halogen, -OH, $C_{1-6}$ alkyl or $C_{1-6}$ haloalkyl; wherein the above groups are optionally substituted with one or more D, up to fully deuterated. In another embodiment, each R' is independently H, D, F or -OH. In another embodiment, each R' is independently H, D or F.

In another embodiment, two adjacent R' and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated. In another embodiment, two adjacent R' and the atoms to which they are attached are taken together to form $C_{6-10}$ aryl; wherein the above group is optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated. In another embodiment, two adjacent R' and the atoms to which they are attached are taken together to form 5- to 10-membered heteroaryl; wherein the above group is optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

In one embodiment, each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated. In another embodiment, each R" is independently H, D, halogen, -CN, -OH, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

Any technical solution in any one of the above specific embodiments, or any combination thereof, may be combined with any technical solution in other specific embodiments or any combination thereof. For example, any technical solution of $X_1$-$X_{6>}$ $R_a$, R, $R_A$-$R_c$, $P_1$-$P_4$, Y, W, $R_Y$, $R_1$, $R_b$, R*, $Q_1$-$Q_3$, $Y_1$, $Y_2$, $R_2$, Z, $R_z$, R' and R", or any combination thereof may be combined. The present disclosure is intended to include all combination of such technical solutions, which are not exhaustively listed here to save space.

In a more specific embodiment, the present disclosure relates to the following technical solutions:

1. A compound of formula (I), or a tautomer, a stereoisomer, a prodrug, a crystal form, a pharmaceutically acceptable salt, a hydrate or a solvate thereof:

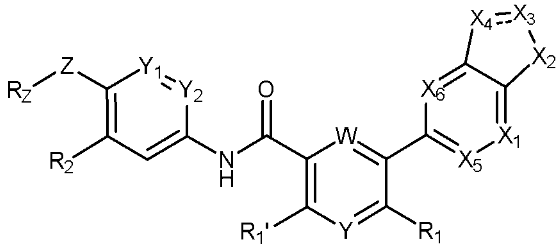

(I)

wherein

$X_1$ is N or $CR_{X1}$;
$X_2$ is $CR_{X2}R_{X2'}$ or C(O);
$X_3$ is $CR_{X3}R_{X3'}$, $CR_{X3}$ or $NR_{X3}$;
$X_4$ is $CR_{X4}Rx_{4'}$, $CR_{X4}$, $NR_{X4}$ or C(O);
$X_5$ is N or $CR_{X5}$;
$X_6$ is N or $CR_{X6}$;
- - - - is single or double bonds;

$R_{X1}$ is H, D, halogen, $C_{1-6}$ alkyl or $C_{1-6}$ haloalkyl; wherein the above groups are optionally substituted with one or more D, up to fully deuterated;

$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;

$R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with one or more $R_a$;

$R_{X4}$ and $R_{X4'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more $R_a$;

or, $R_{X3}$, $R_{X4}$ and the atoms to which they are attached are taken together to form $C_{3-8}$ cycloalkyl, 4- to 10-membered heterocyclyl, $C_{6-10}$ aryl or 5- to 10-membered heteroaryl; wherein the above groups are optionally substituted with one or more R;

$R_{X5}$ is H, D, halogen, $C_{1-6}$ alkyl or $C_{1-6}$ haloalkyl; wherein the above groups are optionally substituted with one or more D, up to fully deuterated;

$R_{X6}$ is H, D or halogen;

Y is $CR_Y$ or N;

$R_1$ is 3- to 10-membered heterocyclyl, which is optionally substituted with one or more $R_b$;

Ry is H, D or halogen;

or, $R_1$, $R_Y$ and the carbon atoms to which they are attached are taken together to form $C_{3-8}$ cycloalkyl, 4- to 10-membered heterocyclyl, $C_{6-10}$ aryl or 5- to 10-membered heteroaryl, which is optionally substituted with one or more R*;

$R_1$' is H, D, halogen or $-NH_2$;

W is $CR_W$ or N;

$R_W$ is selected from H, D, halogen and $-NH_2$;

$Y_1$ is $CR_{Y1}$ or N;

$Y_2$ is $CR_{Y2}$ or N;

$R_{Y1}$, $R_{Y2}$ and $R_2$ are independently H, D, halogen, -CN, $-NO_2$, $C_{1-6}$ alkyl or $C_{1-6}$ haloalkyl; wherein the above groups are optionally substituted with one or more $R_c$;

Z is a chemical bond, O or $S(O)_{0-2}$;

$R_Z$ is H, D, $C_{1-6}$ alkyl or $C_{1-6}$ haloalkyl; wherein the above groups are optionally substituted with one or more D, up to fully deuterated;

or $-Z-R_Z$ together represents $-SF_5$;

each of $R_a$, $R_b$ and $R_c$ is independently H, D, halogen, -CN, $-OR_A$, $-NR_BR_C$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;

each R is independently H, D, halogen, -CN, $-OR_A$, $-C(O)R_A$, $-NR_BR_C$, $-NR_AC(O)R_B$, $-S(O)_2R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with one or more R';

each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4-to 10-membered heterocyclyl; wherein the above groups are optionally substituted with one or more R';

wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with one or more R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more R";

or two adjacent R' and the atoms to which they are attached are taken together to form $C_{6-10}$ aryl or 5-to 10-membered heteroaryl; wherein the above groups are optionally substituted with one or more -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;

each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated;

with the proviso that, when $X_1$ is N, $X_2$ is $CH_2$, $X_3$ is $NR_{X3}$, $X_4$ is C(O), and $X_5$ is CH, Y is not N.

2. The compound according to technical solution 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$ or C(O);
$X_3$ is $CR_{X3}R_{X3'}$ or $NR_{X3}$;
$X_4$ is C(O);
$X_5$ is N or $CR_{X5}$;
$X_6$ is N or $CR_{X6}$, alternatively $CR_{X6}$;
- - - - is single bond;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with 1 to 6 (e.g., 1, 2, 3, 4, 5 or 6) $R_a$;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{3-8}$ cycloalkyl, 4- to 10-membered heterocyclyl, $C_{6-10}$ aryl or 5- to 10-membered heteroaryl, which is optionally substituted with 1 to 6 (e.g., 1, 2, 3, 4, 5 or 6) R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl; wherein the above groups are optionally substituted with one or more D, up to fully deuterated;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
each $R_a$ is independently H, D, halogen, -CN or $-NR_BR_C$;
each R* is independently H, D, halogen, -CN, -OH, oxo, $-NR_AC(O)R_B$, $C_{1-6}$ alkyl or $C_{1-6}$ haloalkyl, or two R* and the atom(s) to which they are attached are taken together to form $C_{3-8}$ cycloalkyl or 4- to 10-membered heterocyclyl; wherein the above groups are optionally substituted with 1 to 6 (e.g., 1, 2, 3, 4, 5 or 6) R';
wherein $R_A$, $R_B$ and $R_C$ are independently H, D, $C_{1-6}$ alkyl or $C_{1-6}$ haloalkyl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated.

3. The compound according to technical solution 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single or double bonds;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered

heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy or N;
$R_1$ is 3- to 10-membered heterocyclyl, which is optionally substituted with 1 to 6 (e.g., 1, 2, 3, 4, 5 or 6) $R_b$;
Ry is H, D or halogen;
or, $R_1$, $R_Y$ and the carbon atoms to which they are attached are taken together to form $C_{3-8}$ cycloalkyl, 4- to 10-membered heterocyclyl, $C_{6-10}$ aryl or 5- to 10-membered heteroaryl, which is optionally substituted with 1 to 6 (e.g., 1, 2, 3, 4, 5 or 6) R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl; wherein the above groups are optionally substituted with one or more D, up to fully deuterated;
Z is a chemical bond, O or $S(O)_{0-2;}$
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
each $R_b$ is independently H, D, halogen, -CN, $-OR_A$, $-NR_BR_C$, $C_{1-6}$ alkyl, $C_{1-6}$ haloalkyl, $C_{3-6}$ cycloalkyl, 3- to 7-membered heterocyclyl, alkenyl, alkynyl, $C_{6-10}$ aryl or 5- to 10-membered heteroaryl; wherein the above groups are optionally substituted with one or more D, up to fully deuterated;
each R is independently H, D, halogen, -CN, $-OR_A$, $-C(O)R_A$, $-NR_BR_C$, $-NR_AC(O)R_B$, $-S(O)_2R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4-to 10-membered heterocyclyl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

4. The compound according to technical solution 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$ or C(O);
$X_3$ is $CR_{X3}R_{X3'}$ or $NR_{X3}$;
$X_4$ is C(O);
$X_5$ is N or $CR_{X5}$;
$X_6$ is N or $CR_{X6}$, alternatively $CR_{X6}$;
- - - - is single bond;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2}$, are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;

$R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with 1 to 6 (e.g., 1, 2, 3, 4, 5 or 6) $R_a$;

$R_{X5}$ is H, D, halogen, $C_{1-6}$ alkyl or $C_{1-6}$ haloalkyl; wherein the above groups are optionally substituted with one or more D, up to fully deuterated;

$R_{X6}$ is H, D or halogen, alternatively H or D;

Y is CRy;

and $R_1$, $R_Y$ and the carbon atoms to which they are attached are taken together to form $C_{6-10}$ aryl or 5-to 10-membered heteroaryl, which is optionally substituted with two or more R*;

$R_1$' is H, D, halogen or $-NH_2$, alternatively H or D;

W is $CR_W$ or N, alternatively $CR_W$;

$R_W$ is selected from H, D, halogen and $-NH_2$, alternatively H or D;

$Y_1$ is $CR_{Y1}$ or N;

$Y_2$ is $CR_{Y2}$ or N;

$R_{Y1}$, $R_{Y2}$ and $R_2$ are independently H, D, halogen, -CN, $-NO_2$, $C_{1-6}$ alkyl or $C_{1-6}$ haloalkyl;

Z is a chemical bond, O or $S(O)_{0-2}$;

$R_Z$ is H, D, $C_{1-6}$ alkyl or $C_{1-6}$ haloalkyl; wherein the above groups are optionally substituted with 1 to 6 (e.g., 1, 2, 3, 4, 5 or 6) D, up to fully deuterated;

or $-Z-R_Z$ together represents $-SF_5$;

each $R_a$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;

each R* is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1 to 6 (e.g., 1, 2, 3, 4, 5 or 6) R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";

each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

5. The compound according to technical solution 1, or a tautomer, a stereoisomer, a prodrug, a crystal form, a pharmaceutically acceptable salt, a hydrate or a solvate thereof, wherein

$X_1$ is N or $CR_{X1}$;

$X_2$ is $CR_{X2}R_{X2'}$ or C(O);

$X_3$ is $NR_{X3}$;

$X_4$ is C(O);

$X_5$ is N or $CR_{X5}$;

$X_6$ is N or $CR_{X6}$, alternatively $CR_{X6}$;

- - - - is single bond;

$R_{X1}$ is H, D, halogen, $C_{1-6}$ alkyl or $C_{1-6}$ haloalkyl; wherein the above groups are optionally substituted with one or more D, up to fully deuterated;

$R_{X2}$ and $R_{X2'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;

$R_{X3}$ is H, $C_{1-6}$ alkyl, $C_{1-6}$ haloalkyl or $C_{3-6}$ cycloalkyl; wherein the above groups are optionally substituted with one or more D, up to fully deuterated;

$R_{X5}$ is H, D, halogen, $C_{1-6}$ alkyl or $C_{1-6}$ haloalkyl; wherein the above groups are optionally substituted with one or more D, up to fully deuterated;

$R_{X6}$ is H, D or halogen, alternatively H or D;

Y is CRy;

and $R_1$, $R_Y$ and the carbon atoms to which they are attached are taken together to form $C_{6-10}$ aryl or 5-to 10-membered heteroaryl, which is optionally substituted with two or more R*;

$R_1$' is H, D, halogen or $-NH_2$, alternatively H or D;

W is $CR_W$ or N, alternatively $CR_W$;

$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1 to 6 (e.g. 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated.

6. The compound according to technical solution 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$;
$X_3$ is $NR_{X3}$;
$X_4$ is C(O);
$X_5$ is N or $CR_{X5}$;
$X_6$ is N or $CR_{X6}$, alternatively $CR_{X6}$;
- - - - is single bond;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$ is H, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5-to 10-membered heteroaryl, which is optionally substituted with two or more R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1 to 6 (e.g. 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated.

7. The compound according to technical solution 1, or a tautomer, a stereoisomer, a prodrug, a crystal form, a pharmaceutically acceptable salt, a hydrate or a solvate thereof, wherein

$X_1$ is N or $CR_{X1}$;

$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single or double bonds;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form 5-membered heteroaryl; which is optionally substituted with 1 to 3 (e.g., 1, 2 or 3) R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is $CR_Y$ or N;
$R_1$ is 3- to 10-membered heterocyclyl, which is optionally substituted with 1 to 6 (e.g., 1, 2, 3, 4, 5 or 6) $R_b$;
Ry is H, D or halogen;
or, $R_1$, $R_Y$ and the carbon atoms to which they are attached are taken together to form $C_{6-10}$ aryl or 5-to 10-membered heteroaryl, which is optionally substituted with 1 to 6 (e.g., 1, 2, 3, 4, 5 or 6) R*;
$R_1$' is H, D, halogen or -$NH_2$, alternatively H or D;
W is $CR_W$ or N, alternatively $CR_W$;
$R_W$ is selected from H, D, halogen and -$NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, -$NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or -Z-$R_Z$ together represents -$SF_5$;
each $R_b$ is independently H, D, halogen, -CN, -OH, -$NH_2$, -NH$C_{1-6}$ alkyl, -N($C_{1-6}$ alkyl)$_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
each R is independently H, D, halogen, -CN, -OH, -C(O)$R_A$, -$NR_BR_C$, -$NR_A$C(O)$R_B$, -$S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R* is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

8. The compound according to technical solution 1, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$ or C(O);
$X_3$ is $CR_{X3}$;

$X_4$ is $CR_{X4}$;
$X_5$ is N or $CR_{X5}$;
$X_6$ is N or $CR_{X6}$, alternatively $CR_{X6}$;
- - - - is single bond;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form 5-membered heteroaryl; which is optionally substituted with 1 to 3 (e.g., 1, 2 or 3) R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is $CR_Y$ or N;
$R_1$ is 5- to 6-membered heterocyclyl, which is optionally substituted with 1 to 6 (e.g., 1, 2, 3, 4, 5 or 6) $R_b$;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
Ry is H, D or halogen;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
each $R_b$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
each R is independently H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

9. The compound according to technical solution 1, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X2}$ and $R_{X2'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) R;
$R_{X5}$ is H, D, halogen, $C_{1-6}$ alkyl or $C_{1-6}$ haloalkyl; wherein the above groups are optionally substituted with one or more D, up to fully deuterated;

$R_{X6}$ is H, D or halogen, alternatively H or D;
Y is CRy;
and $R_1$, $R_Y$ and the carbon atoms to which they are attached are taken together to form $C_{6-10}$ aryl or 5-to 10-membered heteroaryl, which is optionally substituted with two or more R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
each R is independently H, D, halogen, -CN, $-C(O)R_A$, $C_{1-6}$ alkyl or $C_{1-6}$ haloalkyl; wherein the above groups are optionally substituted with one or more D, up to fully deuterated;
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1 to 6 (e.g. 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated;
$R_A$ is H, D, $C_{1-6}$ alkyl, $C_{1-6}$ haloalkyl, $C_{3-6}$ cycloalkyl or 3- to 7-membered heterocyclyl.

10. The compound according to technical solution 1, or a tautomer, a stereoisomer, a prodrug, a crystal form, a pharmaceutically acceptable salt, a hydrate or a solvate thereof, wherein

$X_1$ is N or CRxiN;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2}$, are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5-to 10-membered heteroaryl, which is optionally substituted with two or more R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
each R is independently H, D, $-C(O)R_A$, halogen, -CN, $C_{1-6}$ alkyl, $C_{1-6}$ haloalkyl, $C_{3-6}$ cycloalkyl, 3-to 7-membered heterocyclyl, $C_{6-10}$ aryl or 5- to 10-membered heteroaryl; wherein the above groups are optionally substituted with

1 to 6 (e.g., 1, 2, 3, 4, 5 or 6) R'; wherein the above groups are optionally substituted with one or more D, up to fully deuterated;

wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1 to 6 (e.g. 1, 2, 3, 4, 5 or 6) R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated;

$R_A$ is H, D, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated.

11. The compound according to technical solution 1, or a tautomer, a stereoisomer, a prodrug, a crystal form, a pharmaceutically acceptable salt, a hydrate or a solvate thereof, wherein

$X_1$ is N;

$X_2$ is $CR_{X2}R_{X2'}$ or C(O);

$X_3$ is $CR_{X3}$;

$X_4$ is $CR_{X4}$;

$X_5$ is N or $CR_{X5}$;

$X_6$ is N or $CR_{X6}$, alternatively $CR_{X6}$;

- - - - is single bond;

$R_{X2}$ and $R_{X2'}$ are independently H, D, OH, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;

$R_{X3}$, $R_{X4}$ and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) R;

$R_{X5}$ is H, D, halogen, $C_{1-6}$ alkyl or $C_{1-6}$ haloalkyl; wherein the above groups are optionally substituted with one or more D, up to fully deuterated;

$R_{X6}$ is H, D or halogen, alternatively H or D;

Y is CRy;

and $R_1$, $R_Y$ and the carbon atoms to which they are attached are taken together to form $C_{6-10}$ aryl or 5-to 10-membered heteroaryl, which is optionally substituted with two or more R*;

$R_1'$ is H, D, halogen or $-NH_2$, alternatively H or D;

W is $CR_W$ or N, alternatively $CR_W$;

$R_W$ is selected from H, D, halogen and $-NH_2$, alternatively H or D;

$Y_1$ is $CR_{Y1}$ or N;

$Y_2$ is $CR_{Y2}$ or N;

$R_{Y1}$, $R_{Y2}$ and $R_2$ are independently H, D, halogen, -CN, $-NO_2$, $C_{1-6}$ alkyl or $C_{1-6}$ haloalkyl;

Z is a chemical bond, O or $S(O)_{0-2}$;

$R_Z$ is H, D, $C_{1-6}$ alkyl or $C_{1-6}$ haloalkyl; wherein the above groups are optionally substituted with one or more D, up to fully deuterated;

or $-Z-R_Z$ together represents $-SF_5$;

wherein one R is pyrimidin-2-yl, pyrimidin-4-yl, pyrazin-2-yl, triazinyl or tetrazinyl, and the other R are independently H, D, halogen, -CN, $C_{1-6}$ alkyl or $C_{1-6}$ haloalkyl; wherein the above groups are optionally substituted with 1 to 6 (e.g., 1, 2, 3, 4, 5 or 6) R';

wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1 to 6 (e.g. 1, 2, 3, 4, 5 or 6) R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated;

or two adjacent R' and the atoms to which they are attached are taken together to form $C_{6-10}$ aryl or 5-to 10-membered heteroaryl; wherein the above groups are optionally substituted with 1 to 6 (e.g. 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

12. The compound according to technical solution 1, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X2}$ and $R_{X2'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5-to 10-membered heteroaryl, which is optionally substituted with two or more R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
wherein one R is pyridin-2-yl, pyrimidin-4-yl or pyrazin-2-yl, and the other R are independently H, D, halogen, -CN, $C_{1-6}$ alkyl or $C_{1-6}$ haloalkyl; wherein the above groups are optionally substituted with 1 to 6 (e.g., 1, 2, 3, 4, 5 or 6) R';
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1 to 6 (e.g. 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated.

13. The compound according to technical solution 1, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$;
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X2}$ and $R_{X2'}$ are independently H, D, halogen, OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5-to 10-membered heteroaryl, which is optionally substituted with two or more R*;
$R_1$' is H, D, halogen or $-NH_2$, alternatively H or D;
W is $CR_W$ or N, alternatively $CR_W$;

$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
wherein one R is pyrimidin-2-yl, and the other R are independently H, D, halogen, -CN, $C_{1-6}$ alkyl or $C_{1-6}$ haloalkyl; wherein the above groups are optionally substituted with 1 to 6 (e.g., 1, 2, 3, 4, 5 or 6) R';
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1 to 6 (e.g. 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated;
or two adjacent R' and the atoms to which they are attached are taken together to form $C_{6-10}$ aryl or 5-to 10-membered heteroaryl; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

14. The compound according to technical solution 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X1}$ is H, D, halogen, $C_{1-6}$ alkyl or $C_{1-6}$ haloalkyl; wherein the above groups are optionally substituted with 1 to 6 (e.g., 1, 2, 3, 4, 5 or 6) D, up to fully deuterated;
$R_{X2}$ and $R_{X2'}$ are independently H, D, halogen, OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{3-8}$ cycloalkyl or 4- to 10-membered heterocyclyl, which is optionally substituted with two or more R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to

which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4-to 10-membered heterocyclyl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated.

15. The compound according to any one of technical solutions 1 to 14, or a tautomer, a stereoisomer, a prodrug, a crystal form, a pharmaceutically acceptable salt, a hydrate or a solvate thereof, and the compound is a compound of formula (II):

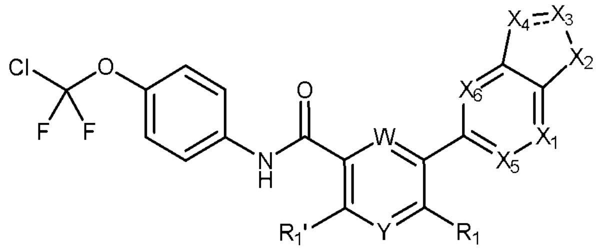
(II)

wherein $X_1$-$X_6$, Y, W, $R_1$ and $R_1$' are defined as in any one of technical solutions 1 to 14.
16. The compound according to technical solution 15, or a tautomer, a stereoisomer, a prodrug, a crystal form, a pharmaceutically acceptable salt, a hydrate or a solvate thereof, and the compound is a compound of formula (III):

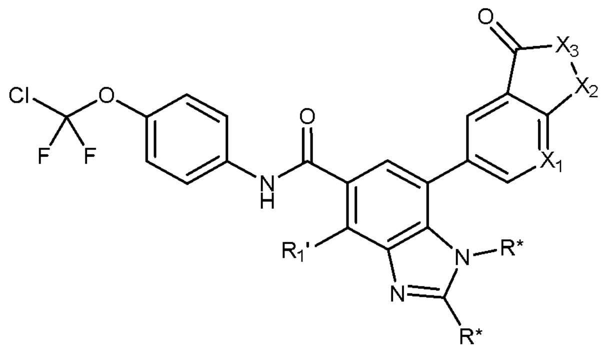
(III)

wherein

$X_1$ is N or $CR_{X1}$, alternatively N;
$X_2$ is $CR_{X2}R_{X2'}$ or C(O), alternatively $CR_{X2}R_{X2'}$;
$X_3$ is $CR_{X3}R_{X3'}$ or $NR_{X3}$;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$ and $R_{X3}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3}$, and the carbon atom to which they are attached are taken together to form $C_{1-6}$ alkylidene; wherein the above groups are optionally substituted with 1 to 6 (e.g., 1, 2, 3, 4, 5 or 6) $R_a$;
each $R_a$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
$R_1$' is H, D, halogen or $-NH_2$, alternatively H or D;
each R* is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$

cycloalkyl, 3- to 7-membered heterocyclyl, $C_{6-10}$ aryl or 5- to 10-membered heteroaryl, or two R* and the atoms to which they are attached are taken together to form $C_{3-8}$ cycloalkyl or 4- to 10-membered heterocyclyl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

17. The compound according to technical solution 16, or a tautomer, a stereoisomer, a prodrug, a crystal form, a pharmaceutically acceptable salt, a hydrate or a solvate thereof, wherein

$X_1$ is N or CH;
$X_2$ is $CH_2$, CHD, $CD_2$ or C(O);
$X_3$ is $CR_{X3}R_{X3'}$ or $NR_{X3}$;
$R_{X3}$ and $R_{X3}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3}$, and the carbon atom to which they are attached are taken together to form $C_{1-6}$ alkylidene; wherein the above groups are optionally substituted with 1 to 6 (e.g., 1, 2, 3, 4, 5 or 6) $R_a$;
each $R_a$ is independently H, D, halogen, $-NH_2$, $-NHC_{1-6}$ alkyl or $-N(C_{1-6}$ alkyl$)_2$; wherein the above groups are optionally substituted with one or more D, up to fully deuterated;
$R_1$' is H, D, halogen or $-NH_2$, alternatively H or D;
each R* is independently H, D, halogen, $C_{1-6}$ alkyl or $C_{1-6}$ haloalkyl, or two R* and the atoms to which they are attached are taken together to form 4- to 10-membered heterocyclyl; wherein the above groups are optionally substituted with 1 to 6 (e.g., 1, 2, 3, 4, 5 or 6) R';
each R' is independently H, D, halogen, -OH, $C_{1-6}$ alkyl or $C_{1-6}$ haloalkyl; wherein the above groups are optionally substituted with one or more D, up to fully deuterated.

18. The compound according to technical solution 16, or a tautomer, a stereoisomer, a prodrug, a crystal form, a pharmaceutically acceptable salt, a hydrate or a solvate thereof, wherein

$X_1$ is N or CH;
$X_2$ is $CH_2$ or $CD_2$;
$X_3$ is $CR_{X3}R_{X3'}$ or $NR_{X3}$;
$R_{X3}$ and $R_{X3'}$ are independently H, D, halogen, $C_{1-6}$ alkyl, $C_{1-6}$ haloalkyl or $C_{3-6}$ cycloalkyl; wherein the above groups are optionally substituted with one or more D, up to fully deuterated;
$R_1$' is H, D, halogen or $-NH_2$, alternatively H or D;
each R* is independently H, D, halogen, $C_{1-6}$ alkyl or $C_{1-6}$ haloalkyl, or two R* and the atoms to which they are attached are taken together to form 4- to 10-membered heterocyclyl; wherein the above groups are optionally substituted with 1 to 6 (e.g., 1, 2, 3, 4, 5 or 6) R';
each R' is independently H, D, halogen, -OH, $C_{1-6}$ alkyl or $C_{1-6}$ haloalkyl; wherein the above groups are optionally substituted with one or more D, up to fully deuterated.

19. The compound according to technical solution 16, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$;
$X_3$ is $CR_{X3}R_{X3'}$ or $NR_{X3}$;
$R_{X2}$ and $R_{X2'}$ are independently H, D, $C_{1-6}$ alkyl or $C_{1-6}$ haloalkyl; wherein the above groups are optionally substituted with one or more D, up to fully deuterated;
$R_{X3}$ and $R_{X3'}$ are independently H, D, halogen, $C_{1-6}$ alkyl or $C_{1-6}$ haloalkyl, or $R_{X3}$, $R_{X3'}$ and the carbon atom to which they are attached are taken together to form $C_{1-6}$ alkylidene; wherein the above groups are optionally substituted with 1 to 6 (e.g., 1, 2, 3, 4, 5 or 6) $R_a$;
each $R_a$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy or $C_{1-6}$ haloalkoxy; wherein the above groups are optionally substituted with one or more D, up to fully deuterated;
$R_1$' is H, D, halogen or $-NH_2$, alternatively H or D;

each R* is independently H, D, halogen, -CN, -OH, $C_{1-6}$ alkyl or $C_{1-6}$ haloalkyl, or two R* and the atom(s) to which they are attached are taken together to form $C_{3-8}$ cycloalkyl or 4- to 10-membered heterocyclyl; wherein the above groups are optionally substituted with 1 to 6 (e.g., 1, 2, 3, 4, 5 or 6) R';

each R' is independently H, D, halogen, -CN, -OH, $C_{1-6}$ alkyl or $C_{1-6}$ haloalkyl.

20. The compound according to technical solution 15, or a tautomer, a stereoisomer, a prodrug, a crystal form, a pharmaceutically acceptable salt, a hydrate or a solvate thereof, and the compound is a compound of formula (III-1):

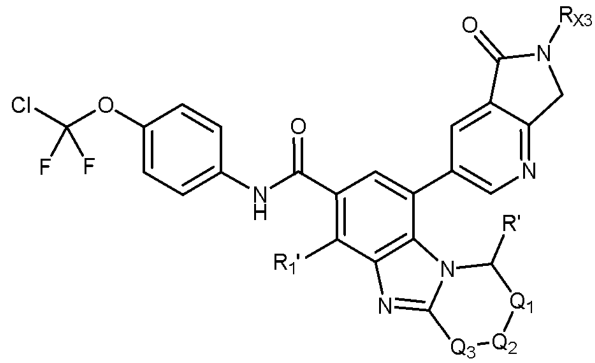

(III-1)

wherein

$R_{X3}$ is H, $C_{1-6}$ alkyl, $C_{1-6}$ haloalkyl or $C_{3-6}$ cycloalkyl; wherein the above groups are optionally substituted with one or more D, up to fully deuterated;

$R_1$' is H, D, halogen or $-NH_2$, alternatively H or D;

$Q_1$ is $C(R')_2$ or $C(R')_2C(R')_2$;

$Q_2$ is $C(R')_2$, O, NR', S, S(O) or $S(O)_2$;

$Q_3$ is $C(R')_2$, $C(R')_2C(R')_2$, O, NR', S, S(O) or $S(O)_2$;

each R' is independently H, D, halogen, -OH, $C_{1-6}$ alkyl or $C_{1-6}$ haloalkyl; wherein the above groups are optionally substituted with one or more D, up to fully deuterated.

21. The compound according to technical solution 15, or a tautomer, a stereoisomer, a prodrug, a crystal form, a pharmaceutically acceptable salt, a hydrate or a solvate thereof, and the compound is a compound of formula (III-2):

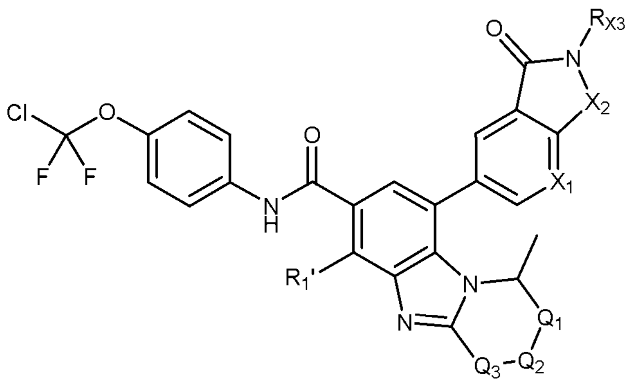

(III-2)

wherein

$X_1$ is N or $CR_{X1}$, alternatively N;

$X_2$ is $CR_{X2}R_{X2'}$ or C(O), alternatively $CR_{X2}R_{X2'}$;

$R_{X1}$ is H, D, halogen, $C_{1-6}$ alkyl or $C_{1-6}$ haloalkyl; wherein the above groups are optionally substituted with one or more D, up to fully deuterated;

$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;

$R_{X3}$ is H, $C_{1-6}$ alkyl, $C_{1-6}$ haloalkyl or $C_{3-6}$ cycloalkyl, alternatively $C_{1-6}$ alkyl or $C_{1-6}$ haloalkyl; wherein the above

groups are optionally substituted with one or more D, up to fully deuterated;

$R_1$' is H, D, halogen or $-NH_2$, alternatively H or D;

$Q_1$ is $C_{1-2}$ alkylene or $C_{1-2}$ haloalkylene; wherein the above groups are optionally substituted with one or more D, up to fully deuterated;

$Q_2$ and $Q_3$ are independently O, S, S(O), $S(O)_2$, NR', $C_{1-2}$ alkylene or $C_{1-2}$ haloalkylene, alternatively O, S, $C_{1-2}$ alkylene or $C_{1-2}$ haloalkylene; wherein the above groups are optionally substituted with 1 to 4 (e.g. 1, 2, 3 or 4) halogen or OH; wherein the above groups are also optionally substituted with one or more D, up to fully deuterated;

each R' is independently H, D, halogen, -OH, $C_{1-6}$ alkyl or $C_{1-6}$ haloalkyl.

22. The compound according to technical solution 21, or a tautomer, a stereoisomer, a prodrug, a crystal form, a pharmaceutically acceptable salt, a hydrate or a solvate thereof, wherein

$X_1$ is N;

$X_2$ is $CR_{X2}R_{X2'}$;

$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;

$R_{X3}$ is Me;

$R_1$' is H, D, halogen or $-NH_2$, alternatively H or D;

$Q_1$ is $CH_2$, CHD or $CD_2$;

$Q_2$ is O, S, $CH_2$, CHD or $CD_2$;

$Q_3$ is O, S, $CH_2$, CHD or $CD_2$.

23. The compound according to technical solution 15, or a tautomer, a stereoisomer, a prodrug, a crystal form, a pharmaceutically acceptable salt, a hydrate or a solvate thereof, and the compound is a compound of formula (III-2a):

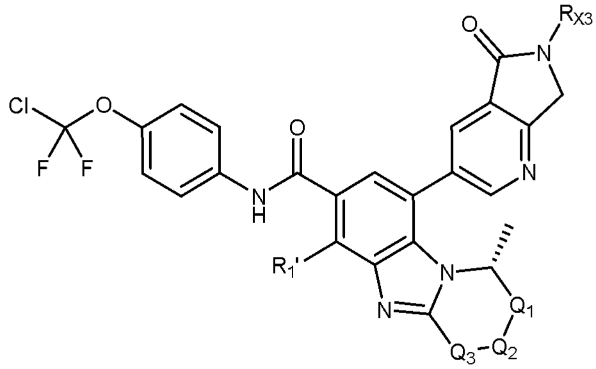

(III-2a)

wherein

$R_{X3}$ is independently H, $C_{1-6}$ alkyl, $C_{1-6}$ haloalkyl or $C_{3-6}$ cycloalkyl; wherein the above groups are optionally substituted with one or more D, up to fully deuterated;

$R_1$' is H, D, halogen or $-NH_2$, alternatively H or D;

$Q_1$ is $C(R')_2$ or $C(R')_2C(R')_2$;

$Q_2$ is $C(R')_2$, O, S, S(O) or $S(O)_2$;

$Q_3$ is $C(R')_2$, $C(R')_2C(R')_2$, O, S, S(O) or $S(O)_2$;

each R' is independently H, D, halogen, -OH, $C_{1-6}$ alkyl or $C_{1-6}$ haloalkyl; wherein the above groups are optionally substituted with one or more D, up to fully deuterated.

24. The compound according to technical solution 23, or a tautomer, a stereoisomer, a prodrug, a crystal form, a pharmaceutically acceptable salt, a hydrate or a solvate thereof, wherein

$R_{X3}$ is independently H, $C_{1-6}$ alkyl, $C_{1-6}$ haloalkyl or $C_{3-6}$ cycloalkyl; wherein the above groups are optionally substituted with one or more D, up to fully deuterated;

$R_1$' is H, D, halogen or $-NH_2$;

$Q_1$ is $CH_2$, $(CH_2)_2$, CHD, $(CHD)_2$, $CD_2$ or $(CD_2)_2$;

$Q_2$ is $CH_2$, CHD, $CD_2$, O, S, S(O) or $S(O)_2$;

$Q_3$ is $C(R')_2$, S or $S(O)_2$;
each R' is independently H, D, F or -OH.

25. The compound according to technical solution 15, or a tautomer, a stereoisomer, a prodrug, a crystal form, a pharmaceutically acceptable salt, a hydrate or a solvate thereof, and the compound is a compound of formula (III-3):

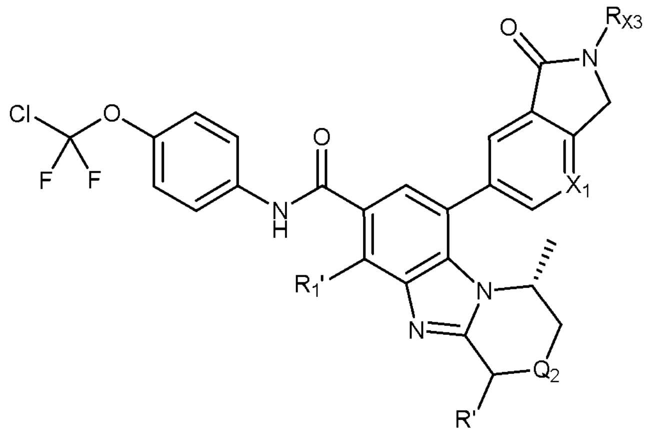

(III-3)

wherein

$X_1$ is N or $CR_{X1}$, alternatively N;
$R_{X1}$ is H, D, halogen, $C_{1-6}$ alkyl or $C_{1-6}$ haloalkyl; wherein the above groups are optionally substituted with one or more D, up to fully deuterated;
$R_{X3}$ is H, $C_{1-6}$ alkyl, $C_{1-6}$ haloalkyl, $C_{3-6}$ cycloalkyl or 3- to 7-membered heterocyclyl, alternatively $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_1$' is H, D, halogen or $-NH_2$, alternatively H or D;
$Q_2$ is O, S or NR', alternatively O or S;
R' is H, D, halogen, OH, $C_{1-6}$ alkyl or $C_{1-6}$ haloalkyl, alternatively H, D, $C_{1-6}$ alkyl or $C_{1-6}$ haloalkyl; wherein the above groups are optionally substituted with one or more D, up to fully deuterated.

26. The compound according to technical solution 25, or a tautomer, a stereoisomer, a prodrug, a crystal form, a pharmaceutically acceptable salt, a hydrate or a solvate thereof, wherein

$X_1$ is N;
$R_{X3}$ is Me, $CD_3$ or cyclopropyl;
$R_1$' is H, D, halogen or $-NH_2$, alternatively H or D;
$Q_2$ is O;
R' is H or D.

27. The compound according to technical solution 15, or a tautomer, a stereoisomer, a prodrug, a crystal form, a pharmaceutically acceptable salt, a hydrate or a solvate thereof, and the compound is a compound of formula (III-3a):

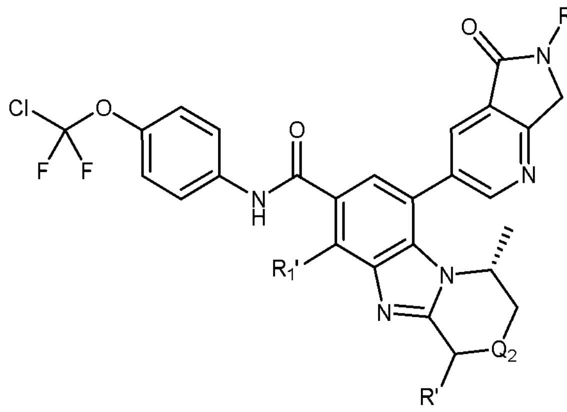

(III-3a)

wherein

$R_{X3}$ is $C_{1-6}$ alkyl, $C_{1-6}$ haloalkyl or $C_{3-6}$ cycloalkyl; wherein the above groups are optionally substituted with one or more D, up to fully deuterated;
$R_1$' is H, D, halogen or $-NH_2$, alternatively H or D;
$Q_2$ is $CH_2$, $CD_2$ or O;
R' is H, D or F.

28. The compound according to technical solution 15, or a tautomer, a stereoisomer, a prodrug, a crystal form, a pharmaceutically acceptable salt, a hydrate or a solvate thereof, and the compound is a compound of formula (IV):

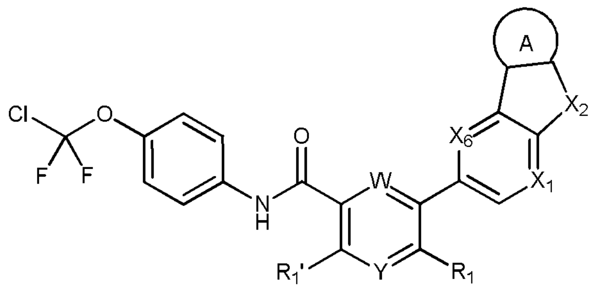

(IV)

wherein

$X_1$ is N or $CR_{X1}$;
$X_2$ is $CR_{X2}R_{X2'}$ or C(O), alternatively $CR_{X2}R_{X2'}$;
$X_6$ is N or $CR_{X6}$, alternatively $CR_{X6}$;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1 to 4 (e.g., 1, 2, 3 or 4) R;
Y is $CR_Y$ or N, alternatively CRy;
$R_1$ is 3- to 10-membered heterocyclyl, which is optionally substituted with 1 to 6 (e.g., 1, 2, 3, 4, 5 or 6) $R_b$;
Ry is H, D or halogen;
or, $R_1$, $R_Y$ and the carbon atoms to which they are attached are taken together to form $C_{6-10}$ aryl or 5-to 10-membered heteroaryl, which is optionally substituted with 1 to 6 (e.g., 1, 2, 3, 4, 5 or 6) R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
each $R_b$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to

fully deuterated;
each R is independently H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R* is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

29. The compound according to technical solution 28, or a tautomer, a stereoisomer, a prodrug, a crystal form, a pharmaceutically acceptable salt, a hydrate or a solvate thereof, wherein ring A is

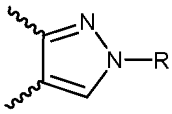,

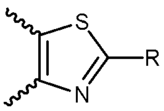, 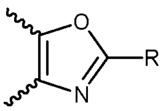, 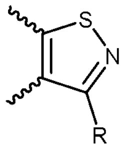, 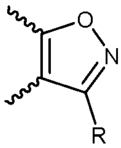, 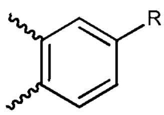, 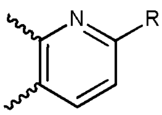,

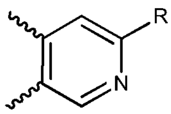

or

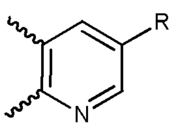.

30. The compound according to technical solution 28, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$;
$X_6$ is N or $CR_{X6}$, alternatively $CR_{X6}$;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
ring A is 5-membered heteroaryl, which is optionally substituted with 1 to 3 (e.g., 1, 2 or 3) R;

Y is CRy;

$R_1$, $R_Y$ and the carbon atoms to which they are attached are taken together to form $C_{6-10}$ aryl or 5- to 6-membered heteroaryl, which is optionally substituted with 1 to 6 (e.g., 1, 2, 3, 4, 5 or 6) R*;

$R_1$' is H, D, halogen or $-NH_2$, alternatively H or D;

W is $CR_W$ or N, alternatively $CR_W$;

$R_W$ is selected from H, D, halogen and $-NH_2$, alternatively H or D;

each R is independently H, D, halogen, -CN, -OH, $-C(O)R_A$, $-NR_BR_C$, $-NR_AC(O)R_B$, $C_{1-6}$ alkyl, $C_{1-6}$ haloalkyl, $C_{1-6}$ alkoxy, $C_{1-6}$ haloalkoxy, $C_{6-10}$ aryl or 5- to 10-membered heteroaryl; wherein the above groups are optionally substituted with 1 to 6 (e.g., 1, 2, 3, 4, 5 or 6) R';

wherein each of $R_A$, $R_B$ and $R_C$ is independently H, D, $C_{1-6}$ alkyl or $C_{1-6}$ haloalkyl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';

each R* is independently H, D, halogen, -CN, -OH, $C_{1-6}$ alkyl, $C_{1-6}$ haloalkyl, $C_{1-6}$ alkoxy or $C_{1-6}$ haloalkoxy, or two R* and the atom(s) to which they are attached are taken together to form $C_{3-8}$ cycloalkyl or 4- to 10-membered heterocyclyl; wherein the above groups are optionally substituted with 1 to 6 (e.g., 1, 2, 3, 4, 5 or 6) R';

each R' is independently H, D, halogen, -CN, -OH, $C_{1-6}$ alkyl or $C_{1-6}$ haloalkyl.

31. The compound according to technical solution 15, or a tautomer, a stereoisomer, a prodrug, a crystal form, a pharmaceutically acceptable salt, a hydrate or a solvate thereof, and the compound is a compound of formula (IV-1):

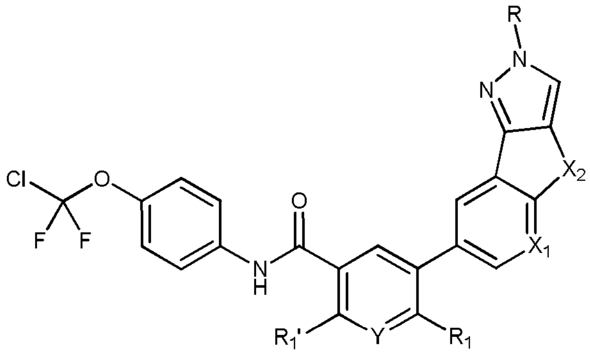

(IV-1)

wherein

$X_1$ is N or $CR_{X1}$;

$X_2$ is $CR_{X2}R_{X2'}$ or C(O);

$R_{X1}$ is H, D, halogen, $C_{1-6}$ alkyl or $C_{1-6}$ haloalkyl; wherein the above groups are optionally substituted with one or more D, up to fully deuterated;

$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;

Y is CRy or N;

$R_1$ is 3- to 10-membered heterocyclyl, which is optionally substituted with 1 to 6 (e.g., 1, 2, 3, 4, 5 or 6) $R_b$;

Ry is H, D or halogen;

or, $R_1$, $R_Y$ and the carbon atoms to which they are attached are taken together to form $C_{6-10}$ aryl or 5-to 10-membered heteroaryl, which is optionally substituted with 1 to 6 (e.g., 1, 2, 3, 4, 5 or 6) R*;

$R_1$' is H, D, halogen or $-NH_2$, alternatively H or D;

each $R_b$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;

R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';

wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';

each R* is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1 to 6 (e.g., 1, 2, 3, 4, 5 or 6) R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";

each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

32. The compound according to any one of technical solutions 28 to 31, or a tautomer, a stereoisomer, a prodrug, a crystal form, a pharmaceutically acceptable salt, a hydrate or a solvate thereof, wherein $R_1$ is 4- to 10-membered heterocyclyl, which is optionally substituted with 1 to 6 (e.g. 1, 2, 3, 4, 5 or 6) $R_b$;
alternatively, $R_1$ is

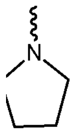 , 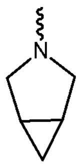 , 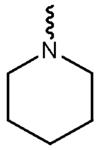 , 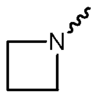 , 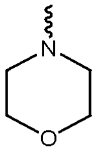 , 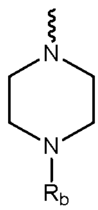 , 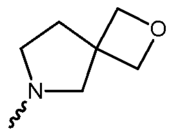 ,

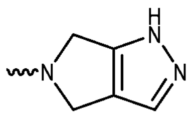 , 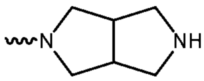 , 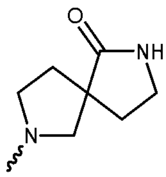

or

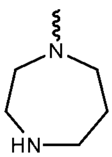 ,

wherein the above groups are optionally substituted with 1 to 6 (e.g., 1, 2, 3, 4, 5 or 6) $R_b$.

33. The compound according to any one of technical solutions 28 to 32, or a tautomer, a stereoisomer, a prodrug, a crystal form, a pharmaceutically acceptable salt, a hydrate or a solvate thereof, wherein $R_1$, $R_Y$ and the carbon atoms to which they are attached are taken together to form 5-membered heterocyclyl or 5-membered heteroaryl, which is optionally substituted with 1 to 6 (e.g. 1, 2, 3, 4, 5 or 6) R*;
alternatively, $R_1$, $R_Y$ and the carbon atoms to which they are attached are taken together to form

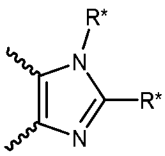 , 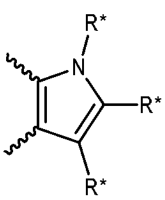 , 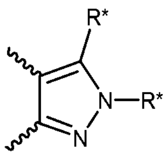

or

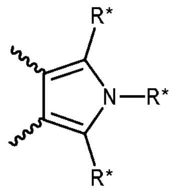

.

34. The compound according to technical solution 15, or a tautomer, a stereoisomer, a prodrug, a crystal form, a pharmaceutically acceptable salt, a hydrate or a solvate thereof, and the compound is a compound of formula (IV-2):

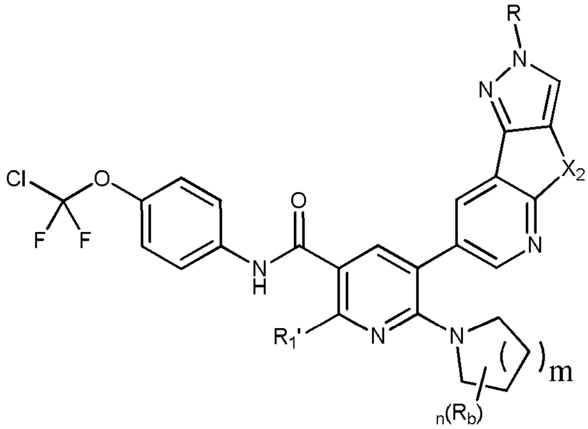

(IV-2)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
m is 0, 1, 2 or 3;
n is 0, 1, 2, 3, 4 or 5;
each $R_b$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

35. The compound according to technical solution 15, or a tautomer, a stereoisomer, a prodrug, a crystal form, a pharmaceutically acceptable salt, a hydrate or a solvate thereof, and the compound is a compound of formula (IV-2a):

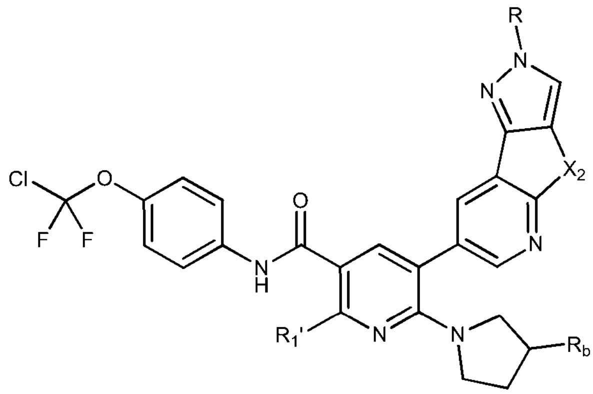

(IV-2a)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_b$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

36. The compound according to technical solution 35, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$R_b$ is H, D, halogen, -OH, $-NH_2$, $-NHC_{1-6}$ alkyl or $-N(C_{1-6}$ alkyl$)_2$; wherein the above groups are optionally substituted with one or more D, up to fully deuterated;
alternatively,
$R_b$ is halogen or -OH.

37. The compound according to technical solution 15, or a tautomer, a stereoisomer, a prodrug, a crystal form, a pharmaceutically acceptable salt, a hydrate or a solvate thereof, and the compound is a compound of formula (IV-2a):

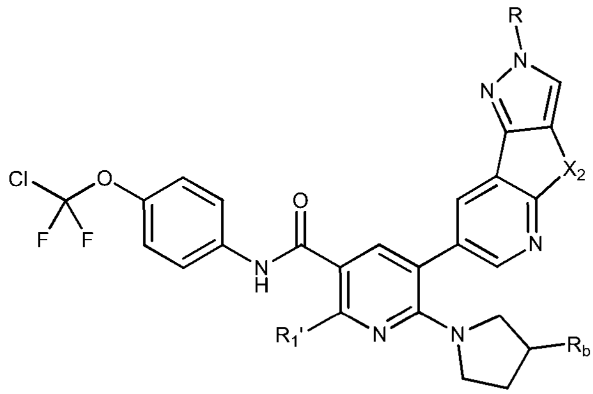

(IV-2a)

wherein

$R_1$' is H, D, halogen or $-NH_2$, alternatively H or D;
$X_2$ is $CR_{X2}R_{X2'}$ or C(O), alternatively $CR_{X2}R_{X2'}$;
$R_{X2}$ and $R_{X2'}$ are independently H, D, halogen, -OH, $C_{1-6}$ alkyl or $C_{1-6}$ haloalkyl, alternatively H, D, $C_{1-6}$ alkyl or $C_{1-6}$ haloalkyl; wherein the above groups are optionally substituted with 1 to 6 (e.g., 1, 2, 3, 4, 5 or 6) D, up to fully deuterated;
$R_b$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, alternatively H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy or $C_{1-6}$ haloalkoxy; wherein the above groups are optionally substituted with 1 to 6 (e.g., 1, 2, 3, 4, 5 or 6) D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, $C_{1-6}$ alkyl, $C_{1-6}$ haloalkyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

38. The compound according to technical solution 15, or a tautomer, a stereoisomer, a prodrug, a crystal form, a pharmaceutically acceptable salt, a hydrate or a solvate thereof, and the compound is a compound of formula (IV-2b):

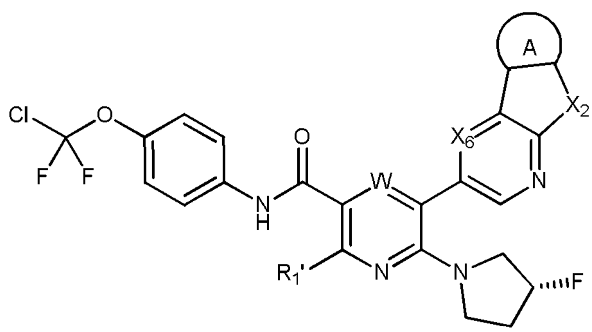

(IV-2b)

wherein

W is $CR_W$ or N;
$R_W$ is selected from H, D, halogen and $-NH_2$, alternatively H or D;
$R_1$' is H, D, halogen or $-NH_2$, alternatively H or D;
$X_2$ is $CR_{X2}R_{X2'}$ or C(O);
$X_6$ is N or $CR_{X6}$, alternatively $CR_{X6}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1 to 4 (e.g., 1, 2, 3 or 4) R;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

39. The compound according to technical solution 38, or a tautomer, a stereoisomer, a prodrug, a crystal form, a pharmaceutically acceptable salt, a hydrate or a solvate thereof, wherein ring A is

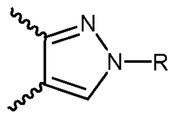
,

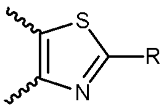
,
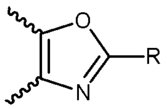
,
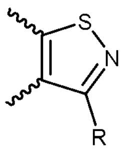
,
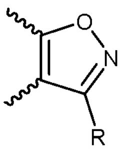
,
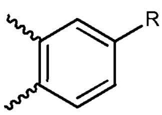
,
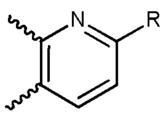
,

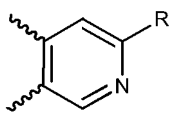

or

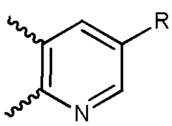
.

40. The compound according to technical solution 38, or a tautomer, a stereoisomer, a prodrug, a crystal form, a pharmaceutically acceptable salt, a hydrate or a solvate thereof, wherein one R is pyrimidin-2-yl, pyrimidin-4-yl, pyrazin-2-yl, triazinyl or tetrazinyl, which is optionally substituted with 1 to 3 (e.g. 1, 2 or 3) R'.
41. The compound according to technical solution 15, or a tautomer, a stereoisomer, a prodrug, a crystal form, a pharmaceutically acceptable salt, a hydrate or a solvate thereof, and the compound is a compound of formula (IV-2c):

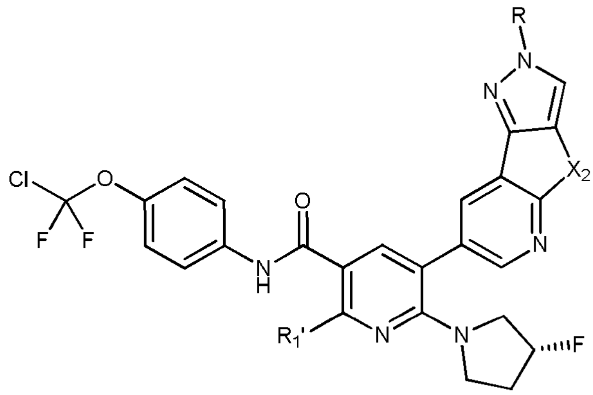

(IV-2c)

wherein

$R_1$' is H, D, halogen or -$NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
R is H, D, halogen, -CN, -OH, -C(O)$R_A$, -$NR_BR_C$, -$NR_A$C(O)$R_B$, -S(O)$_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

42. The compound according to technical solution 41, or a tautomer, a stereoisomer, a prodrug, a crystal form, a pharmaceutically acceptable salt, a hydrate or a solvate thereof, wherein

$R_1$' is H, D, halogen or -$NH_2$, alternatively H or D;
$X_2$ is $CR_{X2}R_{X2'}$;
$R_{X2}$ and $R_{X2'}$ are independently H, D, halogen or -OH;
R is H, D, -C(O)$R_A$, -S(O)$_2R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each $R_A$ is independently $C_{1-6}$ alkyl, $C_{1-6}$ haloalkyl, $C_{3-6}$ cycloalkyl or 3- to 7-membered heterocyclyl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, or $C_{1-6}$ alkoxy$C_{1-6}$ haloalkoxy; wherein the above groups are optionally substituted with one or more D, up to fully deuterated.

43. The compound according to technical solution 42, or a tautomer, a stereoisomer, a prodrug, a crystal form, a pharmaceutically acceptable salt, a hydrate or a solvate thereof, wherein

$R_1$' is H, D, halogen or -$NH_2$, alternatively H or D;
$X_2$ is $CR_{X2}R_{X2'}$;
$R_{X2}$ and $R_{X2'}$ are independently H, D, halogen or -OH;
R is H, D, -C(O)$R_A$, -S(O)$_2R_A$, $C_{1-6}$ alkyl, $C_{1-6}$ haloalkyl, $C_{3-6}$ cycloalkyl, 3- to 7-membered heterocyclyl or 5- to 10-membered heteroaryl; wherein the above groups are optionally substituted with 1 to 6 (e.g., 1, 2, 3, 4, 5 or 6) R'; wherein each $R_A$ is independently $C_{1-6}$ alkyl, $C_{1-6}$ haloalkyl or $C_{3-6}$ cycloalkyl; wherein the above groups are optionally substituted with 1 to 6 (e.g., 1, 2, 3, 4, 5 or 6) R';

each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1 to 6 (e.g., 1, 2, 3, 4, 5 or 6) R";

each R" is independently H, D, halogen, -CN, -OH, $C_{1-3}$ alkyl, $C_{1-3}$ haloalkyl, or $C_{1-3}$ alkoxy$C_{1-3}$ haloalkoxy; wherein the above groups are optionally substituted with one or more D, up to fully deuterated.

44. The compound according to technical solution 43, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;

$X_2$ is $CR_{X2}R_{X2'}$;

$R_{X2}$ and $R_{X2'}$ are independently H, D, halogen or -OH;

R is H, D, $C_{1-6}$ alkyl, $C_{1-6}$ haloalkyl, $C_{3-6}$ cycloalkyl, 3- to 7-membered heterocyclyl or 5- to 10-membered heteroaryl; wherein the above groups are optionally substituted with 1 to 6 (e.g., 1, 2, 3, 4, 5 or 6) R';

each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1 to 6 (e.g., 1, 2, 3, 4, 5 or 6) R";

each R" is independently H, D, halogen, -CN, -OH, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

45. The compound according to technical solution 15, or a tautomer, a stereoisomer, a prodrug, a crystal form, a pharmaceutically acceptable salt, a hydrate or a solvate thereof, and the compound is a compound of formula (IV-2d):

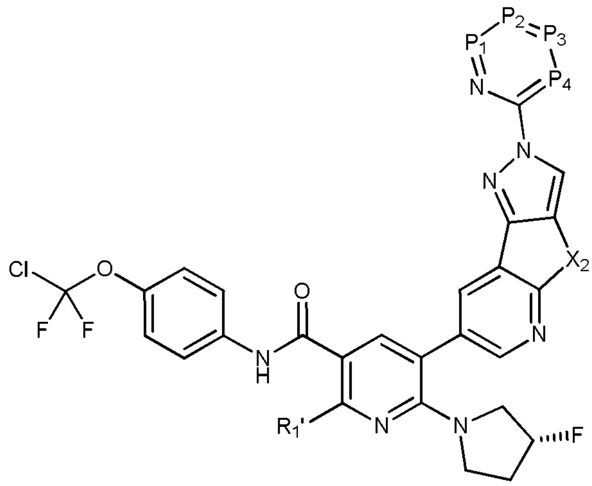

(IV-2d)

wherein

$R_1$' is H, D, halogen or $-NH_2$, alternatively H or D;

$X_2$ is $CR_{X2}R_{X2'}$ or C(O);

$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;

$P_1$ is N or $CR_{P1}$;

$P_2$ is N or $CR_{P2}$;

$P_3$ is N or $CR_{P3}$;

$P_4$ is N or $CR_{P4}$;

$R_{P1}$, $R_{P2}$, $R_{P3}$ and $R_{P4}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g. 1, 2, 3, 4, 5 or 6) R";

each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more

D, up to fully deuterated.

46. The compound according to technical solution 45, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

47. The compound according to technical solution 15, or a tautomer, a stereoisomer, a prodrug, a crystal form, a pharmaceutically acceptable salt, a hydrate or a solvate thereof, and the compound is a compound of formula (IV-2e):

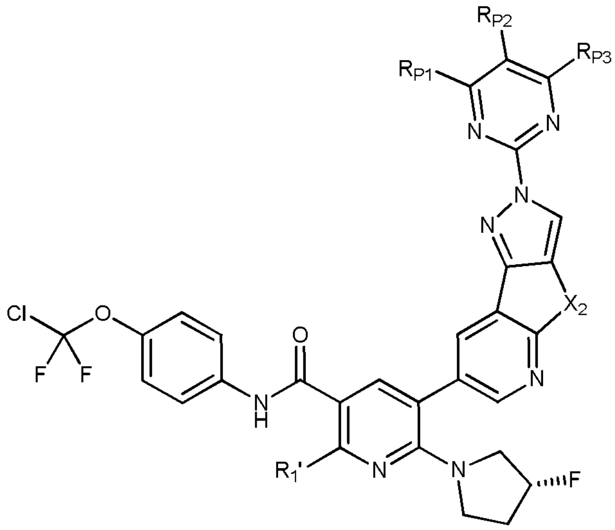
(IV-2e)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{P1}$, $R_{P2}$ and $R_{P3}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g. 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

48. The compound according to technical solution 47, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;

$R_{P1}$, $R_{P2}$ and $R_{P3}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

49. The compound according to technical solution 47, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ is H, D, halogen, -CN, $C_{1-3}$ alkyl, $C_{1-3}$ haloalkyl, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
$R_{P2}$ is H;
$R_{P3}$ is H, $C_{1-3}$ alkyl or $C_{1-3}$ haloalkyl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

50. The compound according to technical solution 47, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3-to 7-membered heterocyclyl, phenyl or 5- or 6-membered heteroaryl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

51. The compound according to technical solution 50, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, 3- to 7-membered heterocyclyl or phenyl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

52. The compound according to technical solution 51, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy or phenyl; wherein the above groups are optionally substituted with one or more D, up to fully deuterated.

53. The compound according to technical solution 15, or a tautomer, a stereoisomer, a prodrug, a crystal form, a pharmaceutically acceptable salt, a hydrate or a solvate thereof, and the compound is a compound of formula (V):

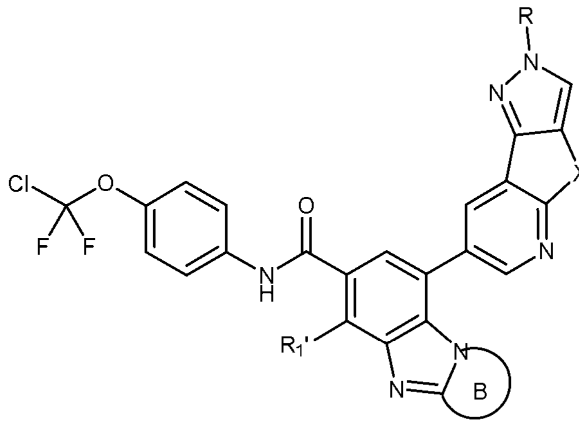

(V)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
ring B is 4- to 10-membered heterocyclyl, which is optionally substituted with 1 to 6 (e.g., 1, 2, 3, 4, 5 or 6) R';
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

54. The compound according to technical solution 15, or a tautomer, a stereoisomer, a prodrug, a crystal form, a pharmaceutically acceptable salt, a hydrate or a solvate thereof, and the compound is a compound of formula (V-1):

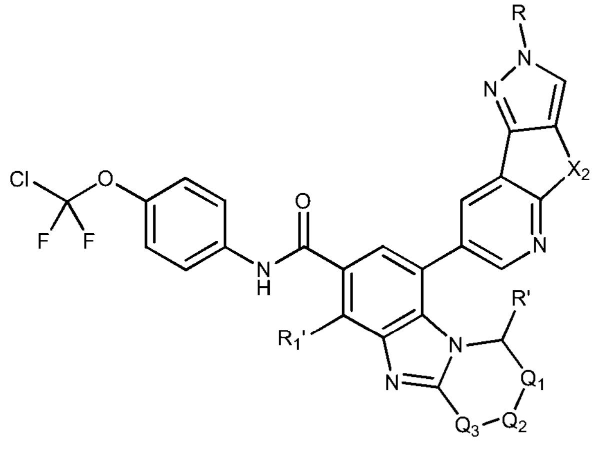

(V-1)

wherein

$R_1$' is H, D, halogen or $-NH_2$, alternatively H or D;
$X_2$ is $CR_{X2}R_{X2'}$ or C(O);

$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$Q_1$ is $C(R')_2$ or $C(R')_2C(R')_2$;
$Q_2$ is $C(R')_2$, O, NR', S, S(O) or $S(O)_2$;
$Q_3$ is $C(R')_2$, $C(R')_2C(R')_2$, O, NR', S, S(O) or $S(O)_2$;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

55. The compound according to technical solution 54, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$Q_1$ is $CH_2$, $(CH_2)_2$, CHD, $(CHD)_2$, $CD_2$ or $(CD_2)_2$;
$Q_2$ is $CH_2$, CHD, $CD_2$, O, S, S(O) or $S(O)_2$;
$Q_3$ is $CH_2$, CHD, $CD_2$, CHF, CDF, CH(OH), CD(OH), S or $S(O)_2$;
alternatively,
$R_b$ is halogen or -OH;
$Q_1$ is $CH_2$ or $CD_2$;
$Q_2$ is $CH_2$, CHD, $CD_2$, O or S;
$Q_3$ is $CH_2$, CHD, $CD_2$, CHF, CDF, CH(OH) or CD(OH).

56. The compound according to technical solution 15, or a tautomer, a stereoisomer, a prodrug, a crystal form, a pharmaceutically acceptable salt, a hydrate or a solvate thereof, and the compound is a compound of formula (V-2):

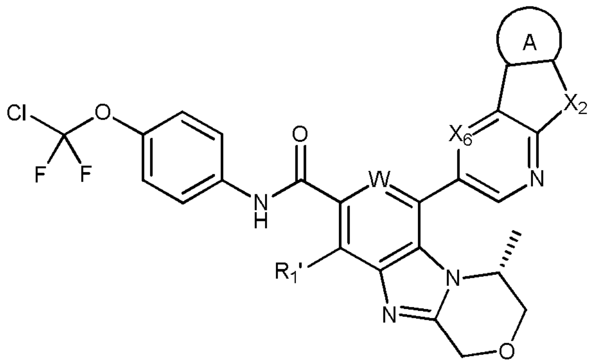

(V-2)

wherein

W is $CR_W$ or N;
$R_W$ is selected from H, D, halogen and $-NH_2$, alternatively H or D;
$R_1$' is H, D, halogen or $-NH_2$, alternatively H or D;
$X_2$ is $CR_{X2}R_{X2'}$ or C(O);
$X_6$ is N or $CR_{X6}$, alternatively $CR_{X6}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;

ring A is phenyl or 5- to 6-membered heteroaryl, which is optionally substituted with 1 to 6 (e.g., 1, 2, 3, 4, 5 or 6) R;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

57. The compound according to technical solution 56, or a tautomer, a stereoisomer, a prodrug, a crystal form, a pharmaceutically acceptable salt, a hydrate or a solvate thereof, wherein ring A is

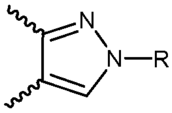
,

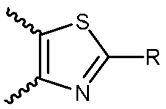
,
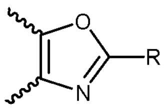
,
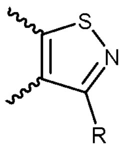
,
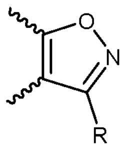
,
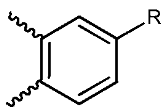
,
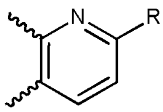
,

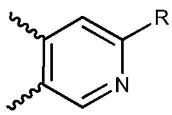

or

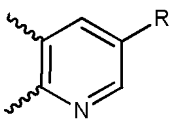
.

58. The compound according to technical solution 56, or a tautomer, a stereoisomer, a prodrug, a crystal form, a pharmaceutically acceptable salt, a hydrate or a solvate thereof, wherein one R is pyrimidin-2-yl, pyrimidin-4-yl, pyrazin-2-yl, triazinyl or tetrazinyl, which is optionally substituted with 1 to 3 (e.g. 1, 2 or 3) R'.
59. The compound according to technical solution 15, or a tautomer, a stereoisomer, a prodrug, a crystal form, a pharmaceutically acceptable salt, a hydrate or a solvate thereof, and the compound is a compound of formula (V-3):

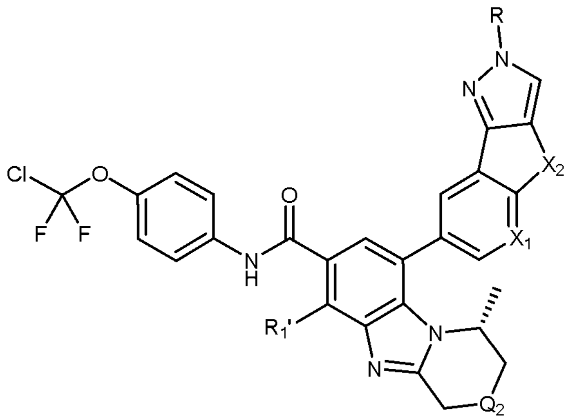

(V-3)

wherein

$X_1$ is N or $CR_{X1}$, alternatively N;
$R_{X1}$ is H, D, halogen, $C_{1-6}$ alkyl or $C_{1-6}$ haloalkyl; wherein the above groups are optionally substituted with one or more D, up to fully deuterated;
$X_2$ is $CR_{X2}R_{X2'}$ or C(O), alternatively $CR_{X2}R_{X2'}$;
$R_{X2}$ and $R_{X2'}$ are independently H, D, halogen, -OH, $C_{1-6}$ alkyl or $C_{1-6}$ haloalkyl, alternatively H, D, halogen, $C_{1-6}$ alkyl or $C_{1-6}$ haloalkyl; wherein the above groups are optionally substituted with one or more D, up to fully deuterated;
$R_1$' is H, D, halogen or $-NH_2$, alternatively H or D;
$Q_2$ is O, S or NR', alternatively O or S, still alternatively O;
R' is H, D, -CN, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-to 7-membered heterocyclyl, $C_{6-10}$ aryl or 5- to 10-membered heteroaryl, alternatively H, D, -CN or halogen; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
R is H, D, $-C(O)R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl, alternatively H, D, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R'; wherein the above groups are also optionally substituted with one or more D, up to fully deuterated;
$R_A$ is H, D, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated.

60. The compound according to technical solution 59, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$;
$R_{X2}$ and $R_{X2'}$ are independently H or D;
$R_1$' is H, D, halogen or $-NH_2$, alternatively H or D;
$Q_2$ is O;
R is H, D, Et, $CH_2CF_3$, isopropyl, $CD(CD_3)_2$, cyclopropyl, oxetanyl, pyrazin-2-yl, pyridin-2-yl or pyrimidin-5-yl.

61. The compound according to technical solution 15, or a tautomer, a stereoisomer, a prodrug, a crystal form, a pharmaceutically acceptable salt, a hydrate or a solvate thereof, and the compound is a compound of formula (V-4):

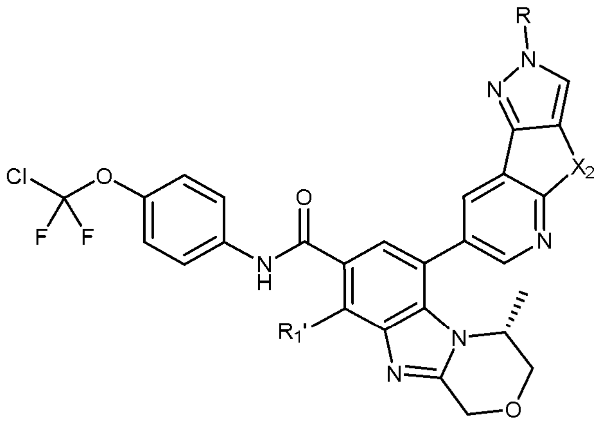

(V-4)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

62. The compound according to technical solution 61, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
R is H, D, $-C(O)R_A$, $-S(O)_2R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each $R_A$ is independently $C_{1-6}$ alkyl, $C_{1-6}$ haloalkyl, $C_{3-6}$ cycloalkyl or 3- to 7-membered heterocyclyl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy; wherein the above groups are optionally substituted with one or more D, up to fully deuterated.

63. The compound according to technical solution 62, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;

R is H, D, -C(O)$R_A$, -S(O)$_2R_A$, $C_{1-6}$ alkyl, $C_{1-6}$ haloalkyl, $C_{3-6}$ cycloalkyl, 3- to 7-membered heterocyclyl or 5- to 10-membered heteroaryl; wherein the above groups are optionally substituted with 1 to 6 (e.g., 1, 2, 3, 4, 5 or 6) R'; wherein each $R_A$ is independently $C_{1-6}$ alkyl, $C_{1-6}$ haloalkyl or $C_{3-6}$ cycloalkyl; wherein the above groups are optionally substituted with 1 to 6 (e.g., 1, 2, 3, 4, 5 or 6) R';
each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-3}$ alkyl, $C_{1-3}$ haloalkyl, $C_{1-3}$ alkoxy$C_{1-3}$ haloalkoxy; wherein the above groups are optionally substituted with one or more D, up to fully deuterated.

64. The compound according to technical solution 63, or a tautomer, a stereoisomer, a prodrug, a crystal form, a pharmaceutically acceptable salt, a hydrate or a solvate thereof, wherein

$R_1$' is H, D, halogen or -$NH_2$, alternatively H or D;
$X_2$ is $CR_{X2}R_{X2'}$;
$R_{X2}$ and $R_{X2'}$ are independently H, D, halogen or -OH;
R is H, D, $C_{1-6}$ alkyl, $C_{1-6}$ haloalkyl, $C_{3-6}$ cycloalkyl, 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

65. The compound according to technical solution 15, or a tautomer, a stereoisomer, a prodrug, a crystal form, a pharmaceutically acceptable salt, a hydrate or a solvate thereof, and the compound is a compound of formula (V-4):

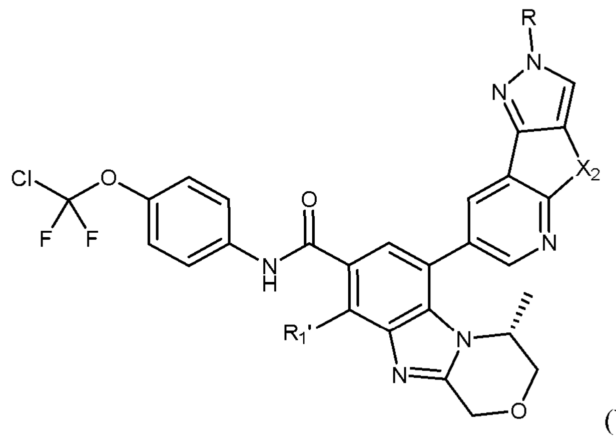

(V-4)

wherein

$R_1$' is H, D, halogen or -$NH_2$, alternatively H or D;
$X_2$ is $CR_{X2}R_{X2'}$ or C(O), alternatively $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
R is H, D, -C(O)$R_A$, $C_{1-6}$ alkyl or $C_{1-6}$ haloalkyl; wherein the above groups are optionally substituted with one or more D, up to fully deuterated;
$R_A$ is H, D, $C_{1-6}$ alkyl, $C_{1-6}$ haloalkyl, $C_{3-6}$ cycloalkyl or 3- to 7-membered heterocyclyl.

66. The compound according to technical solution 65, or a tautomer, a stereoisomer, a prodrug, a crystal form, a pharmaceutically acceptable salt, a hydrate or a solvate thereof, wherein

$R_1$' is H, D, halogen or -$NH_2$, alternatively H or D;
$X_2$ is $CR_{X2}R_{X2'}$;
$R_{X2}$ and $R_{X2'}$ are independently H or D;

R is H, D, Et, $CH_2CF_3$, isopropyl or $CD(CD_3)_2$.

67. The compound according to technical solution 15, or a tautomer, a stereoisomer, a prodrug, a crystal form, a pharmaceutically acceptable salt, a hydrate or a solvate thereof, and the compound is a compound of formula (V-5):

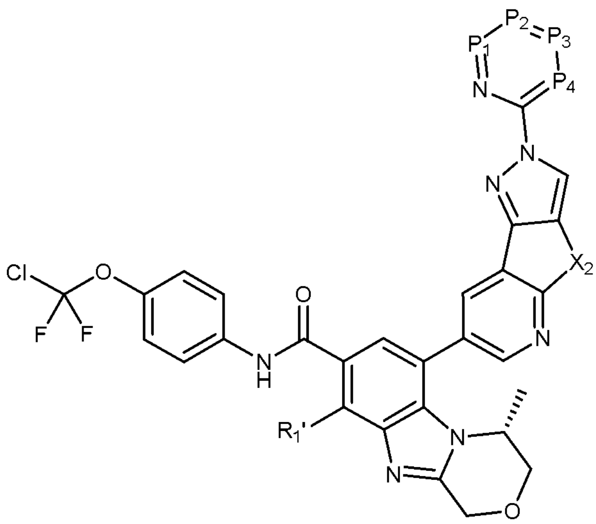

(V-5)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g. 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

68. The compound according to technical solution 67, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

69. The compound according to technical solution 15, or a tautomer, a stereoisomer, a prodrug, a crystal form, a pharmaceutically acceptable salt, a hydrate or a solvate thereof, and the compound is a compound of formula (V-5):

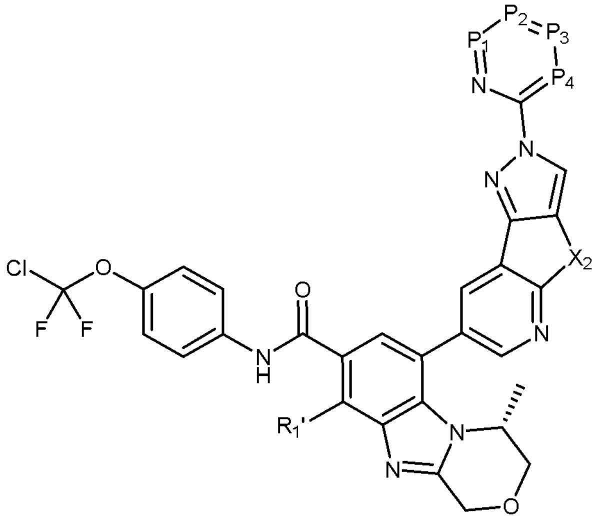

(V-5)

wherein

$R_1$' is H, D, halogen or $-NH_2$, alternatively H or D;
$X_2$ is $CR_{X2}R_{X2'}$;
$R_{X2}$ and $R_{X2'}$ are independently H, D, OH, halogen, $C_{1-6}$ alkyl or $C_{1-6}$ haloalkyl, alternatively H, D, halogen, $C_{1-6}$ alkyl or $C_{1-6}$ haloalkyl; wherein the above groups are optionally substituted with one or more D, up to fully deuterated;
$P_1$ is $CR_{P1}$;
$P_2$ is N or $CR_{P2}$;
$P_3$ is N or $CR_{P3}$;
$P_4$ is N or $CR_{P4}$;
and alternatively, at least one of $P_3$ and $P_4$ is N;
$R_{P1}$, $R_{P2}$, $R_{P3}$ and $R_{P4}$ are independently H, D, halogen, -CN, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, halogen, -CN, $C_{1-6}$ alkyl or $C_{1-6}$ haloalkyl; wherein the above groups are optionally substituted with 1 to 6 (e.g. 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
or $R_{P1}$ and $R_{P2}$, $R_{P2}$ and $R_{P3}$, or $R_{P3}$ and $R_{P4}$, and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

70. The compound according to technical solution 69, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
$P_1$ is $CR_{P1}$;
$P_2$ is $CR_{P2}$;
$P_3$ is N or $CR_{P3}$;
$P_4$ is N or $CR_{P4}$;
and at least one of $P_3$ and $P_4$ is N;
$R_{P1}$ is H, D, halogen, -CN, $C_{1-6}$ alkyl or $C_{1-6}$ haloalkyl; wherein the above groups are optionally substituted with one or more D, up to fully deuterated;
$R_{P2}$ is H, D, halogen or -CN;
$R_{P3}$ is H, D, $C_{1-6}$ alkyl or $C_{1-6}$ haloalkyl; wherein the above groups are optionally substituted with one or more D, up to fully deuterated;
$R_{P4}$ is H or D.

71. The compound according to technical solution 69, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H or D;
$P_1$ is $CR_{P1}$;
$P_2$ is $CR_{P2}$;
$P_3$ is N or $CR_{P3}$;
$P_4$ is N or $CR_{P4}$;
and at least one of $P_3$ and $P_4$ is N;
$R_{P1}$ is H, D, -CN or Me;
$R_{P2}$ is H, D, F or -CN;
$R_{P3}$ is H, D or Me;
$R_{P4}$ is H or D.

72. The compound according to technical solution 15, or a tautomer, a stereoisomer, a prodrug, a crystal form, a pharmaceutically acceptable salt, a hydrate or a solvate thereof, and the compound is a compound of formula (V-5):

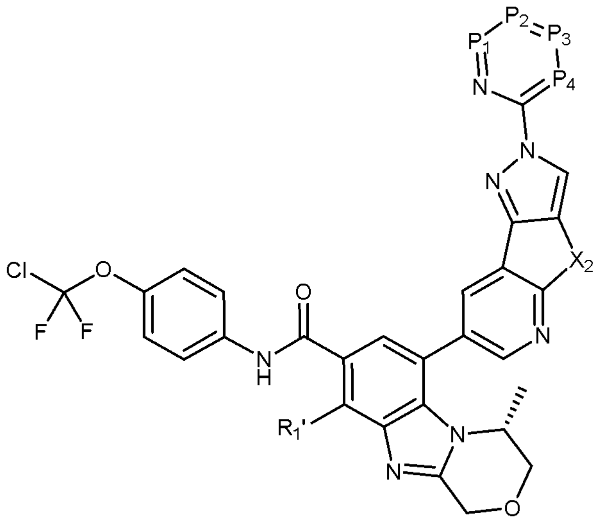
(V-5)

wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
$P_1$ is $CR_{P1}$;
$P_2$ is N or $CR_{P2}$;
$P_3$ is N or $CR_{P3}$;
$P_4$ is $CR_{P4}$;
$R_{P1}$, $R_{P2}$, $R_{P3}$ and $R_{P4}$ are independently H, D, halogen, -CN, $C_{1-6}$ alkyl or $C_{1-6}$ haloalkyl; wherein the above groups are optionally substituted with one or more D, up to fully deuterated.

73. The compound according to technical solution 72, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H or D;
$P_1$ is $CR_{P1}$;
$P_2$ is N or $CR_{P2}$;

$P_3$ is N or $CR_{P3}$;
$P_4$ is $CR_{P4}$;
$R_{P1}$ is H or D;
$R_{P2}$ is H or D;
$R_{P3}$ is H or D;
$R_{P4}$ is H or D.

74. The compound according to technical solution 15, or a tautomer, a stereoisomer, a prodrug, a crystal form, a pharmaceutically acceptable salt, a hydrate or a solvate thereof, and the compound is a compound of formula (V-6):

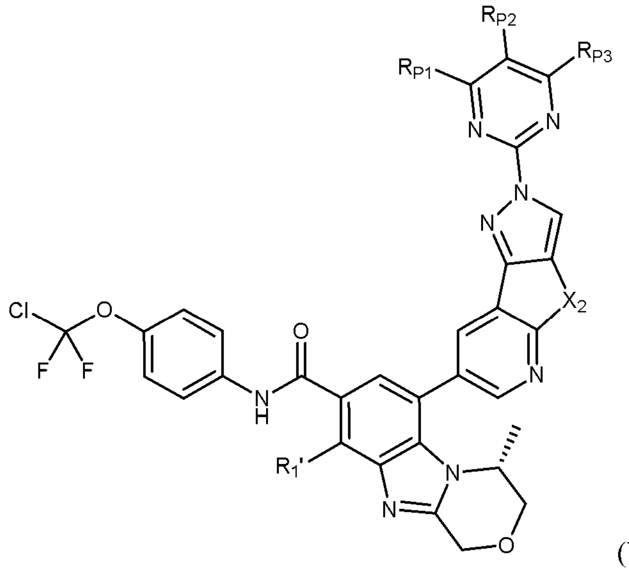

(V-6)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{P1}$, $R_{P2}$ and $R_{P3}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g. 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

75. The compound according to technical solution 74, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$, $R_{P2}$ and $R_{P3}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

76. The compound according to technical solution 74, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;

$X_2$ is $CR_{X2}R_{X2'}$;
$R_{X2}$ and $R_{X2'}$ are independently H, D, halogen or -OH;
$R_{P1}$ is H, D, halogen, -CN, $C_{1-3}$ alkyl, $C_{1-3}$ haloalkyl, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
$R_{P2}$ is H;
$R_{P3}$ is H, $C_{1-3}$ alkyl or $C_{1-3}$ haloalkyl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

77. The compound according to technical solution 74, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3-to 7-membered heterocyclyl, phenyl or 5- or 6-membered heteroaryl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

78. The compound according to technical solution 77, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, 3- to 7-membered heterocyclyl or phenyl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

79. The compound according to technical solution 78, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy or phenyl; wherein the above groups are optionally substituted with one or more D, up to fully deuterated.

80. The compound according to technical solution 15, or a tautomer, a stereoisomer, a prodrug, a crystal form, a pharmaceutically acceptable salt, a hydrate or a solvate thereof, and the compound is a compound of formula (V-6):

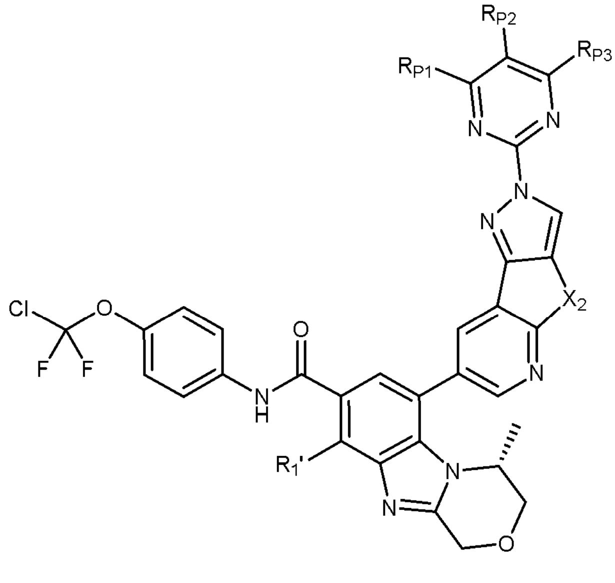

(V-6)

wherein

$R_1$' is H, D, halogen or -$NH_2$, alternatively H or D;
$X_2$ is $CR_{X2}R_{X2'}$;
$R_{X2}$ and $R_{X2'}$ are independently H, D, halogen, OH, $C_{1-6}$ alkyl or $C_{1-6}$ haloalkyl, alternatively H, D, halogen, $C_{1-6}$ alkyl or $C_{1-6}$ haloalkyl; wherein the above groups are optionally substituted with one or more D, up to fully deuterated;
$R_{P1}$, $R_{P2}$ and $R_{P3}$ are independently H, D, halogen, -CN, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, halogen, -CN, $C_{1-6}$ alkyl or $C_{1-6}$ haloalkyl; wherein the above groups are optionally substituted with 1 to 6 (e.g. 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
or $R_{P1}$ and $R_{P2}$, or $R_{P2}$ and $R_{P3}$ and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

81. The compound according to technical solution 80, or a tautomer, a stereoisomer, a prodrug, a crystal form, a pharmaceutically acceptable salt, a hydrate or a solvate thereof, wherein

$R_1$' is H, D, halogen or -$NH_2$, alternatively H or D;
$X_2$ is $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
$R_{P1}$ is H, D, halogen, -CN, $C_{1-6}$ alkyl or $C_{1-6}$ haloalkyl; wherein the above groups are optionally substituted with one or more D, up to fully deuterated;
$R_{P2}$ is H, D, halogen or -CN;
$R_{P3}$ is H, D, $C_{1-6}$ alkyl or $C_{1-6}$ haloalkyl; wherein the above groups are optionally substituted with one or more D, up to fully deuterated.

82. The compound according to technical solution 80, or a tautomer, a stereoisomer, a prodrug, a crystal form, a pharmaceutically acceptable salt, a hydrate or a solvate thereof, wherein

$R_1$' is H, D, halogen or -$NH_2$, alternatively H or D;
$X_2$ is $CR_{X2}R_{X2'}$;
$R_{X2}$ and $R_{X2'}$ are independently H or D;
$R_{P1}$ is H, D, -CN or Me;
$R_{P2}$ is H, D, F or -CN;
$R_{P3}$ is H, D or Me.

83. The compound according to technical solution 15, or a tautomer, a stereoisomer, a prodrug, a crystal form, a pharmaceutically acceptable salt, a hydrate or a solvate thereof, and the compound is a compound of formula (VI):

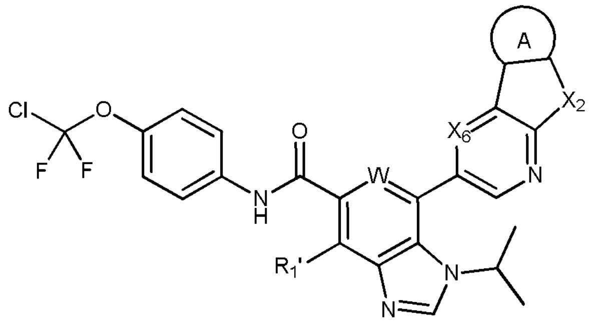

(VI)

wherein

W is $CR_W$ or N;
$R_W$ is selected from H, D, halogen and $-NH_2$, alternatively H or D;
$R_1$' is H, D, halogen or $-NH_2$, alternatively H or D;
$X_2$ is $CR_{X2}R_{X2'}$ or C(O);
$X_6$ is N or $CR_{X6}$, alternatively $CR_{X6}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1 to 4 (e.g., 1, 2, 3 or 4) R;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

84. The compound according to technical solution 83, or a tautomer, a stereoisomer, a prodrug, a crystal form, a pharmaceutically acceptable salt, a hydrate or a solvate thereof, wherein ring A is

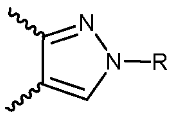,

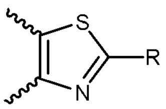, 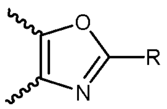, 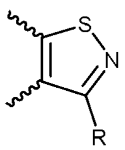, 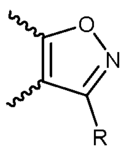, 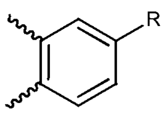, 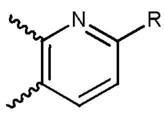,

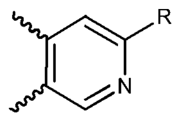

or

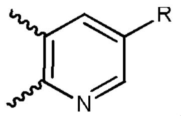

.

85. The compound according to technical solution 83, or a tautomer, a stereoisomer, a prodrug, a crystal form, a pharmaceutically acceptable salt, a hydrate or a solvate thereof, wherein one R is pyrimidin-2-yl, pyrimidin-4-yl, pyrazin-2-yl, triazinyl or tetrazinyl, which is optionally substituted with 1 to 3 (e.g. 1, 2 or 3) R'.

86. The compound according to technical solution 15, or a tautomer, a stereoisomer, a prodrug, a crystal form, a pharmaceutically acceptable salt, a hydrate or a solvate thereof, and the compound is a compound of formula (VI-1):

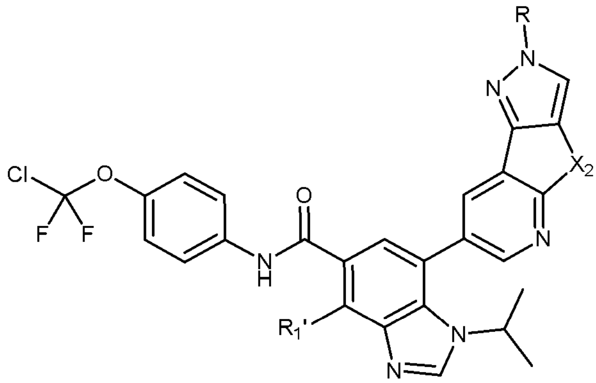

(VI-1)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

87. The compound according to technical solution 86, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
R is H, D, $-C(O)R_A$, $-S(O)_2R_A$, $C_{1-6}$ alkyl, $C_{1-6}$ haloalkyl, $C_{3-6}$ cycloalkyl, 3- to 7-membered heterocyclyl, $C_{6-10}$ aryl

or 5- to 10-membered heteroaryl; wherein the above groups are optionally substituted with 1 to 6 (e.g., 1, 2, 3, 4, 5 or 6) R';

wherein each $R_A$ is independently $C_{1-6}$ alkyl, $C_{1-6}$ haloalkyl, $C_{3-6}$ cycloalkyl or 3- to 7-membered heterocyclyl; wherein the above groups are optionally substituted with 1 to 6 (e.g., 1, 2, 3, 4, 5 or 6) R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";

each R" is independently H, D, halogen, -CN, -OH, $C_{1-6}$ alkyl, $C_{1-6}$ haloalkyl, $C_{1-6}$ alkoxy$C_{1-6}$ haloalkoxy; wherein the above groups are optionally substituted with one or more D, up to fully deuterated.

88. The compound according to technical solution 87, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;

$X_2$ is $CR_{X2}R_{X2'}$;

$R_{X2}$ and $R_{X2'}$ are independently H, D, halogen or -OH;

R is H, D, $-C(O)R_A$, $-S(O)_2R_A$, $C_{1-6}$ alkyl, $C_{1-6}$ haloalkyl, $C_{3-6}$ cycloalkyl, 3- to 7-membered heterocyclyl or 5- to 10-membered heteroaryl; wherein the above groups are optionally substituted with 1 to 6 (e.g., 1, 2, 3, 4, 5 or 6) R';

wherein each $R_A$ is independently $C_{1-6}$ alkyl, $C_{1-6}$ haloalkyl or $C_{3-6}$ cycloalkyl; wherein the above groups are optionally substituted with 1 to 6 (e.g., 1, 2, 3, 4, 5 or 6) R';

each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1 to 6 (e.g., 1, 2, 3, 4, 5 or 6) R";

each R" is independently H, D, halogen, -CN, -OH, $C_{1-3}$ alkyl, $C_{1-3}$ haloalkyl, $C_{1-3}$ alkoxy$C_{1-3}$ haloalkoxy; wherein the above groups are optionally substituted with one or more D, up to fully deuterated.

89. The compound according to technical solution 88, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;

$X_2$ is $CR_{X2}R_{X2'}$;

$R_{X2}$ and $R_{X2'}$ are independently H, D, halogen or -OH;

R is H, D, $C_{1-6}$ alkyl, $C_{1-6}$ haloalkyl, $C_{3-6}$ cycloalkyl, 3- to 7-membered heterocyclyl or 5- to 10-membered heteroaryl; wherein the above groups are optionally substituted with 1 to 6 (e.g., 1, 2, 3, 4, 5 or 6) R';

each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1 to 6 (e.g., 1, 2, 3, 4, 5 or 6) R";

each R" is independently H, D, halogen, -CN, -OH, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

90. The compound according to technical solution 15, or a tautomer, a stereoisomer, a prodrug, a crystal form, a pharmaceutically acceptable salt, a hydrate or a solvate thereof, and the compound is a compound of formula (VI-2):

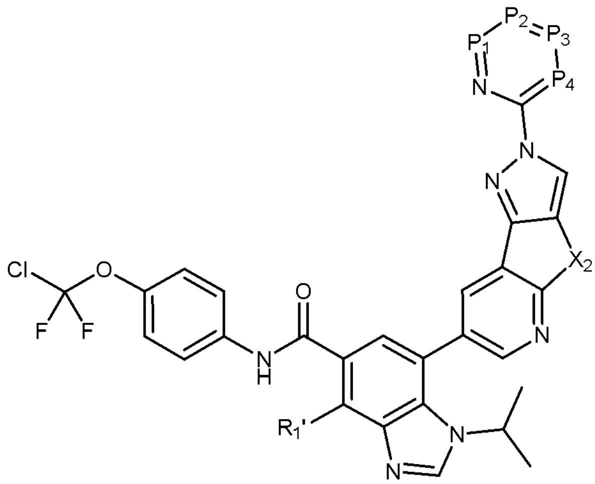

(VI-2)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g. 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

91. The compound according to technical solution 90, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

92. The compound according to technical solution 15, or a tautomer, a stereoisomer, a prodrug, a crystal form, a pharmaceutically acceptable salt, a hydrate or a solvate thereof, and the compound is a compound of formula (VI-3):

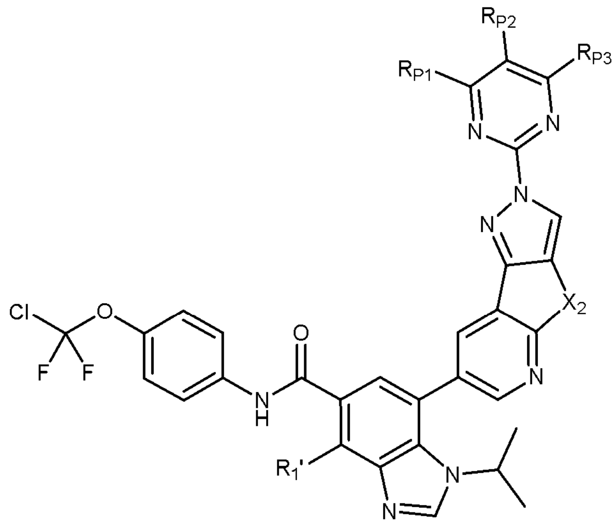

(VI-3)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{P1}$, $R_{P2}$ and $R_{P3}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g. 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

93. The compound according to technical solution 92, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$, $R_{P2}$ and $R_{P3}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

94. The compound according to technical solution 92, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ is H, D, halogen, -CN, $C_{1-3}$ alkyl, $C_{1-3}$ haloalkyl, $C_{3-6}$ cycloalkyl, 3- to 7-membered heterocyclyl, phenyl or 5- or 6-membered heteroaryl; wherein the above groups are optionally substituted with 1 to 6 (e.g. 1, 2, 3, 4, 5 or 6) R";
$R_{P2}$ is H;
$R_{P3}$ is H, $C_{1-3}$ alkyl or $C_{1-3}$ haloalkyl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

95. The compound according to technical solution 92, or a tautomer, a stereoisomer, a prodrug, a crystal form, a

pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3-to 7-membered heterocyclyl, phenyl or 5- or 6-membered heteroaryl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

96. The compound according to technical solution 95, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, 3- to 7-membered heterocyclyl or phenyl; wherein the above groups are optionally substituted with 1 to 6 (e.g. 1, 2, 3, 4, 5 or 6) R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

97. The compound according to technical solution 96, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy or phenyl; wherein the above groups are optionally substituted with one or more D, up to fully deuterated.

98. The compound according to technical solution 15, or a tautomer, a stereoisomer, a prodrug, a crystal form, a pharmaceutically acceptable salt, a hydrate or a solvate thereof, and the compound is a compound of formula (VII):

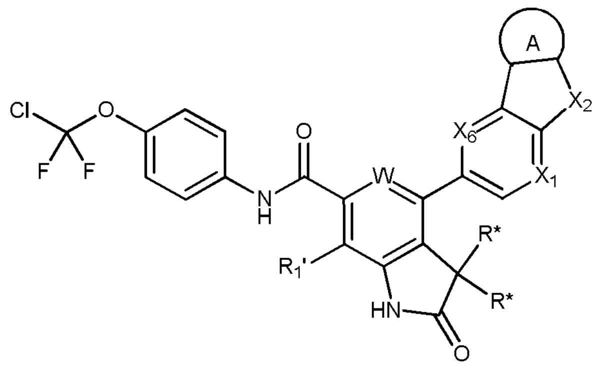
(VII)

wherein

W is $CR_W$ or N;
$R_W$ is selected from H, D, halogen and $-NH_2$, alternatively H or D;
$R_1$' is H, D, halogen or $-NH_2$, alternatively H or D;
$X_1$ is N or $CR_{X1}$, alternatively N;
$X_6$ is N or $CR_{X6}$, alternatively $CR_{X6}$;
$R_{X1}$ is H, D, halogen, $C_{1-6}$ alkyl or $C_{1-6}$ haloalkyl; wherein the above groups are optionally substituted with one or

more D, up to fully deuterated;
$X_2$ is $CR_{X2}R_{X2'}$ or C(O), alternatively $CR_{X2}R_{X2'}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1 to 4 (e.g., 1, 2, 3 or 4) R;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4-to 10-membered heterocyclyl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

99. The compound according to technical solution 98, or a tautomer, a stereoisomer, a prodrug, a crystal form, a pharmaceutically acceptable salt, a hydrate or a solvate thereof, wherein ring A is

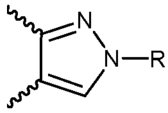
,

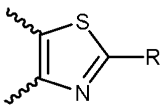 , 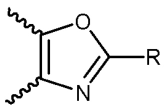 , 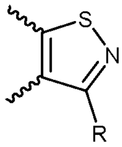 , 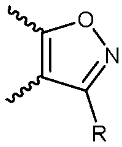 , 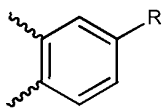 , 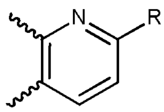 ,

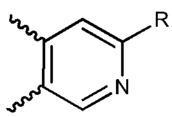

or

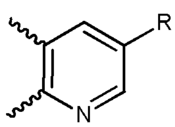
.

100. The compound according to technical solution 98, or a tautomer, a stereoisomer, a prodrug, a crystal form, a pharmaceutically acceptable salt, a hydrate or a solvate thereof, wherein one R is pyrimidin-2-yl, pyrimidin-4-yl, pyrazin-2-yl, triazinyl or tetrazinyl, which is optionally substituted with 1 to 3 (e.g. 1, 2 or 3) R'.
101. The compound according to technical solution 15, or a tautomer, a stereoisomer, a prodrug, a crystal form, a pharmaceutically acceptable salt, a hydrate or a solvate thereof, and the compound is a compound of formula (VII-1):

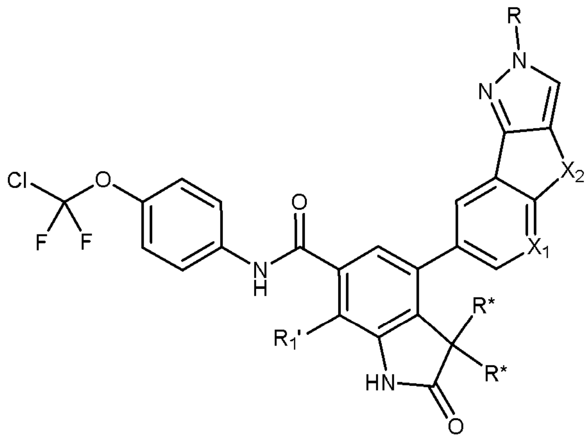

(VII-1)

wherein

$R_1$' is H, D, halogen or $-NH_2$, alternatively H or D;
$X_1$ is N or $CR_{X1}$, alternatively N;
$R_{X1}$ is H, D, halogen, $C_{1-6}$ alkyl or $C_{1-6}$ haloalkyl; wherein the above groups are optionally substituted with one or more D, up to fully deuterated;
$X_2$ is $CR_{X2}R_{X2'}$ or C(O), alternatively $CR_{X2}R_{X2'}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, $C_{1-6}$ alkyl, $C_{1-6}$ haloalkyl, $C_{6-10}$ aryl or 5- to 10-membered heteroaryl; wherein the above groups are optionally substituted with 1 to 6 (e.g., 1, 2, 3, 4, 5 or 6) R';
each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom to which they are attached are taken together to form $C_{3-8}$ cycloalkyl or 4- to 10-membered heterocyclyl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl;
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R'.

102. The compound according to technical solution 15, or a tautomer, a stereoisomer, a prodrug, a crystal form, a pharmaceutically acceptable salt, a hydrate or a solvate thereof, and the compound is a compound of formula (VII-2):

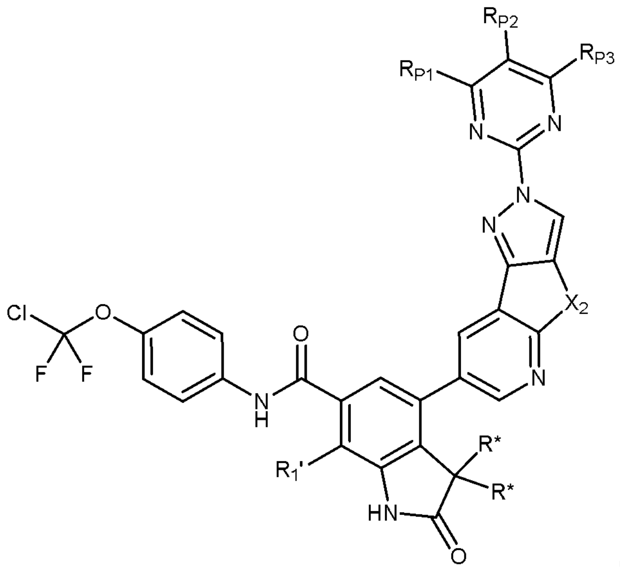

(VII-2)

wherein

$R_1$' is H, D, halogen or $-NH_2$, alternatively H or D;
$X_2$ is $CR_{X2}R_{X2'}$ or C(O), alternatively $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
each R* is independently H, D, halogen, $C_{1-6}$ alkyl or $C_{1-6}$ haloalkyl, or two R* and the atom to which they are attached are taken together to form $C_{3-8}$ cycloalkyl or 4- to 10-membered heterocyclyl; wherein the above groups are optionally substituted with 1 to 6 (e.g., 1, 2, 3, 4, 5 or 6) R';
each R' is independently H, D, halogen, -CN, -OH, $C_{1-6}$ alkyl or $C_{1-6}$ haloalkyl;
$R_{P1}$, $R_{P2}$ and $R_{P3}$ are independently H, D, halogen, -CN, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, halogen, -CN, $C_{1-6}$ alkyl or $C_{1-6}$ haloalkyl; wherein the above groups are optionally substituted with 1 to 6 (e.g. 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
or $R_{P1}$ and $R_{P2}$, or $R_{P2}$ and $R_{P3}$ and the atoms to which they are attached are taken together to form $C_{6-10}$ aryl or 5- to 10-membered heteroaryl; wherein the above groups are optionally substituted with 1 to 6 (e.g., 1, 2, 3, 4, 5 or 6) -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

103. The compound according to technical solution 15, or a tautomer, a stereoisomer, a prodrug, a crystal form, a pharmaceutically acceptable salt, a hydrate or a solvate thereof, and the compound is a compound of formula (VIII):

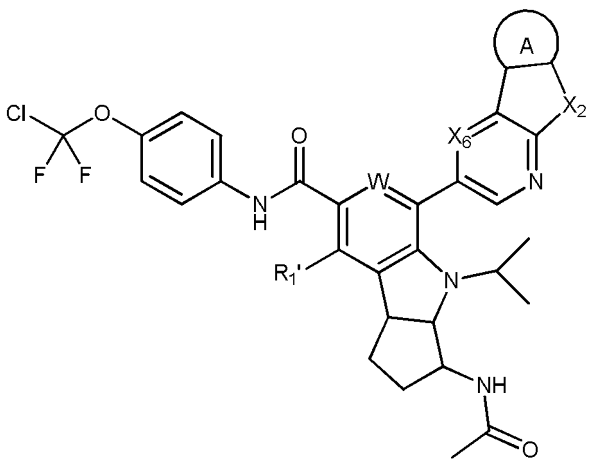

(VIII)

wherein

W is $CR_W$ or N;
$R_W$ is selected from H, D, halogen and $-NH_2$, alternatively H or D;
$R_1$' is H, D, halogen or $-NH_2$, alternatively H or D;
$X_2$ is $CR_{X2}R_{X2'}$ or C(O);
$X_6$ is N or $CR_{X6}$, alternatively $CR_{X6}$;
$R_{X2}$ and $R_{x2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1 to 4 (e.g., 1, 2, 3 or 4) R;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1 to 6 (e.g., 1, 2, 3, 4, 5 or 6) R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1 to 6 (e.g., 1, 2, 3, 4, 5 or 6) R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

104. The compound according to technical solution 103, or a tautomer, a stereoisomer, a prodrug, a crystal form, a pharmaceutically acceptable salt, a hydrate or a solvate thereof, wherein ring A is

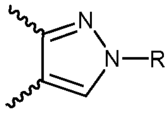
,

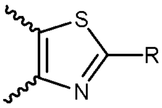 , 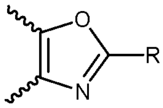 , 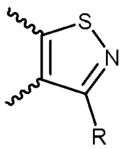 , 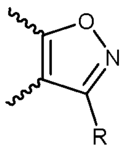 , 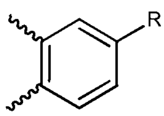 , 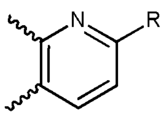 ,

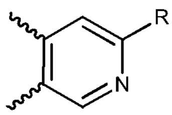

or

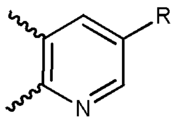
.

105. The compound according to technical solution 103, or a tautomer, a stereoisomer, a prodrug, a crystal form, a pharmaceutically acceptable salt, a hydrate or a solvate thereof, wherein one R is pyrimidin-2-yl, pyrimidin-4-yl, pyrazin-2-yl, triazinyl or tetrazinyl, which is optionally substituted with 1 to 3 (e.g., 1, 2 or 3) R'.

106. A compound, or a tautomer, a stereoisomer, a prodrug, a crystal form, a pharmaceutically acceptable salt, a hydrate or a solvate thereof, wherein the compound is selected from:

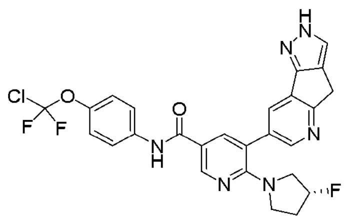,
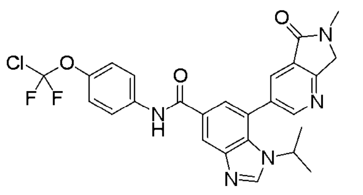,
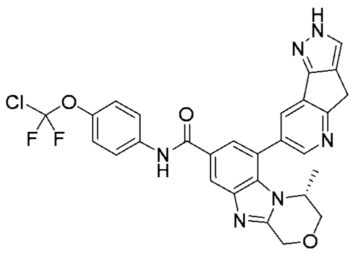,

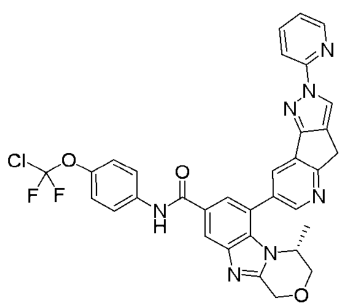,
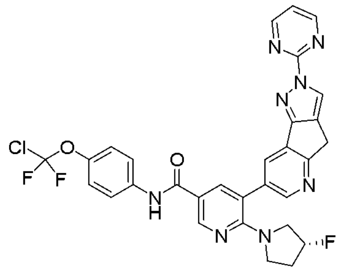,
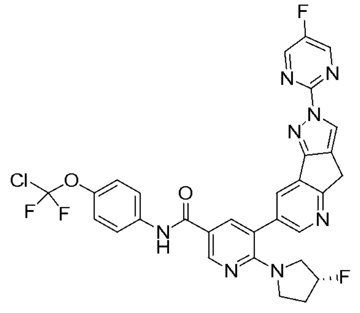,

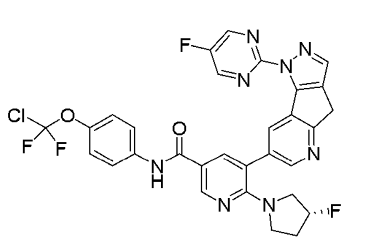,
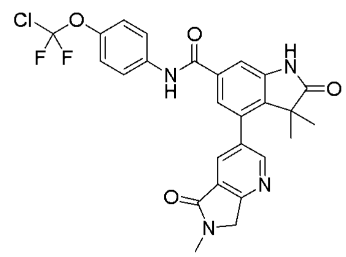,
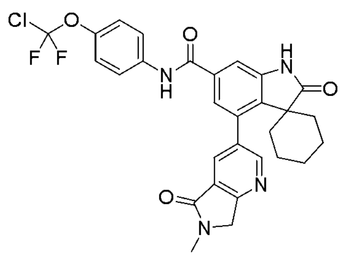,

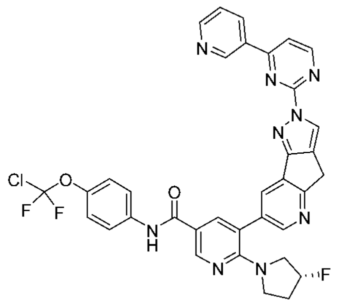,
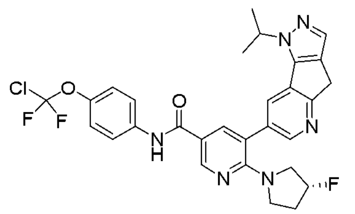,
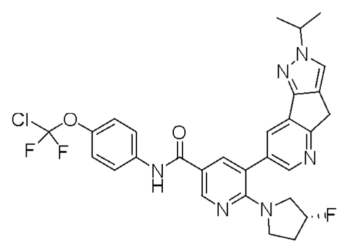,

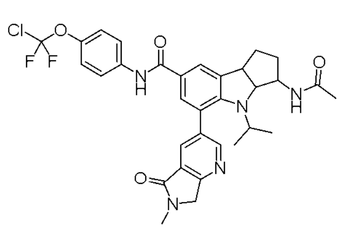,
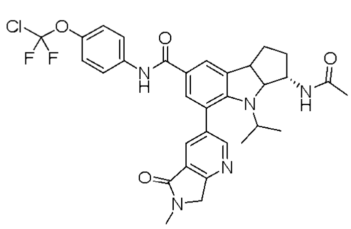,
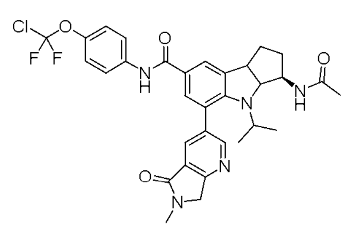,

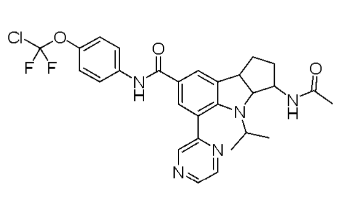,
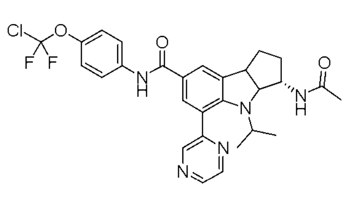,
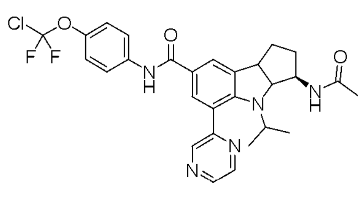,

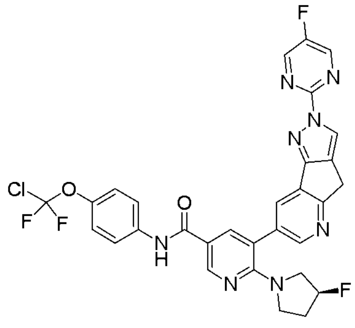 ,

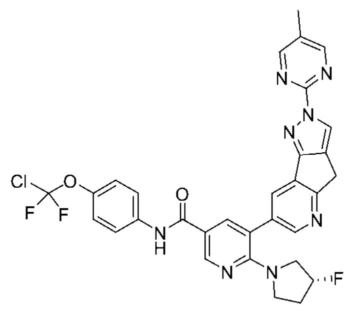 ,

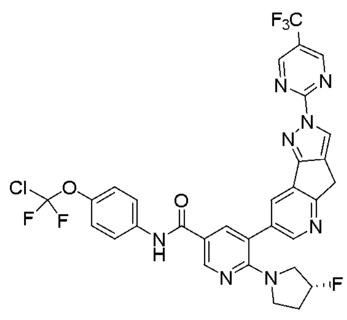 ,

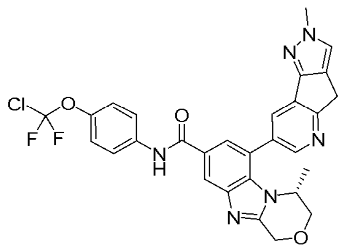 ,

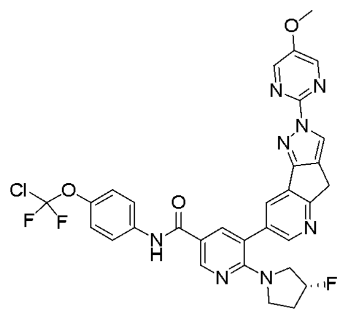 ,

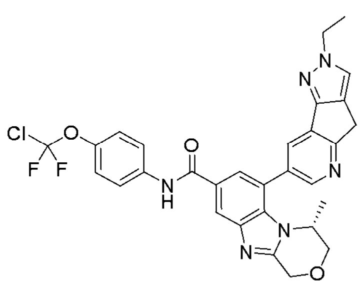 ,

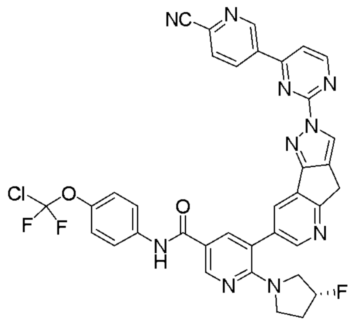 ,

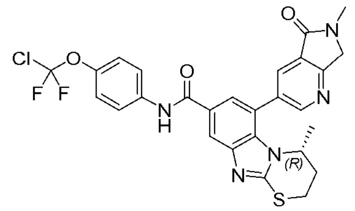 ,

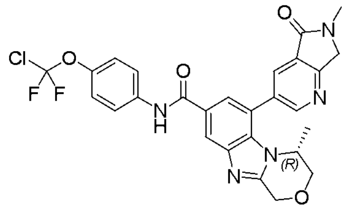 ,

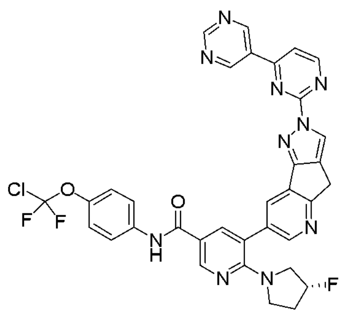 ,

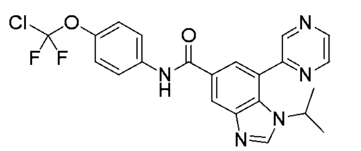 ,

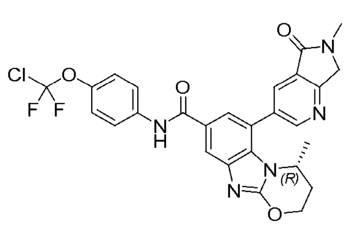 ,

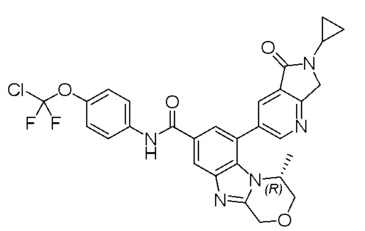 ,

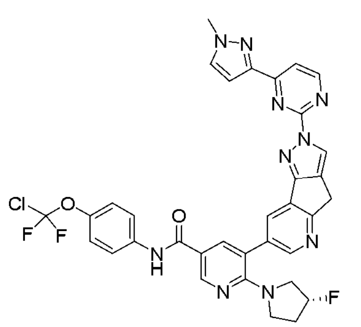 ,

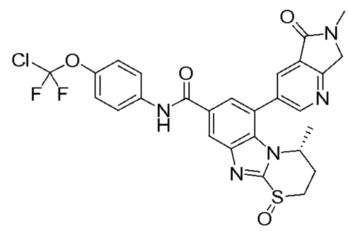 ,

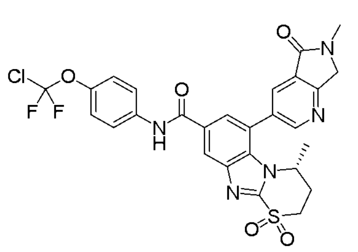
,
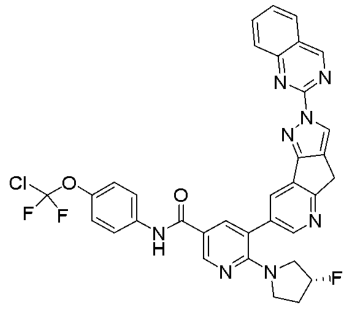
,
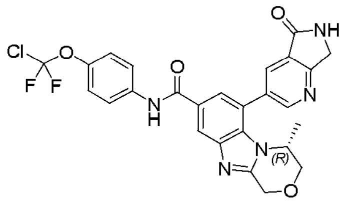
,

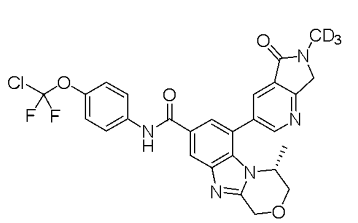
,
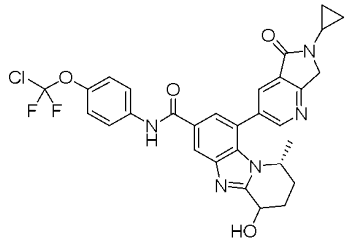
,
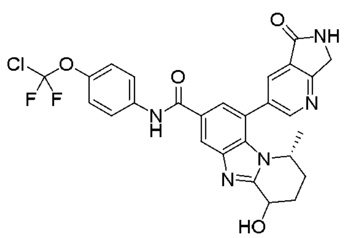
,

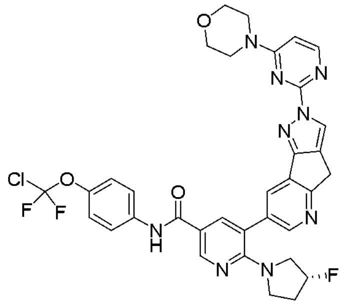
,
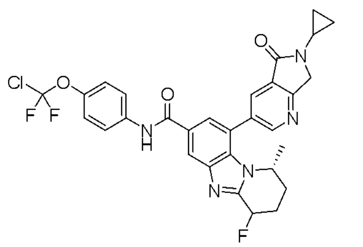
,
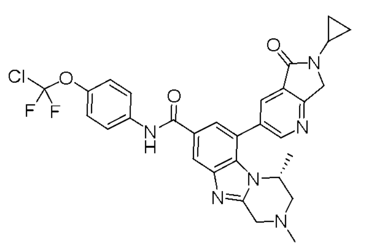
,

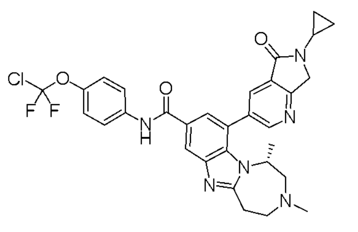
,
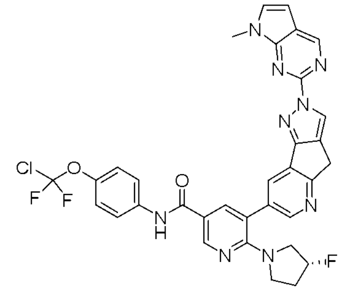
,
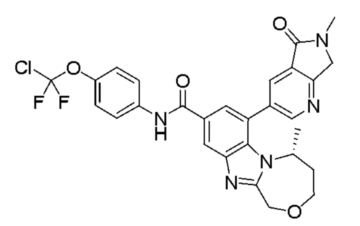
,

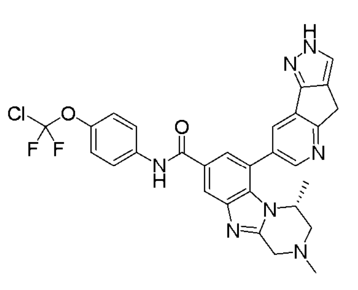
,
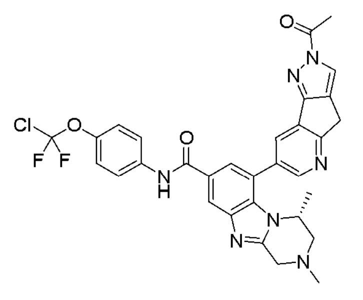
,
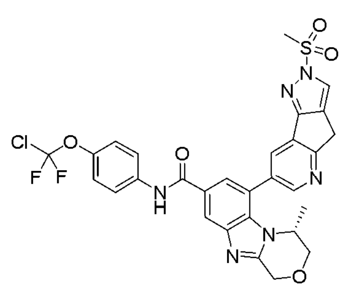
,

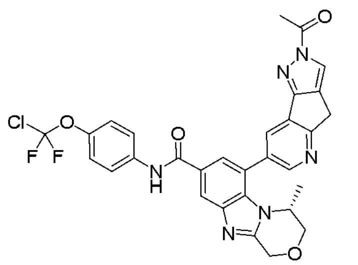 ,

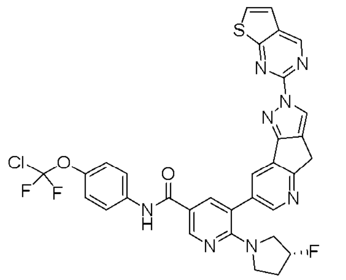 ,

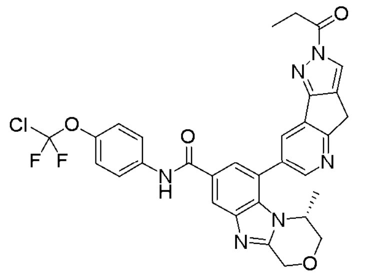 ,

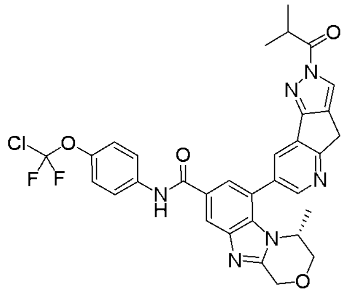 ,

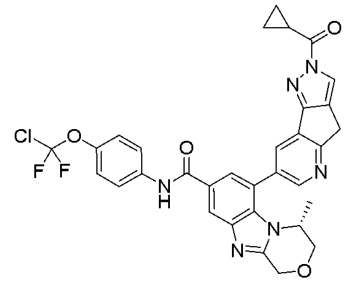 ,

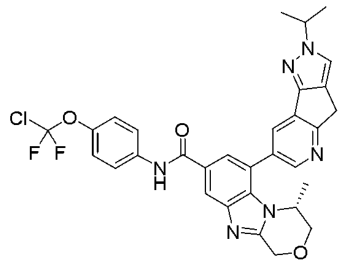 ,

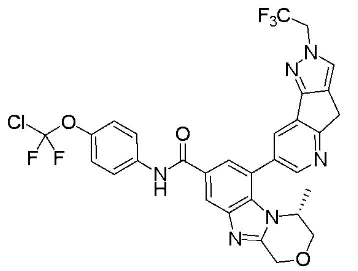 ,

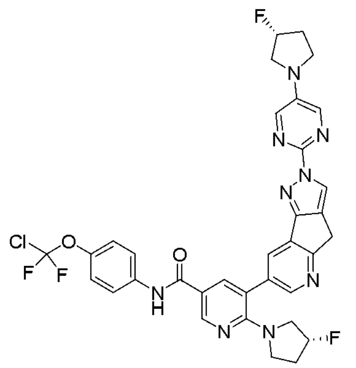 ,

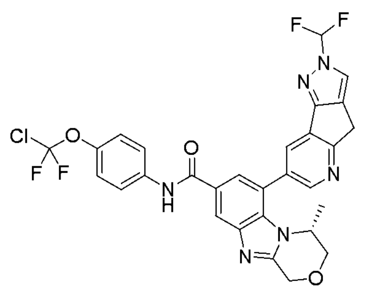 ,

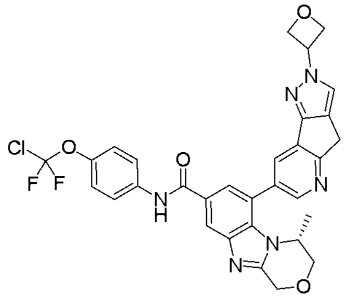 ,

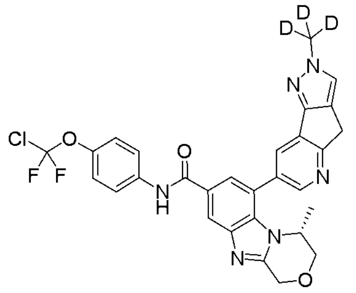 ,

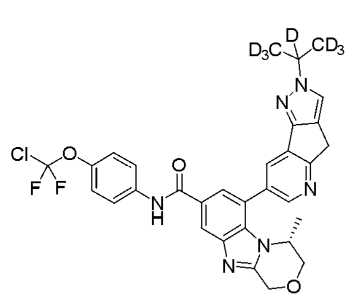 ,

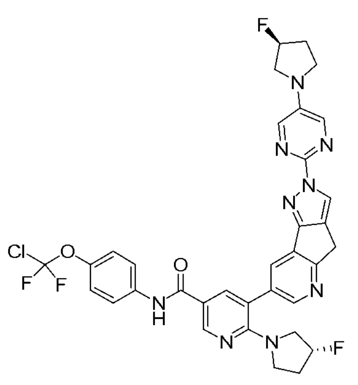 ,

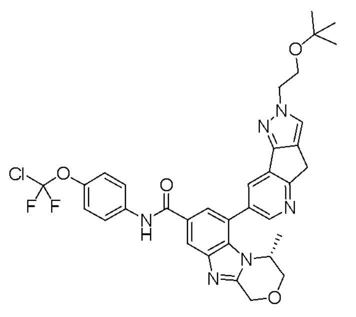 ,

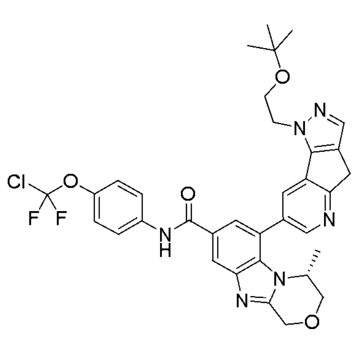 ,

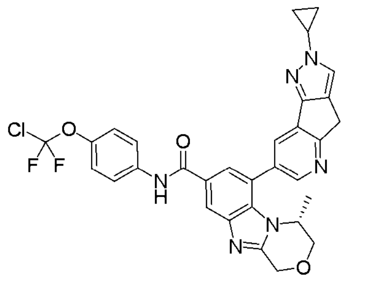
,
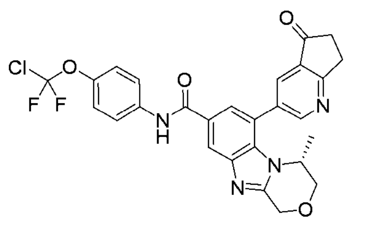
,
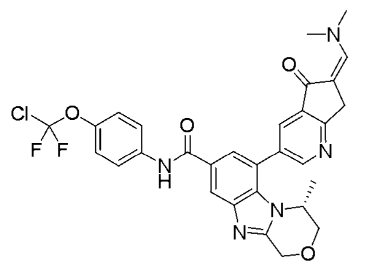
,
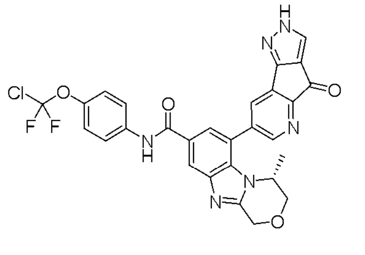
,
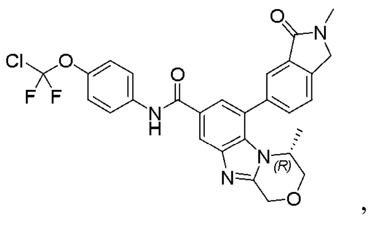
,
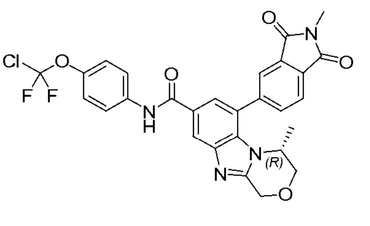
,
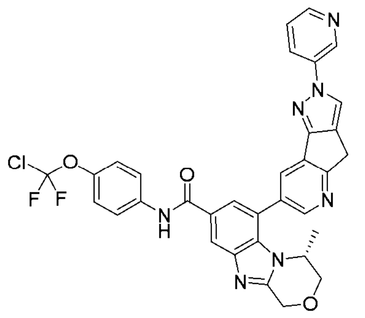
,
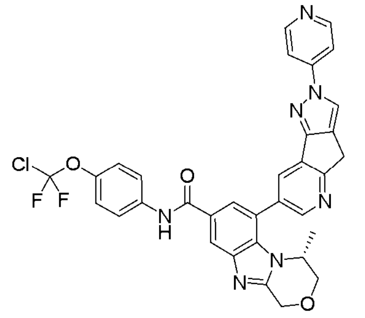
,
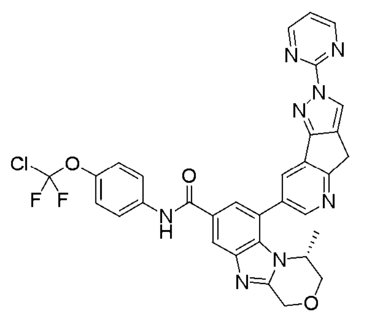
,
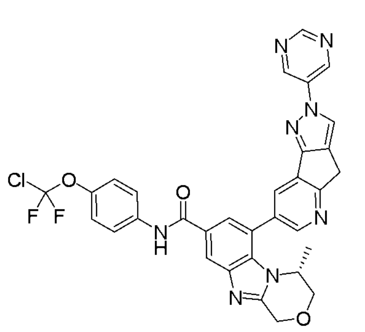
,
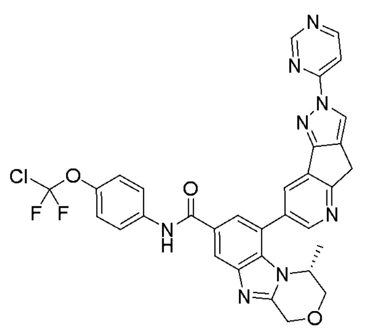
,
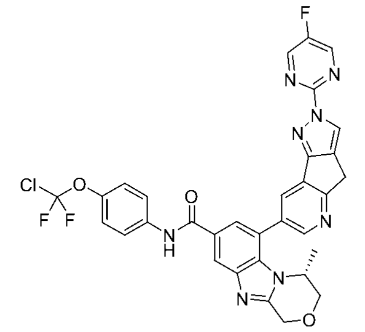
,
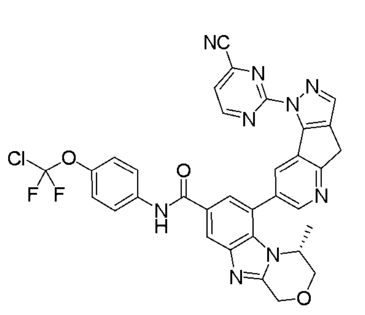
,
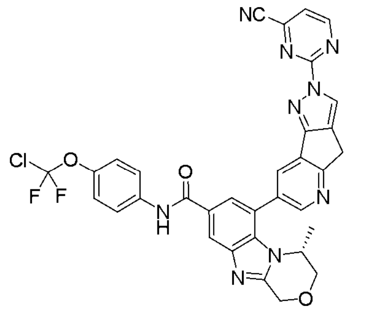
,
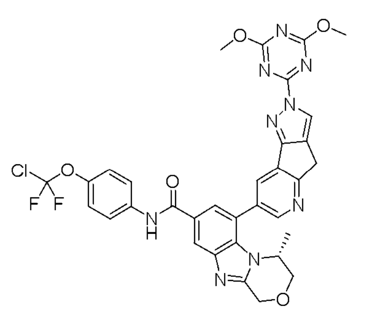
,

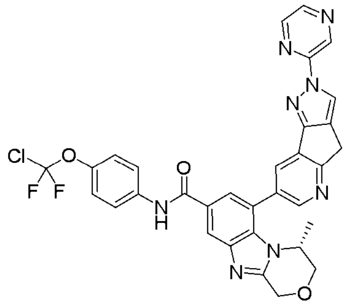,
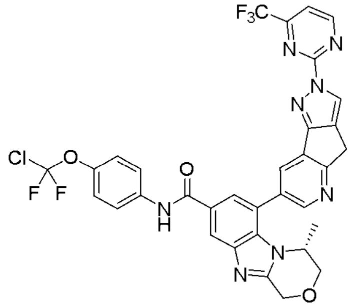,
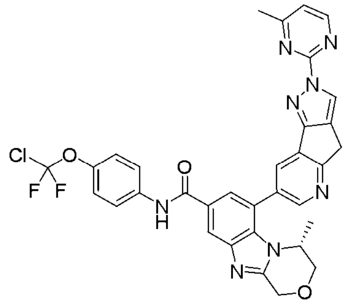,

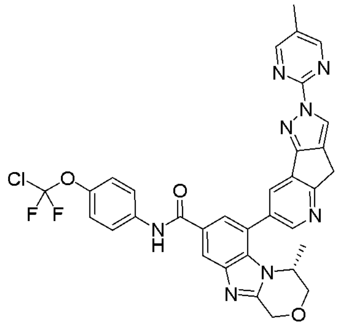,
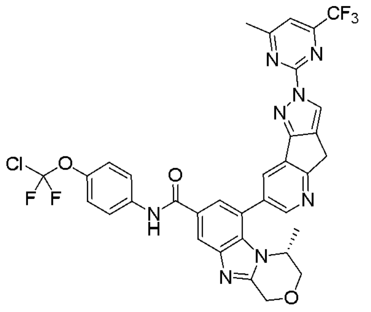,
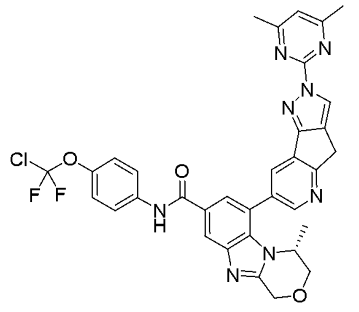,

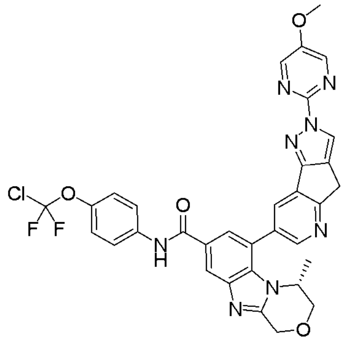,
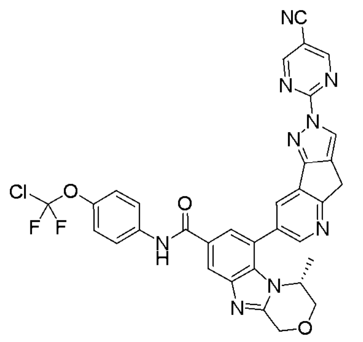,
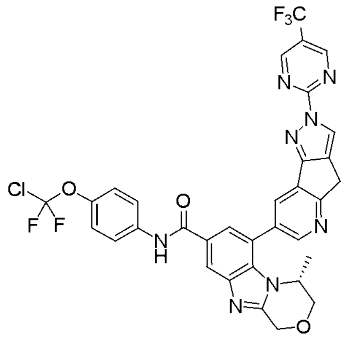,

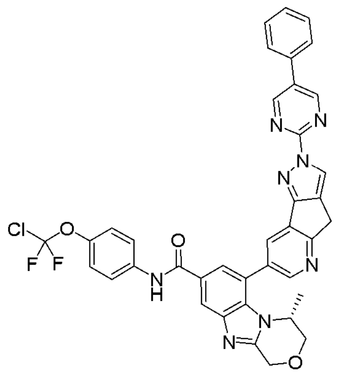,
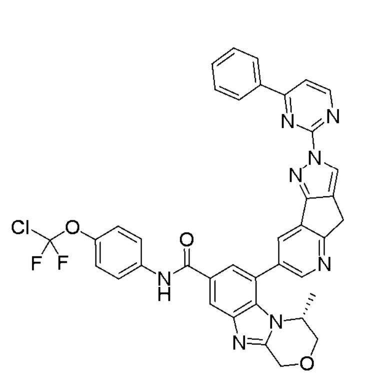,
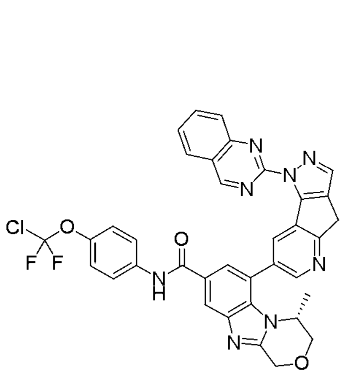,

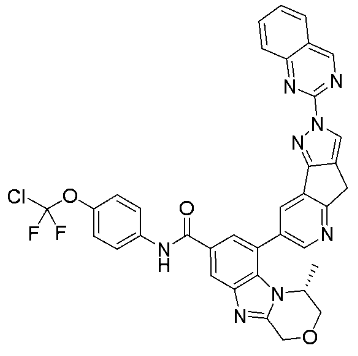,

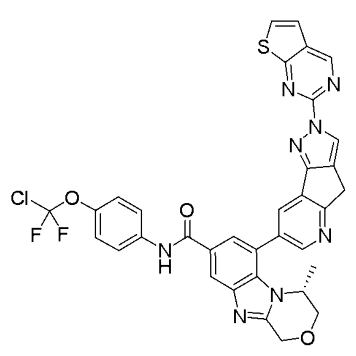,

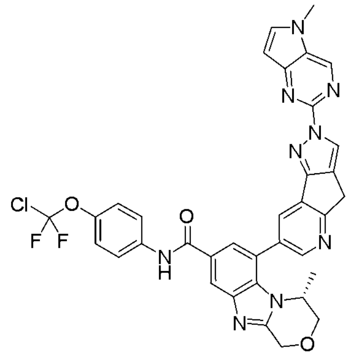,

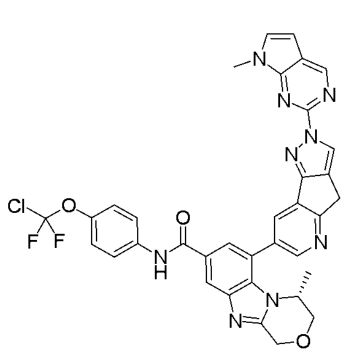,

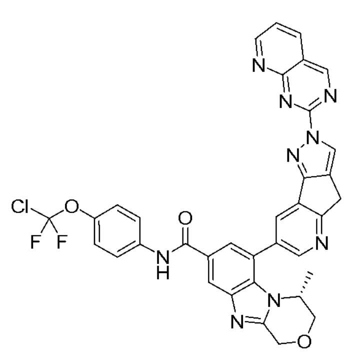,

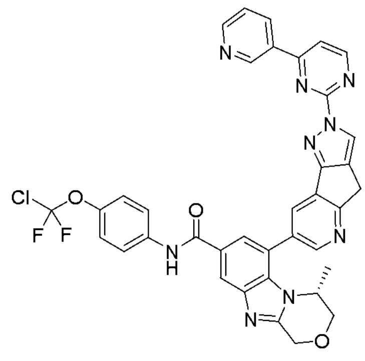,

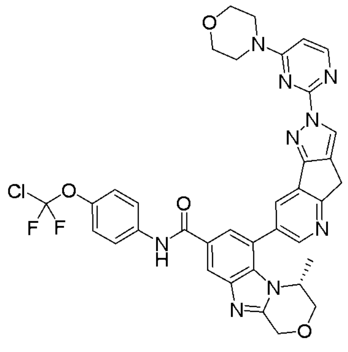,

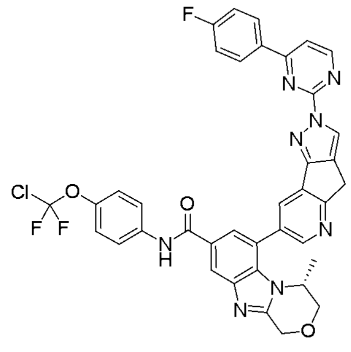,

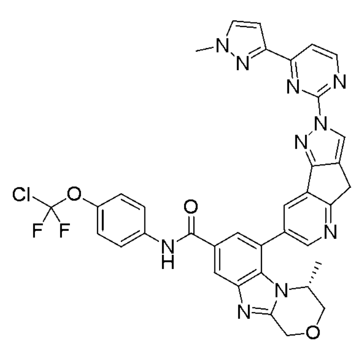,

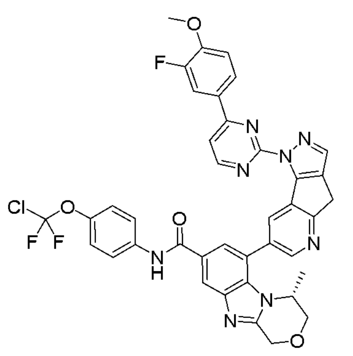,

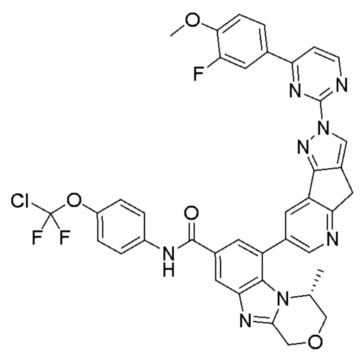,

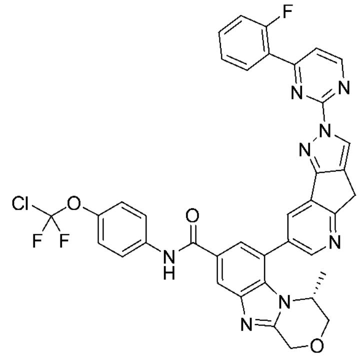,

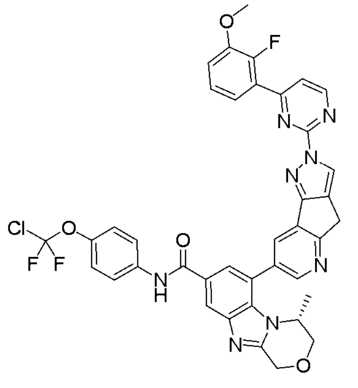 ,

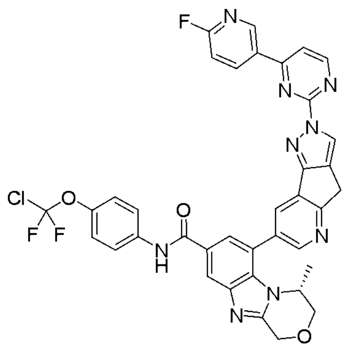 ,

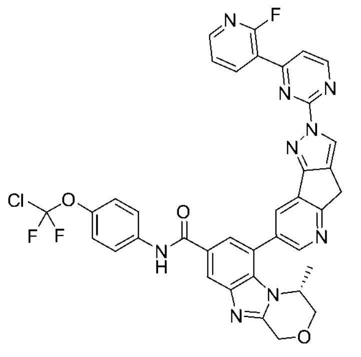 ,

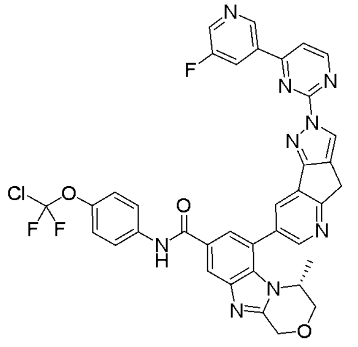 ,

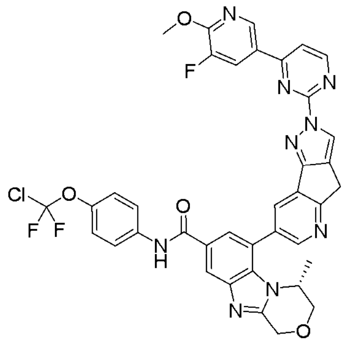 ,

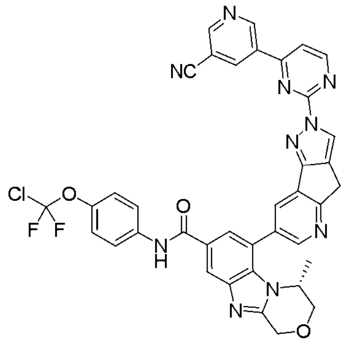 ,

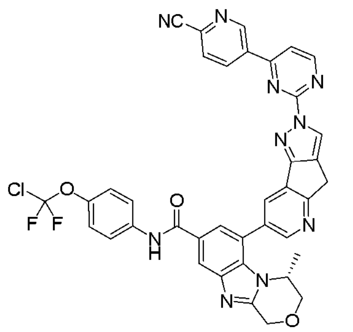 ,

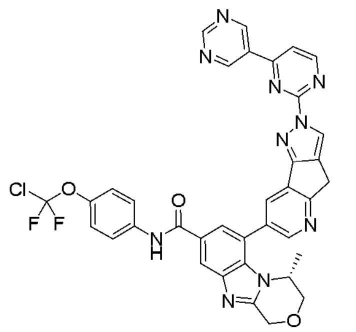 ,

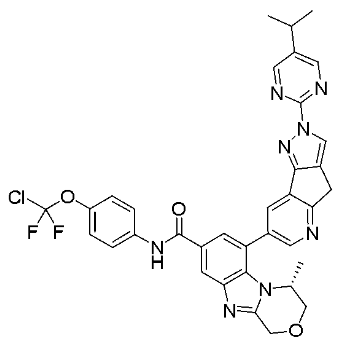 ,

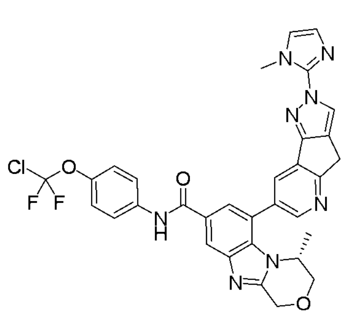 ,

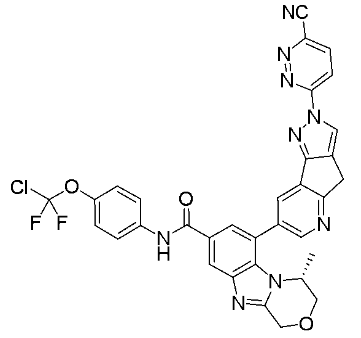 ,

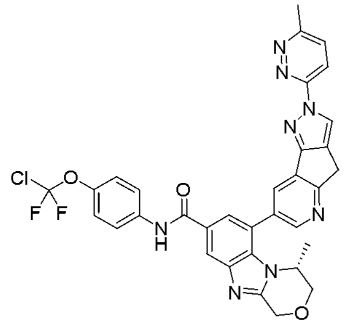 ,

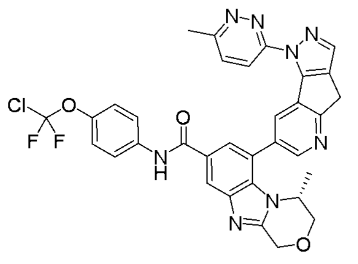,
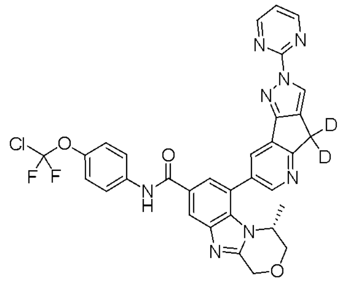,
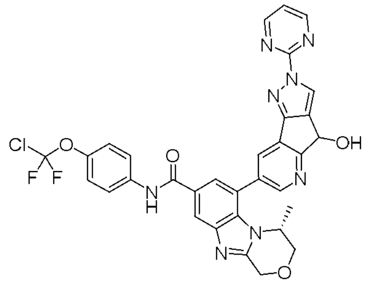,
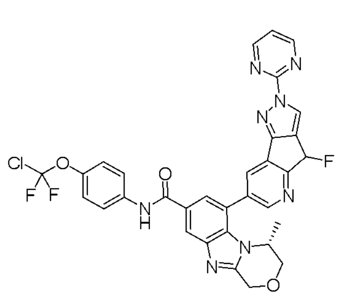,
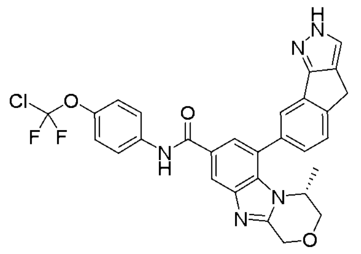,
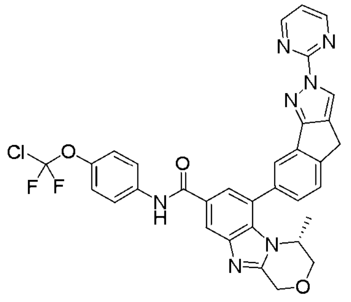,
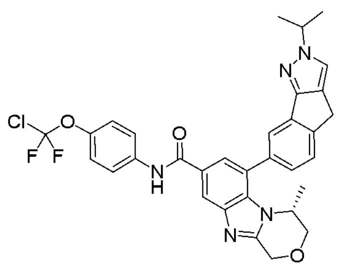,
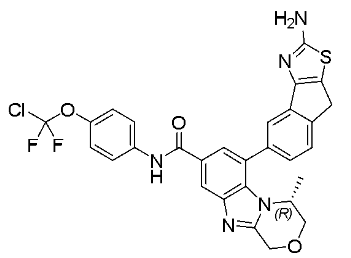,
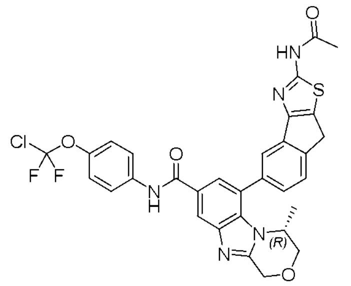,
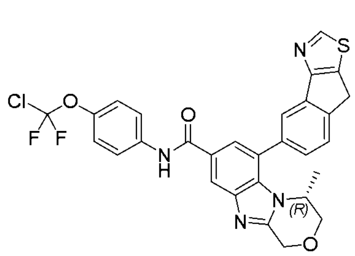,
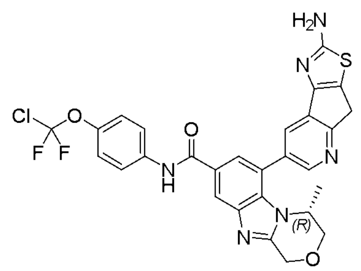,
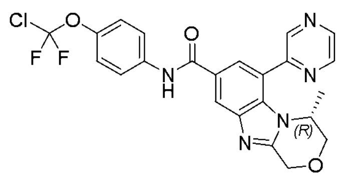,
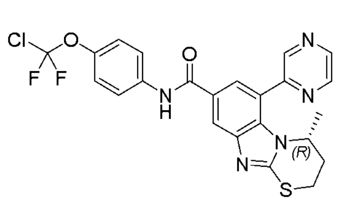,
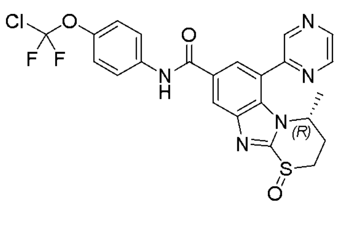,
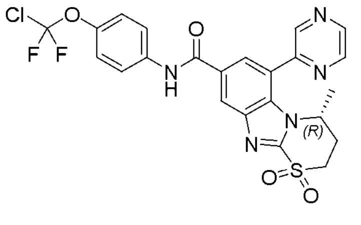,

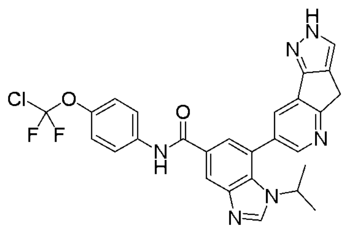 , 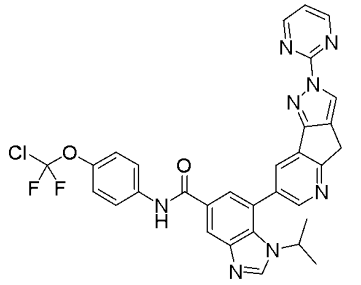 , 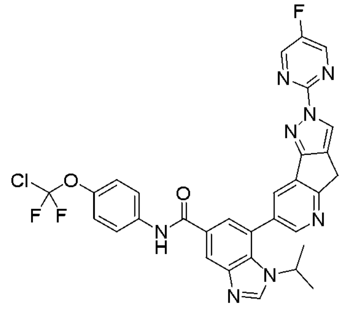 ,

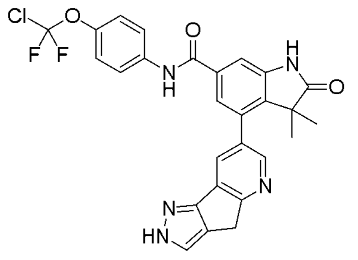 , 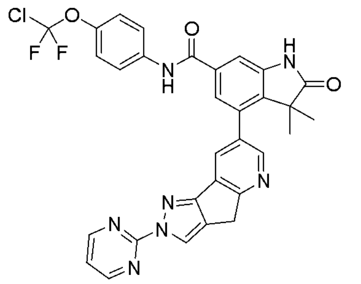 , 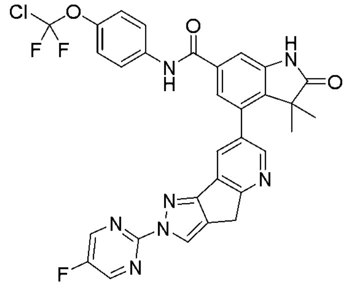 ,

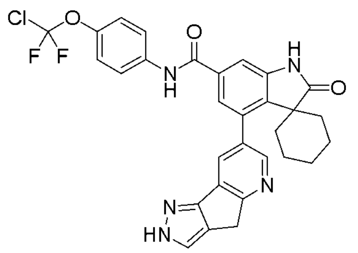 , 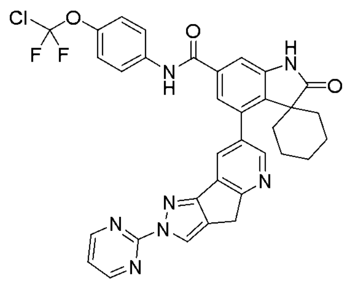 , 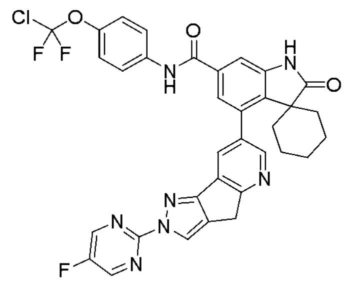 ,

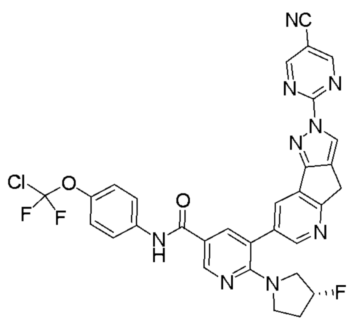 , 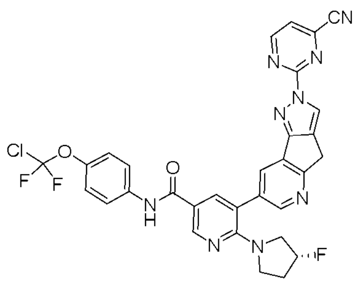 , 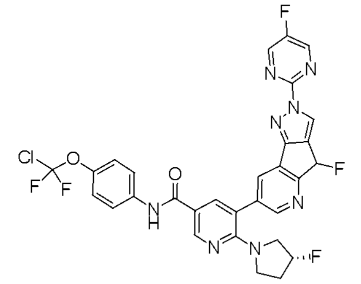 ,

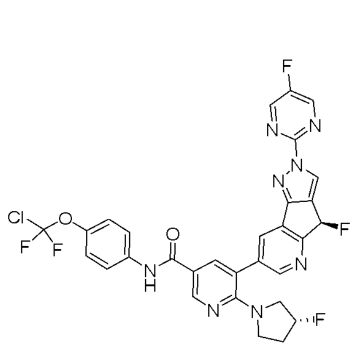 , 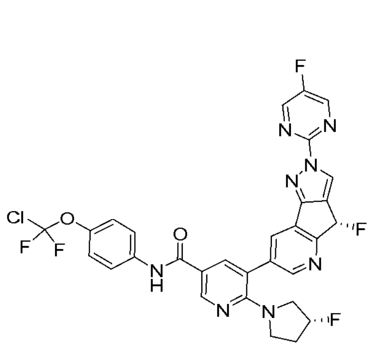 , 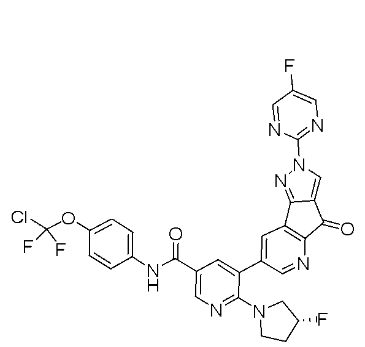 ,

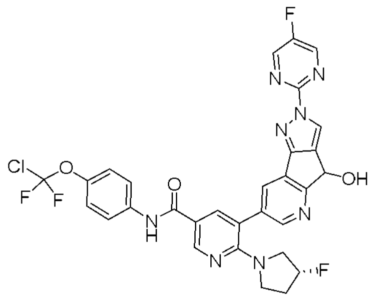
,
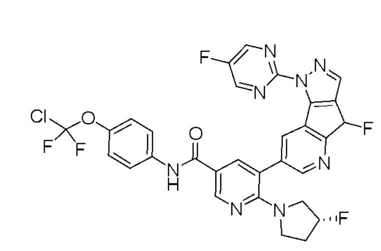
,
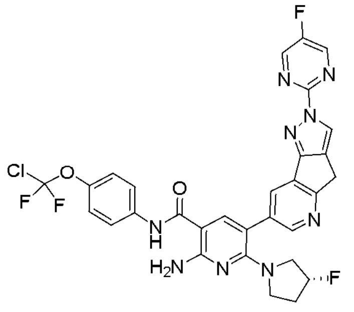
,

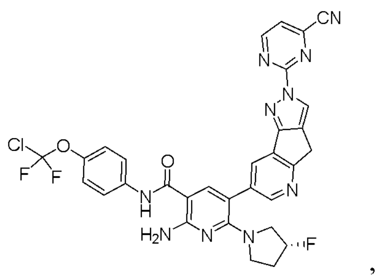
,
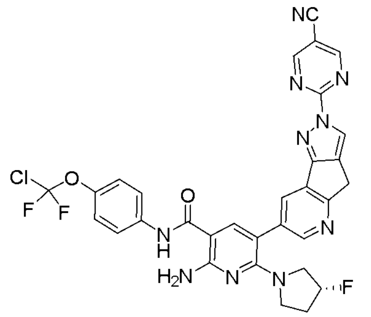
,
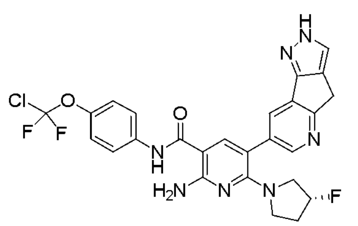
,

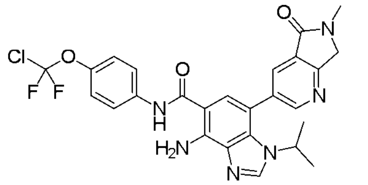
,
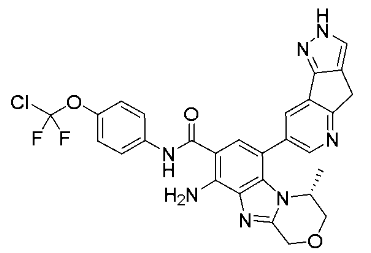
,
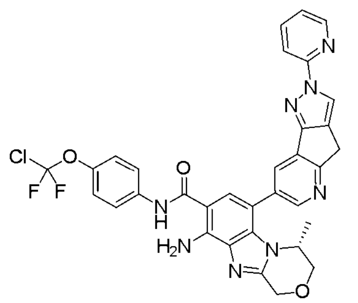
,

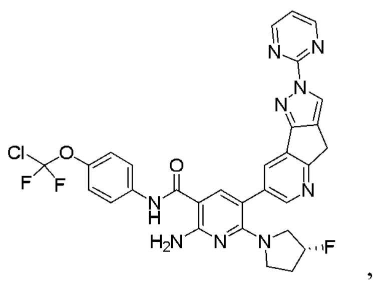
,
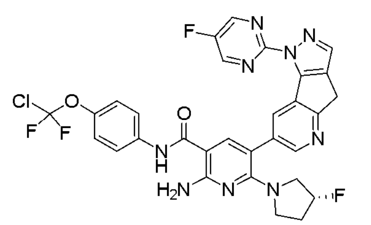
,
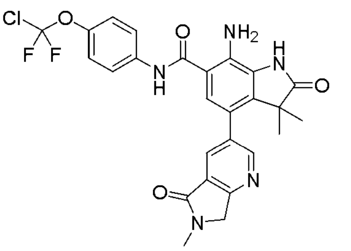
,

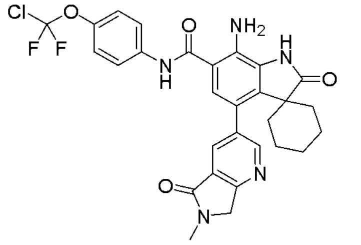
,
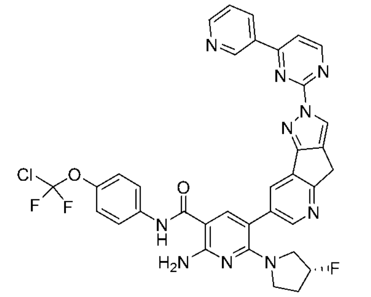
,
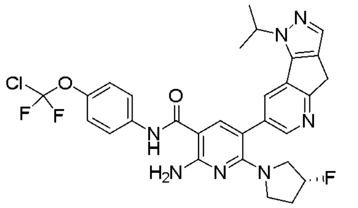
,

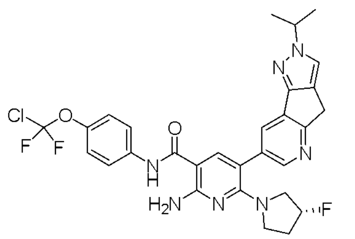,

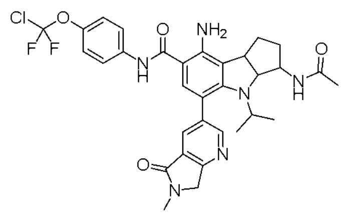,

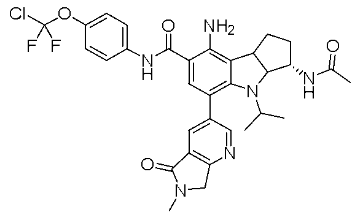,

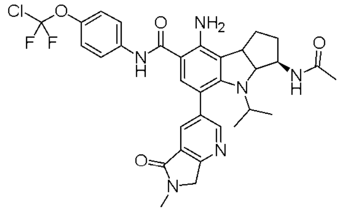,

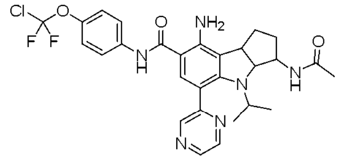,

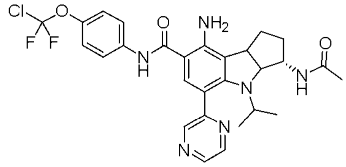,

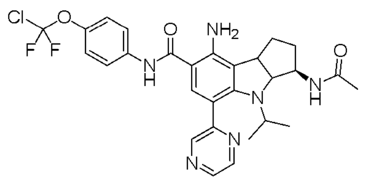,

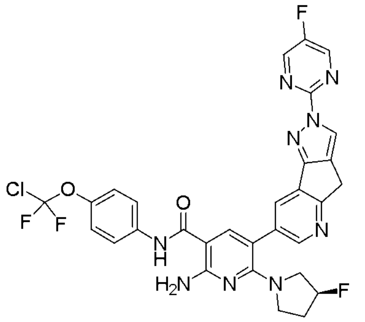,

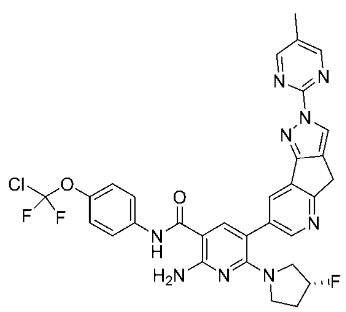,

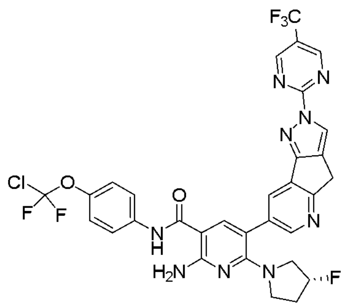,

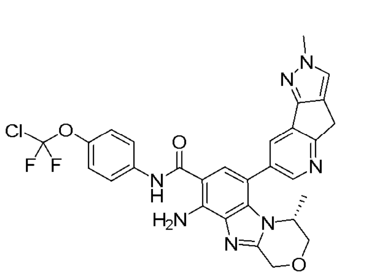,

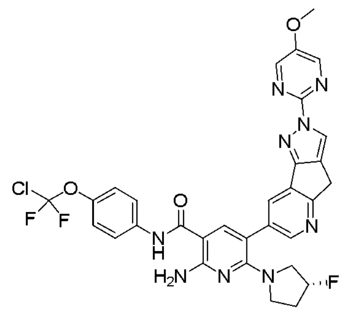,

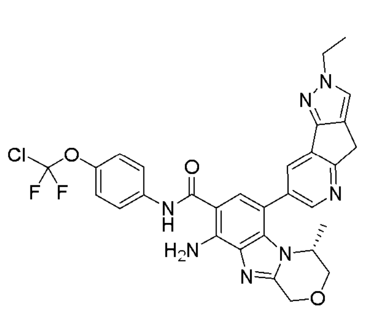,

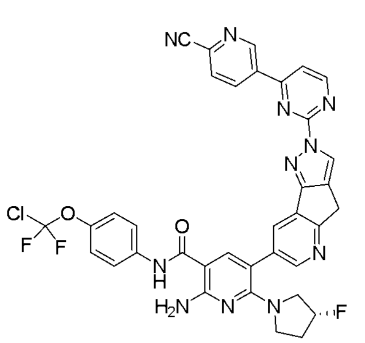,

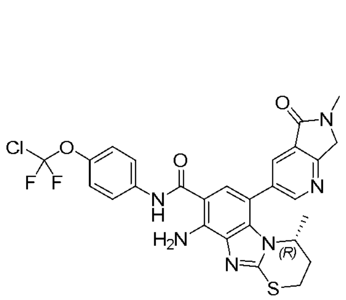,

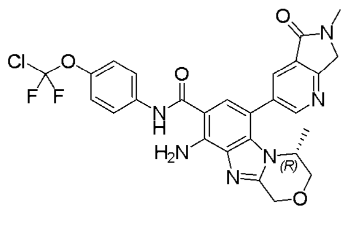,
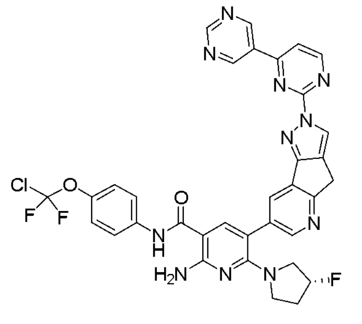,
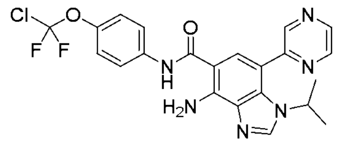,

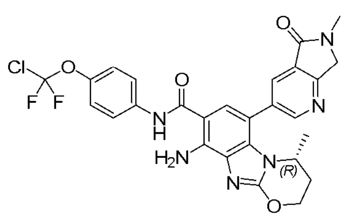,
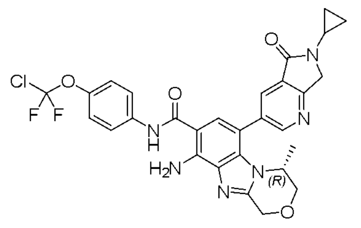,
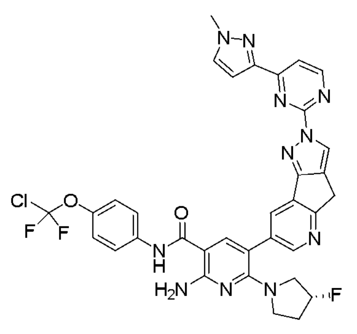,

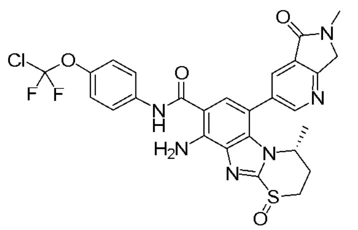,
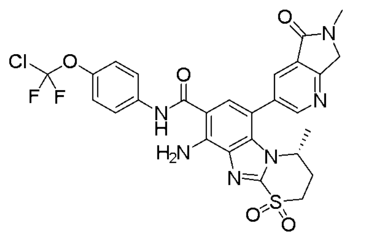,
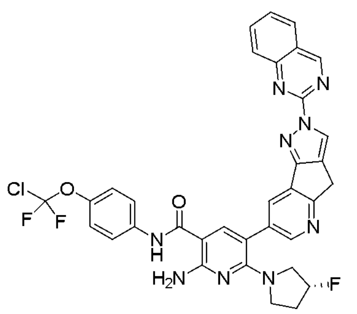,

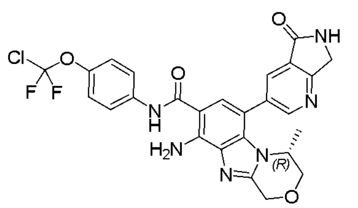,
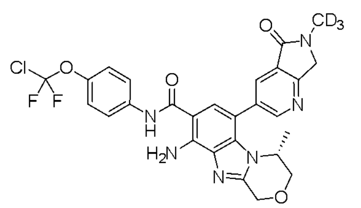,
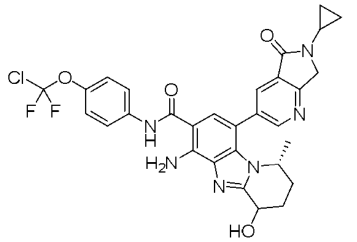,

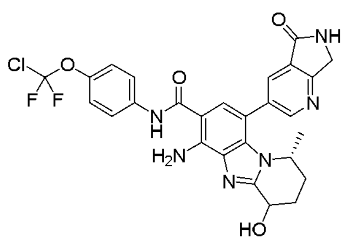,
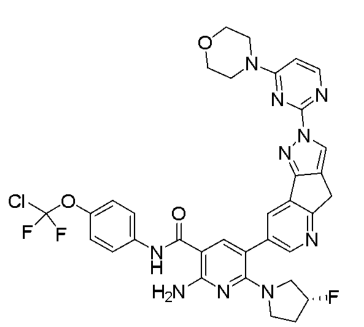,
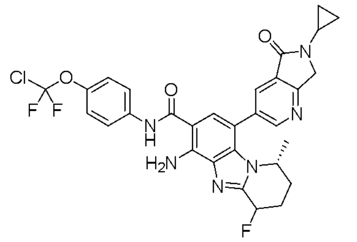,

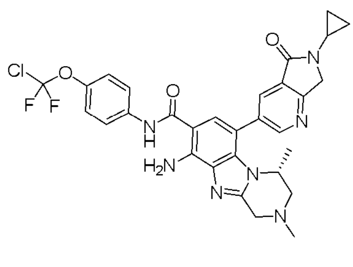 , 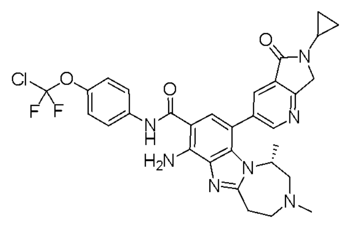 , 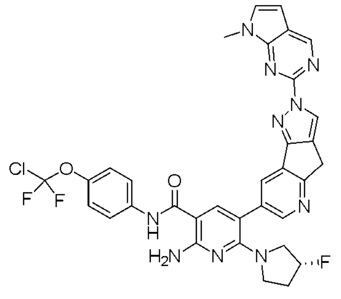 ,

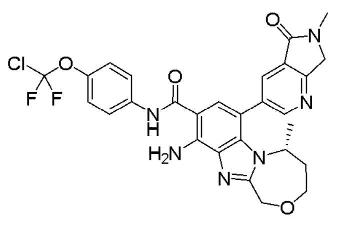 , 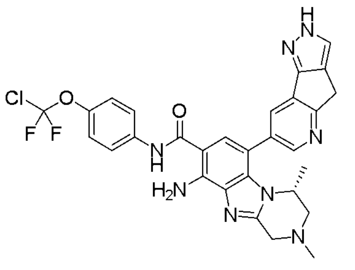 , 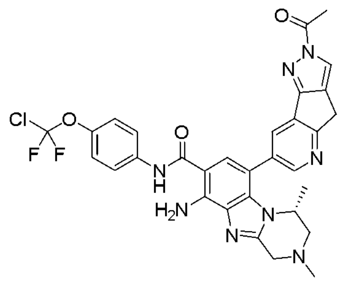 ,

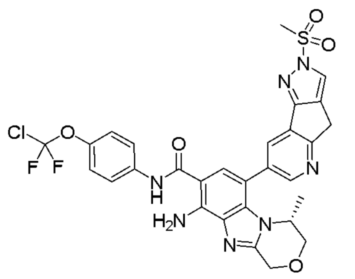 , 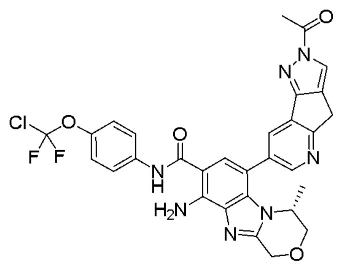 , 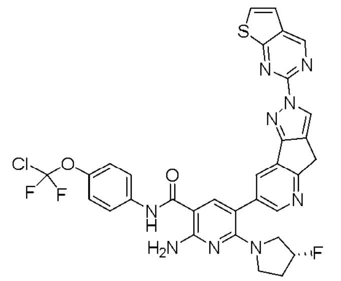 ,

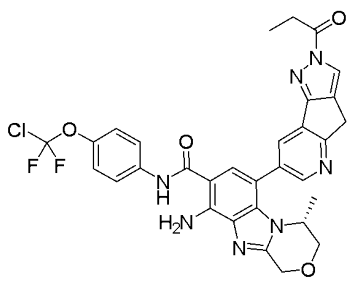 , 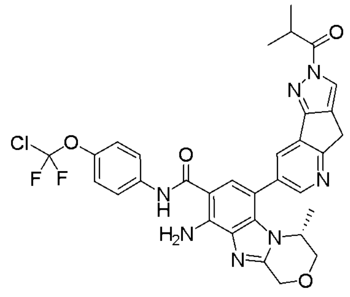 , 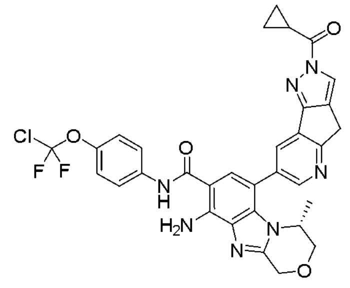 ,

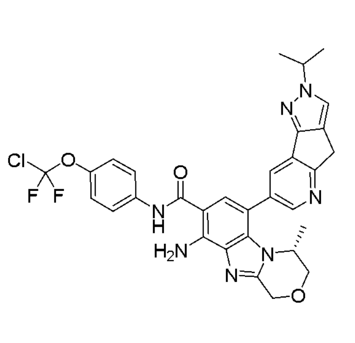 , 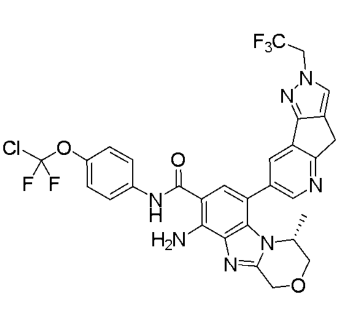 , 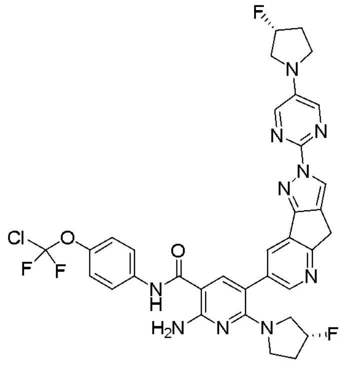 ,

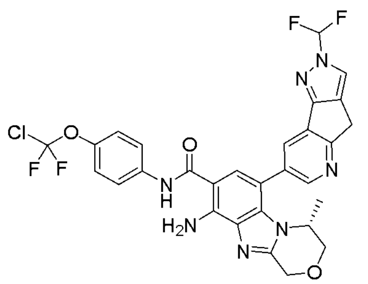,
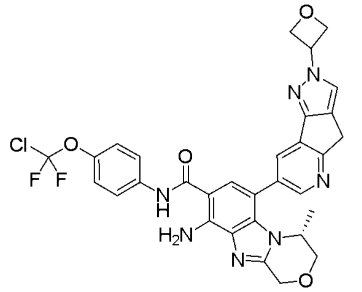,
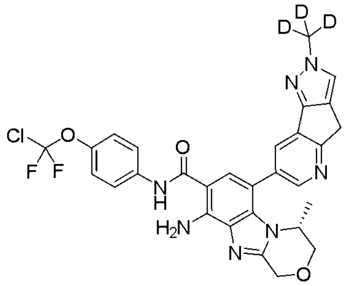,

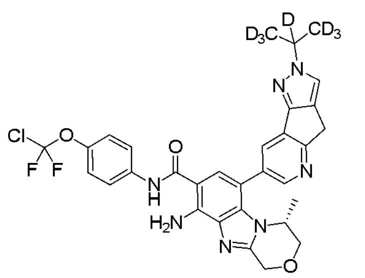,
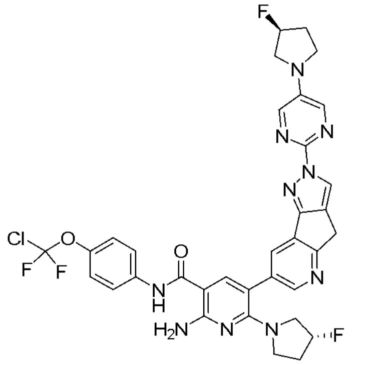,
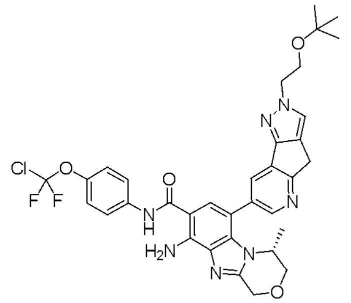,

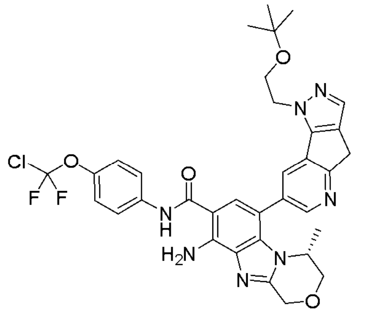,
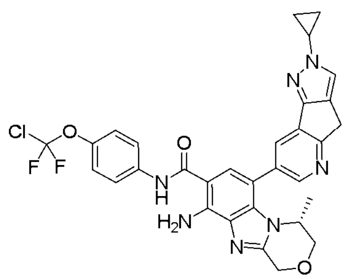,
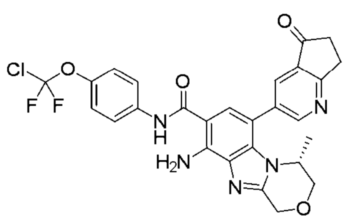,

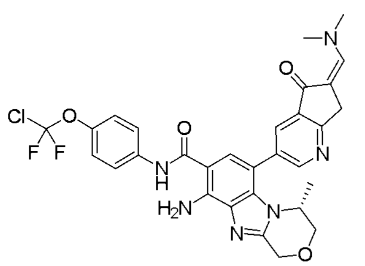,
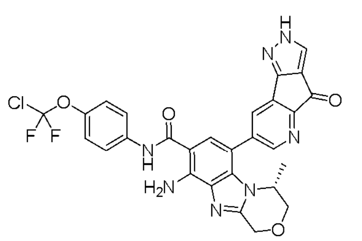,
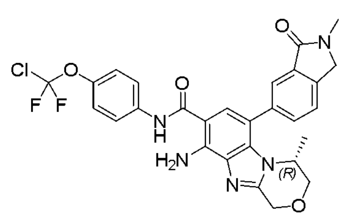,

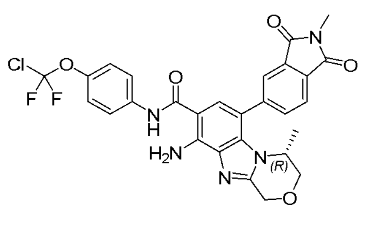,
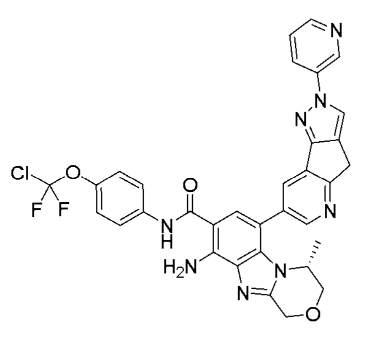,
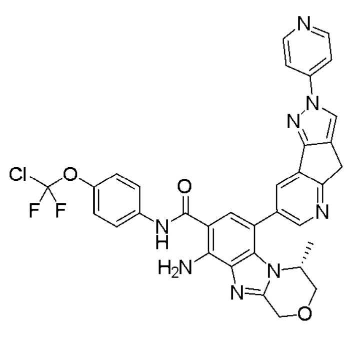,

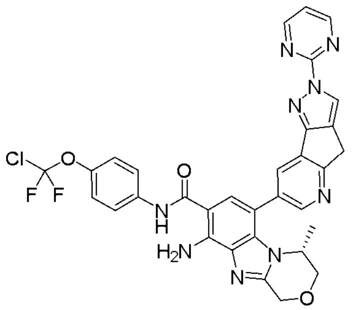,

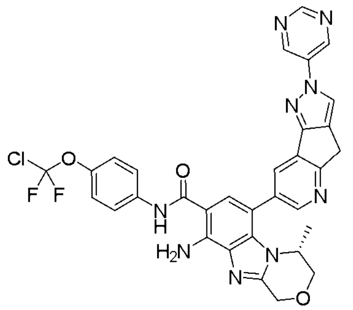,

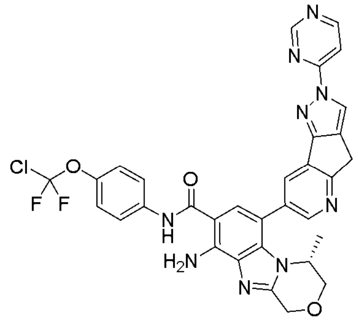,

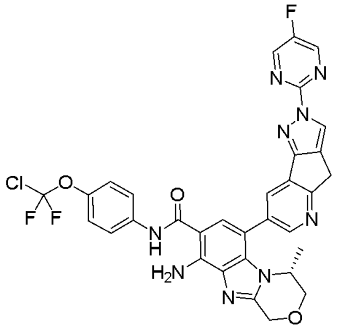,

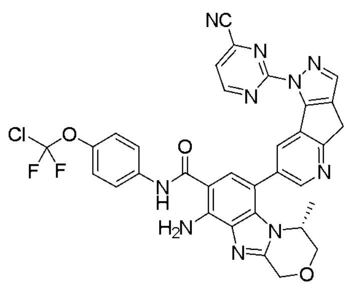,

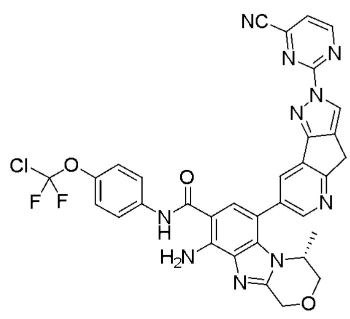,

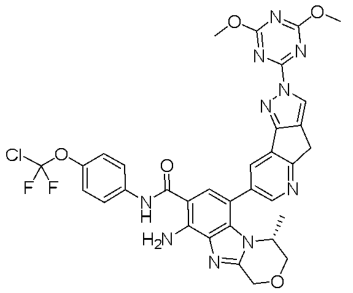,

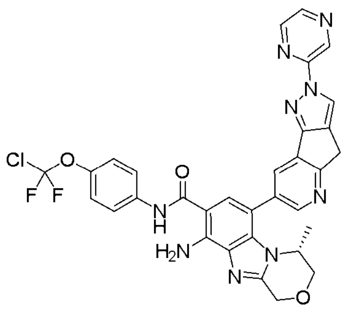,

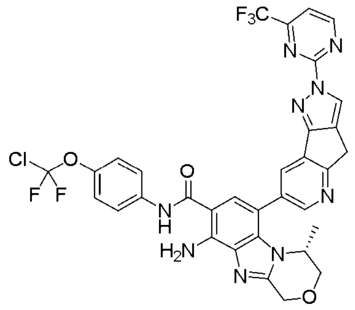,

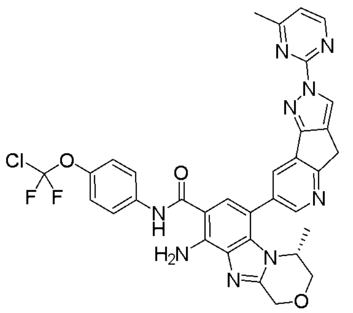,

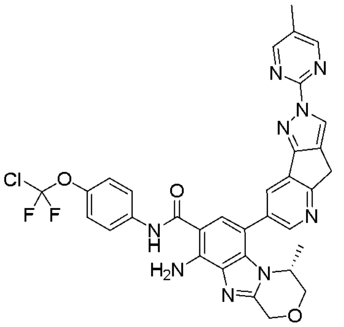,

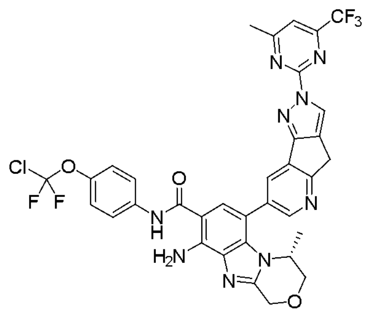,

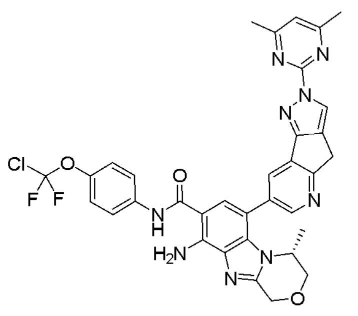,

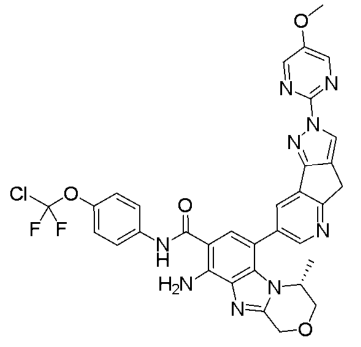,

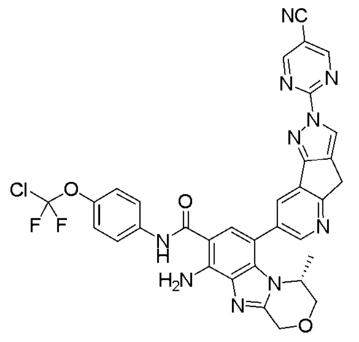,

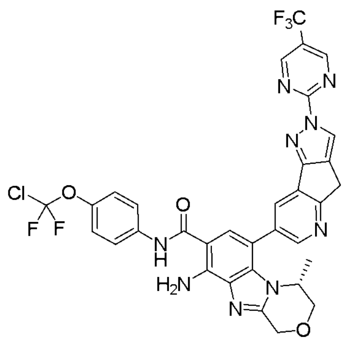,

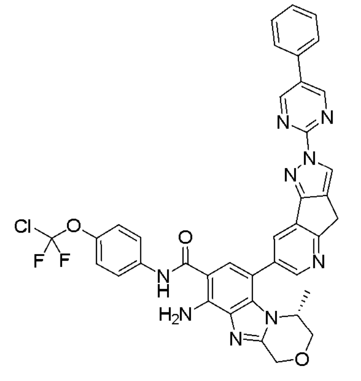,

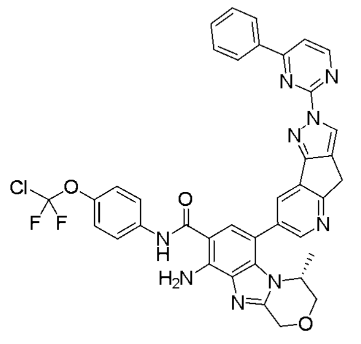,

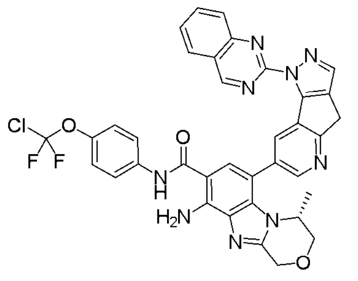,

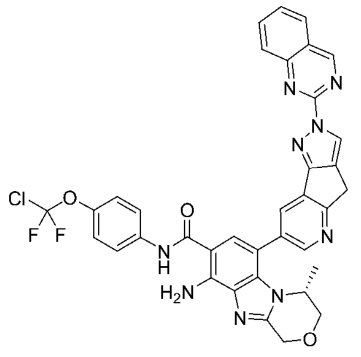,

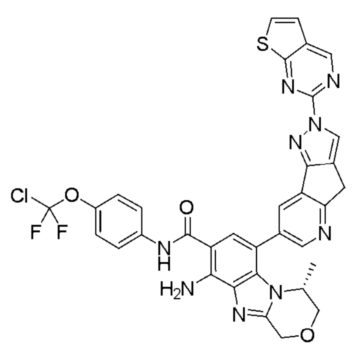,

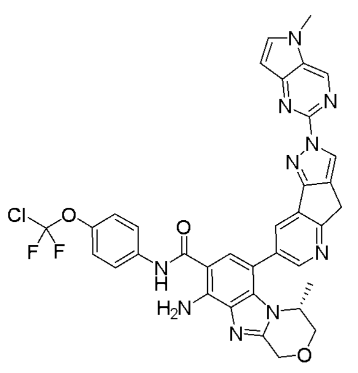,

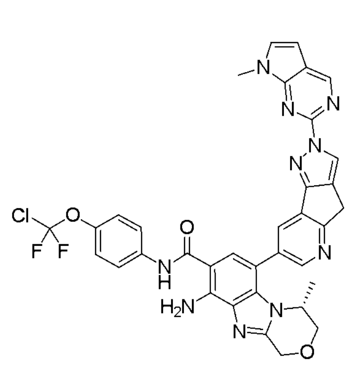,

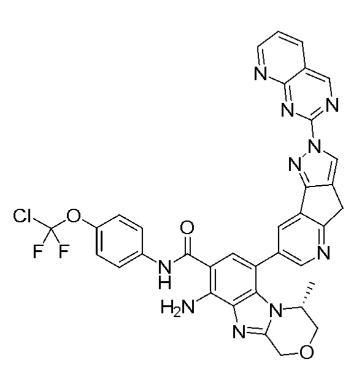,

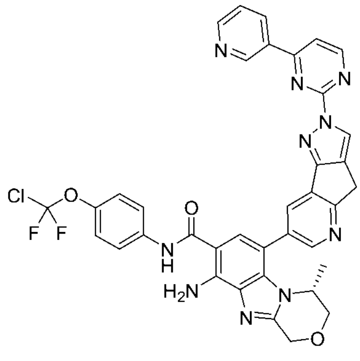,
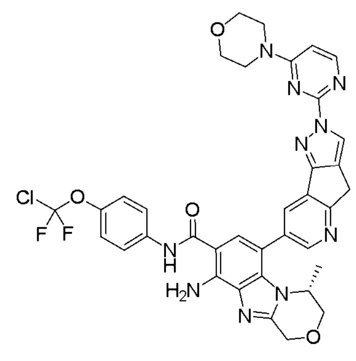,
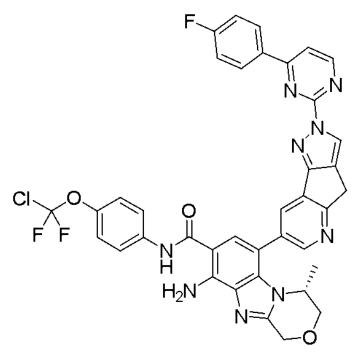,

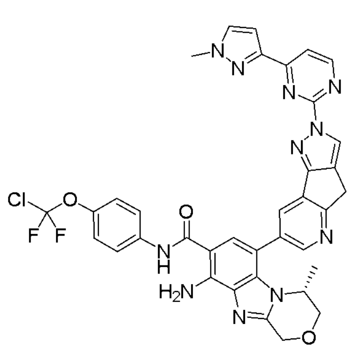,
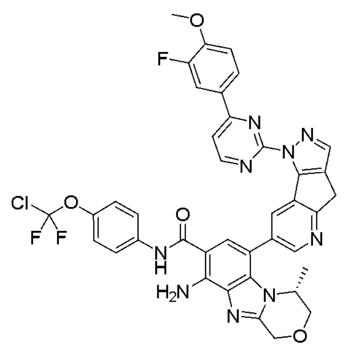,
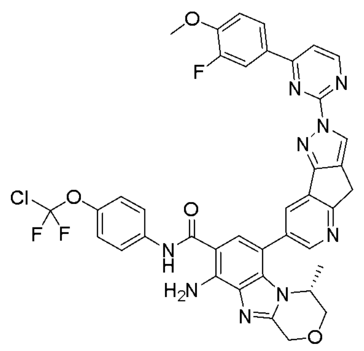,

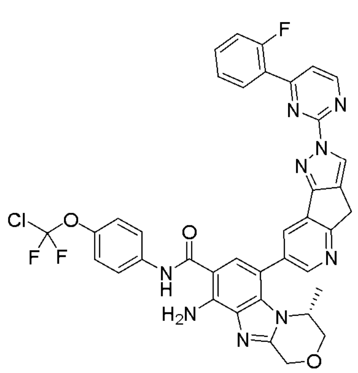,
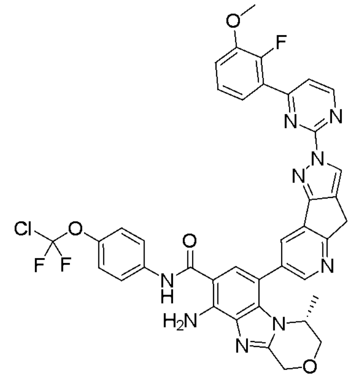,
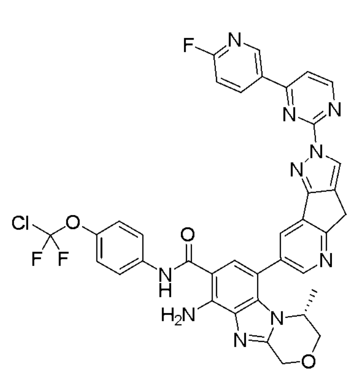,

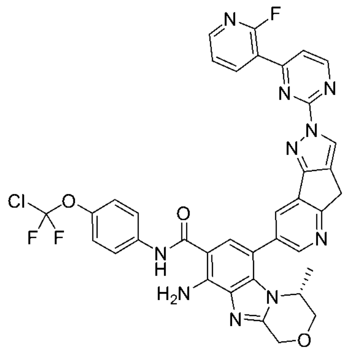,
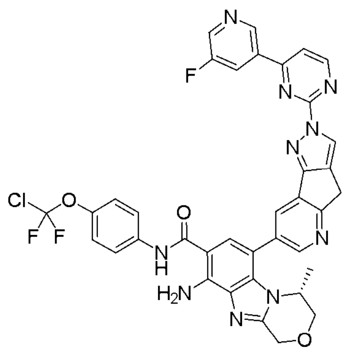,
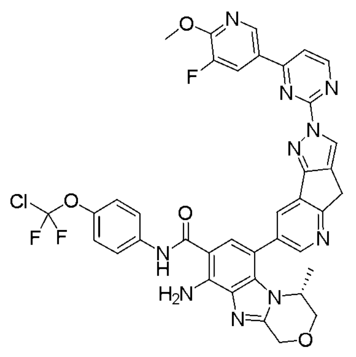,

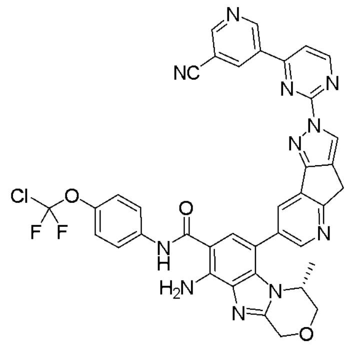,
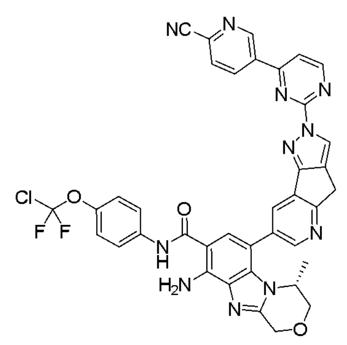,
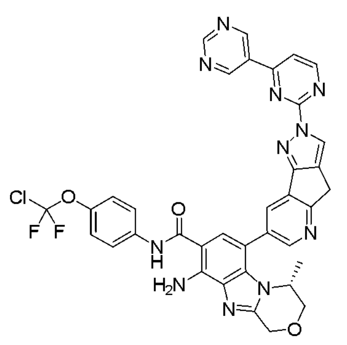,

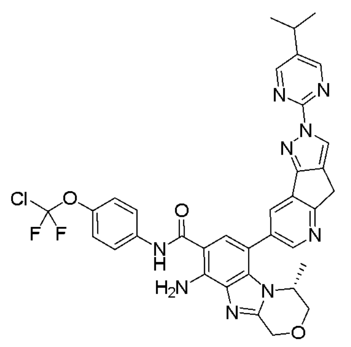,
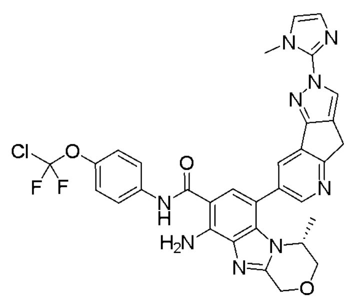,
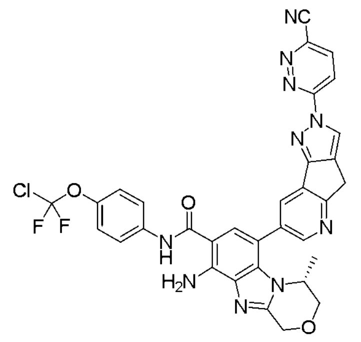,

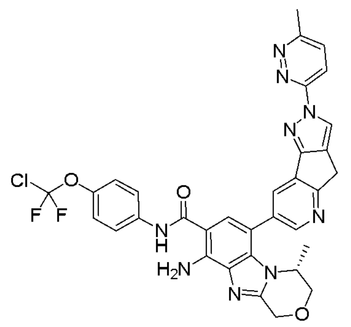,
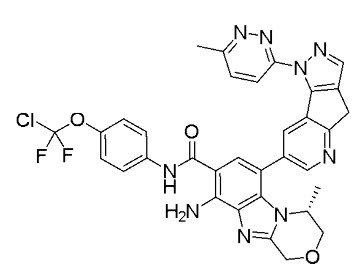,
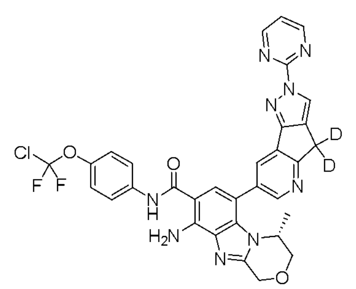,

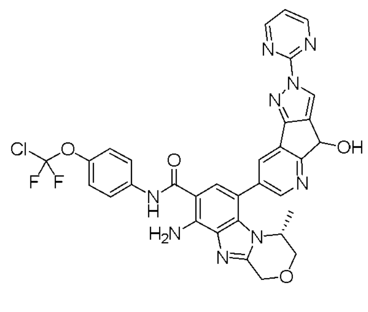,
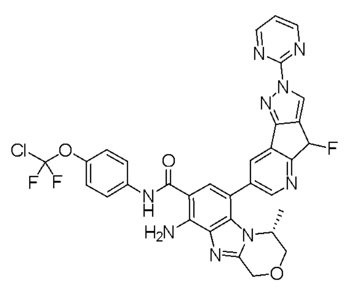,
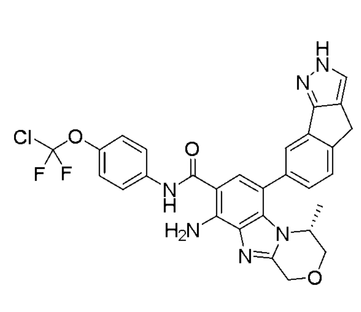,

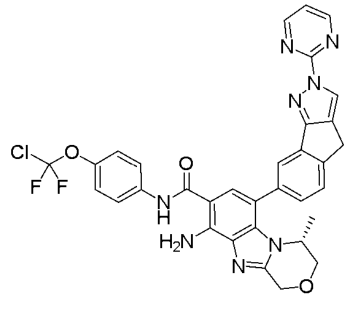,
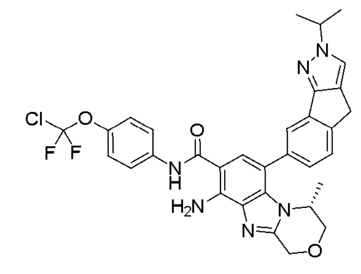,
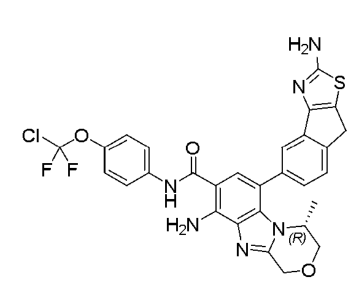,

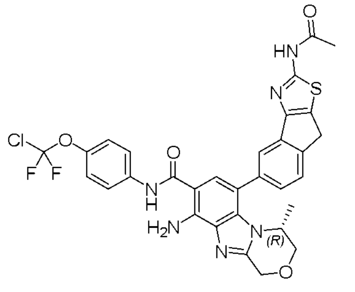
,
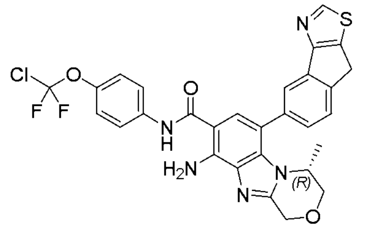
,
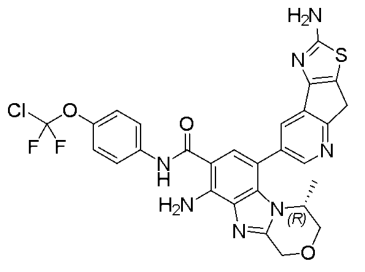
,
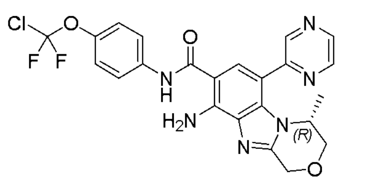
,
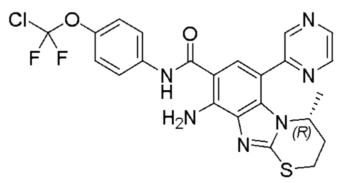
,
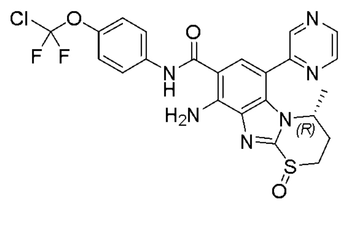
,
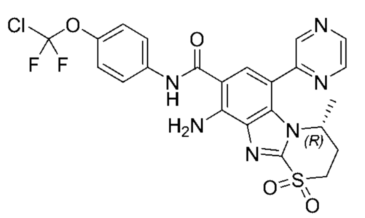
,
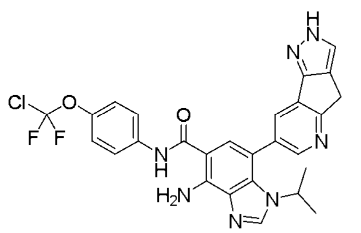
,
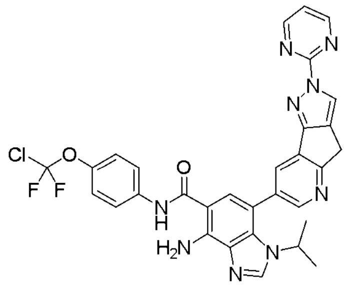
,
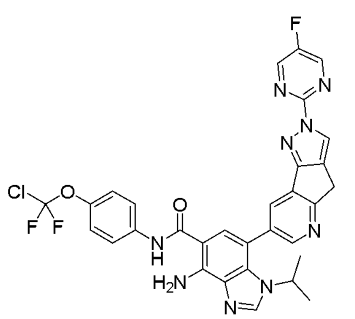
,
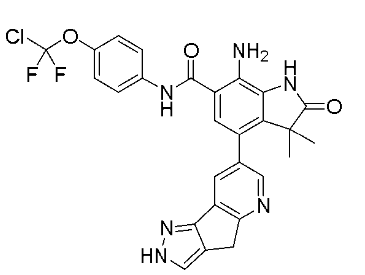
,
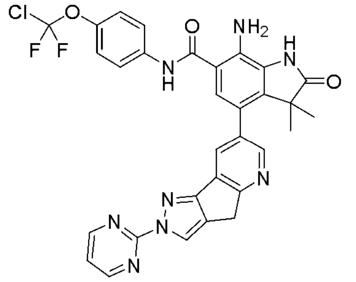
,
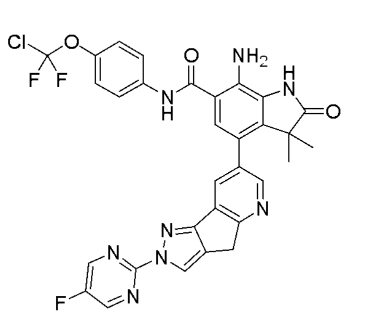
,
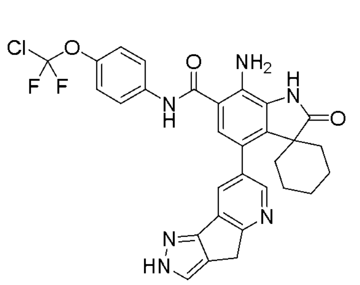
,
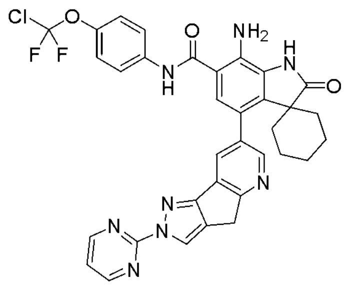
,

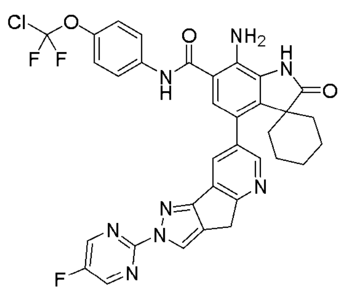
,
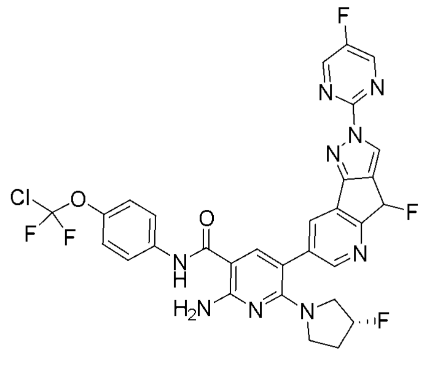
,

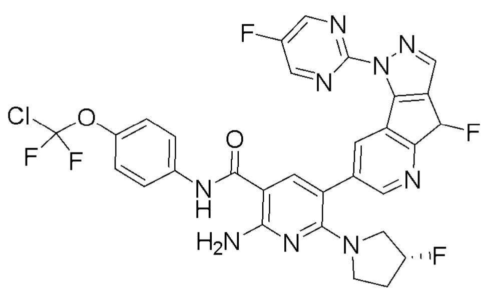
,
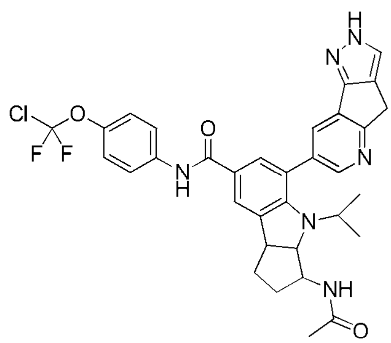
,

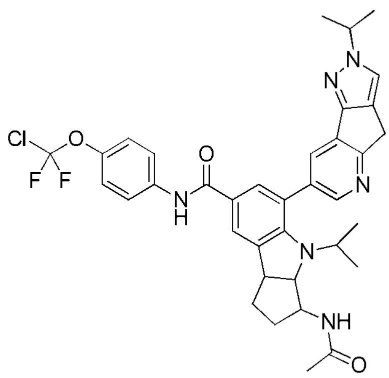
,
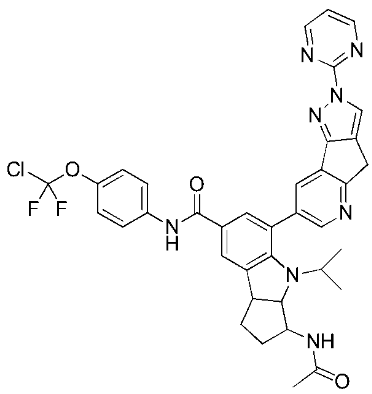
,
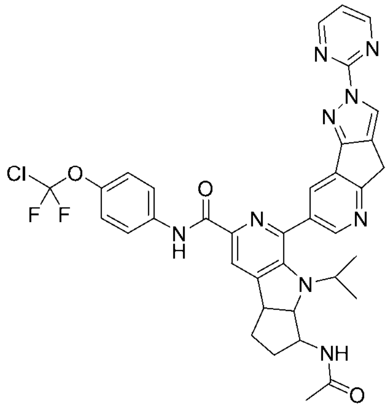
,

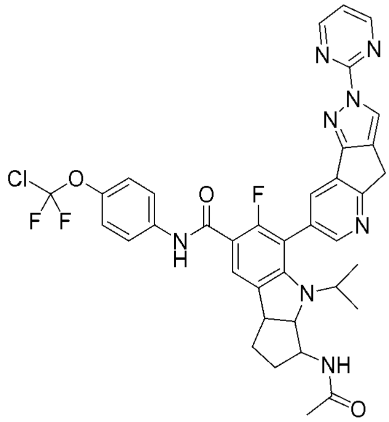
,
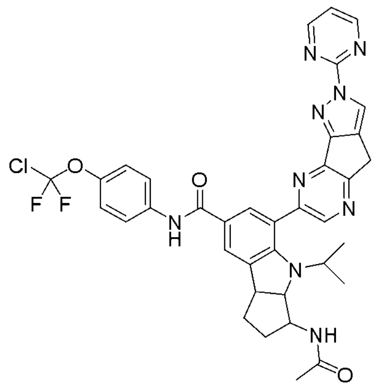
,
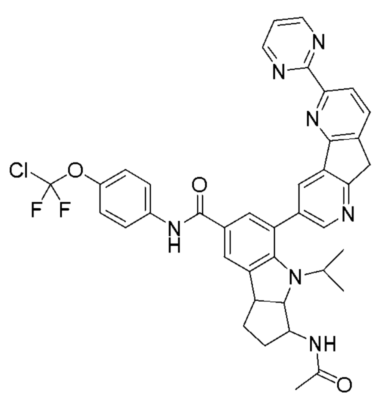
,

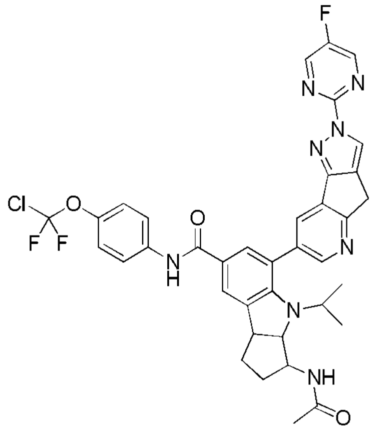,
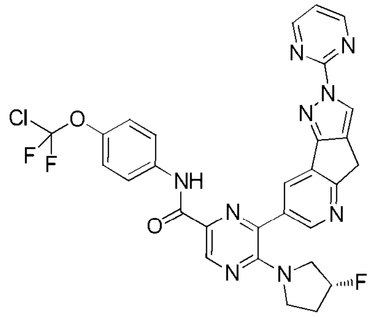,
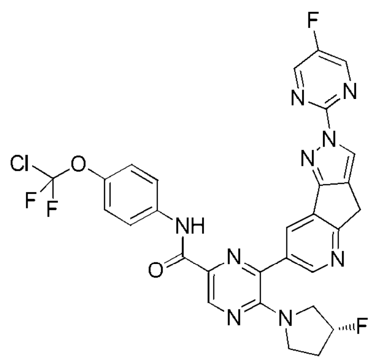,

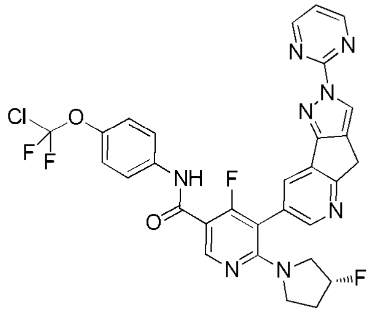,
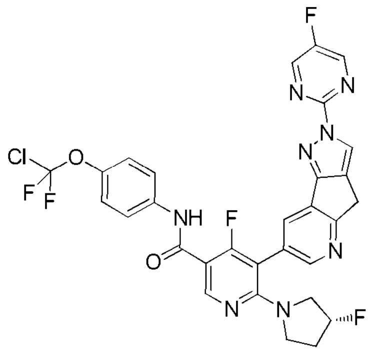,
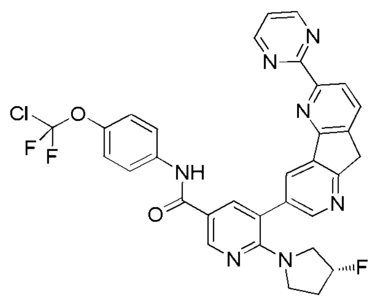,

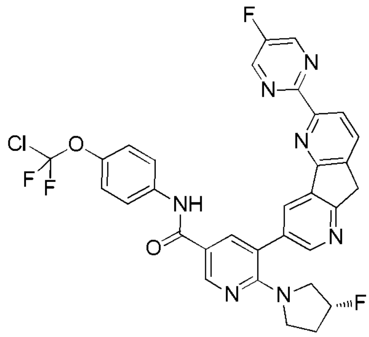,
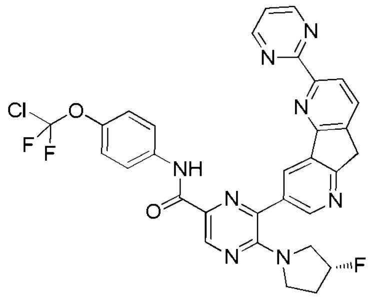,
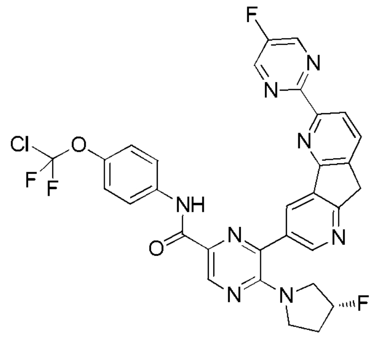,

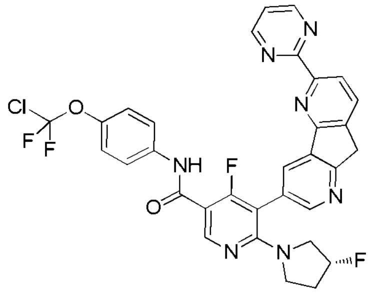,
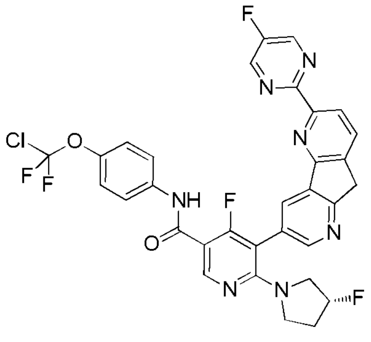,
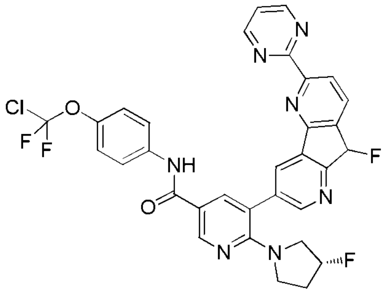,

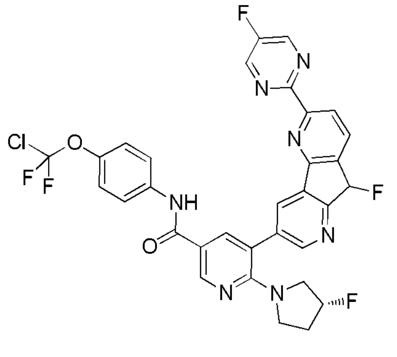

,

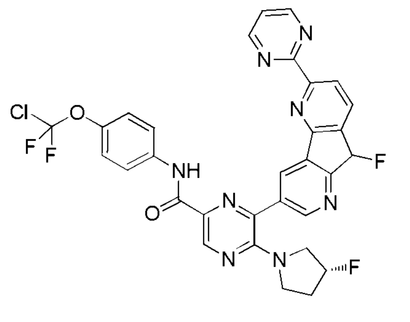

,

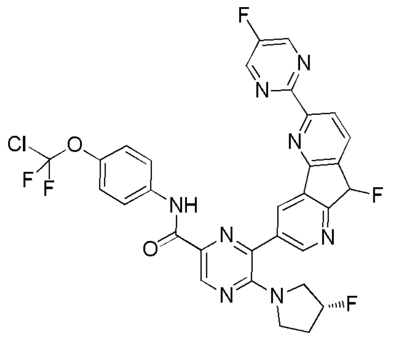

,

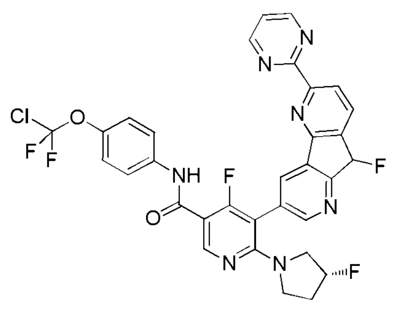

,

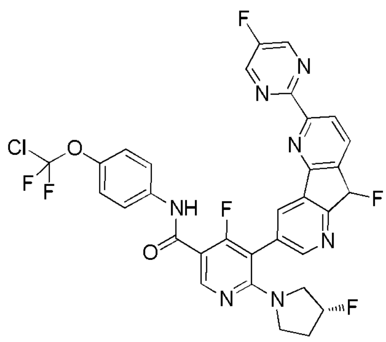

,

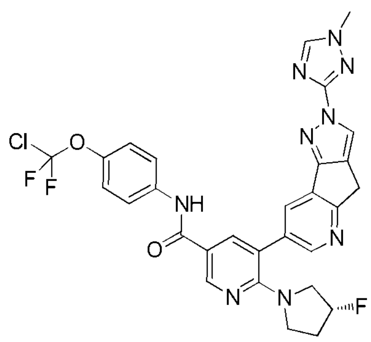

,

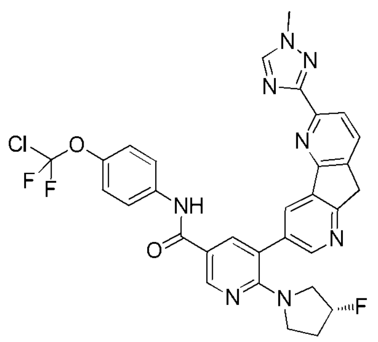

,

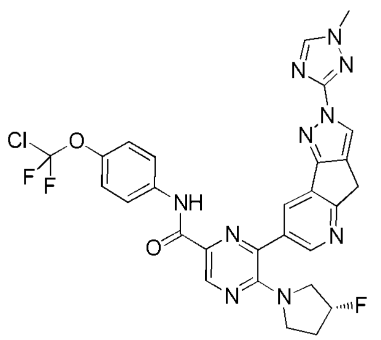

,

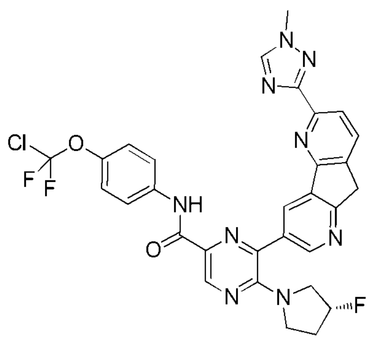

,

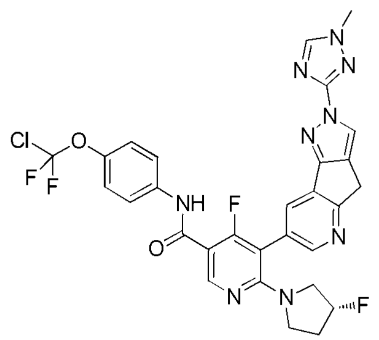

,

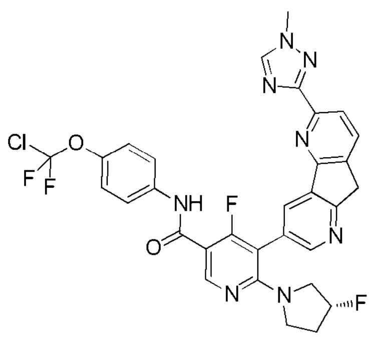,
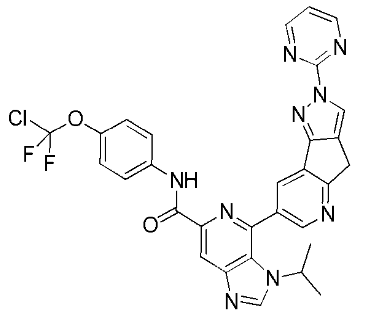,
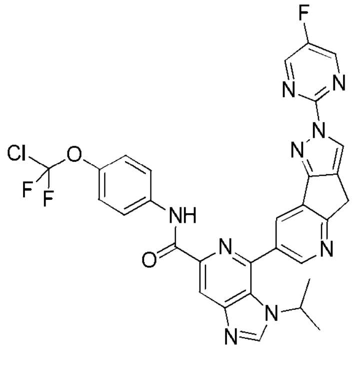,

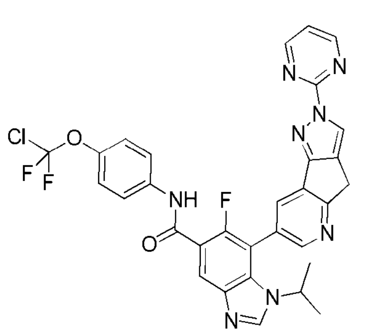,
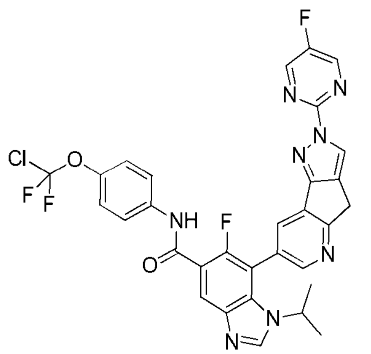,
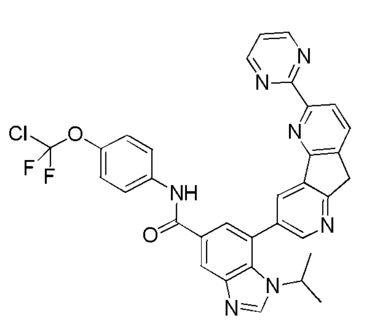,

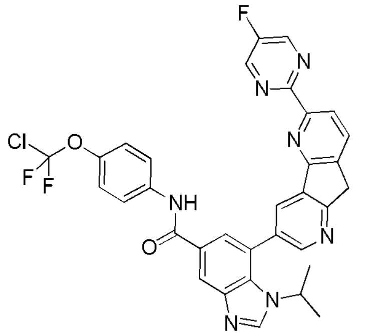,
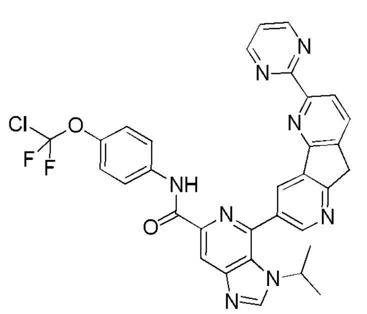,
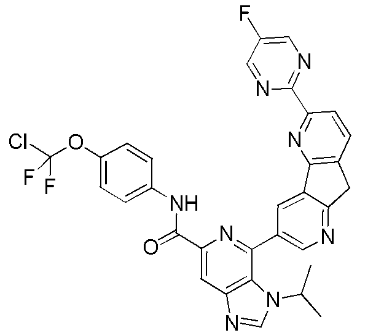,

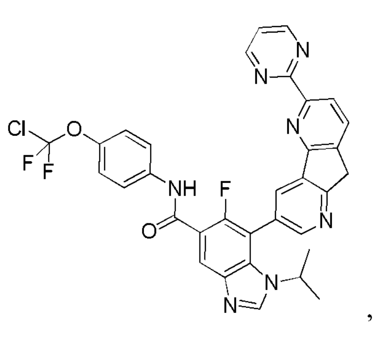,
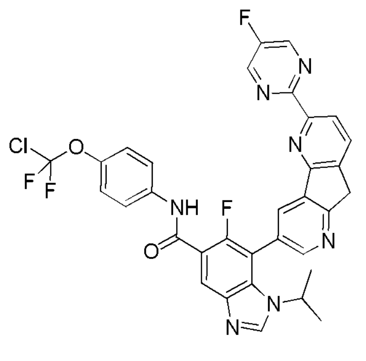,
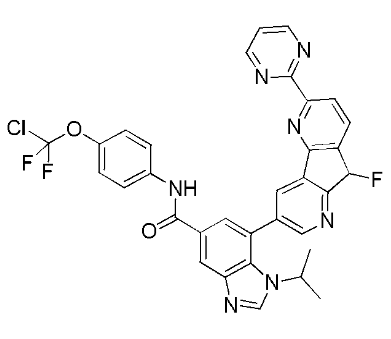,

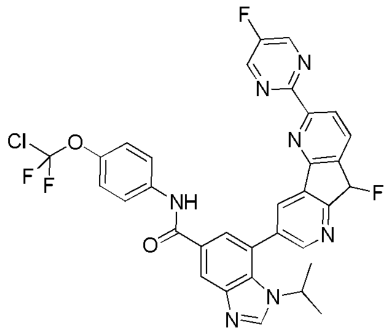 ,

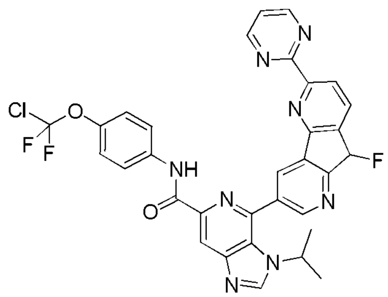 ,

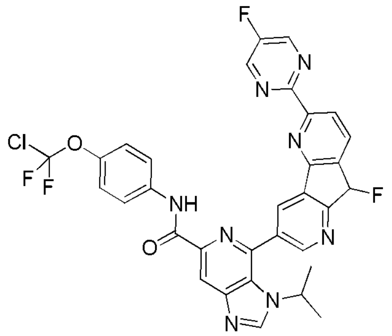 ,

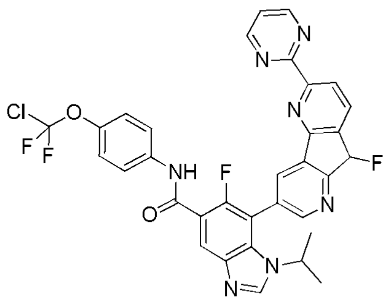 ,

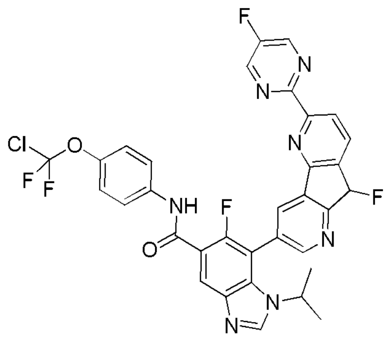 ,

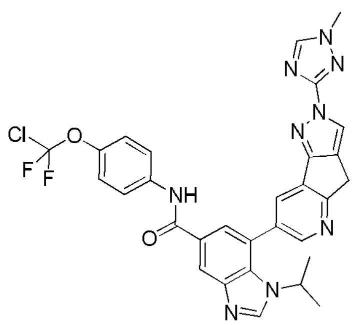 ,

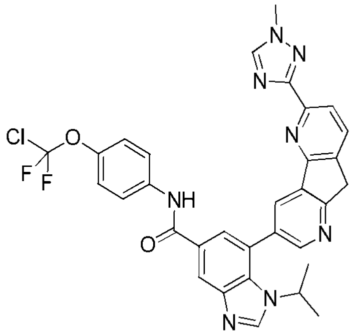 ,

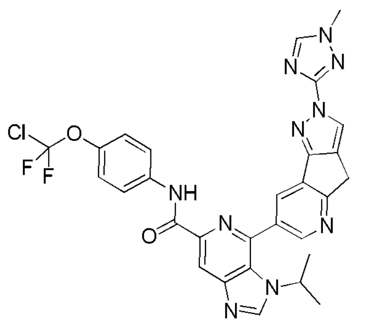 ,

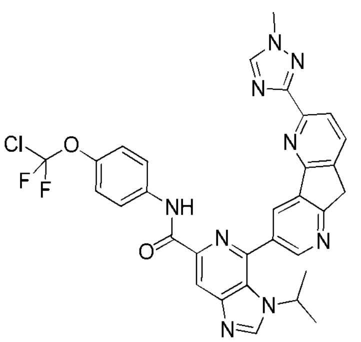 ,

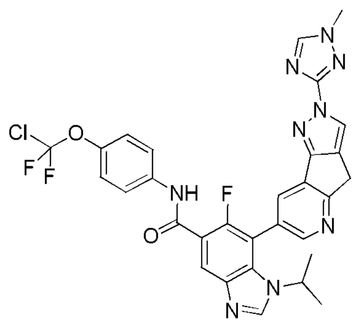 ,

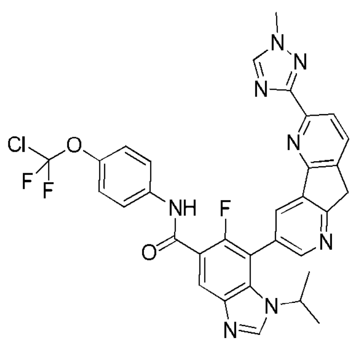,
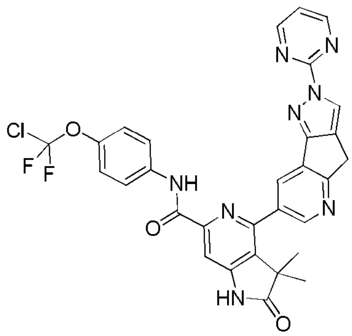,
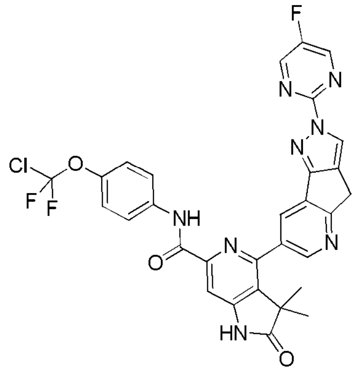,

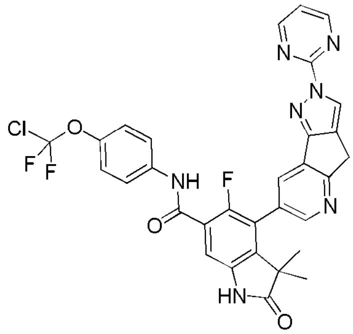,
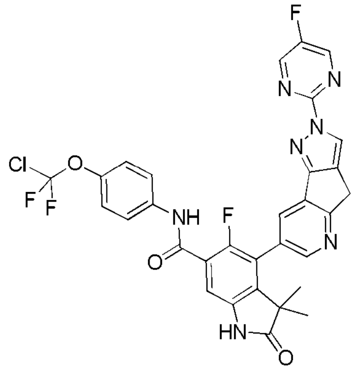,
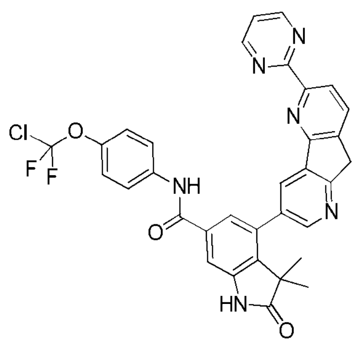,

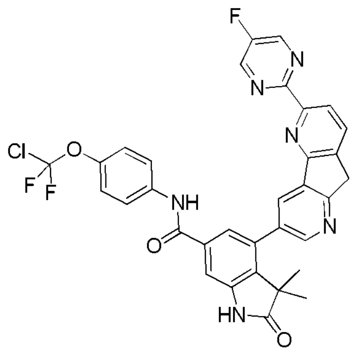,
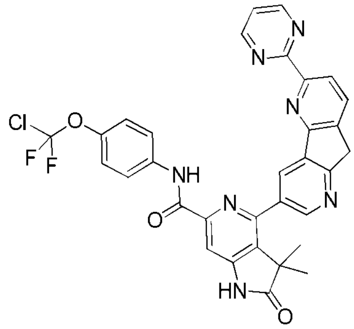,
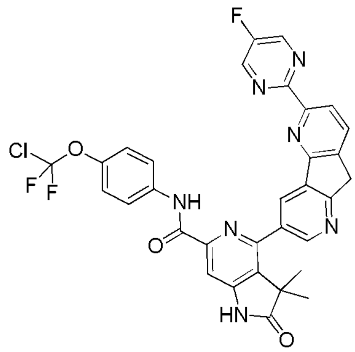,

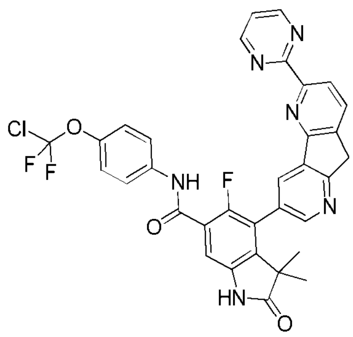,
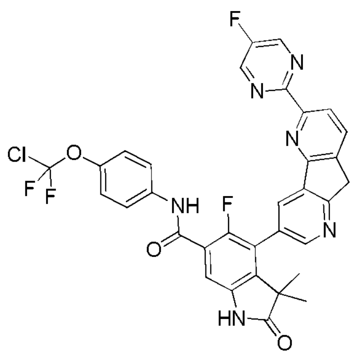,
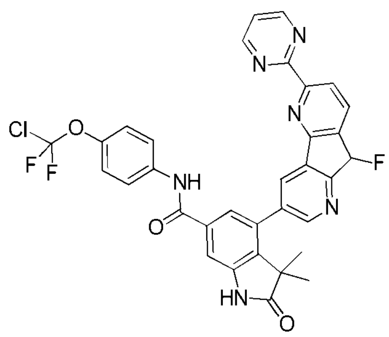,

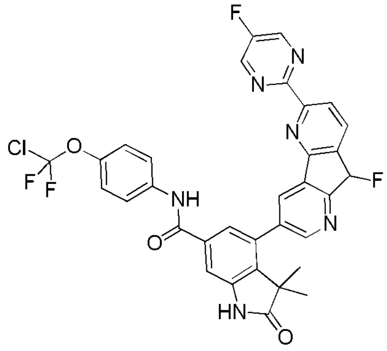
,
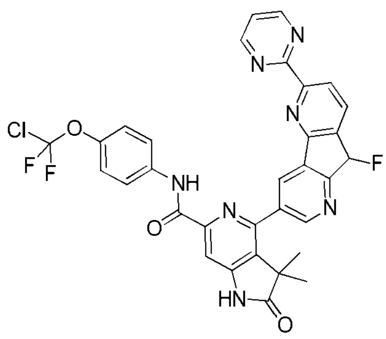
,
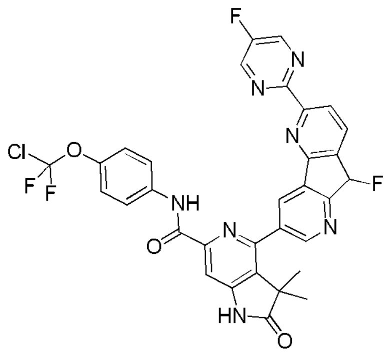
,
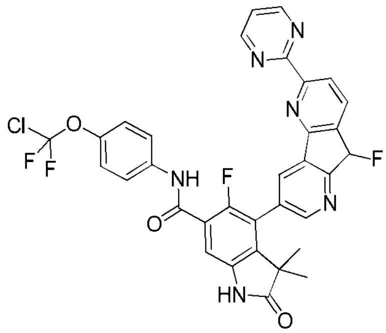
,
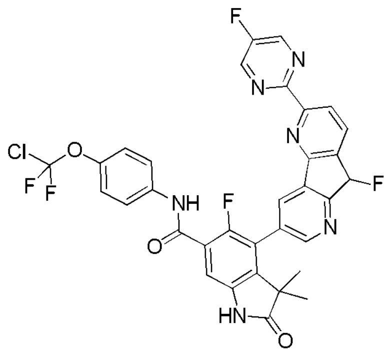
,
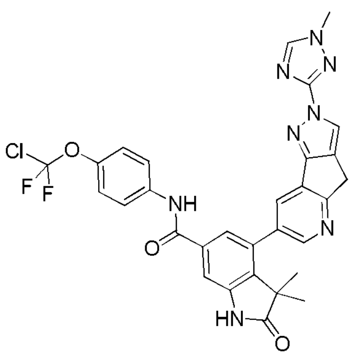
,
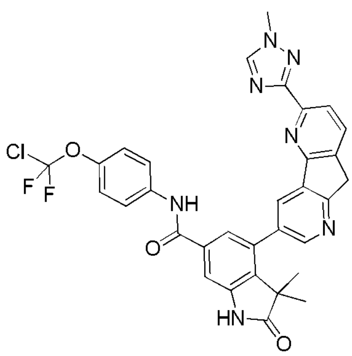
,
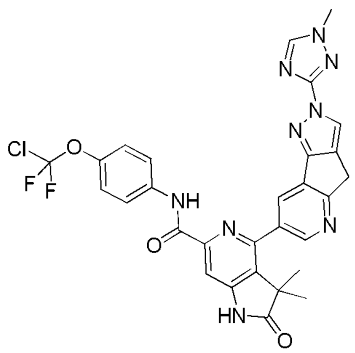
,
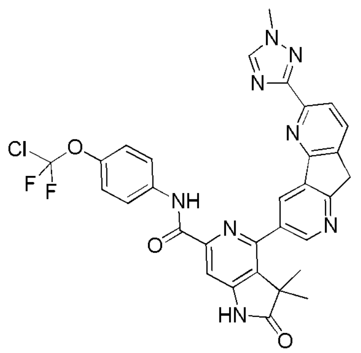
,
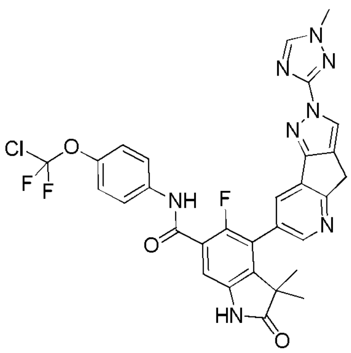
,

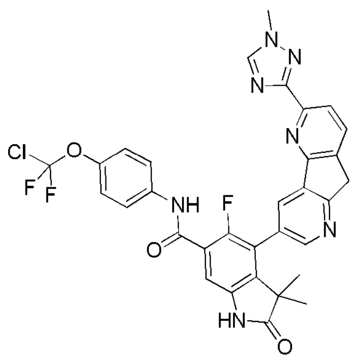

,

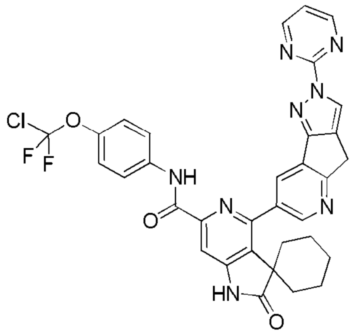

,

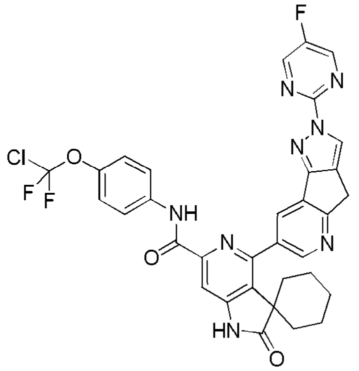

,

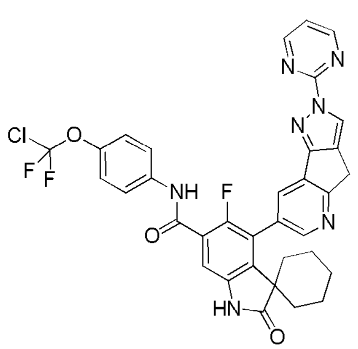

,

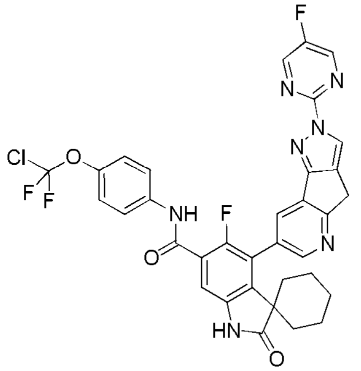

,

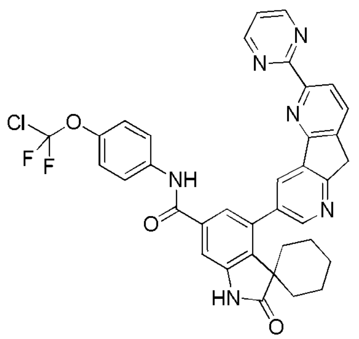

,

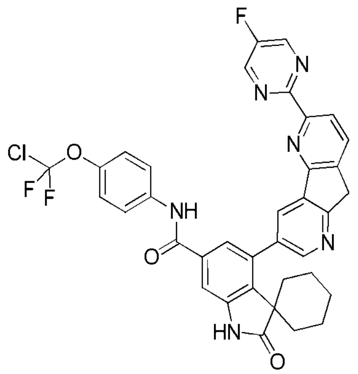

,

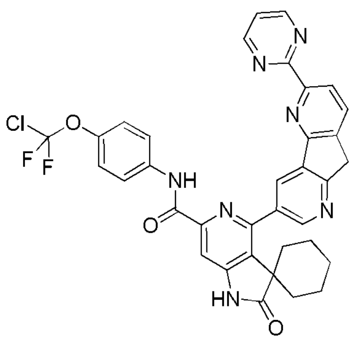

,

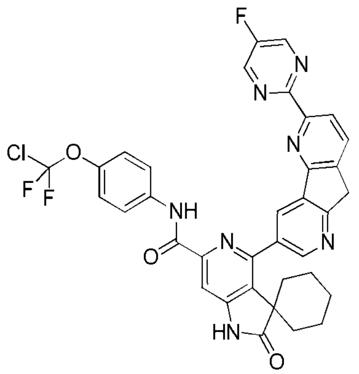

,

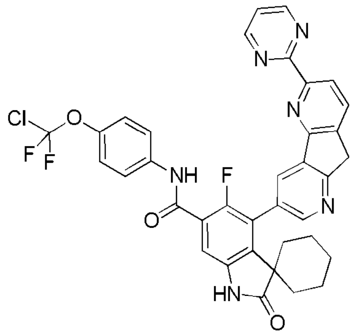

,

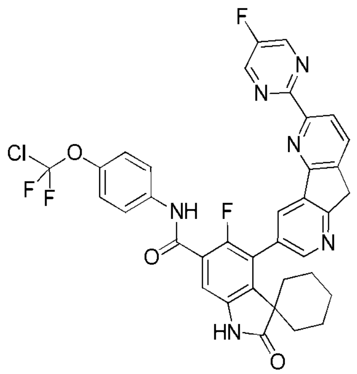

,

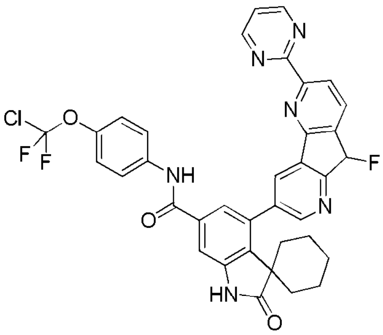

,

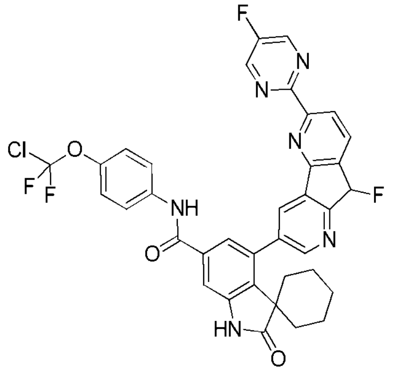,

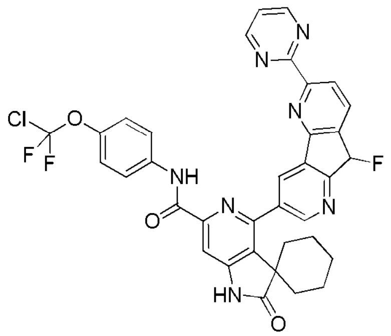,

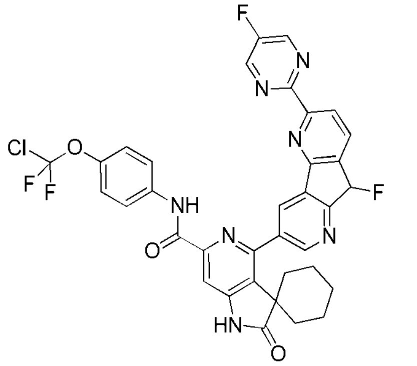,

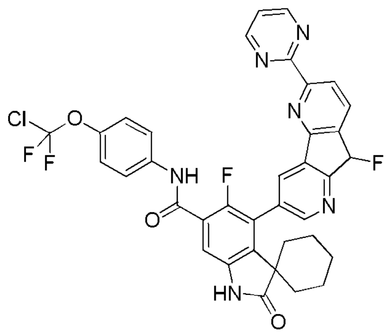,

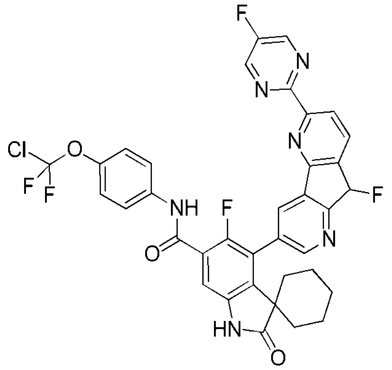,

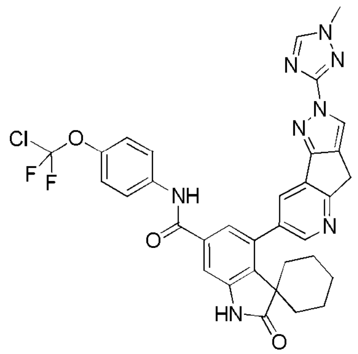,

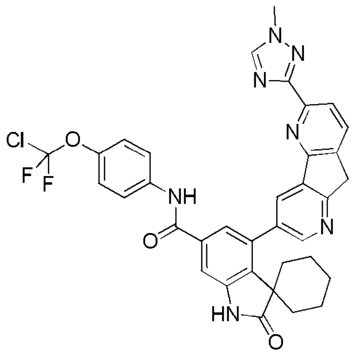,

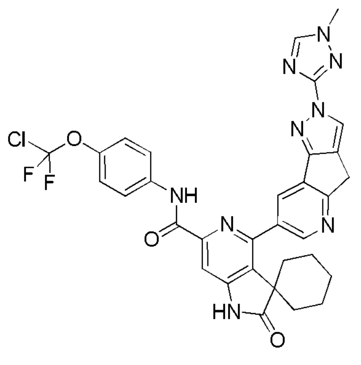,

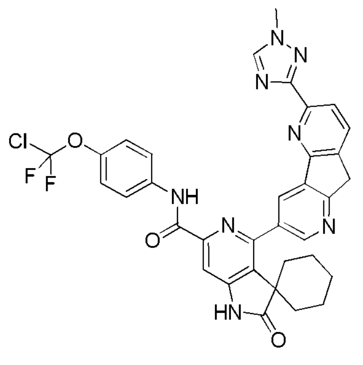,

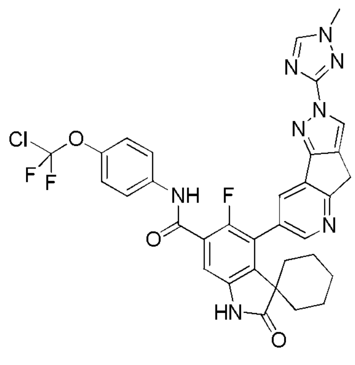,

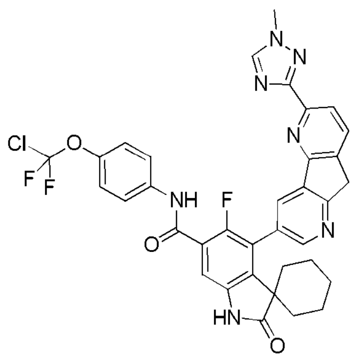,
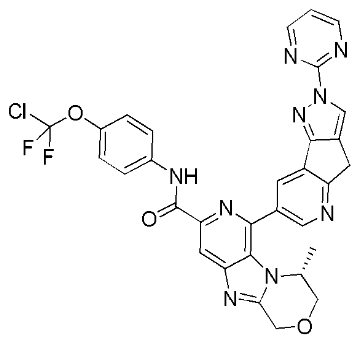,
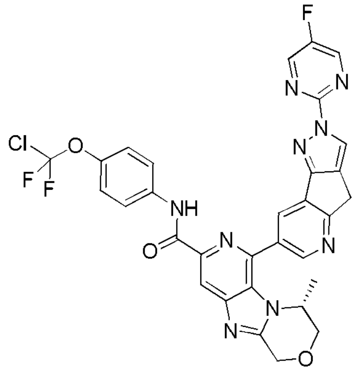,

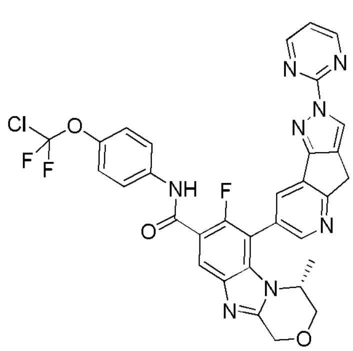,
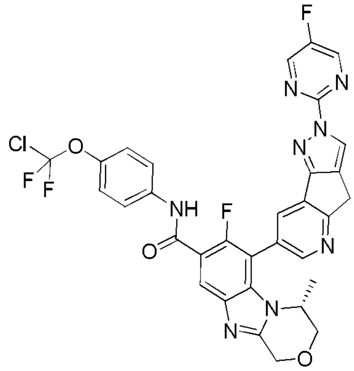,
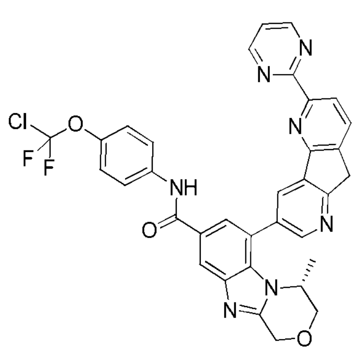,

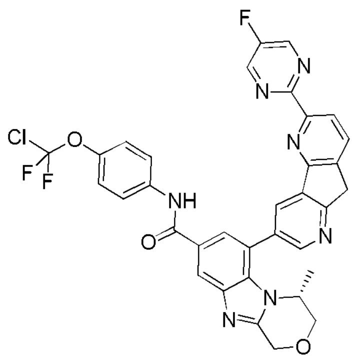,
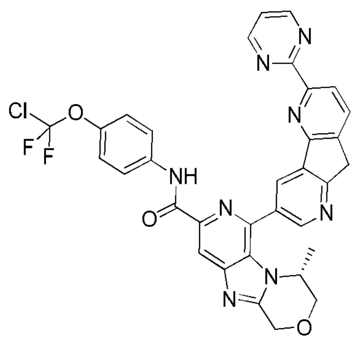,
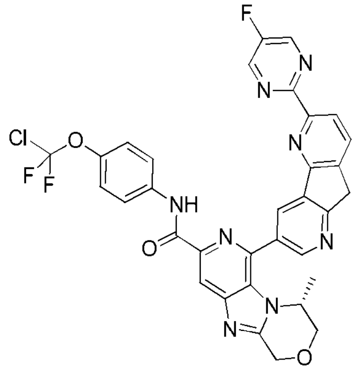,

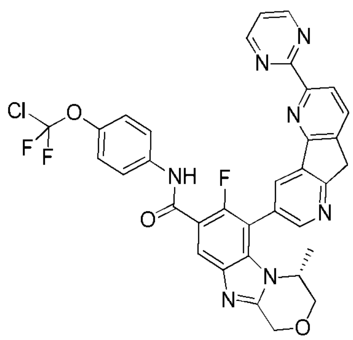,
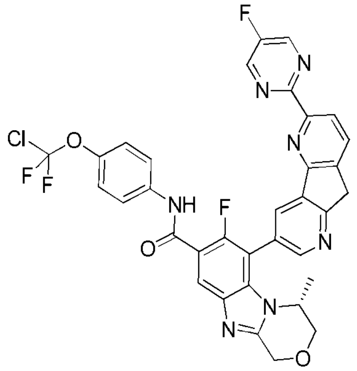,
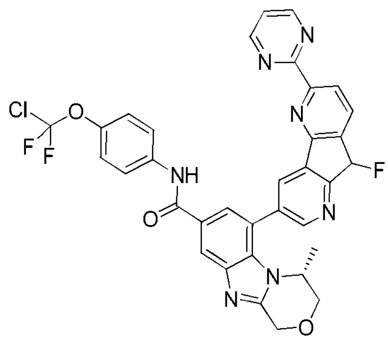,

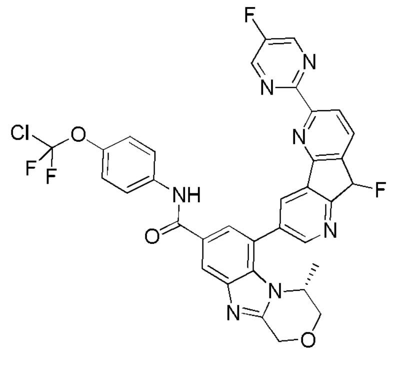,

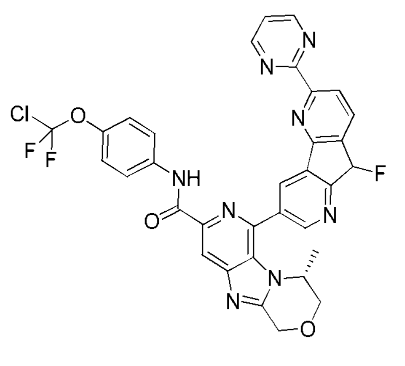,

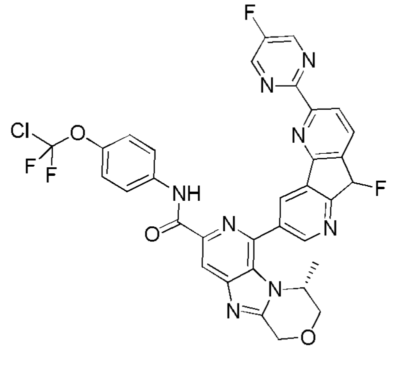,

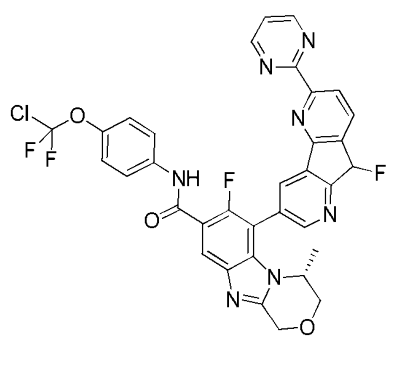,

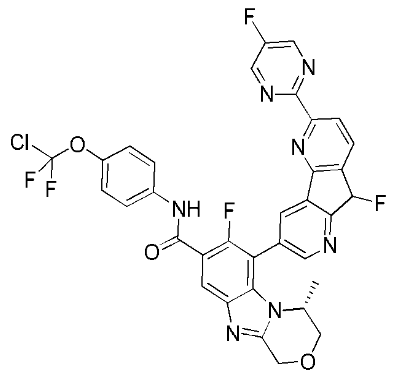,

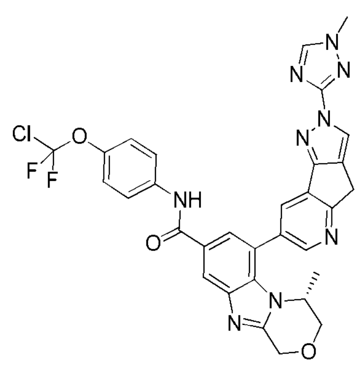,

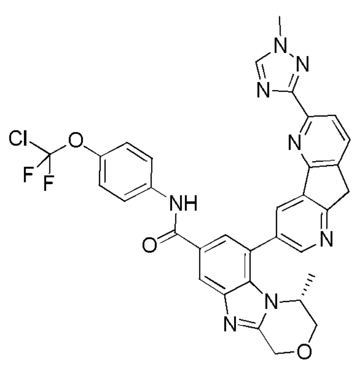,

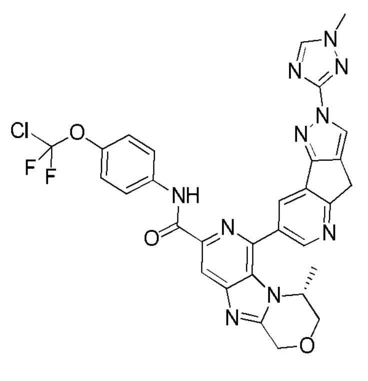,

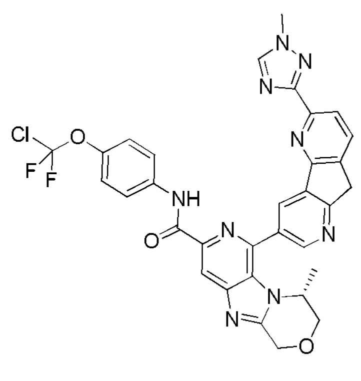,

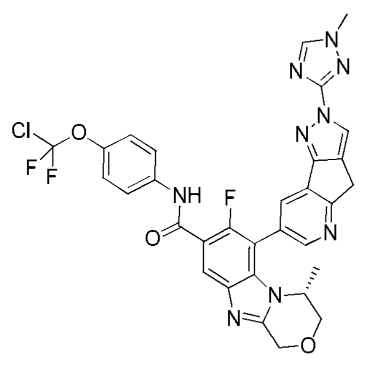,

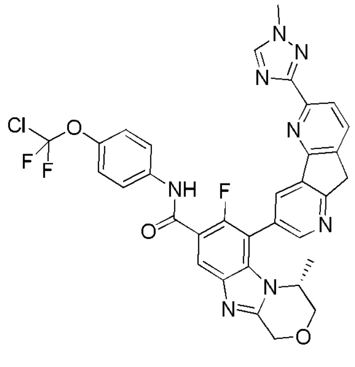,

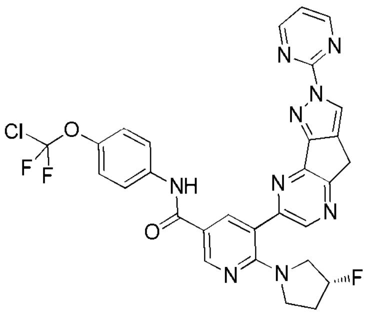,

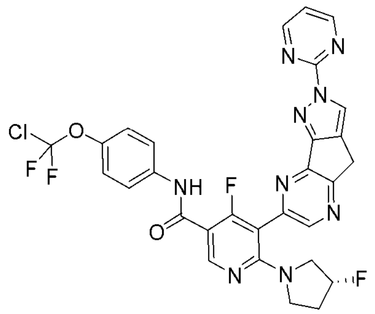,

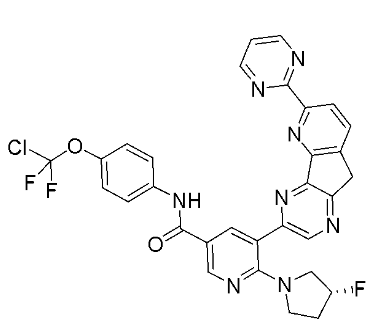,

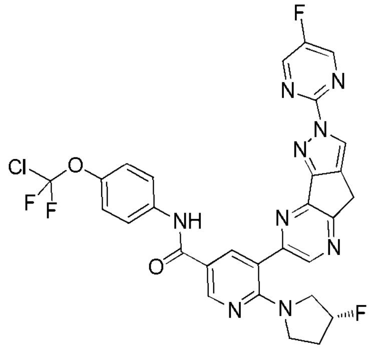,

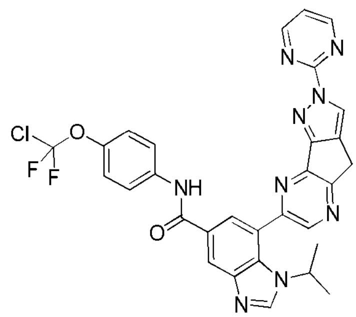,

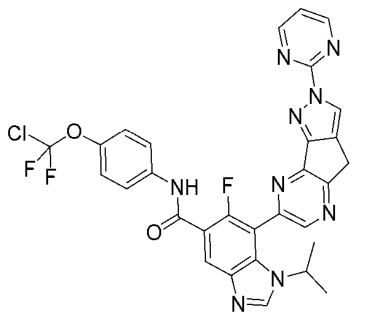,

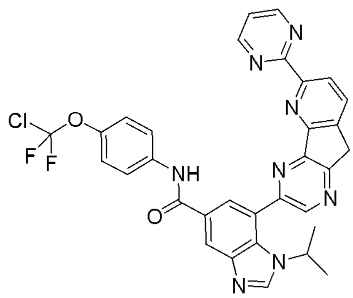

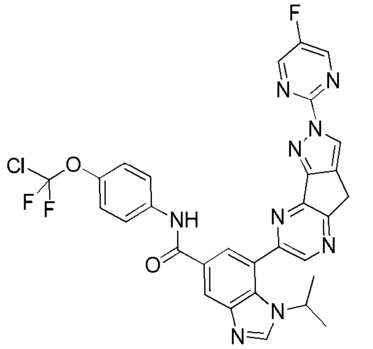,

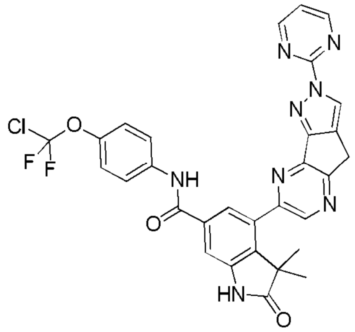,

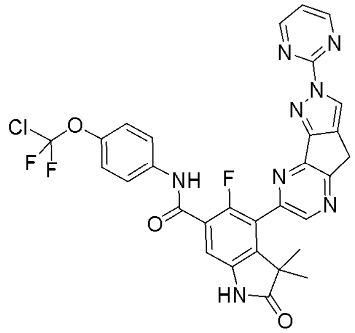,

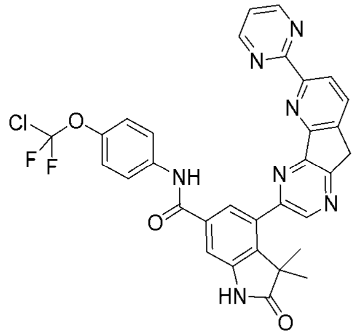,

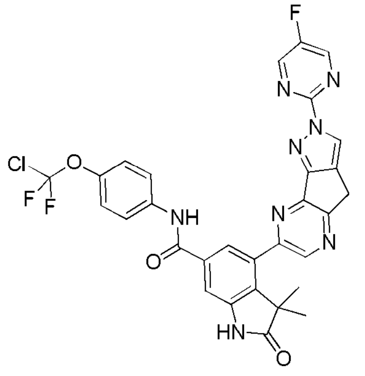 , 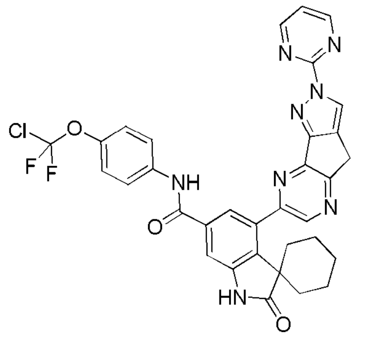 , 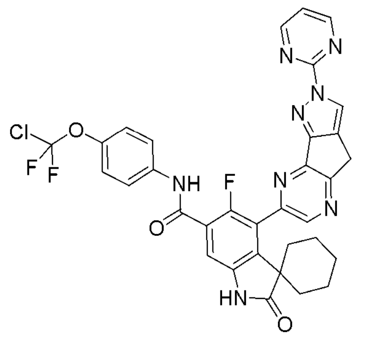 ,

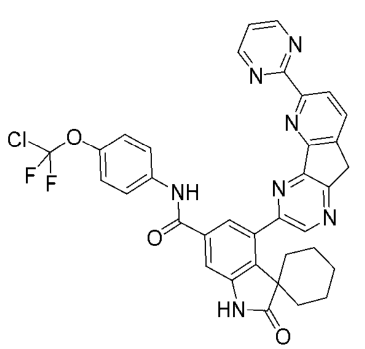 , 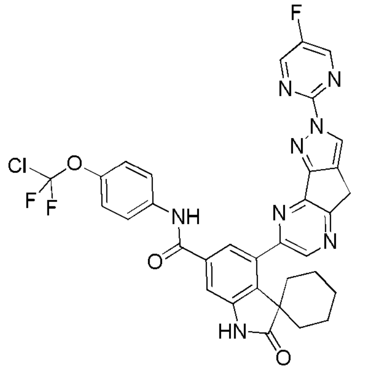 , 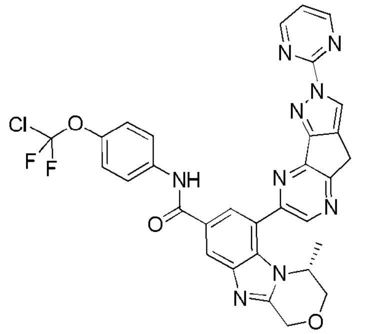 ,

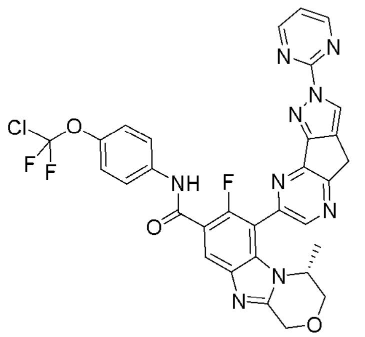 , 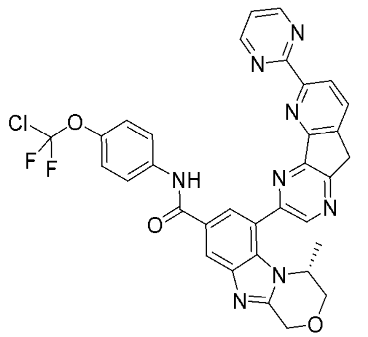 , 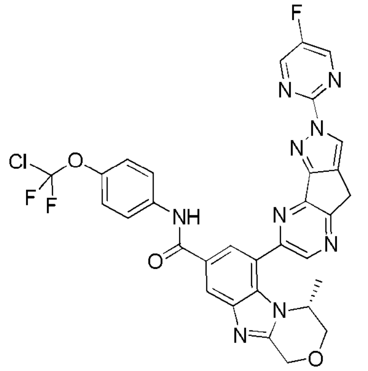 ,

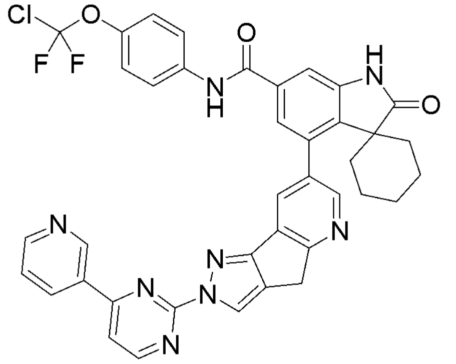 , 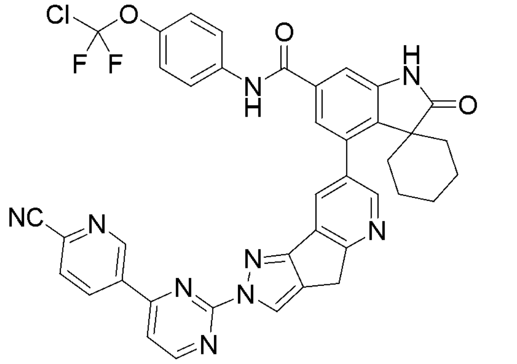 ,

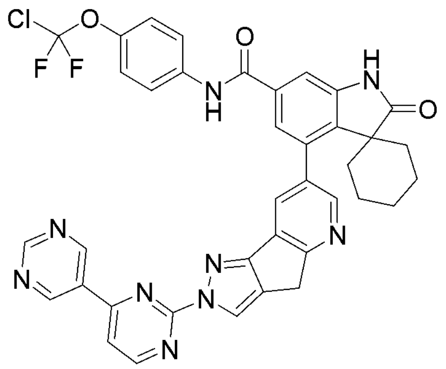
,

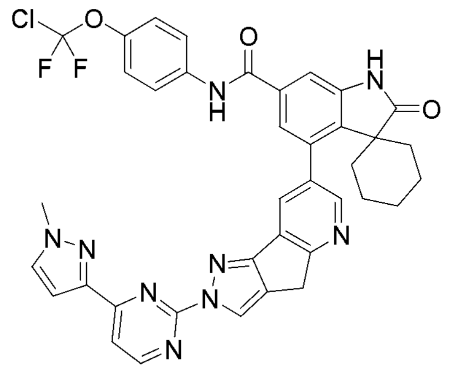
,

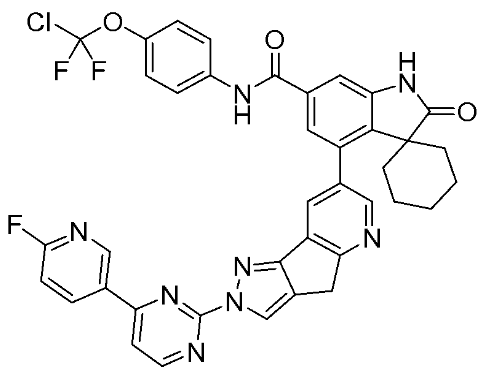
,

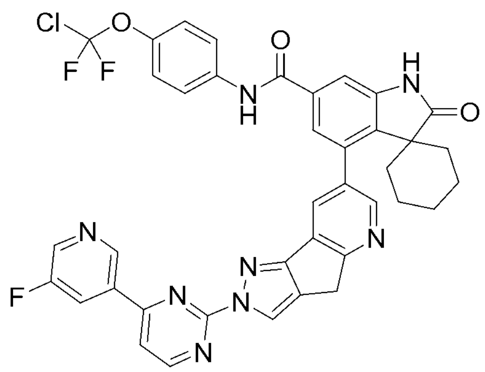
,

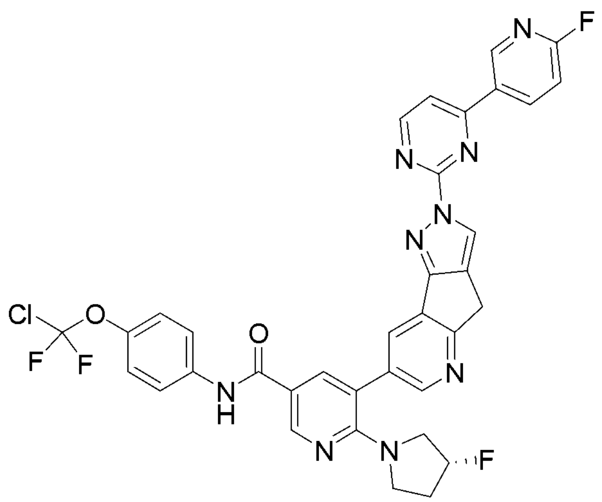

and

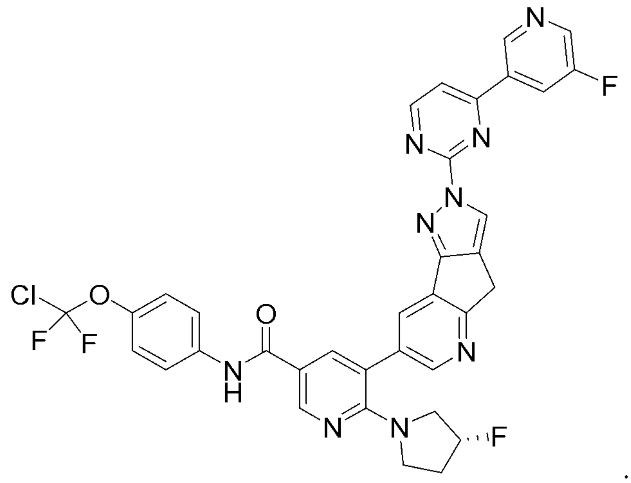

.

107. A pharmaceutical composition, comprising the compound according to any one of technical solutions 1 to 106, or a tautomer, a stereoisomer, a prodrug, a crystal form, a pharmaceutically acceptable salt, a hydrate or a solvate thereof, and a pharmaceutically acceptable excipient, and optionally, other therapeutic agent(s).
108. A unit dosage form, comprising the pharmaceutical composition according to technical solution 107.
109. Use of the compound according to any one of technical solutions 1 to 106, or a tautomer, a stereoisomer, a prodrug, a crystal form, a pharmaceutically acceptable salt, a hydrate or a solvate thereof, or the pharmaceutical composition according to technical solution 107, or the unit dosage form according to technical solution 108, in the manufacture of a medicament for the treatment and/or prevention of a wild-type and/or mutated Ber-Abl1 kinase-mediated disease;

alternatively, wherein the mutation of the mutated Ber-Abl1 kinase is selected from: T3151, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V and A424T;
alternatively, wherein the mutation of the mutated Ber-Abl1 kinase is selected from: T315I and T315M;
alternatively, wherein the mutation of the mutated Ber-Abl1 kinase is T3151;
alternatively, wherein the mutation of the mutated Ber-Abl1 kinase is selected from: P465S, V468F, I502L, A337V and A424T.

110. A method of treating and/or preventing a wild-type and/or mutated Ber-Abl1 kinase-mediated disease in a subject, comprising administering to the subject the compound according to any one of technical solutions 1 to 106 or a pharmaceutically acceptable salt, a stereoisomer, a solvate, a hydrate, a polymorph, a prodrug or an isotopic variant thereof, or the pharmaceutical composition according to technical solution 107, or the unit dosage form according to technical solution 108;

alternatively, wherein the mutation of the mutated Ber-Abl1 kinase is selected from: T3151, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V and A424T;
alternatively, wherein the mutation of the mutated Ber-Abl1 kinase is selected from: T315I and T315M;
alternatively, wherein the mutation of the mutated Ber-Abl1 kinase is T3151;
alternatively, wherein the mutation of the mutated Ber-Abl1 kinase is selected from: P465S, V468F, I502L, A337V and A424T.

111. The compound according to any one of technical solutions 1 to 106, or a tautomer, a stereoisomer, a prodrug, a crystal form, a pharmaceutically acceptable salt, a hydrate or a solvate thereof, or the pharmaceutical composition according to technical solution 107, or the unit dosage form according to technical solution 108, for use in the treatment and/or prevention of a wild-type and/or mutated Ber-Abl1 kinase-mediated disease;

alternatively, wherein the mutation of the mutated Ber-Abl1 kinase is selected from: T3151, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V and A424T;
alternatively, wherein the mutation of the mutated Ber-Abl1 kinase is selected from: T315I and T315M;
alternatively, wherein the mutation of the mutated Ber-Abl1 kinase is T3151;

alternatively, wherein the mutation of the mutated Ber-Abl1 kinase is selected from: P465S, V468F, I502L, A337V and A424T.

112. The use according to technical solution 108 or the method according to technical solution 109 or the compound or composition for use according to technical solution 110, wherein the wild-type and/or mutated Bcr-Abl1 kinase-mediated disease is selected from: leukemia, non-Hodgkin's lymphoma, Hodgkin's lymphoma, myeloma, neurodegenerative diseases, solid tumors, immune diseases and fibrosis.

113. The use according to technical solution 108 or the method according to technical solution 109 or the compound or composition for use according to technical solution 110, wherein the wild-type and/or mutated Ber-Abl1 kinase-mediated disease is selected from acute lymphoblastic leukemia, acute myeloid leukemia, acute promyelocytic leukemia, chronic lymphocytic lymphoma, chronic myeloid leukemia, chronic myelomonocytic leukemia, chronic neutrophilic leukemia, acute undifferentiated leukemia, anaplastic large cell lymphoma, prolymphocytic leukemia, juvenile myelomonocytic leukemia, adult T-cell leukemia, acute myeloid leukemia with trilineage myelodysplasia, mixed lineage leukemia, myelodysplastic syndrome, amyotrophic lateral sclerosis, Parkinson's disease and Alzheimer's disease; alternatively, the wild-type and/or mutated Ber-Abl1 kinase-mediated disease is selected from acute lymphoblastic leukemia and chronic myeloid leukemia.

114. A method of preparing the compound according to any one of technical solutions 1 to 106.

115. An intermediate compound or a salt thereof, wherein the intermediate compound is one of the following compounds:

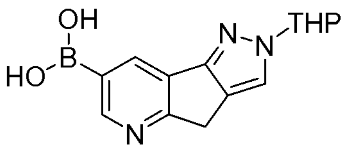 , 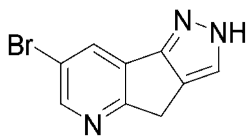 , 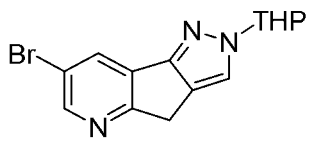 ,

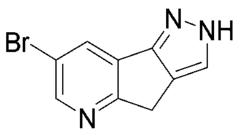 , 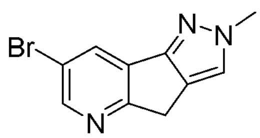 , 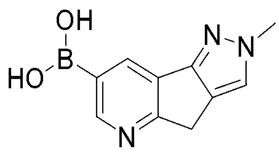 , 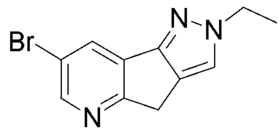 ,

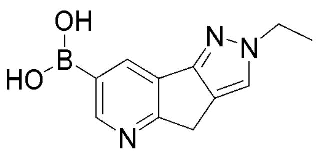 , 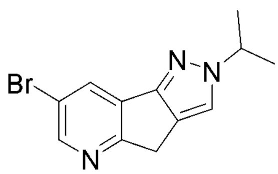 , 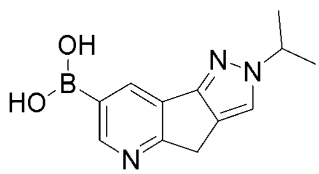 ,

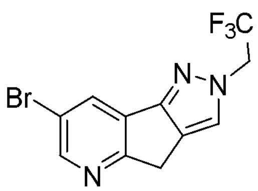 , 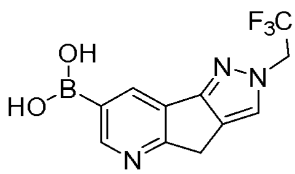 , 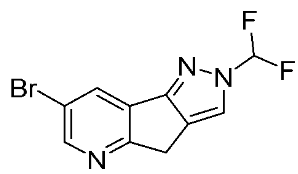 ,

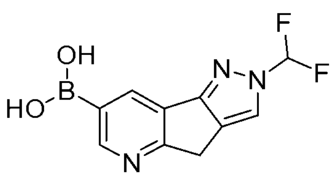 , 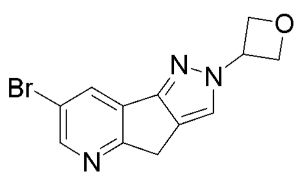 , 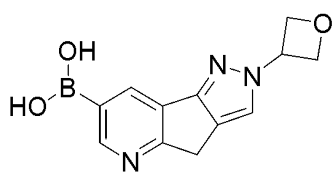 ,

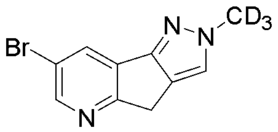,
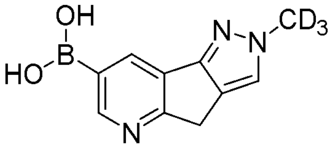,
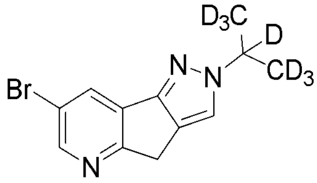,
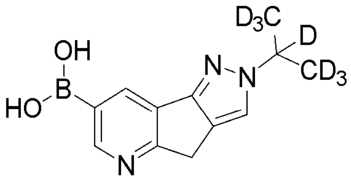,
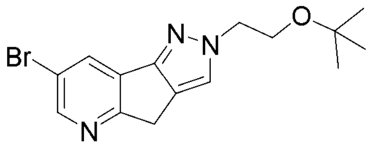,
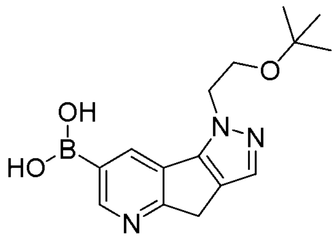,
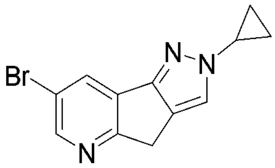,
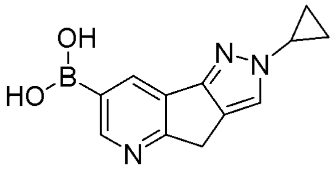,
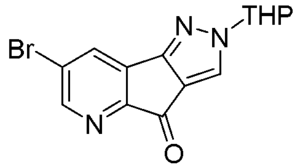,
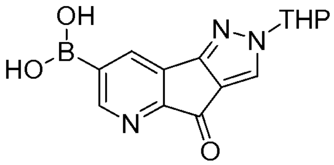,
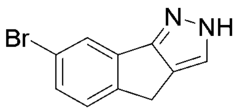,
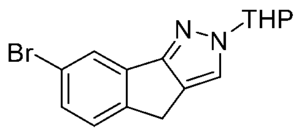,
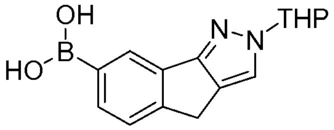,
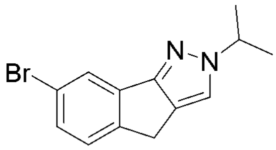,
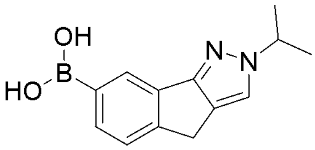,
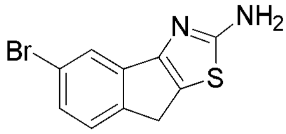 or 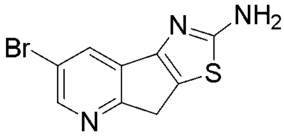.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

"Tautomer" refers to an isomer in which one functional group in a compound changes its structure into another functional group, wherein the compound and the isomer can quickly convert between each other, thus being in dynamic equilibrium; this two isomers are called tautomers.

It will be understood by those skilled in the art that the organic compounds can form complexes with solvents in which they are reacted or from which they are precipitated or crystallized. These complexes are known as "solvates." When the solvent is water, the complex is known as "hydrate." The present disclosure encompasses all solvates of the compounds of the present disclosure.

The term "solvate" refers to forms of a compound or a salt thereof, which are associated with a solvent, usually by

a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula $R \cdot x\ H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates ($R \cdot 0.5\ H_2O$)) and polyhydrates (x is a number greater than 1, for example, dihydrates ($R \cdot 2\ H_2O$) and hexahydrates ($R \cdot 6\ H_2O$)).

Compounds of the present disclosure may be in an amorphous or a crystalline form (crystal form or polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes, which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as $^2H$, $^3H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., $^3H$ and $^{14}C$), can be used for the measurement of the distribution of drug and/or substrate in tissue. Tritium, which is $^3H$ and carbon-14, which is $^{14}C$ isotope, are yet alternative, because they are easy to prepare and detect. Furthermore, replaced by heavier isotopes, such as deuterium, which is $^2H$, may provide therapeutic benefits due to the higher metabolic stability, such as prolonging the half-life in vivo or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects in vivo by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which is incorporated herein by reference.

The prodrugs are any covalently bonded compounds of the present disclosure, which release the parent compound in vivo when the prodrug is administered to a patient. Prodrugs are typically prepared by modifying functional groups in such a way that the modifications can be cleaved either by routine manipulation or decompose in vivo to yield the parent compound. Prodrugs include, for example, compounds of the present disclosure wherein the hydroxyl, amino or sulfhydryl groups are bonded to any group that, when administered to a patient, cleaves to form the hydroxyl, amino or sulfhydryl groups. Thus, representative examples of prodrugs include (but are not limited to) the acetate/acetamide, formate/formamide and benzoate/benzamide derivatives of the hydroxyl, sulfhydryl or amino functional groups of the compounds of formula (I). Furthermore, in the case of carboxylic acid (-COOH), esters such as methyl esters and ethyl esters, etc. can be employed. The ester itself may be active in their own and/or hydrolyzable under in vivo conditions in the human body. Suitable pharmaceutically acceptable in vivo hydrolysable ester groups include those groups that can readily break down in the human body to release the parent acids or salts thereof.

Pharmaceutical compositions, formulations and kits

In another aspect, the present disclosure provides a pharmaceutical composition comprising a compound disclosed herein (also referred to as the "active ingredient") and a pharmaceutically acceptable excipient. In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other suitable containers) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

The pharmaceutical composition provided by the present disclosure can be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, for example, over 3 months, 6 months, 1 year, 2 years, 3 years, 5 years, etc, or may be continued indefinitely, for example, for the rest of the subject's life. In certain embodiments, the chronic administration is intended to provide a constant level of the compound in the blood, e.g., within the therapeutic window over the extended period of time.

The pharmaceutical compositions of the present disclosure may be further delivered using a variety of dosing methods. For example, in certain embodiments, the pharmaceutical composition may be given as a bolus, e.g., in order to raise the concentration of the compound in the blood to an effective level rapidly. The placement of the bolus dose depends on the desired systemic levels of the active ingredient, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other

mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with alternative doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and yet alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavours and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavouring agent such as peppermint, methyl salicylate, or orange flavouring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or formulation. All such known transdermal formulations and ingredients are included within the scope provided herein.

The compounds provided herein can also be administered by a transdermal device. Accordingly, transdermal administration may be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

The compounds disclosed herein can also be administered in sustained release forms or from sustained release drug delivery systems. A description of representative sustained release materials may be found in Remington's Pharmaceutical Sciences.

The present disclosure also relates to the pharmaceutically acceptable formulations of a compound disclosed herein. In one embodiment, the formulation comprises water. In another embodiment, the formulation comprises a cyclodextrin derivative. The most common cyclodextrins are α-, β- and γ- cyclodextrins consisting of 6, 7 and 8 α-1,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, e.g., for example, sulfobutyl ether β-cyclodextrin, also known as Captisol. See, e.g., U.S. 5,376,645. In certain embodiments, the formulation comprises hexapropyl-β-cyclodextrin (e.g., 10-50% in water).

Indications

In another aspect, provided is the use of the compound of formula (I) disclosed herein (including all individual embodiments and generic subsets disclosed herein) or a tautomer, stereoisomer, prodrug, crystal form, pharmaceutically acceptable salt, hydrate or solvate thereof as a drug.

In one embodiment, the compound of the present disclosure can be used in the treatment and/or prevention of a wild-type Ber-Abl1 kinase-mediated disease.

In another embodiment, the compound of the present disclosure is a Ber-Abl1 allosteric inhibitor targeting the myristoyl pocket (type IV pocket, far from the ATP site), which can solve the drug resistance problem caused by type I and type II Ber-Abl1 inhibitors that bind to the ATP site. The main causes of the drug resistance of type I and type II Ber-Abl1 inhibitors include Bcr-Abl1 point mutations, gene amplification, overexpression of ABC transporters, or Bcr-Abl1-independent mechanisms. The development of drug resistance of a drug leads to disease relapse in a patient, and mutations in the Ber-Abl1 kinase domain are considered the most significant factor. Point mutations in the abl1 kinase domain have been found in 30% to 90% of patients who develop drug resistance.

In another embodiment, the compound of the present disclosure can be used in the treatment and/or prevention of a mutated Ber-Abl1 kinase-mediated disease. In one specific embodiment, the mutation of the mutated Ber-Abl1 kinase may be located at the ATP binding site. In a more specific embodiment, the mutation at the ATP binding site is selected from T315I, T315M, F317L, E355G and V299L. In a more specific embodiment, the mutation at the ATP binding site is T315I. In a more specific embodiment, the mutation at the ATP binding site is T315M. In another specific embodiment, the mutation of the mutated Ber-Abl1 kinase may be located at the P-loop. In a more specific embodiment, the mutation located at the P-loop is selected from M244V, K247R, L248V, G250E, G250R, Q252H, Q252R, Y253F, Y253H, E255K and E255V. In a more specific embodiment, the mutation located at the P-loop is selected from M244V, G250E, Q252H, Y253H, E255K and E255V. In another specific embodiment, the mutation of the mutated Ber-Abl1 kinase may be located at the A-loop. In a more specific embodiment, the mutation located at the A-loop is selected from V379I, A380T, F382L, L384M, L387M, L387F, L387V, M388L, Y393C, H396P, H396R, H396A and A397P. In a more specific embodiment, the mutation located at the A-loop is selected from H396P, H396R, H396A and A397P. In another specific embodiment, the mutation of the mutated Ber-Abl1 kinase may be located at the SH2 domain interface or the SH3 domain interface. In a more specific embodiment, the mutation located at the SH2 domain interface or the SH3 domain interface is selected from P223S, K294E, M351T and F359V. In another specific embodiment, the mutation of the mutated Ber-Abl1 kinase is selected from T3151, Y253F, Y253H, E255K, E255V, M351T, G250E, F359C, F359V, H396P, H396R, M244V, E355G, F317L, M237I, Q252H, Q252R, D276G, L248V and F486S.

In another embodiment, the compound of the present disclosure can solve the problem of drug resistance caused by asciminib.

In another embodiment, the compound of the present disclosure can be used in the treatment and/or prevention of a mutated Ber-Abl1 kinase-mediated disease. In one specific embodiment, the mutation of the mutated Ber-Abl1 kinase may be located at the myristoyl binding site. In a more specific embodiment, the mutation at the myristoyl binding site is selected from E459K, P465S, V468F, 1502L, C464W, A337V and A424T. In a more specific embodiment, the mutation at the myristoyl binding site is selected from E459K, P465S, V468F, I502L, A337V and A424T.

In another embodiment, the wild-type and/or mutated Ber-Abl1 kinase-mediated disease is selected from leukemia, non-Hodgkin's lymphoma, Hodgkin's lymphoma, myeloma, neurodegenerative diseases, solid tumors, immune diseases and fibrosis.

In one specific embodiment, the wild-type and/or mutated Ber-Abl1 kinase-mediated disease is selected from leukemia, non-Hodgkin's lymphoma, Hodgkin's lymphoma and myeloma, for example, acute lymphocytic leukemia (ALL), acute myeloid leukemia (AML), acute promyelocytic leukemia (APL), chronic lymphocytic lymphoma (CLL), chronic myeloid leukemia (CML), chronic myelomonocytic leukemia (CMML), chronic neutrophilic leukemia (CNL), acute undifferentiated leukemia (AUL), anaplastic large cell lymphoma (ALCL), prolymphocytic leukemia (PML), juvenile myelomonocytic leukemia (JMML), adult T-cell leukemia (ALL), acute myeloid leukemia with trilineage myelodysplasia (AML/TMDS), mixed lineage leukemia (MLL), myelodysplastic syndrome (MDS), myeloproliferative disorder (MPD), diffuse large B-cell lymphoma, follicular lymphoma, mantle cell lymphoma, marginal zone lymphoma (e.g., splenic marginal zone lymphoma, extranodal marginal zone B-cell lymphoma), Burkitt's lymphoma, Waldenstrom's macroglobulinemia (lymphoplasmacytic lymphoma), primary central nervous system lymphoma, small lymphocytic lymphoma, precursor B-cell lymphoblastic leukemia, hairy cell leukemia, mucosa-associated lymphoid tissue lymphoma, plasma cell myeloma, plasmacytoma, and multiple myeloma. Other examples of hematological cancers include myelodysplastic disorders (MPD), such as polycythemia vera (PV), essential thrombocytopenia (ET), and idiopathic primary myelofibrosis (IMF/IPF/PMF). In another specific embodiment, the wild-type and mutated Ber-Abl1 kinase-mediated disease is selected from acute myeloid leukemia (AML) and chronic myeloid leukemia (CML).

In another specific embodiment, the wild-type and/or mutated Ber-Abl1 kinase-mediated disease is selected from neurodegenerative diseases, such as amyotrophic lateral sclerosis (ALS), Parkinson's disease (PD), Alzheimer's disease (AD), frontotemporal dementia (FTD), Niemann-Pick disease type C (NPC).

In another specific embodiment, the wild-type and/or mutated Ber-Abl1 kinase-mediated disease is selected from solid tumors, such as bone cancer, bone metastasis, breast cancer, gastro-esophageal cancer, pancreatic cancer, ovarian cancer, prostate cancer, lung cancer, colon cancer, and head and neck cancer.

In another specific embodiment, the wild-type and/or mutated Ber-Abl1 kinase-mediated disease is selected from immune diseases, such as arthritis, multiple sclerosis, osteoporosis, irritable bowel syndrome, inflammatory bowel disease, Crohn's disease, lupus, rheumatoid arthritis, psoriatic arthritis, osteoarthritis, Still's disease, juvenile arthritis,

diabetes, myasthenia gravis, Hashimoto's thyroiditis, Ord's thyroiditis, Graves' disease, Sjögren's syndrome, Guillain-Barré syndrome, acute disseminated encephalomyelitis, Addison's disease, opsoclonus syndrome, ankylosing spondylitis, antiphospholipid antibody syndrome, aplastic anemia, autoimmune hepatitis, celiac disease, Goodpasture's syndrome, idiopathic thrombocytopenic purpura, optic neuritis, scleroderma, primary biliary cirrhosis, Reiter's syndrome, Takayasu's arteritis, warm autoimmune hemolytic anemia, Wegener's granulomatosis, psoriasis, Alopecia universalis, Behcet's disease, chronic fatigue, dysautonomia, endometriosis, interstitial cystitis, neuromyotonia, scleroderma and vulvodynia, asthma, appendicitis, blepharitis, bronchiolitis, bronchitis, bursitis, cervicitis, cholangitis, cholecystitis, colitis, conjunctivitis, colitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, inflammatory enteritis, suppurative inflammation, laryngitis, mastitis, meningitis, myelitis, myocarditis, myositis, nephritis, oophoritis, orchitis, osteitis, otitis, pancreatitis, mumps, pericarditis, peritonitis, pharyngitis, pleurisy, phlebitis, pneumonia, pneumonitis, proctitis, prostatitis, pyelonephritis, rhinitis, salpingitis, sinusitis, stomatitis, synovitis, tendinitis, tonsillitis, uveitis, vaginitis, vasculitis, vulvitis graft-versus-host disease, transplantation, transfusion, anaphylaxis, allergic reaction, type I hypersensitivity, allergic conjunctivitis, allergic rhinitis and atopic dermatitis.

In another specific embodiment, the wild-type and/or mutated Ber-Abl1 kinase-mediated disease is selected from inflammatory diseases, such as arthritis, asthma, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis fibrositis, gastritis, gastroenteritis, hepatitis, hidradenitis suppurativa, laryngitis, mastitis, meningitis, osteomyelitis myocarditis, myositis, nephritis, oophoritis, orchitis, osteitis, otitis, pancreatitis, mumps, pericarditis, peritonitis, pharyngitis, pleurisy, phlebitis, pneumonia, lung disease, proctitis, prostatitis, pyelonephritis, rhinitis, salpingitis, sinusitis, stomatitis, synovitis, tendinitis, tonsillitis, uveitis, vaginitis, vasculitis and vulvitis.

In another specific embodiment, the wild-type and/or mutated Ber-Abl1 kinase-mediated disease is selected from autoimmune diseases, such as lupus and Sjögren's syndrome, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease, Sjögren's syndrome, Guillain-Barre syndrome, acute disseminated encephalomyelitis, Addison's disease, opsoclonus-myoclonus syndrome, ankylosing spondylitis, antiphospholipid antibody syndrome, aplastic anemia, autoimmune hepatitis, celiac disease, Goodpasture's syndrome, idiopathic thrombocytopenic purpura, optic neuritis, scleroderma, primary biliary cirrhosis, Reiter's syndrome, Takayasu's arteritis, hemolytic anemia, Wegener's granulomatosis, psoriasis, alopecia universalis, Behcet's disease, chronic fatigue, dysautonomia, endometriosis, interstitial cystitis, neuromyotonia, scleroderma and vulvodynia.

In another specific embodiment, the wild-type and/or mutated Ber-Abl1 kinase-mediated disease is selected from heteroimmune diseases, such as graft-versus-host disease, transplantation, transfusion, anaphylaxis, allergic reaction, type I hypersensitivity reaction, allergic conjunctivitis, allergic rhinitis, and atopic dermatitis.

In another specific embodiment, the wild-type and/or mutated Ber-Abl1 kinase-mediated disease is selected from fibrosis, such as pulmonary fibrosis, idiopathic pulmonary fibrosis (IPF), usual interstitial pneumonia (UIP), interstitial lung disease, cryptogenic fibrosing alveolitis (CFA), bronchiolitis obliterans, bronchiectasis, fatty liver disease, steatosis (e.g., nonalcoholic steatohepatitis (NASH), cholestatic liver disease (e.g., primary biliary cirrhosis (PBC)), cirrhosis, alcohol-induced liver fibrosis, bile duct damage, biliary fibrosis, cholestasis, and bile duct disease.

In the treatment methods of the present disclosure, "effective amount" is intended to mean an amount or dosage sufficient to produce the desired therapeutic benefit in an individual in need of such treatment. The effective amount or dosage of the compounds of the present disclosure can be determined by conventional methods (e.g., modeling, dose escalation, or clinical trials) and conventional factors (e.g., the mode or route of drug delivery, the pharmacokinetics of the agent, the severity and course of the infection, the individual's health status and weight, and the judgment of the treating physician). Exemplary dosages are in a range of about 0.1 mg to 1 g per day, or about 1 mg to 50 mg per day, or about 50 mg to 250 mg per day, or about 250 mg to 1 g per day. The total dosage may be in single or divided dosage units (e.g., BID, TID, QID).

After improvement of the patient's disease occurs, the dosage may be adjusted for preventive or maintenance treatment. For example, the dosage or frequency of administration, or both, may be reduced to an amount that maintains the desired therapeutic or preventive effect, depending on the symptoms. Of course, if the symptoms have been alleviated to an appropriate degree, then treatment may be discontinued. However, the patient may require long-term intermittent treatment upon recurrence of any symptom. The patient may also require long-term slow treatment.

Drug combinations

The compounds of the present disclosure described herein may be used in combination with one or more other active ingredients in pharmaceutical compositions or methods to treat the diseases and conditions described herein. Other additional active ingredients include other therapeutic agents or agents that mitigate the adverse effects of the therapeutic

agent against the intended disease target. The combination may be used to increase efficacy, ameliorate other disease symptoms, reduce one or more negative effects, or reduce the required dosage of the compounds of the present disclosure. The additional active ingredients may be formulated as separate pharmaceutical compositions from the compounds of the present disclosure or may be included in a single pharmaceutical composition with the compounds of the present disclosure. The additional active ingredients may be administered simultaneously with, prior to, or after the administration of the compounds of the present disclosure.

Combination agents include those additional active ingredients known or observed to be effective in treating the diseases and conditions described herein, including those effective against another target associated with the disease. For example, the compositions and formulations of the present disclosure, and methods of treatment may further comprise other drugs or medicaments, such as other active agents useful for treating or ameliorating the target disease or associated symptoms or conditions. For cancer indications, such other agents include, but are not limited to, kinase inhibitors, such as EGFR inhibitors (e.g., erlotinib, gefitinib); Raf inhibitors (e.g., vemurafenib), VEGFR inhibitors (e.g., sunitinib); standard chemotherapeutic agents, such as alkylating agents, antimetabolites, antitumor antibiotics, topoisomerase inhibitors, platinum drugs, mitotic inhibitors, antibodies, hormone therapy or corticosteroids. For pain indications, suitable combination agents include antiinflammatory agents, such as NSAID. The pharmaceutical compositions of the present disclosure may additionally comprise one or more of such active agents, and methods of treatment may additionally comprise administering an effective amount of one or more of such active agents.

## Examples

The present disclosure will be further elaborated with specific examples below. It should be understood that these examples are only used to illustrate the present disclosure and are not used to limit the scope of the present disclosure. The experimental methods without specific conditions in the following examples are usually in accordance with the conventional conditions or the conditions suggested by the manufacturer. Unless otherwise specified, parts and percentages are parts by weight and percentages by weight.

Generally, in the preparation process, each reaction is carried out in an inert solvent at room temperature to reflux temperature (such as 0 °C to 100 °C, alternatively 0 °C to 80 °C). The reaction time is usually 0.1 to 60 hours, and alternatively 0.5 to 24 hours.

Abbreviations used herein have the following meanings:

$Pd(dppf)Cl_2$: [1,1'-Bis(diphenylphosphino)ferrocene]dichloropalladium(II)
$Pd_2(dba)_3$: Tris(dibenzylideneacetone)dipalladium(0)
XPhos Pd G2: Chloro(2-dicyclohexylphosphino-2',4',6'-triisopropyl-1,1'-biphenyl)[2-(2'-amino-1,1'-biphenyl)]palladium(II)
NBS: N-Bromosuccinimide
PTSA: p-Toluenesulfonic acid
HATU: O-(7-azabenzotriazol-1-yl)-N,N,N',N'-tetramethyluronium hexafluorophosphate
DMAP: 4-Dimethylaminopyridine
$B_2Pin_2$: Bis(pinacolato)diboron
t-Bu XPhos: 2-Di-tert-butylphosphino-2',4',6'-triisopropylbiphenyl
DAST: Diethylaminosulfur trifluoride
TEA: Triethylamine
DIEA: N,N-Diisopropylethylamine
TFA: Trifluoroacetic acid
HCOOH: Formic acid
AcOH: Acetic acid
$K_2CO_3$: Potassium carbonate
KOAc: Potassium acetate
EtOH: Ethanol
DCM: Dichloromethane
THF: Tetrahydrofuran
DMF: N,N-Dimethylformamide
DMSO: Dimethyl sulfoxide
TsCl: p-Toluenesulfonyl chloride
AIBN: Azobisisobutyronitrile
$SOCl_2$: Thionyl chloride
LiHMDS: Lithium bis(trimethylsilyl)amide
TBAF: Tetrabutylammonium fluoride

m-CPBA: meta-Chloroperoxybenzoic acid

**Preparation of intermediate A-1 (R)-5-bromo-N-(4-(chlorodifluoromethoxy)phenyl)-6-(3-fluoropyrrolidin-1-yl) nicotinamide**

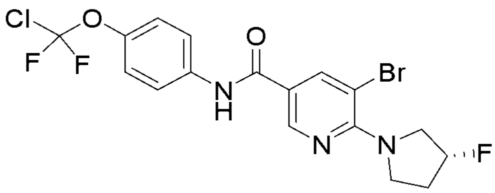

A-1

The following synthetic route was adopted:

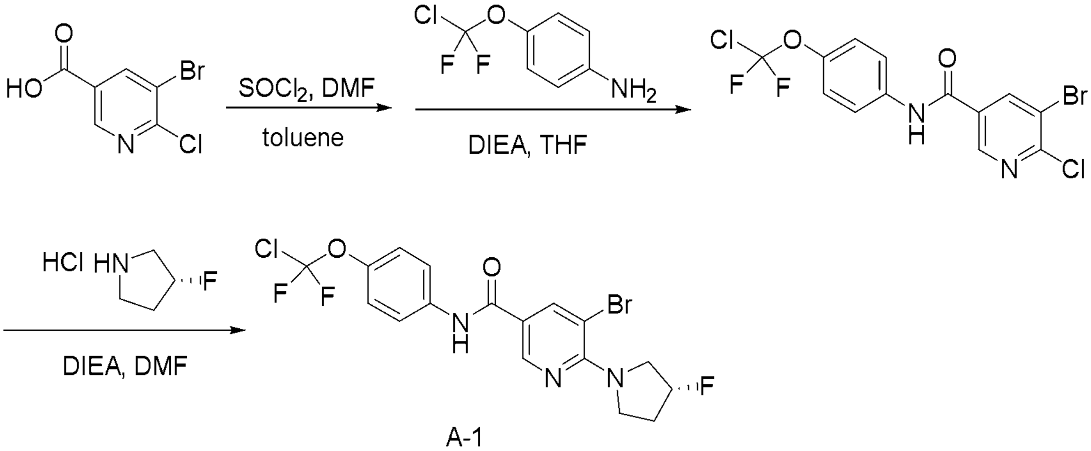


A-1

Step 1: Synthesis of compound 5-bromo-6-chloro-N-(4-(chlorodifluoromethoxy)phenyl)nicotinamide

5-Bromo-6-chloronicotinic acid (24.4 g, 103.62 mmol), toluene (160 mL) and DMF (2 mL, 15.07 mmol) were added to a reaction flask. Thionyl chloride (50 mL, 688.4 mmol) was added dropwise at 0 °C. After the addition was completed, the mixture was heated to 80 °C and reacted for 5 hours. The reaction was monitored by TLC until it was completed. The mixture was cooled to room temperature, and concentrated under reduced pressure to remove toluene and excess thionyl chloride. Anhydrous THF (160 mL) was added to the residue, and the mixture was cooled to -20 °C. DIEA (26.72 g, 207.24 mmol) was added dropwise, and then a solution of 4-(chlorodifluoromethoxy)aniline (20 g, 103.62 mmol) in THF (40 mL) was slowly added dropwise. After the addition was completed, the mixture was stirred and reacted at -20 °C for 30 minutes. The mixture was warmed to room temperature and reacted for 18 hours. The reaction was monitored by TLC until it was completed. The mixture was concentrated under reduced pressure to remove tetrahydrofuran. 250 mL of methyl tert-butyl ether was added to dilute the mixture, and the resulting mixture was successively washed with 0.5 M hydrochloric acid (250 mL x 2), saturated sodium bicarbonate (250 mL x 2), and saturated sodium chloride (200 mL). The mixture was dried over anhydrous sodium sulfate, filtered, and concentrated to give 44.69 g of a light yellow solid, with a yield of 99%. LC-MS (APCI): m/z=410.9 $(M+1)^+$.

Step 2: Synthesis of intermediate compound A-1

5-Bromo-6-chloro-N-(4-(chlorodifluoromethoxy)phenyl)nicotinamide (36.7 g, 89.36 mmol), (R)-3-fluoropyrrolidine hydrochloride (13.6 g, 107.23 mmol), DMF (250 mL) and DIEA (37 g, 268 mmol) were added to a reaction flask. The mixture was heated to 130 °C, stirred and reacted for 2 hours. The reaction was monitored by TLC until it was completed. The resulting mixture was cooled to room temperature, and the reaction solution was poured into 500 mL of ice water. 300 mL of ethyl acetate was added. The resulting mixture was stirred at room temperature until the solid was completely dissolved. The aqueous layer was extracted with 200 mL of ethyl acetate twice. The organic phases were combined, washed with saturated brine, dried over anhydrous sodium sulfate, filtered, and concentrated to give a yellow solid. 250 mL of n-heptane was added and the resulting mixture was stirred at room temperature for 10 minutes. The mixture was filtered.

The filter cake was washed with petroleum ether, and vacuum dried to give 38 g of a white solid, with a yield of 92.0%. LC-MS (APCI): m/z=464.3 $(M+1)^+$.

**Preparation of intermediate A-2 7-bromo-N-(4-(chlorodifluoromethoxy)phenyl)-1-isopropyl-1H-benzo[d]imidazole-5-carboxamide**

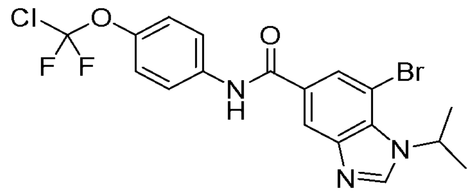

A-2

The following synthetic route was adopted:

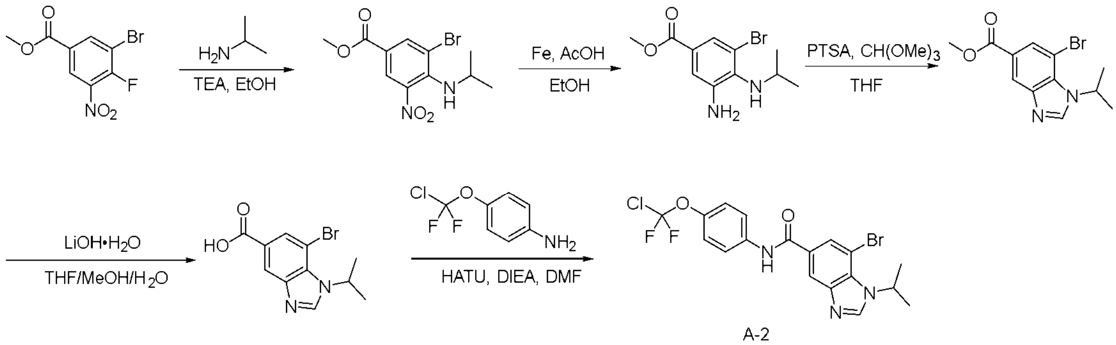


Step 1: Synthesis of compound methyl 3-bromo-4-(isopropylamino)-5-nitrobenzoate

Methyl 3-bromo-4-fluoro-5-nitrobenzoate (5.0 g, 18.0 mmol), isopropylamine (1.1 g, 18.0 mmol), triethylamine (2.7 g, 27.0 mmol) and ethanol (50 mL) were added to a reaction flask. The mixture was heated to *50* °C, stirred and reacted for 4 to 6 hours under nitrogen. The reaction was monitored by TLC until it was completed. The resulting mixture was concentrated to remove the solvent. The residue was purified by column chromatography, and vacuum dried to give 5.1 g of a light yellow solid, with a yield of 89%. LC-MS (APCI): m/z=317.2 $(M+1)^+$.

Step 2: Synthesis of compound methyl 3-amino-5-bromo-4-(isopropylamino)benzoate

Methyl 3-bromo-4-(isopropylamino)-5-nitrobenzoate (4.5 g, 14.2 mmol), reduced iron powder (8.0 g, 142 mmol), ethanol (30 mL) and glacial acetic acid (2 mL) were added to a reaction flask. After the addition was completed, the mixture was heated to 70 °C, stirred and reacted for 2 h. The reaction was monitored by TLC until it was completed. The mixture was filtered while hot. The filtrate was concentrated and then directly added to the next step reaction. LC-MS (APCI): m/z=287.4 $(M+1)^+$.

Step 3: Synthesis of compound methyl 7-bromo-1-isopropyl-1H-benzo[d]imidazole-5-carboxylate

Methyl 3-amino-5-bromo-4-(isopropylamino)benzoate and trimethyl orthoformate (7.5 g, 71.0 mmol) were dissolved in 40 mL of anhydrous THF. PTSA (0.24 g, 1.4 mmol) was added, and the mixture was heated to reflux, stirred and reacted under nitrogen overnight. The reaction was monitored by TLC until it was completed. Saturated aqueous solution of ammonium chloride was added to quench the reaction. The mixture was extracted with ethyl acetate 3 to 4 times. The organic phases were combined, and washed with saturated brine 3 times. The organic phase was separated, concentrated, and then purified by silica gel column chromatography to give 2.74 g of a light yellow solid, with a two-step yield of 65%. LC-MS (APCI): m/z=297.2 $(M+1)^+$.

Step 4: Synthesis of compound 7-bromo-1-isopropyl-1H-benzo[d]imidazole-5-carboxylic acid

Methyl 7-bromo-1-isopropyl-1H-benzo[d]imidazole-5-carboxylate (2.74 g, 9.3 mmol) and lithium hydroxide monohydrate (1.95 g, 46.5 mmol) were added to a reaction flask, and the mixture was dissolved in 10 mL of tetrahydrofuran, 10 mL of methanol and 10 mL of water. The mixture was stirred and reacted at room temperature overnight. The reaction was monitored by TLC until it was completed. The pH was adjusted to weakly acidic with 1N dilute hydrochloric acid, and the mixture was extracted with ethyl acetate 3 to 4 times. The organic phases were combined, washed with saturated brine 3 times, concentrated to remove the solvent, and purified by silica gel column chromatography to give 2.3 g of a white solid, with a yield of 88%. LC-MS (APCI): m/z=283.6 $(M+1)^+$.

Step 5: Synthesis of intermediate compound A-2

7-Bromo-1-isopropyl-1H-benzo[d]imidazole-5-carboxylic acid (2.3 g, 8.2 mmol), 4-(chlorodifluoromethoxy)aniline (1.9 g, 9.8 mmol), HATU (4.7 g, 12.3 mmol) and DIEA (2.1 g, 16.4 mmol) were dissolved in 25 mL of anhydrous DMF. The mixture was stirred and reacted at room temperature under nitrogen for 5 to 6 h. The reaction was monitored by TLC until it was completed. Water was added to dilute the mixture. The resulting mixture was extracted with ethyl acetate 3 to 4 times. The organic phases were combined, washed with saturated brine 3 times, concentrated to remove the solvent, and purified by silica gel column chromatography to give 2.8 g of a yellow solid, with a yield of 76%. LC-MS (APCI): m/z=458.2 $(M+1)^+$.

**Preparation of intermediate A-3 4-bromo-N-(4-(chlorodifluoromethoxy)phenyl)-3,3-dimethyl-2-oxoindoline-6-carboxamide**

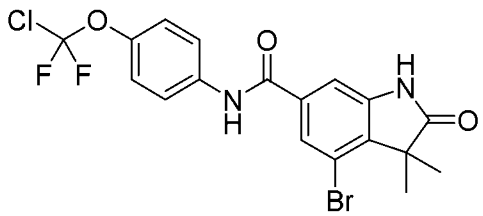

A-3

The following synthetic route was adopted:

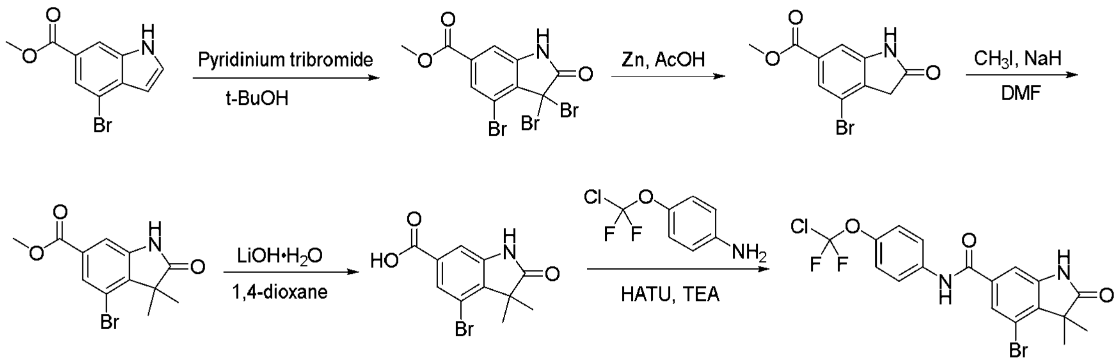


Step 1: Synthesis of compound methyl 3,3,4-tribromo-2-oxoindoline-6-carboxylate

Methyl 4-bromoindole-6-carboxylate (5.0 g, 19.7 mmol), pyridinium tribromide (19.2 g, 60 mmol) and tert-butanol (50 mL) were added to a reaction flask. The mixture was stirred and reacted at room temperature under nitrogen overnight. The reaction was monitored by TLC until it was completed. 50 mL of saturated aqueous solution of citric acid was added to quench the reaction. The mixture was extracted with ethyl acetate 3 to 4 times. The organic phases were combined, washed with saturated brine, and concentrated to remove the solvent. The residue was purified by column chromatography, and vacuum dried to give 5.3 g of a yellow solid, with a yield of 63%. LC-MS (APCI): m/z=425.8 $(M+1)^+$.

Step 2: Synthesis of compound methyl 4-bromo-2-oxoindoline-6-carboxylate

Methyl 3,3,4-tribromo-2-oxoindoline-6-carboxylate (4.2 g, 10 mmol), zinc powder (2.0 g, 30 mmol), and acetic acid (20 mL) were added to a reaction flask. The mixture was heated to 80 °C, stirred and reacted overnight. The reaction was monitored by TLC until it was completed. The resulting mixture was filtered to remove the catalyst, and concentrated to remove the most of the acetic acid. The resulting mixture was diluted with added water. The resulting mixture was extracted with ethyl acetate 3 to 4 times. The organic phases were combined, washed successively with saturated aqueous solution of sodium bicarbonate and saturated brine 3 times. The mixture was concentrated to remove the solvent, and purified by silica gel column chromatography to give 1.29 g of a light yellow solid, with a yield of 48%. LC-MS (APCI): m/z=270.1 $(M+1)^+$.

Step 3: Synthesis of compound methyl 4-bromo-3,3-dimethyl-2-oxoindoline-6-carboxylate

Methyl 4-bromo-2-oxoindoline-6-carboxylate (1.3 g, 4.8 mmol) was dissolved in 10 mL of anhydrous DMF. Sodium hydride (0.42 g, 10.6 mmol) was added in batches in an ice bath. After the addition was completed, the mixture was stirred and reacted for 0.5 h. Iodomethane (2.0 g, 14.4 mmol) was added. The mixture was warmed to room temperature, stirred and reacted for 3 to 4 h. The reaction was monitored by TLC until it was completed. The reaction solution was concentrated, and then purified by silica gel column chromatography to give 1.24 g of product, with a yield of 87%. LC-MS (APCI): m/z=298.7 $(M+1)^+$.

Step 4: Synthesis of compound 4-bromo-3,3-dimethyl-2-oxoindoline-6-carboxylic acid

Methyl 4-bromo-3,3-dimethyl-2-oxoindoline-6-carboxylate (2.8 g, 9.3 mmol), lithium hydroxide monohydrate (1.95 g, 46.5 mmol) and 1,4-dioxane (15 mL) were added to a reaction flask. The mixture was stirred and reacted at room temperature overnight. The reaction was monitored by TLC until it was completed. The pH was adjusted to weakly acidic with 1N dilute hydrochloric acid. The mixture was extracted with ethyl acetate 3 to 4 times. The organic phases were combined, washed with saturated brine 3 times, concentrated to remove the solvent, and purified by silica gel column chromatography to give 2.0 g of a yellow solid, with a yield of 77%. LC-MS (APCI): m/z=283.9 $(M+1)^+$.

Step 5: Synthesis of intermediate compound A-3

4-Bromo-3,3-dimethyl-2-oxoindoline-6-carboxylic acid (2.32 g, 8.2 mmol), 4-(chlorodifluoromethoxy)aniline (1.9 g, 9.8 mmol), HATU (4.7 g, 12.3 mmol) and TEA (1.66 g, 16.4 mmol) were added to a reaction flask. The mixture was dissolved in 25 mL of anhydrous DMF. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated to remove the solvent, and purified by silica gel column chromatography to give 2.8 g of a yellow solid, with a yield of 74%. LC-MS (APCI): m/z=459.2 $(M+1)^+$.

**Preparation of intermediate A-4 4'-bromo-N-(4-(chlorodifluoromethoxy)phenyl)-2'-oxospiro[cyclohexane-1,3'-indoline]-6'-carboxamide**

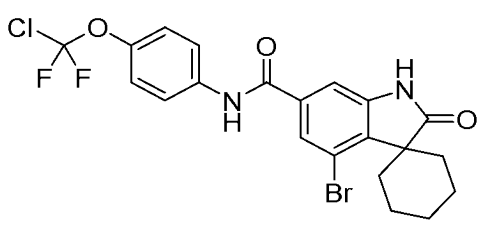

A-4

The following synthetic route was adopted:

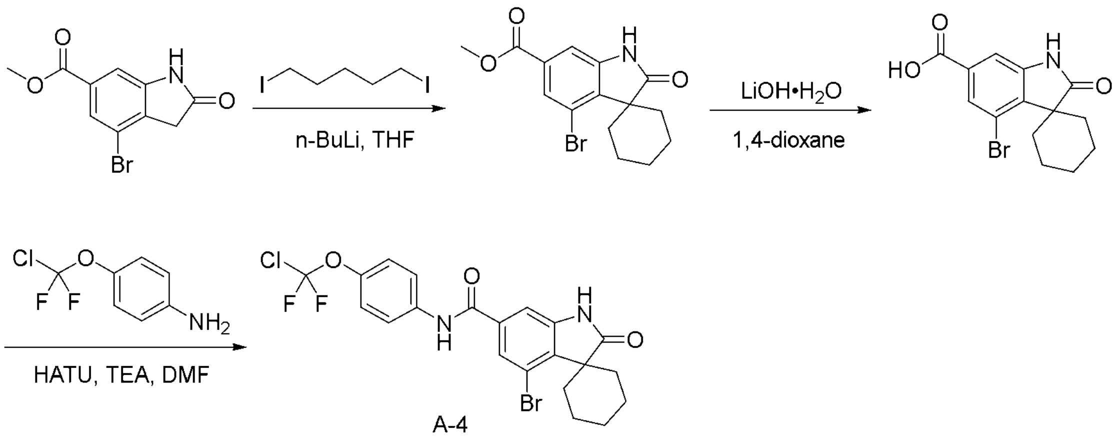


A-4

Step 1: Synthesis of compound methyl 4'-bromo-2'-oxospiro[cyclohexane-1,3'-indoline]-6'-carboxylate

Methyl 4-bromo-2-oxoindoline-6-carboxylate (1.3 g, 4.8 mmol) and anhydrous THF (15 mL) were added to a reaction flask. n-Butyllithium (6.6 mL, 10.6 mmol) was added dropwise at -78 °C under nitrogen. After the addition was completed, the mixture was stirred and reacted for 0.5 hours. 1,5-Diiodopentane (4.7 g, 14.4 mmol) was slowly added dropwise. The mixture was warmed to room temperature, stirred and reacted for 3 to 4 hours. The reaction was monitored by TLC until it was completed. Saturated ammonium chloride was added to quench the reaction. The resulting mixture was extracted with ethyl acetate 3 to 4 times. The organic phases were combined, washed with saturated brine, concentrated, and then purified by silica gel column chromatography to give 1.13 g of product, with a yield of 70%. LC-MS (APCI): m/z=338.7 $(M+1)^+$.

Step 2: Synthesis of compound 4'-bromo-2'-oxospiro[cyclohexane-1,3'-indoline]-6'-carboxylic acid

Methyl 4'-bromo-2'-oxospiro[cyclohexane-1,3'-indoline]-6'-carboxylate (3.1 g, 9.3 mmol), lithium hydroxide monohydrate (1.95 g, 46.5 mmol) and 1,4-dioxane (15 mL) were added to a reaction flask. The mixture was stirred and reacted at room temperature overnight. The reaction was monitored by TLC until it was completed. The pH was adjusted to weakly acidic with 1N dilute hydrochloric acid. The resulting mixture was extracted with ethyl acetate 3 to 4 times. The organic phases were combined, washed with saturated brine 3 times, concentrated to remove the solvent, and purified by silica gel column chromatography to give 1.9 g of a yellow solid, with a yield of 64%. LC-MS (APCI): m/z=324.4 $(M+1)^+$.

Step 3: Synthesis of intermediate compound A-4

4'-Bromo-2'-oxospiro[cyclohexane-1,3'-indoline]-6'-carboxylic acid (2.65 g, 8.2 mmol), 4-(chlorodifluoromethoxy)aniline (1.9 g, 9.8 mmol), HATU (4.7 g, 12.3 mmol) and TEA (1.66 g, 16.4 mmol) were dissolved in 25 mL of anhydrous DMF. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated to remove the solvent, and purified by silica gel column chromatography to give 2.2 g of a yellow solid, with a yield of 54%. LC-MS (APCI): m/z=499.2 $(M+1)^+$.

**Preparation of intermediate A-5 3-acetamido-5-bromo-N-(4-(chlorodifluoromethoxy)phenyl)-4-isopropyl-1,2,3,3a,4,8b-hexahydrocyclopenta[b]indole-7-carboxamide**

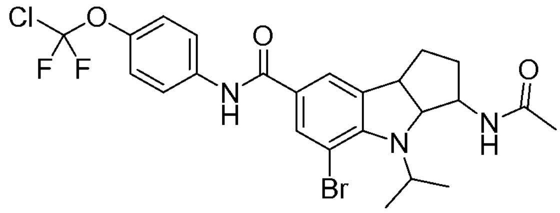

A-5

The following synthetic route was adopted:

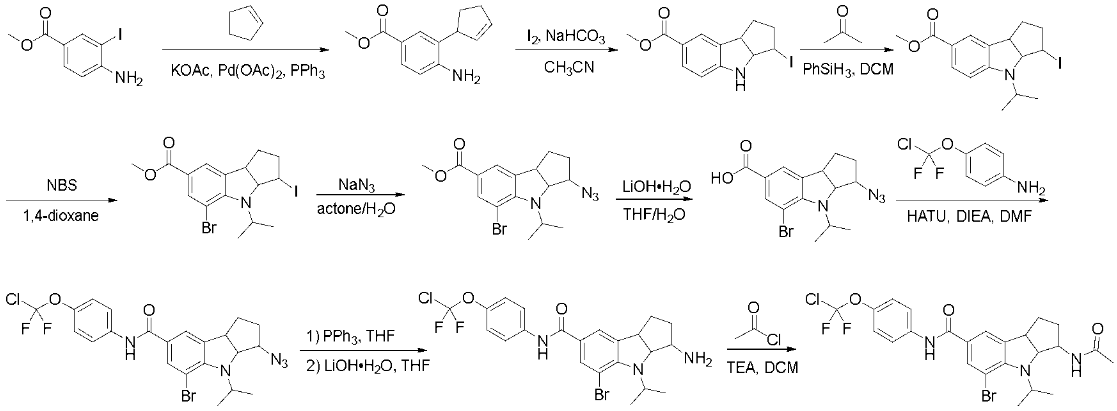


A-5

Step 1: Synthesis of compound methyl 4-amino-3-(cyclopent-2-en-1-yl)benzoate

Methyl 4-amino-3-iodobenzoate (5.0 g, 18 mmol), cyclopentene (1.84 g, 27 mmol), potassium acetate (3.5 g, 36 mmol), palladium(II) acetate (0.2 g, 0.9 mmol) and triphenylphosphine (0.5 g, 1.8 mmol) were added to a reaction flask, and the mixture was dissolved in 20 mL of anhydrous DMF. The mixture was heated to 80 °C, stirred and reacted under nitrogen overnight. The reaction was monitored by TLC until it was completed. Saturated aqueous solution of ammonium chloride was added to quench the reaction. The mixture was extracted with ethyl acetate 3 to 4 times. The organic phases were combined, washed with saturated brine, concentrated, and then purified by silica gel column chromatography to give 1.83 g of a light yellow oily liquid, with a yield of 47%. LC-MS(APCI): m/z=218.2 $(M+1)^+$.

Step 2: Synthesis of compound methyl 3-iodo-1,2,3,3a,4,8b-hexahydrocyclopenta[b]indole-7-carboxylate

Methyl 4-amino-3-(cyclopent-2-en-1-yl)benzoate (1.8 g, 8.3 mmol), elemental iodine (4.2 g, 16.6 mmol), sodium bicarbonate (2.1 g, 24.9 mmol) and acetonitrile (20 mL) were added to a reaction flask. The mixture was stirred and reacted at room temperature overnight. The reaction was monitored by TLC until it was completed. Saturated sodium sulfite solution was added to quench the reaction. The mixture was extracted with ethyl acetate 3 to 4 times. The organic phases were combined, washed with saturated brine 3 times, concentrated to remove the solvent, and purified by silica gel column chromatography to give 1.48 g of a light yellow oily solid, with a yield of 52%. LC-MS (APCI): m/z=344.6 $(M+1)^+$.

Step 3: Synthesis of compound methyl 3-iodo-4-isopropyl-1,2,3,3a,4,8b-hexahydrocyclopenta[b]indole-7-carboxylate

Methyl 3-iodo-1,2,3,3a,4,8b-hexahydrocyclopenta[b]indole-7-carboxylate (1.4 g, 4.1 mmol), acetone (0.5 g, 8.2 mmol), phenylsilane (0.97 g, 9.02 mmol) and anhydrous DCM (20 mL) were added to a reaction flask. The mixture was stirred and reacted at room temperature for 4 to 6 hours. The reaction was monitored by TLC until it was completed. The mixture was concentrated to remove the solvent. The residue was purified by column chromatography, and vacuum dried to give 1.2 g of a light yellow solid, with a yield of 77%. LC-MS (APCI): m/z=386.3 $(M+1)^+$.

Step 4: Synthesis of compound methyl 5-bromo-3-iodo-4-isopropyl-1,2,3,3a,4,8b-hexahydrocyclopenta[b]indole-7-carboxylate

Methyl 3-iodo-4-isopropyl-1,2,3,3a,4,8b-hexahydrocyclopenta[b]indole-7-carboxylate (1.2 g, 3.1 mmol), NBS (0.83 g, 4.7 mmol) and 1,4-dioxane (20 mL) were added to a reaction flask. The mixture was stirred and reacted at room

temperature under nitrogen overnight. The reaction was monitored by TLC until it was completed. The mixture was concentrated, and then purified by column chromatography, and vacuum dried to give 0.92 g of a light yellow solid, with a yield of 64%. LC-MS (APCI): m/z=464.4 $(M+1)^+$.

Step 5: Synthesis of compound methyl 3-azido-5-bromo-4-isopropyl-1,2,3,3a,4,8b-hexahydrocyclopenta[b]indole-7-carboxylate

Methyl 5-bromo-3-iodo-4-isopropyl-1,2,3,3a,4,8b-hexahydrocyclopenta[b]indole-7-carboxylate (0.92 g, 2.0 mmol) and sodium azide (260 mg, 4.0 mmol) were dissolved in 10 mL of acetone and 5 mL of water. The mixture was heated to 50 °C, stirred and reacted under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated to remove the solvent, and purified by silica gel column chromatography to give 695 mg of a light yellow solid, with a yield of 92%. LC-MS (APCI): m/z=379.2 $(M+1)^+$.

Step 6: Synthesis of compound 3-azido-5-bromo-4-isopropyl-1,2,3,3a,4,8b-hexahydrocyclopenta[b]indole-7-carboxylic acid

Methyl 3-azido-5-bromo-4-isopropyl-1,2,3,3a,4,8b-hexahydrocyclopenta[b]indole-7-carboxylate (3.5 g, 9.3 mmol), lithium hydroxide monohydrate (1.95 g, 46.5 mmol), THF (10 mL) and water (10 mL) were added to a reaction flask. The mixture was stirred and reacted at room temperature overnight. The reaction was monitored by TLC until it was completed. The pH was adjusted to weakly acidic with 1N dilute hydrochloric acid. The resulting mixture was extracted with ethyl acetate 3 to 4 times. The organic phases were combined, washed with saturated brine 3 times, concentrated to remove the solvent, and purified by silica gel column chromatography to give 2.94 g of an off-white solid, with a yield of 87%. LC-MS (APCI): m/z=365.6 $(M+1)^+$.

Step 7 Synthesis of compound 3-azido-5-bromo-N-(4-(chlorodifluoromethoxy)phenyl)-4-isopropyl-1,2,3,3a,4,8b-hexahydrocyclopenta[b]indole-7-carboxamide

3-Azide-5-bromo-4-isopropyl-1,2,3,3a,4,8b-hexahydrocyclopenta[b]indole-7-carboxylic acid (2.99 g, 8.2 mmol), 4-(chlorodifluoromethoxy)aniline (1.9 g, 9.8 mmol), HATU (4.7 g, 12.3 mmol) and DIEA (2.1 g, 16.4 mmol) were added to a reaction flask. The mixture was dissolved in 25 mL of anhydrous DMF. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated to remove the solvent, and purified by silica gel column chromatography to give 3.05 g of a yellow solid, with a yield of 69%. LC-MS (APCI): m/z=540.6 $(M+1)^+$.

Step 8 Synthesis of compound 3-amino-5-bromo-N-(4-(chlorodifluoromethoxy)phenyl)-4-isopropyl-1,2,3,3a,4,8b-hexahydrocyclopenta[b]indole-7-carboxamide

3-Azide-5-bromo-N-(4-(chlorodifluoromethoxy)phenyl)-4-isopropyl-1,2,3,3a,4,8b-hexahydrocyclopenta[b]indole-7-carboxamide (1.5 g, 2.8 mmol), triphenylphosphine (1.46 g, 5.6 mmol), and anhydrous THF (10 mL) were added to a reaction flask. The mixture was heated to 60 °C and reacted overnight. The reaction was monitored by TLC until it was completed. The resulting mixture was cooled to room temperature, and then lithium hydroxide monohydrate (0.47 g, 11.2 mmol) was added. The mixture was reacted at room temperature for 1 hour. The reaction was monitored by TLC until it was completed. The resulting mixture was concentrated, and then purified by silica gel column chromatography to give 488 mg of product, with a yield of 34%. LC-MS (APCI): m/z=514.7 $(M+1)^+$.

Step 9: Synthesis of intermediate compound A-5

3-Amino-5-bromo-N-(4-(chlorodifluoromethoxy)phenyl)-4-isopropyl-1,2,3,3a,4,8b-hexahydrocyclopenta[b]indole-7-carboxamide (488 mg, 0.95 mmol), TEA (192 mg, 1.9 mmol) and anhydrous DCM (10 mL) were added to a reaction flask. Acetyl chloride (89 mg, 1.14 mmol) was added dropwise in an ice bath. The resulting mixture was warmed to room temperature, stirred and reacted for 1 to 2 hours. The reaction was monitored by TLC until it was completed. Water was added to quench the reaction. The organic phase was separated, concentrated, and then purified by silica gel column chromatography to give 480 mg of product, with a yield of 91%. LC-MS (APCI): m/z=556.7 $(M+1)^+$.

**Preparation of intermediate A-6 (1R)-9-bromo-N-(4-(chlorodifluoromethoxy)phenyl)-4-hydroxy-1-methyl-1,2,3,4-tetrahydrobenzo[4,5]imidazo[1,2-a]pyridine-7-carboxamide**

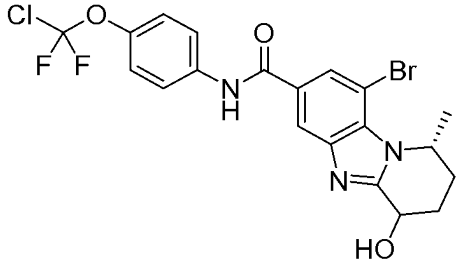

A-6

The following synthetic route was adopted:

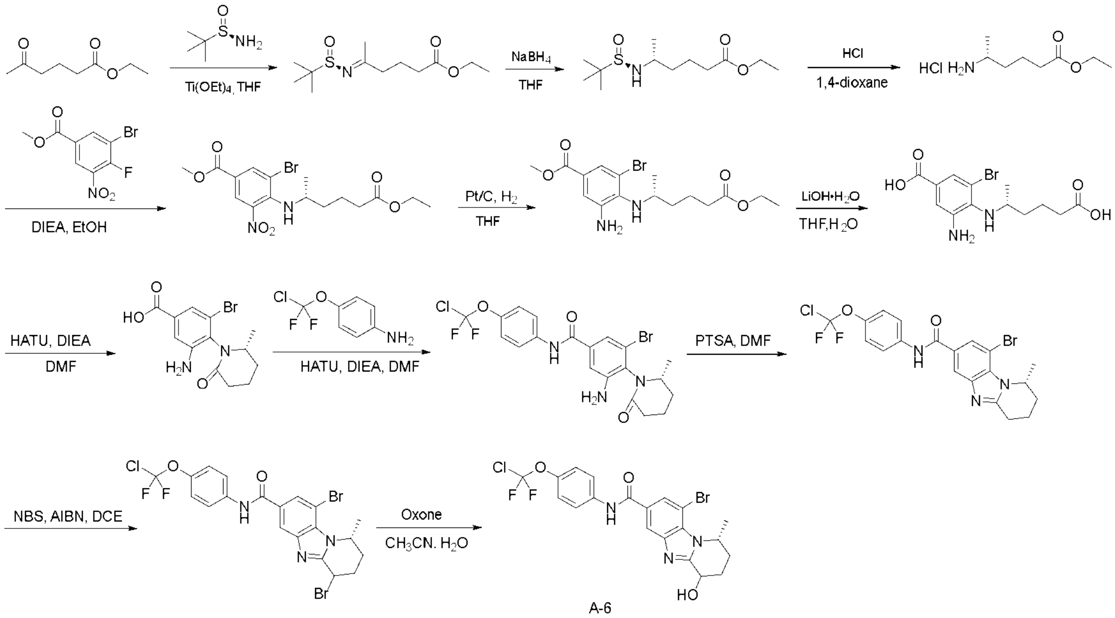


Step 1: Synthesis of compound ethyl (R,E)-5-((tert-butylsulfinyl)imino)hexanoate

Ethyl 4-acetylbutyrate (10.0 g, 63.3 mmol), (R)-tert-butylsulfinamide (9.2 g, 75.9 mmol) and titanium(IV) ethoxide (36 g, 127 mmol) were added to a reaction flask, and the mixture was dissolved in 100 mL of anhydrous THF. The mixture was heated to reflux, stirred and reacted under nitrogen for 12 hours. The reaction was monitored by TLC until it was completed. 50 mL of water was added to quench the reaction. The mixture was extracted with ethyl acetate 3 to 4 times. The organic phases were combined, washed with saturated brine, concentrated, and then purified by silica gel column chromatography to give 14.0 g of an anhydrous oily liquid, with a yield of 85%. LC-MS (APCI): m/z=262.2 $(M+1)^+$.

Step 2: Synthesis of compound ethyl (R)-5-(((R)-tert-butylsulfinyl)amino)hexanoate

Ethyl (R,E)-5-((tert-butylsulfinyl)imino)hexanoate (14.0 g, 53.6 mmol) and THF (80 mL) were added to a reaction flask. Sodium borohydride (3.1 g, 80.5 mmol) was added in batches in an ice bath. After the addition was completed, the mixture was stirred and reacted at room temperature under nitrogen overnight. The reaction was monitored by TLC until it was completed. Water was added to quench the reaction. The resulting mixture was extracted with ethyl acetate 3 to 4 times. The organic phases were combined, washed with saturated brine, concentrated, and then purified by silica gel column chromatography to give 10.7 g of a colorless oily liquid, with a yield of 76%. LC-MS (APCI): m/z=264.7 $(M+1)^+$.

Step 3: Synthesis of compound ethyl (R)-5-aminohexanoate hydrochloride

Ethyl (R)-5-(((R)-tert-butylsulfinyl)amino)hexanoate (10.7 g, 41 mmol) and a 4 M solution of hydrogen chloride in 1,4-dioxane (50 mL) were added to a reaction flask. The mixture was stirred and reacted at room temperature for 2 to 4 hours. The reaction was monitored by TLC until it was completed. The resulting mixture was concentrated, and then directly added to the next step reaction. LC-MS (APCI): m/z=160.7 $(M+1)^+$.

Step 4: Synthesis of compound methyl (R)-3-bromo-4-((6-ethoxy-6-oxohexan-2-yl)amino)-5-nitrobenzoate

Ethyl (R)-5-aminohexanoate hydrochloride, methyl 3-bromo-4-fluoro-5-nitrobenzoate (11.3 g, 41 mmol), DIEA (13.2 g, 102.5 mmol) and anhydrous ethanol (60 mL) were added to a reaction flask. The mixture was heated to 60 °C, stirred and reacted under nitrogen for 6 to 8 hours. The reaction was monitored by TLC until it was completed. The resulting mixture was concentrated to remove the solvent, and purified by silica gel column chromatography to give 10.1 g of a yellow oily liquid, with a two-step yield of 59%. LC-MS (APCI): m/z=417.5 $(M+1)^+$.

Step 5: Synthesis of compound methyl (R)-3-amino-5-bromo-4-((6-ethoxy-6-oxohexan-2-yl)amino)benzoate

Methyl (R)-3-bromo-4-((6-ethoxy-6-oxohexan-2-yl)amino)-5-nitrobenzoate (10.1 g, 24.3 mmol) and THF (70 mL) were added to a reaction flask. The mixture was stirred until dissolved. A catalytic amount of Pt/C was added. The reaction system was purged with hydrogen, and then the mixture was stirred and reacted at room temperature overnight. The reaction was monitored by TLC until it was completed. The resulting mixture was filtered to remove the catalyst. The filtrate was concentrated, and then directly added to the next step reaction. LC-MS (APCI): m/z=387.3 $(M+1)^+$.

Step 6: Synthesis of compound (R)-3-amino-5-bromo-4-((5-carboxypentan-2-yl)amino)benzoic acid

Methyl (R)-3-amino-5-bromo-4-((6-ethoxy-6-oxohexan-2-yl)amino)benzoate and lithium hydroxide monohydrate (5.1 g, 121.5 mmol) were added to a reaction flask. The mixture was dissolved in 30 mL of tetrahydrofuran and 30 mL of water. The mixture was stirred and reacted at room temperature overnight. The reaction was monitored by TLC until it was completed. The pH was adjusted to weakly acidic with 1N dilute hydrochloric acid. The resulting mixture was extracted with ethyl acetate 3 to 4 times. The organic phases were combined, washed with saturated brine 3 times, concentrated to remove the solvent, and purified by silica gel column chromatography to give 5.7 g of an off-white solid, with a yield of 68%. LC-MS (APCI): m/z=345.6 $(M+1)^+$.

Step 7 Synthesis of compound (R)-3-amino-5-bromo-4-(2-methyl-6-oxopiperidin-1-yl)benzoic acid

(R)-3-Amino-5-bromo-4-((5-carboxypentan-2-yl)amino)benzoic acid (2.8 g, 8.2 mmol), HATU (4.7 g, 12.3 mmol), DIEA (2.1 g, 16.4 mmol) and anhydrous DMF (25 mL) were added to a reaction flask. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated to remove the solvent, and purified by silica gel column chromatography to give 1.92 g of a yellow solid, with a yield of 72%. LC-MS (APCI): m/z=327.6 $(M+1)^+$.

Step 8: Synthesis of compound (R)-3-amino-5-bromo-N-(4-(chlorodifluoromethoxy)phenyl)-4-(2-methyl-6-oxopiperidin-1-yl)benzamide

(R)-3-Amino-5-bromo-4-(2-methyl-6-oxopiperidin-1-yl)benzoic acid (2.7 g, 8.2 mmol), 4-(chlorodifluoromethoxy)aniline (1.9 g, 9.8 mmol), HATU (4.7 g, 12.3 mmol) and DIEA (2.1 g, 16.4 mmol) were added to a reaction flask. The mixture was dissolved in 25 mL of anhydrous DMF. The resulting mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated to remove the solvent, and purified by silica gel column chromatography to give 2.96 g of a light yellow solid, with a yield of 72%. LC-MS (APCI): m/z=502.3 $(M+1)^+$.

Step 9: Synthesis of compound (R)-9-bromo-N-(4-(chlorodifluoromethoxy)phenyl)-1-methyl-1,2,3,4-tetrahydrobenzo [4,5]imidazo[1,2-a]pyridine-7-carboxamide

(R)-3-Amino-5-bromo-N-(4-(chlorodifluoromethoxy)phenyl)-4-(2-methyl-6-oxopiperidin-1-yl)benzamide (1.5 g, 3.0 mmol), p-toluenesulfonic acid (258 mg, 1.5 mmol) and DMF (10 mL) were added to a reaction flask. The mixture was

stirred and reacted at room temperature for 1 to 2 hours. The reaction was monitored by TLC until it was completed. Water was added to quench the reaction. The resulting mixture was extracted with ethyl acetate 3 to 4 times. The organic phases were combined, washed with saturated brine, concentrated, and then purified by silica gel column chromatography to give 1.32 g of product, with a yield of 91%. LC-MS (APCI): m/z=484.7 $(M+1)^+$.

Step 10 Synthesis of compound (1R)-4,9-dibromo-N-(4-(chlorodifluoromethoxy)phenyl)-1-methyl-1,2,3,4-tetrahydrobenzo[4,5]imidazo[1,2-a]pyridine-7-carboxamide

(R)-9-Bromo-N-(4-(chlorodifluoromethoxy)phenyl)-1-methyl-1,2,3,4-tetrahydrobenzo[4,5]imidazo[1,2-a]pyridine-7-carboxamide (1.3 g, 2.7 mmol), NBS (0.56 g, 3.2 mmol) and dichloroethane (20 mL) were added to a reaction flask. AIBN (0.5 g, 2.7 mmol) was added under nitrogen. The mixture was heated to 50 °C, stirred and reacted overnight. The reaction was monitored by TLC until it was completed. The resulting mixture was concentrated to remove the solvent. The residue was purified by silica gel column chromatography, and vacuum dried to give 1.1 g of a light yellow solid as a product, with a yield of 74%. LC-MS (APCI): m/z=562.1 $(M+1)^+$.

Step 11 Synthesis of intermediate compound A-6

(1R)-4,9-Dibromo-N-(4-(chlorodifluoromethoxy)phenyl)-1-methyl-1,2,3,4-tetrahydrobenzo[4,5]imidazo[1,2-a] pyridine-7-carboxamide (1.1 g, 2.0 mmol), acetonitrile (10 mL) and water (10 mL) were added to a reaction flask. Potassium peroxymonosulfate (2.1 g, 6.0 mmol) was added in an ice bath. After the addition was completed, the mixture was warmed to room temperature, stirred and reacted overnight. The reaction was monitored by TLC until it was completed. Saturated aqueous solution of sodium sulfite was added to quench the reaction. The resulting mixture was extracted with ethyl acetate 3 to 4 times. The organic phases were combined, washed with saturated brine, concentrated, and then purified by silica gel column chromatography to give 329 mg of product, with a yield of 33%. LC-MS(APCI): m/z=500.3 $(M+1)^+$.

**Preparation of intermediate A-7 (R)-6-bromo-N-(4-(chlorodifluoromethoxy)phenyl)-2,4-dimethyl-1,2,3,4-tetrahydrobenzo[4,5]imidazo[1,2-a]pyrazine-8-carboxamide**

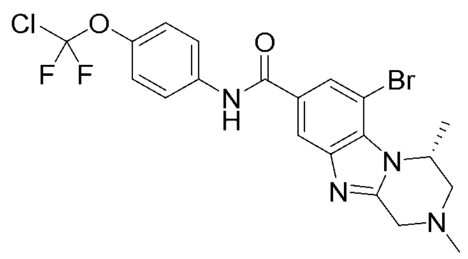

A-7

The following synthetic route was adopted:

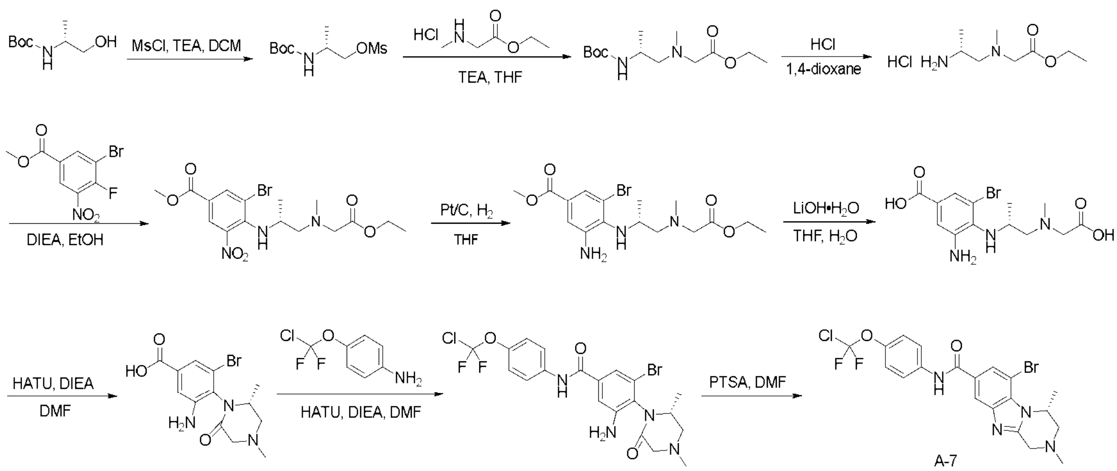

Step 1: Synthesis of compound (R)-2-((tert-butoxycarbonyl)amino)propyl methanesulfonate

N-Boc-D-alaninol (10.0 g, 57.1 mmol), triethylamine (8.6 g, 85.7 mmol) and anhydrous dichloromethane (100 mL) were added to a reaction flask. Methanesulfonyl chloride (7.2 g, 62.8 mmol) was added dropwise in an ice bath under nitrogen. After the addition was completed, the mixture was warmed to room temperature and reacted for 1 to 2 hours. The reaction was monitored by TLC until it was completed. 50 mL of water was added to quench the reaction. The resulting mixture was extracted with dichloromethane 3 to 4 times. The organic phases were combined, washed with saturated brine, concentrated, and then purified by silica gel column chromatography to give 13.1 g of an anhydrous oily liquid, with a yield of 91%. LC-MS (APCI): m/z=254.2 $(M+1)^+$.

Step 2: Synthesis of compound ethyl (R)-N-(2-((tert-butoxycarbonyl)amino)propyl)-N-methylglycinate

(R)-2-((tert-Butoxycarbonyl)amino)propyl methanesulfonate (13.1 g, 51.8 mmol) was dissolved in 80 mL of tetrahydrofuran, and 2-(methylamino)ethyl acetate hydrochloride (9.5 g, 62.1 mmol) was added. Triethylamine (10.5 g, 103.6 mmol) was slowly added dropwise at room temperature. After the addition was completed, the mixture was stirred and reacted at room temperature under nitrogen overnight. The reaction was monitored by TLC until it was completed. Water was added to quench the reaction. The resulting mixture was extracted with ethyl acetate 3 to 4 times. The organic phases were combined, washed with saturated brine, concentrated, and then purified by silica gel column chromatography to give 10.8 g of a colorless oily liquid, with a yield of 76%. LC-MS (APCI): m/z=275.7 $(M+1)^+$.

Step 3: Synthesis of compound ethyl (R)-N-(2-aminopropyl)-N-methylglycinate hydrochloride

Ethyl (R)-N-(2-((tert-butoxycarbonyl)amino)propyl)-N-methylglycinate (10.8 g, 39.4 mmol) and a 4 M solution of hydrogen chloride in 1,4-dioxane (80 mL, 320 mmol) were added to a reaction flask. The mixture was stirred and reacted at room temperature for 2 to 4 hours. The reaction was monitored by TLC until it was completed. The resulting mixture was concentrated, and then directly added to the next step reaction. LC-MS (APCI): m/z=175.2 $(M+1)^+$.

Step 4: Synthesis of compound methyl (R)-3-bromo-4-((1-((2-ethoxy-2-oxoethyl)(methyl)amino)propan-2-yl)amino)-5-nitrobenzoate

Ethyl (R)-N-(2-aminopropyl)-N-methylglycinate hydrochloride, methyl 3-bromo-4-fluoro-5-nitrobenzoate (11.3 g, 41 mmol), DIEA (13.2 g, 102.5 mmol) and anhydrous ethanol (60 mL) were added to a reaction flask. The mixture was heated to 60 °C, stirred and reacted under nitrogen for 6 to 8 hours. The reaction was monitored by TLC until it was completed. The mixture was concentrated, and then purified by silica gel column chromatography to give 9.3 g of a yellow oily liquid, with a two-step yield of 55%. LC-MS (APCI): m/z=432.5 $(M+1)^+$.

Step 5: Synthesis of compound methyl (R)-3-amino-5-bromo-4-((1-((2-ethoxy-2-oxoethyl)(methyl)amino)propan-2-yl) amino)benzoate

Methyl (R)-3-bromo-4-((1-((2-ethoxy-2-oxoethyl)(methyl)amino)propan-2-yl)amino)-5-nitrobenzoate (9.3 g, 21.6 mmol), tetrahydrofuran (70 mL) and a catalytic amount of Pt/C were added to a reaction flask. The reaction system was purged with hydrogen, and then the mixture was stirred and reacted at room temperature overnight. The reaction was monitored by TLC until it was completed. The mixture was filtered to remove the catalyst. The filtrate was concentrated, and then directly added to the next step reaction. LC-MS (APCI): m/z=402.3 $(M+1)^+$.

Step 6: Synthesis of compound (R)-3-amino-5-bromo-4-((1-((carboxymethyl)(methyl)amino)propan-2-yl)amino)benzoic acid

Methyl (R)-3-amino-5-bromo-4-((1-((2-ethoxy-2-oxoethyl)(methyl)amino)propan-2-yl)amino)benzoate and lithium hydroxide monohydrate (5.1 g, 121.5 mmol) were added to a reaction flask. The mixture was dissolved in tetrahydrofuran (30 mL) and water (30 mL). The mixture was stirred and reacted at room temperature overnight. The reaction was monitored by TLC until it was completed. The pH was adjusted to weakly acidic with 1N dilute hydrochloric acid. The mixture was extracted with ethyl acetate 3 to 4 times. The organic phases were combined, washed with saturated brine 3 times, concentrated, and then purified by silica gel column chromatography to give 4.2 g of an off-white solid, with a yield of 54%. LC-MS (APCI): m/z=360.6 $(M+1)^+$.

Step 7 Synthesis of compound (R)-3-amino-5-bromo-4-(2,4-dimethyl-6-oxopiperazin-1-yl)benzoic acid

(R)-3-Amino-5-bromo-4-((1-((carboxymethyl)(methyl)amino)propan-2-yl)amino)benzoic acid (2.9 g, 8.2 mmol), HATU (4.7 g, 12.3 mmol) and DIEA (2.1 g, 16.4 mmol) were added to a reaction flask. The mixture was dissolved in 25 mL of anhydrous DMF. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 2.1 g of a yellowish-brown solid, with a yield of 76%. LC-MS (APCI): m/z=342.5 $(M+1)^+$.

Step 8: Synthesis of compound (R)-3-amino-5-bromo-N-(4-(chlorodifluoromethoxy)phenyl)-4-(2,4-dimethyl-6-oxopiperazin-1-yl)benzamide

(R)-3-Amino-5-bromo-4-(2,4-dimethyl-6-oxopiperazin-1-yl)benzoic acid (2.8 g, 8.2 mmol), 4-(chlorodifluoromethoxy)aniline (1.9 g, 9.8 mmol), HATU (4.7 g, 12.3 mmol) and DIEA (2.1 g, 16.4 mmol) were added to a reaction flask, and the mixture was dissolved in 25 mL of anhydrous DMF. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 2.4 g of a light yellow solid, with a yield of 57%. LC-MS (APCI): m/z=517.3 $(M+1)^+$.

Step 9: Synthesis of intermediate compound A-7

(R)-3-Amino-5-bromo-N-(4-(chlorodifluoromethoxy)phenyl)-4-(2,4-dimethyl-6-oxopiperazin-1-yl)benzamide (1.5 g, 3.0 mmol), p-toluenesulfonic acid (258 mg, 1.5 mmol) and DMF (10 mL) were added to a reaction flask. The mixture was stirred and reacted at room temperature for 1 to 2 hours. The reaction was monitored by TLC until it was completed. Water was added to quench the reaction. The resulting mixture was extracted with ethyl acetate 3 to 4 times. The organic phases were combined, washed with saturated brine, concentrated, and then purified by silica gel column chromatography to give 1.22 g of product, with a yield of 82%. LC-MS (APCI): m/z=499.3 $(M+1)^+$.

**<u>Preparation of intermediate A-8 (R)-6-bromo-N-(4-(chlorodifluoromethoxy)phenyl)-4-methyl-3,4-dihydro-2H-benzo[4,5]imidazo[2,1-b][1,3]thiazine-8-carboxamide</u>**

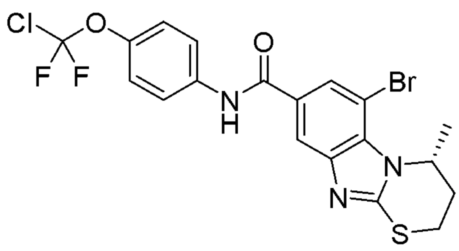

A-8

The following synthetic route was adopted:

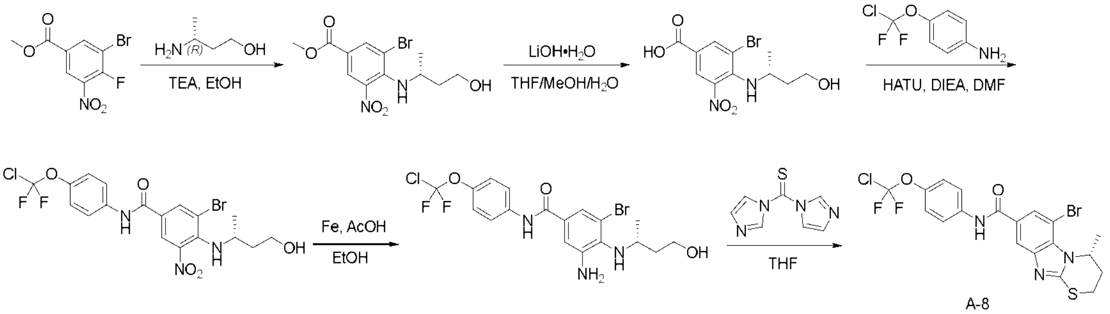


A-8

Step 1: Synthesis of compound methyl (R)-3-bromo-4-((4-hydroxybutan-2-yl)amino)-5-nitrobenzoate

Methyl 3-bromo-4-fluoro-5-nitrobenzoate (4.7 g, 16.9 mmol), (R)-3-aminobutanol (1.8 g, 20.3 mmol), triethylamine (2.7 g, 27.0 mmol) and ethanol (50 mL) were added to a reaction flask. The mixture was heated to *50* °C, stirred and reacted under nitrogen for 4 to 6 hours. The reaction was monitored by TLC until it was completed. The mixture was concentrated, and then purified by silica gel column chromatography, and vacuum dried to give 5.3 g of a light yellow solid as a product, with a yield of 90%. LC-MS (APCI): m/z=347.3 $(M+1)^+$.

Step 2: Synthesis of compound (R)-3-bromo-4-((4-hydroxybutan-2-yl)amino)-5-nitrobenzoic acid

Methyl (R)-3-bromo-4-((4-hydroxybutan-2-yl)amino)-5-nitrobenzoate (3.2 g, 9.3 mmol) and lithium hydroxide monohydrate (1.95 g, 46.5 mmol) were added to a reaction flask, and the mixture was dissolved in tetrahydrofuran (10 mL), methanol (10 mL) and water (10 mL). The mixture was stirred and reacted at room temperature overnight. The reaction was monitored by TLC until it was completed. The pH was adjusted to weakly acidic with 1N dilute hydrochloric acid. The resulting mixture was extracted with ethyl acetate 3 to 4 times. The organic phases were combined, washed with saturated brine 3 times, concentrated, and then purified by silica gel column chromatography to give 2.6 g of a white solid, with a yield of 85%. LC-MS (APCI): m/z=333.4 $(M+1)^+$.

Step 3: Synthesis of compound (R)-3-bromo-N-(4-(chlorodifluoromethoxy)phenyl)-4-((4-hydroxybutan-2-yl)amino)-5-nitrobenzamide

(R)-3-Bromo-4-((4-hydroxybutan-2-yl)amino)-5-nitrobenzoic acid (2.72 g, 8.2 mmol), 4-(chlorodifluoromethoxy) aniline (1.9 g, 9.8 mmol), HATU (4.7 g, 12.3 mmol) and DIEA (2.1 g, 16.4 mmol) were added to a reaction flask, and the mixture was dissolved in 25 mL of anhydrous DMF. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 2.5 g of a yellow solid, with a yield of 61%. LC-MS (APCI): m/z=508.2 $(M+1)^+$.

Step 4: Synthesis of compound (R)-3-amino-5-bromo-N-(4-(chlorodifluoromethoxy)phenyl)-4-((4-hydroxybutan-2-yl) amino)benzamide

(R)-3-Bromo-N-(4-(chlorodifluoromethoxy)phenyl)-4-((4-hydroxybutan-2-yl)amino)-5-nitrobenzamide (7.2 g, 14.2 mmol), reduced iron powder (8.0 g, 142 mmol), ethanol (40 mL) and glacial acetic acid (2 mL) were added to a reaction flask. After the addition was completed, the mixture was heated to 70 °C, stirred and reacted for 2 h. The reaction was monitored by TLC until it was completed. The mixture was filtered while hot. The filtrate was concentrated, and then directly added to the next step reaction. LC-MS (APCI): m/z=478.4 $(M+1)^+$.

Step 5: Synthesis of intermediate compound A-8

(R)-3-Amino-5-bromo-N-(4-(chlorodifluoromethoxy)phenyl)-4-((4-hydroxybutan-2-yl)amino)benzamide, N,N'-thiocarbonyldiimidazole (2.9 g, 16.4 mmol) and anhydrous THF (25 mL) were added to a reaction flask. The mixture was stirred and reacted at room temperature under nitrogen overnight. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 2.63 g of a light yellow solid, with a yield of 37%. LC-MS (APCI): m/z=502.3 $(M+1)^+$.

**Preparation of intermediate A-9 (R)-6-bromo-N-(4-(chlorodifluoromethoxy)phenyl)-4-methyl-3,4-dihydro-1H-benzo[4,5]imidazo[2,1-c][1,4]oxazine-8-carboxamide**

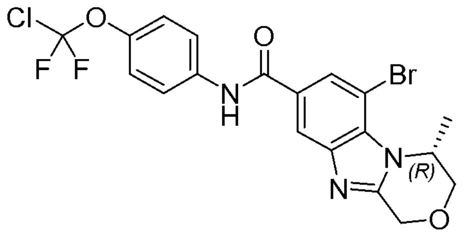

A-9

The following synthetic route was adopted:

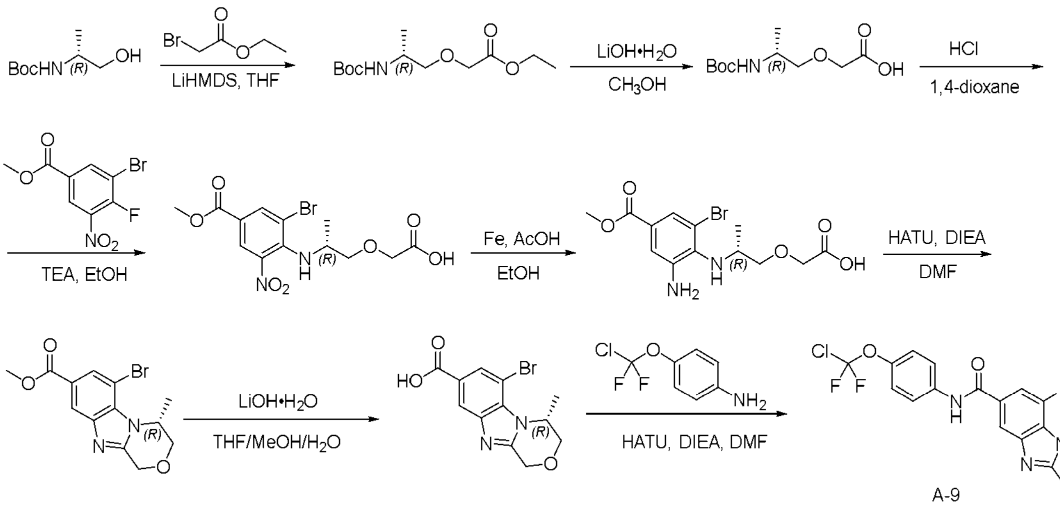


A-9

Step 1: Synthesis of compound ethyl (R)-2-(2-((tert-butoxycarbonyl)amino)propoxy)acetate

N-Boc-D-alaninol (5.0 g, 28.6 mmol), ethyl 2-bromoacetate (5.7 g, 34.3 mmol) and anhydrous THF (20 mL) were added to a reaction flask. The mixture was cooled to 0 °C under nitrogen, and LiHMDS (43 mL, 42.9 mmol) was slowly added dropwise. After the addition was completed, the mixture was warmed to room temperature, stirred and reacted overnight. The reaction was monitored by TLC until it was completed. Saturated aqueous solution of ammonium chloride was added to quench the reaction. The mixture was extracted with ethyl acetate 3 to 4 times. The organic phases were combined, washed with saturated brine, concentrated, and then purified by silica gel column chromatography to give 5.1 g of a colorless oily liquid, with a yield of 68%. LC-MS(APCI): m/z=262.1 $(M+1)^+$.

Step 2: Synthesis of compound (R)-2-(2-((tert-butoxycarbonyl)amino)propoxy)acetic acid

Ethyl (R)-2-(2-((tert-butoxycarbonyl)amino)propoxy)acetate (5.1 g, 19.5 mmol), lithium hydroxide monohydrate (4.1 g, 97.5 mmol) and methanol (20 mL) were added to a reaction flask. The mixture was stirred and reacted at room temperature overnight. The reaction was monitored by TLC until it was completed. The pH was adjusted to weakly acidic with 1N dilute hydrochloric acid. The resulting mixture was extracted with ethyl acetate 3 to 4 times. The organic phases were combined, washed with saturated brine 3 times, concentrated, and then purified by silica gel column chromatography to give 3.95 g of a light yellow oily solid, with a yield of 87%. LC-MS (APCI): m/z=234.6 $(M+1)^+$.

Step 3: Synthesis of compound (R)-2-(2-((2-bromo-4-(methoxycarbonyl)-6-nitrophenyl)amino)propoxy)acetic acid

(R)-2-(2-((tert-Butoxycarbonyl)amino)propoxy)acetic acid (3.95 g, 16.9 mmol) and a 4M solution of hydrochloride in 1,4-dioxane (20 mL, 80 mmol) were added to a reaction flask. The mixture was stirred and reacted at room temperature for 1 hour. The reaction was monitored by TLC until it was completed. The resulting mixture was concentrated to remove the solvent. Methyl 3-bromo-4-fluoro-5-nitrobenzoate (4.7 g, 16.9 mmol), triethylamine (2.7 g, 27.0 mmol) and ethanol (50 mL) were added to a reaction flask. The mixture was heated to *50* °C, stirred and reacted under nitrogen for 4 to 6 hours. The reaction was monitored by TLC until it was completed. The resulting mixture was concentrated to remove the solvent. The residue was purified by column chromatography, and vacuum dried to give 5.3 g of a light yellow solid as a product, with a yield of 81%. LC-MS (APCI): m/z=391.3 $(M+1)^+$.

Step 4: Synthesis of compound (R)-2-(2-((2-amino-6-bromo-4-(methoxycarbonyl)phenyl)amino)propoxy)acetic acid

(R)-2-(2-((2-Bromo-4-(methoxycarbonyl)-6-nitrophenyl)amino)propoxy)acetic acid (5.5 g, 14.2 mmol), reduced iron powder (8.0 g, 142 mmol), ethanol (30 mL) and glacial acetic acid (2 mL) were added to a reaction flask. After the addition was completed, the mixture was heated to 70 °C, stirred and reacted for 2 h. The reaction was monitored by TLC until it was completed. The mixture was filtered while hot. The filtrate was concentrated, and then directly added to the next step reaction. LC-MS (APCI): m/z=361.4 $(M+1)^+$.

Step 5: Synthesis of compound methyl (R)-6-bromo-4-methyl-3,4-dihydro-1H-benzo[4,5]imidazo[2,1-c][1,4]oxazine-8-carboxylate

(R)-2-(2-((2-Amino-6-bromo-4-(methoxycarbonyl)phenyl)amino)propoxy)acetic acid (2.95 g, 8.2 mmol), HATU (4.7 g, 12.3 mmol) and DIEA (2.1 g, 16.4 mmol) were added to a reaction flask, and the mixture was dissolved in 25 mL of anhydrous DMF. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 1.54 g of a yellow solid, with a yield of 58%. LC-MS (APCI): m/z=325.2 $(M+1)^+$.

Step 6: Synthesis of compound (R)-6-bromo-4-methyl-3,4-dihydro-1H-benzo[4,5]imidazo[2,1-c][1,4]oxazine-8-carboxylic acid

Methyl (R)-6-bromo-4-methyl-3,4-dihydro-1H-benzo[4,5]imidazo[2,1-c][1,4]oxazine-8-carboxylate (3.0 g, 9.3 mmol) and lithium hydroxide monohydrate (1.95 g, 46.5 mmol) were added to a reaction flask, and the mixture was dissolved in tetrahydrofuran (10 mL), methanol (10 mL) and water (10 mL). The mixture was stirred and reacted at room temperature overnight. The reaction was monitored by TLC until it was completed. The pH was adjusted to weakly acidic with 1N dilute hydrochloric acid. The resulting mixture was extracted with ethyl acetate 3 to 4 times. The organic phases were combined, washed with saturated brine 3 times, concentrated, and then purified by silica gel column chromatography to give 2.3 g of a white solid, with a yield of 80%. LC-MS (APCI): m/z=311.6 $(M+1)^+$.

Step 7: Synthesis of intermediate compound A-9

(R)-6-Bromo-4-methyl-3,4-dihydro-1H-benzo[4,5]imidazo[2,1-c][1,4]oxazine-8-carboxylic acid (2.54 g, 8.2 mmol), 4-(chlorodifluoromethoxy)aniline (1.9 g, 9.8 mmol), HATU (4.7 g, 12.3 mmol) and DIEA (2.1 g, 16.4 mmol) were added to a reaction flask, and the mixture was dissolved in 25 mL of anhydrous DMF. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 2.74 g of a yellow solid, with a yield of 69%. LC-MS (APCI): m/z=486.6 $(M+1)^+$.

**Preparation of intermediate A-10 tert-butyl (R)-10-bromo-8-((4-(chlorodifluoromethoxy)phenyl)carbamoyl)-1-methyl-1,2,4,5-tetrahydro-3H-benzo[4,5]imidazo[1,2-d][1,4]diazepine-3-carboxylate**

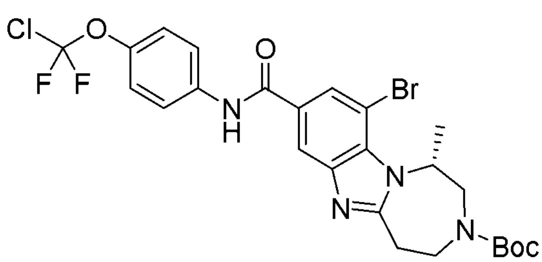

A-10

The following synthetic route was adopted:

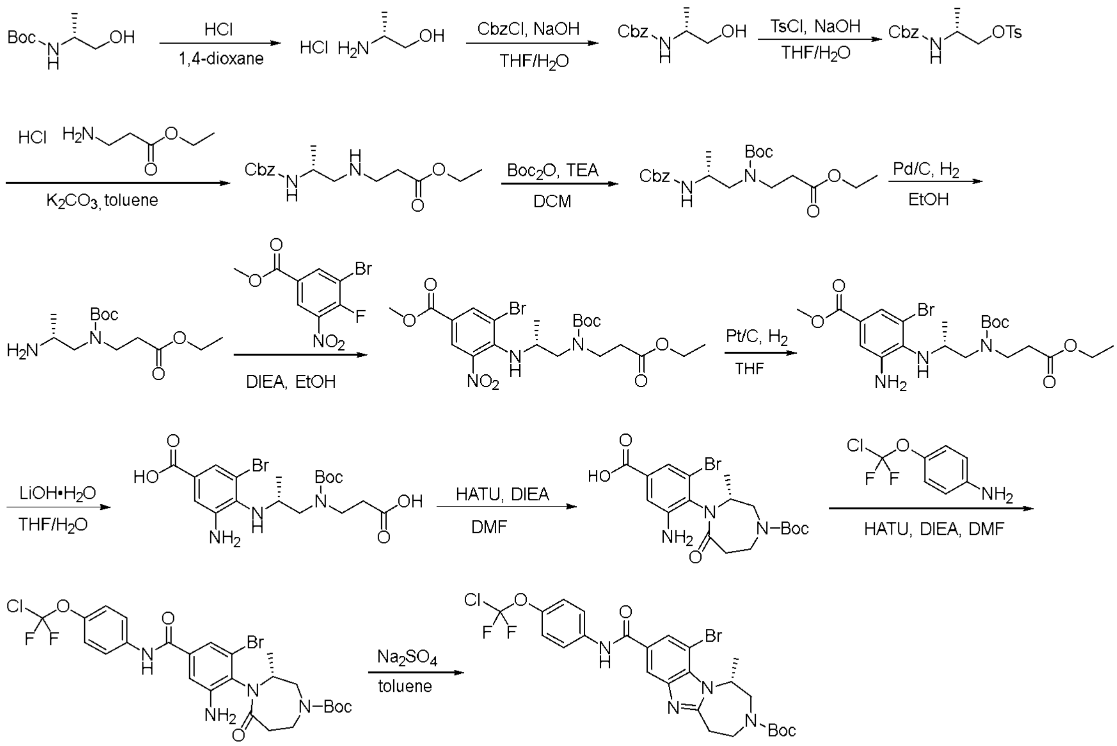


A-10

Step 1: Synthesis of compound (R)-2-aminopropan-1-ol hydrochloride

N-Boc-D-alaninol (10.0 g, 57.1 mmol) and a 4 M solution of hydrogen chloride in 1,4-dioxane (80 mL, 320 mmol) were added to a reaction flask. The mixture was stirred and reacted at room temperature for 4 to 6 hours. The reaction was monitored by TLC until it was completed. The mixture was concentrated to remove the solvent, and directly added to the next step reaction. LC-MS (APCI): m/z=76.1 $(M+1)^+$.

Step 2: Synthesis of compound benzyl (R)-(1-hydroxypropan-2-yl)carbamate

(R)-2-Aminopropan-1-ol hydrochloride, tetrahydrofuran (60 mL) and water (40 mL) were added to a reaction flask. Sodium hydroxide (5.7 g, 143 mmol) was added in batches in an ice bath. After the addition was completed, benzyl chloroformate (11.6 g, 68.5 mmol) was slowly added dropwise under nitrogen. After the addition was completed, the mixture was warmed to room temperature, stirred and reacted overnight. The reaction was monitored by TLC until it was completed. The layers were separated. The aqueous phase was extracted with ethyl acetate 3 to 4 times. The organic phases were combined, washed with saturated brine, concentrated, and then purified by silica gel column chromatography to give 10.4 g of a colorless oily liquid, with a yield of 87%. LC-MS (APCI): m/z=210.1 $(M+1)^+$.

Step 3: Synthesis of compound (R)-2-(((benzyloxy)carbonyl)amino)propyl-4-methylbenzenesulfonate

Benzyl (R)-(1-hydroxypropan-2-yl)carbamate (10.4 g, 49.8 mmol), tetrahydrofuran (60 mL) and water (40 mL) were added to a reaction flask. Sodium hydroxide (5.7 g, 143 mmol) was added in batches in an ice bath. After the addition was completed, p-toluenesulfonyl chloride (13.1 g, 68.5 mmol) was added in batches under nitrogen. After the addition was completed, the mixture was warmed to room temperature, stirred and reacted overnight. The reaction was monitored by TLC until it was completed. The layers were separated, and the aqueous phase was extracted with ethyl acetate 3 to 4 times. The organic phases were combined, washed with saturated brine, concentrated, and then purified by silica gel column chromatography to give 13.0 g of a light yellow oily liquid, with a yield of 72%. LC-MS (APCI): m/z=364.2 $(M+1)^+$.

Step 4: Synthesis of compound ethyl (R)-3-((2-(((benzyloxy)carbonyl)amino)propyl)amino)propionate

(R)-2-(((Benzyloxy)carbonyl)amino)propyl-4-methylbenzenesulfonate (13.0 g, 35.8 mmol), ethyl 3-aminopropionate hydrochloride (6.6 g, 43 mmol), potassium carbonate (9.9 g, 71.6 mmol) and toluene (90 mL) were added to a reaction flask. The mixture was heated to 90 °C, stirred and reacted under nitrogen for 6 to 8 hours. The reaction was

monitored by TLC until it was completed. The mixture was concentrated to remove the solvent, and purified by silica gel column chromatography to give 5.84 g of a yellow oily liquid, with a yield of 53%. LC-MS (APCI): m/z=309.6 $(M+1)^+$.

Step 5: Synthesis of compound ethyl (R)-3-((2-(((benzyloxy)carbonyl)amino)propyl)(tert-butoxycarbonyl)amino)propionate

Ethyl (R)-3-((2-(((benzyloxy)carbonyl)amino)propyl)amino)propionate (5.84 g, 19 mmol), di-tert-butyl dicarbonate (6.2 g, 28.4 mmol) and triethylamine (3.84 g, 38 mmol) were added to a reaction flask, and the mixture was dissolved in 60 mL of dichloromethane. The mixture was stirred and reacted at room temperature for 5 hours. The reaction was monitored by TLC until it was completed. Water was added to quench the reaction. The organic phase was separated, washed with saturated brine, concentrated, and then purified by silica gel column chromatography to give 6.74 g of a yellow oily liquid, with a yield of 87%. LC-MS (APCI): m/z=409.6 $(M+1)^+$.

Step 6: Synthesis of compound ethyl (R)-3-((2-aminopropyl)(tert-butyloxycarbonyl)amino)propionate

Ethyl (R)-3-((2-(((benzyloxy)carbonyl)amino)propyl)(tert-butyloxycarbonyl)amino)propionate (6.74 g, 16.5 mmol), ethanol (50 mL), and a catalytic amount of Pd/C were added to a reaction flask. The reaction system was purged with hydrogen, and then the mixture was stirred and reacted at room temperature overnight. The reaction was monitored by TLC until it was completed. The mixture was filtered to remove the catalyst. The filtrate was concentrated, and then directly added to the next step reaction. LC-MS (APCI): m/z=275.8 $(M+1)^+$.

Step 7 Synthesis of compound methyl (R)-3-bromo-4-((1-((tert-butoxycarbonyl)(3-ethoxy-3-oxopropyl)amino)propan-2-yl)amino)-5-nitrobenzoate

Ethyl (R)-3-((2-aminopropyl)(tert-butyloxycarbonyl)amino)propionate, methyl 3-bromo-4-fluoro-5-nitrobenzoate (5.0 g, 18.1 mmol), DIEA (4.2 g, 33 mmol) and anhydrous ethanol (50 mL) were added to a reaction flask. The mixture was heated to 60 °C, stirred and reacted under nitrogen for 6 to 8 hours. The reaction was monitored by TLC until it was completed. The mixture was concentrated to remove the solvent, and purified by silica gel column chromatography to give 5.4 g of a yellow oily liquid, with a two-step yield of 62%. LC-MS (APCI): m/z=532.5 $(M+1)^+$.

Step 8: Synthesis of compound methyl (R)-3-amino-5-bromo-4-((1-((tert-butoxycarbonyl)(3-ethoxy-3-oxopropyl)amino)propan-2-yl)amino)benzoate

Methyl (R)-3-bromo-4-((1-((tert-butyloxycarbonyl)(3-ethoxy-3-oxopropyl)amino)propan-2-yl)amino)-5-nitrobenzoate (5.4 g, 10.3 mmol), tetrahydrofuran (70 mL) and a catalytic amount of Pt/C were added to a reaction flask. The reaction system was purged with hydrogen, and then the mixture was stirred and reacted at room temperature overnight. The reaction was monitored by TLC until it was completed. The mixture was filtered to remove the catalyst. The filtrate was concentrated, and then directly used for the next step reaction. LC-MS (APCI): m/z=502.3 $(M+1)^+$.

Step 9: Synthesis of compound (R)-3-amino-5-bromo-4-((1-(tert-butoxycarbonyl)(2-carboxyethyl)amino)propan-2-yl)amino)benzoic acid

Methyl (R)-3-amino-5-bromo-4-((1-((tert-butyloxycarbonyl)(3-ethoxy-3-oxopropyl)amino)propan-2-yl)amino)benzoate and lithium hydroxide monohydrate (5.1 g, 121.5 mmol) were added to a reaction flask, and the mixture was dissolved in tetrahydrofuran (30 mL) and water (30 mL). The mixture was stirred and reacted at room temperature overnight. The reaction was monitored by TLC until it was completed. The pH was adjusted to weakly acidic with 1N dilute hydrochloric acid. The resulting mixture was extracted with ethyl acetate 3 to 4 times. The organic phases were combined, washed with saturated brine 3 times, concentrated to remove the solvent, and purified by silica gel column chromatography to give 3.4 g of a white solid, with a yield of 71%. LC-MS (APCI): m/z=460.6 $(M+1)^+$.

Step 10 Synthesis of compound (R)-3-amino-5-bromo-4-(4-(tert-butoxycarbonyl)-2-methyl-7-oxo-1,4-diazepan-1-yl)benzoic acid

(R)-3-Amino-5-bromo-4-((1-(tert-butoxycarbonyl)(2-carboxyethyl)amino)propan-2-yl)amino)benzoic acid (3.8 g, 8.2 mmol), HATU (4.7 g, 12.3 mmol) and DIEA (2.1 g, 16.4 mmol) were added to a reaction flask, and the mixture was dissolved in anhydrous DMF (40 mL). The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3

times, concentrated, and then purified by silica gel column chromatography to give 2.4 g of a yellow solid, with a yield of 66%. LC-MS (APCI): m/z=442.3 $(M+1)^+$.

Step 11 Synthesis of compound tert-butyl (R)-4-(2-amino-6-bromo-4-((4-(chlorodifluoromethoxy)phenyl)carbamoyl) phenyl)-3-methyl-5-oxo-1,4-diazepane-1-carboxylate

(R)-3-Amino-5-bromo-4-(4-(tert-butoxycarbonyl)-2-methyl-7-oxo-1,4-diazepan-1-yl)benzoic acid (3.6 g, 8.2 mmol), 4-(chlorodifluoromethoxy)aniline (1.9 g, 9.8 mmol), HATU (4.7 g, 12.3 mmol) and DIEA (2.1 g, 16.4 mmol) were added to a reaction flask, and the mixture was dissolved in anhydrous DMF (25 mL). The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 2.42 g of a light yellow solid, with a yield of 48%. LC-MS (APCI): m/z=617.3 $(M+1)^+$.

Step 12 Synthesis of intermediate compound A-10

tert-Butyl (R)-4-(2-amino-6-bromo-4-((4-(chlorodifluoromethoxy)phenyl)carbamoyl)phenyl)-3-methyl-5-oxo-1,4-diazepane-1-carboxylate (1.85 g, 3.0 mmol), anhydrous sodium sulfate (430 mg, 3.0 mmol) and toluene (10 mL) were added to a reaction flask. The mixture was heated to 70 °C, stirred and reacted for 1 to 2 hours. The reaction was monitored by TLC until it was completed. Water was added to quench the reaction. The resulting mixture was extracted with ethyl acetate 3 to 4 times. The organic phases were combined, washed with saturated brine, concentrated, and then purified by silica gel column chromatography to give 1.63 g of product, with a yield of 91%. LC-MS (APCI): m/z=599.7 $(M+1)^+$.

**<u>Preparation of intermediate A-11 (R)-7-bromo-N-(4-(chlorodifluoromethoxy)phenyl)-5-methyl-4,5-dihydro-1H,3H-benzo[4,5]imidazo[2,1-c][1,4]oxazepine-9-carboxamide</u>**

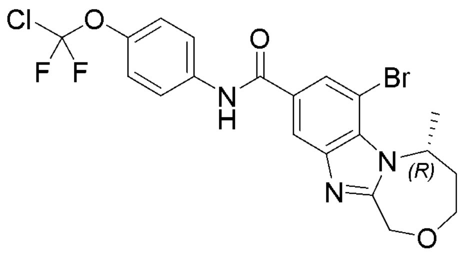

A-11

The following synthetic route was adopted:

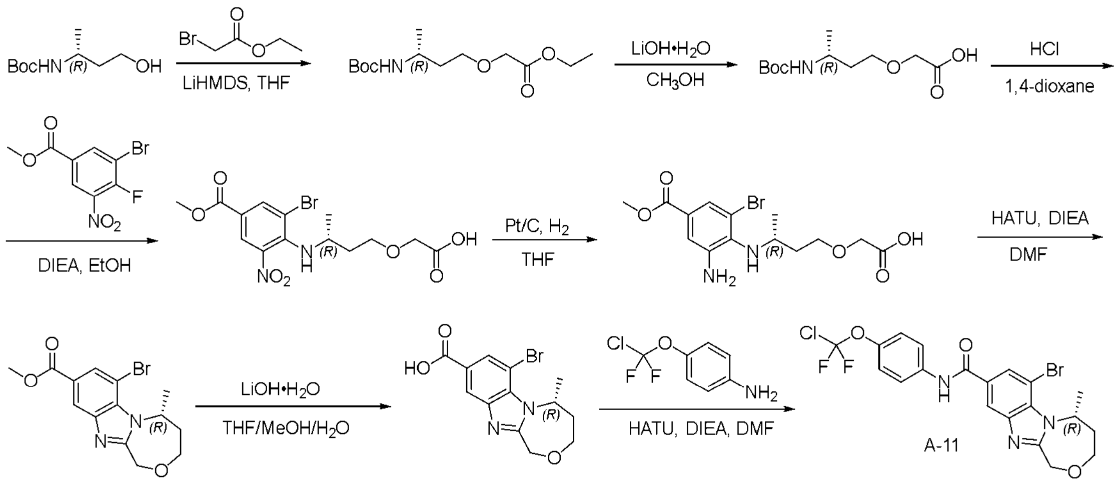

Step 1: Synthesis of compound ethyl (R)-2-(3-((tert-butoxycarbonyl)amino)butoxy)acetate

tert-Butyl (R)-(4-hydroxybutan-2-yl)carbamate (5.0 g, 26.4 mmol), ethyl 2-bromoacetate (6.6 g, 39.7 mmol) and anhydrous tetrahydrofuran (40 mL) were added to a reaction flask. LiHMDS (29 mL, 29.0 mmol) was slowly added dropwise under nitrogen in an ice bath. After the addition was completed, the mixture was warmed to room temperature, stirred and reacted for 6 to 8 hours. The reaction was monitored by TLC until it was completed. Saturated aqueous solution of ammonium chloride was added to quench the reaction. The resulting mixture was extracted with ethyl acetate 3 to 4 times. The organic phases were combined, washed with saturated brine, concentrated, and then purified by silica gel column chromatography to give 4.3 g of a light yellow oily liquid, with a yield of 59%. LC-MS (APCI): m/z=276.5 $(M+1)^+$.

Step 2: Synthesis of compound (R)-2-(3-((tert-butoxycarbonyl)amino)butoxy)acetic acid

Ethyl (R)-2-(3-((tert-butoxycarbonyl)amino)butoxy)acetate (4.3 g, 15.6 mmol), lithium hydroxide monohydrate (3.3 g, 78.2 mmol) and methanol (30 mL) were added to a reaction flask. The mixture was stirred and reacted at room temperature overnight. The reaction was monitored by TLC until it was completed. The pH was adjusted to weakly acidic with 1N dilute hydrochloric acid. The resulting mixture was extracted with ethyl acetate 3 to 4 times. The organic phases were combined, washed with saturated brine 3 times, concentrated to remove the solvent, and purified by silica gel column chromatography to give 2.9 g of a light yellow oily liquid, with a yield of 75%. LC-MS (APCI): m/z=248.1 $(M+1)^+$.

Step 3: Synthesis of compound (R)-2-(3-((2-bromo-4-(methoxycarbonyl)-6-nitrophenyl)amino)butoxy)acetic acid

(R)-2-(3-((tert-Butoxycarbonyl)amino)butoxy)acetic acid (2.9 g, 11.7 mmol) and a 4 M solution of hydrogen chloride in 1,4-dioxane (30 mL, 120 mmol) were added to a reaction flask. The mixture was stirred and reacted at room temperature for 1 to 2 hours. The reaction was monitored by TLC until it was completed. The mixture was concentrated to remove the solvent. Methyl 3-bromo-4-fluoro-5-nitrobenzoate (3.88 g, 14.0 mmol), DIEA (4.2 g, 33 mmol) and anhydrous ethanol (50 mL) were added. The mixture was heated to 60 °C, stirred and reacted under nitrogen for 6 to 8 hours. The reaction was monitored by TLC until it was completed. The mixture was concentrated to remove the solvent, and purified by silica gel column chromatography to give 2.2 g of a yellow oily liquid, with a two-step yield of 47%. LC-MS (APCI): m/z=405.5 $(M+1)^+$.

Step 4: Synthesis of compound (R)-2-(3-((2-amino-6-bromo-4-(methoxycarbonyl)phenyl)amino)butoxy)acetic acid

(R)-2-(3-((2-Bromo-4-(methoxycarbonyl)-6-nitrophenyl)amino)butoxy)acetic acid (2.2 g, 5.4 mmol), tetrahydrofuran (40 mL) and a catalytic amount of Pt/C were added to a reaction flask. The reaction system was purged with hydrogen, and then the mixture was stirred and reacted at room temperature overnight. The reaction was monitored by TLC until it was completed. The mixture was filtered to remove the catalyst. The filtrate was concentrated, and then directly added to the next step reaction. LC-MS (APCI): m/z=375.3 $(M+1)^+$.

Step 5: Synthesis of compound methyl (R)-7-bromo-5-methyl-4,5-dihydro-1H,3H-benzo[4,5]imidazo[2,1-c][1,4]oxazepine-9-carboxylate

(R)-2-(3-((2-Amino-6-bromo-4-(methoxycarbonyl)phenyl)amino)butoxy)acetic acid, HATU (3.1 g, 8.1 mmol), DIEA (1.4 g, 10.8 mmol) and anhydrous DMF (20 mL) were added to a reaction flask. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated to remove the solvent, and purified by silica gel column chromatography to give 1.3 g of a light yellow solid, with a yield of 71%. LC-MS (APCI): m/z=339.6 $(M+1)^+$.

Step 6: Synthesis of compound (R)-7-bromo-5-methyl-4,5-dihydro-1H,3H-benzo[4,5]imidazo[2,1-c][1,4]oxazepine-9-carboxylic acid

Methyl (R)-7-bromo-5-methyl-4,5-dihydro-1H,3H-benzo[4,5]imidazo[2,1-c][1,4]oxazepine-9-carboxylate (1.3 g, 3.8 mmol) and lithium hydroxide monohydrate (0.8 g, 19.2 mmol) were added to a reaction flask, and the mixture was dissolved in 5 mL of tetrahydrofuran, 5 mL of methanol and 5 mL of water. The mixture was stirred and reacted at room temperature overnight. The reaction was monitored by TLC until it was completed. The pH was adjusted to weakly acidic with 1N dilute hydrochloric acid. The resulting mixture was extracted with ethyl acetate 3 to 4 times. The organic phases were combined, washed with saturated brine 3 times, concentrated to remove the solvent, and purified by silica gel column chromatography to give 1.06 g of an off-white solid, with a yield of 86%. LC-MS (APCI): m/z=325.6 $(M+1)^+$.

Step 7: Synthesis of intermediate compound A-11

(R)-7-Bromo-5-methyl-4,5-dihydro-1H,3H-benzo[4,5]imidazo[2,1-c][1,4]oxazepine-9-carboxylic acid (266 mg, 0.82 mmol), 4-(chlorodifluoromethoxy)aniline (190 mg, 0.98 mmol), HATU (0.47 g, 1.23 mmol) and DIEA (210 mg, 1.64 mmol) were added to a reaction flask, and the mixture was dissolved in 8 mL of anhydrous DMF. The mixture was stirred and reacted at room temperature under nitrogen for 5 to 6 h. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 196 mg of a yellow solid, with a yield of 48%. LC-MS (APCI): m/z=500.3 $(M+1)^+$.

**Preparation of intermediate A-12 (R)-2-amino-5-bromo-N-(4-(chlorodifluoromethoxy)phenyl)-6-(3-fluoropyrrolidin-1-yl)nicotinamide**

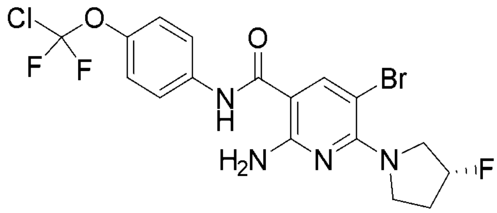

A-12

The following synthetic route was adopted:

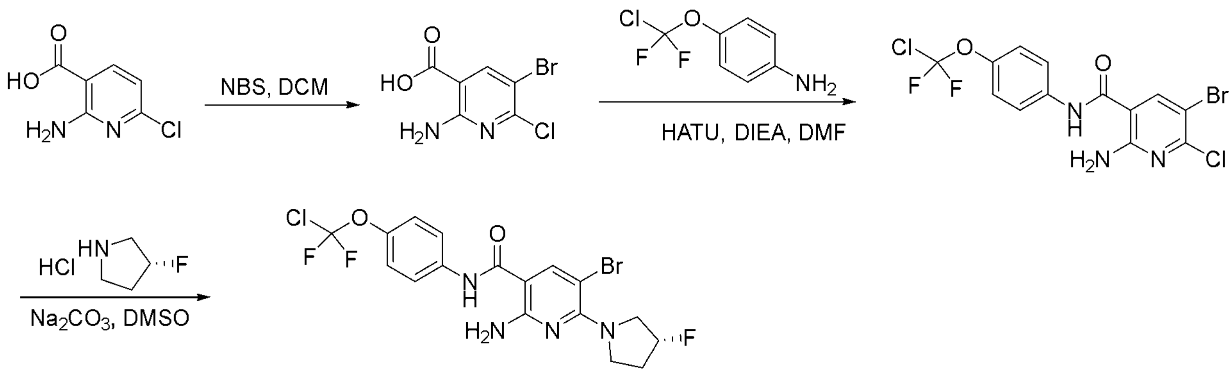


A-12

Step 1: Synthesis of compound 2-amino-5-bromo-6-chloronicotinic acid

2-Amino-6-chloronicotinic acid (5.0 g, 29.1 mmol) and dichloromethane (50 mL) were added to a reaction flask. NBS (6.21 g, 34.9 mmol) was added under nitrogen. The mixture was stirred and reacted at room temperature for 4 to 6 hours. The reaction was monitored by TLC until it was completed. The mixture was concentrated to remove the solvent. 80 mL of water was added, and the mixture was slurried for 1 hour. The mixture was filtered. The filter cake was vacuum dried to give 5.9 g of product, with a yield of 81%. LC-MS (APCI): m/z=251.2 $(M+1)^+$.

Step 2: Synthesis of compound 2-amino-5-bromo-6-chloro-N-(4-(chlorodifluoromethoxy)phenyl)nicotinamide

2-Amino-5-bromo-6-chloronicotinic acid (5.9 g, 23.6 mmol), 4-(chlorodifluoromethoxy)aniline (5.5 g, 28.3 mmol) and 40 mL of anhydrous DMF were added to a reaction flask. HATU (13.4 g, 35.4 mmol) and DIEA (6.1 g, 47.2 mmol) were added in an ice bath. The mixture was stirred and reacted at room temperature for 12 hours. The reaction was monitored by TLC until it was completed. The mixture was diluted with added water. The resulting mixture was extracted with ethyl acetate 3 to 4 times. The organic phases were combined, washed with saturated brine, concentrated, and then purified by silica gel column chromatography to give 6.5 g of a yellow solid, with a yield of 65%. LC-MS (APCI): m/z=425.8 $(M+1)^+$.

Step 3: Synthesis of intermediate compound A-11

2-Amino-5-bromo-6-chloro-N-(4-(chlorodifluoromethoxy)phenyl)nicotinamide (3.0 g, 7.06 mmol), (R)-3-fluoropyrrolidine hydrochloride (0.9 g, 7.06 mmol) and anhydrous sodium carbonate (1.5 g, 14.1 mmol) were added to a reaction flask. 20 mL of DMSO was added under nitrogen. The mixture was heated to 70 °C, stirred and reacted for 6 to 8 hours. The reaction was monitored by TLC until it was completed. Water was added to quench the reaction. The resulting mixture was extracted with ethyl acetate 3 to 4 times. The organic phases were combined, concentrated, and then purified by silica gel column chromatography to give 3.1 g of a light yellow solid, with a yield of 91%. LC-MS (APCI): m/z=479.3 $(M+1)^+$.

**Preparation of intermediate B-1 6-methyl-3-(4,4,5,5-tetramethyl-1,3,2-dioxaborolan-2-yl)-6,7-dihydro-5H-pyrrolo[3,4-b]pyridin-5-one**

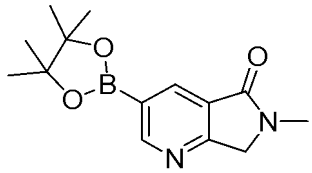

B-1

The following synthetic route was adopted:

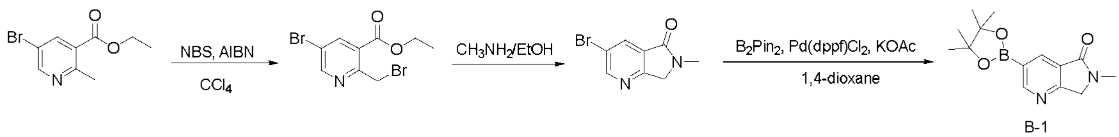


Step 1: Synthesis of compound ethyl 5-bromo-2-(bromomethyl)nicotinate

Ethyl 2-methyl-5-bromonicotinate (5.0 g, 20.6 mmol), NBS (3.8 g, 21.6 mmol) and carbon tetrachloride (50 mL) were added to a reaction flask. AIBN (0.7 g, 4.1 mmol) was added under nitrogen. The mixture was heated to 50 °C, stirred and reacted overnight. The reaction was monitored by TLC until it was completed. The mixture was concentrated to remove the solvent. The residue was purified by column chromatography, and vacuum dried to give 5.3 g of a light yellow liquid as a product, with a yield of 81%. LC-MS (APCI): m/z=321.8 $(M+1)^+$.

Step 2: Synthesis of compound 3-bromo-6-methyl-6,7-dihydro-5H-pyrrolo[3,4-b]pyridin-5-one

Ethyl 5-bromo-2-(bromomethyl)nicotinate (3.2 g, 10 mmol), an an aqueous solution of methylamine (0.37 g, 12 mmol) and ethanol (10 mL) were added to a reaction flask. The mixture was heated to 60 °C, stirred and reacted overnight. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 2.0 g of a white solid, with a yield of 89%. LC-MS (APCI): m/z=227.1 $(M+1)^+$.

Step 3: Synthesis of intermediate compound B-1

3-Bromo-6-methyl-6,7-dihydro-5H-pyrrolo[3,4-b]pyridin-5-one (0.7 g, 3.1 mmol), $B_2Pin_2$ (2.4 g, 9.3 mmol), Pd(dppf)$Cl_2$ (117 mg, 0.16 mmol) and potassium acetate (0.6 g, 6.2 mmol) were added to a reaction flask, and the mixture was dissolved in 10 mL of anhydrous 1,4-dioxane. The mixture was microwaved and heated to 100 °C, and reacted for 2 hours. The reaction was monitored by TLC until it was completed. The mixture was concentrated, and then purified by silica gel column chromatography to give 569 mg of product, with a yield of 67%. LC-MS (APCI): m/z=275.7 $(M+1)^+$.

**Preparation of intermediate B-2 6-cyclopropyl-3-(4,4,5,5-tetramethyl-1,3,2-dioxaborolan-2-yl)-6,7-dihydro-5H-pyrrolo[3,4-b]pyridin-5-one**

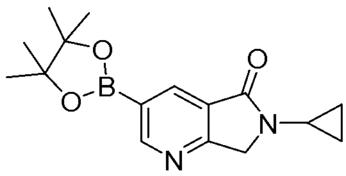

B-2

The following synthetic route was adopted:

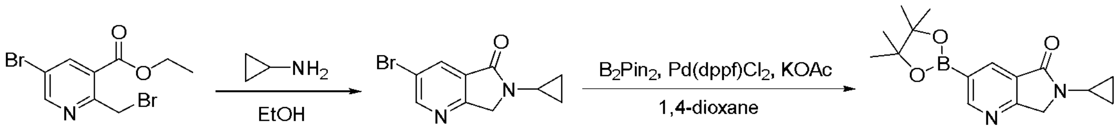


Step 1: Synthesis of compound 3-bromo-6-cyclopropyl-6,7-dihydro-SH-pyrrolo[3,4-b]pyridin-5-one

Ethyl 5-bromo-2-(bromomethyl)nicotinate (3.2 g, 10 mmol) and cyclopropylamine (0.68 g, 12 mmol) were added to a reaction flask, and the mixture was dissolved in 10 mL of ethanol. The mixture was heated to 60 °C, stirred and reacted overnight. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 2.1 g of a white solid, with a yield of 82%. LC-MS (APCI): m/z=253.1 $(M+1)^+$.

Step 2: Synthesis of intermediate compound B-2

3-Bromo-6-cyclopropyl-6,7-dihydro-5H-pyrrolo[3,4-b]pyridin-5-one (0.78 g, 3.1 mmol), $B_2Pin_2$ (2.4 g, 9.3 mmol), $Pd(dppf)Cl_2$ (117 mg, 0.16 mmol) and potassium acetate (0.6 g, 6.2 mmol) were added to a reaction flask, and the mixture was dissolved in 10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 623 mg of product, with a yield of 67%. LC-MS (APCI): m/z=301.7 $(M+1)^+$.

**Preparation of intermediate B-3 (2-(tetrahydro-2H-pyran-2-yl)-2,4-dihydropyrazolo[3',4':3,4]cyclopenta[1,2-b]pyridin-7-yl)boronic acid**

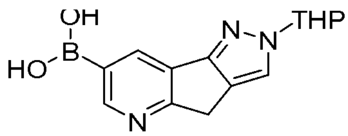

B-3

The following synthetic route was adopted:

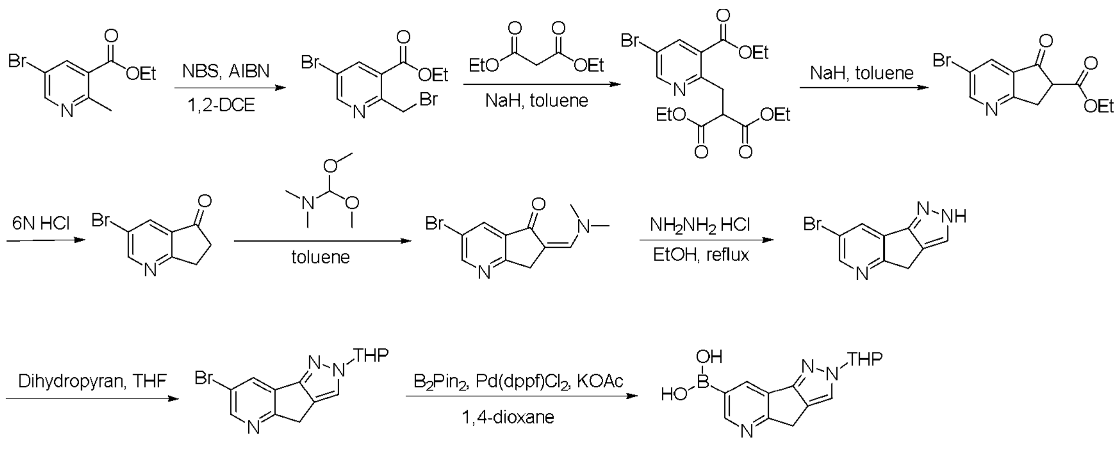


B-3

Step 1: Synthesis of compound ethyl 5-bromo-2-(bromomethyl)nicotinate

Ethyl 2-methyl-5-bromonicotinate (5.0 g, 20.6 mmol), NBS (3.8 g, 21.6 mmol) and 1,2-dichloroethane (50 mL) were added to a reaction flask. AIBN (0.7 g, 4.1 mmol) was added under nitrogen. The mixture was heated to 50 °C, stirred and reacted overnight. The reaction was monitored by TLC until it was completed. The mixture was concentrated to remove the solvent. The residue was purified by column chromatography, and vacuum dried to give 5.3 g of a light yellow liquid as a product, with a yield of 81%. LC-MS (APCI): m/z=321.8 $(M+1)^+$.

Step 2: Synthesis of compound diethyl 2-((5-bromo-3-(ethoxycarbonyl)pyridin-2-yl)methyl)malonate

Ethyl 5-bromo-2-(bromomethyl)nicotinate (3.2 g, 10 mmol) and diethyl malonate (1.92 g, 12 mmol) were added to a reaction flask, and the mixture was dissolved in 15 mL of anhydrous toluene. Sodium hydride (600 mg, 15 mmol) was added in batches in an ice bath. After the addition was completed, the mixture was warmed to room temperature, stirred and reacted under nitrogen overnight. The reaction was monitored by TLC until it was completed. The mixture was diluted with added water. The resulting mixture was extracted with ethyl acetate 3 to 4 times. The organic phases were combined, washed with saturated brine 3 times, concentrated to remove the solvent, and purified by silica gel column chromatography to give 3.2 g of a light yellow oily liquid, with a yield of 81%. LC-MS (APCI): m/z=402.1 $(M+1)^+$.

Step 3: Synthesis of compound ethyl 3-bromo-5-oxo-6,7-dihydro-5H-cyclopenta[b]pyridine-6-carboxylate

Diethyl 2-((5-bromo-3-(ethoxycarbonyl)pyridin-2-yl)methyl)malonate (3.2 g, 8.0 mmol) and anhydrous toluene (15 mL) were added to a reaction flask. Sodium hydride (800 mg, 20 mmol) was added in batches in an ice bath. After the addition was completed, the mixture was warmed to room temperature, stirred and reacted under nitrogen overnight. The reaction was monitored by TLC until it was completed. The mixture was diluted with added water. The resulting mixture was extracted with ethyl acetate 3 to 4 times. The organic phases were combined, washed with saturated brine 3 times, concentrated, and then purified by silica gel column chromatography to give 3.2 g of a light yellow oily liquid, with a yield of 81%. LC-MS (APCI): m/z=284.4 $(M+1)^+$.

Step 4: Synthesis of compound 3-bromo-6,7-dihydro-5H-cyclopenta[b]pyridin-5-one

Ethyl 3-bromo-5-oxo-6,7-dihydro-5H-cyclopenta[b]pyridine-6-carboxylate (3.2 g, 11.3 mmol) and a 6 N aqueous solution of hydrochloric acid (50 mL) were added to a reaction flask. The mixture was heated to 100 °C, stirred and reacted for 24 hours. The reaction was monitored by TLC until it was completed. The mixture was concentrated to remove the solvent. The resulting mixture was extracted with ethyl acetate 3 to 4 times. The organic phases were combined, washed with saturated sodium bicarbonate solution, concentrated, and then purified by silica gel column chromatography to give 1.1 g of an off-white solid, with a yield of 47%. LC-MS (APCI): m/z=212.3 $(M+1)^+$.

Step 5 Synthesis of compound (Z)-3-bromo-6-((dimethylamino)methylene)-6,7-dihydro-5H-cyclopenta[b]pyridin-5-one

3-Bromo-6,7-dihydro-5H-cyclopenta[b]pyridin-5-one (1.1 g, 5.2 mmol) and N,N-dimethylformamide dimethyl acetal (930 mg, 7.8 mmol) were added to a reaction flask, and the mixture was dissolved in 25 mL of anhydrous toluene.

The mixture was heated to 110 °C, stirred and reacted overnight. The reaction was monitored by TLC until it was completed. The mixture was concentrated to remove the solvent. The residue was purified by silica gel column chromatography to give 1.17 g of a light yellow solid, with a yield of 85%. LC-MS (APCI): m/z=267.4 $(M+1)^+$.

Step 6: Synthesis of compound 7-bromo-2,4-dihydropyrazolo[3',4':3,4]cyclopenta[1,2-b]pyridine

(Z)-3-Bromo-6-((dimethylamino)methylene)-6,7-dihydro-5H-cyclopenta[b]pyridin-5-one (1.17 g, 4.4 mmol), hydrazine hydrochloride (603 mg, 8.8 mmol) and anhydrous ethanol (20 mL) were added to a reaction flask. The mixture was heated to 70 °C, stirred and reacted under nitrogen for 12 hours. The reaction was monitored by TLC until it was completed. The mixture was concentrated to remove the solvent. The residue was purified by silica gel column chromatography to give 0.86 g of a light yellow solid, with a yield of 78%. LC-MS (APCI): m/z=236.4 $(M+1)^+$.

Step 7 Synthesis of compound 7-bromo-2-(tetrahydro-2H-pyran-2-yl)-2,4-dihydropyrazolo[3',4':3,4]cyclopenta[1,2-b] pyridine

7-Bromo-2,4-dihydropyrazolo[3',4':3,4]cyclopenta[1,2-b]pyridine (860 mg, 3.66 mmol), dihydropyran (615 mg, 7.32 mmol) and anhydrous tetrahydrofuran (10 mL) were added to a reaction flask. The mixture was stirred and reacted at room temperature for 2 to 3 hours. The reaction was monitored by TLC until it was completed. The mixture was concentrated to remove the solvent. The residue was purified by silica gel column chromatography to give 1.12 g of a light yellow solid, with a yield of 96%. LC-MS (APCI): m/z=320.1 $(M+1)^+$.

Step 8: Synthesis of intermediate compound B-3

7-Bromo-2-(tetrahydro-2H-pyran-2-yl)-2,4-dihydropyrazolo[3',4':3,4]cyclopenta[1,2-b]pyridine (0.99 g, 3.1 mmol), $B_2Pin_2$ (2.4 g, 9.3 mmol), $Pd(dppf)Cl_2$ (117 mg, 0.16 mmol) and potassium acetate (0.6 g, 6.2 mmol) were added to a reaction flask, and the mixture was dissolved in 10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 742 mg of product, with a yield of 84%. LC-MS (APCI): m/z=286.7 $(M+1)^+$.

**<u>Example 1 Preparation of (R)-N-(4-(chlorodifluoromethoxy)phenyl)-5-(2,4-dihydropyrazolo[3',4':3,4]cyclopenta[1,2-b]pyridin-7-yl)-6-(3-fluoropyrrolidin-1-yl)nicotinamide (compound T-1)</u>**

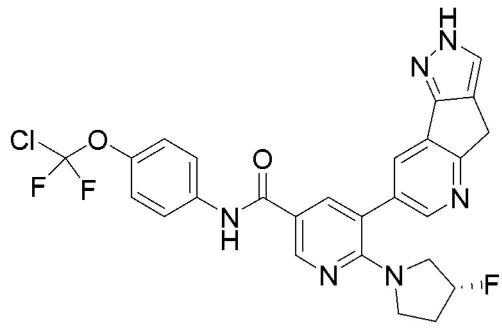

T-1

The following synthetic route was adopted:

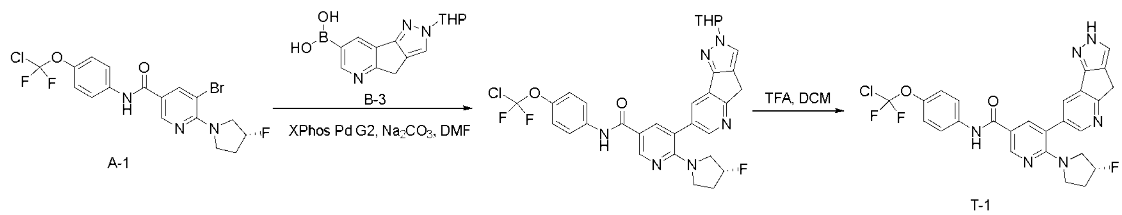

Step 1: Synthesis of compound N-(4-(chlorodifluoromethoxy)phenyl)-6-((R)-3-fluoropyrrolidin-1-yl)-5-(2-(tetrahydro-2H-pyran-2-yl)-2,4-dihydropyrazolo[3',4':3,4]cyclopenta[1,2-b]pyridin-7-yl)nicotinamide

Intermediate compound A-1 (180 mg, 0.39 mmol), intermediate compound B-3 (167 mg, 0.588 mmol), XPhos Pd G2 (16 mg, 0.02 mmol), sodium carbonate (125 mg, 1.18 mmol) and anhydrous DMF (5 mL) were added to a reaction flask. The mixture was purged with nitrogen for 5 minutes. The mixture was microwaved and heated to 90 °C, and reacted for 1 hour. The reaction was monitored by TLC until it was completed. The resulting mixture was concentrated, and then purified by silica gel column chromatography to give 139 mg of product, with a yield of 57%. LC-MS(APCI): m/z=625.7 $(M+1)^+$.

Step 2: Synthesis of compound T-1

N-(4-(Chlorodifluoromethoxy)phenyl)-6-((R)-3-fluoropyrrolidin-1-yl)-5-(2-(tetrahydro-2H-pyran-2-yl)-2,4-dihydropyrazolo[3',4':3,4]cyclopenta[1,2-b]pyridin-7-yl)nicotinamide (106 mg, 0.17 mmol), anhydrous dichloromethane (5 mL) and trifluoroacetic acid (1 mL) were added to a reaction flask. The mixture was stirred and reacted at room temperature for 1 to 2 hours. The reaction was monitored by TLC until it was completed. The resulting mixture was concentrated to remove the solvent, and dichloromethane was added. The resulting mixture was washed with saturated aqueous solution of sodium bicarbonate three times. The organic phase was concentrated, and then purified by silica gel column chromatography to give 57 mg of a white solid, with a yield of 62%. LC-MS (APCI): m/z=541.1 $(M+1)^+$. $^1H$ NMR (400 MHz, DMSO-$d_6$) δ 10.23 (s, 1H), 8.92 (s, 1H), 8.67 (s, 1H), 8.41 (s, 1H), 8.31 (s, 1H), 7.26 (d, J = 8.4 Hz, 2H), 6.82 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.11 (dd, J = 9.5, 3.8 Hz, 2H).

**Example 2 Preparation of N-(4-(chlorodifluoromethoxy)phenyl)-1-isopropyl-7-(6-methyl-5-oxo-6,7-dihydro-5H-pyrrolo[3,4-b]pyridin-3-yl)-1H-benzo[d]imidazole-5-carboxamide (compound T-2)**

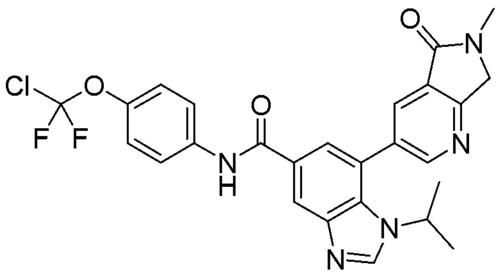

T-2

The following synthetic route was adopted:

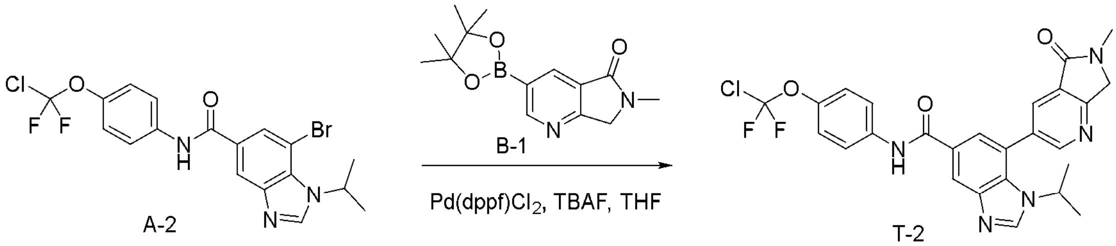


Intermediate compoundA-2 (178 mg, 0.39 mmol), intermediate compound B-1 (161 mg, 0.588 mmol), Pd(dppf)$Cl_2$ (32 mg, 0.02 mmol), tetrabutylammonium fluoride (1.2 mL, 1.18 mmol) and tetrahydrofuran (5 mL) were added to a reaction flask. The mixture was purged with nitrogen for 5 minutes. The mixture was microwaved and heated to 70 °C, and reacted for 1 hour. The reaction was monitored by TLC until it was completed. The resulting mixture was concentrated, and purified by silica gel column chromatography to give 112 mg of product, with a yield of 55%. LC-MS (APCI): m/z=526.5 $(M+1)^+$. $^1H$ NMR (400 MHz, DMSO-$d_6$) δ 10.23 (s, 1H), 8.91 (s, 1H), 8.82 (s, 1H), 8.73 (s, 1H), 8.62 (d, J = 2.5 Hz, 1H), 8.21 (d, J = 2.3 Hz, 1H), 7.82 (d, J = 8.8 Hz, 2H), 7.34 (d, J = 8.9 Hz, 2H), 4.83 (m, 1H), 3.67 (s, 3H), 3.21 (s, 2H), 1.71 (d, J = 6.4 Hz, 6H).

**Example 3 Preparation of (R)-N-(4-(chlorodifluoromethoxy)phenyl)-6-(2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-3)**

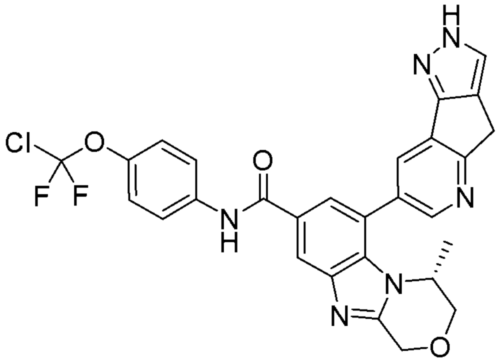

T-3

The following synthetic route was adopted:

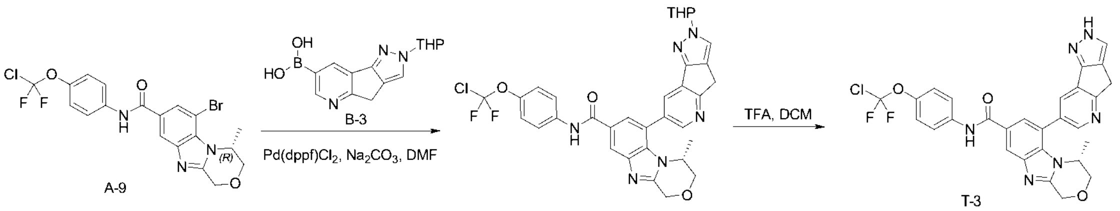


Step 1: Synthesis of compound (4R)-N-(4-(chlorodifluoromethoxy)phenyl)-4-methyl-6-(2-(tetrahydro-2H-pyran-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide

Intermediate compound A-9 (189 mg, 0.39 mmol), intermediate compound B-3 (167 mg, 0.588 mmol), Pd(dppf)$Cl_2$ (32 mg, 0.02 mmol), sodium carbonate (125 mg, 1.18 mmol) and 5 mL of anhydrous DMF were added to a reaction flask. The mixture was purged with nitrogen for 5 minutes. The mixture was microwaved and heated to 90 °C, and reacted for 1 hour. The reaction was monitored by TLC until it was completed. The resulting mixture was concentrated, and then purified by silica gel column chromatography to give 110 mg of product, with a yield of 44%. LC-MS(APCI): m/z=647.4 $(M+1)^+$.

Step 2: Synthesis of compound T-3

(4R)-N-(4-(Chlorodifluoromethoxy)phenyl)-4-methyl-6-(2-(tetrahydro-2H-pyran-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (110 mg, 0.17 mmol) was dissolved in 5 mL of anhydrous dichloromethane. 1 mL of trifluoroacetic acid was added. The resulting mixture was stirred and reacted at room temperature for 1 to 2 hours. The reaction was monitored by TLC until it was completed. The resulting mixture was concentrated to remove the solvent, and dichloromethane was added. The mixture was washed with saturated aqueous solution of sodium bicarbonate three times. The organic phase was concentrated, and then purified by silica gel column chromatography to give 68 mg of a white solid, with a yield of 71%. LC-MS (APCI): m/z=563.3 $(M+1)^+$. $^1H$ NMR (400 MHz, DMSO-$d_6$) $\delta$ 10.23 (s, 1H), 8.94 (s, 1H), 8.61 (s, 1H), 8.42 (d, J = 2.3 Hz, 1H), 8.21 (d, J = 2.2 Hz, 1H), 7.63 (d, J = 8.8 Hz, 2H), 7.38 (d, J = 7.6 Hz, 1H), 7.24 (d, J = 8.8 Hz, 2H), 5.03 (s, 2H), 4.24 (s, 2H), 4.07 (m, 1H), 3.94-3.72 (m, 2H), 1.67 (d, J = 8.6 Hz, 3H).

**Example 4 Preparation of (R)-N-(4-(chlorodifluoromethoxy)phenyl)-4-methyl-6-(2-(pyridin-2-yl)-2,4-dihydropyrazolo[3',4':3,4]cyclopenta[1,2-b]pyridin-7-yl)-3,4-dihydro-1H-benzo[4,5]imidazo|2,1-c][1,4]oxazine-8-carboxamide (compound T-4)**

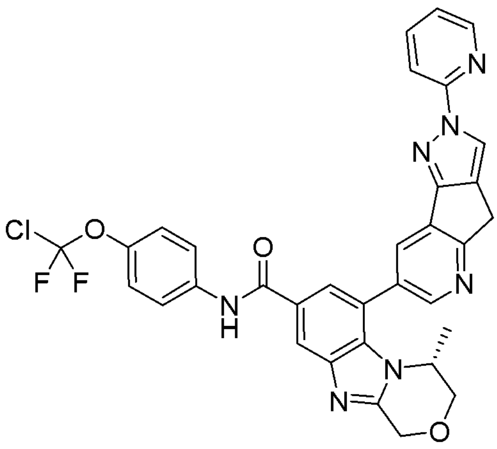

T-4

The following synthetic route was adopted:

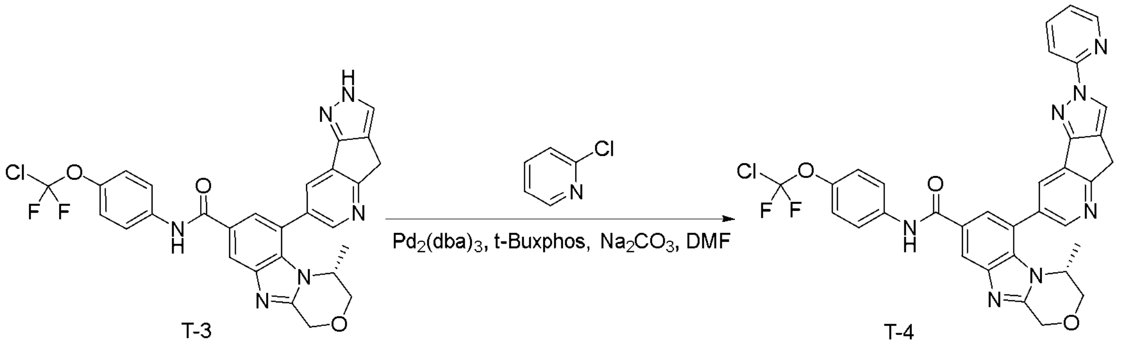


T-3

T-4

Compound T-3 (100 mg, 0.18 mmol), 2-chloropyridine (61 mg, 0.54 mmol), $Pd_2(dba)_3$ (18 mg, 0.02 mmol), 2-di-tert-butylphosphino-2',4',6'-triisopropylbiphenyl (17 mg, 0.04 mmol) and sodium carbonate (57 mg, 0.54 mmol) were added to a reaction flask, and the mixture was dissolved in 3 mL of anhydrous DMF. The mixture was purged with nitrogen for 5 minutes. The mixture was microwaved and heated to 90 °C, and reacted for 3 hours. The reaction was monitored by TLC until it was completed. The resulting mixture was concentrated, and then purified by silica gel column chromatography to give 16 mg of product, with a yield of 14%. LC-MS (APCI): m/z=640.4 $(M+1)^+$. $^1$H NMR (400 MHz, DMSO-$d_6$) $\delta$ 10.23 (s, 1H), 8.91 (s, 1H), 8.61 (s, 1H), 8.45 (d, J = 2.6 Hz, 1H), 8.34 (d, J = 2.3 Hz, 1H), 8.23 (d, J = 2.3 Hz, 1H), 7.64 (d, J = 8.8 Hz, 2H), 7.55-7.38 (m, 3H), 7.32 (d, J = 7.6 Hz, 1H), 7.24 (d, J = 8.6 Hz, 2H), 5.03 (s, 2H), 3.92 (m, 1H), 3.94-3.71 (m, 2H), 1.64 (d, J = 8.6 Hz, 3H).

**Example 5 Preparation of (R)-N-(4-(chlorodifluoromethoxy)phenyl)-6-(3-fluoropvrrolidin-1-yl)-5-(2-(pyrimidin-2-yl)-2,4-dihydropyrazolo[3',4':3,4]cyclopenta[1,2-b]pyridin-7-yl)nicotinamide (compound T-5)**

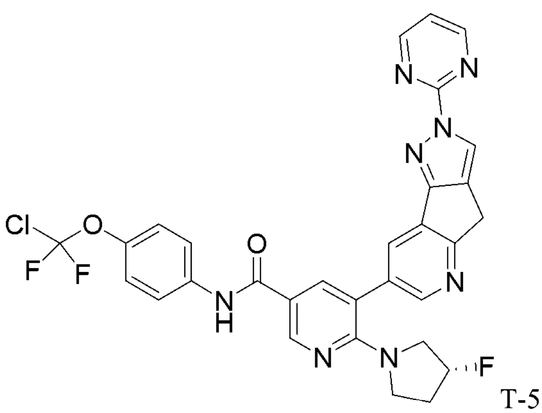

T-5

The following synthetic route was adopted:

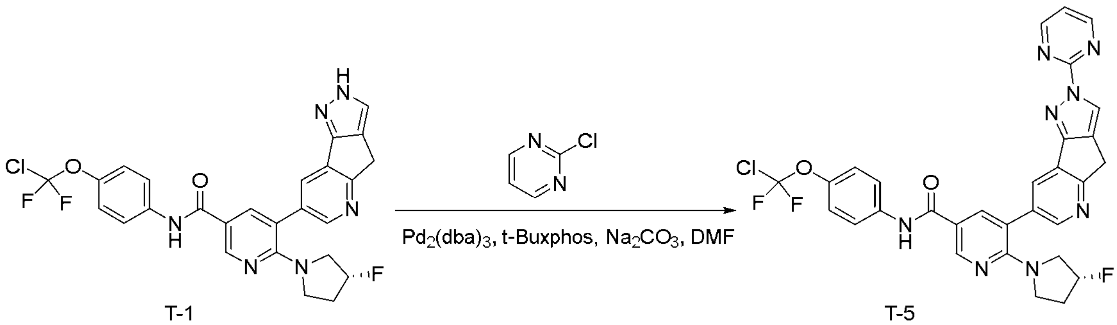


T-1 T-5

Example 5 was prepared using compound T-1 and 2-chloropyrimidine as the raw materials according to the method described in example 4, with a yield of 66%. LC-MS (APCI): m/z=619.4(M+1)$^+$. $^1$H NMR (400 MHz, DMSO-$d_6$) δ 10.23 (s, 1H), 8.93 (s, 1H), 8.66 (s, 1H), 8.41 (s, 1H), 8.31 (s, 1H), 8.05-7.64 (m, 3H), 7.26 (d, J = 8.4 Hz, 2H), 6.82 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.13 (dd, J = 9.5, 3.8 Hz, 2H).

**Example 6 Preparation of (R)-N-(4-(chlorodifluoromethoxy)phenyl)-5-(2-(5-fluoropyrimidin-2-yl)-2,4-dihydropyrazolo[3',4':3,4]cyclopenta[1,2-b]pyridin-7-yl)-6-(3-fluoropyrrolidin-1-yl)nicotinamide (compound T-6-A) and (R)-N-(4-(chlorodifluoromethoxy)phenyl)-5-(1-(5-fluoropyrimidin-2-yl)-1,4-dihydropyrazolo[3',4':3,4]cyclopenta[1,2-b]pyridin-7-yl)-6-(3-fluoropyrrolidin-1-yl)nicotinamide (compound T-6-B)**

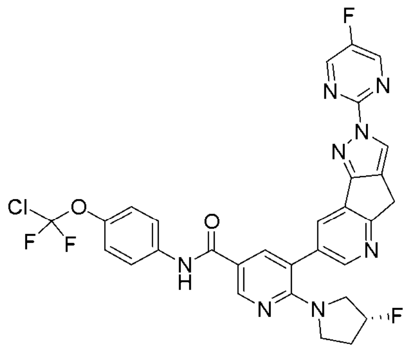
T-6-A,
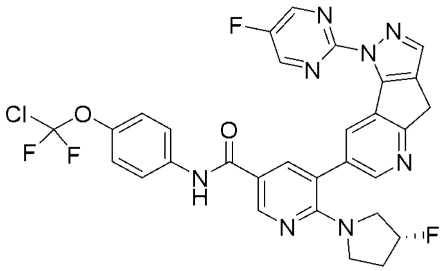
T-6-B

The following synthetic route was adopted:

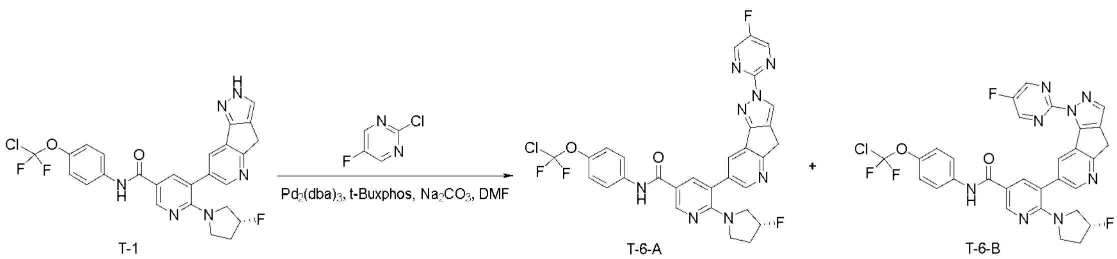


T-1 T-6-A T-6-B

Compounds T-6-A and T-6-B were prepared using compound T-1 and 2-chloro-5-fluoropyrimidine as the raw materials according to the method described in example 4, with yields of 32% and 11%, respectively. Compound T-6-A: LC-MS (APCI): m/z=619.4(M+1)$^+$. $^1$H NMR (400 MHz, DMSO-$d_6$) δ 10.23 (s, 1H), 8.91 (s, 1H), 8.67 (s, 1H), 8.43 (s, 1H), 8.31 (s, 1H), 8.05-7.64 (m, 2H), 7.26 (d, J = 8.4 Hz, 2H), 6.82 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.13 (dd, J = 9.5, 3.8 Hz, 2H).

Compound T-6-B: LC-MS (APCI): m/z=619.7(M+1)$^+$. $^1$H NMR (400 MHz, DMSO-$d_6$) δ 10.21 (s, 1H), 8.91 (s, 1H), 8.66 (s, 1H), 8.41 (s, 1H), 8.31 (s, 1H), 8.05-7.64 (m, 2H), 7.26 (d, J = 8.4 Hz, 2H), 6.83 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.11 (dd, J = 9.5, 3.8 Hz, 2H).

**Example 7 Preparation of N-(4-(chlorodifluoromethoxy)phenyl)-3,3-dimethyl-4-(6-methyl-5-oxo-6,7-dihydro-5H-pyrrolo[3,4-b]pyridin-3-yl)-2-oxoindoline-6-carboxamide (compound T-7)**

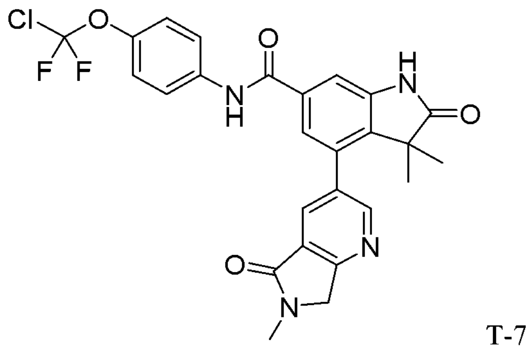

T-7

The following synthetic route was adopted:

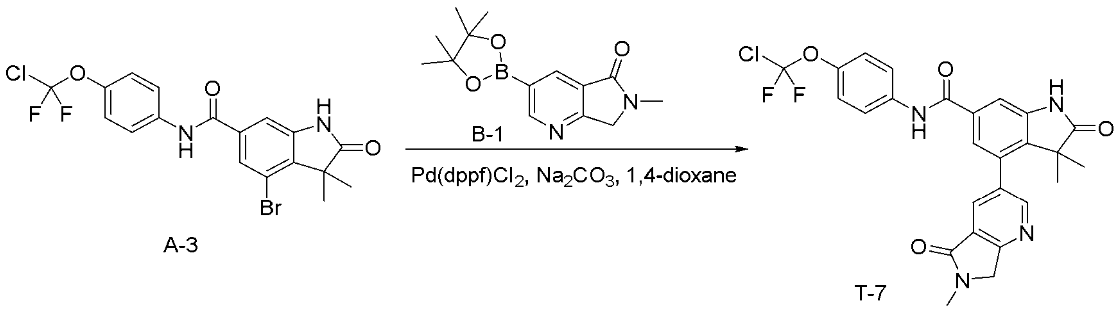


Intermediate compound A-3 (178 mg, 0.39 mmol), intermediate compound B-1 (161 mg, 0.588 mmol), Pd(dppf)$Cl_2$ (32 mg, 0.02 mmol), sodium carbonate (125 mg, 1.18 mmol) and 1,4-dioxane (5 mL) were added to a reaction flask. The mixture was purged with nitrogen for 5 minutes. The mixture was microwaved and heated to 80 °C, and reacted for 1 hour. The reaction was monitored by TLC until it was completed. The resulting mixture was concentrated, and then purified by silica gel column chromatography to give 160 mg of product, with a yield of 78%. LC-MS (APCI): m/z=527.4 $(M+1)^+$. $^1H$ NMR (400 MHz, DMSO-$d_6$) δ 10.23 (s, 1H), 8.82 (s, 1H), 8.64 (s, 1H), 8.51 (d, J = 2.4 Hz, 1H), 8.24 (d, J = 2.4 Hz, 1H), 7.81 (d, J = 9.0 Hz, 2H), 7.34 (d, J = 8.8 Hz, 2H), 3.61 (s, 3H), 3.21 (s, 2H), 1.47 (s, 6H).

**Example 8 Preparation of N-(4-(chlorodifluoromethoxy)phenyl)-4'-(6-methyl-5-oxo-6,7-dihydro-5H-pyrrolo[3,4-b]pyridin-3-yl)-2'-oxospiro[cyclohexane-1,3'-indoline]-6'-carboxamide (compound T-8)**

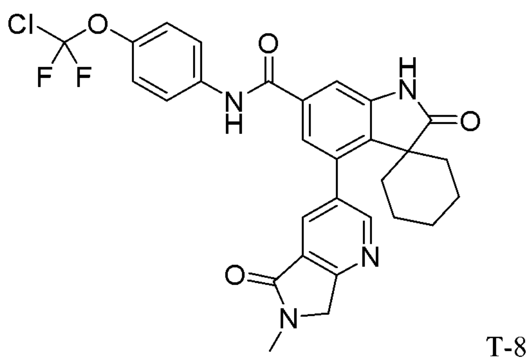

T-8

The following synthetic route was adopted:

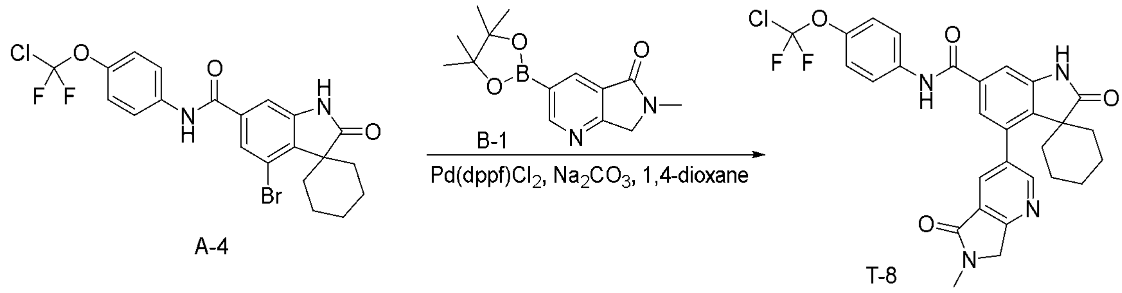


A-4

T-8

Intermediate compound A-4 (197 mg, 0.39 mmol), intermediate compound B-1 (161 mg, 0.588 mmol), Pd(dppf)$Cl_2$ (32 mg, 0.02 mmol), sodium carbonate (125 mg, 1.18 mmol) and 1,4-dioxane (5 mL) were added to a reaction flask. The mixture was purged with nitrogen for 5 minutes. The mixture was microwaved and heated to 100 °C, and reacted for 1 hour. The reaction was monitored by TLC until it was completed. The resulting mixture was concentrated, and then purified by silica gel column chromatography to give 91 mg of product, with a yield of 41%. LC-MS (APCI): m/z=567.6 $(M+1)^+$. $^1H$ NMR (400 MHz, DMSO-$d_6$) δ 10.23 (s, 1H), 8.80 (s, 1H), 8.61 (s, 1H), 8.53 (d, J = 2.4 Hz, 1H), 8.24 (d, J = 2.4 Hz, 1H), 7.81 (d, J = 9.0 Hz, 2H), 7.34 (d, J = 8.8 Hz, 2H), 3.63 (s, 3H), 3.20 (s, 2H), 1.81 (m, 4H), 1.43-1.22 (m, 7H).

**Example 9 Preparation of (R)-N-(4-(chlorodifluoromethoxy)phenyl)-6-(3-fluoropyrrolidin-1-yl)-5-(2-(4-(pyridin-3-yl)pyrimidin-2-yl)-2,4-dihydropyrazolo[3',4':3,4]cyclopenta[1,2-b]pyridin-7-yl)nicotinamide (compound T-9)**

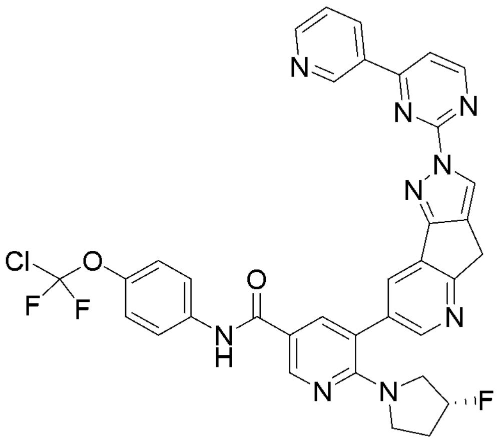

T-9

The following synthetic route was adopted:

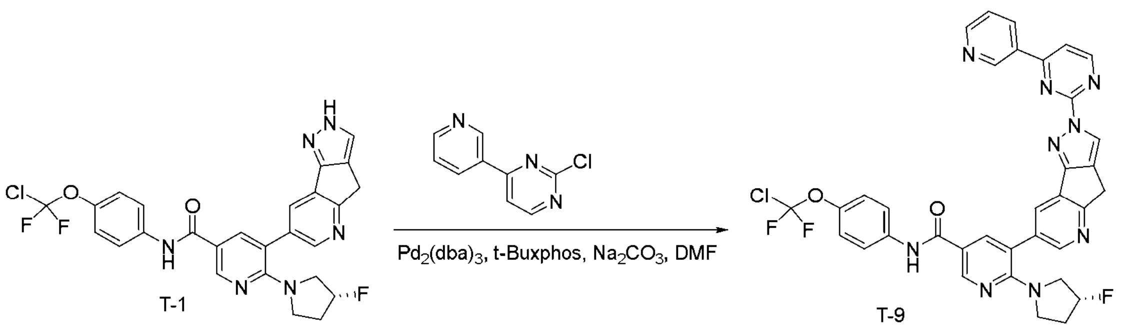


T-1

T-9

Example 9 was prepared using compound T-1 and 4-(3-pyridyl)-2-chloropyrimidine as the raw materials according to the method described in example 4, with a yield of 31%. LC-MS (APCI): m/z=696.7$(M+1)^+$. $^1H$ NMR (400 MHz, DMSO-$d_6$) δ 10.23 (s, 1H), 8.93 (s, 1H), 8.66 (s, 1H), 8.41 (s, 1H), 8.31 (s, 1H), 8.05-7.64 (m, 4H), 7.44-7.28 (m, 4 H), 7.26 (d, J = 8.4 Hz, 2H), 6.82 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.13 (dd, J = 9.5, 3.8 Hz, 2H).

**Example 10 Preparation of (R)-N-(4-(chlorodifluoromethoxy)phenyl)-6-(3-fluoropyrrolidin-1-yl)-5-(1-isopropyl-1,4-dihydropyrazolo[3',4':3,4]cyclopenta[1,2-b]pyridin-7-yl)nicotinamide (compound T-10-A) and (R)-N-(4-(chlorodifluoromethoxy)phenyl)-6-(3-fluoropyrrolidin-1-yl)-5-(2-isopropyl-2,4-dihydropyrazolo[3',4':3,4]cyclopenta[1,2-b]pyridin-7-yl)nicotinamide (compound T-10-B)**

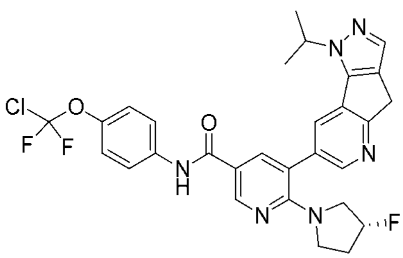
T-10-A,
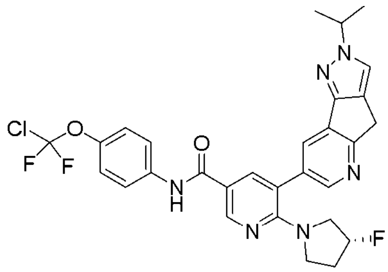
T-10-B

The following synthetic route was adopted:

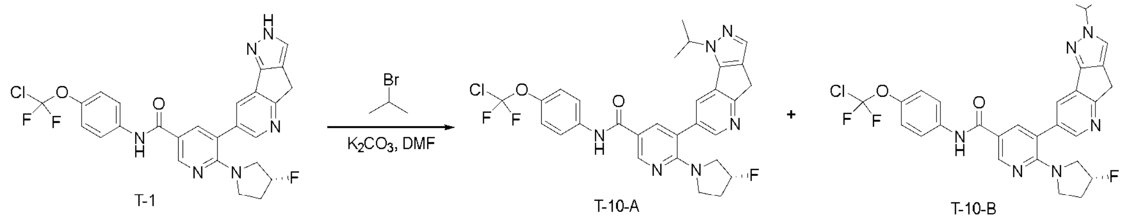

T-1 T-10-A T-10-B

Compound T-1 (200 mg, 0.37 mmol), 2-bromopropane (68 mg, 0.56 mmol) and potassium carbonate (128 mg, 0.82 mmol) were dissolved in 5 mL of anhydrous DMF. The mixture was heated to 50 °C, stirred and reacted overnight. The reaction was monitored by TLC until it was completed. The mixture was diluted with added water. The resulting mixture was extracted with ethyl acetate 3 to 4 times. The organic phases were combined, washed with saturated brine, concentrated, and then purified by silica gel column chromatography to give 38 mg of an off-white solid T-10-A and 68 mg of an off-white solid T-10-B, with yields of 18% and 32%, respectively. Compound T-10-A: LC-MS (APCI): m/z=583.3 $(M+1)^+$. $^1$H NMR (400 MHz, DMSO-$d_6$) δ 10.23 (s, 1H), 8.93 (s, 1H), 8.66 (s, 1H), 8.41 (s, 1H), 8.31 (s, 1H), 8.21 (s, 1H), 7.24 (d, J = 8.4 Hz, 2H), 6.81 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.13 (dd, J = 9.5, 3.8 Hz, 2H).

Compound T-10-B: LC-MS (APCI): m/z=583.5 $(M+1)^+$. $^1$H NMR (400 MHz, DMSO-$d_6$) δ 10.21 (s, 1H), 8.92 (s, 1H), 8.66 (s, 1H), 8.41 (s, 1H), 8.31 (s, 1H), 8.21 (s, 1H), 7.24 (d, J = 8.4 Hz, 2H), 6.81 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.12 (dd, J = 9.5, 3.8 Hz, 2H).

**Example 11 Preparation of 3-acetamido-N-(4-(chlorodifluoromethoxy)phenyl)-4-isopropyl-5-(6-methyl-5-oxo-6,7-dihydro-5H-pyrrolo[3,4-b]pyridin-3-yl)-1,2,3,3a,4,8b-hexahydrocyclopenta[b]indole-7-carboxamide (compound T-11), (3S)-3-acetamido-N-(4-(chlorodifluoromethoxy)phenyl)-4-isopropyl-5-(6-methyl-5-oxo-6,7-dihydro-5H-pyrrolo[3,4-b]pyridin-3-yl)-1,2,3,3a,4,8b-hexahydrocyclopenta[b]indole-7-carboxamide (compound T-11-A) and (3R)-3-acetamido-N-(4-(chlorodifluoromethoxy)phenyl)-4-isopropyl-5-(6-methyl-5-oxo-6,7-dihydro-5H-pyrrolo[3,4-b]pyridin-3-yl)-1,2,3,3a,4,8b-hexahydrocyclopenta[b]indole-7-carboxamide (compound T-11-B)**

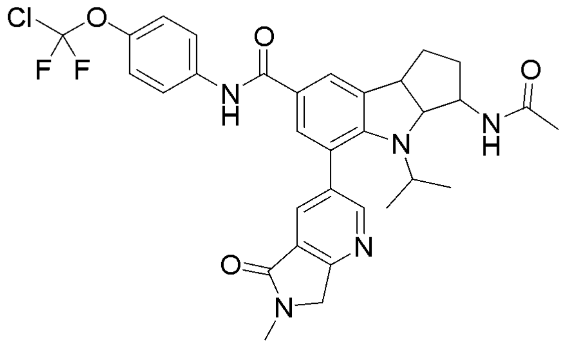

T-11,

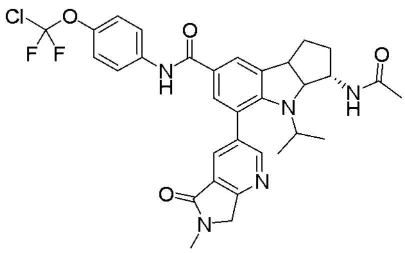

T-11-A and

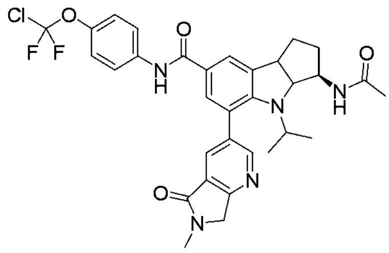

T-11-B

The following synthetic route was adopted:

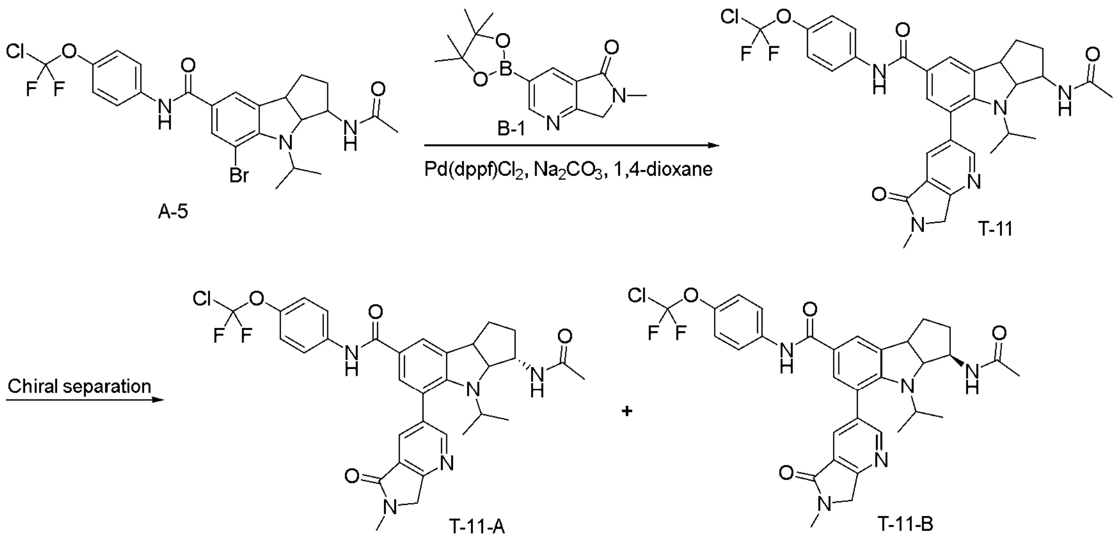


Step 1: Synthesis of compound T-11

Intermediate compound A-5 (216 mg, 0.39 mmol), intermediate compound B-1 (161 mg, 0.588 mmol), Pd(dppf)$Cl_2$ (32 mg, 0.02 mmol), sodium carbonate (125 mg, 1.18 mmol) and 1,4-dioxane (5 mL) were added to a reaction flask. The mixture was purged with nitrogen for 5 minutes. The mixture was microwaved and heated to 100 °C, and reacted for 1 hour. The reaction was monitored by TLC until it was completed. The resulting mixture was concentrated, and then purified by silica gel column chromatography to give 134 mg of product, with a yield of 55%. LC-MS (APCI): m/z=624.3 $(M+1)^+$. $^1H$ NMR (400 MHz, DMSO-$d_6$) δ 10.22 (s, 1H), 8.82 (s, 1H), 8.69 (s, 1H), 8.63 (d, J = 2.5 Hz, 1H), 8.21 (d, J = 2.3 Hz, 1H), 7.87 (d, J = 9.2 Hz, 2H), 7.33 (d, J = 8.9 Hz, 2H), 4.35 (s, 3H), 4.21 (s, 2H), 3.47 (m, 1H), 3.21 (m, 2H), 2.85 (d, J = 11.6 Hz, 1H), 2.24 (s, 2H), 1.47-1.26 (m, 4H), 1.14 (d, J = 2.8 Hz, 6H).

Step 2: Preparation of compounds T-11-A and T-11-B

The racemic compounds T-11 were separated by supercritical fluid chromatography to give the target products T-11-A (retention time: 1.06 min, relative content: 48.1%) and T-11-B (retention time: 1.36 min, relative content: 51.9%).

Chromatographic separation conditions:

Column: CHIRALPAK, IH-3, 50×4.6 mm I.D., 3 μm
Column temperature: 35 °C
Flow rate: 3.0 mL/min
UV detection wavelength: 220 nm
Mobile phase: carbon dioxide:isopropanol (0.05% diethylamine) = 75:25

**Example 12 Preparation of 3-acetamido-N-(4-(chlorodifluoromethoxy)phenyl)-4-isopropyl-5-(pyrazin-2-yl)-1,2,3,3a,4,8b-hexahydrocyclopenta[b]indole-7-carboxamide (compound T-12), (3S)-3-acetamido-N-(4-(chlorodifluoromethoxy)phenyl)-4-isopropyl-5-(pyrazin-2-yl)-1,2,3,3a,4,8b-hexahydrocyclopenta[b]indole-7-carboxamide (compound T-12-A) and (3R)-3-acetamido-N-(4-(chlorodifluoromethoxy)phenyl)-4-isopropyl-5-(pyrazin-2-yl)-1,2,3,3a,4,8b-hexahvdrocyclopentalblindole-7-carboxamide (compound T-12-B)**

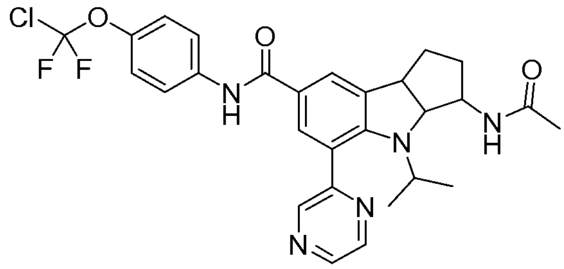

T-12,

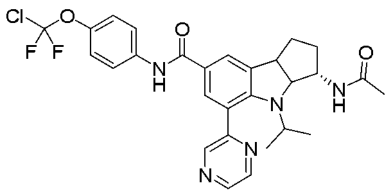

T-12-A and

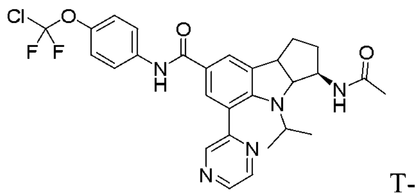

T-12-B

The following synthetic route was adopted:

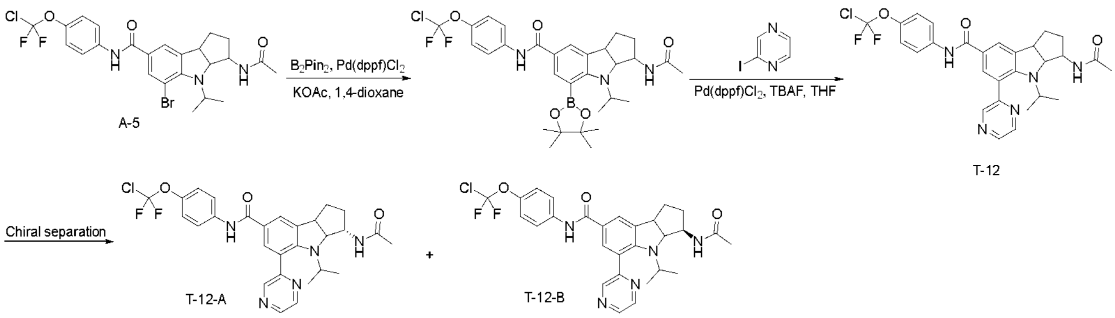


Step 1: Synthesis of compound 3-acetamido-N-(4-(chlorodifluoromethoxy)phenyl)-4-isopropyl-5-(4,4,5,5-tetramethyl-1,3,2-dioxaborolan-2-yl)-1,2,3,3a,4,8b-hexahydrocyclopenta[b]indole-7-carboxamide

Intermediate compounds A-5 (1.72 g, 3.1 mmol), $B_2Pin_2$ (2.4 g, 9.3 mmol), $Pd(dppf)Cl_2$ (117 mg, 0.16 mmol) and potassium acetate (0.6 g, 6.2 mmol) were dissolved in 10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 766 mg of product, with a yield of 41%. LC-MS (APCI): m/z=604.7 $(M+1)^+$.

Step 2: Synthesis of compound T-12

3-Acetamido-N-(4-(chlorodifluoromethoxy)phenyl)-4-isopropyl-5-(4,4,5,5-tetramethyl-1,3,2-dioxaborolan-2-yl)-1,2,3,3a,4,8b-hexahydrocyclopenta[b]indole-7-carboxamide (235 mg, 0.39 mmol), 2-iodopyrazine (121 mg, 0.588 mmol), $Pd(dppf)Cl_2$ (32 mg, 0.02 mmol), TBAF (1.2 mL, 1.18 mmol) and tetrahydrofuran (5 mL) were added to a reaction flask. The mixture was purged with nitrogen for 5 minutes. The mixture was microwaved and heated to 70 °C, and reacted for 1 hour. The reaction was monitored by TLC until it was completed. The resulting mixture was concentrated, and then purified by silica gel column chromatography to give 84 mg of product, with a yield of 39%. LC-MS (APCI): m/z=556.4 $(M+1)^+$. $^1H$ NMR (400 MHz, DMSO-$d_6$) δ 10.23 (s, 1H), 8.82-8.71 (m, 2H), 8.66 (s, 1H), 8.61 (d, J = 2.5 Hz, 1H), 8.21 (d, J = 2.3 Hz, 1H), 7.87 (d, J = 9.2 Hz, 2H), 7.33 (d, J = 8.9 Hz, 2H), 4.35 (s, 3H), 4.21 (s, 2H), 3.47 (m, 1H), 3.21 (m, 2H), 2.83 (d, J = 11.4 Hz, 1H), 2.24 (s, 2H), 1.47-1.26 (m, 4H), 1.14 (d, J = 2.8 Hz, 6H).

Step 3: Synthesis of compound T-12-A and compound T-12-B

The racemic compounds T-12 were separated by supercritical fluid chromatography to give the target products T-12-A (retention time: 1.21 min, relative content: 48.3%) and T-12-B (retention time: 1.41 min, relative content: 51.7%).

Chromatographic separation conditions:

Column: CHIRALPAK, IH-3, 50×4.6 mm I.D., 3 μm
Column temperature: 35 °C
Flow rate: 3.0 mL/min
UV detection wavelength: 220 nm
Mobile phase: carbon dioxide:isopropanol (0.05% diethylamine) = 75:25

**Example 13 Preparation of (S)-N-(4-(chlorodifluoromethoxy)phenyl)-5-(2-(5-fluoropyrimidin-2-yl)-2,4-dihydropyrazolo[3',4':3,4]cyclopenta[1,2-b]pyridin-7-yl)-6-(3-fluoropyrrolidin-1-yl)nicotinamide (compound T-13)**

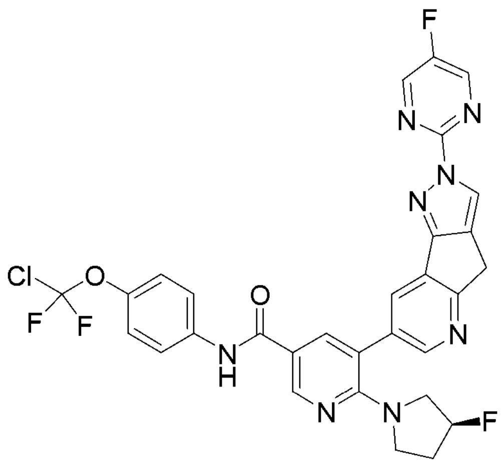

T-13

The following synthetic route was adopted:

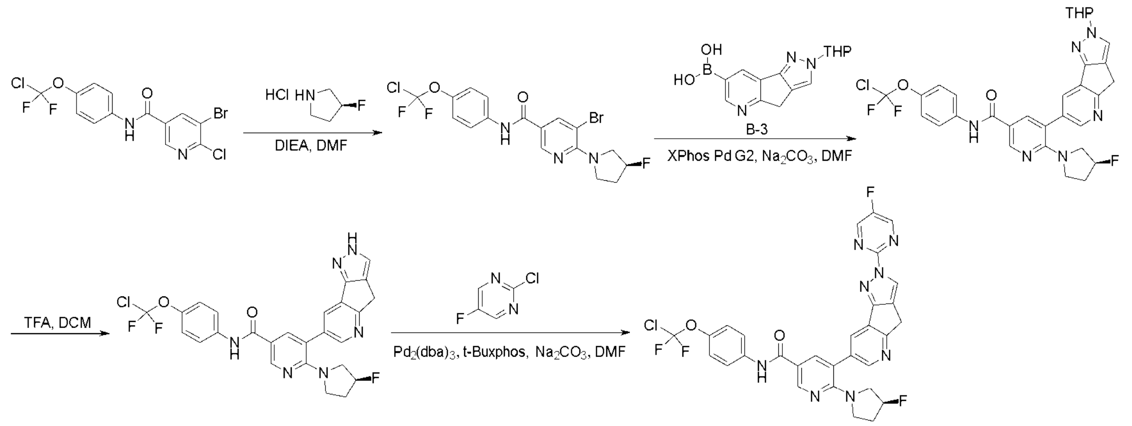


T-13

Step 1: Synthesis of compound (S)-5-bromo-N-(4-(chlorodifluoromethoxy)phenyl)-6-(3-fluoropyrrolidin-1-yl)nicotinamide

5-Bromo-6-chloro-N-(4-(chlorodifluoromethoxy)phenyl)nicotinamide (3.7 g, 8.9 mmol), (S)-3-fluoropyrrolidine hydrochloride (1.4 g, 10.7 mmol), DMF (25 mL) and DIEA (3.7 g, 2.7 mmol) were added to a reaction flask. The mixture was heated to 130 °C, stirred and reacted for 2 hours. The reaction was monitored by TLC until it was completed. The mixture was cooled to room temperature. The reaction solution was poured into 500 mL of ice water, and 300 mL of ethyl acetate was added. The mixture was stirred at room temperature until the solid was completely dissolved. The aqueous layer was extracted with 200 mL of ethyl acetate twice. The organic phases were combined, washed with saturated brine, dried over anhydrous sodium sulfate, filtered, and concentrated to give a yellow solid. Then, 25 mL of petroleum ether was added, and the mixture was stirred at room temperature for 10 minutes. The mixture was filtered. The filter cake was washed with petroleum ether. The filter cake was vacuum dried to give 3.8 g of a white solid, with a yield of 92.0%. LC-MS (APCI): m/z=464.3 $(M+1)^+$.

Step 2: Synthesis of compound N-(4-(chlorodifluoromethoxy)phenyl)-6-((S)-3-fluoropyrrolidin-1-yl)-5-(2-(tetrahydro-2H-pyran-2-yl)-2,4-dihydropyrazolo[3',4':3,4]cyclopenta[1,2-b]pyridin-7-yl)nicotinamide

(S)-5-Bromo-N-(4-(chlorodifluoromethoxy)phenyl)-6-(3-fluoropyrrolidin-1-yl)nicotinamide (180 mg, 0.39 mmol), intermediate compound B-3 (167 mg, 0.588 mmol), XPhos Pd G2 (16 mg, 0.02 mmol), sodium carbonate (125 mg, 1.18 mmol) and anhydrous DMF (5 mL) were added to a reaction flask. The mixture was purged with nitrogen for 5 minutes. The mixture was microwaved and heated to 90 °C, and reacted for 1 hour. The reaction was monitored by TLC until it was completed. The resulting mixture was concentrated, and then purified by silica gel column chromatography to give 139 mg of product, with a yield of 57%. LC-MS (APCI): m/z=625.7 $(M+1)^+$.

Step 3: Synthesis of compound (S)-N-(4-(chlorodifluoromethoxy)phenyl)-5-(2,4-dihydropyrazolo[3',4':3,4]cyclopenta [1,2-b]pyridin-7-yl)-6-(3-fluoropyrrolidin-1-yl)nicotinamide

N-(4-(Chlorodifluoromethoxy)phenyl)-6-((S)-3-fluoropyrrolidin-1-yl)-5-(2-(tetrahydro-2H-pyran-2-yl)-2,4-dihydropyrazolo[3',4':3,4]cyclopenta[1,2-b]pyridin-7-yl)nicotinamide (106 mg, 0.17 mmol) was dissolved in anhydrous dichloromethane (5 mL), and 1 mL of trifluoroacetic acid was added. The mixture was stirred and reacted at room temperature for 1 to 2 hours. The reaction was monitored by TLC until it was completed. The mixture was concentrated to remove the solvent, and dichloromethane was added. The resulting mixture was washed with saturated aqueous solution of sodium bicarbonate three times. The organic phase was concentrated and then purified by silica gel column chromatography to give 57 mg of a white solid, with a yield of 62%. LC-MS (APCI): m/z=541.1 $(M+1)^+$.

Step 4: Synthesis of compound T-13

Example 13 was prepared using (S)-N-(4-(chlorodifluoromethoxy)phenyl)-5-(2,4-dihydropyrazolo[3',4':3,4]cyclopenta[1,2-b]pyridin-7-yl)-6-(3-fluoropyrrolidin-1-yl)nicotinamide and 5-fluoro-2-chloropyrimidine as the raw materials according to the method described in example 4, with a yield of 55%. LC-MS (APCI): m/z=637.7$(M+1)^+$. $^1H$ NMR (400 MHz, DMSO-$d_6$) δ 10.23 (s, 1H), 8.93 (s, 1H), 8.66 (s, 1H), 8.41 (s, 1H), 8.31 (s, 1H), 8.21 (s, 1H), 7.58 (d, J = 11.2 Hz, 2H), 7.24 (d, J = 8.4 Hz, 2H), 6.81 (d, J = 8.4 Hz, 2H), 5.27-5.15 (d, J = 22.3 Hz, 1H), 4.31 (s, 2H), 3.94 (m, 1H), 3.91-3.76 (m, 1H),

3.55 (d, J = 8.6 Hz, 2H), 2.14 (dd, J = 9.5, 3.8 Hz, 2H).

**Example 14 Preparation of (R)-N-(4-(chlorodifluoromethoxy)phenyl)-6-(3-fluoropyrrolidin-1-yl)-5-(2-(5-methylpyrimidin-2-yl)-2,4-dihydropyrazolo[3',4':3,4]cyclopenta[1,2-b]pyridin-7-yl)nicotinamide (compound T-14)**

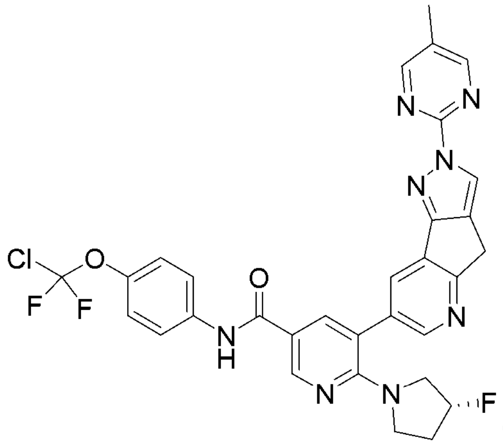

T-14

The following synthetic route was adopted:

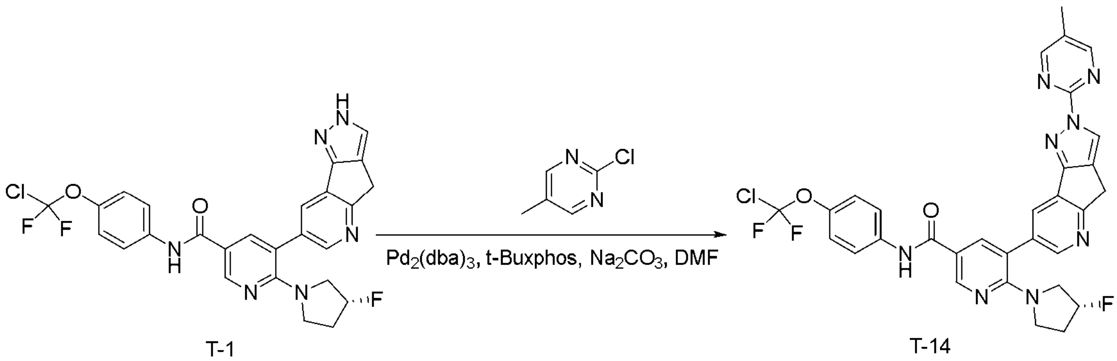


T-1 T-14

Example 14 was prepared using compound T-1 and 2-chloro-5-methylpyrimidine as the raw materials according to the method described in example 4, with a yield of 28%. LC-MS (APCI): m/z=633.2(M+1)$^+$. $^1$H NMR (400 MHz, DMSO-$d_6$) δ 10.23 (s, 1H), 8.93 (s, 1H), 8.66 (s, 1H), 8.41 (s, 1H), 8.31 (s, 1H), 8.21 (s, 1H), 7.55 (d, J = 11.2 Hz, 2H), 7.24 (d, J = 8.4 Hz, 2H), 6.81 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.38 (s, 3H), 2.11 (dd, J = 9.5, 3.8 Hz, 2H).

**Example 15 Preparation of (R)-N-(4-(chlorodifluoromethoxy)phenyl)-6-(3-fluoropyrrolidin-1-yl)-5-(2-(5-(trifluoromethyl)pyrimidin-2-yl)-2,4-dihydropyrazolo[3',4':3,4]cyclopenta[1,2-b]pyridin-7-yl)nicotinamide (compound T-15)**

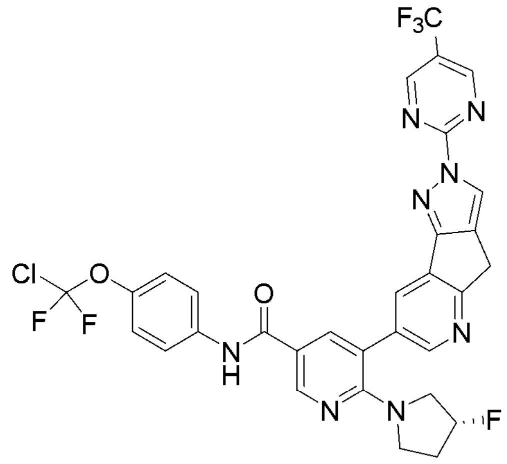

T-15

The following synthetic route was adopted:

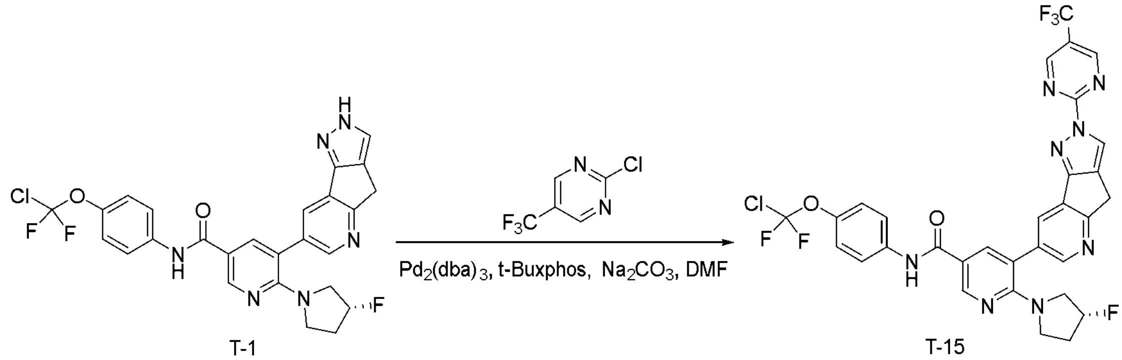


T-1 T-15

Example 15 was prepared using compound T-1 and 2-chloro-5-(trifluoromethyl)pyrimidine as the raw materials according to the method described in example 4, with a yield of 26%. LC-MS (APCI): m/z=687.1$(M+1)^+$. $^1H$ NMR (400 MHz, DMSO-$d_6$) δ 10.21 (s, 1H), 8.92 (s, 1H), 8.66 (s, 1H), 8.41 (s, 1H), 8.31 (s, 1H), 8.21 (s, 1H), 7.54 (d, J = 11.2 Hz, 2H), 7.24 (d, J = 8.4 Hz, 2H), 6.81 (d, J = 8.4 Hz, 2H), 5.29-5.15 (d, J = 22.3 Hz, 1H), 4.31 (s, 2H), 3.94 (m, 1H), 3.91-3.76 (m, 1H), 3.53 (d, J = 8.6 Hz, 2H), 2.11 (dd, J = 9.5, 3.8 Hz, 2H).

**Example 16 Preparation of (R)-N-(4-(chlorodifluoromethoxy)phenyl)-4-methyl-6-(2-methyl-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-16)**

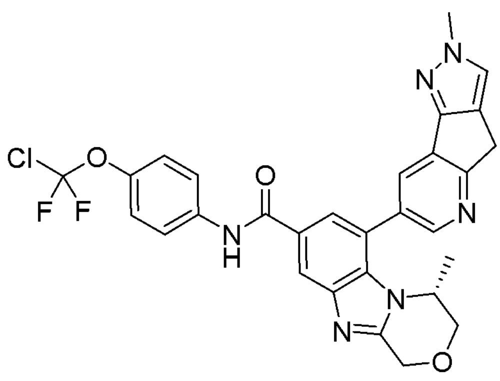

T-16

The following synthetic route was adopted:

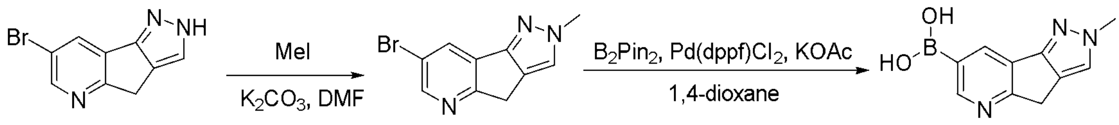


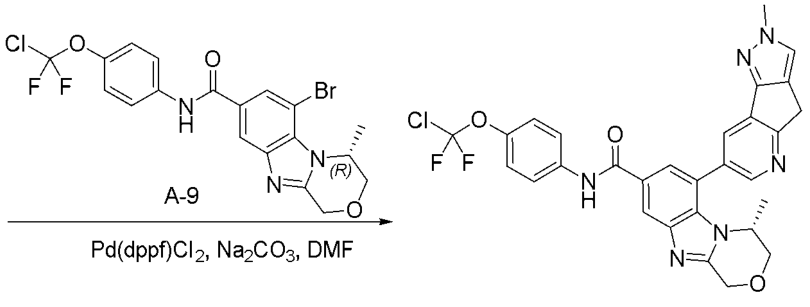


T-16

Step 1: Synthesis of compound 7-bromo-2-methyl-2,4-dihydropyrazolo[3',4':3,4]cyclopenta[1,2-b]pyridine

7-Bromo-2,4-dihydropyrazolo[3',4':3,4]cyclopenta[1,2-b]pyridine (500 mg, 2.13 mmol), iodomethane (362 mg, 2.55 mmol) and potassium carbonate (441 mg, 3.2 mmol) were dissolved in 5 mL of anhydrous DMF. The mixture was stirred and reacted at room temperature for 2 to 4 hours. The reaction was monitored by TLC until it was completed. The mixture was diluted with added water. The resulting mixture was extracted with ethyl acetate 3 to 4 times. The organic phases were combined, washed with saturated brine, concentrated, and then purified by silica gel column chromatography to give 445 mg of an off-white solid, with a yield of 84%. LC-MS (APCI): m/z=250.4 $(M+1)^+$.

Step 2: Synthesis of compound (2-methyl-2,4-dihydropyrazolo[3',4':3,4]cyclopenta[1,2-b]pyridin-7-yl)boronic acid

7-Bromo-2-methyl-2,4-dihydropyrazolo[3',4':3,4]cyclopenta[1,2-b]pyridine (772 mg, 3.1 mmol), $B_2Pin_2$ (2.4 g, 9.3 mmol), Pd(dppf)$Cl_2$ (117 mg, 0.16 mmol) and potassium acetate (0.6 g, 6.2 mmol) were dissolved in 10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 413 mg of product, with a yield of 62%. LC-MS (APCI): m/z=216.7 $(M+1)^+$.

Step 3: Synthesis of compound T-16

Intermediate compound A-9 (189 mg, 0.39 mmol), (2-methyl-2,4-dihydropyrazolo[3',4':3,4]cyclopenta[1,2-b]pyridin-7-yl)boronic acid (126 mg, 0.588 mmol), Pd(dppf)$Cl_2$ (32 mg, 0.02 mmol), sodium carbonate (125 mg, 1.18 mmol) and anhydrous DMF (5 mL) were added to a reaction flask. The mixture was purged with nitrogen for 5 minutes. The mixture was microwaved and heated to 90 °C, and reacted for 1 hour. The reaction was monitored by TLC until it was completed. The resulting mixture was concentrated, and purified by silica gel column chromatography to give 81 mg of product, with a yield of 36%. LC-MS (APCI): m/z=577.4 $(M+1)^+$. $^1$H NMR (400 MHz, DMSO-$d_6$) δ 10.22 (s, 1H), 8.91 (s, 1H), 8.62 (s, 1H), 8.34 (d, J = 2.3 Hz, 1H), 8.21 (d, J = 2.2 Hz, 1H), 7.63 (d, J = 8.8 Hz, 2H), 7.31 (d, J = 7.6 Hz, 1H), 7.24 (d, J = 8.6 Hz, 2H), 5.06 (s, 2H), 4.14 (s, 2H), 3.93 (m, 1H), 3.94-3.71 (m, 2H), 3.26 (s, 3H), 1.63 (d, J = 8.6 Hz, 3H).

**Example 17 Preparation of (R)-N-(4-(chlorodifluoromethoxy)phenyl)-6-(3-fluoropyrrolidin-1-yl)-5-(2-(5-methoxypyrimidin-2-yl)-2,4-dihydropyrazolo[3',4':3,4]cyclopenta[1,2-b]pyridin-7-yl)nicotinamide (compound T-17)**

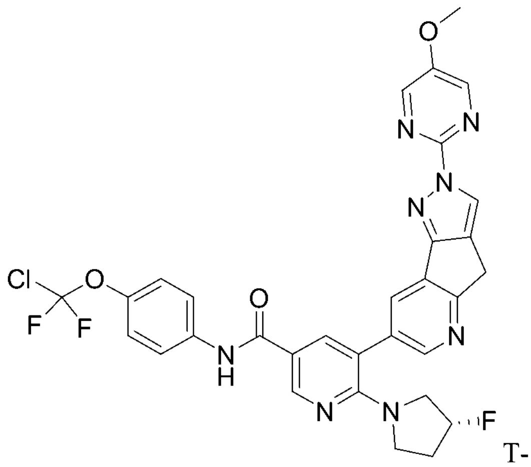

T-17

The following synthetic route was adopted:

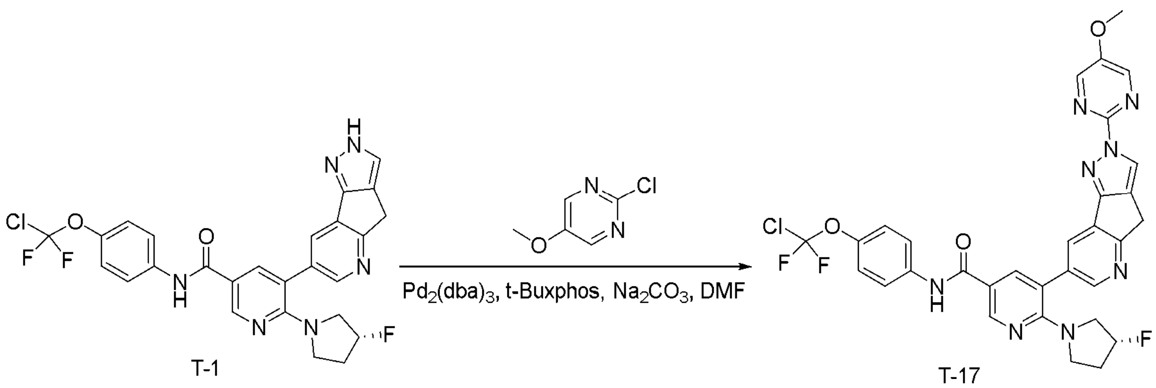


Example 17 was prepared using compound T-1 and 2-chloro-5-methoxypyrimidine as the raw materials according to the method described in example 4, with a yield of 48%. LC-MS (APCI): m/z=649.7(M+1)+. $^1$H NMR (400 MHz, DMSO-$d_6$) δ 10.22 (s, 1H), 8.93 (s, 1H), 8.67 (s, 1H), 8.41 (s, 1H), 8.31 (s, 1H), 8.21 (s, 1H), 7.54 (d, J = 11.2 Hz, 2H), 7.24 (d, J = 8.4 Hz, 2H), 6.81 (d, J = 8.4 Hz, 2H), 5.31-5.15 (d, J = 22.3 Hz, 1H), 4.31 (s, 2H), 3.94 (m, 1H), 3.91-3.76 (m, 1H), 3.87 (s, 3H), 3.55 (d, J = 8.6 Hz, 2H), 2.12 (dd, J = 9.5, 3.8 Hz, 2H).

**Example 18 Preparation of (R)-N-(4-(chlorodifluoromethoxy)phenyl)-6-(2-ethyl-2,4-dihydropyrazolo[3',4':3,4] cyclopenta[1,2-b]pyridin-7-yl)-4-methyl-3,4-dihydro-1H-benzo[4,5]imidazo[2,1-c][1,4]oxazine-8-carboxamide (compound T-18)**

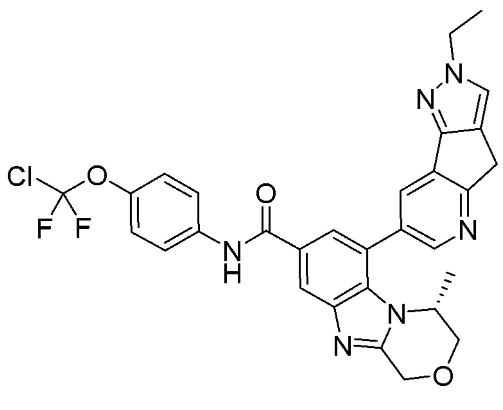

T-18

The following synthetic route was adopted:

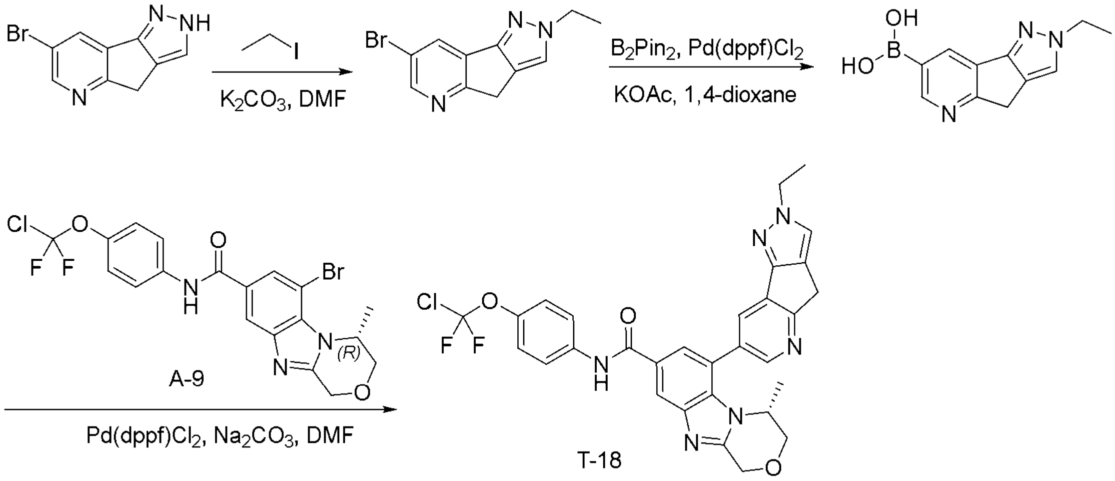


A-9

T-18

Example 18 was prepared using 7-bromo-2,4-dihydropyrazolo[3',4':3,4]cyclopenta[1,2-b]pyridine, iodoethane and intermediate compound A-9 as the raw materials according to the method described in example 16, with an overall yield of 23%. LC-MS (APCI): m/z=591.2 $(M+1)^+$. $^1H$ NMR (400 MHz, DMSO-$d_6$) δ 10.23 (s, 1H), 8.92 (s, 1H), 8.62 (s, 1H), 8.34 (d, J = 2.3 Hz, 1H), 8.21 (d, J = 2.2 Hz, 1H), 7.61 (d, J = 8.8 Hz, 2H), 7.31 (d, J = 7.6 Hz, 1H), 7.24 (d, J = 8.6 Hz, 2H), 5.06 (s, 2H), 4.14 (s, 2H), 3.93 (m, 1H), 3.94-3.71 (m, 2H), 3.21 (q, J = 9.6 Hz, 2H), 1.65 (d, J = 8.6 Hz, 3H), 1.49 (t, J = 4.6 Hz, 3H).

**Example 19 Preparation of (R)-N-(4-(chlorodifluoromethoxy)phenyl)-5-(2-(4-(6-cyanopyridin-3-yl)pyrimidin-2-yl)-2,4-dihydropyrazolo[3',4':3,4]cyclopenta[1,2-b]pyridin-7-yl)-6-(3-fluoropyrrolidin-1-yl)nicotinamide (compound T-19)**

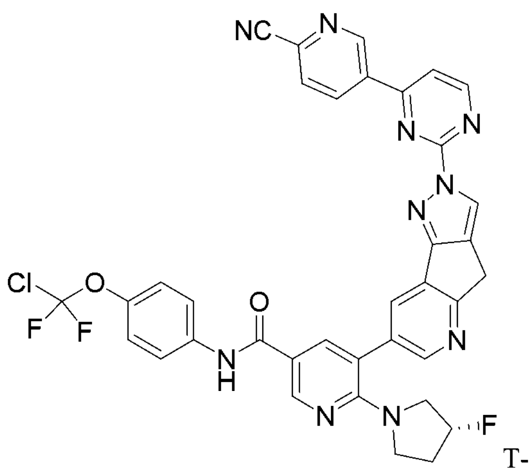

T-19

The following synthetic route was adopted:

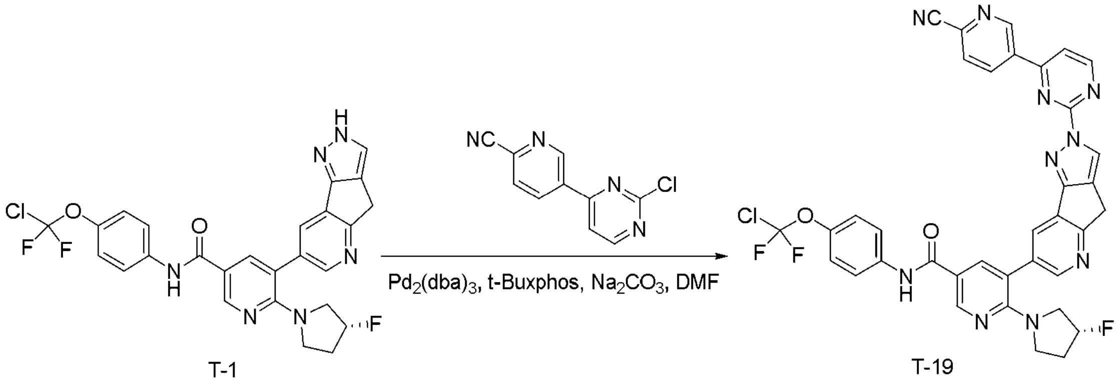


T-1 T-19

Example 19 was prepared using compound T-1 and 2-chloro-4-(6-cyanopyridin-3-yl)pyrimidine as the raw materials according to the method described in example 4, with a yield of 15%. LC-MS (APCI): m/z=721.1(M+1)$^+$. $^1$H NMR (400 MHz, DMSO-$d_6$) δ 10.23 (s, 1H), 8.92 (s, 1H), 8.66 (s, 1H), 8.41 (s, 1H), 8.31 (s, 1H), 8.21 (s, 1H), 8.11-7.86 (m, 3H), 7.55 (d, J = 11.2 Hz, 2H), 7.24 (d, J = 8.4 Hz, 2H), 6.81 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.11 (dd, J = 9.5, 3.8 Hz, 2H).

**Example 20 Preparation of (R)-N-(4-(chlorodifluoromethoxy)phenyl)-4-methyl-6-(6-methyl-5-oxo-6,7-dihydro-5H-pyrrolo[3,4-b]pyridin-3-yl)-3,4-dihydro-2H-benzo[4,5]imidazo[2,1-b][1,3]thiazine-8-carboxamide (compound T-20)**

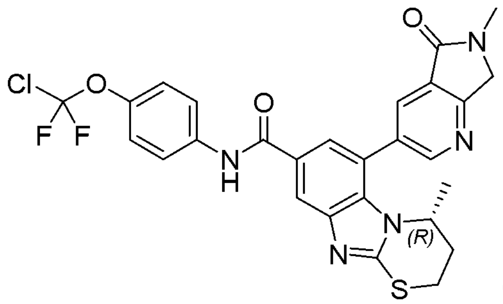

T-20

The following synthetic route was adopted:

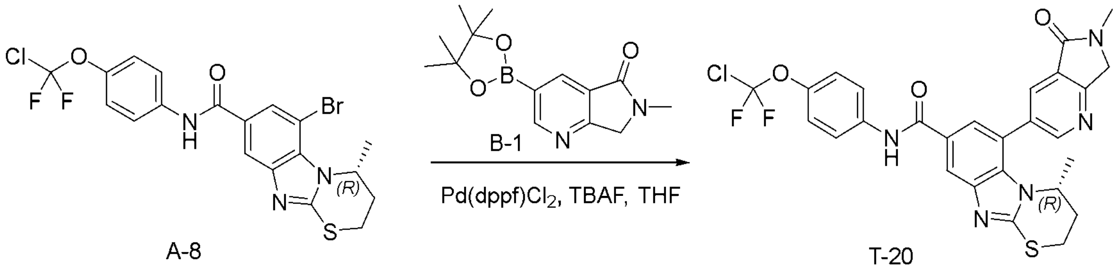


A-8 T-20

Intermediate compound A-8 (195 mg, 0.39 mmol), intermediate compound B-1 (161 mg, 0.588 mmol), [1,1'-bis(diphenylphosphino)ferrocene]dichloropalladium(II) (32 mg, 0.02 mmol), tetrabutylammonium fluoride (1.2 mL, 1.18 mmol) and tetrahydrofuran (5 mL) were added to a reaction flask. The mixture was purged with nitrogen for 5 minutes. The mixture was microwaved and heated to 70 °C, and reacted for 1 hour. The reaction was monitored by TLC until it was completed. The resulting mixture was concentrated, and purified by silica gel column chromatography to give 160 mg of product, with a yield of 72%. LC-MS (APCI): m/z=570.4 (M+1)$^+$. $^1$H NMR (400 MHz, DMSO-$d_6$) δ 10.23 (s, 1H), 8.83 (s, 1H), 8.73 (s, 1H), 8.63 (d, J = 2.5 Hz, 1H), 8.25 (d, J = 2.3 Hz, 1H), 7.81 (d, J = 9.0 Hz, 2H), 7.32 (d, J = 8.8 Hz, 2H), 3.68 (s, 3H), 3.51 (m, 1H), 3.22 (s, 2H), 2.45 (m, 2H), 2.09 (t, J = 4.4 Hz, 2H), 1.80 (d, J = 8.4 Hz, 3H).

**Example 21 Preparation of (R)-N-(4-(chlorodifluoromethoxy)phenyl)-4-methyl-6-(6-methyl-5-oxo-6,7-dihydro-5H-pyrrolo[3,4-b]pyridin-3-yl)-3,4-dihydro-1H-benzo[4,5]imidazo[2,1-c][1,4]oxazine-8-carboxamide (compound T-21)**

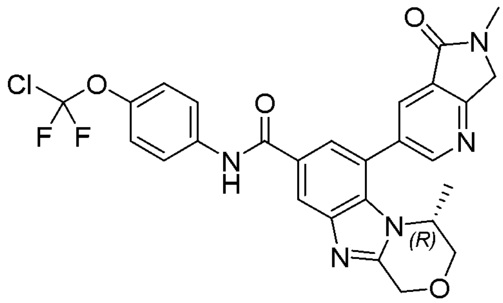

T-21

The following synthetic route was adopted:

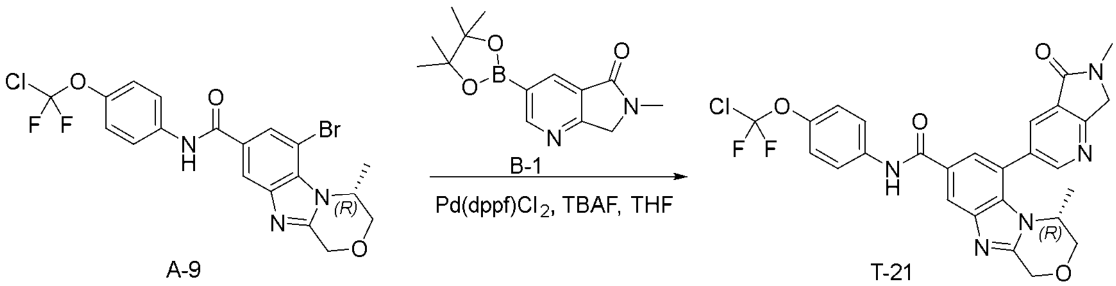


Intermediate compound A-9 (189 mg, 0.39 mmol), intermediate compound B-1 (161 mg, 0.588 mmol), [1,1'-bis(diphenylphosphino)ferrocene]dichloropalladium(II) (32 mg, 0.02 mmol), tetrabutylammonium fluoride (1.2 mL, 1.18 mmol) and tetrahydrofuran (5 mL) were added to a reaction flask. The mixture was purged with nitrogen for 5 minutes. The mixture was microwaved and heated to 70 °C, and reacted for 1 hour. The reaction was monitored by TLC until it was completed. The resulting mixture was concentrated, and purified by silica gel column chromatography to give 118 mg of product, with a yield of 55%. LC-MS (APCI): m/z=554.5 $(M+1)^+$. $^1H$ NMR (400 MHz, DMSO-$d_6$) δ 10.21 (s, 1H), 8.81 (s, 1H), 8.63 (s, 1H), 8.51 (d, J = 2.4 Hz, 1H), 8.24 (d, J = 2.3 Hz, 1H), 7.84 (d, J = 9.0 Hz, 2H), 7.31 (d, J = 8.8 Hz, 2H), 4.94 (s, 2H), 3.64 (s, 3H), 3.52 (m, 1H), 3.22 (m, 2H), 3.18 (s, 2H),1.86 (d, J = 8.6 Hz, 3H).

**Example 22 Preparation of (R)-5-(2-([4,5'-bipyrimidin-2-yl)-2,4-dihydropyrazolo[3',4':3,4]cyclopenta[1,2-b]pyridin-7-yl)-N-(4-(chlorodifluoromethoxy)phenyl)-6-(3-fluoropyrrolidin-1-yl)nicotinamide (compound T-22)**

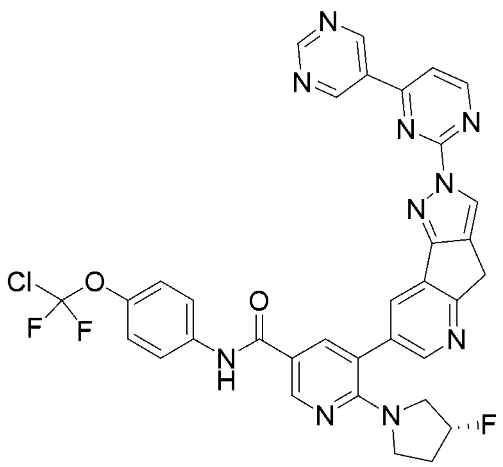

T-22

The following synthetic route was adopted:

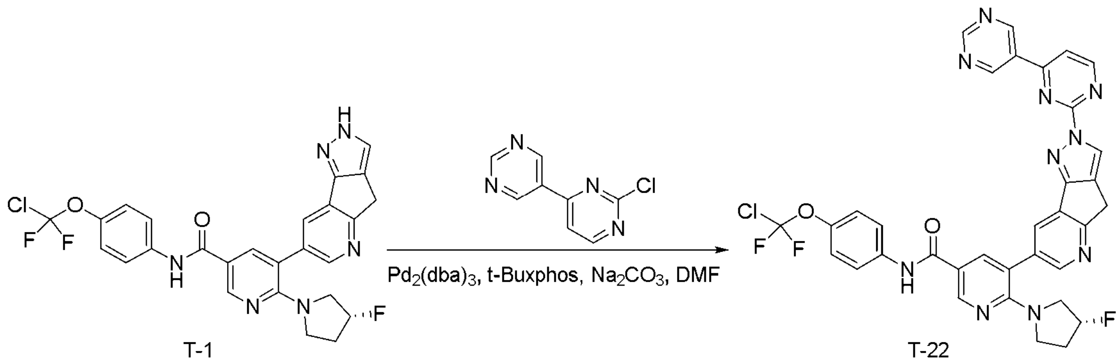


T-1 T-22

Example 22 was prepared using compound T-1 and 4-(pyrimidin-5-yl)-2-chloropyrimidine as the raw materials according to the method described in example 4, with a yield of 26%. LC-MS (APCI): m/z=697.4$(M+1)^+$. $^1H$ NMR (400 MHz, DMSO-$d_6$) δ 10.22 (s, 1H), 8.91 (s, 1H), 8.66 (s, 1H), 8.41 (s, 1H), 8.31 (s, 1H), 8.21 (s, 1H), 8.11 (d, J = 7.7 Hz, 2H), 7.84 (s, 1H), 7.55 (d, J = 11.2 Hz, 2H), 7.24 (d, J = 8.4 Hz, 2H), 6.83 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.13 (dd, J = 9.5, 3.8 Hz, 2H).

**Example 23 Preparation of N-(4-(chlorodifluoromethoxy)phenyl)-1-isopropyl-7-(pyrazin-2-yl)-1H-benzo[d]imidazole-5-carboxamide (compound T-23)**

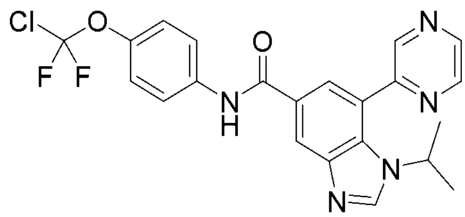

T-23

The following synthetic route was adopted:

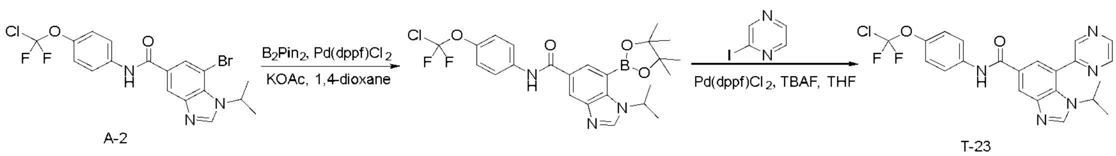


A-2 T-23

Step 1: Synthesis of compound N-(4-(chlorodifluoromethoxy)phenyl)-1-isopropyl-7-(4,4,5,5-tetramethyl-1,3,2-dioxaborolan-2-yl)-1H-benzo[d]imidazole-5-carboxamide

Intermediate compound A-2 (1.4 g, 3.1 mmol), bis(pinacolato)diboron (2.4 g, 9.3 mmol), [1,1'-bis(diphenylphosphino)ferrocene]dichloropalladium(II) (117 mg, 0.16 mmol) and potassium acetate (0.6 g, 6.2 mmol) were added to a reaction flask, and the mixture was dissolved in 10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 689 mg of product, with a yield of 44%. LC-MS (APCI): m/z=506.7 $(M+1)^+$.

Step 2: Synthesis of compound T-23

N-(4-(Chlorodifluoromethoxy)phenyl)-1-isopropyl-7-(4,4,5,5-tetramethyl-1,3,2-dioxaborolan-2-yl)-1H-benzo [d]imidazole-5-carboxamide (198 mg, 0.39 mmol), 2-iodopyrazine (92.9 mg, 0.588 mmol), [1,1'-bis(diphenylphosphino) ferrocene]dichloropalladium(II) (32 mg, 0.02 mmol), tetrabutylammonium fluoride (1.2 ml, 1.2 mmol) and 5 mL of tetrahydrofuran were added to a reaction flask. The mixture was purged with nitrogen for 5 minutes. The mixture was

microwaved and heated to 70 °C, and reacted for 1 hour. The reaction was monitored by TLC until it was completed. The resulting mixture was concentrated, and then purified by silica gel column chromatography to give 109 mg of product, with a yield of 61%. LC-MS(APCI): m/z=458.1 $(M+1)^+$. 1H NMR (400 MHz, DMSO-$d_6$) δ 10.23 (s, 1H), 8.87 - 8.79 (m, 2H), 8.76 - 8.70 (m, 1H), 8.63 (d, J = 2.5 Hz, 1H), 8.21 (d, J = 2.3 Hz, 1H), 7.87 (d, J = 9.1 Hz, 2H), 7.34 (d, J = 8.9 Hz, 2H), 4.87 (m, 1H), 1.75 (d, J = 6.4 Hz, 6H).

**Example 24 Preparation of (R)-N-(4-(chlorodifluoromethoxy)phenyl)-4-methyl-6-(6-methyl-5-oxo-6,7-dihydro-5H-pyrrolo[3,4-b]pyridin-3-yl)-3,4-dihydro-2H-benzo[4,5]imidazo[2,1-b][1,3]oxazine-8-carboxamide (compound T-24)**

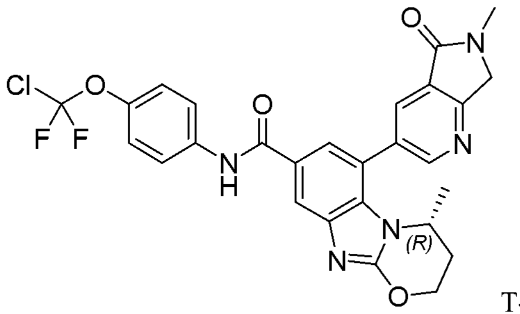

T-24

The following synthetic route was adopted:

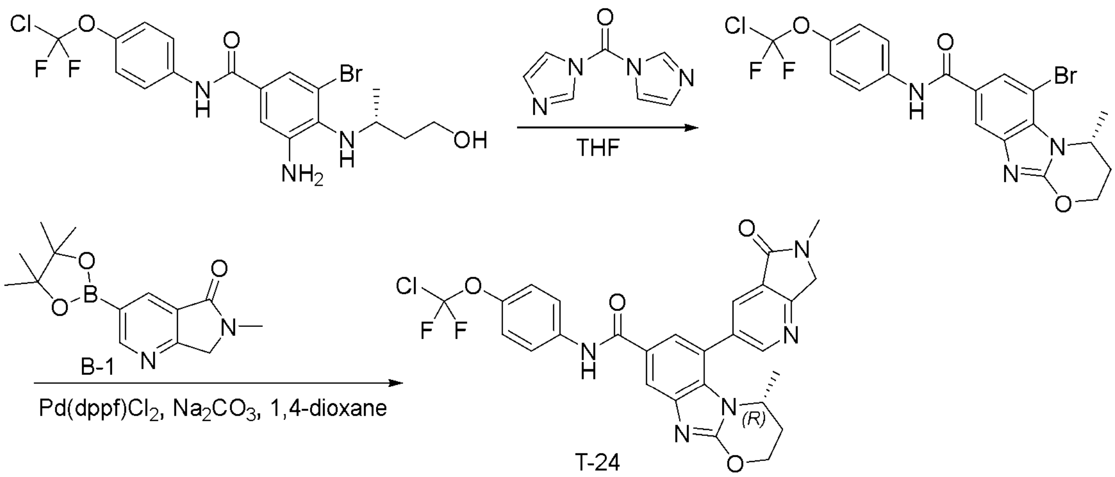


Step 1: Synthesis of compound (R)-6-bromo-N-(4-(chlorodifluoromethoxy)phenyl)-4-methyl-3,4-dihydro-2H-benzo[4,5]imidazo[2,1-b][1,3]oxazine-8-carboxamide

(R)-3-Amino-5-bromo-N-(4-(chlorodifluoromethoxy)phenyl)-4-((4-hydroxybutan-2-yl)amino)benzamide (5.21 g, 10.9 mmol) and N,N'-carbonyldiimidazole (2.9 g, 16.4 mmol) were dissolved in 35 mL of anhydrous THF. The mixture was stirred and reacted at room temperature under nitrogen overnight. The reaction was monitored by TLC until it was completed. The mixture was diluted with added water. The resulting mixture was extracted with ethyl acetate 3 to 4 times. The organic phases were combined, washed with saturated brine 3 times, concentrated to remove the solvent, and purified by silica gel column chromatography to give 1.32 g of a light yellow solid, with a yield of 25%. LC-MS (APCI): m/z=486.3 $(M+1)^+$.

Step 2: Synthesis of compound T-24

(R)-6-Bromo-N-(4-(chlorodifluoromethoxy)phenyl)-4-methyl-3,4-dihydro-2H-benzo[4,5]imidazo[2,1-b][1,3]oxazine-8-carboxamide (200 mg, 0.41 mmol), intermediate compound B-1 (169 mg, 0.62 mmol), [1,1'-bis(diphenylphosphino)ferrocene]dichloropalladium(II) (15 mg, 0.02 mmol) and sodium carbonate (130 mg, 1.23 mmol) were dissolved in

10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 106 mg of product, with a yield of 47%. LC-MS (APCI): m/z=554.7 $(M+1)^+$. $^1H$ NMR (400 MHz, DMSO-$d_6$) $\delta$ 10.25 (s, 1H), 8.82 (s, 1H), 8.63 (s, 1H), 8.52 (d, J = 2.4 Hz, 1H), 8.24 (d, J = 2.3 Hz, 1H), 7.84 (d, J = 9.0 Hz, 2H), 7.31 (d, J = 8.8 Hz, 2H), 4.05 (s, 2H), 3.64 (s, 3H), 3.51 (m, 1H), 3.22 (m, 2H), 3.18 (s, 2H),1.87 (d, J = 8.6 Hz, 3H).

**Example 25 Preparation of (R)-N-(4-(chlorodifluoromethoxy)phenyl)-6-(6-cyclopropyl-5-oxo-6,7-dihydro-5H-pyrrolo[3,4-b]pyridin-3-yl)-4-methyl-3,4-dihydro-1H-benzo[4,5]imidazo[2,1-c][1,4]oxazine-8-carboxamide (compound T-25)**

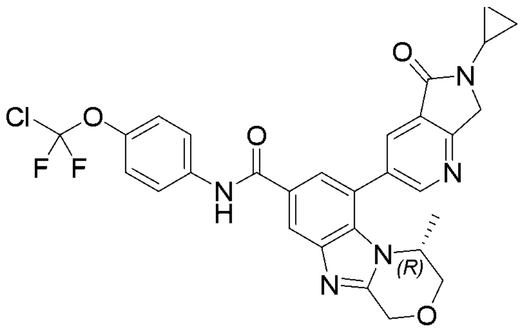

T-25

The following synthetic route was adopted:

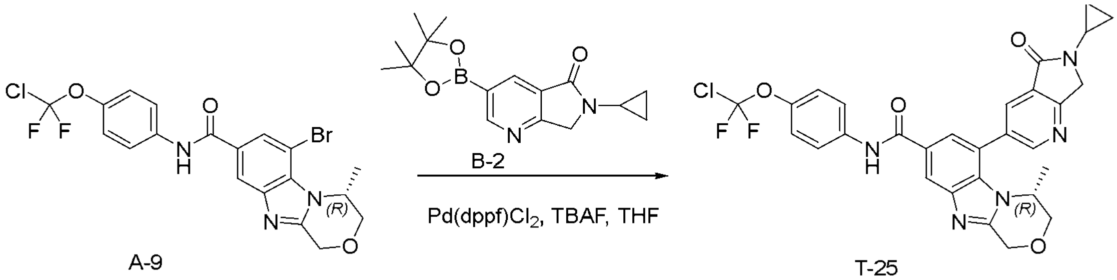


Intermediate compound A-9 (189 mg, 0.39 mmol), intermediate compound B-2 (176 mg, 0.588 mmol), [1,1'-bis(diphenylphosphino)ferrocene]dichloropalladium(II) (32 mg, 0.02 mmol), tetrabutylammonium fluoride (1.2 mL, 1.18 mmol) and tetrahydrofuran (5 mL) were added to a reaction flask. The mixture was purged with nitrogen for 5 minutes. The mixture was microwaved and heated to 70 °C, and reacted for 1 hour. The reaction was monitored by TLC until it was completed. The resulting mixture was concentrated, and purified by silica gel column chromatography to give 153 mg of product, with a yield of 68%. LC-MS(APCI): m/z=580.4 $(M+1)^+$. $^1H$ NMR (400 MHz, DMSO-$d_6$) $\delta$ 10.21 (s, 1H), 8.71 (s, 1H), 8.63 (s, 1H), 8.52 (d, J = 2.3 Hz, 1H), 8.21 (d, J = 2.3 Hz, 1H), 7.64 (d, J = 9.0 Hz, 2H), 7.30 (d, J = 8.8 Hz, 2H), 4.94 (s, 2H), 3.61 (m, 1H), 3.52 (m, 1H), 3.22 (m, 2H), 3.18 (s, 2H), 2.34-2.07 (m, 4 H), 1.81 (d, J = 8.6 Hz, 3H).

**Example 26 Preparation of (R)-N-(4-(chlorodifluoromethoxy)phenyl)-6-(3-fluoropyrrolidin-1-yl)-5-(2-(4-(1-methyl-1H-pyrazol-3-yl)pyrimidin-2-yl)-2,4-dihydropyrazolo[3',4':3,4]cyclopenta[1,2-b]pyridin-7-yl)nicotinamide (compound T-26)**

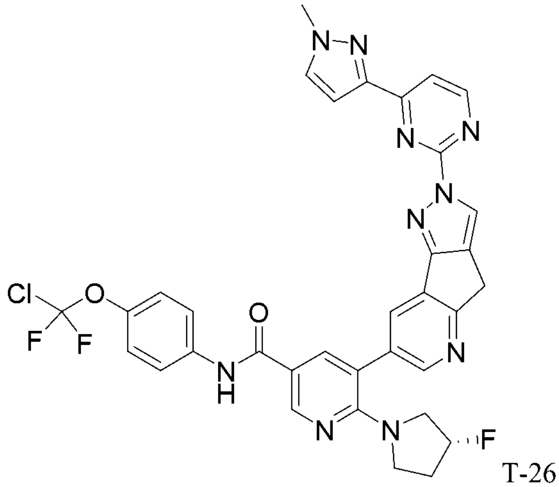

T-26

The following synthetic route was adopted:

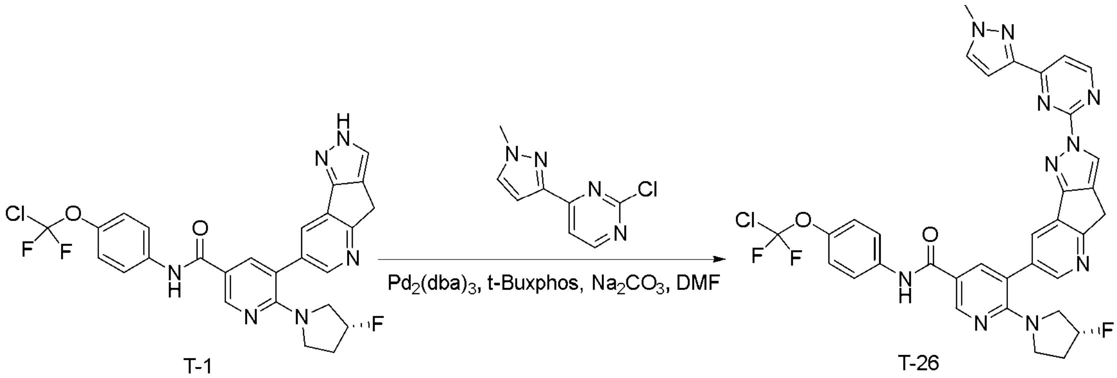


Example 26 was prepared using compound T-1 and 4-(1-methylpyrazol-3-yl)-2-chloropyrimidine as the raw materials according to the method described in example 4, with a yield of 9%. LC-MS (APCI): m/z=699.3(M+1)$^+$. $^1$H NMR (400 MHz, DMSO-$d_6$) δ 10.23 (s, 1H), 8.92 (s, 1H), 8.66 (s, 1H), 8.41 (s, 1H), 8.31 (s, 1H), 8.21 (s, 1H), 8.15 (d, J = 7.7 Hz, 1H), 7.84 (s, 1H), 7.55 (d, J = 11.2 Hz, 2H), 7.24 (d, J = 8.4 Hz, 2H), 6.83 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 3.28 (s, 3H), 2.12 (dd, J = 9.5, 3.8 Hz, 2H).

**Examples 27 and 28: Preparation of (4R)-N-(4-(chlorodifluoromethoxy)phenyl)-4-methyl-6-(6-methyl-5-oxo-6,7-dihydro-5H-pyrrolo[3,4-b]pyridin-3-yl)-3,4-dihydro-2H-benzo[4,5]imidazo[2,1-b][1,3]thiazine-8-carboxamide 1-oxide (compound T-27) and (R)-N-(4-(chlorodifluoromethoxy)phenyl)-4-methyl-6-(6-methyl-5-oxo-6,7-dihydro-5H-pyrrolo[3,4-b]pyridin-3-yl)-3,4-dihydro-2H-benzo[4,5]imidazo[2,1-b][1,3]thiazine-8-carboxamide 1,1-dioxide (compound T-28)**

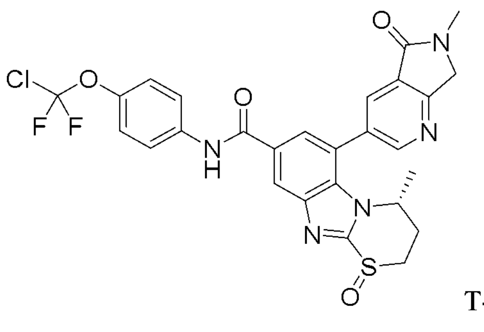

T-27,

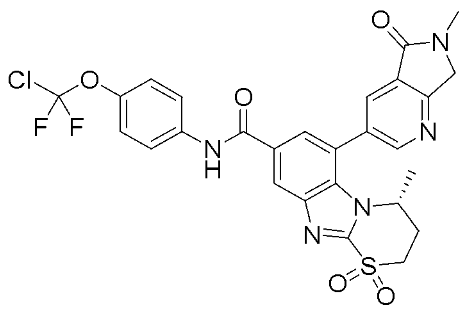

T-28

The following synthetic route was adopted:

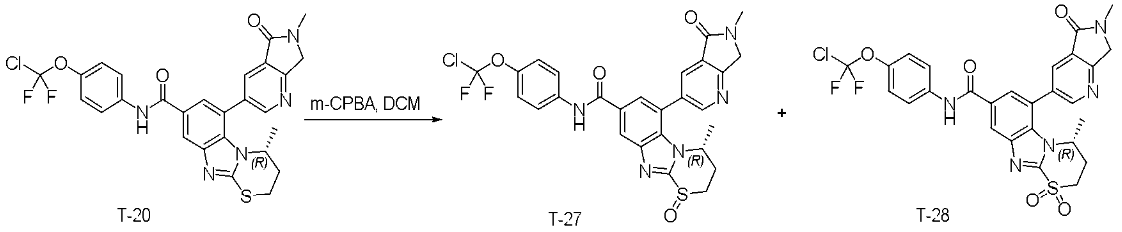


Compound T-20 (200 mg, 0.35 mmol) was dissolved in 10 mL of anhydrous dichloromethane. m-Chloroperoxybenzoic acid (91 mg, 0.53 mmol) was added in batches in an ice bath. After the addition was completed, the mixture was stirred and reacted at room temperature under nitrogen overnight. The reaction was monitored by TLC until it was completed. Water was added to quench the reaction. The resulting mixture was extracted with dichloromethane 3 to 4 times. The organic phases were combined, washed with saturated brine, concentrated, and then purified by silica gel column chromatography to give 37 mg of product T-27 and 88 mg of product T-28, with yields of 18% and 42%, respectively. Compound T-27: LC-MS (APCI): m/z=586.1 $(M+1)^+$. $^1H$ NMR (400 MHz, DMSO-$d_6$) δ 10.23 (s, 1H), 8.92 (s, 1H), 8.73 (s, 1H), 8.61 (d, J = 2.2 Hz, 1H), 8.28 (d, J = 2.2 Hz, 1H), 7.64 (d, J = 9.0 Hz, 2H), 7.30 (d, J = 8.8 Hz, 2H), 3.69 (s, 3H), 3.27 (m, 1H), 2.18 (t, J = 9.0 Hz, 2H), 2.06-1.88 (m, 2 H), 1.73 (d, J = 8.6 Hz, 3H).

Compound T-28: LC-MS (APCI): m/z=602.3 $(M+1)^+$. $^1H$ NMR (400 MHz, DMSO-$d_6$) δ 10.22 (s, 1H), 8.92 (s, 1H), 8.73 (s, 1H), 8.61 (d, J = 2.2 Hz, 1H), 8.25 (d, J = 2.2 Hz, 1H), 7.62 (d, J = 9.2 Hz, 2H), 7.31 (d, J = 8.8 Hz, 2H), 3.69 (s, 3H), 3.27 (m, 1H), 2.77 (t, J = 9.0 Hz, 2H), 2.06-1.88 (m, 2 H), 1.72 (d, J = 8.6 Hz, 3H).

**Example 29 Preparation of (R)-N-(4-(chlorodifluoromethoxy)phenyl)-6-(3-fluoropyrrolidin-1-yl)-5-(2-(quinazolin-2-yl)-2,4-dihydropyrazolo[3',4':3,4]cyclopenta[1,2-b]pyridin-7-yl)nicotinamide (compound T-29)**

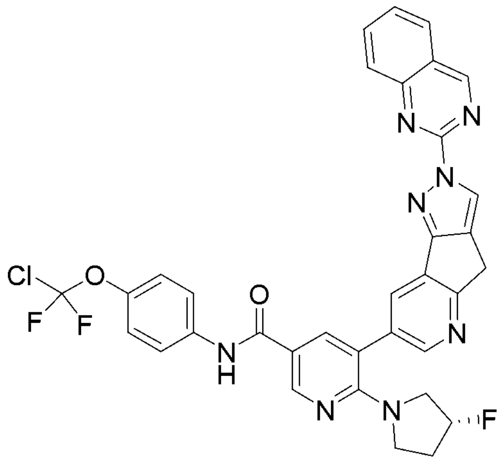

T-29

The following synthetic route was adopted:

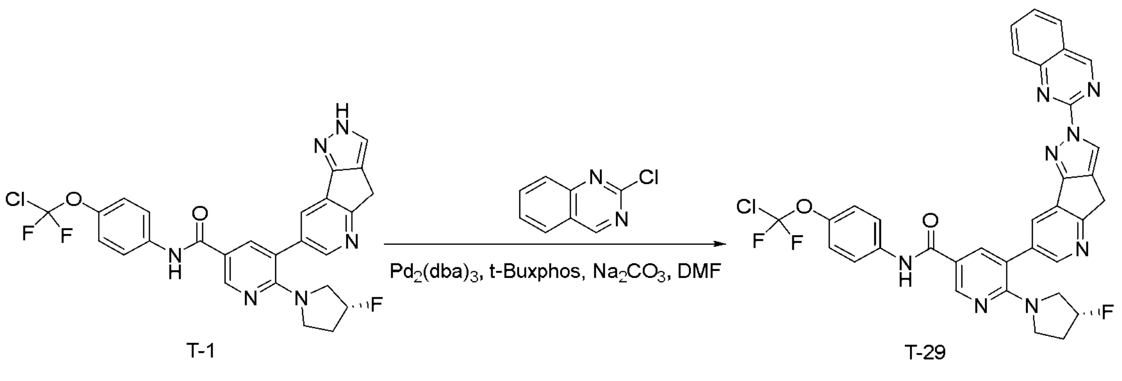


Example 29 was prepared using compound T-1 and 2-chloroquinazoline as the raw materials according to the

method described in example 4, with a yield of 14%. LC-MS (APCI): m/z=669.3(M+1)+. 1H NMR (400 MHz, DMSO-$d_6$) δ 10.23 (s, 1H), 8.91 (s, 1H), 8.66 (s, 1H), 8.41 (s, 1H), 8.31 (s, 1H), 8.21 (s, 1H), 8.11 (d, J = 7.7 Hz, 2H), 7.84 (s, 1H), 7.55 (d, J = 11.2 Hz, 2H), 7.24 (d, J = 8.4 Hz, 2H), 6.83 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.11 (dd, J = 9.5, 3.8 Hz, 2H).

**Example 30 Preparation of (R)-N-(4-(chlorodifluoromethoxy)phenyl)-4-methyl-6-(5-oxo-6,7-dihydro-5H-pyrrolo[3,4-b]pyridin-3-yl)-3,4-dihydro-1H-benzo[4,5]imidazo[2,1-c][1,4]oxazine-8-carboxamide (compound T-30)**

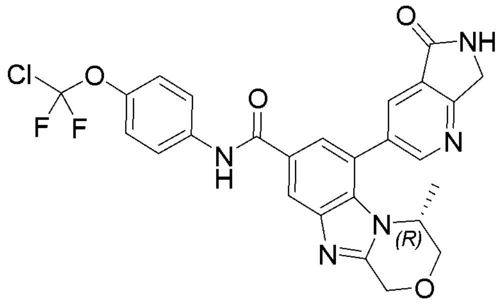

T-30

The following synthetic route was adopted:

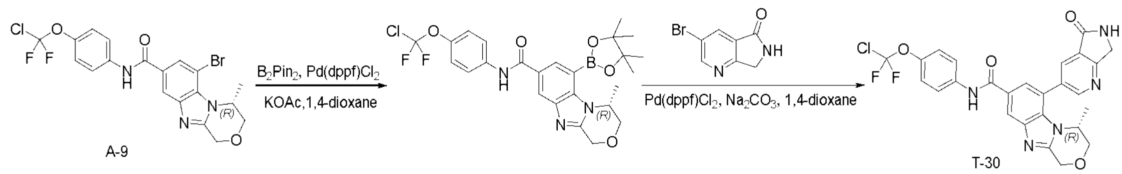


Step 1: Synthesis of compound (R)-N-(4-(chlorodifluoromethoxy)phenyl)-4-methyl-6-(4,4,5,5-tetramethyl-1,3,2-dioxaborolan-2-yl)-3,4-dihydro-1H-benzo[4,5]imidazo[2,1-c][1,4]oxazine-8-carboxamide

Intermediate compound A-9 (1.5 g, 3.1 mmol), bis(pinacolato)diboron (2.4 g, 9.3 mmol), [1,1'-bis(diphenylphosphino)ferrocene]dichloropalladium(II) (117 mg, 0.16 mmol) and potassium acetate (0.6 g, 6.2 mmol) were dissolved in 10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 578 mg of product, with a yield of 35%. LC-MS (APCI): m/z=534.7 (M+1)+.

Step 2: Synthesis of compound T-30

(R)-N-(4-(Chlorodifluoromethoxy)phenyl)-4-methyl-6-(4,4,5,5-tetramethyl-1,3,2-dioxaborolan-2-yl)-3,4-dihydro-1H-benzo[4,5]imidazo[2,1-c][1,4]oxazine-8-carboxamide (208 mg, 0.39 mmol), 3-bromo-6,7-dihydro-5H-pyrrolo[3,4-B]pyridin-5-one (125 mg, 0.588 mmol), [1,1'-bis(diphenylphosphino)ferrocene]dichloropalladium(II) (32 mg, 0.02 mmol), sodium carbonate (1.2 mL, 1.18 mmol) and 1,4-dioxane (5 mL) were added to a reaction flask. The mixture was purged with nitrogen for 5 minutes. The mixture was microwaved and heated to 100 °C, and reacted for 2 hours. The reaction was monitored by TLC until it was completed. The resulting mixture was concentrated, and purified by silica gel column chromatography to give 115 mg of product, with a yield of 55%. LC-MS (APCI): m/z=540.4 (M+1)+. 1H NMR (400 MHz, DMSO-$d_6$) δ 10.23 (s, 1H), 8.90 (s, 1H), 8.68 (s, 1H), 8.62 (d, J = 2.2 Hz, 1H), 8.26 (d, J = 2.2 Hz, 1H), 7.64 (d, J = 9.0 Hz, 2H), 7.30 (d, J = 8.8 Hz, 2H), 4.25 (s, 2H), 4.08 (s, 2H), 3.44-3.30 (m, 2H), 3.27 (m, 1H), 1.59 (d, J = 8.6 Hz, 3H).

**Example 31 Preparation of (R)-N-(4-(chlorodifluoromethoxy)phenyl)-4-methyl-6-(6-(methyl-*d*3)-5-oxo-6,7-dihydro-5H-pyrrolo[3,4-b]pyridin-3-yl)-3,4-dihydro-1H-benzo[4,5]imidazo[2,1-c][1,4]oxazine-8-carboxamide (compound T-31)**

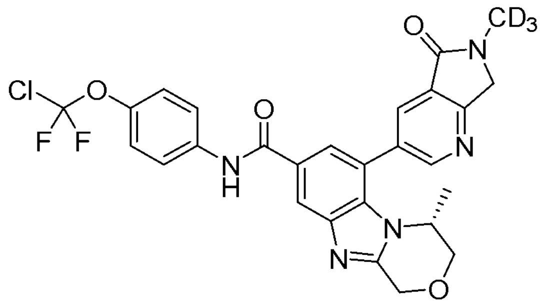

T-31

The following synthetic route was adopted:

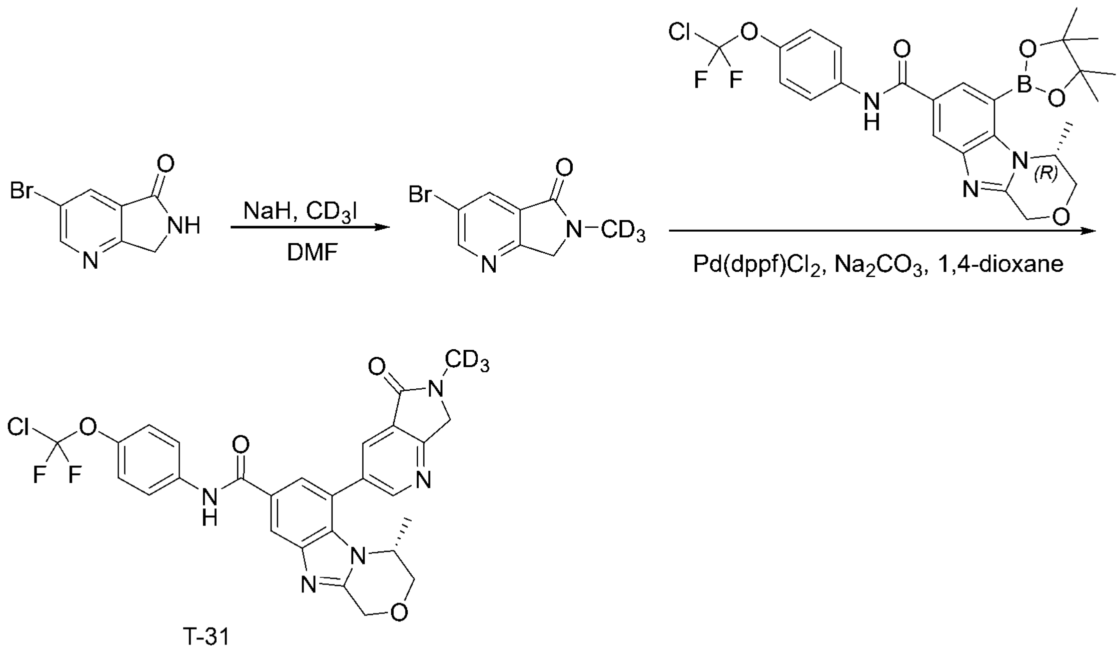


T-31

Step 1: Synthesis of compound 3-bromo-6-(methyl-*d*$_3$)-6,7-dihydro-5H-pyrrolo[3,4-b]pyridin-5-one

3-Bromo-6,7-dihydro-5H-pyrrolo[3,4-B]pyridin-5-one (1.0 g, 4.7 mmol) was dissolved in 10 mL of anhydrous DMF. Sodium hydride (283 mg, 7.1 mmol) was added in batches in an ice bath. After the addition was completed, the mixture was stirred and reacted under nitrogen for 0.5 h. Deuterated iodomethane (818 mg, 5.6 mmol) was added. The mixture was stirred and reacted at room temperature under nitrogen overnight. The reaction was monitored by TLC until it was completed. Water was added to quench the reaction. The resulting mixture was extracted with ethyl acetate 3 to 4 times. The organic phases were combined, washed with saturated brine, concentrated, and then purified by silica gel column chromatography to give 969 mg of a target product, with a yield of 90%. LC-MS (APCI): m/z=230.4 $(M+1)^+$.

Step 2: Synthesis of compound T-31

(R)-N-(4-(Chlorodifluoromethoxy)phenyl)-4-methyl-6-(4,4,5,5-tetramethyl-1,3,2-dioxaborolan-2-yl)-3,4-dihydro-1H-benzo[4,5]imidazo[2,1-c][1,4]oxazine-8-carboxamide (208 mg, 0.39 mmol), 3-bromo-6-(methyl-*d*$_3$)-6,7-dihydro-5H-pyrrolo[3,4-b]pyridin-5-one (135 mg, 0.588 mmol), [1,1'-bis(diphenylphosphino)ferrocene]dichloropalladium(II) (32 mg, 0.02 mmol), sodium carbonate (1.2 mL, 1.18 mmol) and 1,4-dioxane (5 mL) were added to a reaction flask. The mixture was purged with nitrogen for 5 minutes. The mixture was microwaved and heated to 100 °C, and reacted for 2 hours. The reaction was monitored by TLC until it was completed. The resulting mixture was concentrated, and purified by silica gel column chromatography to give 104 mg of product, with a yield of 48%. LC-MS (APCI): m/z=557.6 $(M+1)^+$. $^1H$ NMR (400 MHz, DMSO-*d*$_6$) $\delta$ 10.22 (s, 1H), 8.91 (s, 1H), 8.66 (s, 1H), 8.62 (d, J = 2.2 Hz, 1H), 8.26 (d, J = 2.2 Hz, 1H), 7.64 (d, J = 9.0 Hz, 2H), 7.32 (d, J = 8.8 Hz, 2H), 4.25 (s, 2H), 4.12 (s, 2H), 3.44-3.30 (m, 2H), 3.27 (m, 1H), 1.62 (d, J = 8.6 Hz, 3H).

**Example 32 Preparation of (1R)-N-(4-(chlorodifluoromethoxy)phenyl)-9-(6-cyclopropyl-5-oxo-6,7-dihydro-5H-pyrrolo[3,4-b]pyridin-3-yl)-4-hydroxy-1-methyl-1,2,3,4-tetrahydrobenzo[4,5]imidazo[1,2-a]pyridine-7-carboxamide (compound T-32)**

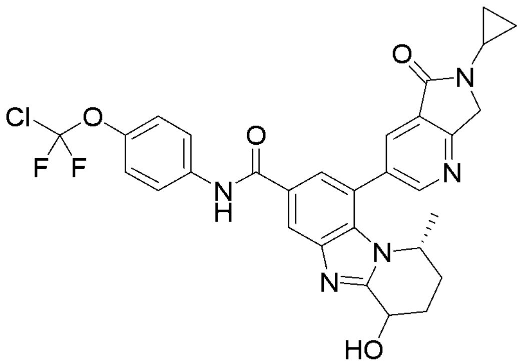

T-32

The following synthetic route was adopted:

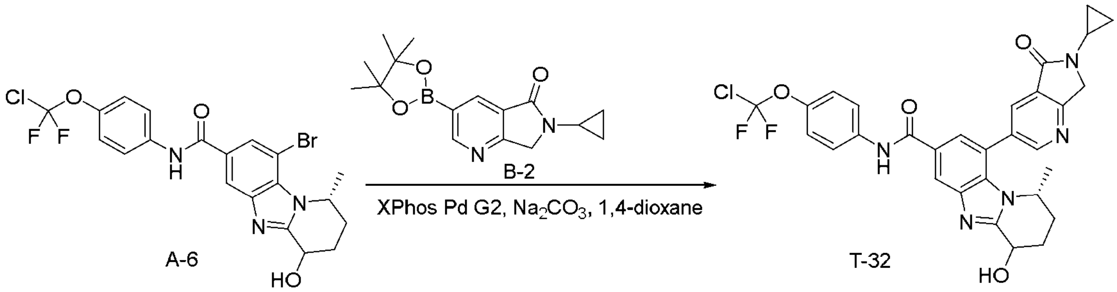


Intermediate compound A-6 (195 mg, 0.39 mmol), intermediate compound B-2 (176 mg, 0.588 mmol), XPhos Pd G2 (16 mg, 0.02 mmol), sodium carbonate (125 mg, 1.18 mmol) and 1,4-dioxane (5 mL) were added to a reaction flask. The mixture was purged with nitrogen for 5 minutes. The mixture was microwaved and heated to 100 °C, and reacted for 1 hour. The reaction was monitored by TLC until it was completed. The resulting mixture was concentrated, and purified by silica gel column chromatography to give 95 mg of product, with a yield of 41%. LC-MS (APCI): m/z=594.3 $(M+1)^+$. $^1H$ NMR (400 MHz, DMSO-$d_6$) δ 10.22 (s, 1H), 8.88 (s, 1H), 8.66 (s, 1H), 8.61 (d, J = 2.2 Hz, 1H), 8.23 (d, J = 2.2 Hz, 1H), 7.64 (d, J = 9.0 Hz, 2H), 7.32 (d, J = 8.8 Hz, 2H), 4.75 (m, 1H), 4.28 (s, 2H), 4.12 (m, 1H), 3.64 (m, 1H), 3.44-3.30 (m, 2H), 3.18-3.04 (m, 4H), 2.34 (m, 2H), 1.62 (d, J = 8.6 Hz, 3H).

**Example 33 Preparation of (1R)-N-(4-(chlorodifluoromethoxy)phenyl)-4-hydroxy-1-methyl-9-(5-oxo-6,7-dihydro-5H-pyrrolo[3,4-b]pyridin-3-yl)-1,2,3,4-tetrahydrobenzo[4,5]imidazo[1,2-a]pyridine-7-carboxamide (compound T-33)**

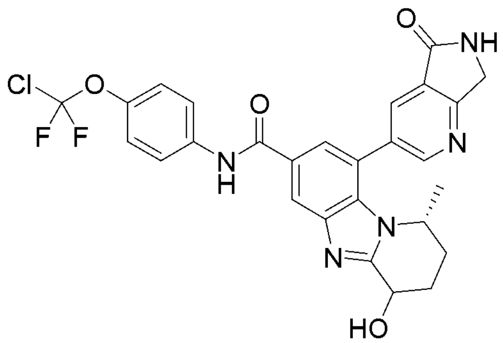

T-33

The following synthetic route was adopted:

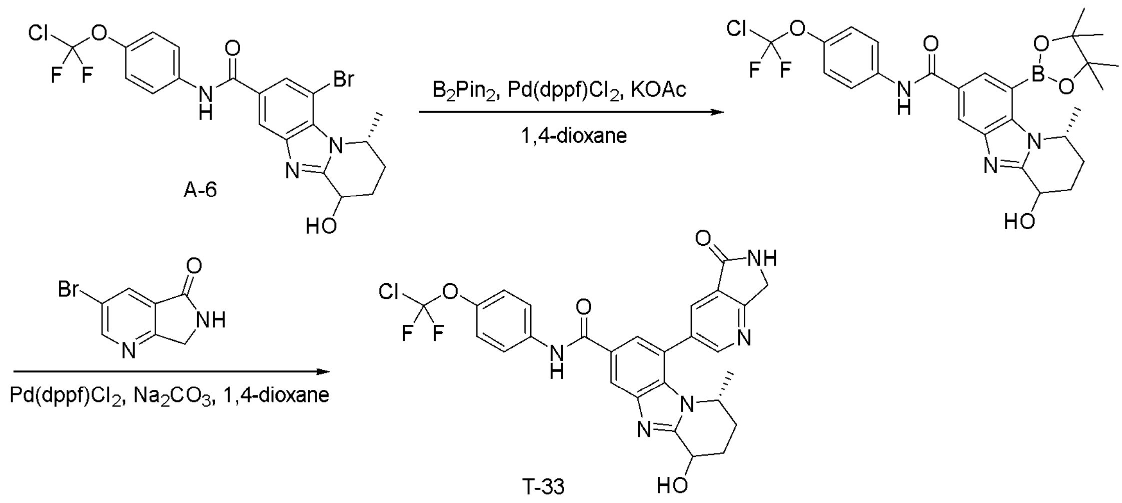


A-6

T-33

Step 1: Synthesis of compound (1R)-N-(4-(chlorodifluoromethoxy)phenyl)-4-hydroxy-1-methyl-9-(4,4,5,5-tetramethyl-1,3,2-dioxaborolan-2-yl)-1,2,3,4-tetrahydrobenzo[4,5]imidazo[1,2-a]pyridine-7-carboxamide

Intermediate compound A-6 (1.55 g, 3.1 mmol), bis(pinacolato)diboron (2.4 g, 9.3 mmol), [1,1'-bis(diphenylphosphino)ferrocene]dichloropalladium(II) (117 mg, 0.16 mmol) and potassium acetate (0.6 g, 6.2 mmol) were dissolved in anhydrous 1,4-dioxane (10 mL). The mixture was microwaved and heated to 100 °C, and reacted for 2 hours. The reaction was monitored by TLC until it was completed. The reaction solution was concentrated, and then purified by silica gel column chromatography to give 492 mg of product, with a yield of 29%. LC-MS (APCI): m/z=548.7 $(M+1)^+$.

Step 2: Synthesis of compound T-33

(1R)-N-(4-(Chlorodifluoromethoxy)phenyl)-4-hydroxy-1-methyl-9-(4,4,5,5-tetramethyl-1,3,2-dioxaborolan-2-yl)-1,2,3,4-tetrahydrobenzo[4,5]imidazo[1,2-a]pyridine-7-carboxamide (213 mg, 0.39 mmol), 3-bromo-6,7-dihydro-5H-pyrrolo[3,4-B]pyridin-5-one (125 mg, 0.588 mmol), [1,1'-bis(diphenylphosphino)ferrocene]dichloropalladium(II) (32 mg, 0.02 mmol), sodium carbonate (125 mg, 1.18 mmol) and 1,4-dioxane (5 mL) were added to a reaction flask. The mixture was purged with nitrogen for 5 minutes. The mixture was microwaved and heated to 90 °C, and reacted for 1 hour. The reaction was monitored by TLC until it was completed. The resulting mixture was concentrated, and then purified by silica gel column chromatography to give 73 mg of product, with a yield of 34%. LC-MS (APCI): m/z=554.6 $(M+1)^+$. $^1H$ NMR (400 MHz, DMSO-$d_6$) δ 10.23 (s, 1H), 8.79 (s, 1H), 8.66 (s, 1H), 8.62 (d, J = 2.2 Hz, 1H), 8.23 (d, J = 2.2 Hz, 1H), 7.64 (d, J = 9.0 Hz, 2H), 7.32 (d, J = 8.8 Hz, 2H), 4.75 (m, 1H), 4.28 (s, 2H), 3.61 (m, 1H), 3.40-3.30 (m, 2H), 2.31 (m, 2H), 1.67 (d, J = 8.4 Hz, 3H).

**Example 34 Preparation of (R)-N-(4-(chlorodifluoromethoxy)phenyl)-6-(3-fluoropyrrolidin-1-yl)-5-(2-(4-morpholinopyrimidin-2-yl)-2,4-dihydropyrazolo[3',4':3,4]cyclopenta[1,2-b]pyridin-7-yl)nicotinamide (compound T-34)**

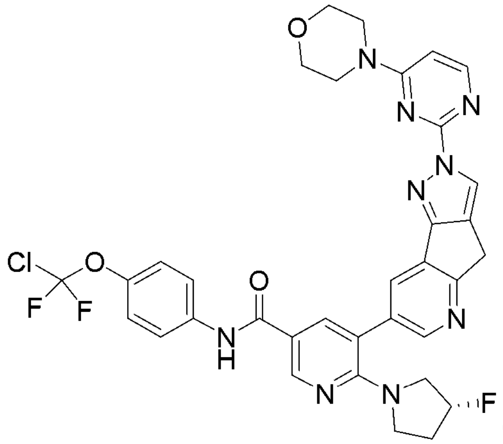

T-34

The following synthetic route was adopted:

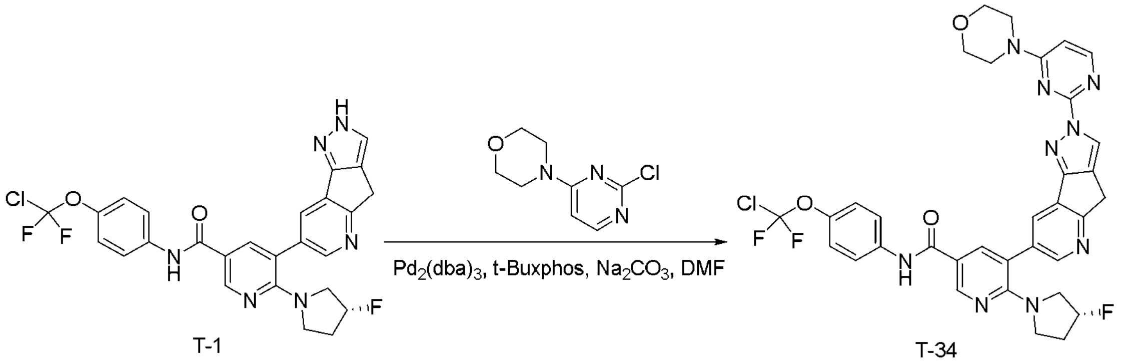


T-1

T-34

Example 34 was prepared using compound T-1 and 2-chloro-4-morpholinopyrimidine as the raw materials according to the method described in example 4, with a yield of 37%. LC-MS (APCI): m/z=704.8(M+1)$^+$. $^1$H NMR (400 MHz, DMSO-$d_6$) δ 10.23 (s, 1H), 8.91 (s, 1H), 8.66 (s, 1H), 8.41 (s, 1H), 8.31 (s, 1H), 7.82 (s, 1H), 7.56 (d, J = 11.2 Hz, 2H), 7.21 (d, J = 8.4 Hz, 2H), 6.94 (d, J = 8.4 Hz, 2H), 5.27-5.15 (d, J = 22.3 Hz, 1H), 4.31 (s, 2H), 3.94 (m, 1H), 3.91-3.76 (m, 1H), 3.84 (t, J = 4.4 Hz, 4H), 3.55 (d, J = 8.6 Hz, 2H), 3.21 (t, J = 4.4 Hz, 4H), 2.13 (dd, J = 9.5, 3.8 Hz, 2H).

**Example 35 Preparation of (1R)-N-(4-(chlorodifluoromethoxy)phenyl)-9-(6-cyclopropyl-5-oxo-6,7-dihydro-5H-pyrrolo[3,4-b]pyridin-3-yl)-4-fluoro-1-methyl-1,2,3,4-tetrahydrobenzo[4,5]imidazo[1,2-alpyridine-7-carboxamide (compound T-35)**

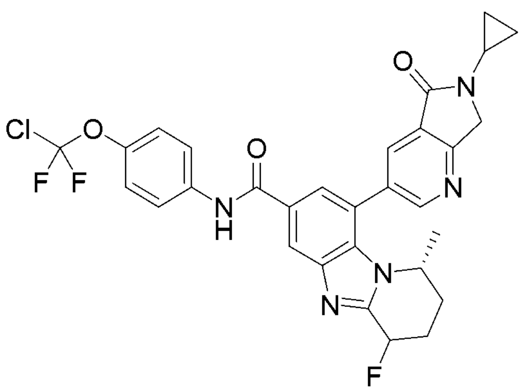

T-35

The following synthetic route was adopted:

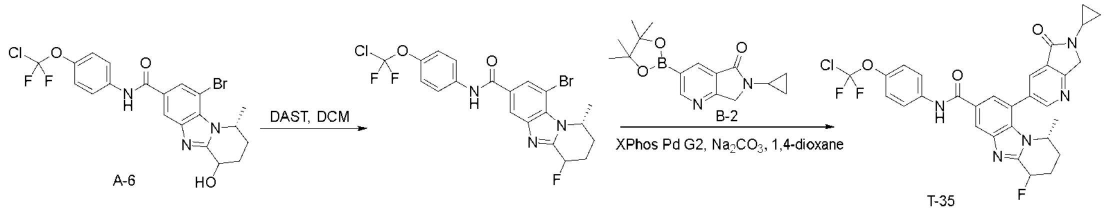


Step 1: Synthesis of compound (1R)-9-bromo-N-(4-(chlorodifluoromethoxy)phenyl)-4-fluoro-1-methyl-1,2,3,4-tetrahydrobenzo[4,5]imidazo[1,2-a]pyridine-7-carboxamide

Intermediate compoundA-6 (500 mg, 1.0 mmol) and anhydrous dichloromethane (10 mL) were added to a reaction flask. The mixture was cooled to -78 °C under nitrogen, and DAST (241 mg, 1.5 mmol) was slowly added dropwise. After the addition was completed, the mixture was stirred and reacted at a low temperature overnight. The reaction was monitored by TLC until it was completed. Water was slowly added dropwise to quench the reaction. The organic phase was concentrated, and then purified by silica gel column chromatography to give 351 mg of a light yellow solid, with a yield of 70%. LC-MS (APCI): m/z=502.3 $(M+1)^+$.

Step 2: Synthesis of compound T-35

(1R)-9-Bromo-N-(4-(chlorodifluoromethoxy)phenyl)-4-fluoro-1-methyl-1,2,3,4-tetrahydrobenzo[4,5]imidazo[1,2-a]pyridine-7-carboxamide (195 mg, 0.39 mmol), intermediate compound B-2 (176 mg, 0.588 mmol), XPhos Pd G2 (16 mg, 0.02 mmol), sodium carbonate (125 mg, 1.18 mmol) and 1,4-dioxane (5 mL) were added to a reaction flask. The mixture was purged with nitrogen for 5 minutes. The mixture was microwaved and heated to 100 °C, and reacted for 1 hour. The reaction was monitored by TLC until it was completed. The resulting mixture was concentrated, and then purified by silica gel column chromatography to give 123 mg of product, with a yield of 53%. LC-MS (APCI): m/z=596.7 $(M+1)^+$. $^1H$ NMR (400 MHz, DMSO-$d_6$) $\delta$ 10.22 (s, 1H), 8.88 (s, 1H), 8.67 (s, 1H), 8.69 (d, J = 2.3 Hz, 1H), 8.21 (d, J = 2.2 Hz, 1H), 7.65 (d, J = 8.8 Hz, 2H), 7.22 (d, J = 8.8 Hz, 2H), 5.16 (d, J = 12.6 Hz, 1H), 4.33 (m, 1H), 4.14 (m, 1H), 3.44-3.21 (m, 4H), 2.34-2.16 (m, 2H), 2.11-1.94 (m, 2H), 1.74 (d, J = 8.6 Hz, 3H).

**Example 36 Preparation of (R)-N-(4-(chlorodifluoromethoxy)phenyl)-6-(6-cyclopropyl-5-oxo-6,7-dihydro-5H-pyrrolo[3,4-b]pyridin-3-yl)-2,4-dimethyl-1,2,3,4-tetrahydrobenzo[4,5]imidazo[1,2-a]pyrazine-8-carboxamide (compound T-36)**

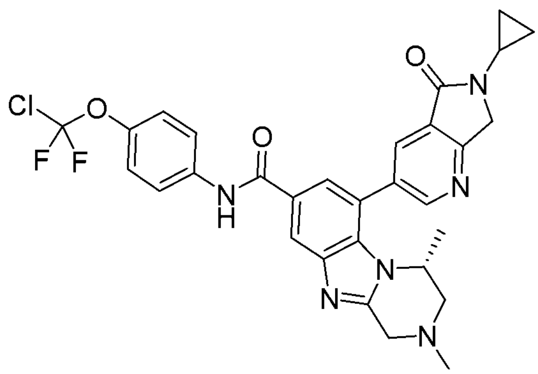

T-36

The following synthetic route was adopted:

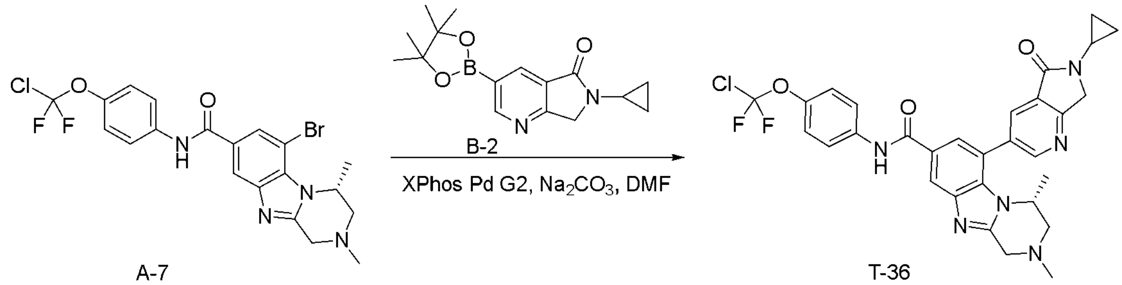


A-7 T-36

Intermediate compound A-7 (194 mg, 0.39 mmol), intermediate compound B-2 (176 mg, 0.588 mmol), XPhos Pd G2 (16 mg, 0.02 mmol), sodium carbonate (125 mg, 1.18 mmol) and DMF (5 mL) were added to a reaction flask. The mixture was purged with nitrogen for 5 minutes. The mixture was microwaved and heated to 100 °C, and reacted for 1 hour. The reaction was monitored by TLC until it was completed. The resulting mixture was concentrated, and then purified by silica gel column chromatography to give 55 mg of product, with a yield of 24%. LC-MS (APCI): m/z=593.4 $(M+1)^+$. $^1H$ NMR (400 MHz, DMSO-$d_6$) δ 10.23 (s, 1H), 8.75 (s, 1H), 8.65 (s, 1H), 8.44 (d, J = 2.3 Hz, 1H), 8.21 (d, J = 2.2 Hz, 1H), 7.63 (d, J = 8.8 Hz, 2H), 7.24 (d, J = 8.8 Hz, 2H), 4.33 (m, 1H), 4.26 (s, 2H), 3.44-3.21 (m, 4H), 3.15 (m, 1H), 3.08 (s, 3H), 2.12-1.94 (m, 2H), 1.70 (d, J = 8.6 Hz, 3H).

## Example 37 Preparation of (R)-N-(4-(chlorodifluoromethoxy)phenyl)-10-(6-cyclopropyl-5-oxo-6,7-dihydro-5H-pyrrolo[3,4-b]pyridin-3-yl)-1,3-dimethyl-2,3,4,5-tetrahydro-1H-benzo[4,5]imidazo[1,2-d][1,4]diazepine-8-carboxamide (compound T-37)

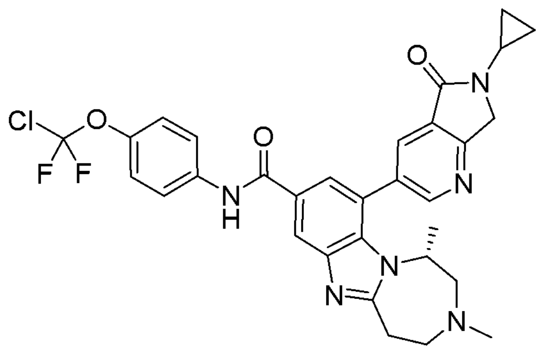

T-37

The following synthetic route was adopted:

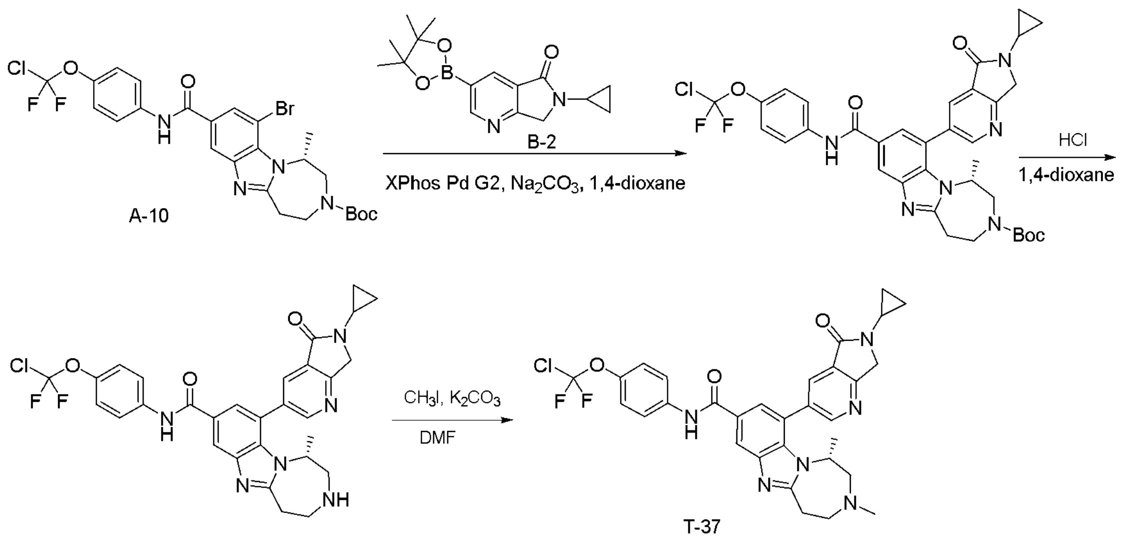


A-10 T-37

Step 1: Synthesis of compound tert-butyl (R)-10-bromo-8-((4-(chlorodifluoromethoxy)phenyl)carbamoyl)-1-methyl-1,2,4,5-tetrahydro-3H-benzo[4,5]imidazo[1,2-d][1,4]diazepine-3-carboxylate

Intermediate compound A-10 (2.3 g, 3.9 mmol), intermediate compound B-2 (1.76 g, 5.9 mmol), XPhos Pd G2 (160 mg, 0.2 mmol), sodium carbonate (1.25 g, 11.8 mmol) and 1,4-dioxane (40 mL) were added to a reaction flask. The mixture was purged with nitrogen for 5 minutes. The mixture was microwaved and heated to 100 °C, and reacted for 1 hour. The reaction was monitored by TLC until it was completed. Then, the resulting mixture was concentrated, and purified by silica gel column chromatography to give 783 mg of product, with a yield of 29%. LC-MS (APCI): m/z=693.3 $(M+1)^+$.

Step 14: Synthesis of compound (R)-N-(4-(chlorodifluoromethoxy)phenyl)-10-(6-cyclopropyl-5-oxo-6,7-dihydro-5H-pyrrolo[3,4-b]pyridin-3-yl)-1-methyl-2,3,4,5-tetrahydro-1H-benzo[4,5]imidazo[1,2-d][1,4]diazepine-8-carboxamide

tert-Butyl (R)-10-bromo-8-((4-(chlorodifluoromethoxy)phenyl)carbamoyl)-1-methyl-1,2,4,5-tetrahydro-3H-benzo[4,5]imidazo[1,2-d][1,4]diazepine-3-carboxylate (783 mg, 1.13 mmol) and a 4 M solution of hydrogen chloride in 1,4-dioxane (5 mL, 20 mmol) were added to a reaction flask. The mixture was stirred and reacted at room temperature for 1 hour. The reaction was monitored by TLC until it was completed. The resulting mixture was concentrated to remove the solvent, and directly added to the next step reaction. LC-MS (APCI): m/z=593.3 $(M+1)^+$.

Step 15: Synthesis of compound T-37

(R)-N-(4-(Chlorodifluoromethoxy)phenyl)-10-(6-cyclopropyl-5-oxo-6,7-dihydro-5H-pyrrolo[3,4-b]pyridin-3-yl)-1-methyl-2,3,4,5-tetrahydro-1H-benzo[4,5]imidazo[1,2-d][1,4]diazepine-8-carboxamide, iodomethane (160 mg, 1.13 mmol), potassium carbonate (234 mg, 1.7 mmol) and DMF (5 mL) were added to a reaction flask. The mixture was stirred and reacted at room temperature for 2 to 4 hours. The reaction was monitored by TLC until it was completed. Water was added to quench the reaction. The resulting mixture was extracted with ethyl acetate 3 to 4 times. The organic phases were combined, washed with saturated brine, concentrated, and then purified by silica gel column chromatography to give 295 mg of product, with a yield of 43%. LC-MS (APCI): m/z=607.4 $(M+1)^+$. $^1$H NMR (400 MHz, DMSO-$d_6$) δ 10.21 (s, 1H), 8.74 (s, 1H), 8.65 (s, 1H), 8.45 (d, J = 2.3 Hz, 1H), 8.22 (d, J = 2.2 Hz, 1H), 7.63 (d, J = 8.8 Hz, 2H), 7.24 (d, J = 8.8 Hz, 2H), 4.33 (m, 1H), 4.22 (t, J = 4.5 Hz, 2H), 3.68 (t, J = 4.4 Hz, 2H), 3.44-3.21 (m, 4H), 3.15 (m, 1H), 3.09 (s, 3H), 2.11-1.94 (m, 2H), 1.68 (d, J = 8.6 Hz, 3H).

**Example 38 Preparation of (R)-N-(4-(chlorodifluoromethoxy)phenyl)-6-(3-fluoropyrrolidin-1-yl)-5-(2-(7-methyl-7H-pyrrolo[2,3-d]pyrimidin-2-yl)-2,4-dihydropyrazolo[3',4':3,4]cyclopenta[1,2-b]pyridin-7-yl)nicotinamide (compound T-38)**

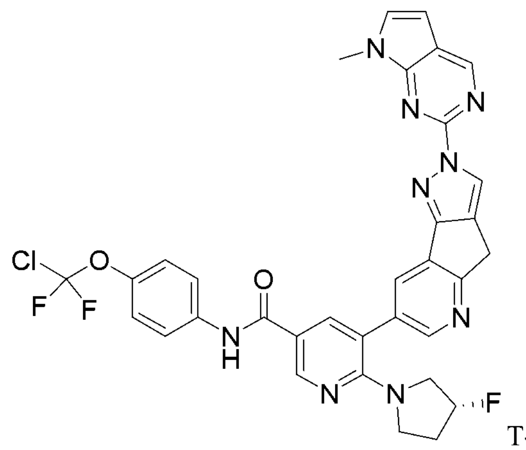

T-38

The following synthetic route was adopted:

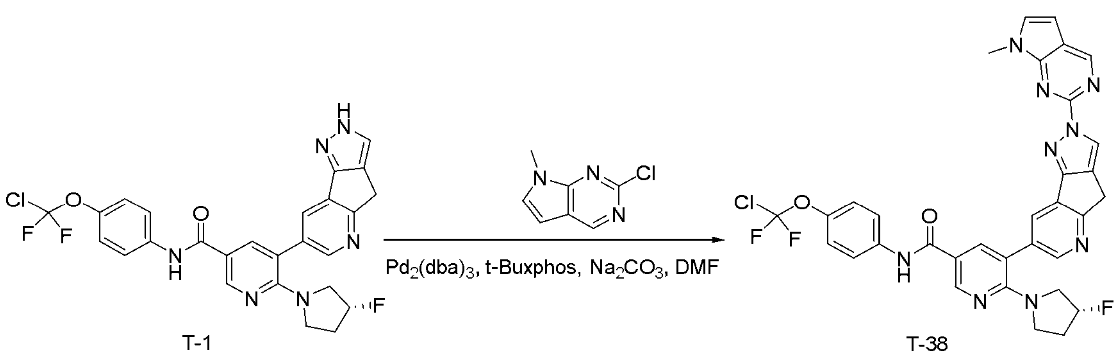


Example 38 was prepared using compound T-1 and 2-chloro-7-methylpyrrolopyrimidine as the raw materials according to the method described in example 4, with a yield of 20%. LC-MS (APCI): m/z=672.4(M+1)$^+$. $^1$H NMR (400 MHz, DMSO-$d_6$) δ 10.23 (s, 1H), 8.91 (s, 1H), 8.66 (s, 1H), 8.41 (s, 1H), 8.33 (s, 1H), 8.24 (s, 1H), 7.84 (s, 1H), 7.55 (d, J = 9.2 Hz, 2H), 7.24 (d, J = 8.4 Hz, 2H), 6.83 (d, J = 8.4 Hz, 2H), 5.27-5.15 (d, J = 22.3 Hz, 1H), 4.31 (s, 2H), 3.94 (m, 1H), 3.91-3.76 (m, 1H), 3.67 (s, 3H), 3.55 (d, J = 8.6 Hz, 2H), 2.12 (dd, J = 9.5, 3.8 Hz, 2H).

**Example 39 Preparation of (R)-N-(4-(chlorodifluoromethoxy)phenyl)-5-methyl-7-(6-methyl-5-oxo-6,7-dihydro-5H-pyrrolo[3,4-b]pyridin-3-yl)-4,5-dihydro-1H,3H-benzo[4,5]imidazo[2,1-c][1,4]oxazepine-9-carboxamide (compound T-39)**

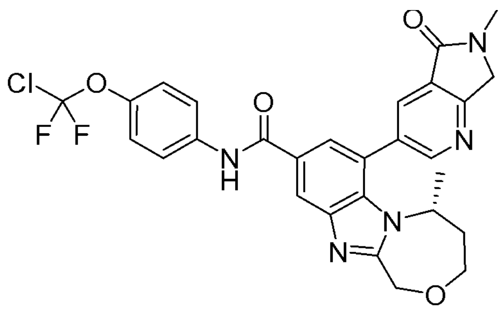

T-39

The following synthetic route was adopted:

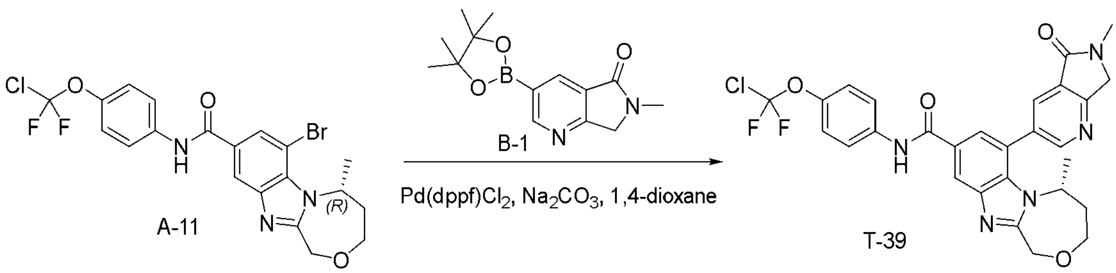


Intermediate compound A-11 (195 mg, 0.39 mmol), intermediate compound B-1 (161 mg, 0.588 mmol), [1,1'-bis(diphenylphosphino)ferrocene]dichloropalladium(II) (32 mg, 0.02 mmol), sodium carbonate (125 mg, 1.18 mmol) and 1,4-dioxane (5 mL) were added to a reaction flask. The mixture was purged with nitrogen for 5 minutes. The mixture was microwaved and heated to 100 °C, and reacted for 1 hour. The reaction was monitored by TLC until it was completed. The resulting mixture was concentrated, and purified by silica gel column chromatography to give 64 mg of product, with a yield of 29%. LC-MS (APCI): m/z=568.3 (M+1)$^+$. $^1$H NMR (400 MHz, DMSO-$d_6$) δ 10.23 (s, 1H), 8.77 (s, 1H), 8.62 (s, 1H), 8.45 (d, J = 2.3 Hz, 1H), 8.22 (d, J = 2.2 Hz, 1H), 7.63 (d, J = 8.8 Hz, 2H), 7.24 (d, J = 8.8 Hz, 2H), 5.37 (s, 2H), 4.55 (s, 2H), 4.20 (t, J = 4.5 Hz, 2H), 3.68 (m, 1H), 3.52 (s, 3H), 3.15 (m, 1H), 3.09 (m, 2H), 2.11-1.94 (m, 2H), 1.67 (d, J = 8.6 Hz, 3H).

**Example 40 Preparation of (R)-N-(4-(chlorodifluoromethoxy)phenyl)-6-(2,4-dihydropyrazolo[3',4':3,4]cyclopenta[1,2-b]pyridin-7-yl)-2,4-dimethyl-1,2,3,4-tetrahydrobenzo[4,5]imidazo[1,2-a]pyrazine-8-carboxamide (compound T-40)**

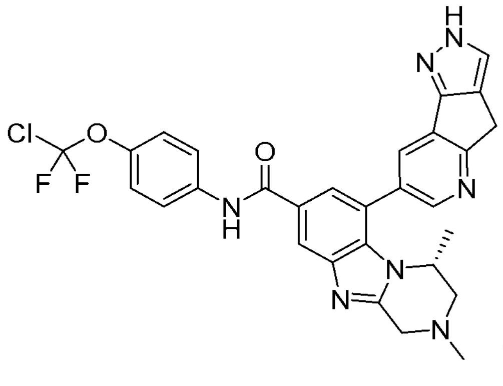

T-40

The following synthetic route was adopted:

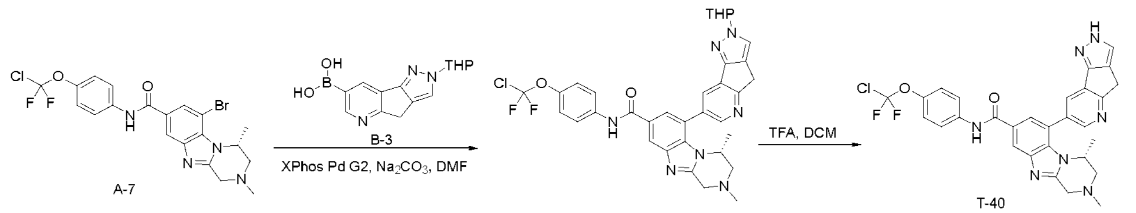


Step 1: Synthesis of compound (4R)-N-(4-(chlorodifluoromethoxy)phenyl)-2,4-dimethyl-6-(2-(tetrahydro-2H-pyran-2-yl)-2,4-dihydropyrazolo[3',4':3,4]cyclopenta[1,2-b]pyridin-7-yl)-1,2,3,4-tetrahydrobenzo[4,5]imidazo[1,2-a]pyrazine-8-carboxamide

Intermediate compound A-7 (194 mg, 0.39 mmol), intermediate compound B-3 (167 mg, 0.588 mmol), XPhos Pd G2 (16 mg, 0.02 mmol), sodium carbonate (125 mg, 1.18 mmol) and anhydrous DMF (5 mL) were added to a reaction flask. The mixture was purged with nitrogen for 5 minutes. The mixture was microwaved and heated to 90 °C, and reacted for 1 hour. The reaction was monitored by TLC until it was completed. Then, the resulting mixture was concentrated, and purified by silica gel column chromatography to give 85 mg of product, with a yield of 33%. LC-MS (APCI): m/z=660.3 $(M+1)^+$.

Step 2: Synthesis of compound T-40

(4R)-N-(4-(Chlorodifluoromethoxy)phenyl)-2,4-dimethyl-6-(2-(tetrahydro-2H-pyran-2-yl)-2,4-dihydropyrazolo [3',4':3,4]cyclopenta[1,2-b]pyridin-7-yl)-1,2,3,4-tetrahydrobenzo[4,5]imidazo[1,2-a]pyrazine-8-carboxamide (85 mg, 0.13 mmol) was dissolved in 5 mL of anhydrous dichloromethane, and 1 mL of trifluoroacetic acid was added. The mixture was stirred and reacted at room temperature for 1 to 2 hours. The reaction was monitored by TLC until it was completed. The mixture was concentrated to remove the solvent, and dichloromethane was added. The mixture was washed with saturated aqueous solution of sodium bicarbonate three times. The organic phase was concentrated, and then purified by silica gel column chromatography to give 55 mg of a white solid, with a yield of 73%. LC-MS (APCI): m/z=576.5 $(M+1)^+$. $^1$H NMR (400 MHz, DMSO-$d_6$) $\delta$ 10.21 (s, 1H), 8.92 (s, 1H), 8.61 (s, 1H), 8.44 (d, J = 2.2 Hz, 1H), 8.21 (d, J = 2.2 Hz, 1H), 7.62 (d, J = 8.8 Hz, 2H), 7.34 (d, J = 7.6 Hz, 1H), 7.24 (d, J = 8.8 Hz, 2H), 4.82 (s, 2H), 4.24 (s, 2H), 4.03 (m, 1H), 3.94-3.72 (m, 2H), 3.56 (s, 3H), 1.64 (d, J = 8.6 Hz, 3H).

**Example 41 Preparation of (R)-6-(2-acetyl-2,4-dihydropyrazolo[3',4':3,4]cyclopenta[1,2-b]pyridin-7-yl)-N-(4-(chlorodifluoromethoxy)phenyl)-2,4-dimethyl-1,2,3,4-tetrahydrobenzo[4,5]imidazo[1,2-a]pyrazine-8-carboxamide (compound T-41)**

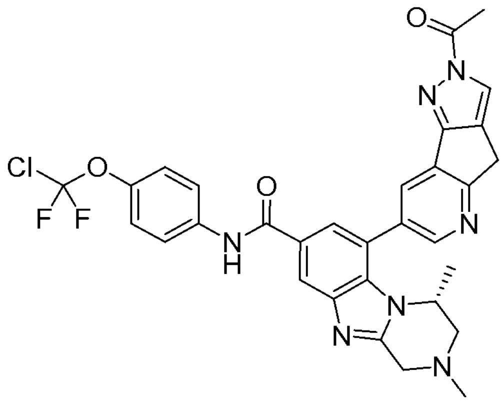

T-41

The following synthetic route was adopted:

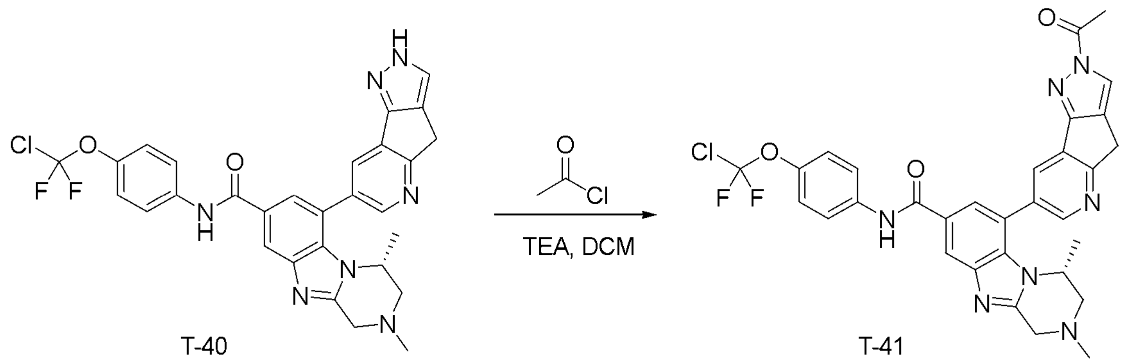


T-40 T-41

Compound T-40 (100 mg, 0.17 mmol) and triethylamine (34 mg, 0.34 mmol) were dissolved in 8 mL of anhydrous dichloromethane. Acetyl chloride (15 mg, 0.19 mmol) was slowly added dropwise in an ice bath. The mixture was warmed to room temperature, stirred and reacted under nitrogen for 2 hours. The reaction was monitored by TLC until it was completed. Water was added to quench the reaction. The resulting mixture was extracted with dichloromethane 3 to 4 times. The organic phases were combined, washed with saturated brine, concentrated, and then purified by silica gel column chromatography to give 81 mg of a target product, with a yield of 77%. LC-MS (APCI): m/z=618.7 $(M+1)^+$. $^1H$ NMR (400 MHz, DMSO-$d_6$) δ 10.23 (s, 1H), 8.94 (s, 1H), 8.62 (s, 1H), 8.38 (d, J = 2.3 Hz, 1H), 8.21 (d, J = 2.2 Hz, 1H), 7.61 (d, J = 8.8 Hz, 2H), 7.33 (d, J = 7.6 Hz, 1H), 7.24 (d, J = 8.8 Hz, 2H), 4.87 (s, 2H), 4.14 (s, 2H), 3.98 (m, 1H), 3.94-3.72 (m, 2H), 3.62 (s, 3H), 2.25 (s, 3H), 1.65 (d, J = 8.6 Hz, 3H).

**Example 42 Preparation of (R)-N-(4-(chlorodifluoromethoxy)phenyl)-4-methyl-6-(2-(methylsulfonyl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-42)**

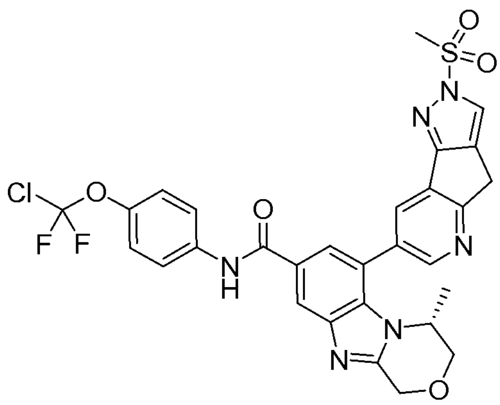

T-42

The following synthetic route was adopted:

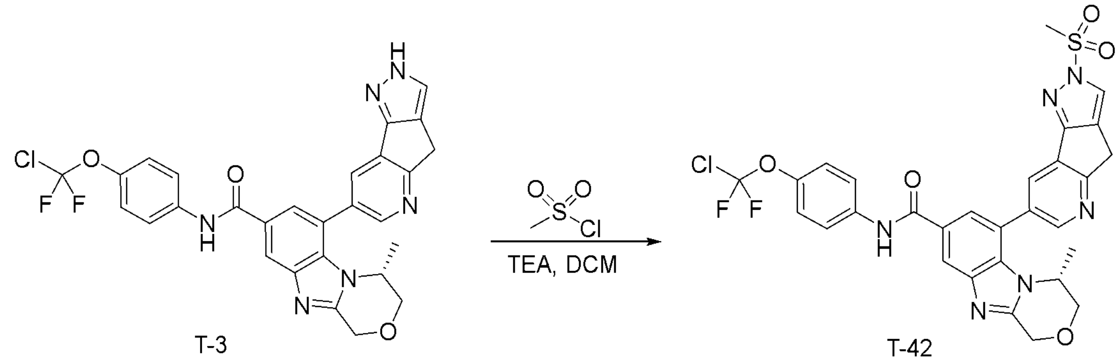


T-3 T-42

Example 42 was prepared using compound T-3 (100 mg, 0.18 mmol) and methanesulfonyl chloride (22.5 mg, 0.19 mmol) as the raw materials according to the method described in example 41, giving 58 mg of a white solid, with an overall yield of 50%. LC-MS (APCI): m/z=641.4 $(M+1)^+$. $^1H$ NMR (400 MHz, DMSO-$d_6$) δ 10.23 (s, 1H), 8.92 (s, 1H), 8.62 (s, 1H), 8.35 (d, J = 2.3 Hz, 1H), 8.21 (d, J = 2.2 Hz, 1H), 7.61 (d, J = 8.8 Hz, 2H), 7.31 (d, J = 7.6 Hz, 1H), 7.24 (d, J = 8.6 Hz, 2H), 5.06 (s, 2H), 4.14 (s, 2H), 3.96 (m, 1H), 3.94-3.71 (m, 2H), 2.88 (s, 3H), 1.65 (d, J = 8.6 Hz, 3H).

**Example 43 Preparation of (R)-6-(2-acetyl-2,4-dihydropyrazolo[3',4':3,4]cyclopenta[1,2-b]pyridin-7-yl)-N-(4-(chlorodifluoromethoxy)phenyl)-4-methyl-3,4-dihydro-1H-benzo[4,5]imidazo[2,1-c][1,4]oxazine-8-carboxamide (compound T-43)**

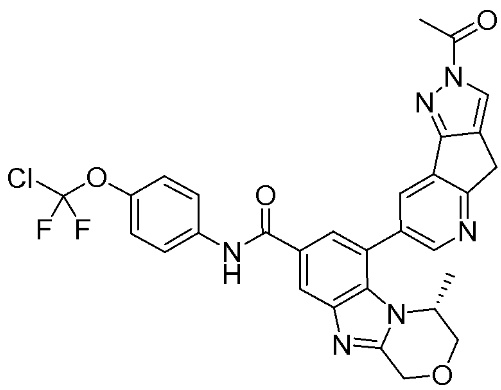

T-43

The following synthetic route was adopted:

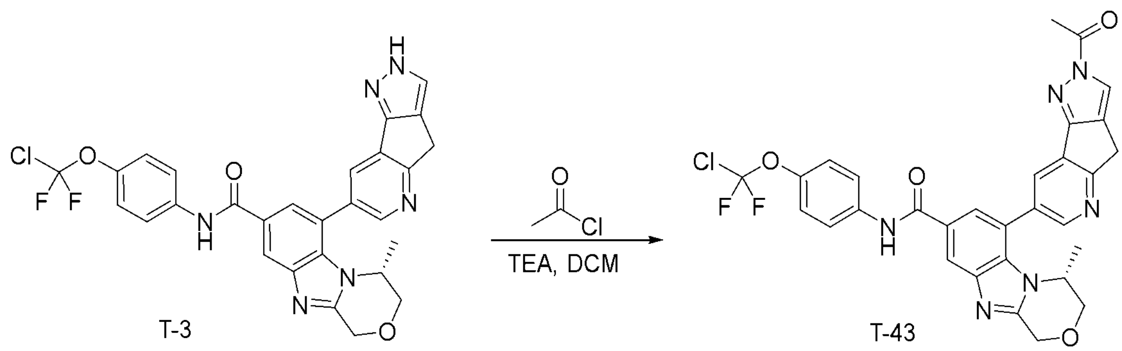


T-3 T-43

Example 43 was prepared using compound T-3 (100 mg, 0.18 mmol) and acetyl chloride (15 mg, 0.19 mmol) as the raw materials according to the method described in example 41, giving 67 mg of a white solid, with an overall yield of 64%. LC-MS (APCI): m/z=605.2 $(M+1)^+$. $^1H$ NMR (400 MHz, DMSO-$d_6$) δ 10.22 (s, 1H), 8.92 (s, 1H), 8.61 (s, 1H), 8.35 (d, J = 2.3 Hz, 1H), 8.21 (d, J = 2.2 Hz, 1H), 7.61 (d, J = 8.8 Hz, 2H), 7.31 (d, J = 7.7 Hz, 1H), 7.24 (d, J = 8.6 Hz, 2H), 5.06 (s, 2H),

4.14 (s, 2H), 3.94 (m, 1H), 3.94-3.71 (m, 2H), 2.54 (s, 3H), 1.65 (d, J = 8.6 Hz, 3H).

**Example 44 Preparation of (R)-N-(4-(chlorodifluoromethoxy)phenyl)-6-(3-fluoropyrrolidin-1-yl)-5-(2-(thieno[2,3-d]pyrimidin-2-yl)-2,4-dihydropyrazolo[3',4':3,4]cyclopenta[1,2-b]pyridin-7-yl)nicotinamide (compound T-44)**

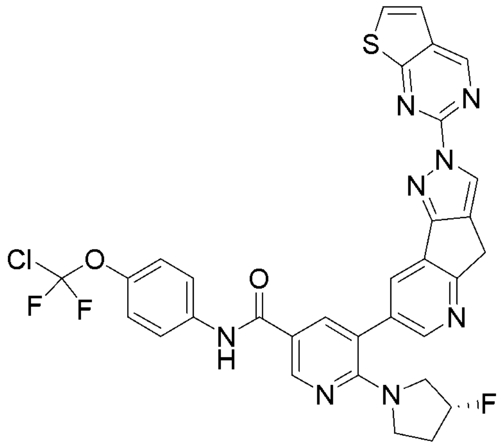

T-44

The following synthetic route was adopted:

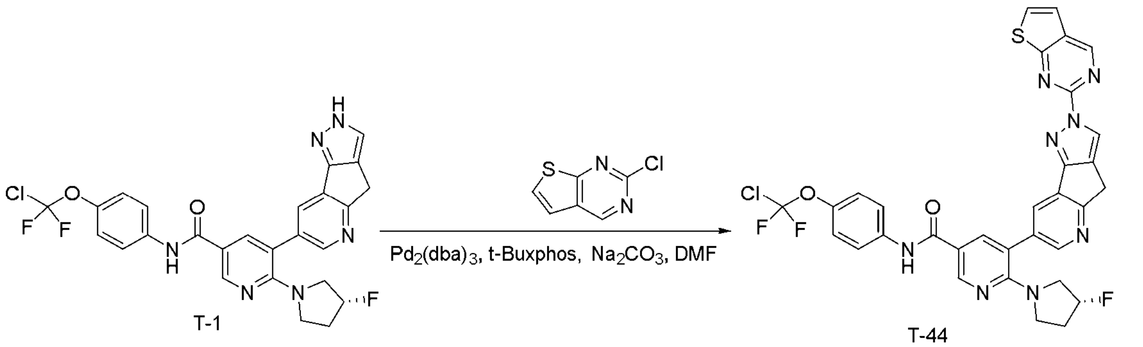


Example 44 was prepared using compound T-1 and 2-chlorothieno[2,3-d]pyrimidine as the raw materials according to the method described in example 4, with a yield of 22%. LC-MS (APCI): m/z=675.7$(M+1)^+$. $^1H$ NMR (400 MHz, DMSO-$d_6$) δ 10.22 (s, 1H), 8.91 (s, 1H), 8.66 (s, 1H), 8.41 (s, 1H), 8.31 (s, 1H), 8.22 (s, 1H), 7.84 (s, 1H), 7.55 (d, J = 8.2 Hz, 2H), 7.22 (d, J = 8.4 Hz, 2H), 6.85 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.14 (dd, J = 9.5, 3.8 Hz, 2H).

**Example 45 Preparation of (R)-N-(4-(chlorodifluoromethoxy)phenyl)-4-methyl-6-(2-propionyl-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-45)**

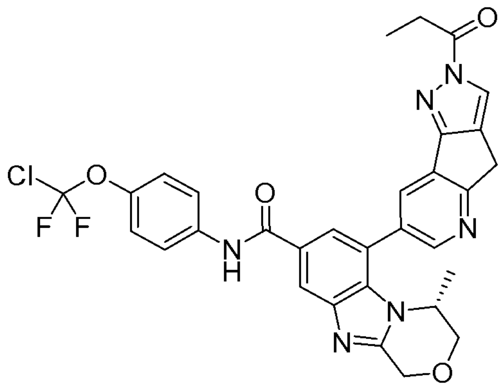

T-45

The following synthetic route was adopted:

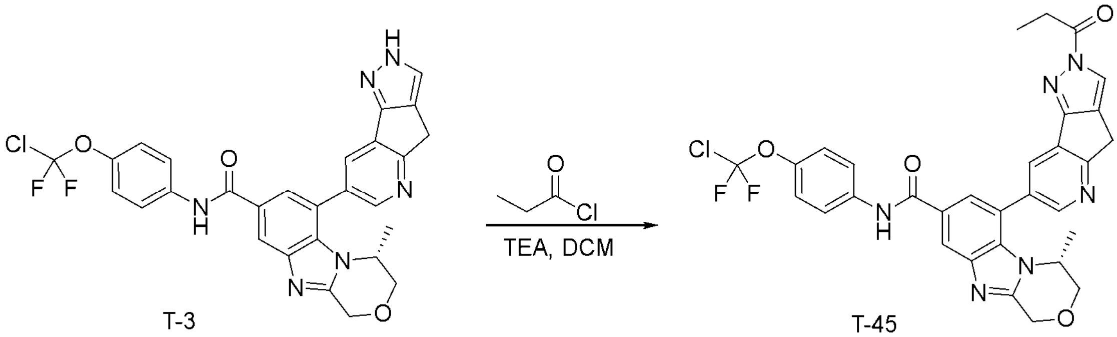


T-3

T-45

Example 45 was prepared using compound T-3 (100 mg, 0.18 mmol) and propionyl chloride (18 mg, 0.19 mmol) as the raw materials according to the method described in example 41, giving 79 mg of a white solid, with an overall yield of 71%. LC-MS (APCI): m/z=619.4 $(M+1)^+$. $^1H$ NMR (400 MHz, DMSO-$d_6$) δ 10.23 (s, 1H), 8.92 (s, 1H), 8.62 (s, 1H), 8.34 (d, J = 2.3 Hz, 1H), 8.21 (d, J = 2.2 Hz, 1H), 7.61 (d, J = 8.8 Hz, 2H), 7.31 (d, J = 7.6 Hz, 1H), 7.24 (d, J = 8.6 Hz, 2H), 5.06 (s, 2H), 4.14 (s, 2H), 3.93 (m, 1H), 3.94-3.71 (m, 2H), 2.27 (m, 2H), 1.65 (d, J = 8.6 Hz, 3H), 1.47 (t, J = 4.6 Hz, 3H).

**Example 46 Preparation of (R)-N-(4-(chlorodifluoromethoxy)phenyl)-6-(2-isobutyryl-2,4-dihydropyrazolo [3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-46)**

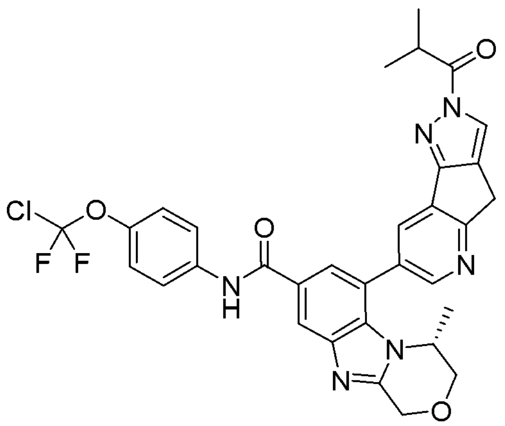

T-46

The following synthetic route was adopted:

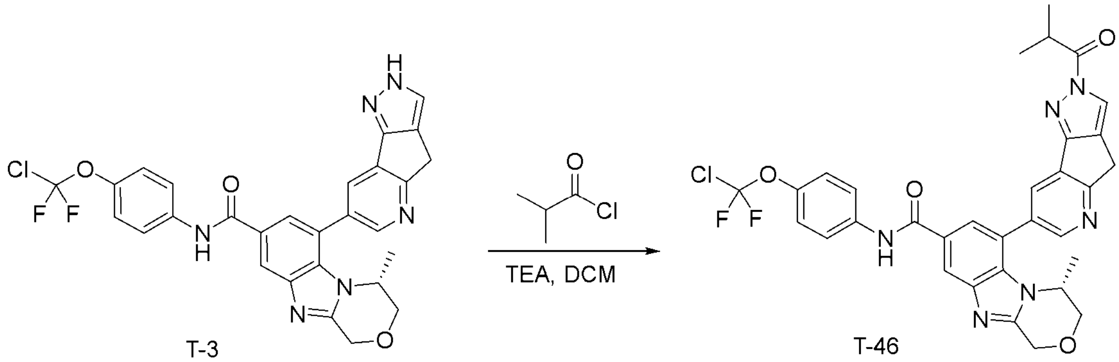


T-3 T-46

Example 46 was prepared using compound T-3 (100 mg, 0.18 mmol) and isobutyryl chloride (20 mg, 0.19 mmol) as the raw materials according to the method described in example 41, giving 55 mg of a white solid, with an overall yield of 48%. LC-MS (APCI): m/z=633.8 (M+1)$^+$. $^1$H NMR (400 MHz, DMSO-$d_6$) δ 10.23 (s, 1H), 8.94 (s, 1H), 8.61 (s, 1H), 8.34 (d, J = 2.2 Hz, 1H), 8.21 (d, J = 2.2 Hz, 1H), 7.60 (d, J = 8.8 Hz, 2H), 7.31 (d, J = 7.6 Hz, 1H), 7.22 (d, J = 8.6 Hz, 2H), 5.06 (s, 2H), 4.14 (s, 2H), 3.93 (m, 1H), 3.94-3.71 (m, 2H), 2.07 (m, 1H), 1.65 (d, J = 8.6 Hz, 3H), 1.47 (d, J = 4.2 Hz, 6H).

**Example 47 Preparation of (R)-N-(4-(chlorodifluoromethoxy)phenyl)-6-(2-(cyclopropylcarbonyl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-47)**

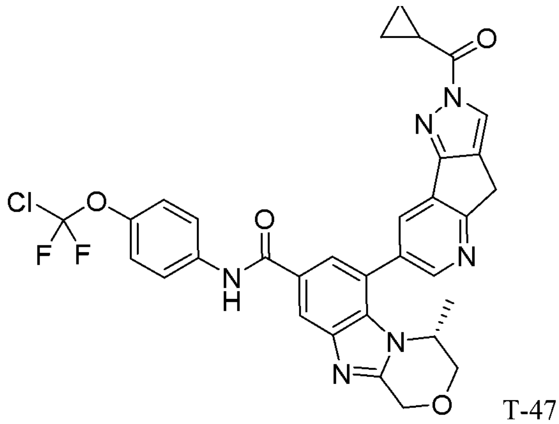

T-47

The following synthetic route was adopted:

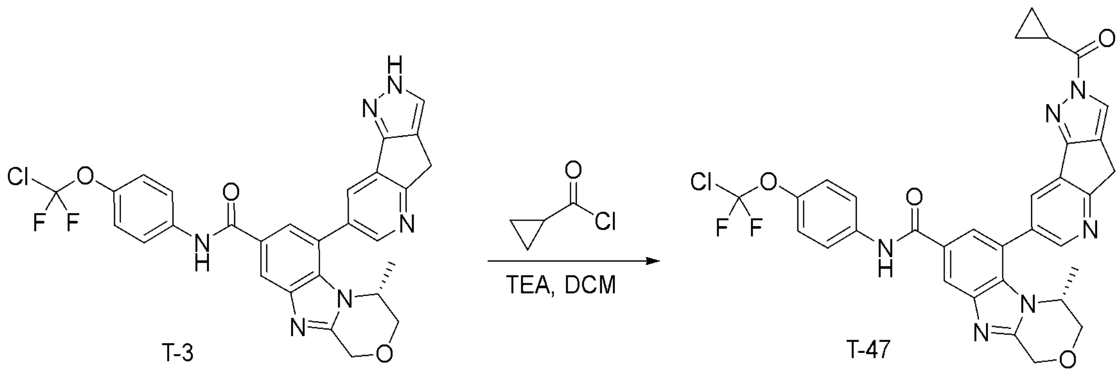


T-3 T-47

Example 47 was prepared using compound T-3 (100 mg, 0.18 mmol) and cyclopropanecarbonyl chloride (20 mg, 0.19 mmol) as the raw materials according to the method described in example 41, giving 31 mg of a white solid, with an overall yield of 27%. LC-MS (APCI): m/z=631.1 (M+1)$^+$. $^1$H NMR (400 MHz, DMSO-$d_6$) δ 10.21 (s, 1H), 8.92 (s, 1H), 8.62 (s, 1H), 8.32 (d, J = 2.3 Hz, 1H), 8.22 (d, J = 2.2 Hz, 1H), 7.61 (d, J = 8.8 Hz, 2H), 7.31 (d, J = 7.6 Hz, 1H), 7.24 (d, J = 8.6 Hz, 2H),

5.06 (s, 2H), 4.14 (s, 2H), 3.93 (m, 1H), 3.94-3.71 (m, 2H), 2.69 (m, 1H), 2.23-2.01 (m, 4H), 1.65 (d, J = 8.6 Hz, 3H).

**Example 48 Preparation of (R)-N-(4-(chlorodifluoromethoxy)phenyl)-6-(2-isopropyl-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-48)**

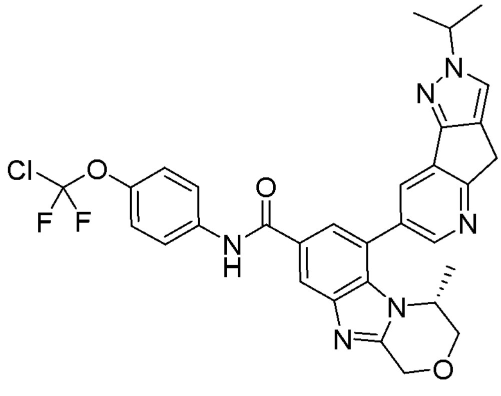

T-48

The following synthetic route was adopted:

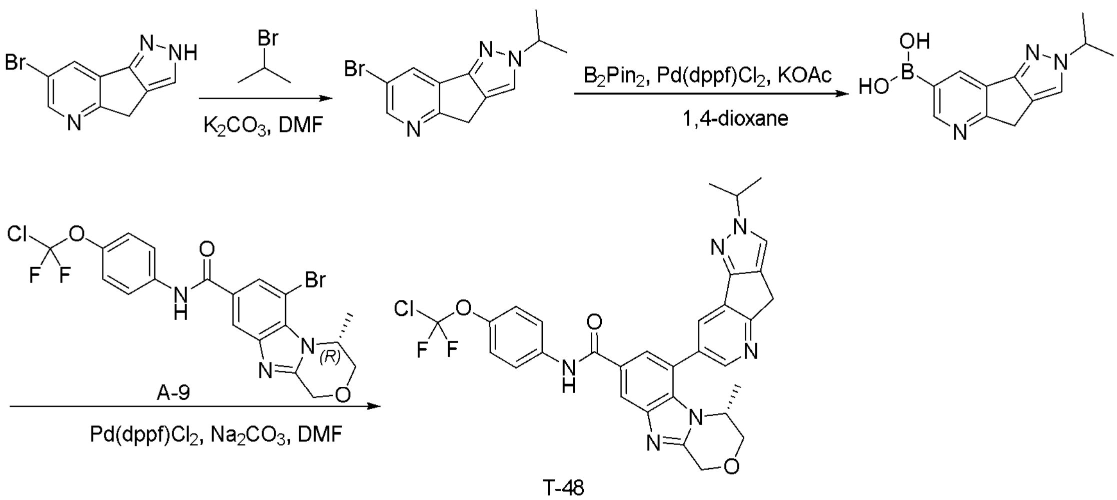


T-48

Example 48 was prepared using 7-bromo-2,4-dihydropyrazolo[3',4':3,4]cyclopenta[1,2-b]pyridine, 2-bromopropane and intermediate compound A-9 as the raw materials according to the method described in example 16, with an overall yield of 28%. LC-MS (APCI): m/z=605.2 $(M+1)^+$. $^1$H NMR (400 MHz, DMSO-$d_6$) δ 10.22 (s, 1H), 8.92 (s, 1H), 8.62 (s, 1H), 8.31 (d, J = 2.3 Hz, 1H), 8.21 (d, J = 2.2 Hz, 1H), 7.61 (d, J = 8.8 Hz, 2H), 7.32 (d, J = 7.6 Hz, 1H), 7.24 (d, J = 8.6 Hz, 2H), 5.06 (s, 2H), 4.14 (s, 2H), 3.93 (m, 1H), 3.94-3.71 (m, 2H), 3.13 (m, 1H), 1.65 (d, J = 8.6 Hz, 3H), 1.44 (d, J = 4.3 Hz, 6H).

**Example 49 Preparation of (R)-N-(4-(chlorodifluoromethoxy)phenyl)-4-methyl-6-(2-(2,2,2-trifluoroethyl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-49)**

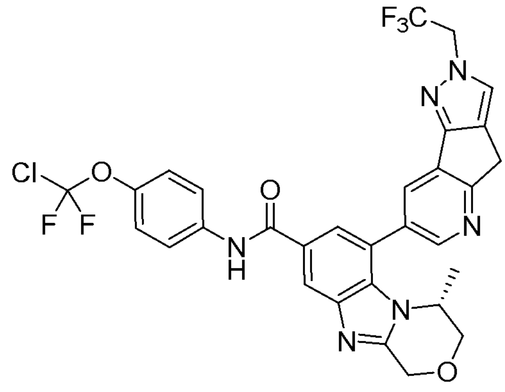

T-49

The following synthetic route was adopted:

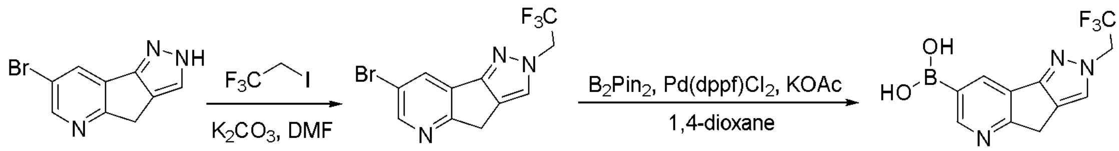


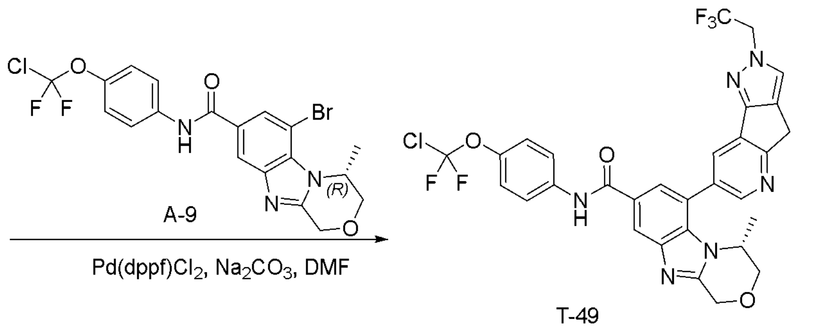


T-49

Example 49 was prepared using 7-bromo-2,4-dihydropyrazolo[3',4':3,4]cyclopenta[1,2-b]pyridine, 2-iodo-1,1,1-trifluoroethane and intermediate compound A-9 as the raw materials according to the method described in example 16, with an overall yield of 11%. LC-MS (APCI): m/z=645.7 $(M+1)^+$. $^1$H NMR (400 MHz, DMSO-$d_6$) δ 10.22 (s, 1H), 8.92 (s, 1H), 8.62 (s, 1H), 8.34 (d, J = 2.3 Hz, 1H), 8.21 (d, J = 2.2 Hz, 1H), 7.61 (d, J = 8.8 Hz, 2H), 7.31 (d, J = 7.6 Hz, 1H), 7.24 (d, J = 8.6 Hz, 2H), 5.04 (s, 2H), 4.17 (s, 2H), 3.93 (m, 1H), 3.94-3.71 (m, 2H), 3.66 (s, 2H), 1.62 (d, J = 8.6 Hz, 3H).

**Example 50 Preparation of N-(4-(chlorodifluoromethoxy)phenyl)-6-((R)-3-fluoropyrrolidin-1-yl)-5-(2-(5-((R)-3-fluoropyrrolidin-1-yl)pyrimidin-2-yl)-2,4-dihydropyrazolo[3',4':3,4]cyclopenta[1,2-b]pyridin-7-yl)nicotinamide (compound T-50)**

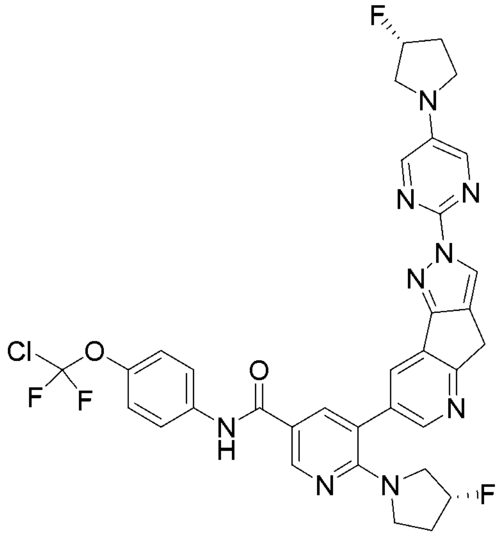

T-50

The following synthetic route was adopted:

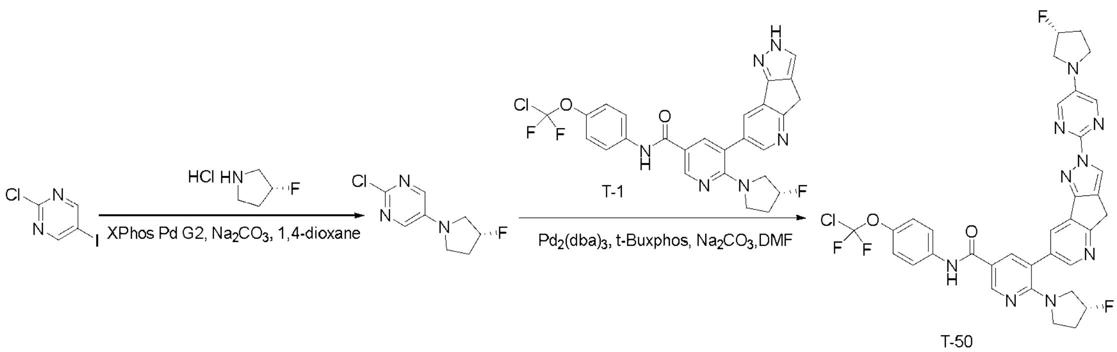


Step 1: Synthesis of compound (R)-2-chloro-5-(3-fluoropyrrolidin-1-yl)pyrimidine

2-Chloro-5-iodopyrimidine (1.0 g, 4.2 mmol), (R)-3-fluoropyrrolidine hydrochloride (1.05 g, 8.4 mmol), XPhos Pd G2 (160 mg, 0.21 mmol), sodium carbonate (1.1 g, 10.5 mmol) and 1,4-dioxane (10 mL) were added to a reaction flask. The mixture was purged with nitrogen for 3 minutes. The mixture was heated to 90 °C, stirred and reacted for 5 hours. The reaction was monitored by TLC until it was completed. The resulting mixture was concentrated, and then purified by silica gel column chromatography to give 464 mg of a yellow oily liquid, with a yield of 51%. LC-MS (APCI): m/z=202.2 $(M+1)^+$.

Step 2: Synthesis of compound T-50

Example 50 was prepared using compound T-1 and (R)-2-chloro-5-(3-fluoropyrrolidin-1-yl)pyrimidine as the raw materials according to the method described in example 4, with a yield of 17%. LC-MS (APCI): m/z=706.7$(M+1)^+$. $^1$H NMR (400 MHz, DMSO-$d_6$) δ 10.23 (s, 1H), 8.91 (s, 1H), 8.66 (s, 1H), 8.41 (s, 1H), 8.31 (s, 1H), 8.21 (s, 1H), 8.11 (s, 1H), 7.84 (s, 1H), 7.24 (d, J = 8.4 Hz, 2H), 6.83 (d, J = 8.4 Hz, 2H), 5.27-5.15 (m, 2H), 4.33 (s, 2H), 3.94 (m, 2H), 3.91-3.74 (m, 2H), 3.55 (m, 4H), 2.11-1.99 (m, 4H).

**Example 51 Preparation of (R)-N-(4-(chlorodifluoromethoxy)phenyl)-6-(2-(difluoromethyl)-2,4-dihydropyrazolo [3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-51)**

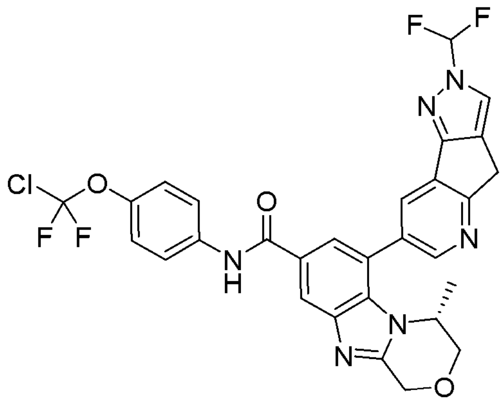

T-51

The following synthetic route was adopted:

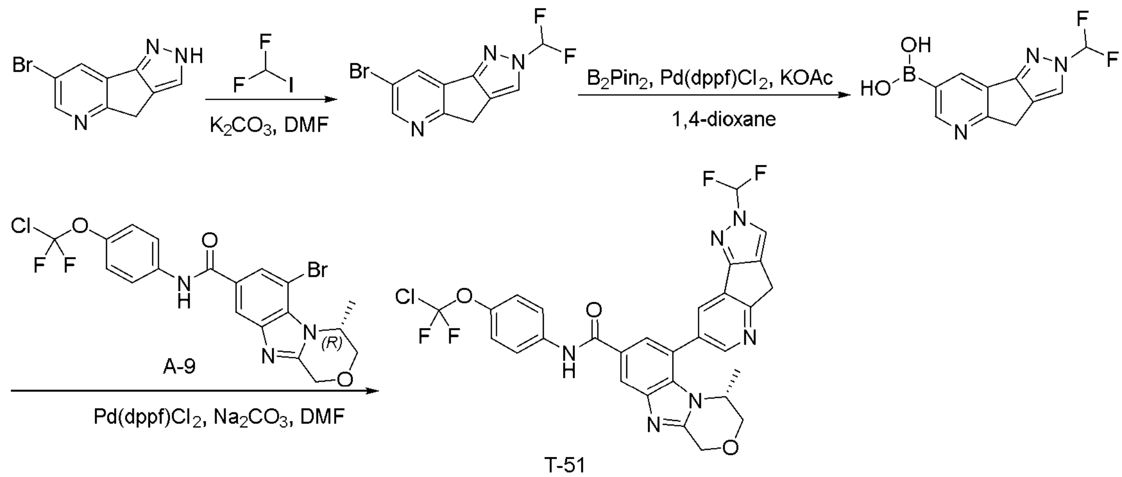


A-9

T-51

Example 51 was prepared using 7-bromo-2,4-dihydropyrazolo[3',4':3,4]cyclopenta[1,2-b]pyridine, difluoroiodomethane and intermediate compound A-9 as the raw materials according to the method described in example 16, with an overall yield of 12%. LC-MS (APCI): m/z=612.4 $(M+1)^+$. $^1H$ NMR (400 MHz, DMSO-$d_6$) δ 10.23 (s, 1H), 8.88 (s, 1H), 8.62 (s, 1H), 8.34 (d, J = 2.3 Hz, 1H), 8.21 (d, J = 2.2 Hz, 1H), 7.61 (d, J = 8.8 Hz, 2H), 7.31 (d, J = 7.6 Hz, 1H), 7.24 (d, J = 8.6 Hz, 2H), 5.06 (s, 2H), 4.42 (s, 1H), 4.14 (s, 2H), 3.98 (m, 1H), 3.95-3.71 (m, 2H), 1.65 (d, J = 8.6 Hz, 3H).

**Example 52 Preparation of (R)-N-(4-(chlorodifluoromethoxy)phenyl)-4-methyl-6-(2-(oxetan-3-yl)-2,4-dihydropyrazolo[3',4':3,4]cyclopenta[1,2-b]pyridin-7-yl)-3,4-dihydro-1H-benzo[4,5]imidazo[2,1-c] [1,4]oxazine-8-carboxamide (compound T-52)**

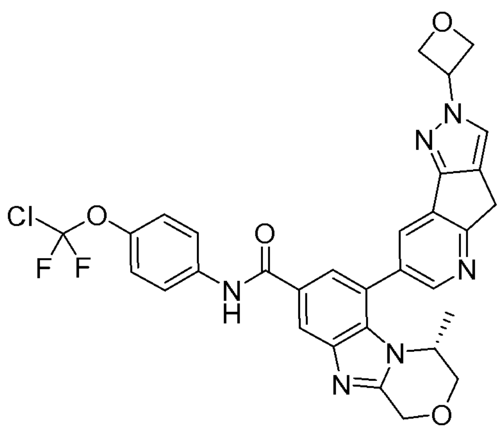

T-52

The following synthetic route was adopted:

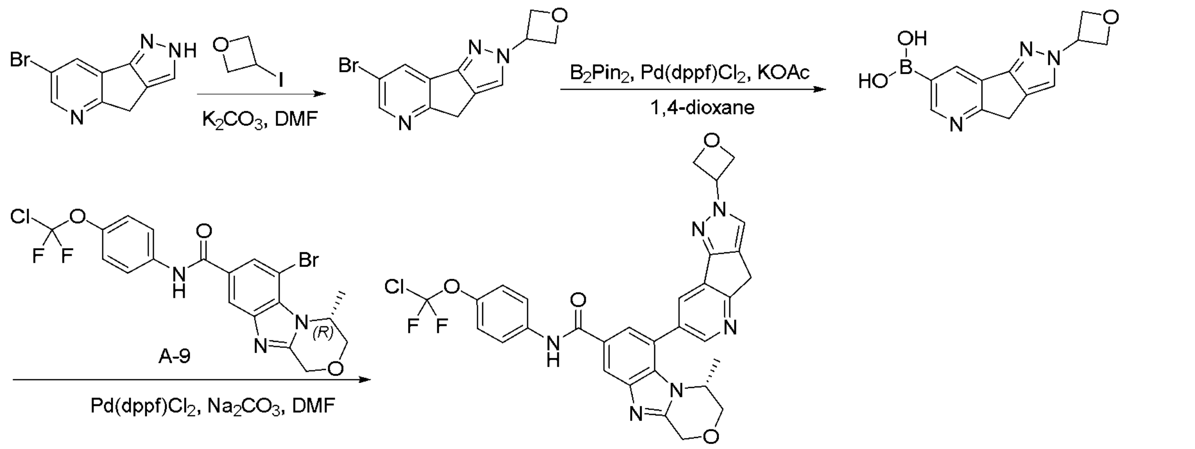


T-52

Example 52 was prepared using 7-bromo-2,4-dihydropyrazolo[3',4':3,4]cyclopenta[1,2-b]pyridine, 3-iodooxetane and intermediate compound A-9 as the raw materials according to the method described in example 16, with an overall yield of 32%. LC-MS (APCI): m/z=619.5 (M+1)$^+$. $^1$H NMR (400 MHz, DMSO-*d$_6$*) δ 10.24 (s, 1H), 8.91 (s, 1H), 8.62 (s, 1H), 8.34 (d, J = 2.3 Hz, 1H), 8.21 (d, J = 2.2 Hz, 1H), 7.61 (d, J = 8.8 Hz, 2H), 7.31 (d, J = 7.6 Hz, 1H), 7.24 (d, J = 8.6 Hz, 2H), 5.06 (s, 2H), 4.38-4.26 (m, 4H), 4.14 (s, 2H), 3.93 (m, 1H), 3.94-3.71 (m, 2H), 3.48 (m, 1H), 1.68 (d, J = 8.6 Hz, 3H).

**Example 53 Preparation of (R)-N-(4-(chlorodifluoromethoxv)phenyl)-4-methyl-6-(2-(methyl-*d$_3$*)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-53)**

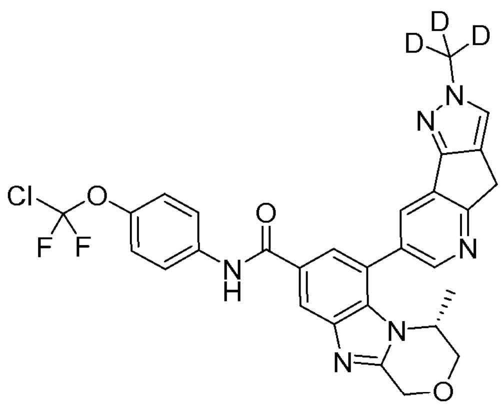

T-53

The following synthetic route was adopted:

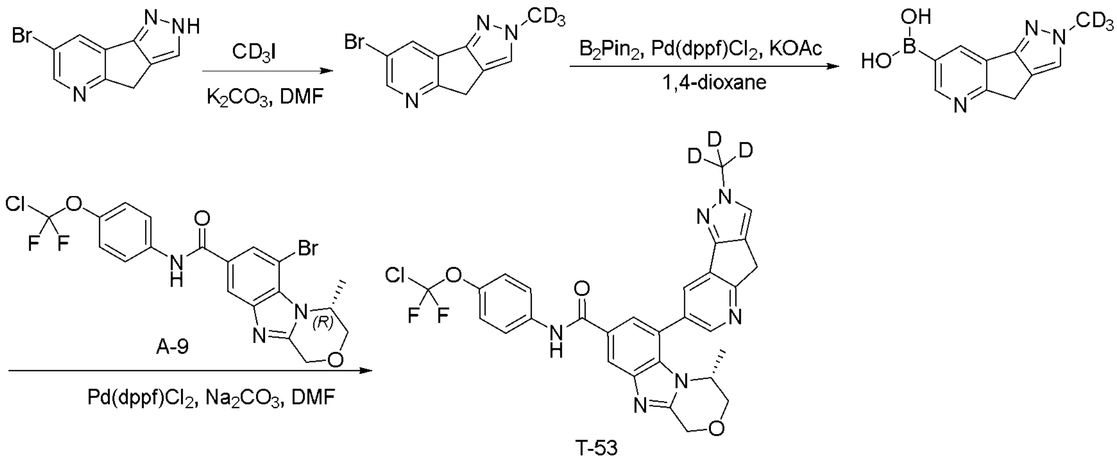


T-53

Example 53 was prepared using 7-bromo-2,4-dihydropyrazolo[3',4':3,4]cyclopenta[1,2-b]pyridine, deuterated iodomethane and intermediate compound A-9 as the raw materials according to the method described in example 16, with an overall yield of 27%. LC-MS (APCI): m/z=580.2 $(M+1)^+$. $^1H$ NMR (400 MHz, DMSO-$d_6$) $\delta$ 10.23 (s, 1H), 8.92 (s, 1H), 8.62 (s, 1H), 8.34 (d, J = 2.3 Hz, 1H), 8.21 (d, J = 2.2 Hz, 1H), 7.61 (d, J = 8.8 Hz, 2H), 7.31 (d, J = 7.6 Hz, 1H), 7.24 (d, J = 8.6 Hz, 2H), 5.06 (s, 2H), 4.14 (s, 2H), 3.93 (m, 1H), 3.94-3.71 (m, 2H), 1.65 (d, J = 8.6 Hz, 3H).

**Example** 54 **Preparation** of **(R)-N-(4-(chlorodifluoromethoxyphenyl)-4-methyl-6-(2-(propan-2-yl-$d_7$)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-54)**

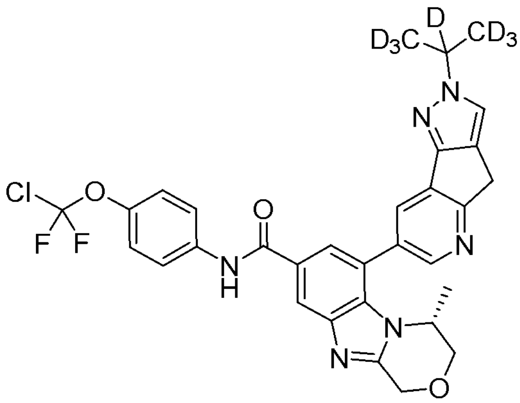

T-54

The following synthetic route was adopted:

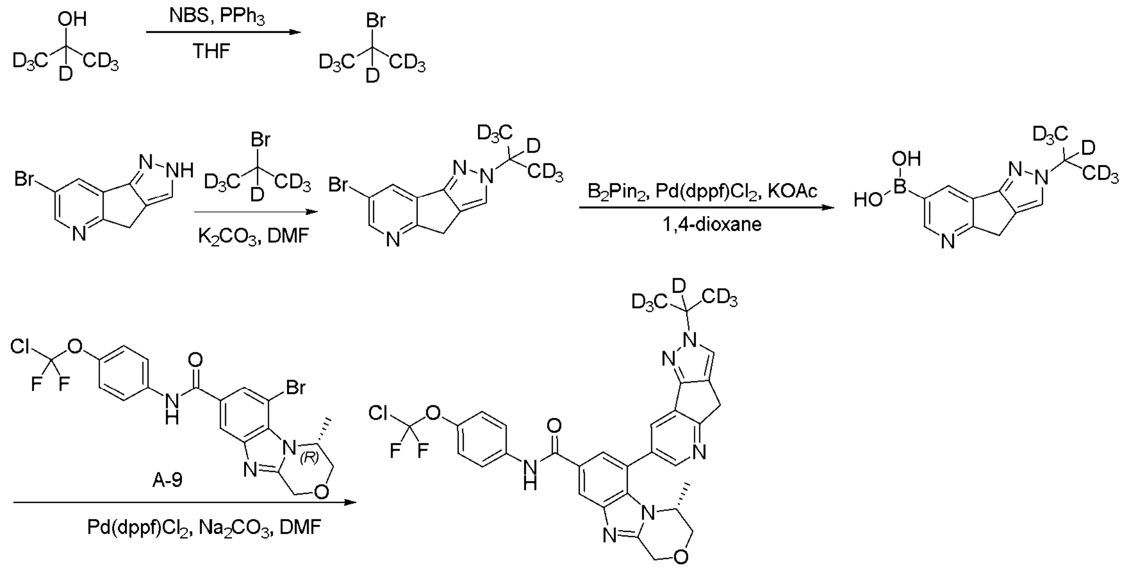


T-54

Step 1: Synthesis of compound 2-bromopropane-1,1,1,2,3,3,3-*d*$_7$

Deuterated isopropanol (2.0 g, 29.8 mmol) and triphenylphosphine (9.4 g, 35.8 mmol) were dissolved in 20 mL of anhydrous tetrahydrofuran. The mixture was cooled to 0 °C under nitrogen, and a solution of NBS (5.8 g, 32.8 mmol) in dichloromethane (10 mL) was slowly added dropwise. After the addition was completed, the mixture was stirred and reacted at room temperature under nitrogen overnight. The reaction was monitored by TLC until it was completed. Water was added to quench the reaction. The resulting mixture was extracted with dichloromethane 3 to 4 times. The organic phases were combined, washed with saturated brine, concentrated, and then purified by silica gel column chromatography to give 1.96 g of a target product, with a yield of 51%.

Step 2: Synthesis of compound T-54

Example 54 was prepared using 7-bromo-2,4-dihydropyrazolo[3',4':3,4]cyclopenta[1,2-b]pyridine, 2-bromopropane-1,1,1,2,3,3,3-*d*$_7$ and intermediate compound A-9 as the raw materials according to the method described in example 16, with an overall yield of 23%. LC-MS (APCI): m/z=612.7 $(M+1)^+$. $^1$H NMR (400 MHz, DMSO-*d*$_6$) δ 10.22 (s, 1H), 8.92 (s, 1H), 8.62 (s, 1H), 8.34 (d, J = 2.3 Hz, 1H), 8.23 (d, J = 2.3 Hz, 1H), 7.64 (d, J = 8.8 Hz, 2H), 7.31 (d, J = 7.6 Hz, 1H), 7.24 (d, J = 8.6 Hz, 2H), 5.06 (s, 2H), 4.14 (s, 2H), 3.93 (m, 1H), 3.94-3.71 (m, 2H), 1.64 (d, J = 8.6 Hz, 3H).

**Example 55 Preparation of N-(4-(chlorodifluoromethoxy)phenyl)-6-((R)-3-fluoropyrrolidin-1-yl)-5-(2-(5-((S)-3-fluoropyrrolidin-1-yl)pyrimidin-2-yl)-2,4-dihydropyrazolo[3',4':3,4]cyclopenta[1,2-b]pyridin-7-yl)nicotinamide (compound T-55)**

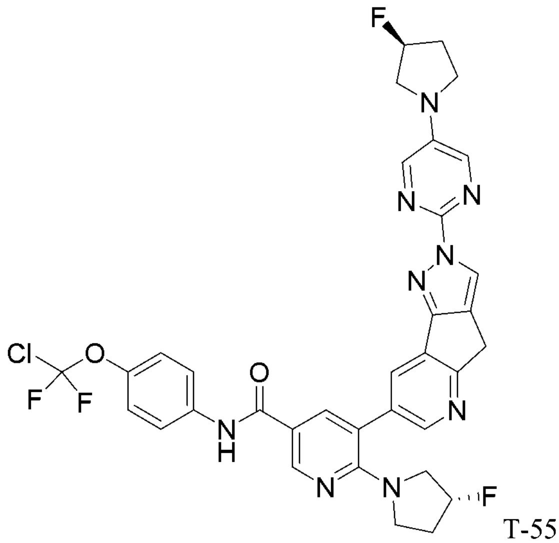

T-55

The following synthetic route was adopted:

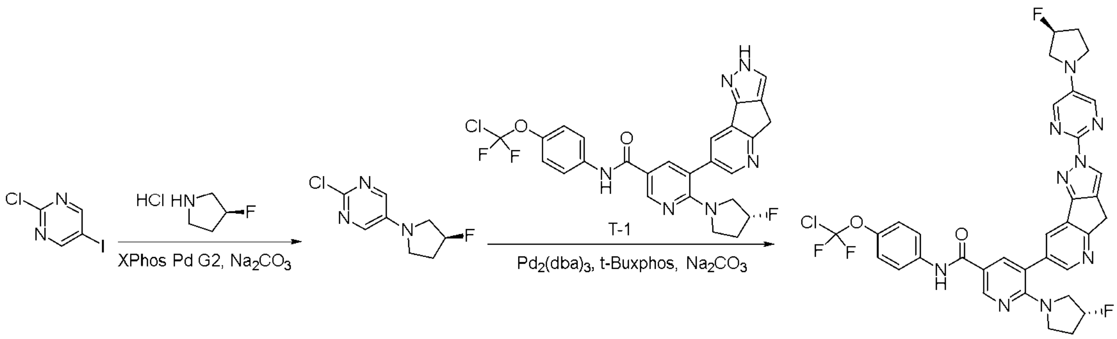


Compound example 55 was prepared using 2-chloro-5-iodopyrimidine and (S)-3-fluoropyrrolidine hydrochloride as the raw materials according to the method described in example 50, with a yield of 19%. LC-MS (APCI): m/z=706.4$(M+1)^+$. $^1H$ NMR (400 MHz, DMSO-$d_6$) δ 10.21 (s, 1H), 8.93 (s, 1H), 8.66 (s, 1H), 8.41 (s, 1H), 8.32 (s, 1H), 8.21 (s, 1H), 8.11 (s, 1H), 7.82 (s, 1H), 7.24 (d, J = 8.4 Hz, 2H), 6.81 (d, J = 8.4 Hz, 2H), 5.27-5.15 (m, 2H), 4.33 (s, 2H), 3.94 (m, 2H), 3.91-3.74 (m, 2H), 3.55 (m, 4H), 2.10-1.97 (m, 4H).

Example 56 Preparation of (R)-6-(2-(2-(tert-butoxy)ethyl)-2,4-**dihydropyrazolo[3',4':3,4]cyclopenta[1,2-b]pyridin-7-yl)-N-(4-(chlorodifluoromethoxy)phenyl)-4-methyl-3,4-dihydro-1H-benzo[4,5]imidazo[2,1-c][1,4]oxazine-8-carboxamide (compound T-56-A) and (R)-6-(1-(2-(tert-butoxy)ethyl)-1,4-dihydropyrazolo[3',4':3,4]cyclopenta[1,2-b]pyridin-7-yl)-N-(4-(chlorodifluoromethoxy)phenyl)-4-methyl-3,4-dihydro-1H-benzo[4,5]imidazo[2,1-c][1,4]oxazine-8-carboxamide (compound T-56-B)**

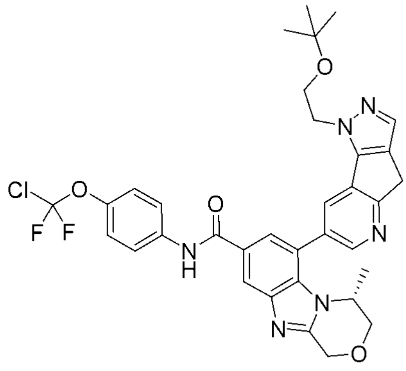

T-56-A,

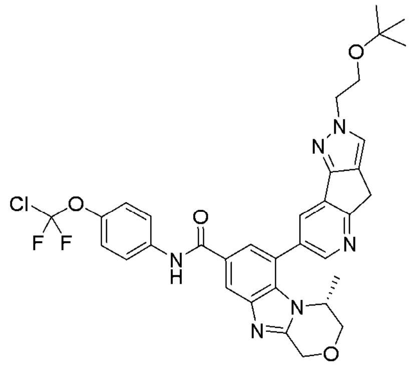

T-56-B

The following synthetic route was adopted:

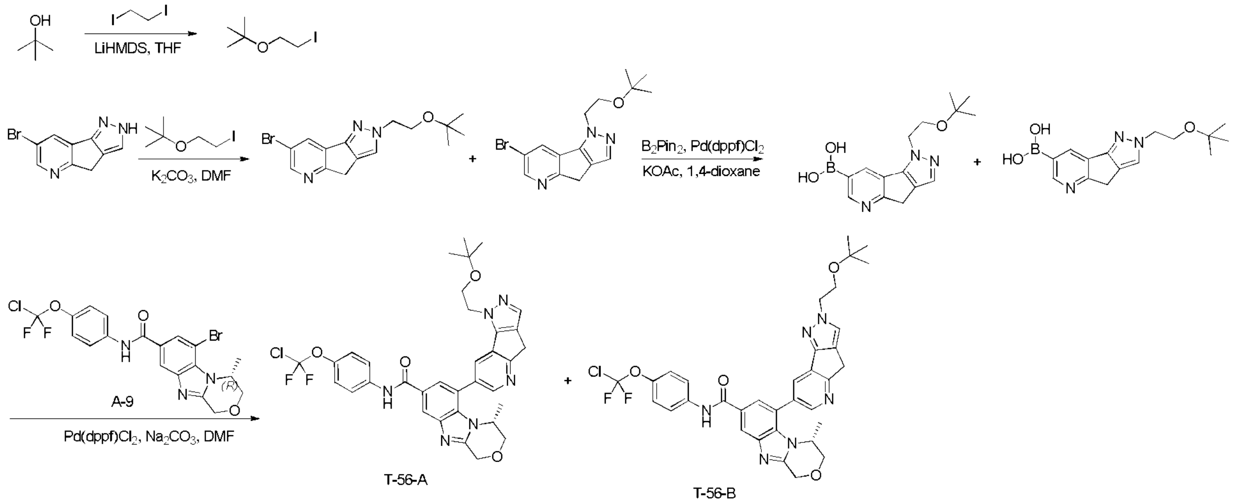


Step 1: Synthesis of compound 2-(2-iodoethoxy)-2-methylpropane

tert-Butanol (2.0 g, 27.0 mmol) and 1,2-diiodoethane (8.4 g, 29.7 mmol) were dissolved in 20 mL of anhydrous tetrahydrofuran. The mixture was cooled to 0 °C under nitrogen, and LiHMDS (41 mL, 40.5 mmol) was slowly added dropwise. After the addition was completed, the mixture was stirred and reacted at room temperature under nitrogen overnight. The reaction was monitored by TLC until it was completed. Water was added to quench the reaction. The resulting mixture was extracted with dichloromethane 3 to 4 times. The organic phases were combined, washed with saturated brine, concentrated, and then purified by silica gel column chromatography to give 4.2 g of a target product, with a yield of 69%.

Step 2: Synthesis of compounds T-56-A and T-56-B

Compounds T-56-A and T-56-B were prepared using 7-bromo-2,4-dihydropyrazolo[3',4':3,4]cyclopenta[1,2-b]pyridine, 2-(2-iodoethoxy)-2-methylpropane and intermediate compound A-9 as the raw materials according to the method described in example 16, with overall yields of 14% and 11%, respectively, wherein the LC-MS (APCI) of T-56-A: m/z=663.7 $(M+1)^+$. $^1$H NMR (400 MHz, DMSO-$d_6$) δ 10.23 (s, 1H), 8.92 (s, 1H), 8.62 (s, 1H), 8.34 (d, J = 2.3 Hz, 1H), 8.21 (d, J = 2.2 Hz, 1H), 7.61 (d, J = 8.8 Hz, 2H), 7.31 (d, J = 7.6 Hz, 1H), 7.24 (d, J = 8.6 Hz, 2H), 5.06 (s, 2H), 4.64 (s, 2H), 4.33 (t, J = 4.4 Hz, 2H), 3.93 (m, 1H), 3.94-3.71 (m, 2H), 3.62 (J = 4.4 Hz, 2H), 1.65 (d, J = 8.6 Hz, 3H), 1.12 (s, 9H).

The LC-MS (APCI) of T-56-B: m/z=663.3 $(M+1)^+$. $^1$H NMR (400 MHz, DMSO-$d_6$) δ 10.23 (s, 1H), 8.88 (s, 1H), 8.63 (s, 1H), 8.34 (d, J = 2.3 Hz, 1H), 8.21 (d, J = 2.2 Hz, 1H), 7.61 (d, J = 8.8 Hz, 2H), 7.32 (d, J = 7.6 Hz, 1H), 7.24 (d, J = 8.6 Hz, 2H), 5.06 (s, 2H), 4.64 (s, 2H), 4.32 (t, J = 4.4 Hz, 2H), 3.90 (m, 1H), 3.94-3.71 (m, 2H), 3.62 (J = 4.4 Hz, 2H), 1.68 (d, J = 8.6 Hz, 3H), 1.13 (s, 9H).

**Example 57 Preparation of (R)-N-(4-(chlorodifluoromethoxy)phenyl)-6-(2-cyclopropyl-2,4-dihydropyrazolo [3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-57)**

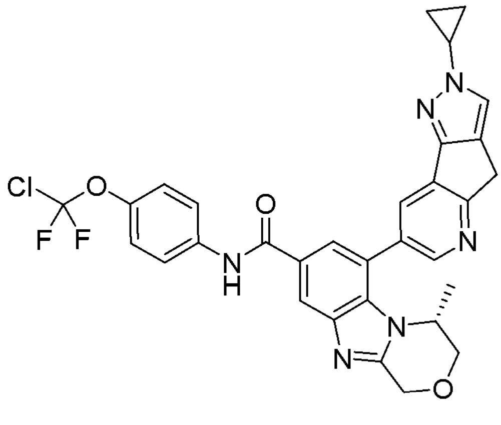

T-57

The following synthetic route was adopted:

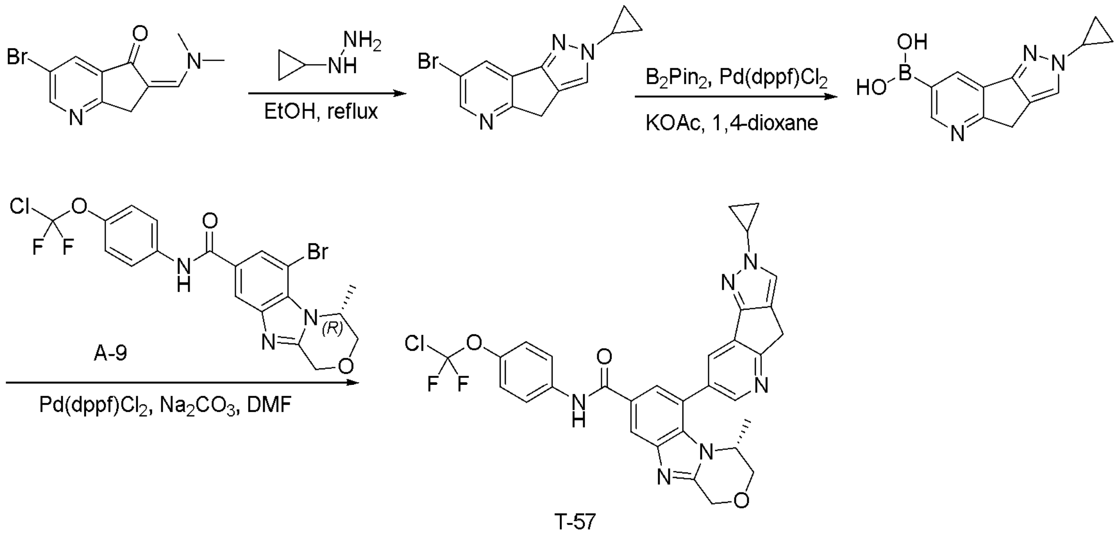


T-57

Step 1: Synthesis of compound 7-bromo-2-cyclopropyl-2,4-dihydropyrazolo[3',4':3,4]cyclopenta[1,2-b]pyridine

(Z)-3-Bromo-6-((dimethylamino)methylene)-6,7-dihydro-5H-cyclopenta[b]pyridin-5-one (1.0 g, 3.76 mmol) and cyclopropylhydrazine (406 mg, 5.64 mmol) were dissolved in ethanol (15 mL). The mixture was heated to reflux, stirred and reacted under nitrogen overnight. The reaction was monitored by TLC until it was completed. The resulting mixture was concentrated to remove the solvent. The residue was purified by silica gel column chromatography to give 558 mg of a light yellow solid, with a yield of 54%. LC-MS (APCI): m/z=276.4 $(M+1)^+$.

Step 2: Synthesis of compound T-57

Example 57 was prepared using 7-bromo-2-cyclopropyl-2,4-dihydropyrazolo[3',4':3,4]cyclopenta[1,2-b]pyridine and intermediate compound A-9 as the raw materials according to the method described in example 16, with an overall yield of 36%. LC-MS (APCI): m/z=603.2 $(M+1)^+$. $^1$H NMR (400 MHz, DMSO-$d_6$) δ 10.23 (s, 1H), 8.91 (s, 1H), 8.62 (s, 1H), 8.34 (d, J = 2.3 Hz, 1H), 8.23 (d, J = 2.3 Hz, 1H), 7.64 (d, J = 8.8 Hz, 2H), 7.31 (d, J = 7.6 Hz, 1H), 7.24 (d, J = 8.6 Hz, 2H), 5.06 (s, 2H), 4.14 (s, 2H), 4.05 (m, 1H), 3.93 (m, 1H), 3.94-3.71 (m, 2H), 2.39-2.16 (m, 4H), 1.64 (d, J = 8.6 Hz, 3H).

**Example 58 Preparation of (R)-N-(4-(chlorodifluoromethoxy)phenyl)-4-methyl-6-(5-oxo-6,7-dihydro-5H-cyclopenta[b]pyridin-3-yl)-3,4-dihydro-1H-benzo[4,5]imidazo[2,1-c][1,4]oxazine-8-carboxamide (compound T-58)**

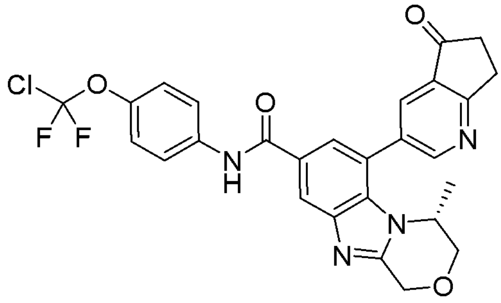

T-58

The following synthetic route was adopted:

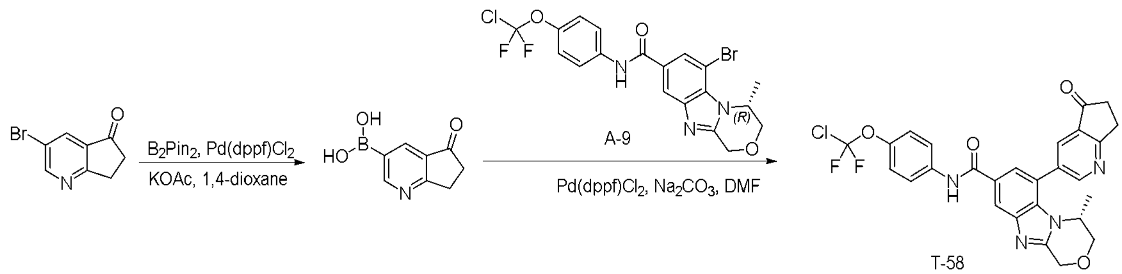


Example 58 was prepared using 3-bromo-6,7-dihydro-5H-cyclopenta[b]pyridin-5-one and intermediate compound A-9 as the raw materials according to the method described in example 16, with an overall yield of 37%. LC-MS (APCI): m/z=539.4 $(M+1)^+$. $^1H$ NMR (400 MHz, DMSO-$d_6$) δ 10.23 (s, 1H), 8.89 (s, 1H), 8.62 (s, 1H), 8.34 (d, J = 2.3 Hz, 1H), 8.23 (d, J = 2.3 Hz, 1H), 7.64 (d, J = 8.8 Hz, 2H), 7.31 (d, J = 7.6 Hz, 1H), 7.24 (d, J = 8.6 Hz, 2H), 5.03 (s, 2H), 3.92 (m, 1H), 3.94-3.71 (m, 2H), 2.65 (t, J = 3.6 Hz, 2H), 2.31 (t, J = 3.6 Hz, 2H), 1.62 (d, J = 8.6 Hz, 3H).

**Example 59 Preparation of (R,Z)-N-(4-(chlorodifluoromethoxy)phenyl)-6-(6-((dimethylamino)methylene)-5-oxo-6,7-dihydro-5H-cyclopenta[b]pyridin-3-yl)-4-methyl-3,4-dihydro-1H-benzo[4,5]imidazo[2,1-c][1,4]oxazine-8-carboxamide (compound T-59)**

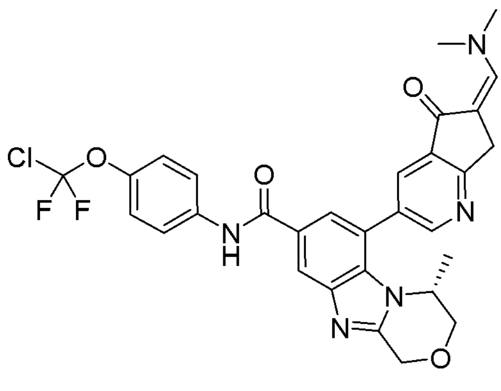

T-59

The following synthetic route was adopted:

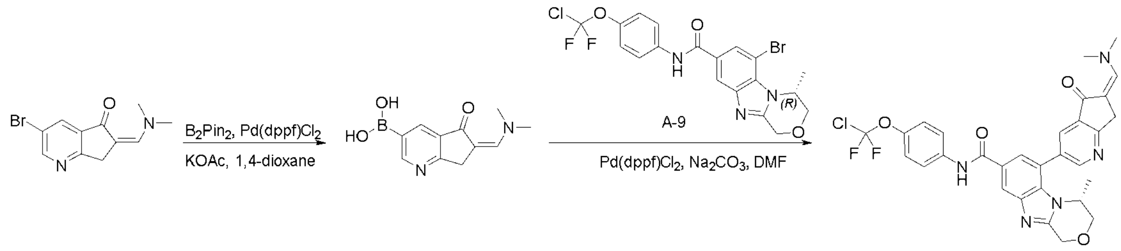


T-59

Example 59 was prepared using (Z)-3-bromo-6-((dimethylamino)methylene)-6,7-dihydro-5H-cyclopenta[b]pyridin-5-one and intermediate compound A-9 as the raw materials according to the method described in example 16, with an overall yield of 19%. LC-MS (APCI): m/z=594.8 $(M+1)^+$. $^1H$ NMR (400 MHz, DMSO-$d_6$) δ 10.23 (s, 1H), 8.92 (s, 1H), 8.62 (s, 1H), 8.34 (d, J = 2.3 Hz, 1H), 8.23 (d, J = 2.3 Hz, 1H), 7.64 (d, J = 8.8 Hz, 2H), 7.31 (d, J = 7.6 Hz, 1H), 7.24 (d, J = 8.6 Hz, 2H), 5.03 (s, 2H), 3.92 (m, 1H), 3.94-3.71 (m, 2H), 3.37 (s, 6H), 2.33 (s, 2H), 1.61 (d, J = 8.6 Hz, 3H).

**Example 60 Preparation of (R)-N-(4-(chlorodifluoromethoxy)phenyl)-4-methyl-6-(4-oxo-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-60)**

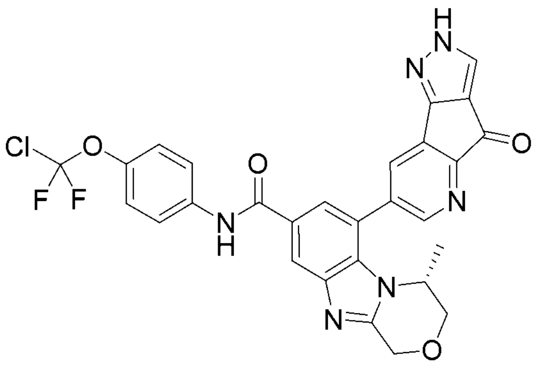

T-60

The following synthetic route was adopted:

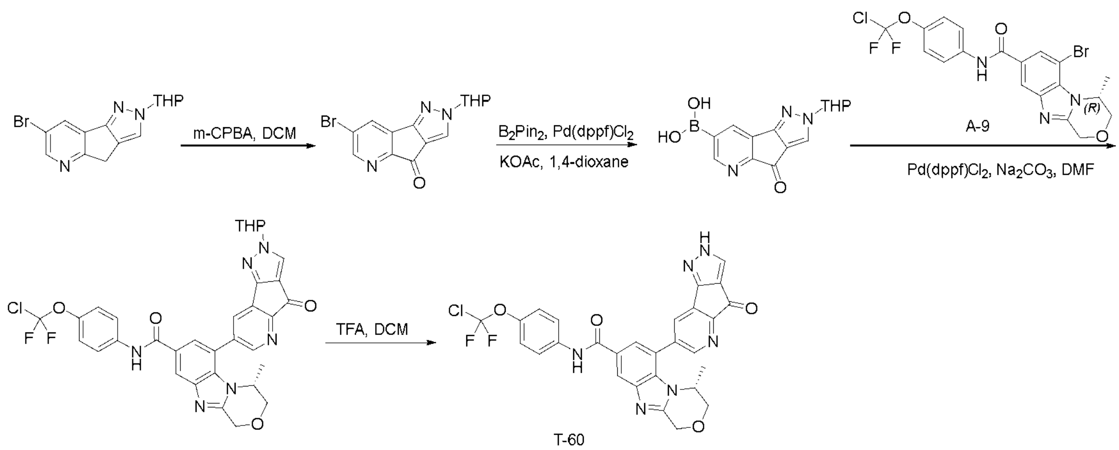


T-60

Step 1: Synthesis of compound 7-bromo-2-(tetrahydro-2H-pyran-2-yl)pyrazolo[3',4':3,4]cyclopenta[1,2-b]pyridin-4(2H)-one

7-Bromo-2-(tetrahydro-2H-pyran-2-yl)-2,4-dihydropyrazolo[3',4':3,4]cyclopenta[1,2-b]pyridine (1.0 g, 3.1 mmol) was dissolved in 20 mL of anhydrous dichloromethane. m-Chloroperoxybenzoic acid (1.1 g, 6.2 mmol) was added in batches under nitrogen. After the addition was completed, the mixture was stirred and reacted at room

temperature overnight. The reaction was monitored by TLC until it was completed. A saturated aqueous solution of sodium sulfite was added to quench the reaction. The resulting mixture was extracted with dichloromethane 2 to 3 times. The organic phases were combined, washed with saturated brine, concentrated, and then purified by silica gel column chromatography to give 671 mg of a light yellow solid, with a yield of 65%. LC-MS (APCI): m/z=334.1 $(M+1)^+$.

Step 2: Synthesis of compound (4-oxo-2-(tetrahydro-2H-pyran-2-yl)-2,4-dihydropyrazolo[3',4':3,4]cyclopenta[1,2-b] pyridin-7-yl)boronic acid

7-Bromo-2-(tetrahydro-2H-pyran-2-yl)pyrazolo[3',4':3,4]cyclopenta[1,2-b]pyridin-4(2H)-one (1.03 g, 3.1 mmol), bis(pinacolato)diboron (2.4 g, 9.3 mmol), [1,1'-bis(diphenylphosphino)ferrocene]dichloropalladium(II) (117 mg, 0.16 mmol) and potassium acetate (0.6 g, 6.2 mmol) were dissolved in 10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 389 mg of product, with a yield of 42%. LC-MS (APCI): m/z=300.7 $(M+1)^+$.

Step 3: Synthesis of compound (4R)-N-(4-(chlorodifluoromethoxy)phenyl)-4-methyl-6-(4-oxo-2-(tetrahydro-2H-pyran-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide

Intermediate compound A-9 (189 mg, 0.39 mmol), (4-oxo-2-(tetrahydro-2H-pyran-2-yl)-2,4-dihydropyrazolo [3',4':3,4]cyclopenta[1,2-b]pyridin-7-yl)boronic acid (176 mg, 0.588 mmol), [1,1'-bis(diphenylphosphino)ferrocene]dichloropalladium(II) (32 mg, 0.02 mmol), sodium carbonate (125 mg, 1.18 mmol) and anhydrous DMF (5 mL) were added to a reaction flask. The mixture was purged with nitrogen for 5 minutes. The mixture was microwaved and heated to 90 °C, and reacted for 1 hour. The reaction was monitored by TLC until it was completed. Then, the resulting mixture was concentrated, and purified by silica gel column chromatography to give 93 mg of product, with a yield of 36%. LC-MS (APCI): m/z=662.4 $(M+1)^+$.

Step 4: Synthesis of compound T-60

(4R)-N-(4-(Chlorodifluoromethoxy)phenyl)-4-methyl-6-(4-oxo-2-(tetrahydro-2H-pyran-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (93 mg, 0.14 mmol) was dissolved in 5 mL of anhydrous dichloromethane, and 0.5 mL of trifluoroacetic acid was added. The mixture was stirred and reacted at room temperature for 2 to 3 hours. The reaction was monitored by TLC until it was completed. The mixture was diluted with added dichloromethane. The resulting mixture was washed with saturated aqueous solution of sodium bicarbonate 2 to 3 times. The organic phase was concentrated, and then purified by silica gel column chromatography to give 41 mg of a light yellow solid, with a yield of 51%. LC-MS (APCI): m/z=577.3 $(M+1)^+$. $^1H$ NMR (400 MHz, DMSO-$d_6$) $\delta$ 10.23 (s, 1H), 8.93 (s, 1H), 8.62 (s, 1H), 8.34 (d, J = 2.3 Hz, 1H), 8.23 (d, J = 2.3 Hz, 1H), 7.64 (d, J = 8.8 Hz, 2H), 7.31 (d, J = 7.6 Hz, 1H), 7.24 (d, J = 8.6 Hz, 2H), 5.03 (s, 2H), 3.92 (m, 1H), 3.94-3.71 (m, 2H), 1.63 (d, J = 8.6 Hz, 3H).

**Example 61 Preparation of (R)-N-(4-(chlorodifluoromethoxy)phenyl)-4-methyl-6-(2-methyl-3-oxoisoindolin-5-yl)-3,4-dihydro-1H-benzo[4,5]imidazo[2,1-c][1,4]oxazine-8-carboxamide (compound T-61)**

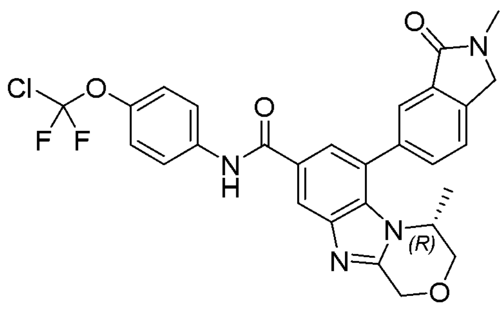

T-61

The following synthetic route was adopted:

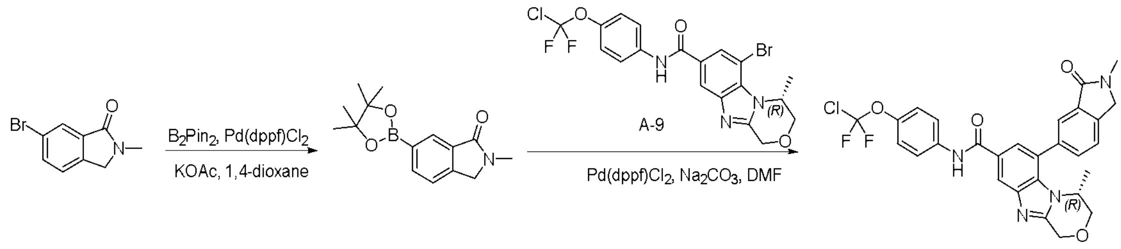


T-61

Compound T-61 was prepared using N-methyl-6-bromoisoindolin-1-one and intermediate compound A-9 as the raw materials via a two-step reaction according to the method described in example 16, with an overall yield of 42%. LC-MS (APCI): m/z=553.2 $(M+1)^+$. $^1H$ NMR (400 MHz, DMSO-$d_6$) δ 10.23 (s, 1H), 8.89 (s, 1H), 8.62 (s, 1H), 8.34 (d, J = 2.3 Hz, 1H), 8.23 (d, J = 2.3 Hz, 1H), 7.64 (d, J = 8.8 Hz, 2H), 7.31 (d, J = 7.6 Hz, 1H), 7.24 (d, J = 8.6 Hz, 2H), 5.01 (s, 2H), 3.91 (m, 1H), 3.94-3.71 (m, 2H), 3.67 (s, 2H), 3.26 (s, 3H), 1.64 (d, J = 8.6 Hz, 3H).

**Example 62 Preparation of (R)-N-(4-(chlorodifluoromethoxy)phenyl)-4-methyl-6-(2-methyl-1,3-dioxoisoindolin-5-yl)-3,4-dihydro-1H-benzo[4,5]imidazo[2,1-c][1,4]oxazine-8-carboxamide (compound T-62)**

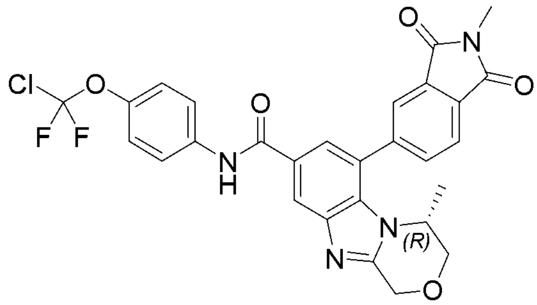

T-62

The following synthetic route was adopted:

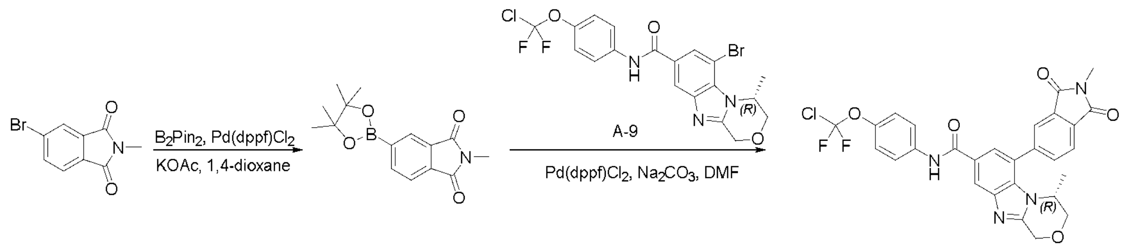


T-62

Compound T-62 was prepared using 4-bromo-N-methylphthalimide and intermediate compound A-9 as the raw materials via a two-step reaction according to the method described in example 16, with an overall yield of 27%. LC-MS (APCI): m/z=567.5 $(M+1)^+$. $^1H$ NMR (400 MHz, DMSO-$d_6$) δ 10.23 (s, 1H), 8.92 (s, 1H), 8.61 (s, 1H), 8.34 (d, J = 2.3 Hz, 1H), 8.23 (d, J = 2.3 Hz, 1H), 7.64 (d, J = 8.8 Hz, 2H), 7.31 (d, J = 7.6 Hz, 1H), 7.24 (d, J = 8.6 Hz, 2H), 5.03 (s, 2H), 4.09 (s, 3H), 3.92 (m, 1H), 3.94-3.71 (m, 2H), 1.63 (d, J = 8.6 Hz, 3H).

Example 63 **Preparation** of **(R)-N-(4-(chlorodifluoromethoxy)phenyl)-4-methyl-6-(2-(pyridin-3-yl)-2,4-dihydropyrazolo[3',4':3,4]cyclopenta[1,2-b]pyridin-7-yl)-3,4-dihydro-1H-benzo[4,5]imidazo[2,1-**cl **[1,4]oxazine-8-carboxamide (compound T-63)**

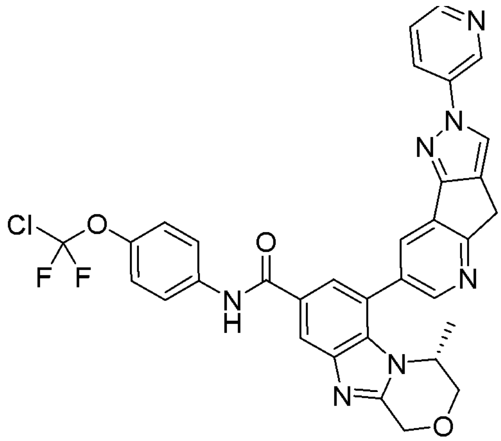

T-63

The following synthetic route was adopted:

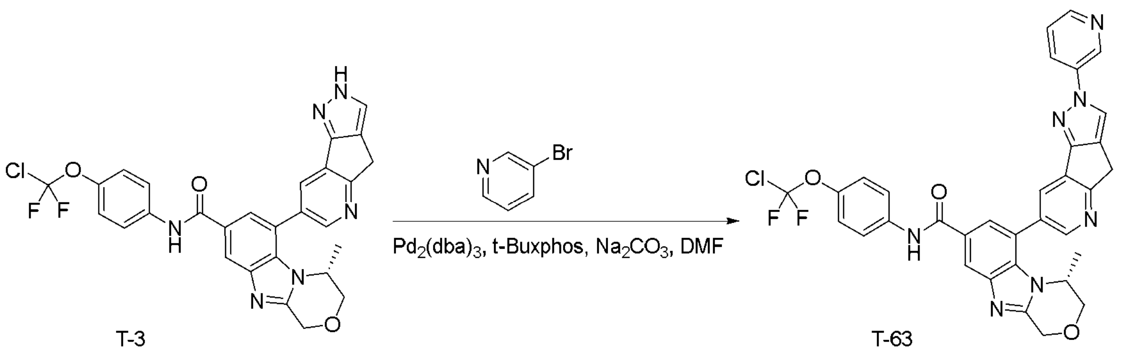


Compound T-63 was prepared using compound T-3 and 3-bromopyridine as the raw materials according to the method described in example 4, with a yield of 34%. LC-MS (APCI): m/z=640.5 $(M+1)^+$. $^1H$ NMR (400 MHz, DMSO-$d_6$) δ 10.23 (s, 1H), 8.93 (s, 1H), 8.61 (s, 1H), 8.45 (d, J = 2.6 Hz, 1H), 8.34 (d, J = 2.3 Hz, 1H), 8.23 (d, J = 2.3 Hz, 1H), 7.65 (d, J = 8.8 Hz, 2H), 7.57-7.39 (m, 3H), 7.34 (d, J = 7.6 Hz, 1H), 7.21 (d, J = 8.6 Hz, 2H), 5.03 (s, 2H), 4.26 (s, 2H), 3.92 (m, 1H), 3.94-3.71 (m, 2H), 1.64 (d, J = 8.6 Hz, 3H).

**Example 64 Preparation of (R)-N-(4-(chlorodifluoromethoxy)phenyl)-4-methyl-6-(2-(pyridin-4-yl)-2,4-dihydropyrazolo[3',4':3,4]cyclopenta[1,2-b]pyridin-7-yl)-3,4-dihydro-1H-benzo[4,5]imidazo[2,1-cl [1,4]oxazine-8-carboxamide (compound T-64)**

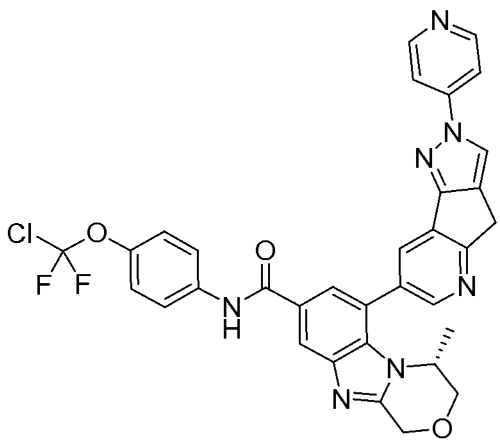

T-64

The following synthetic route was adopted:

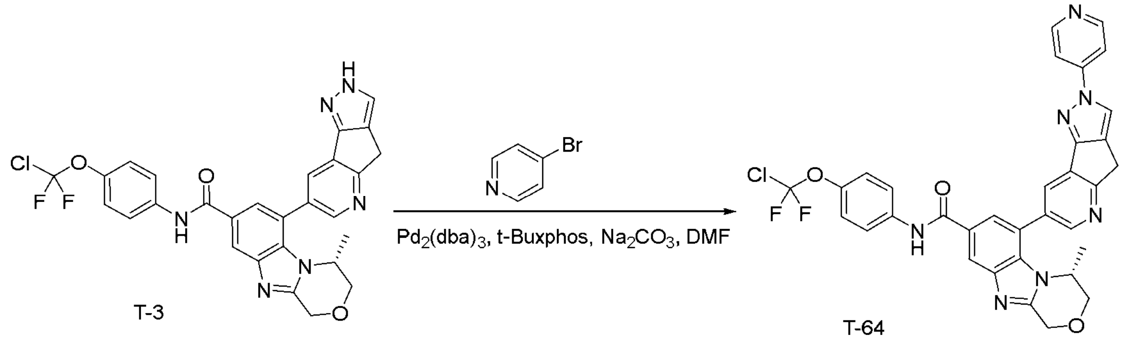

T-3
T-64

Compound T-64 was prepared using compound T-3 and 4-bromopyridine as the raw materials according to the method described in example 4, with a yield of 47%. LC-MS (APCI): m/z=640.5 $(M+1)^+$. $^1$H NMR (400 MHz, DMSO-$d_6$) δ 10.22 (s, 1H), 8.91 (s, 1H), 8.61 (s, 1H), 8.45 (d, J = 2.6 Hz, 1H), 8.34 (d, J = 2.3 Hz, 1H), 8.23 (d, J = 2.3 Hz, 1H), 7.65 (d, J = 8.8 Hz, 2H), 7.57 (d, J = 8.4 Hz, 2H), 7.34 (d, J = 8.6 Hz, 2H), 7.21 (d, J = 8.6 Hz, 2H), 5.03 (s, 2H), 4.26 (s, 2H), 3.92 (m, 1H), 3.94-3.71 (m, 2H), 1.64 (d, J = 8.6 Hz, 3H).

**Example 65 Preparation of (R)-N-(4-(chlorodifluoromethoxy)phenyl)-4-methyl-6-(2-(pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][[1,4]oxazine-8-carboxamide (compound T-65)**

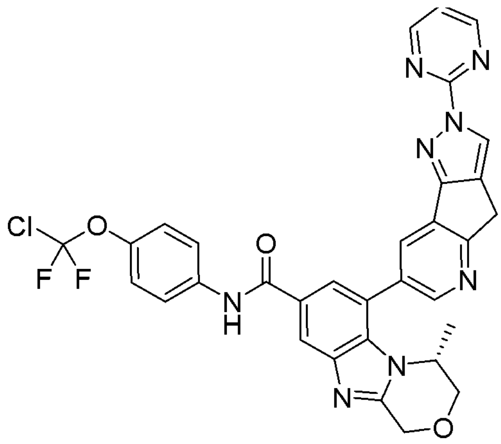
T-65

The following synthetic route was adopted:

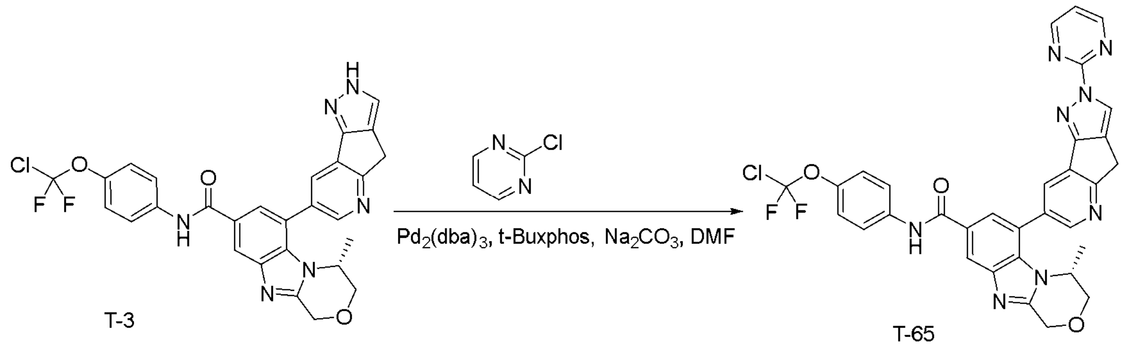

T-3
T-65

Example 65 was prepared using compound T-3 and 2-chloropyrimidine as the raw materials according to the method described in example 4, with a yield of 52%. LC-MS (APCI): m/z=641.2 $(M+1)^+$. $^1$H NMR (400 MHz, DMSO-$d_6$) δ 10.23 (s, 1H), 8.93 (s, 1H), 8.61 (s, 1H), 8.46 (d, J = 2.6 Hz, 1H), 8.35 (d, J = 2.3 Hz, 1H), 8.23 (d, J = 2.3 Hz, 1H), 7.65 (d, J = 8.8 Hz, 1H), 7.57 (d, J = 8.4 Hz, 2H), 7.34 (d, J = 8.6 Hz, 2H), 7.22 (d, J = 8.6 Hz, 2H), 5.03 (s, 2H), 4.26 (s, 2H), 3.92 (m, 1H), 3.94-3.71 (m, 2H), 1.63 (d, J = 8.6 Hz, 3H).

**Example 66 Preparation of (R)-N-(4-(chlorodifluoromethoxy)phenyl)-4-methyl-6-(2-(pyrimidin-5-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-66)**

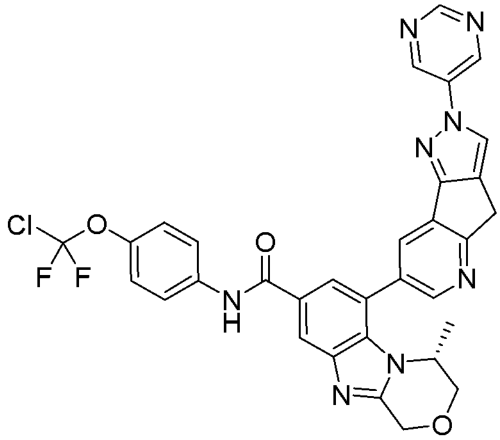

T-66

The following synthetic route was adopted:

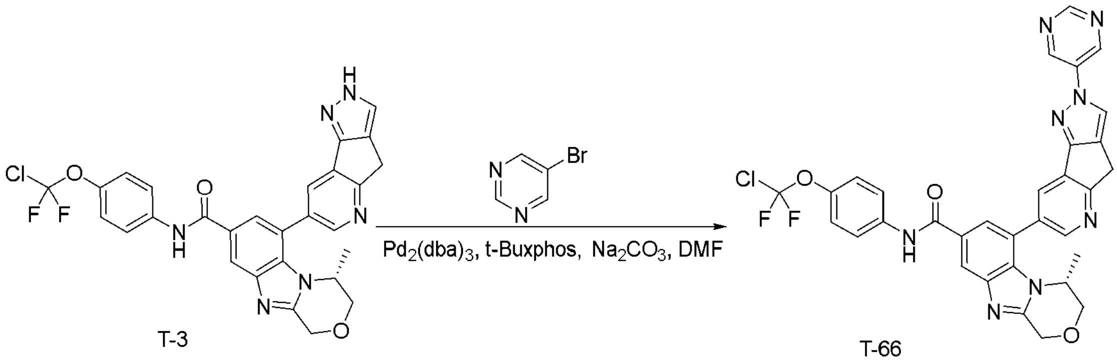


Example 66 was prepared using compound T-3 and 5-brompyrimidine as the raw materials according to the method described in example 4, with a yield of 36%. LC-MS (APCI): m/z=641.3 $(M+1)^+$. $^1H$ NMR (400 MHz, DMSO-$d_6$) δ 10.23 (s, 1H), 8.90 (s, 1H), 8.61 (s, 1H), 8.45 (d, J = 2.6 Hz, 1H), 8.34 (d, J = 2.3 Hz, 1H), 8.21 (d, J = 2.3 Hz, 1H), 7.65 (d, J = 8.8 Hz, 1H), 7.57 (d, J = 8.4 Hz, 2H), 7.34 (d, J = 8.6 Hz, 2H), 7.23 (d, J = 8.6 Hz, 2H), 5.03 (s, 2H), 4.26 (s, 2H), 3.92 (m, 1H), 3.95-3.70 (m, 2H), 1.62 (d, J = 8.6 Hz, 3H).

**Example 67 Preparation of (R)-N-(4-(chlorodifluoromethoxy)phenyl)-4-methyl-6-(2-(pyrimidin-4-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-67)**

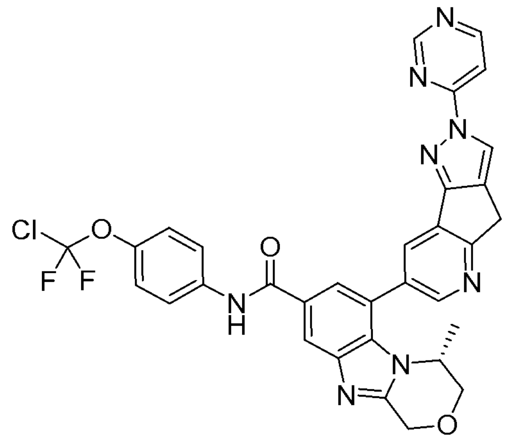

T-67

The following synthetic route was adopted:

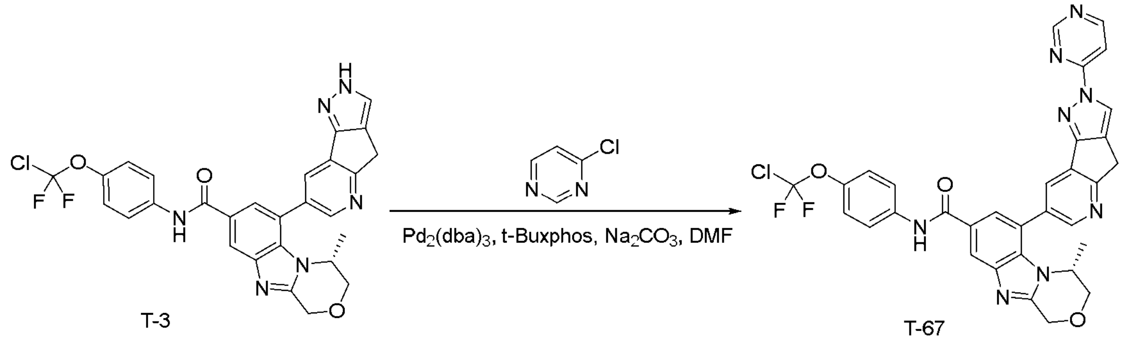


Example 67 was prepared using compound T-3 and 4-chloropyrimidine as the raw materials according to the method described in example 4, with a yield of 48%. LC-MS (APCI): m/z=641.8 (M+1)$^+$. $^1$H NMR (400 MHz, DMSO-$d_6$) δ 10.22 (s, 1H), 8.93 (s, 1H), 8.61 (s, 1H), 8.45 (d, J = 2.6 Hz, 1H), 8.34 (d, J = 2.3 Hz, 1H), 8.23 (d, J = 2.3 Hz, 1H), 7.65 (d, J = 8.8 Hz, 1H), 7.57 (d, J = 8.4 Hz, 2H), 7.34 (d, J = 8.6 Hz, 2H), 7.21 (d, J = 8.6 Hz, 2H), 5.03 (s, 2H), 4.26 (s, 2H), 3.90 (m, 1H), 3.94-3.71 (m, 2H), 1.65 (d, J = 8.6 Hz, 3H).

## Example 68 Preparation of (R)-N-(4-(chlorodifluoromethoxy)phenyl)-6-(2-(5-fluoropyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-68)

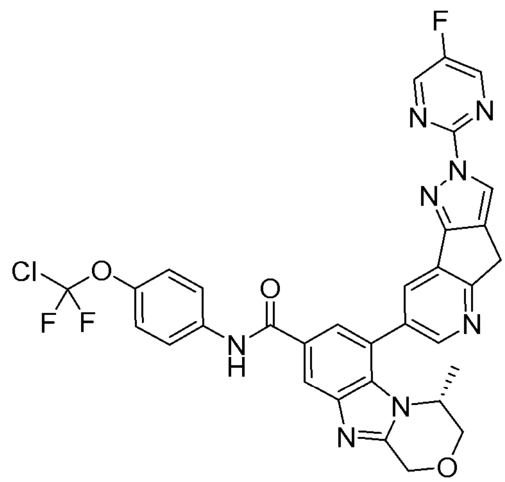

T-68

The following synthetic route was adopted:

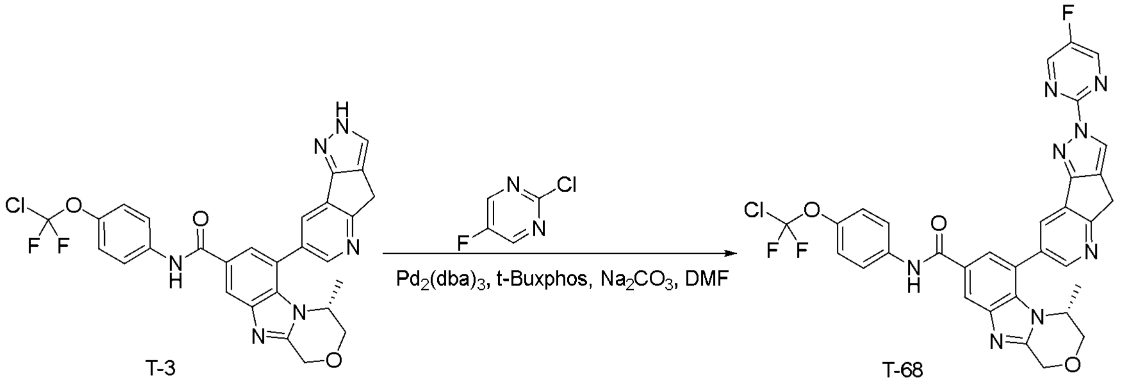

T-3 T-68

Example 68 was prepared using compound T-3 and 2-chloro-5-fluoropyrimidine as the raw materials according to the method described in example 4, with a yield of 53%. LC-MS (APCI): m/z=659.5 $(M+1)^+$. $^1H$ NMR (400 MHz, DMSO-$d_6$) δ 10.22 (s, 1H), 8.91 (s, 1H), 8.61 (s, 1H), 8.45 (s, 1H), 8.34 (d, J = 2.3 Hz, 1H), 8.23 (d, J = 2.3 Hz, 1H), 7.62 (d, J = 8.8 Hz, 1H), 7.57 (d, J = 8.4 Hz, 2H), 7.36 (d, J = 8.6 Hz, 2H), 7.21 (d, J = 8.6 Hz, 2H), 5.03 (s, 2H), 4.26 (s, 2H), 3.92 (m, 1H), 3.94-3.71 (m, 2H), 1.62 (d, J = 8.6 Hz, 3H).

**Example 69 Preparation of (R)-N-(4-(chlorodifluoromethoxy)phenyl)-6-(1-(4-cyanopyrimidin-2-yl)-1,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-69-A) and (R)-N-(4-(chlorodifluoromethoxy)phenyl)-6-(2-(4-cyanopyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-69-B)**

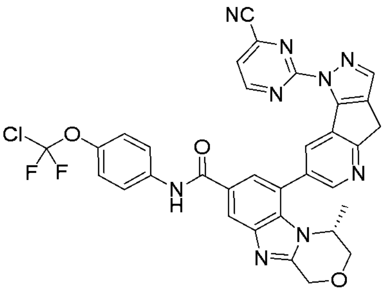
T-69-A and
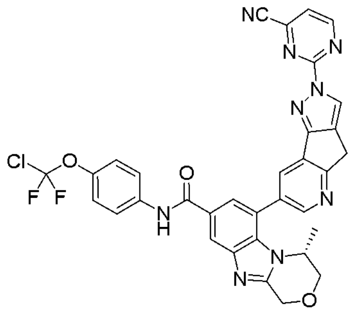
T-69-B

The following synthetic route was adopted:

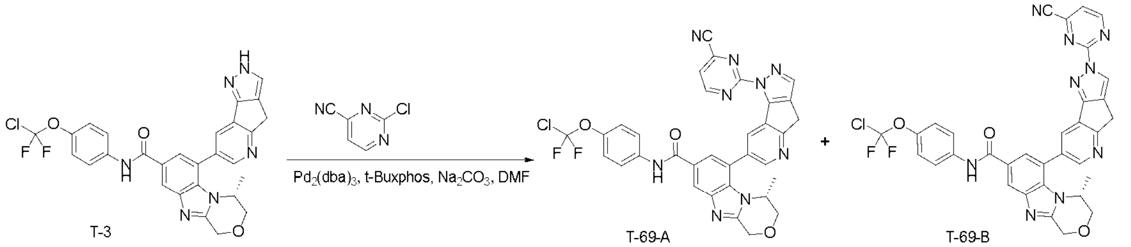

T-3 T-69-A + T-69-B

Compounds T-69-A and T-69-B were prepared using compound T-3 and 2-chloro-4-cyanopyrimidine as the raw materials according to the method described in example 4, with yields of 16% and 28%, respectively. Compound T-69-A: LC-MS (APCI): m/z=666.3 $(M+1)^+$. $^1H$ NMR (400 MHz, DMSO-$d_6$) δ 10.22 (s, 1H), 8.91 (s, 1H), 8.61 (s, 1H), 8.45 (d, J = 2.6 Hz, 1H), 8.34 (d, J = 2.3 Hz, 1H), 8.23 (d, J = 2.3 Hz, 1H), 7.65 (d, J = 8.8 Hz, 1H), 7.57 (d, J = 8.4 Hz, 2H), 7.34 (d, J = 8.6 Hz, 2H), 7.21 (d, J = 8.6 Hz, 2H), 5.03 (s, 2H), 4.26 (s, 2H), 3.92 (m, 1H), 3.94-3.71 (m, 2H), 1.64 (d, J = 8.6 Hz, 3H).

Compound T-69-B: LC-MS (APCI): m/z=666.4 $(M+1)^+$. $^1H$ NMR (400 MHz, DMSO-$d_6$) δ 10.23 (s, 1H), 8.92 (s, 1H), 8.60 (s, 1H), 8.45 (d, J = 2.6 Hz, 1H), 8.31 (d, J = 2.3 Hz, 1H), 8.23 (d, J = 2.3 Hz, 1H), 7.63 (d, J = 8.8 Hz, 1H), 7.57 (d, J = 8.4 Hz, 2H), 7.34 (d, J = 8.6 Hz, 2H), 7.21 (d, J = 8.6 Hz, 2H), 5.03 (s, 2H), 4.26 (s, 2H), 3.92 (m, 1H), 3.94-3.74 (m, 2H), 1.63 (d, J = 8.6 Hz, 3H).

**Example 70 Preparation of (R)-N-(4-(chlorodifluoromethoxy)phenyl)-6-(2-(4,6-dimethoxy-1,3,5-triazin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-70)**

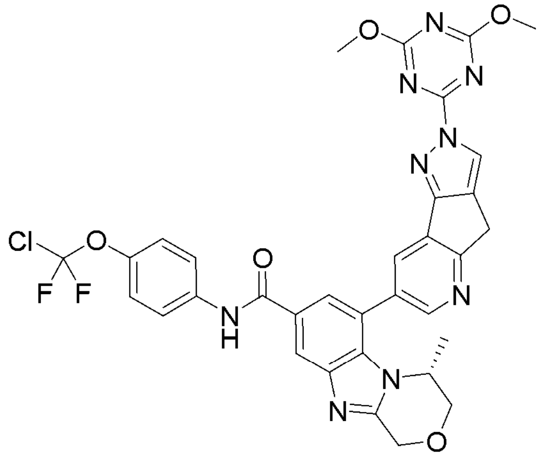

T-70

The following synthetic route was adopted:

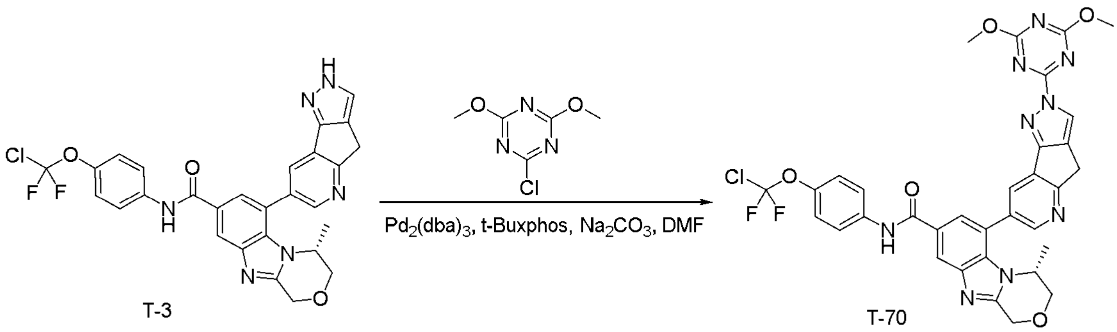


T-3

T-70

Example 70 was prepared using compound T-3 and 2-chloro-4,6-dimethoxy-1,3,5-triazine as the raw materials according to the method described in example 4, with a yield of 10%. LC-MS (APCI): m/z=702.6(M+1)$^+$. $^1$H NMR (400 MHz, DMSO-$d_6$) δ 10.23 (s, 1H), 8.93 (s, 1H), 8.61 (s, 1H), 8.23 (d, J = 2.3 Hz, 1H), 7.65 (d, J = 8.8 Hz, 2H), 7.57 (d, J = 8.4 Hz, 2H), 7.34 (d, J = 8.6 Hz, 2H), 7.21 (d, J = 8.6 Hz, 2H), 5.03 (s, 2H), 4.26 (s, 2H), 4.20 (s, 6H), 3.90 (m, 1H), 3.94-3.71 (m, 2H), 1.61 (d, J = 8.6 Hz, 3H).

**Example 71 Preparation of (R)-N-(4-(chlorodifluoromethoxy)phenyl)-4-methyl-6-(2-(pyrazin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-71)**

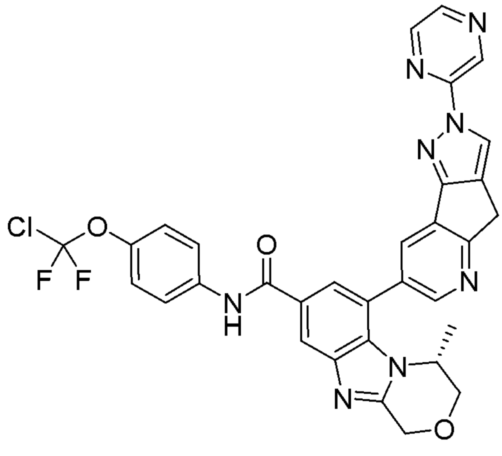

T-71

The following synthetic route was adopted:

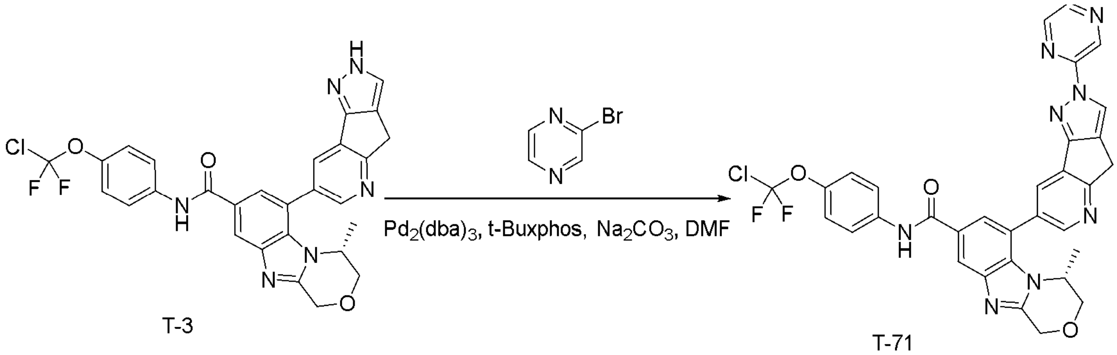


T-3

T-71

Example 71 was prepared using compound T-3 and 2-bromopyrazine as the raw materials according to the method described in example 4, with a yield of 18%. LC-MS (APCI): m/z=641.5(M+1)+. $^1$H NMR (400 MHz, DMSO-$d_6$) δ 10.22 (s, 1H), 8.91 (s, 1H), 8.62 (s, 1H), 8.45 (d, J = 2.6 Hz, 1H), 8.34 (d, J = 2.3 Hz, 1H), 8.23 (d, J = 2.3 Hz, 1H), 7.65 (d, J = 8.8 Hz, 1H), 7.57 (d, J = 8.4 Hz, 2H), 7.34 (d, J = 8.6 Hz, 2H), 7.21 (d, J = 8.6 Hz, 2H), 5.03 (s, 2H), 4.26 (s, 2H), 3.92 (m, 1H), 3.94-3.71 (m, 2H), 1.64 (d, J = 8.6 Hz, 3H).

**Example 72 Preparation of (R)-N-(4-(chlorodifluoromethoxy)phenyl)-4-methyl-6-(2-(4-(trifluoromethyl)pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-72)**

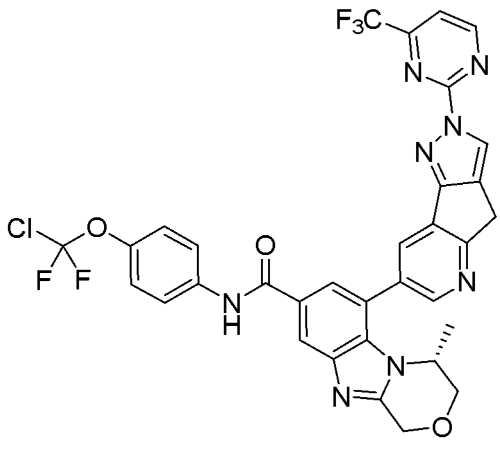

T-72

The following synthetic route was adopted:

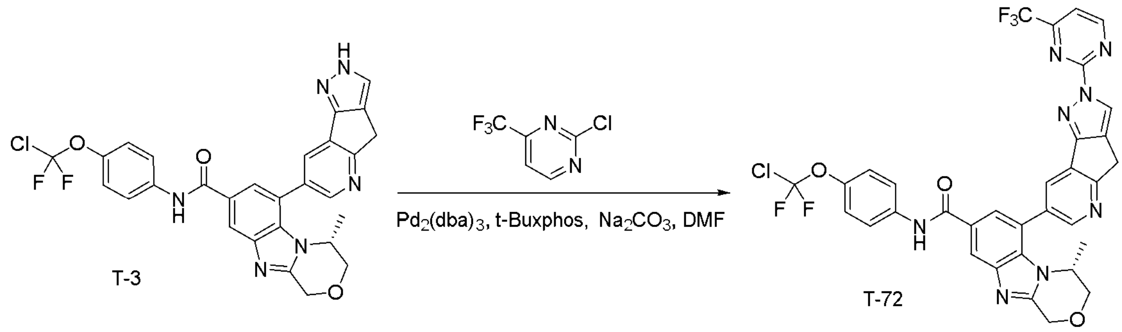


Example 72 was prepared using compound T-3 and 2-chloro-4-trifluoromethylpyrimidine as the raw materials according to the method described in example 4, with a yield of 38%. LC-MS (APCI): m/z=709.1(M+1)$^+$. $^1$H NMR (400 MHz, DMSO-$d_6$) δ 10.23 (s, 1H), 8.92 (s, 1H), 8.62 (s, 1H), 8.45 (d, J = 2.6 Hz, 1H), 8.34 (d, J = 2.3 Hz, 1H), 8.23 (d, J = 2.3 Hz, 1H), 7.65 (d, J = 8.8 Hz, 1H), 7.57 (d, J = 8.6 Hz, 1H), 7.34 (d, J = 8.6 Hz, 2H), 7.21 (d, J = 8.6 Hz, 2H), 5.03 (s, 2H), 4.26 (s, 2H), 3.93 (m, 1H), 3.96-3.71 (m, 2H), 1.62 (d, J = 8.6 Hz, 3H).

**Example 73 Preparation of (R)-N-(4-(chlorodifluoromethoxy)phenyl)-4-methyl-6-(2-(4-methylpyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-73)**

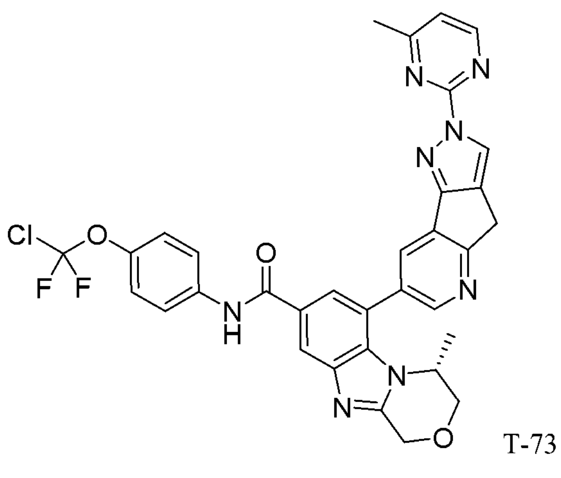
T-73

The following synthetic route was adopted:

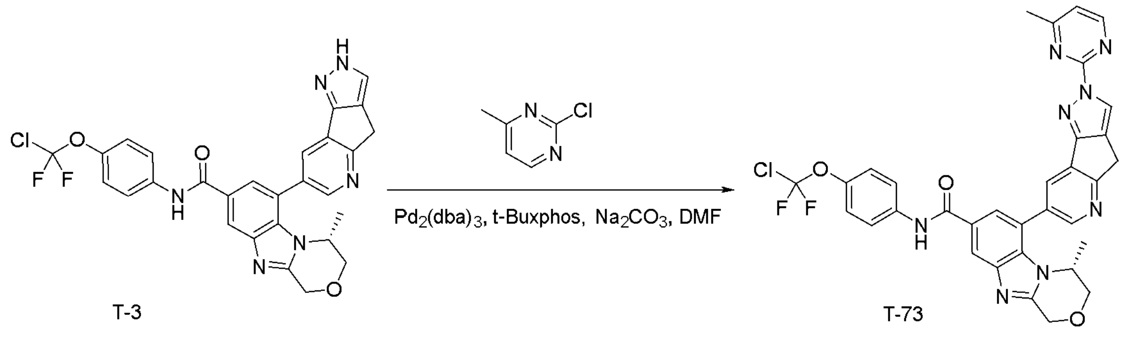


Example 73 was prepared using compound T-3 and 2-chloro-4-methylpyrimidine as the raw materials according to the method described in example 4, with a yield of 58%. LC-MS (APCI): m/z=655.6(M+1)$^+$. $^1$H NMR (400 MHz, DMSO-$d_6$) δ 10.23 (s, 1H), 8.92 (s, 1H), 8.62 (s, 1H), 8.41 (d, J = 2.6 Hz, 1H), 8.36(d, J = 2.3 Hz, 1H), 8.23 (d, J = 2.3 Hz, 1H), 7.65 (d, J = 8.8 Hz, 1H), 7.57 (d, J = 8.4 Hz, 2H), 7.34 (d, J = 8.6 Hz, 2H), 7.21 (d, J = 8.6 Hz, 2H), 5.03 (s, 2H), 4.26 (s, 2H), 3.91 (m, 1H), 3.94-3.71 (m, 2H), 2.35 (s, 3H), 1.64 (d, J = 8.6 Hz, 3H).

**Example 74 Preparation of (R)-N-(4-(chlorodifluoromethoxy)phenyl)-4-methyl-6-(2-(5-methylpyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-74)**

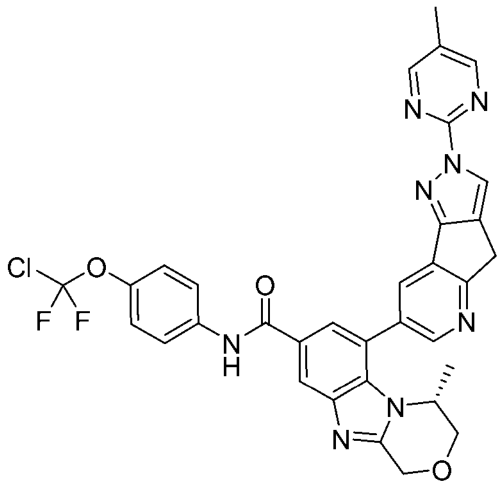

T-74

The following synthetic route was adopted:

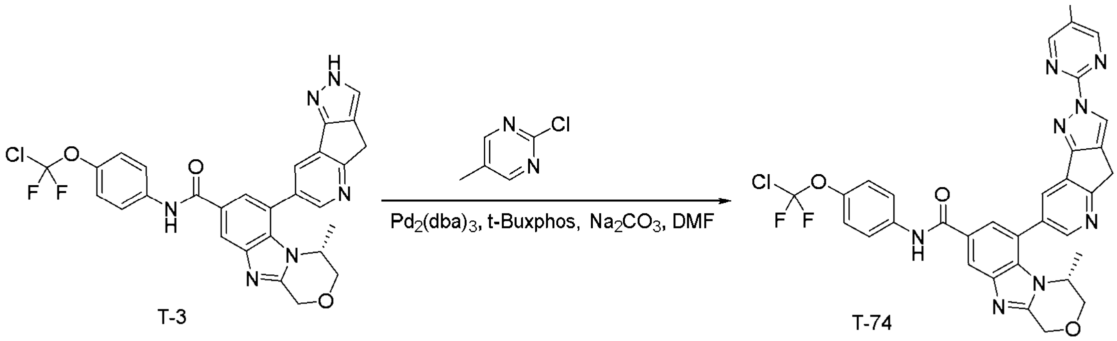


Example 74 was prepared using compound T-3 and 2-chloro-5-methylpyrimidine as the raw materials according to the method described in example 4, with a yield of 28%. LC-MS (APCI): m/z=655.7(M+1)$^+$. $^1$H NMR (400 MHz, DMSO-$d_6$) δ 10.24 (s, 1H), 8.90 (s, 1H), 8.62 (s, 1H), 8.45 (d, J = 2.6 Hz, 1H), 8.34 (d, J = 2.3 Hz, 1H), 8.23 (d, J = 2.3 Hz, 1H), 7.65 (d, J = 8.8 Hz, 1H), 7.57 (d, J = 8.4 Hz, 1H), 7.34 (d, J = 8.6 Hz, 2H), 7.21 (d, J = 8.6 Hz, 2H), 5.03 (s, 2H), 4.22 (s, 2H), 3.91 (m, 1H), 3.94-3.71 (m, 2H), 2.07 (s, 3H), 1.63 (d, J = 8.6 Hz, 3H).

**Example 75 Preparation of (R)-N-(4-(chlorodifluoromethoxy)phenyl)-4-methyl-6-(2-(4-methyl-6-(trifluoromethyl)pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-75).**

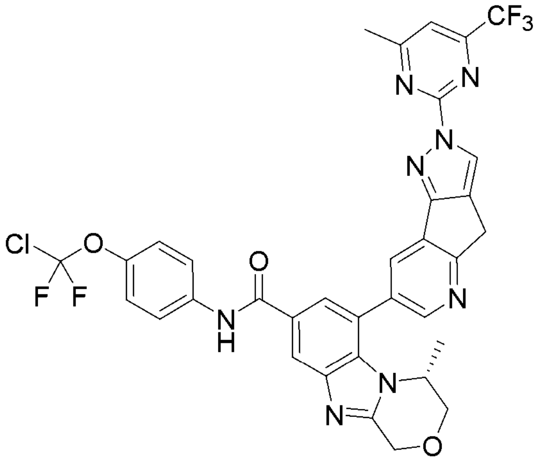

T-75

The following synthetic route was adopted:

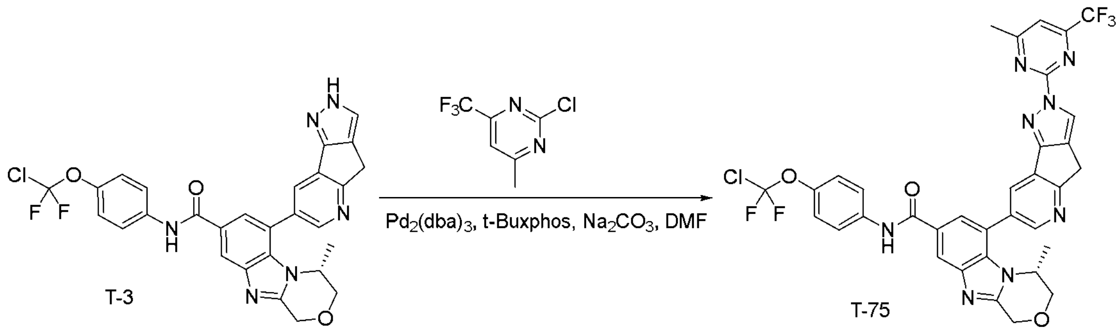


Example 75 was prepared using compound T-3 and 2-chloro-4-methyl-6-(trifluoromethyl)pyrimidine as the raw materials according to the method described in example 4, with a yield of 24%. LC-MS (APCI): m/z=723.6(M+1)$^+$. $^1$H NMR (400 MHz, DMSO-$d_6$) δ 10.22 (s, 1H), 8.93 (s, 1H), 8.62 (s, 1H), 8.45 (d, J = 2.6 Hz, 1H), 8.34 (s, 1H), 7.65 (d, J = 8.8 Hz, 1H), 7.57 (d, J = 8.4 Hz, 2H), 7.34 (d, J = 8.6 Hz, 2H), 7.21 (d, J = 8.6 Hz, 2H), 5.03 (s, 2H), 4.26 (s, 2H), 3.92 (m, 1H), 3.94-3.70 (m, 2H), 2.31 (s, 3H), 1.66 (d, J = 8.6 Hz, 3H).

**Example 76 Preparation of (R)-N-(4-(chlorodifluoromethoxy)phenyl)-6-(2-(4,6-dimethylpyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-76)**

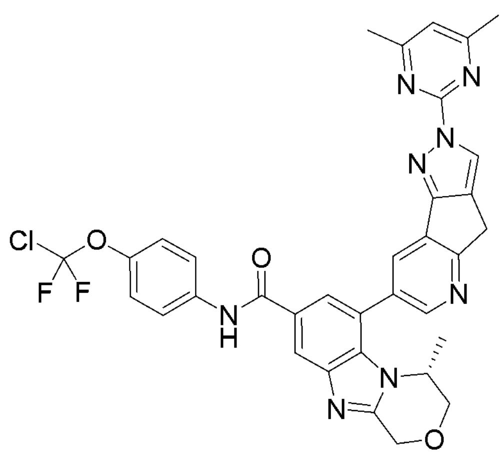

T-76

The following synthetic route was adopted:

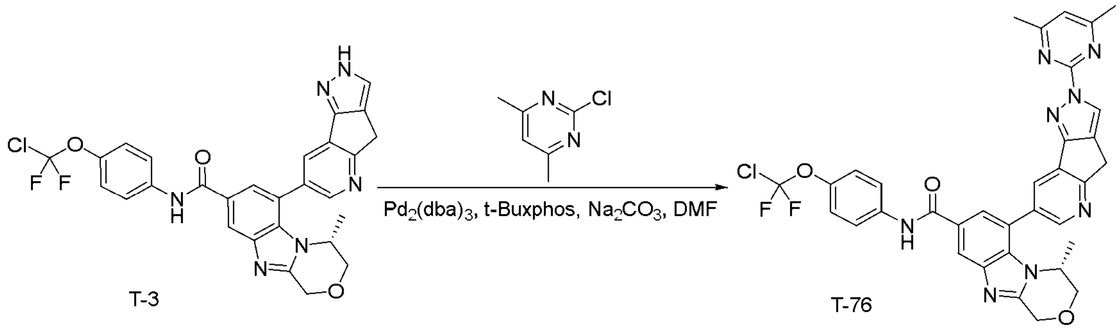


T-3 T-76

Example 76 was prepared using compound T-3 and 2-chloro-4,6-dimethylpyrimidine as the raw materials according to the method described in example 4, with a yield of 35%. LC-MS (APCI): m/z=669.2(M+1)+. $^1$H NMR (400 MHz, DMSO-$d_6$) δ 10.23 (s, 1H), 8.90 (s, 1H), 8.62 (s, 1H), 8.23 (d, J = 2.3 Hz, 1H), 7.65 (d, J = 8.8 Hz, 1H), 7.48 (s, 1H), 7.34 (d, J = 8.6 Hz, 2H), 7.21 (d, J = 8.6 Hz, 2H), 5.03 (s, 2H), 4.26 (s, 2H), 3.92 (m, 1H), 3.94-3.71 (m, 2H), 2.88 (s, 6H), 1.63 (d, J = 8.6 Hz, 3H).

Example 77 Preparation of (R)-N-(4-(chlorodifluoromethoxy)phenyl)-6-(2-(5-methoxypyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-77)

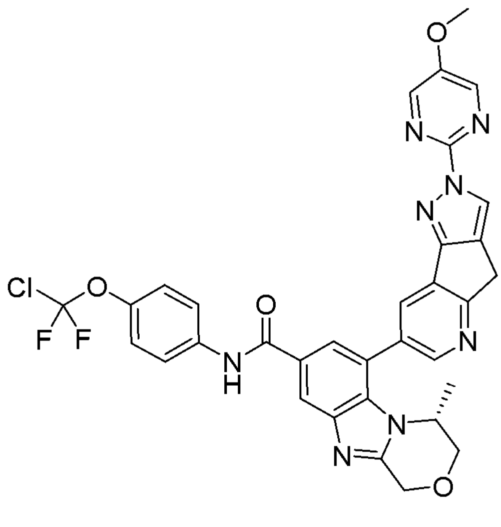

T-77

The following synthetic route was adopted:

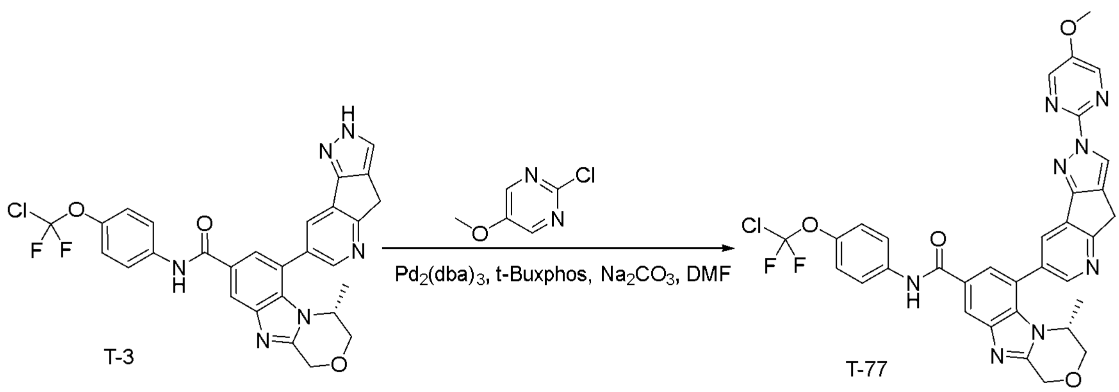


T-3 T-77

Example 77 was prepared using compound T-3 and 2-chloro-5-methoxypyrimidine as the raw materials according to the method described in example 4, with a yield of 64%. LC-MS (APCI): m/z=671.2(M+1)+. $^1$H NMR

(400 MHz, DMSO-$d_6$) δ 10.23 (s, 1H), 8.93 (s, 1H), 8.62 (s, 2H), 8.23 (d, J = 2.3 Hz, 1H), 7.65 (d, J = 8.8 Hz, 1H), 7.57 (d, J = 8.4 Hz, 2H), 7.34 (d, J = 8.6 Hz, 2H), 7.21 (d, J = 8.6 Hz, 2H), 5.03 (s, 2H), 4.26 (s, 2H), 4.12 (s, 3H), 3.91 (m, 1H), 3.94-3.71 (m, 2H), 1.66 (d, J = 8.6 Hz, 3H).

**Example 78 Preparation of (R)-N-(4-(chlorodifluoromethoxy)phenyl)-6-(2-(5-cyanopyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-78)**

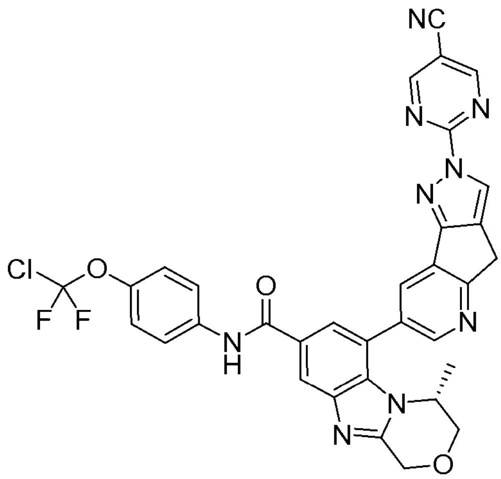

T-78

The following synthetic route was adopted:

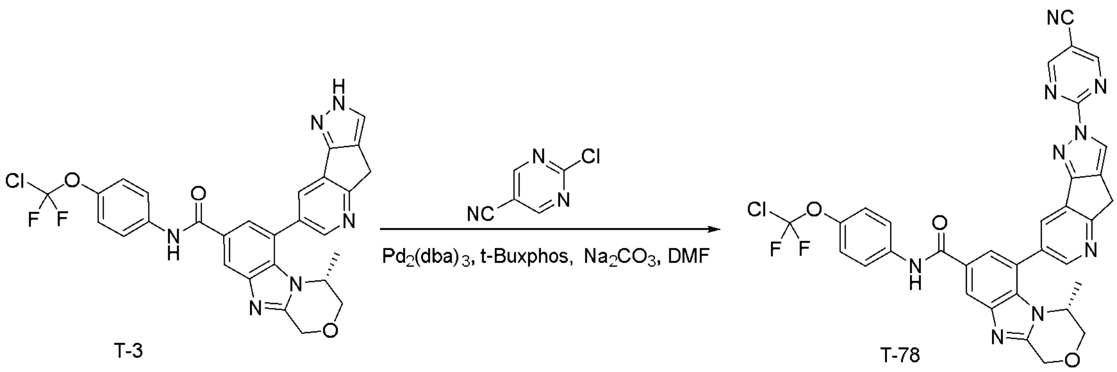


Example 78 was prepared using compound T-3 and 2-chloro-5-cyanopyrimidine as the raw materials according to the method described in example 4, with a yield of 21%. LC-MS (APCI): m/z=666.5(M+1)+. $^1$H NMR (400 MHz, DMSO-$d_6$) δ 10.22 (s, 1H), 8.91 (s, 1H), 8.62 (s, 1H), 8.39 (s, 2H), 7.61 (d, J = 8.8 Hz, 1H), 7.55 (d, J = 8.4 Hz, 2H), 7.34 (d, J = 8.6 Hz, 2H), 7.21 (d, J = 8.6 Hz, 2H), 5.02 (s, 2H), 4.26 (s, 2H), 3.92 (m, 1H), 3.94-3.71 (m, 2H), 1.64 (d, J = 8.6 Hz, 3H).

**Example 79 Preparation of (R)-N-(4-(chlorodifluoromethoxy)phenyl)-4-methyl-6-(2-(5-(trifluoromethyl)pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-79)**

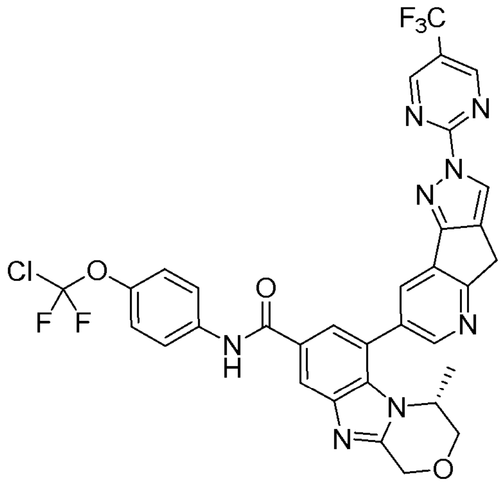

T-79

The following synthetic route was adopted:

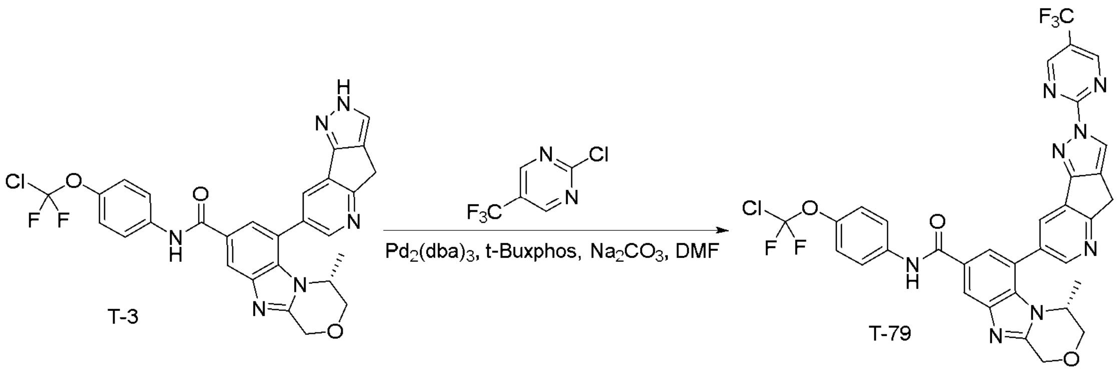


Example 79 was prepared using compound T-3 and 2-chloro-5-trifluoromethylpyrimidine as the raw materials according to the method described in example 4, with a yield of 22%. LC-MS (APCI): m/z=709.5(M+1)$^+$. $^1$H NMR (400 MHz, DMSO-$d_6$) δ 10.23 (s, 1H), 8.91 (s, 1H), 8.62 (s, 1H), 8.55 (s, 2H), 7.63 (d, J = 8.8 Hz, 1H), 7.59 (d, J = 8.4 Hz, 2H), 7.34 (d, J = 8.6 Hz, 2H), 7.21 (d, J = 8.6 Hz, 2H), 5.03 (s, 2H), 4.23 (s, 2H), 3.92 (m, 1H), 3.94-3.71 (m, 2H), 1.62 (d, J = 8.6 Hz, 3H).

**Example 80 Preparation of (R)-N-(4-(chlorodifluoromethoxy)phenyl)-4-methyl-6-(2-(5-phenylpyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-80)**

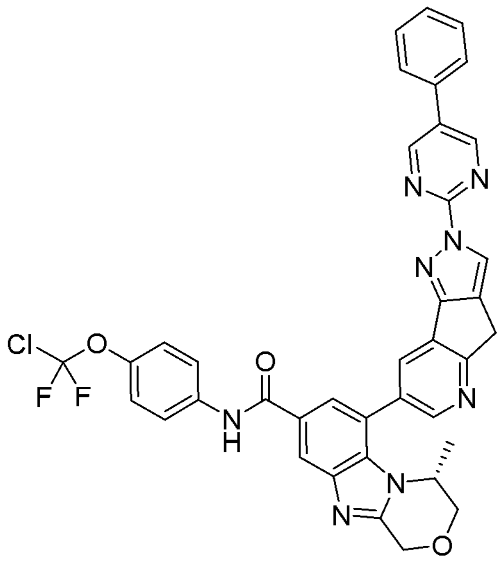

T-80

The following synthetic route was adopted:

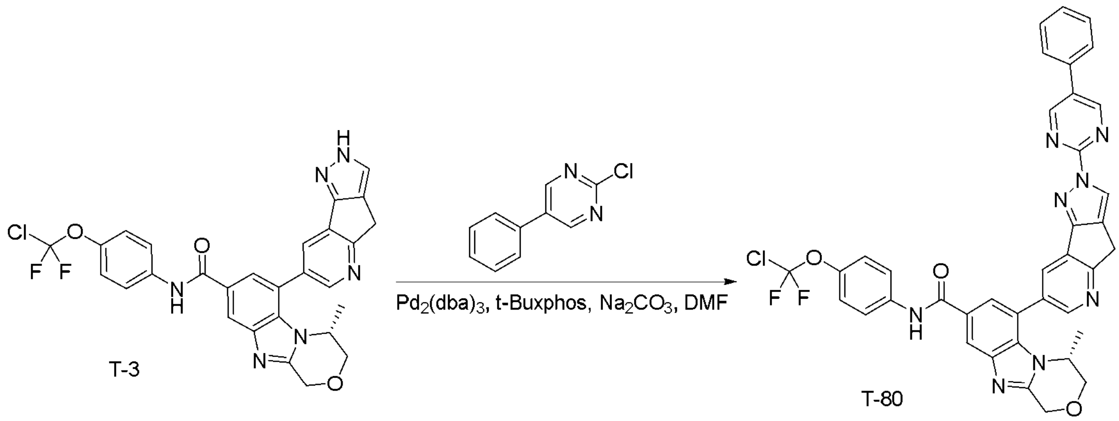


Example 80 was prepared using compound T-3 and 2-chloro-5-phenylpyrimidine as the raw materials according to the method described in example 4, with a yield of 20%. LC-MS (APCI): m/z=717.6(M+1)$^+$. $^1$H NMR (400 MHz, DMSO-$d_6$) δ 10.22 (s, 1H), 8.91 (s, 1H), 8.62 (s, 1H), 8.30 (s, 2H), 7.65 (d, J = 8.8 Hz, 1H), 7.57 (d, J = 8.4 Hz, 1H), 7.34 (d, J = 8.6 Hz, 2H), 7.21 (d, J = 8.6 Hz, 2H), 7.08-6.55 (m, 5H), 5.03 (s, 2H), 4.26 (s, 2H), 3.92 (m, 1H), 3.94-3.71 (m, 2H), 1.64 (d, J = 8.6 Hz, 3H).

**Example 81 Preparation of (R)-N-(4-(chlorodifluoromethoxy)phenyl)-4-methyl-6-(2-(4-phenyloyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4] oxazine-8-carboxamide (compound T-81)**

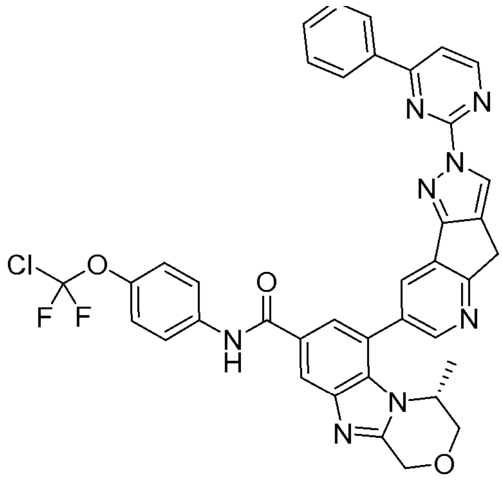

T-81

The following synthetic route was adopted:

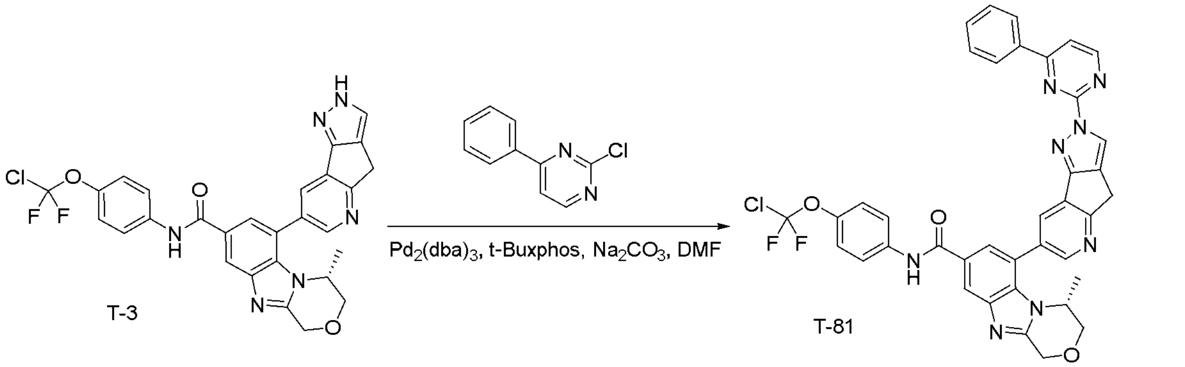


Example 81 was prepared using compound T-3 and 2-chloro-4-phenylpyrimidine as the raw materials according to the method described in example 4, with a yield of 32%. LC-MS (APCI): m/z=717.2(M+1)$^+$. $^1$H NMR (400 MHz, DMSO-*d$_6$*) δ 10.23 (s, 1H), 8.92 (s, 1H), 8.62 (s, 1H), 8.31 (d, J = 2.3 Hz, 1H), 8.03 (d, J = 2.3 Hz, 1H), 7.65 (d, J = 8.8 Hz, 1H), 7.57 (d, J = 8.4 Hz, 1H), 7.34 (d, J = 8.6 Hz, 2H), 7.22 (d, J = 8.6 Hz, 2H), 7.11-6.37 (m, 5H), 5.03 (s, 2H), 4.26 (s, 2H), 3.92 (m, 1H), 3.94-3.71 (m, 2H), 1.64 (d, J = 8.6 Hz, 3H).

**Example 82 Preparation of (R)-N-(4-(chlorodifluoromethoxy)phenyl)-4-methyl-6-(1-(guinazolin-2-yl)-1,4-dihvdropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-82-A) and (R)-N-(4-(chlorodifluoromethoxy)phenyl)-4-methyl-6-(2-(quinazolin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-82-B)**

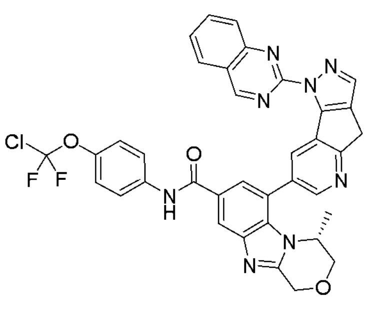

T-82-A and

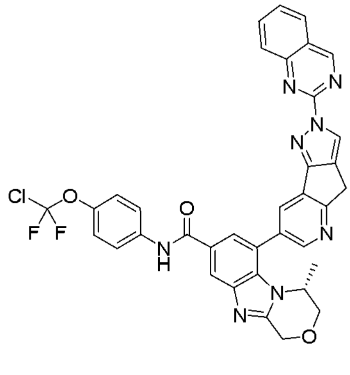

T-82-B

The following synthetic route was adopted:

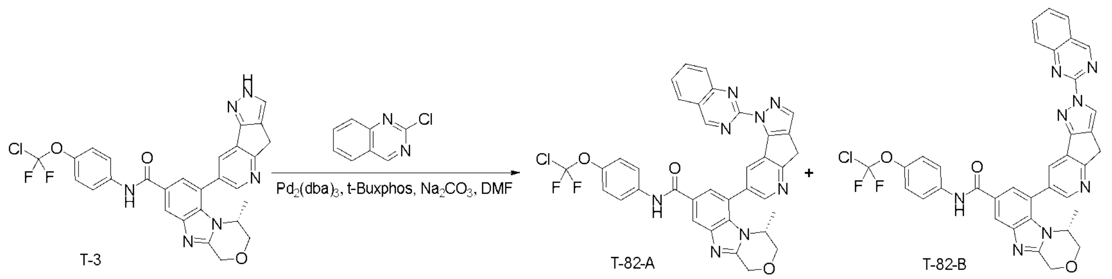


Compounds T-82-A and T-82-B were prepared using compound T-3 and 2-chloroquinazoline as the raw materials according to the method described in example 4, with yields of 8% and 24%, respectively. Compound T-82-A: LC-MS (APCI): m/z=691.2(M+1)$^+$. $^1$H NMR (400 MHz, DMSO-$d_6$) δ 10.23 (s, 1H), 8.92 (s, 1H), 8.62 (s, 1H), 8.41 (s, 1H), 8.33 (s, 1H), 8.23 (d, J = 2.3 Hz, 1H), 7.65 (d, J = 8.8 Hz, 1H), 7.57 (d, J = 8.4 Hz, 2H), 7.44 (d, J = 8.4 Hz, 2H), 7.34 (d, J = 8.6 Hz, 2H), 7.21 (d, J = 8.6 Hz, 2H), 5.03 (s, 2H), 4.26 (s, 2H), 3.92 (m, 1H), 3.94-3.71 (m, 2H), 1.64 (d, J = 8.6 Hz, 3H).

Compound T-82-B: LC-MS (APCI): m/z=691.6(M+1)$^+$. $^1$H NMR (400 MHz, DMSO-$d_6$) δ 10.22 (s, 1H), 8.91 (s, 1H), 8.62 (s, 1H), 8.41 (s, 1H), 8.33 (s, 1H), 8.23 (d, J = 2.3 Hz, 1H), 7.65 (d, J = 8.8 Hz, 1H), 7.57 (d, J = 8.4 Hz, 2H), 7.44 (d, J = 8.4 Hz, 2H), 7.34 (d, J = 8.6 Hz, 2H), 7.22 (d, J = 8.6 Hz, 2H), 5.03 (s, 2H), 4.29 (s, 2H), 3.91 (m, 1H), 3.94-3.76 (m, 2H), 1.63 (d, J = 8.6 Hz, 3H).

**Example 83 Preparation of (R)-N-(4-(chlorodifluoromethoxy)phenyl)-4-methyl-6-(2-(thieno[2,3-d]pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-83)**

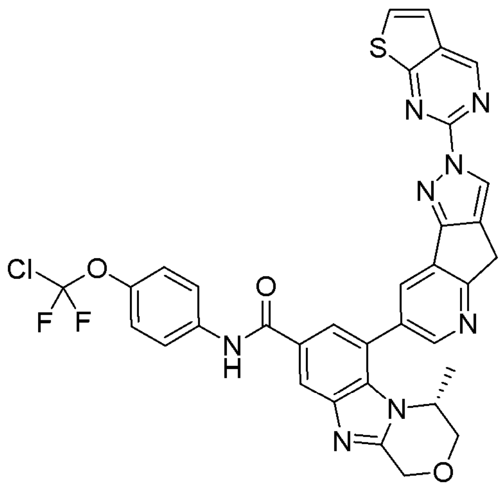

T-83

The following synthetic route was adopted:

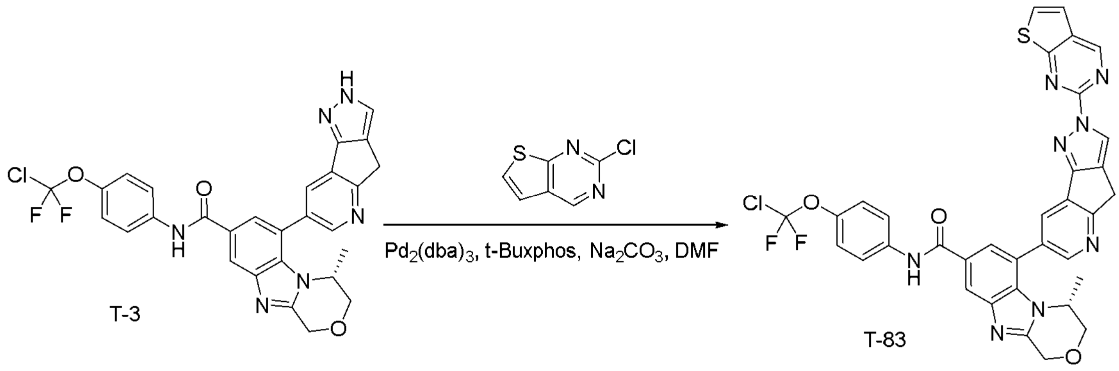


Example 83 was prepared using compound T-3 and 2-chlorothieno[2,3-d]pyrimidine as the raw materials according to the method described in example 4, with a yield of 33%. LC-MS (APCI): m/z=697.1$(M+1)^+$. $^1$H NMR (400 MHz, DMSO-$d_6$) δ 10.23 (s, 1H), 8.91 (s, 1H), 8.62 (s, 1H), 8.41 (s, 1H), 8.34 (s, 1H), 8.21 (d, J = 2.3 Hz, 1H), 7.66 (d, J = 8.8 Hz, 1H), 7.57 (d, J = 8.4 Hz, 1H), 7.34 (d, J = 8.6 Hz, 2H), 7.22 (d, J = 8.6 Hz, 2H), 6.81 (d, J = 8.8 Hz, 1H), 5.03 (s, 2H), 4.30 (s, 2H), 3.91 (m, 1H), 3.95-3.76 (m, 2H), 1.64 (d, J = 8.6 Hz, 3H).

**Example 84 Preparation of (R)-N-(4-(chlorodifluoromethoxy)phenyl)-4-methyl-6-(2-(5-methyl-5H-pyrrolo[3,2-d]pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-84)**

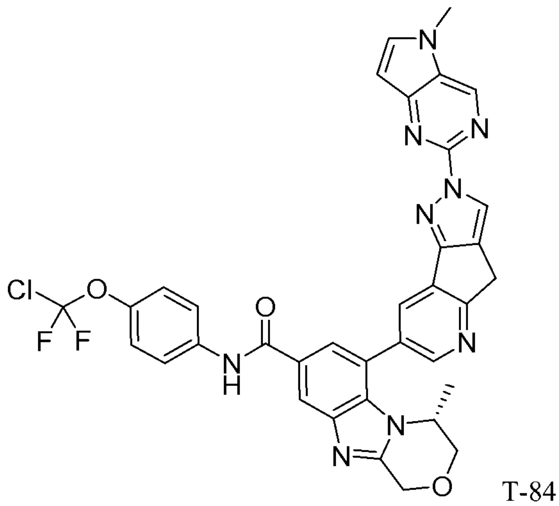

T-84

The following synthetic route was adopted:

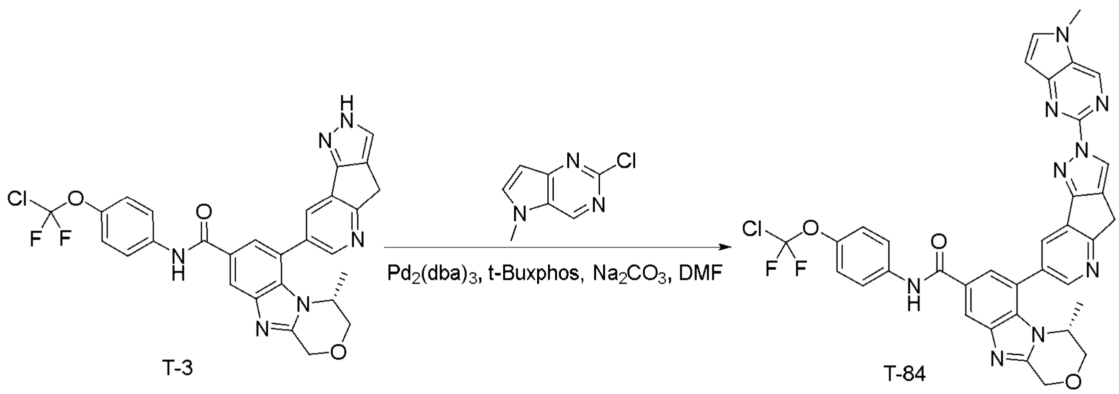


Example 84 was prepared using compound T-3 and 2-chloro-5-methyl-5H-pyrrolo[3,2-d]pyrimidine as the raw

materials according to the method described in example 4, with a yield of 26%. LC-MS (APCI): m/z=694.1(M+1)+. $^1$H NMR (400 MHz, DMSO-$d_6$) δ 10.23 (s, 1H), 8.90 (s, 1H), 8.61 (s, 1H), 8.52 (s, 1H), 8.34 (s, 1H), 8.21 (d, J = 2.3 Hz, 1H), 7.66 (d, J = 8.8 Hz, 1H), 7.57 (d, J = 8.4 Hz, 1H), 7.33 (d, J = 8.6 Hz, 2H), 7.22 (d, J = 8.6 Hz, 2H), 6.93 (d, J = 8.8 Hz, 1H), 5.06 (s, 2H), 4.30 (s, 2H), 3.91 (m, 1H), 3.95-3.76 (m, 2H), 3.58 (s, 3H), 1.62 (d, J = 8.4 Hz, 3H).

**Example 85 Preparation of (R)-N-(4-(chlorodifluoromethoxy)phenyl)-4-methyl-6-(2-(7-methyl-7H-pyrrolo[2,3-d]pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-85)**

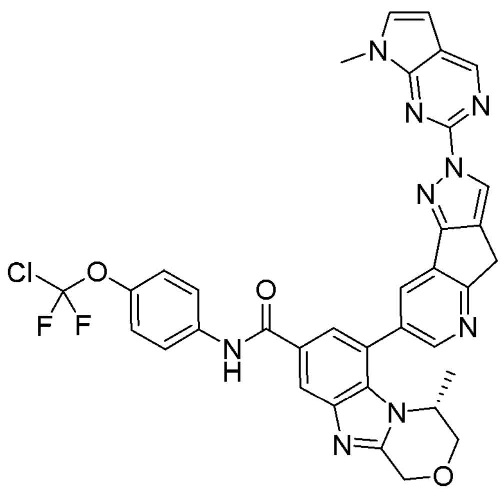

T-85

The following synthetic route was adopted:

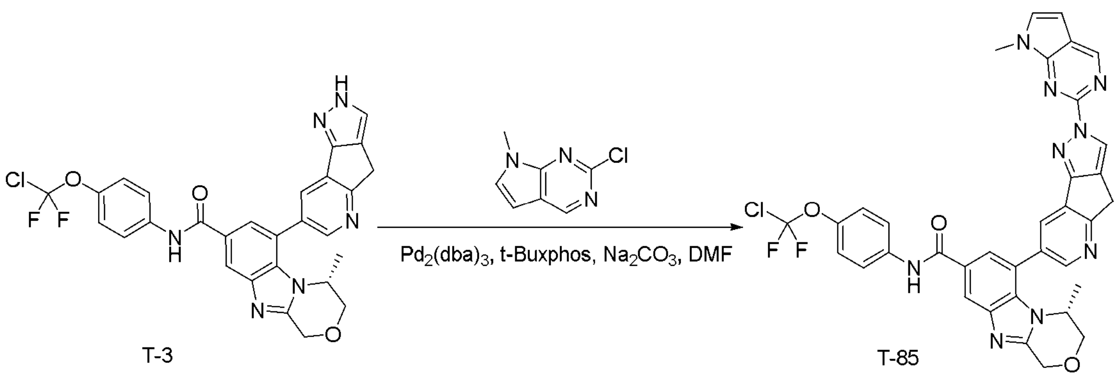


Example 85 was prepared using compound T-3 and 2-chloro-7-methylpyrrolopyrimidine as the raw materials according to the method described in example 4, with a yield of 12%. LC-MS (APCI): m/z=694.2(M+1)+. $^1$H NMR (400 MHz, DMSO-$d_6$) δ 10.22 (s, 1H), 8.93 (s, 1H), 8.62 (s, 1H), 8.41 (s, 1H), 8.34 (s, 1H), 8.21 (d, J = 2.3 Hz, 1H), 7.66 (d, J = 8.8 Hz, 1H), 7.57 (d, J = 8.4 Hz, 1H), 7.34 (d, J = 8.6 Hz, 1H), 7.21 (d, J = 8.6 Hz, 2H), 6.81 (d, J = 8.8 Hz, 2H), 5.03 (s, 2H), 4.32 (s, 2H), 3.91 (m, 1H), 3.95-3.76 (m, 2H), 3.55 (s, 3H),1.64 (d, J = 8.6 Hz, 3H).

**Example 86 Preparation of (R)-N-(4-(chlorodifluoromethoxy)phenyl)-4-methyl-6-(2-(pyrido[2,3-d]pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-86)**

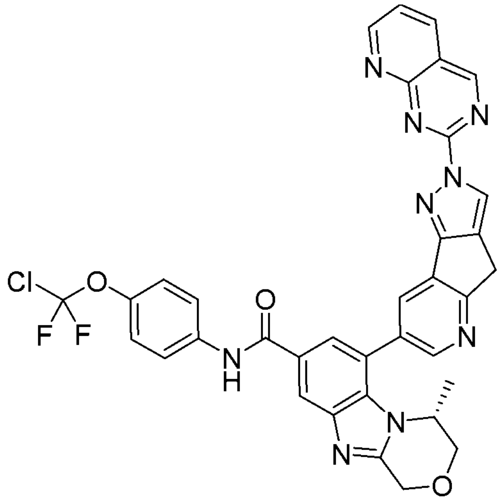

T-86

The following synthetic route was adopted:

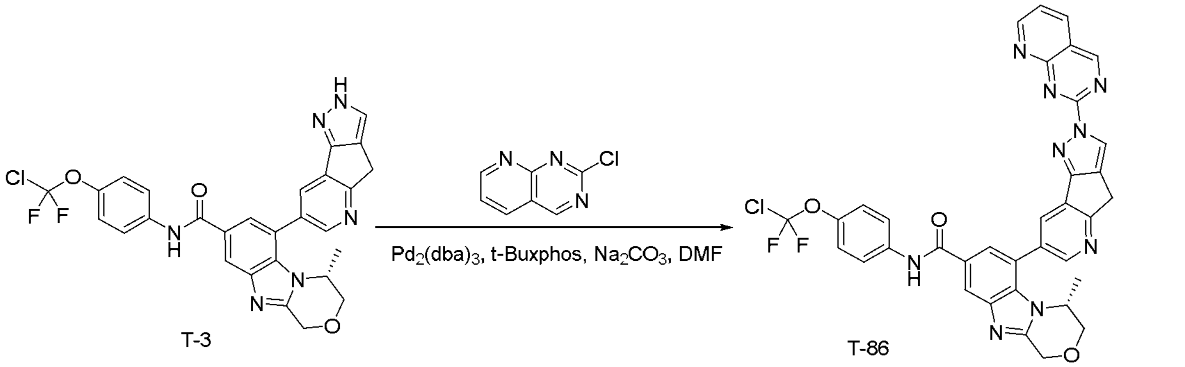


Example 86 was prepared using compound T-3 and 2-chloropyrido[2,3-d]pyrimidine as the raw materials according to the method described in example 4, with a yield of 30%. LC-MS (APCI): m/z=692.6(M+1)$^+$. $^1$H NMR (400 MHz, DMSO-$d_6$) δ 10.23 (s, 1H), 8.92 (s, 1H), 8.62 (s, 1H), 8.41 (s, 1H), 8.34 (s, 1H), 8.21 (d, J = 2.3 Hz, 1H), 7.66 (d, J = 8.8 Hz, 1H), 7.57 (d, J = 8.4 Hz, 2H), 7.34 (d, J = 8.6 Hz, 1H), 7.22 (d, J = 8.4 Hz, 2H), 6.81 (d, J = 8.4 Hz, 2H), 5.03 (s, 2H), 4.31 (s, 2H), 3.94 (m, 1H), 3.95-3.76 (m, 2H), 1.64 (d, J = 8.6 Hz, 3H).

**Example 87 Preparation of (R)-N-(4-(chlorodifluoromethoxy)phenyl)-4-methyl-6-(2-(4-(pyridin-3-yl)pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-87)**

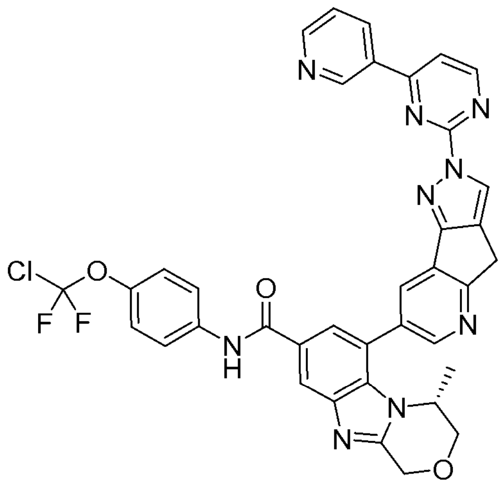

T-87

The following synthetic route was adopted:

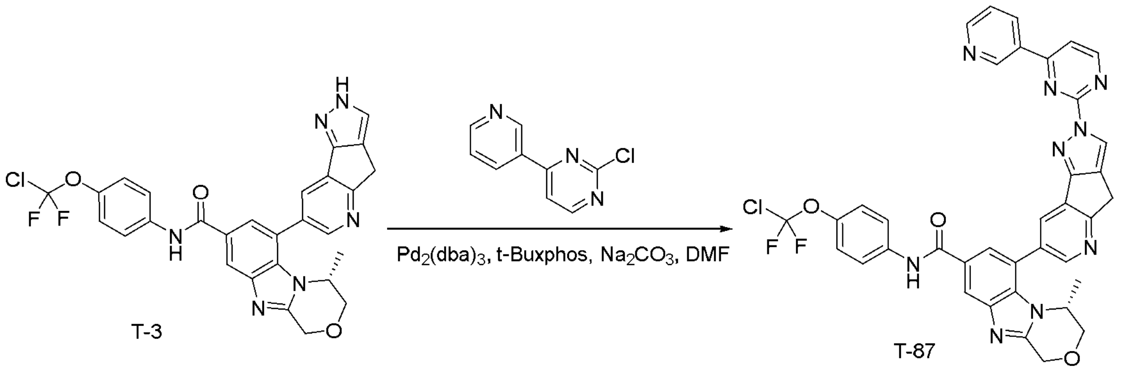


Example 87 was prepared using compound T-3 and 4-(3-pyridyl)-2-chloropyrimidine as the raw materials according to the method described in example 4, with a yield of 61%. LC-MS (APCI): m/z=718.3(M+1)$^+$. $^1$H NMR (400 MHz, DMSO-*d*$_6$) δ 10.23 (s, 1H), 8.92 (s, 1H), 8.62 (s, 1H), 8.41 (s, 1H), 8.34 (s, 1H), 8.29-8.23 (m, 3H), 7.66 (d, J = 8.8 Hz, 1H), 7.57 (d, J = 8.4 Hz, 2H), 7.34 (d, J = 8.6 Hz, 1H), 7.22 (d, J = 8.4 Hz, 2H), 6.81 (d, J = 8.4 Hz, 2H), 5.03 (s, 2H), 4.31 (s, 2H), 3.94 (m, 1H), 3.95-3.76 (m, 2H), 1.64 (d, J = 8.6 Hz, 3H).

**Example 88 Preparation of (R)-N-(4-(chlorodifluoromethoxy)phenyl)-4-methyl-6-(2-(4-morpholinopyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-88)**

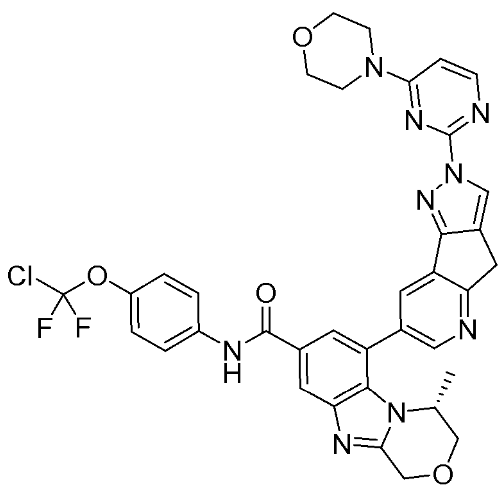

T-88

The following synthetic route was adopted:

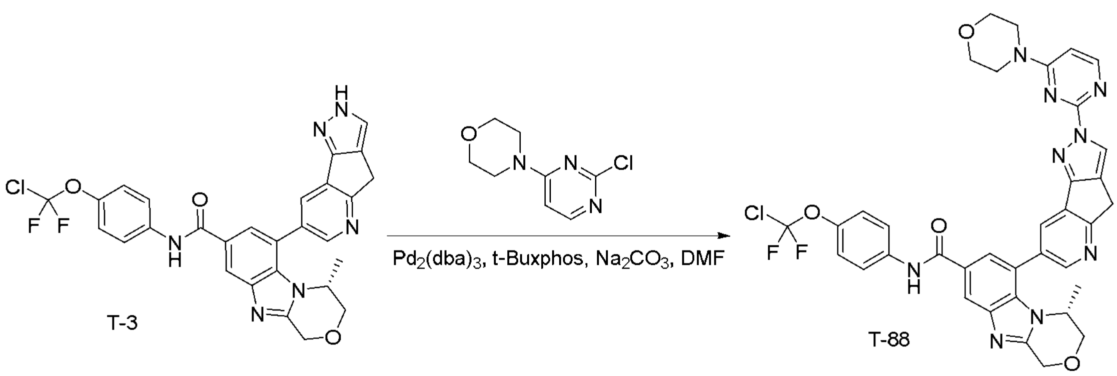


Example 88 was prepared using compound T-3 and 2-chloro-4-morpholinopyrimidine as the raw materials according to the method described in example 4, with a yield of 55%. LC-MS (APCI): m/z=726.3$(M+1)^+$. $^1H$ NMR (400 MHz, DMSO-$d_6$) δ 10.21 (s, 1H), 8.91 (s, 1H), 8.62 (s, 1H), 8.42 (s, 1H), 8.22 (d, J = 2.3 Hz, 1H), 7.88 (d, J = 2.6 Hz, 1H), 7.57 (d, J = 8.4 Hz, 1H), 7.34 (d, J = 8.6 Hz, 1H), 7.22 (d, J = 8.4 Hz, 2H), 6.81 (d, J = 8.4 Hz, 2H), 5.02 (s, 2H), 4.48 (t, J = 4.4 Hz, 4H), 4.31 (s, 2H), 3.94 (m, 1H), 3.95-3.76 (m, 2H), 3.34 (t, J = 4.4 Hz, 4H), 1.64 (d, J = 8.6 Hz, 3H).

**Example 89 Preparation of (R)-N-(4-(chlorodifluoromethoxy)phenyl)-6-(2-(4-(4-fluorophenyl)pyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-89)**

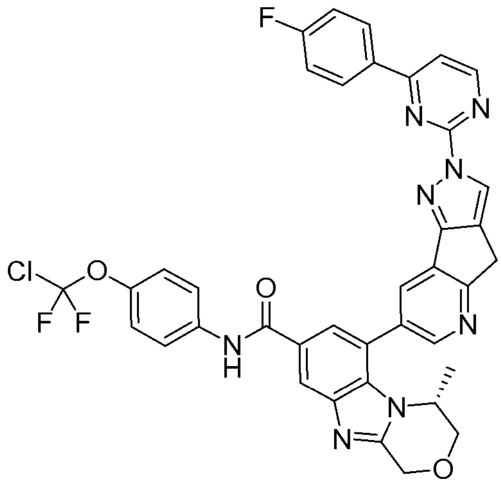

T-89

The following synthetic route was adopted:

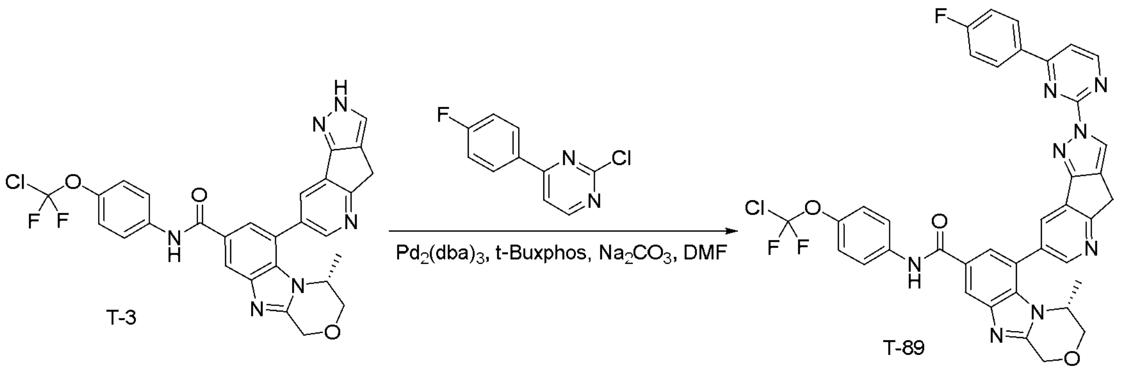


Example 89 was prepared using compound T-3 and 2-chloro-4-(4-fluoro-phenyl)pyrimidine as the raw materials according to the method described in example 4, with a yield of 40%. LC-MS (APCI): m/z=735.2(M+1)$^+$. $^1$H NMR (400 MHz, DMSO-$d_6$) δ 10.23 (s, 1H), 8.92 (s, 1H), 8.62 (s, 1H), 8.42 (s, 1H), 8.33-8.21 (m, 4H), 7.66 (d, J = 8.8 Hz, 2H), 7.33 (d, J = 8.6 Hz, 1H), 7.25 (d, J = 8.4 Hz, 1H), 6.82 (d, J = 8.4 Hz, 2H), 5.03 (s, 2H), 4.30 (s, 2H), 3.94 (m, 1H), 3.95-3.76 (m, 2H), 1.64 (d, J = 8.6 Hz, 3H).

**Example 90 Preparation of (R)-N-(4-(chlorodifluoromethoxy)phenyl)-4-methyl-6-(2-(4-(1-methyl-1H-oyrazol-3-yl)pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-90)**

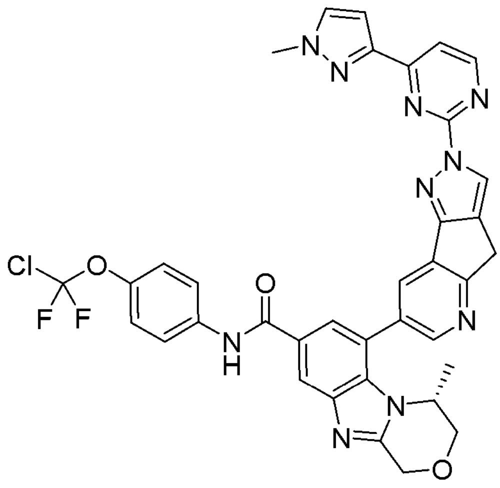

T-90

The following synthetic route was adopted:

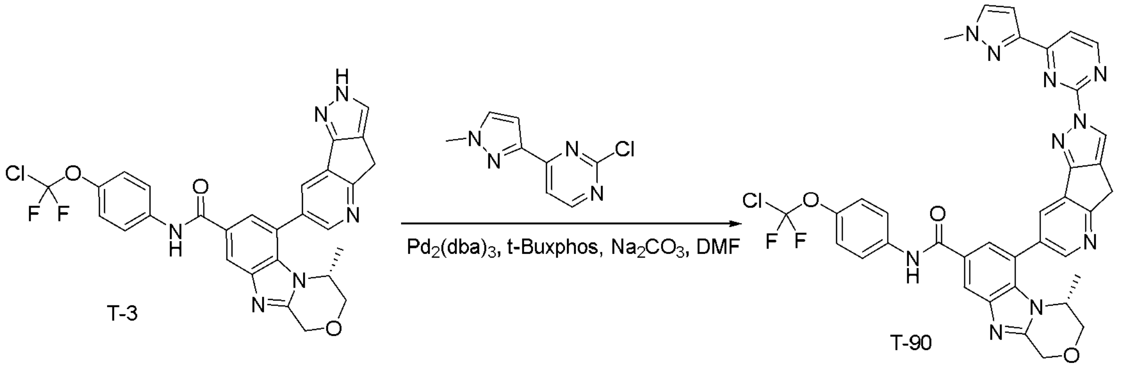


Example 90 was prepared using compound T-3 and 4-(1-methylpyrazol-3-yl)-2-chloropyrimidine as the raw materials according to the method described in example 4, with a yield of 9%. LC-MS (APCI): m/z=721.8(M+1)$^+$. $^1$H NMR (400 MHz, DMSO-$d_6$) δ 10.21 (s, 1H), 8.88 (s, 1H), 8.62 (s, 1H), 8.42 (s, 1H), 8.25-8.01 (m, 4H), 7.66 (d, J = 8.8 Hz, 2H), 7.31 (d, J = 8.6 Hz, 1H), 7.22 (d, J = 8.4 Hz, 1H), 6.81 (d, J = 8.4 Hz, 2H), 5.03 (s, 2H), 4.30 (s, 2H), 3.94 (m, 1H), 3.95-3.76 (m, 2H), 1.63 (d, J = 8.6 Hz, 3H).

**Example 91 Preparation of (R)-N-(4-(chlorodifluoromethoxy)phenyl)-6-(1-(4-(3-fluoro-4-methoxyphenyl)pyrimidin-2-yl)-1,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-91-A) and (R)-N-(4-(chlorodifluoromethoxy)phenyl)-6-(2-(4-(3-fluoro-4-methoxyphenyl)pyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-91-B)**

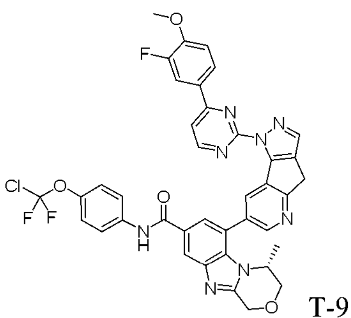

T-91-A and

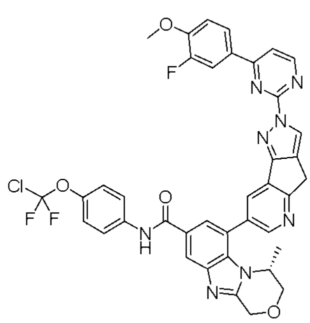

T-91-B

The following synthetic route was adopted:

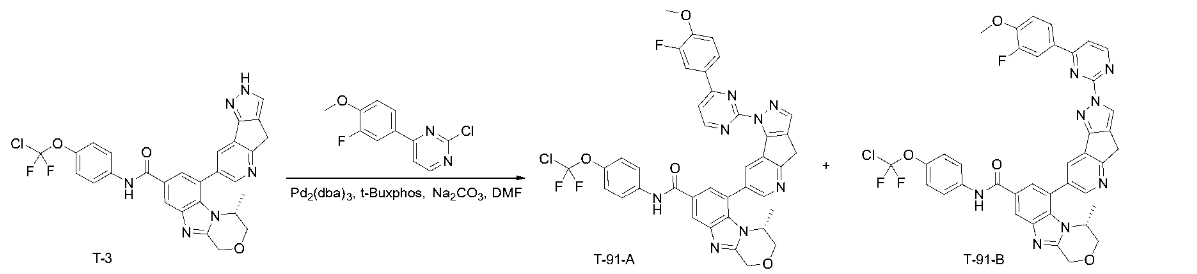


T-3 T-91-A T-91-B

Compounds T-91-A and T-91-B were prepared using compound T-3 and 2-chloro-4-(3-fluoro-4-methoxyphenyl) pyrimidine as the raw materials according to the method described in example 4, with yields of 12% and 25%, respectively. Compound T-91-A: LC-MS (APCI): m/z=764.4(M+1)$^+$. $^1$H NMR (400 MHz, DMSO-$d_6$) δ 10.23 (s, 1H), 8.92 (s, 1H), 8.67 (s, 1H), 8.41 (s, 1H), 8.35-8.21 (m, 4H), 7.66 (d, J = 8.8 Hz, 2H), 7.33 (d, J = 8.6 Hz, 1H), 7.25 (d, J = 8.4 Hz, 2H), 6.82 (d, J = 8.4 Hz, 2H), 5.03 (s, 2H), 4.30 (s, 2H), 4.15 (s, 3H), 3.94 (m, 1H), 3.95-3.76 (m, 2H), 1.64 (d, J = 8.6 Hz, 3H).

Compound T-91-B: LC-MS (APCI): m/z=764.3(M+1)$^+$. $^1$H NMR (400 MHz, DMSO-$d_6$) δ 10.21 (s, 1H), 8.90 (s, 1H), 8.67 (s, 1H), 8.41 (s, 1H), 8.35-8.21 (m, 4H), 7.66 (d, J = 8.8 Hz, 2H), 7.33 (d, J = 8.6 Hz, 1H), 7.26 (d, J = 8.4 Hz, 2H), 6.82 (d, J = 8.4 Hz, 2H), 5.06 (s, 2H), 4.30 (s, 2H), 4.15 (s, 3H), 3.94 (m, 1H), 3.95-3.76 (m, 2H), 1.62 (d, J = 8.6 Hz, 3H).

## Example 92 Preparation of (R)-N-(4-(chlorodifluoromethoxy)phenyl)-6-(2-(4-(2-fluorophenyl)pyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-92)

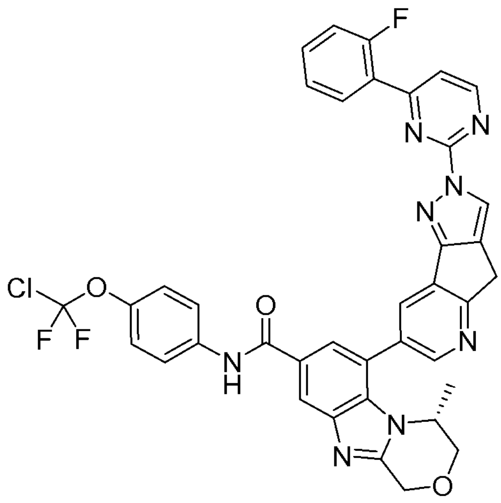

T-92

The following synthetic route was adopted:

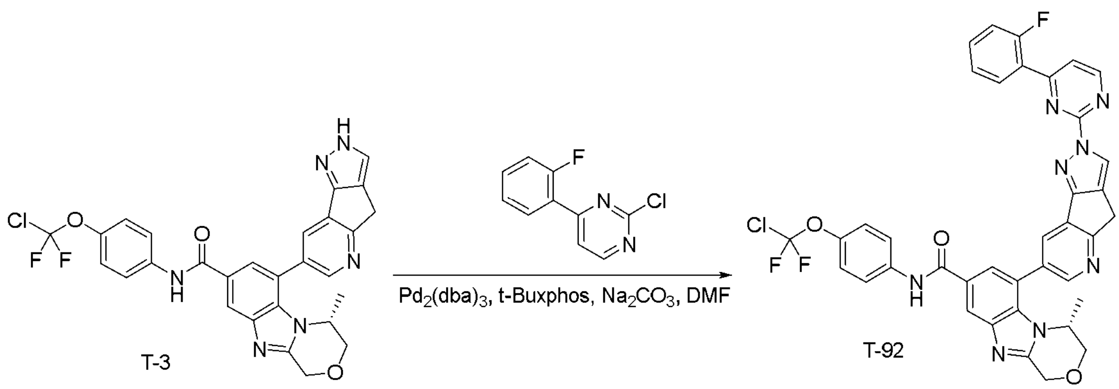


T-3 T-92

Example 92 was prepared using compound T-3 and 2-chloro-4-(2-fluorophenyl)pyrimidine as the raw materials

according to the method described in example 4, with a yield of 30%. LC-MS (APCI): m/z=734.2(M+1)+. 1H NMR (400 MHz, DMSO-$d_6$) δ 10.22 (s, 1H), 8.91 (s, 1H), 8.67 (s, 1H), 8.41 (s, 1H), 8.35-8.21 (m, 3H), 7.66 (d, J = 8.8 Hz, 2H), 7.33 (d, J = 8.6 Hz, 1H), 7.26 (d, J = 8.4 Hz, 2H), 6.82 (d, J = 8.4 Hz, 2H), 6.65-6.38 (m, 2H), 5.06 (s, 2H), 4.31 (s, 2H), 3.96 (m, 1H), 3.95-3.74 (m, 2H), 1.63 (d, J = 8.6 Hz, 3H).

**Example 93 Preparation of (R)-N-(4-(chlorodifluoromethoxy)phenyl)-6-(2-(4-(2-fluoro-3-methoxyphenyl)pyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-93)**

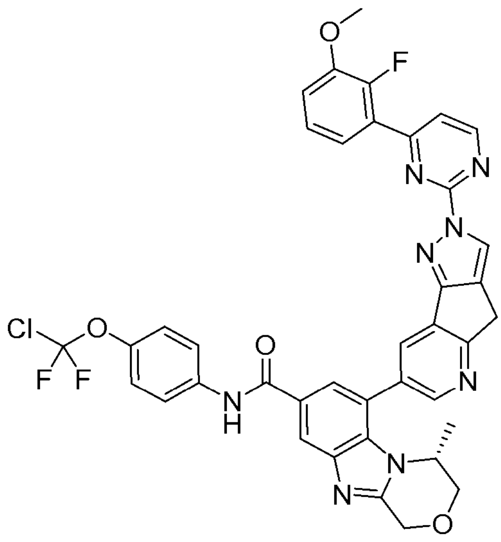

T-93

The following synthetic route was adopted:

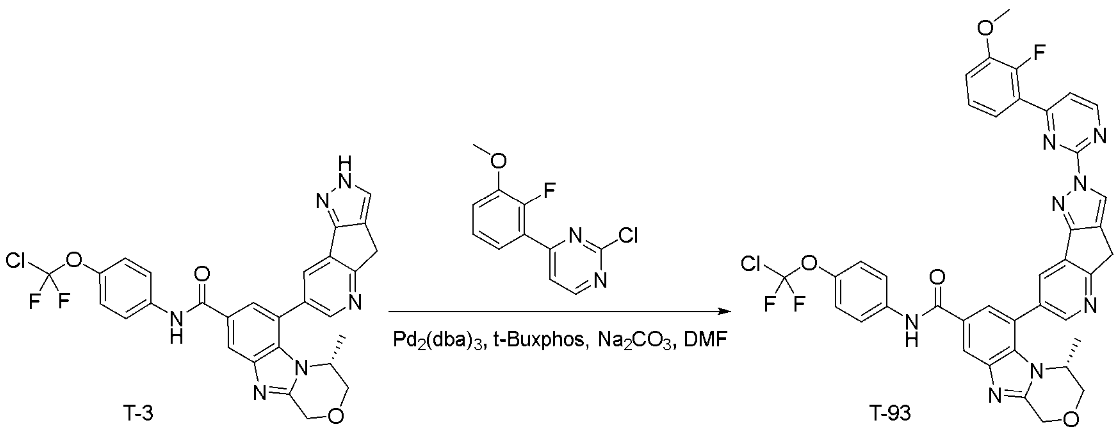


Example 93 was prepared using compound T-3 and 2-chloro-4-(2-fluoro-3-methoxyphenyl)pyrimidine as the raw materials according to the method described in example 4, with a yield of 34%. LC-MS (APCI): m/z=765.1(M+1)+. 1H NMR (400 MHz, DMSO-$d_6$) δ 10.23 (s, 1H), 8.91 (s, 1H), 8.67 (s, 1H), 8.42 (s, 1H), 8.35-8.21 (m, 2H), 7.66 (d, J = 8.8 Hz, 2H), 7.33 (d, J = 8.6 Hz, 1H), 7.26 (d, J = 8.4 Hz, 2H), 6.82 (d, J = 8.4 Hz, 2H), 6.64-6.28 (m, 2H), 5.06 (s, 2H), 4.30 (s, 2H), 4.08 (s, 3H), 3.94 (m, 1H), 3.95-3.76 (m, 2H), 1.62 (d, J = 8.6 Hz, 3H).

**Example 94 Preparation of (R)-N-(4-(chlorodifluoromethoxy)phenyl)-6-(2-(4-(6-fluoropyridin-3-yl)pyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-94)**

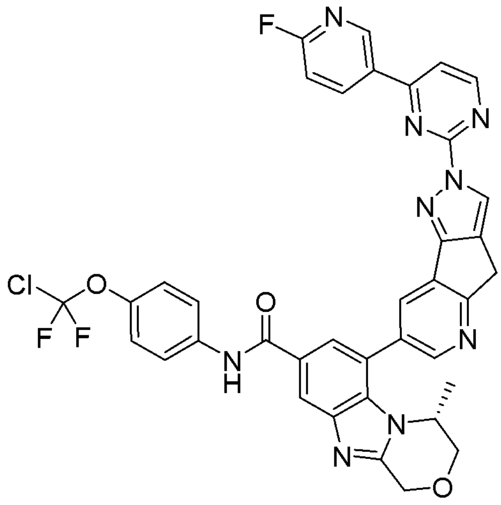

T-94

The following synthetic route was adopted:

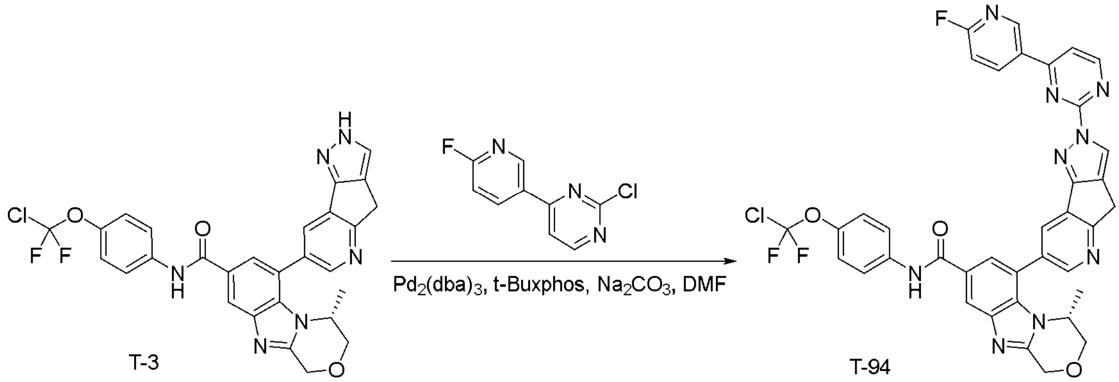


T-3

T-94

Example 94 was prepared using compound T-3 and 2-chloro-4-(6-fluoropyridin-3-yl)pyrimidine as the raw materials according to the method described in example 4, with a yield of 28%. LC-MS (APCI): m/z=736.2$(M+1)^+$. $^1H$ NMR (400 MHz, DMSO-$d_6$) δ 10.22 (s, 1H), 8.91 (s, 1H), 8.67 (s, 1H), 8.41 (s, 1H), 8.35-8.21 (m, 4H), 7.66 (d, J = 8.8 Hz, 2H), 7.33 (d, J = 8.6 Hz, 1H), 7.26 (d, J = 8.4 Hz, 2H), 6.81 (d, J = 8.4 Hz, 2H), 5.06 (s, 2H), 4.30 (s, 2H), 4.15 (s, 3H), 3.94 (m, 1H), 3.88-3.76 (m, 2H), 1.64 (d, J = 8.6 Hz, 3H).

**Example 95 Preparation of (R)-N-(4-(chlorodifluoromethoxy)phenyl)-6-(2-(4-(2-fluoropyridin-3-yl)pyrimidin-2-yl)-2,4-dihydropyrazolo[3',4':3,4]cyclopenta[1,2-b]pyridin-7-yl)-4-methyl-3,4-dihydro-1H-benzo[4,5]imidazo [2,1-c][1,4]oxazine-8-carboxamide (compound T-95)**

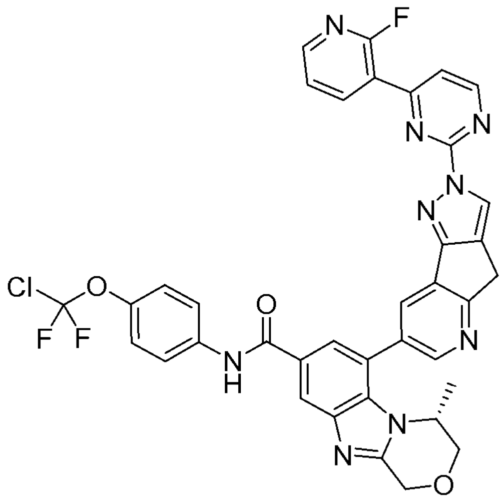

T-95

The following synthetic route was adopted:

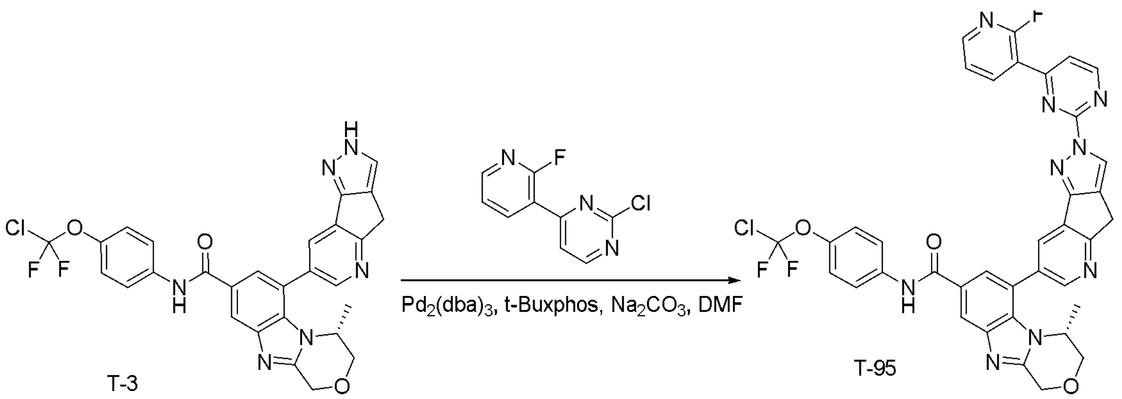


T-3

T-95

Example 95 was prepared using compound T-3 and 2-chloro-4-(2-fluoropyridin-3-yl)pyrimidine as the raw materials according to the method described in example 4, with a yield of 47%. LC-MS (APCI): m/z=736.5$(M+1)^+$. $^1H$ NMR (400 MHz, DMSO-$d_6$) δ 10.23 (s, 1H), 8.91 (s, 1H), 8.67 (s, 1H), 8.41 (s, 1H), 8.35-8.23 (m, 4H), 7.66 (d, J = 7.6 Hz, 2H), 7.33 (d, J = 8.6 Hz, 1H), 7.26 (d, J = 8.4 Hz, 2H), 6.81 (d, J = 8.4 Hz, 2H), 5.06 (s, 2H), 4.30 (s, 2H), 4.15 (s, 3H), 3.94 (m, 1H), 3.95-3.76 (m, 2H), 1.62 (d, J = 8.6 Hz, 3H).

**Example 96 Preparation of (R)-N-(4-(chlorodifluoromethoxy)phenyl)-6-(2-(4-(5-fluoropyridin-3-yl)pyrimidin-2-yl)-2,4-dihydropyrazolo[3',4':3,4]cyclopenta[1,2-b]pyridin-7-yl)-4-methyl-3,4-dihydro-1H-benzo[4,5]imidazo [2,1-c][1,4]oxazine-8-carboxamide (compound T-96)**

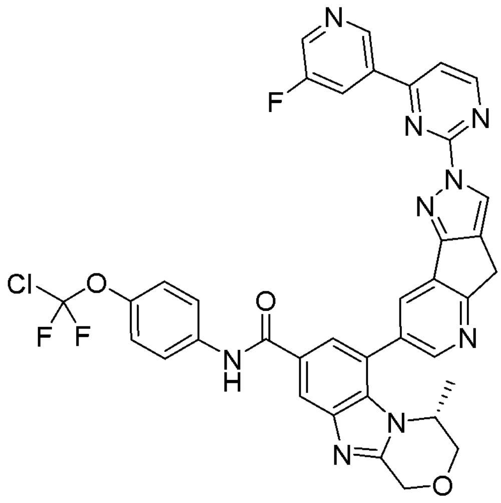

T-96

The following synthetic route was adopted:

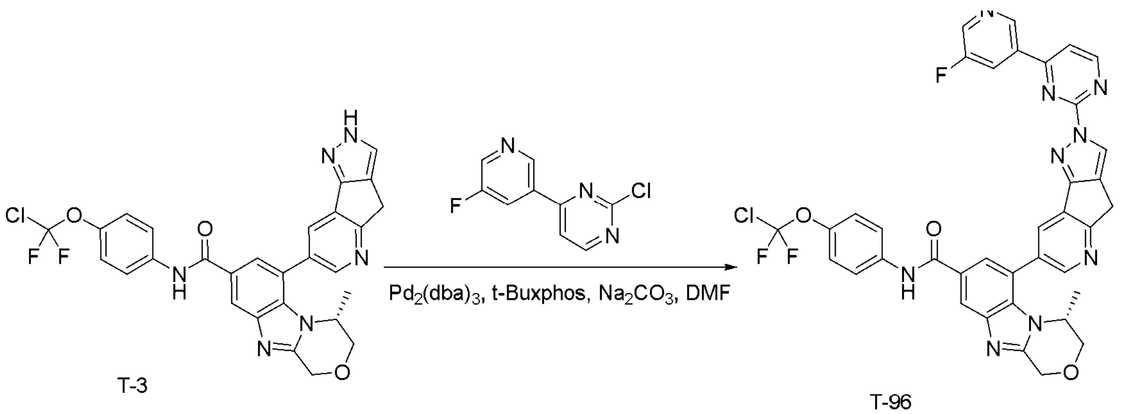


T-3

T-96

Example 96 was prepared using compound T-3 and 2-chloro-4-(5-fluoropyridin-3-yl)pyrimidine as the raw materials according to the method described in example 4, with a yield of 44%. LC-MS (APCI): m/z=736.6$(M+1)^+$. $^1$H NMR (400 MHz, DMSO-$d_6$) δ 10.23 (s, 1H), 8.91 (s, 1H), 8.67 (s, 1H), 8.41 (s, 1H), 8.35 (s, 1H), 8.26 (s, 1H), 8.22 (s, 1H), 7.66 (d, J = 8.8 Hz, 2H), 7.33 (d, J = 8.6 Hz, 1H), 7.26 (d, J = 8.4 Hz, 2H), 6.82 (d, J = 8.4 Hz, 2H), 5.06 (s, 2H), 4.30 (s, 2H), 3.94 (m, 1H), 3.95-3.76 (m, 2H), 1.63 (d, J = 8.6 Hz, 3H).

**Example 97 Preparation of (R)-N-(4-(chlorodifluoromethoxy)phenyl)-6-(2-(4-(5-fluoro-6-methoxyoyridin-3-yl) pyrimidin-2-yl)-2,4-dihydropyrazolo[3',4':3,4]cyclopenta[1,2-b]pyridin-7-yl)-4-methyl-3,4-dihydro-1H-benzo [4,5]imidazo[2,1-c][1,4]oxazine-8-carboxamide (compound T-97)**

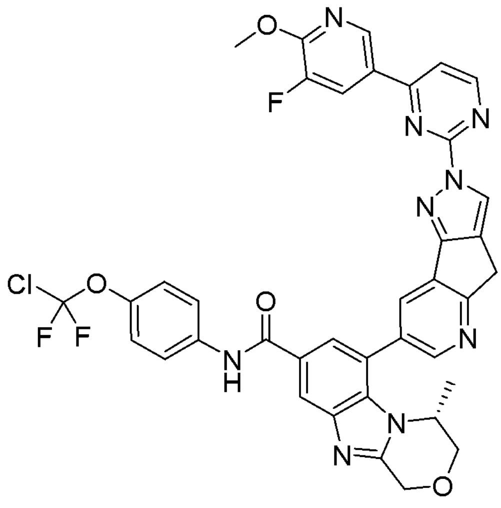

T-97

The following synthetic route was adopted:

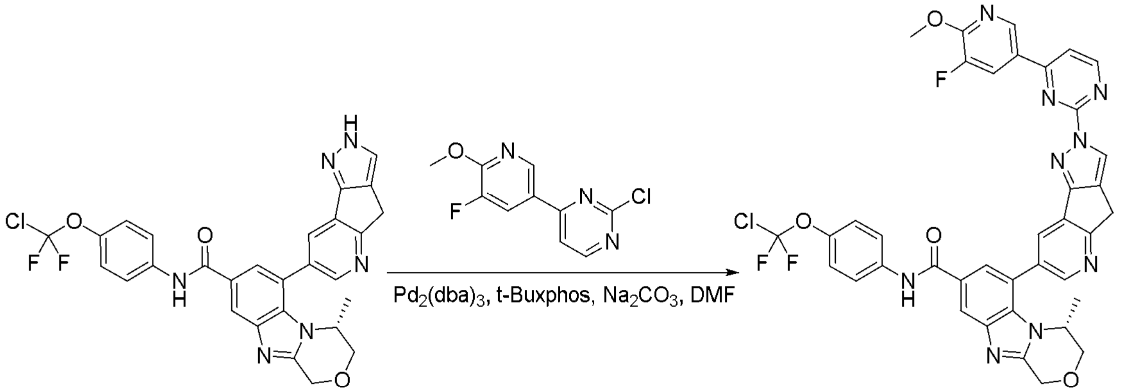


T-3

T-97

Example 97 was prepared using compound T-3 and 2-chloro-4-(5-fluoro-6-methoxypyridin-3-yl)pyrimidine as the raw materials according to the method described in example 4, with a yield of 46%. LC-MS (APCI): m/z=766.7(M+1)$^+$. $^1$H NMR (400 MHz, DMSO-$d_6$) δ 10.22 (s, 1H), 8.91 (s, 1H), 8.67 (s, 1H), 8.41 (s, 1H), 8.35-8.21 (m, 3H), 7.66 (d, J = 8.8 Hz, 2H), 7.33 (d, J = 8.6 Hz, 1H), 7.26 (d, J = 8.4 Hz, 2H), 6.82 (d, J = 8.4 Hz, 2H), 5.06 (s, 2H), 4.30 (s, 2H), 4.13 (s, 3H), 3.94 (m, 1H), 3.95-3.76 (m, 2H), 1.62 (d, J = 8.6 Hz, 3H).

**Example 98 Preparation of (R)-N-(4-(chlorodifluoromethoxy)phenyl)-6-(2-(4-(5-cyanopyridin-3-yl)pyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-98)**

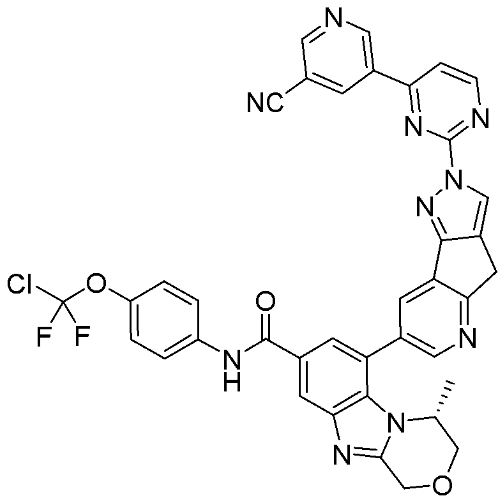

T-98

The following synthetic route was adopted:

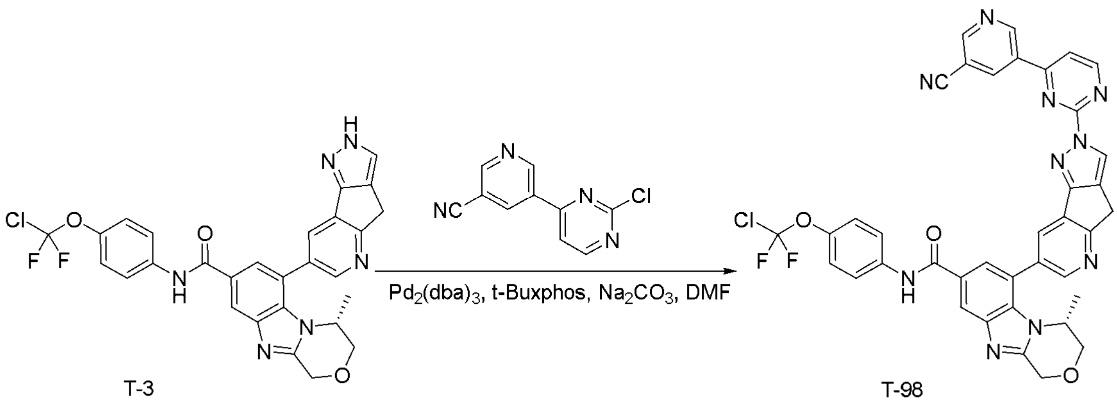


Example 98 was prepared using compound T-3 and 2-chloro-4-(5-cyanopyridin-3-yl)pyrimidine as the raw materials according to the method described in example 4, with a yield of 36%. LC-MS (APCI): m/z=743.2(M+1)$^+$. $^1$H NMR (400 MHz, DMSO-$d_6$) δ 10.21 (s, 1H), 8.90 (s, 1H), 8.67 (s, 1H), 8.41 (s, 1H), 8.36 (s, 1H), 8.23 (s, 1H), 8.19 (s, 1H), 7.66 (d, J = 8.8 Hz, 1H), 7.33 (d, J = 8.6 Hz, 1H), 7.26 (d, J = 8.4 Hz, 2H), 6.82 (d, J = 8.4 Hz, 2H), 5.06 (s, 2H), 4.30 (s, 2H), 3.92 (m, 1H), 3.95-3.76 (m, 2H), 1.63 (d, J = 8.6 Hz, 3H).

**Example 99 Preparation of (R)-N-(4-(chlorodifluoromethoxy)phenyl)-6-(2-(4-(6-cyanopyridin-3-yl)pyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-99)**

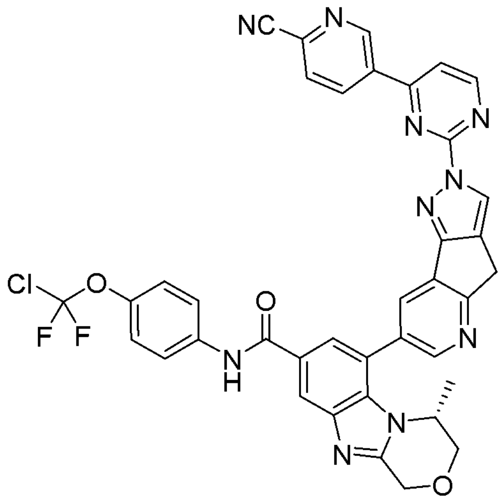

T-99

The following synthetic route was adopted:

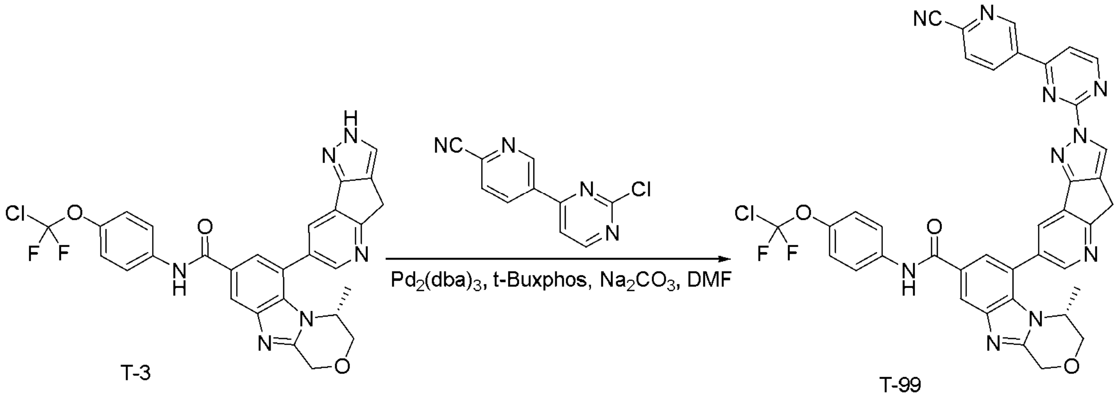


T-3

T-99

Example 99 was prepared using compound T-3 and 2-chloro-4-(6-cyanopyridin-3-yl)pyrimidine as the raw materials according to the method described in example 4, with a yield of 32%. LC-MS (APCI): m/z=743.2(M+1)$^+$. $^1$H NMR (400 MHz, DMSO-$d_6$) δ 10.23 (s, 1H), 8.90 (s, 1H), 8.67 (s, 1H), 8.41 (s, 1H), 8.35-8.21 (m, 3H), 7.66 (d, J = 8.8 Hz, 2H), 7.33 (d, J = 8.6 Hz, 1H), 7.26 (d, J = 8.4 Hz, 2H), 6.82 (d, J = 8.6 Hz, 2H), 5.06 (s, 2H), 4.30 (s, 2H), 4.15 (s, 3H), 3.94 (m, 1H), 3.92-3.76 (m, 2H), 1.66 (d, J = 8.6 Hz, 3H).

**Example 100 Preparation of (R)-6-(2-([4,5'-bipyrimidin]-2-yl)-2,4-dihydropyrazolo[3',4':3,4]cyclopenta[1,2-b]pyridin-7-yl)-N-(4-(chlorodifluoromethoxy)phenyl)-4-methyl-3,4-dihydro-1H-benzo[4,5]imidazo[2,1-c][1,4]oxazine-8-carboxamide (compound T-100)**

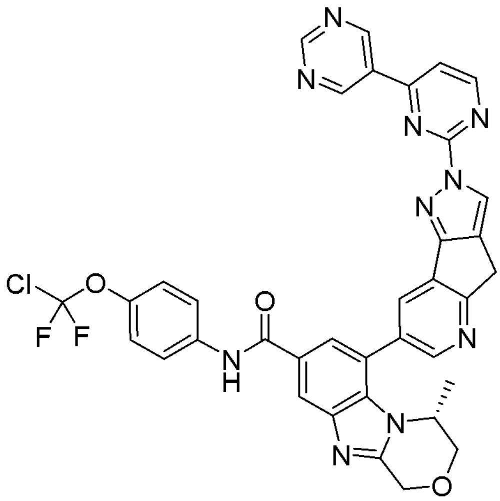

T-100

The following synthetic route was adopted:

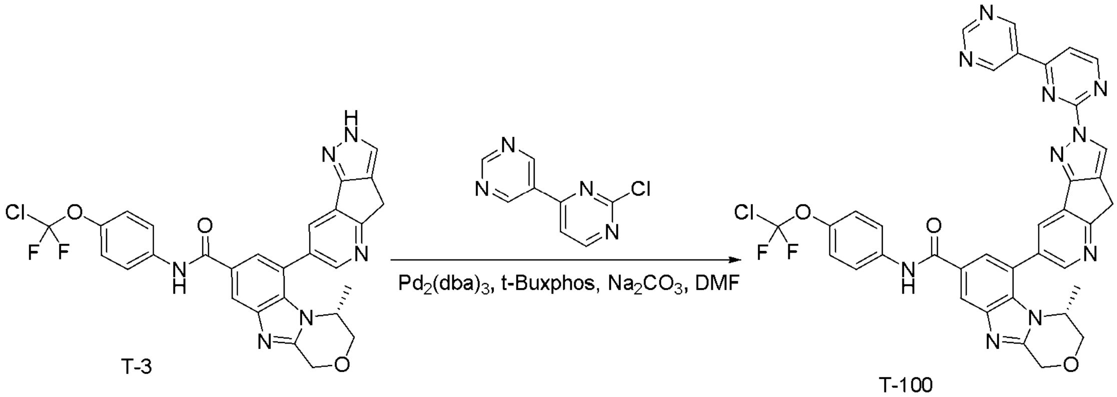


Example 100 was prepared using compound T-3 and 4-(pyrimidin-5-yl)-2-chloropyrimidine as the raw materials according to the method described in example 4, with a yield of 18%. LC-MS (APCI): m/z=719.7(M+1)+. $^1$H NMR (400 MHz, DMSO-$d_6$) δ 10.21 (s, 1H), 8.90 (s, 1H), 8.67 (s, 1H), 8.41 (s, 1H), 8.31 (s, 1H), 8.16 (s, 1H), 8.02-7.87 (m, 2H), 7.66 (d, J = 8.8 Hz, 2H), 7.33 (d, J = 8.6 Hz, 1H), 7.26 (d, J = 8.4 Hz, 2H), 6.82 (d, J = 8.4 Hz, 2H), 5.06 (s, 2H), 4.30 (s, 2H), 4.15 (s, 3H), 3.94 (m, 1H), 3.95-3.76 (m, 2H), 1.64 (d, J = 8.6 Hz, 3H).

**Example 101 Preparation of (R)-N-(4-(chlorodifluoromethoxy)phenyl)-6-(2-(5-isopropyloyrimidin-2-yl)-2,4-dihydropyrazolo[3',4':3,4]cyclooenta[1,2-b]pyridin-7-yl)-4-methyl-3,4-dihydro-1H-benzo[4,5]imidazo[2,1-c][1,4]oxazine-8-carboxamide (compound T-101)**

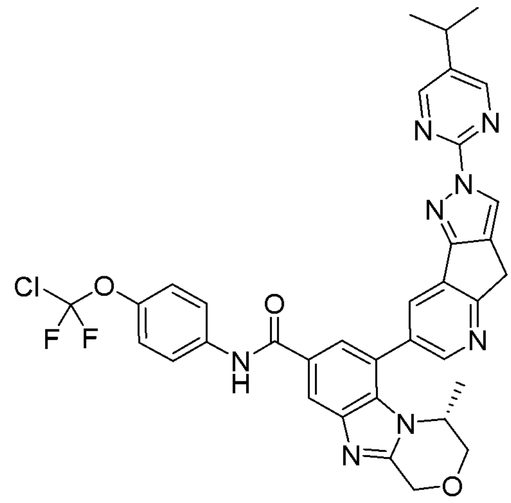

T-101

The following synthetic route was adopted:

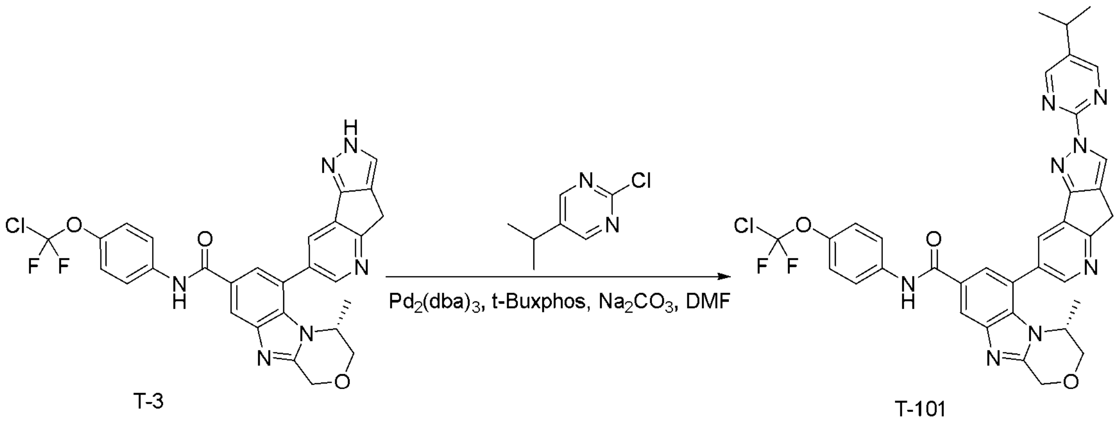


Example 101 was prepared using compound T-3 and 2-chloro-5-isopropylpyrimidine as the raw materials according to the method described in example 4, with a yield of 74%. LC-MS (APCI): m/z=683.4(M+1)$^+$. $^1$H NMR (400 MHz, DMSO-$d_6$) $\delta$ 10.23 (s, 1H), 8.91 (s, 1H), 8.67 (s, 1H), 8.41 (s, 1H), 8.30 (s, 1H), 7.68 (s, 1H), 7.33 (d, J = 8.6 Hz, 1H), 7.26 (d, J = 8.4 Hz, 2H), 6.82 (d, J = 8.4 Hz, 2H), 5.06 (s, 2H), 4.30 (s, 2H), 3.94 (m, 1H), 3.95-3.76 (m, 2H), 2.37 (m, 1H), 1.62 (d, J = 8.6 Hz, 3H), 1.44 (d, J = 6.4 Hz, 6H).

**Example 102 Preparation of (R)-N-(4-(chlorodifluoromethoxy)phenyl)-4-methyl-6-(2-(1-methyl-1H-imidazol-2-yl)-2,4-dihydropyrazolo[3',4':3,4]cyclopenta[1,2-b]pyridin-7-yl)-3,4-dihydro-1H-benzo[4,5]imidazo[2,1-c][1,4] oxazine-8-carboxamide (compound T-102)**

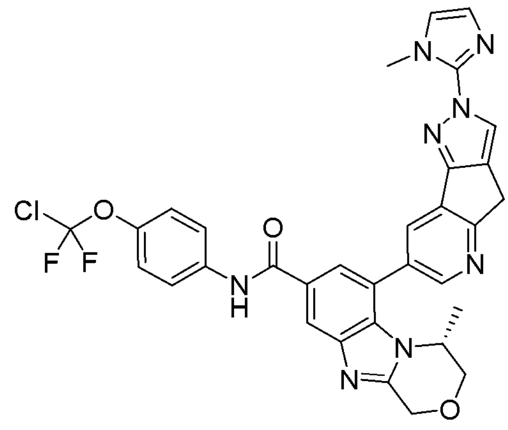

T-102

The following synthetic route was adopted:

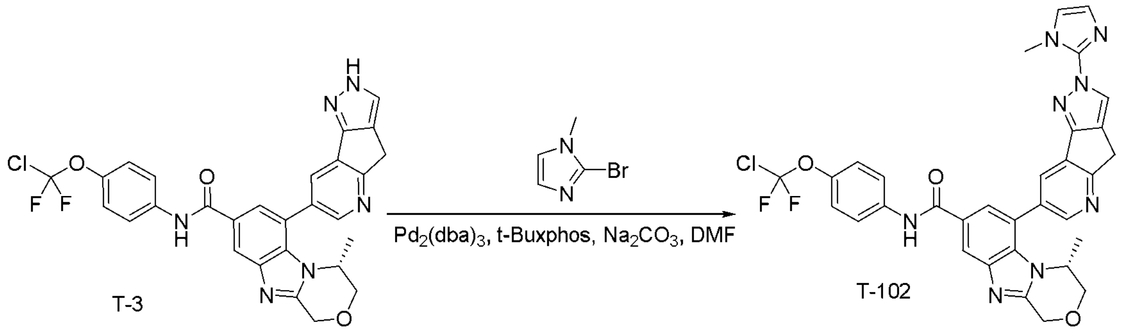


Example 102 was prepared using compound T-3 and 1-methyl-2-bromoimidazole as the raw materials according to the method described in example 4, with a yield of 8%. LC-MS (APCI): m/z=643.5(M+1)$^+$. $^1$H NMR (400 MHz, DMSO-$d_6$) δ 10.22 (s, 1H), 8.92 (s, 1H), 8.67 (s, 1H), 8.41 (s, 1H), 8.30-8.14 (m, 3H), 7.68 (s, 1H), 7.33 (d, J = 8.6 Hz, 1H), 7.26 (d, J = 8.4 Hz, 2H), 6.82 (d, J = 8.4 Hz, 2H), 5.06 (s, 2H), 4.30 (s, 2H), 3.94 (m, 1H), 3.95-3.76 (m, 2H), 3.26 (s, 3H), 1.64 (d, J = 8.6 Hz, 3H).

**Example 103 Preparation of (R)-N-(4-(chlorodifluoromethoxy)phenyl)-6-(2-(6-cyanopyridazin-3-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-103)**

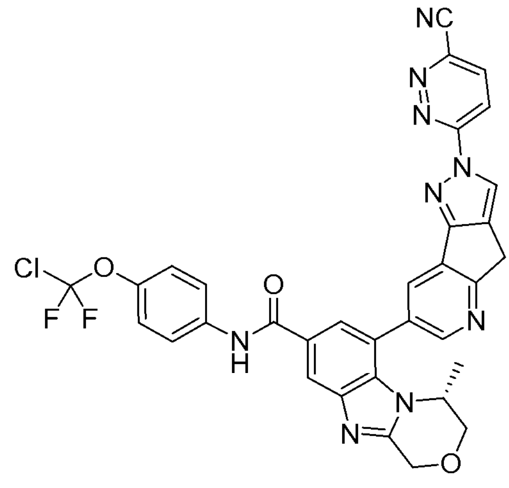

T-103

The following synthetic route was adopted:

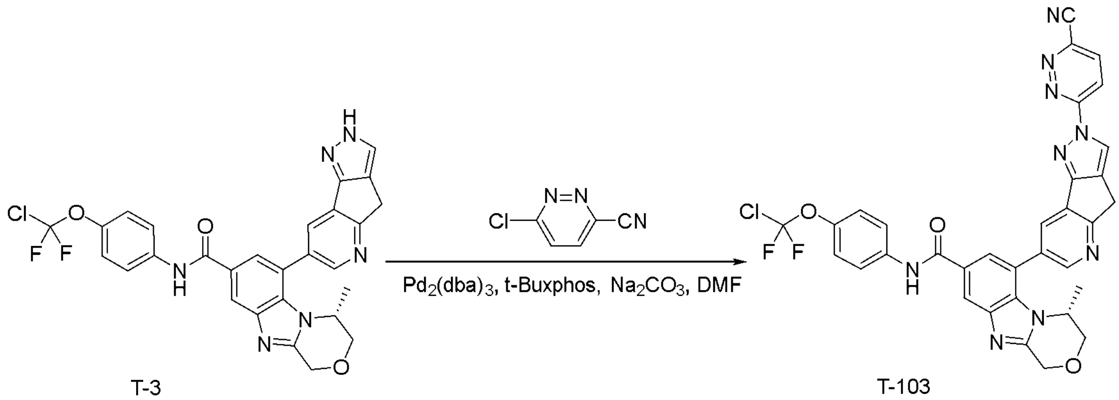


T-3 T-103

Example 103 was prepared using compound T-3 and 3-chloro-6-cyanopyridazine as the raw materials according to the method described in example 4, with a yield of 11%. LC-MS (APCI): m/z=666.4(M+1)+. $^1$H NMR (400 MHz, DMSO-$d_6$) δ 10.23 (s, 1H), 8.93 (s, 1H), 8.67 (s, 1H), 8.41 (s, 1H), 8.30 (s, 1H), 7.68 (s, 1H), 7.33 (d, J = 8.6 Hz, 2H), 7.26 (d, J = 8.4 Hz, 2H), 6.82 (d, J = 8.4 Hz, 2H), 5.06 (s, 2H), 4.30 (s, 2H), 3.94 (m, 1H), 3.91-3.76 (m, 2H), 1.63 (d, J = 8.6 Hz, 3H).

**Example 104 Preparation of (R)-N-(4-(chlorodifluoromethoxy)phenyl)-4-methyl-6-(2-(6-methylpyridazin-3-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-104-A) and (R)-N-(4-(chlorodifluoromethoxy)phenyl)-4-methyl-6-(1-(6-methylpyridazin-3-yl)-1,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide (compound T-104-B)**

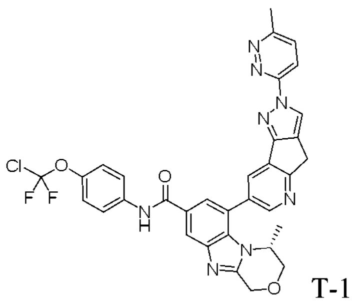

T-104-A and

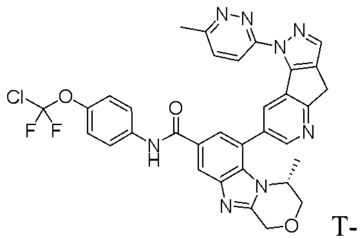

T-104-B

The following synthetic route was adopted:

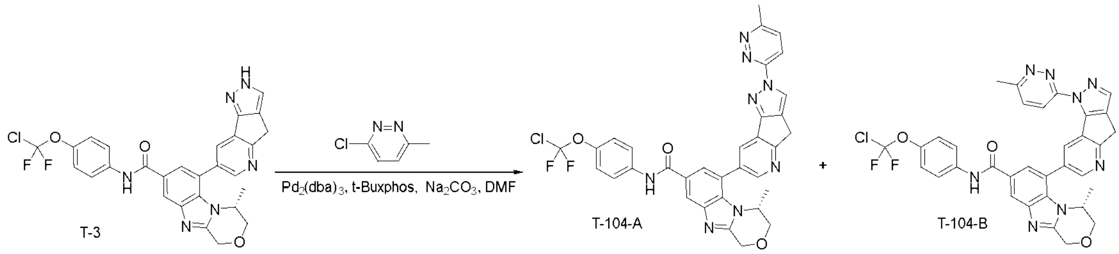


Compounds T-104-A and T-104-B were prepared using compound T-3 and 3-chloro-6-methylpyridazine as the raw materials according to the method described in example 4, with yields of 9% and 27%, respectively. Compound T-104-A: LC-MS (APCI): m/z=655.4(M+1)+. $^1$H NMR (400 MHz, DMSO-$d_6$) δ 10.22 (s, 1H), 8.90 (s, 1H), 8.67 (s, 1H), 8.41 (s, 1H), 8.30 (s, 1H), 7.33 (d, J = 8.6 Hz, 2H), 7.26 (d, J = 8.4 Hz, 2H), 6.82 (d, J = 8.4 Hz, 2H), 5.06 (s, 2H), 4.30 (s, 2H), 3.94 (m, 1H), 3.95-3.76 (m, 2H), 2.33 (s, 3H), 1.62 (d, J = 8.6 Hz, 3H).

Compound T-104-B: LC-MS (APCI): m/z=655.2(M+1)+. $^1$H NMR (400 MHz, DMSO-$d_6$) δ 10.23 (s, 1H), 8.91 (s, 1H), 8.67 (s, 1H), 8.41 (s, 1H), 8.30 (s, 1H), 7.33 (d, J = 8.6 Hz, 2H), 7.26 (d, J = 8.4 Hz, 2H), 6.83 (d, J = 8.4 Hz, 2H), 5.06 (s, 2H), 4.30 (s, 2H), 3.94 (m, 1H), 3.92-3.76 (m, 2H), 2.34 (s, 3H), 1.65 (d, J = 8.6 Hz, 3H).

**Example 105 Preparation of (R)-N-(4-(chlorodifluoromethoxy)phenyl)-4-methyl-6-(2-(pyrimidin-2-yl)-2,4-dihydropyrazolo[3',4':3,4]cyclopenta[1,2-b]pyridin-7-yl-4,4-*d*$_2$)-3,4-dihydro-1H-benzo[4,5]imidazo[2,1-c][1,4]oxazine-8-carboxamide (compound T-105)**

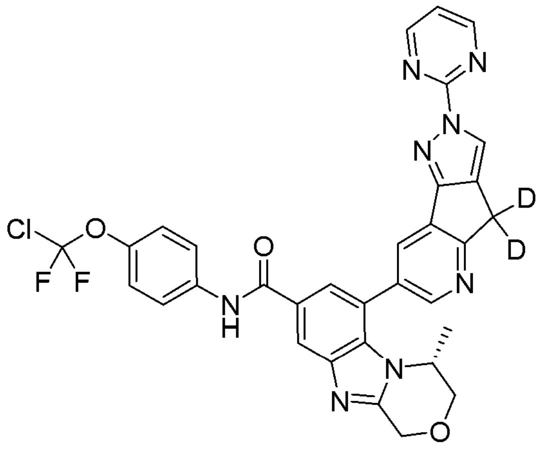

T-105

The following synthetic route was adopted:

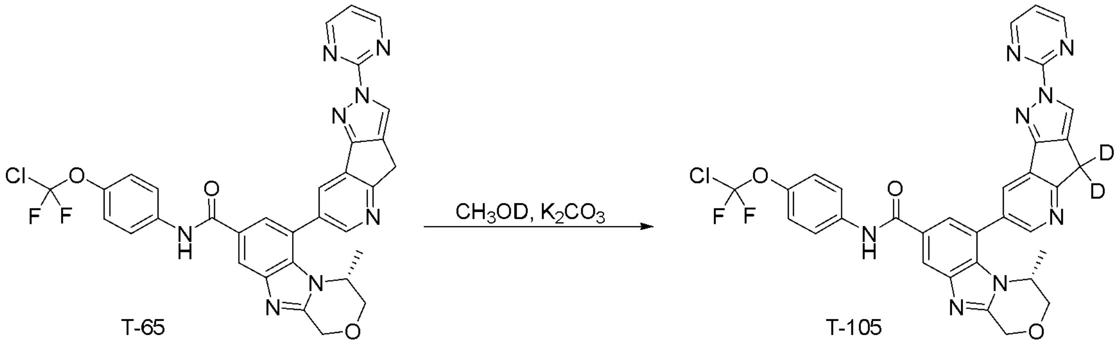


Compound T-65 (100 mg, 0.16 mmol), potassium carbonate (22 mg, 0.32 mmol) and deuterated methanol (5 mL) were added to a reaction flask. The mixture was stirred and reacted at room temperature overnight. The conversion was completed under the in-process control by LC-MS. The mixture was concentrated to remove the solvent. The residue was purified by silica gel column chromatography to give 71 mg of an off-white solid, with a yield of 71%. LC-MS (APCI): m/z=643.3 $(M+1)^+$. $^1$H NMR (400 MHz, DMSO-*d*$_6$) δ 10.23 (s, 1H), 8.92 (s, 1H), 8.67 (s, 1H), 8.41 (s, 1H), 8.30 (s, 1H), 7.85-7.47 (m, 3H), 7.33 (d, J = 8.6 Hz, 1H), 7.26 (d, J = 8.4 Hz, 2H), 6.82 (d, J = 8.4 Hz, 2H), 5.01 (s, 2H), 4.31 (s, 2H), 3.94 (m, 1H), 3.92-3.76 (m, 2H), 1.63 (d, J = 8.6 Hz, 3H).

**Example 106 Preparation of (4R)-N-(4-(chlorodifluoromethoxy)phenyl)-6-(4-hydroxy-2-(pyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-106)**

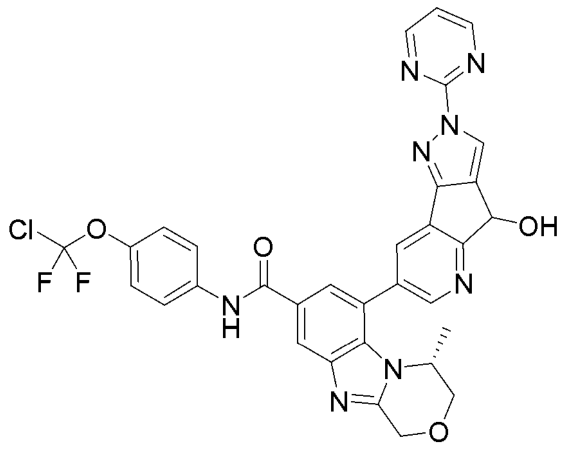

T-106

The following synthetic route was adopted:

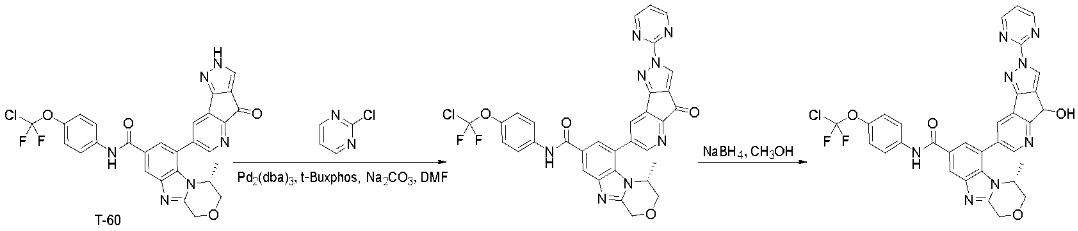


T-60

T-106

Step 1: Synthesis of compound (R)-N-(4-(chlorodifluoromethoxy)phenyl)-4-methyl-6-(4-oxo-2-(pyrimidin-2-yl)-2,4-dihydropyrazolo[3',4':3,4]cyclopenta[1,2-b]pyridin-7-yl)-3,4-dihydro-1H-benzo[4,5]imidazo[2,1-c][1,4]oxazine-8-carboxamide

Compound T-60 (104 mg, 0.18 mmol), 2-chloropyrimidine (61 mg, 0.54 mmol), tris(dibenzylideneacetone) dipalladium(0) (18 mg, 0.02 mmol), 2-di-tert-butylphosphino-2',4',6'-triisopropylbiphenyl (17 mg, 0.04 mmol), sodium carbonate (57 mg, 0.54 mmol) and anhydrous DMF (3 mL) were added to a reaction flask. The mixture was purged with nitrogen for 5 minutes. The mixture was microwaved and heated to 90 °C, and reacted for 3 hours. The reaction was monitored by TLC until it was completed. The resulting mixture was concentrated, and purified by silica gel column chromatography to give 75 mg of a product, with a yield of 64%. LC-MS(APCI): m/z=655.4 $(M+1)^+$.

Step 2: Synthesis of compound T-106

(R)-N-(4-(Chlorodifluoromethoxy)phenyl)-4-methyl-6-(4-oxo-2-(pyrimidin-2-yl)-2,4-dihydropyrazolo[3',4':3,4] cyclopenta[1,2-b]pyridin-7-yl)-3,4-dihydro-1H-benzo[4,5]imidazo[2,1-c][1,4]oxazine-8-carboxamide (75 mg, 0.11 mmol) was dissolved in 5 mL of methanol, and sodium borohydride (8.5 mg, 0.22 mmol) was added. The mixture was stirred and reacted at room temperature for 1 hour. The reaction was monitored by TLC until it was completed. The mixture was concentrated to remove the solvent. The residue was purified by silica gel column chromatography to give 32 mg of an off-white solid, with a yield of 45%. LC-MS (APCI): m/z=657.3 $(M+1)^+$. $^1H$ NMR (400 MHz, DMSO-$d_6$) δ 10.22 (s, 1H), 8.91 (s, 1H), 8.67 (s, 1H), 8.41 (s, 1H), 8.30 (s, 1H), 7.85-7.47 (m, 3H), 7.33 (d, J = 8.6 Hz, 1H), 7.26 (d, J = 8.4 Hz, 2H), 6.82 (d, J = 8.4 Hz, 2H), 6.01 (s, 1H), 4.30 (s, 2H), 3.94 (m, 1H), 3.95-3.76 (m, 2H), 1.63 (d, J = 8.6 Hz, 3H).

**Example 107 Preparation of (4R)-N-(4-(chlorodifluoromethoxy)phenyl)-6-(4-fluoro-2-(pyrimidin-2-yl)-2,4-dihydropyrazolo[3',4':3,4]cyclopenta[1,2-b]pyridin-7-yl)-4-methyl-3,4-dihydro-1H-benzo[4,5]imidazo[2,1-c][1,4]oxazine-8-carboxamide (compound T-107)**

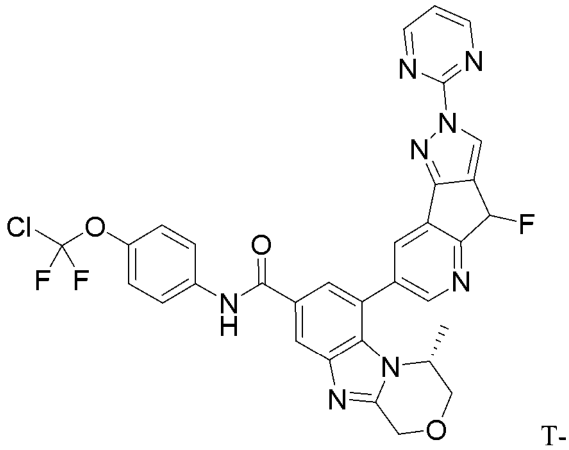

T-107

The following synthetic route was adopted:

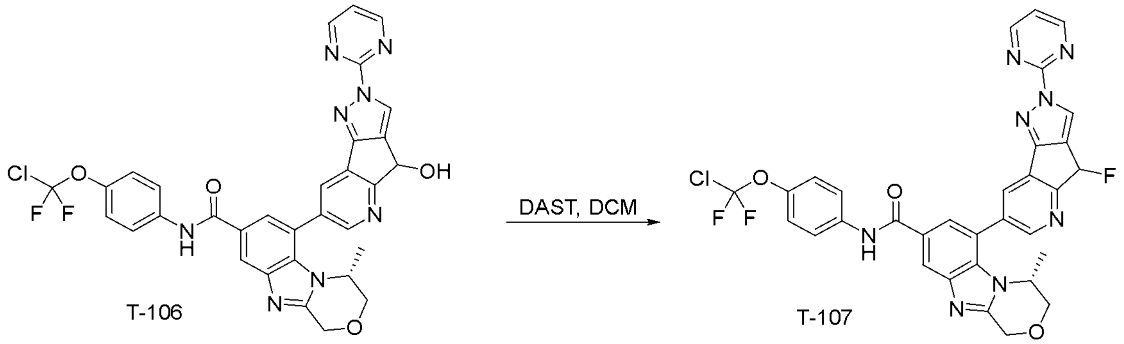


T-106 (80 mg, 0.12 mmol) was dissolved in 5 mL of anhydrous dichloromethane, and the mixture was cooled to -78 °C. DAST (30 mg, 0.18 mmol) was added under nitrogen. The resulting mixture was stirred and reacted at a low temperature for 3 to 4 hours. The reaction was monitored by TLC until it was completed. 5 mL of water was added to quench the reaction. The organic phase was separated, concentrated, and then purified by silica gel column chromatography to give 31 mg of an off-white solid, with a yield of 39%. LC-MS (APCI): m/z=659.4 $(M+1)^+$. $^1H$ NMR (400 MHz, DMSO-$d_6$) δ 10.23 (s, 1H), 8.92 (s, 1H), 8.67 (s, 1H), 8.41 (s, 1H), 8.30 (s, 1H), 7.85-7.47 (m, 3H), 7.33 (d, J = 8.6 Hz, 1H), 7.26 (d, J = 8.4 Hz, 2H), 6.82 (d, J = 8.4 Hz, 2H), 6.33 (d, J = 12.2 Hz, 1H), 4.31 (s, 2H), 3.94 (m, 1H), 3.95-3.76 (m, 2H), 1.63 (d, J = 8.6 Hz, 3H).

**Example 108 Preparation of (R)-N-(4-(chlorodifluoromethoxy)phenyl)-6-(2,4-dihydroindeno[1,2-c]pyrazol-7-yl)-4-methyl-3,4-dihydro-1H-benzo[4,5]imidazo[2,1-c][1,4]xxazine-8-carboxamide (compound T-108)**

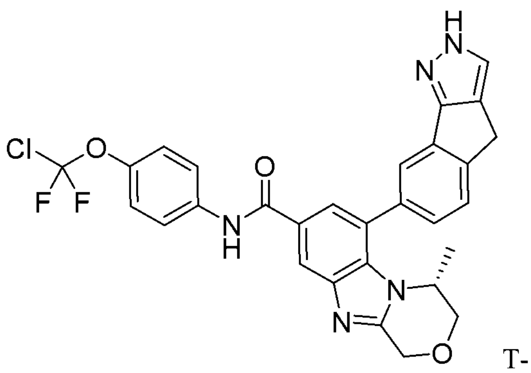

T-108

The following synthetic route was adopted:

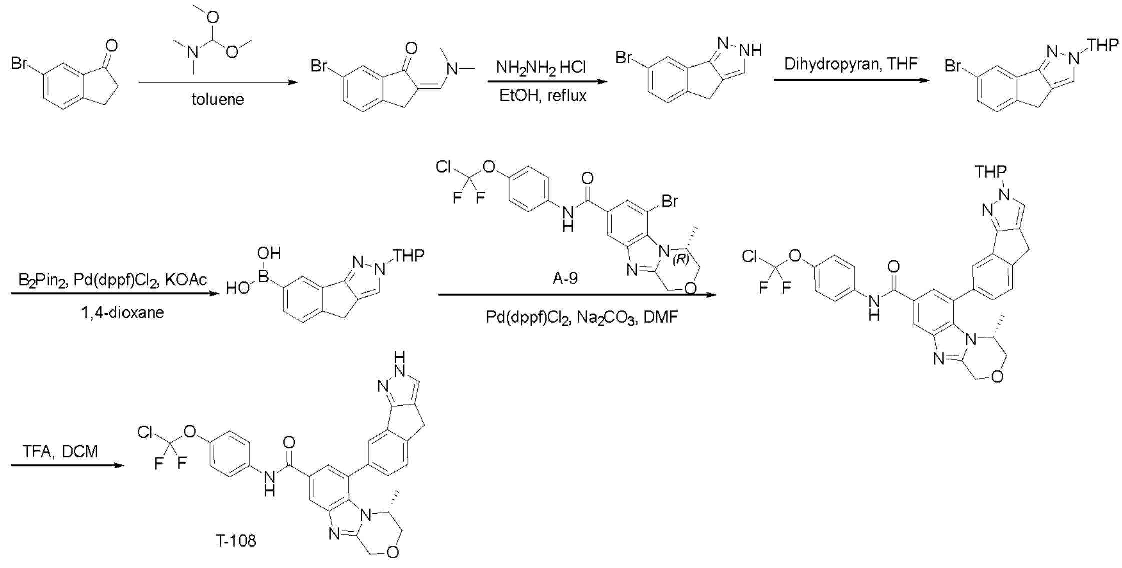

T-108

Step 1: Synthesis of compound (Z)-6-bromo-2-((dimethylamino)methylene)-2,3-dihydro-1H-inden-1-one

6-Bromo-2,3-dihydro-1H-inden-1-one (1.1 g, 5.2 mmol) and N,N-dimethylformamide dimethyl acetal (930 mg, 7.8 mmol) were dissolved in 25 mL of anhydrous toluene. The mixture was heated to 110 °C, stirred and reacted overnight. The reaction was monitored by TLC until it was completed. The mixture was concentrated to remove the solvent. The residue was purified by silica gel column chromatography to give 1.17 g of a light yellow solid, with a yield of 85%. LC-MS (APCI): m/z=266.4 $(M+1)^+$.

Step 2: Synthesis of compound 7-bromo-2,4-dihydroindeno[1,2-c]pyrazole

(Z)-6-Bromo-2-((dimethylamino)methylene)-2,3-dihydro-1H-inden-1-one (1.17 g, 4.4 mmol) and hydrazine hydrochloride (603 mg, 8.8 mmol) were dissolved in 20 mL of anhydrous ethanol. The mixture was heated to 70 °C, stirred and reacted under nitrogen for 12 hours. The reaction was monitored by TLC until it was completed. The mixture was concentrated to remove the solvent. The residue was purified by silica gel column chromatography to give 0.86 g of a light yellow solid, with a yield of 78%. LC-MS (APCI): m/z=235.2 $(M+1)^+$.

Step 3: Synthesis of compound 7-bromo-2-(tetrahydro-2H-pyran-2-yl)-2,4-dihydroindeno[1,2-c]pyrazole

7-Bromo-2,4-dihydroindeno[1,2-c]pyrazole (860 mg, 3.66 mmol) and dihydropyran (615 mg, 7.32 mmol) were dissolved in 10 mL of anhydrous tetrahydrofuran. The mixture was stirred and reacted at room temperature for 2 to 3 hours. The reaction was monitored by TLC until it was completed. The mixture was concentrated to remove the solvent. The residue was purified by silica gel column chromatography to give 1.12 g of a light yellow solid, with a yield of 96%. LC-MS (APCI): m/z=319.4 $(M+1)^+$.

Step 4: Synthesis of compound (2-(tetrahydro-2H-pyran-2-yl)-2,4-dihydroindeno[1,2-c]pyrazol-7-yl)boronic acid

7-Bromo-2-(tetrahydro-2H-pyran-2-yl)-2,4-dihydroindeno[1,2-c]pyrazole (0.99 g, 3.1 mmol), bis(pinacolato) diboron(2.4 g, 9.3 mmol), [1,1'-bis(diphenylphosphino)ferrocene]dichloropalladium(II) (117 mg, 0.16 mmol), potassium acetate (0.6 g, 6.2 mmol) and anhydrous 1,4-dioxane (10 mL) were added to a reaction flask. The mixture was microwaved and heated to 100 °C, and reacted for 2 hours. The reaction was monitored by TLC until it was completed. The reaction solution was concentrated, and then purified by silica gel column chromatography to give 742 mg of product, with a yield of 84%. LC-MS (APCI): m/z=285.7 $(M+1)^+$.

Step 5: Synthesis of compound (4R)-N-(4-(chlorodifluoromethoxy)phenyl)-4-methyl-6-(2-(tetrahydro-2H-pyran-2-yl)-2,4-dihydroindeno[1,2-c]pyrazol-7-yl)-3,4-dihydro-1H-benzo[4,5]imidazo[2,1-c][1,4]oxazine-8-carboxamide

Intermediate compound A-9 (189 mg, 0.39 mmol), 2-(tetrahydro-2H-pyran-2-yl)-2,4-dihydroindeno[1,2-c]pyrazol-7-yl)boronic acid (167 mg, 0.588 mmol), [1,1'-bis(diphenylphosphino)ferrocene]dichloropalladium(II) (32 mg, 0.02 mmol), sodium carbonate (125 mg, 1.18 mmol) and anhydrous DMF (5 mL) were added to a reaction flask. The mixture

was purged with nitrogen for 5 minutes. The mixture was microwaved and heated to 90 °C, and reacted for 1 hour. The reaction was monitored by TLC until it was completed. The resulting mixture was concentrated, and purified by silica gel column chromatography to give 110 mg of product, with a yield of 44%. LC-MS(APCI): m/z=646.7 $(M+1)^+$.

Step 6: Synthesis of compound T-108

(4R)-N-(4-(Chlorodifluoromethoxy)phenyl)-4-methyl-6-(2-(tetrahydro-2H-pyran-2-yl)-2,4-dihydroindeno[1,2-c] pyrazol-7-yl)-3,4-dihydro-1H-benzo[4,5]imidazo[2,1-c][1,4]oxazine-8-carboxamide (110 mg, 0.17 mmol) was dissolved in 5 mL of anhydrous dichloromethane, and 1 mL of trifluoroacetic acid was added. The mixture was stirred and reacted at room temperature for 1 to 2 hours. The reaction was monitored by TLC until it was completed. The mixture was concentrated to remove the solvent, and dichloromethane was added. The resulting mixture was washed with saturated aqueous solution of sodium bicarbonate three times. The organic phase was concentrated, and then purified by silica gel column chromatography to give 68 mg of a white solid, with a yield of 71%. LC-MS(APCI): m/z=562.1 $(M+1)^+$. 1H NMR (400 MHz, DMSO-$d_6$) δ 10.23 (s, 1H), 8.92 (s, 1H), 8.67 (s, 1H), 8.41 (s, 1H), 8.30 (s, 1H), 7.47-7.38 (m, 3H), 7.33 (d, J = 8.6 Hz, 1H), 7.26 (d, J = 8.4 Hz, 2H), 6.82 (d, J = 8.4 Hz, 2H), 4.31 (s, 2H), 4.15 (s, 2H), 3.94 (m, 1H), 3.95-3.76 (m, 2H), 1.63 (d, J = 8.6 Hz, 3H).

**Example 109 Preparation of (R)-N-(4-(chlorodifluoromethoxy)phenyl)-4-methyl-6-(2-(pyrimidin-2-yl)-2,4-dihydroindeno[1,2-c]pyrazol-7-yl)-3,4-dihydro-1H-benzo[4,5]imidazo[2,1-cl [1,4]oxazine-8-carboxamide (compound T-109)**

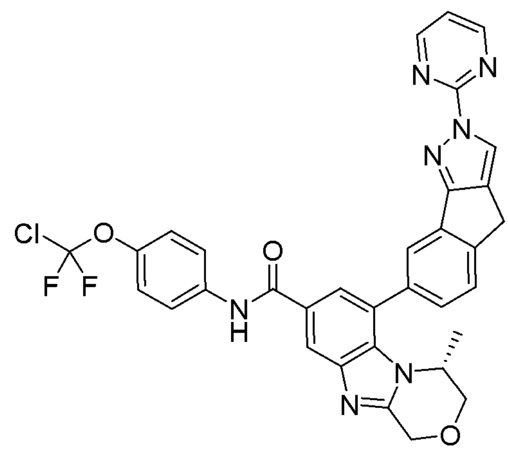

T-109

The following synthetic route was adopted:

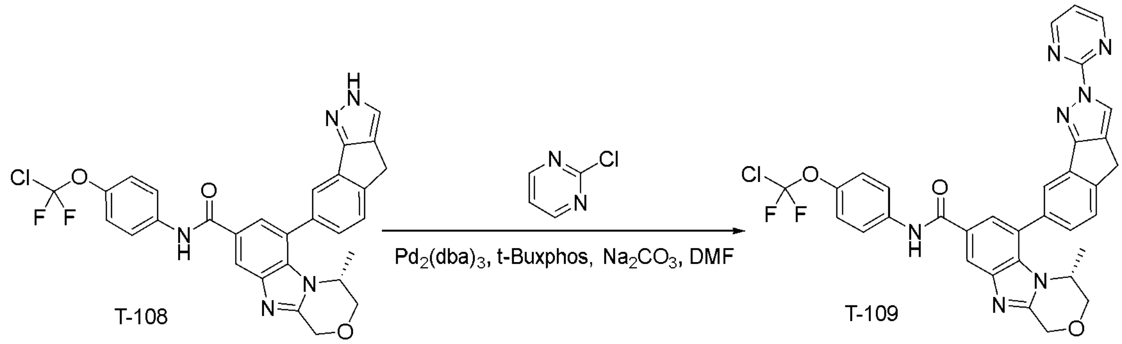


Example T-109 was prepared using compound T-108 and 2-chloropyrimidine as the raw materials according to the method described in example 4, with a yield of 66%. LC-MS (APCI): m/z=640.4$(M+1)^+$. $^1$H NMR (400 MHz, DMSO-$d_6$) δ 10.23 (s, 1H), 8.92 (s, 1H), 8.67 (s, 1H), 8.41 (s, 1H), 8.30 (s, 1H), 7.85-7.40 (m, 4H), 7.33 (d, J = 2.5 Hz, 2H), 7.26 (d, J = 8.4 Hz, 2H), 6.82 (d, J = 8.4 Hz, 2H), 4.31 (s, 2H), 4.18 (s, 2H), 3.94 (m, 1H), 3.95-3.76 (m, 2H), 1.64 (d, J = 8.6 Hz, 3H).

**Example 110 Preparation of (R)-N-(4-(chlorodifluoromethoxy)phenyl)-6-(2-isopropyl-2,4-dihydroindeno[1,2-c]pyrazol-7-yl)-4-methyl-3,4-dihydro-1H-benzo[4,5]imidazo[2,1-c][1,4]oxazine-8-carboxamide (compound T-110)**

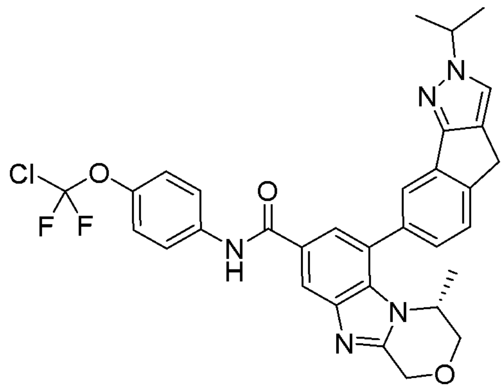

T-110

The following synthetic route was adopted:

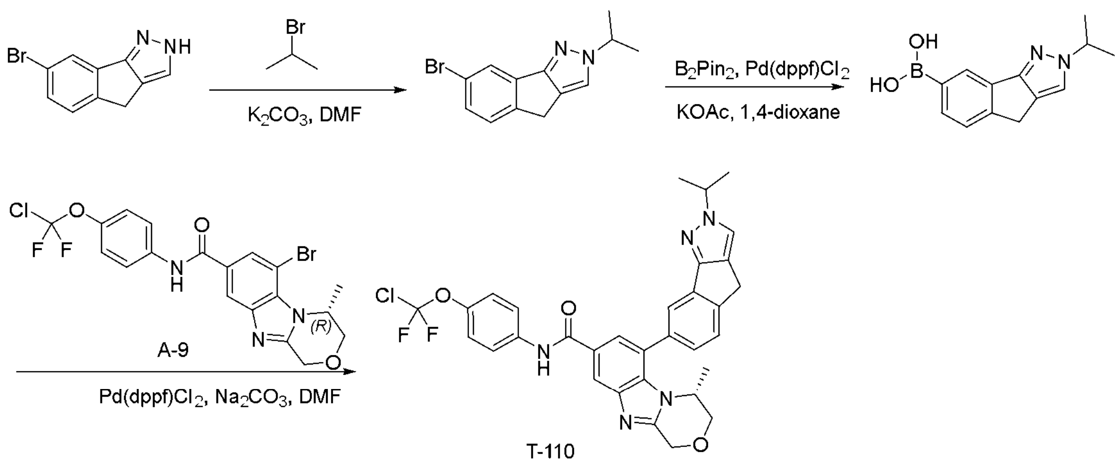


T-110

Step 1: Synthesis of compound 7-bromo-2-isopropyl-2,4-dihydroindeno[1,2-c]pyrazole

7-Bromo-2,4-dihydroindeno[1,2-c]pyrazole (2.34 g, 10 mmol), 2-bromopropane (1.83 g, 15 mmol) and potassium carbonate (2.76 g, 20 mmol) were dissolved in 15 mL of anhydrous DMF. The mixture was heated to 50 °C, stirred and reacted overnight. The reaction was monitored by TLC until it was completed. The mixture was diluted with added water. The resulting mixture was extracted with ethyl acetate 3 to 4 times. The organic phases were combined, washed with saturated brine, concentrated, and then purified by silica gel column chromatography to give 1.87 g of an off-white solid, with a yield of 68%. LC-MS (APCI): m/z=277.3 $(M+1)^+$.

Step 2: Synthesis of compound (2-isopropyl-2,4-dihydroindeno[1,2-c]pyrazol-7-yl)boronic acid

7-Bromo-2-isopropyl-2,4-dihydroindeno[1,2-c]pyrazole (0.86 g, 3.1 mmol), bis(pinacolato)diboron (2.4 g, 9.3 mmol), [1,1'-bis(diphenylphosphino)ferrocene]dichloropalladium(II) (117 mg, 0.16 mmol) and potassium acetate (0.6 g, 6.2 mmol) were dissolved in 10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 427 mg of product, with a yield of 57%. LC-MS (APCI): m/z=243.5 $(M+1)^+$.

Step 3: Synthesis of compound T-110

Intermediate compound A-9 (189 mg, 0.39 mmol), (2-isopropyl-2,4-dihydroindeno[1,2-c]pyrazol-7-yl)boronic

acid (142 mg, 0.588 mmol), [1,1'-bis(diphenylphosphino)ferrocene]dichloropalladium(II) (32 mg, 0.02 mmol), sodium carbonate (125 mg, 1.18 mmol) and anhydrous DMF (5 mL) were added to a reaction flask. The mixture was purged with nitrogen for 5 minutes. The mixture was microwaved and heated to 90 °C, and reacted for 1 hour. The reaction was monitored by TLC until it was completed. Then, the resulting mixture was concentrated, and purified by silica gel column chromatography to give 80 mg of product, with a yield of 34%. LC-MS (APCI): m/z=604.7 $(M+1)^+$. $^1H$ NMR (400 MHz, DMSO-$d_6$) δ 10.23 (s, 1H), 8.92 (s, 1H), 8.67 (s, 1H), 8.41 (s, 1H), 8.30 (s, 1H), 7.32 (d, J = 2.5 Hz, 2H), 7.26 (d, J = 8.4 Hz, 2H), 6.82 (d, J = 8.4 Hz, 2H), 4.31 (s, 2H), 4.18 (s, 2H), 3.94 (m, 1H), 3.95-3.76 (m, 2H), 3.55 (m ,1H), 1.64 (d, J = 8.6 Hz, 3H), 1.47 (d, J = 4.5 Hz, 6H).

**Example 111 Preparation of (R)-6-(2-amino-8H-indeno[1,2-d]thiazol-5-yl)-N-(4-(chlorodifluoromethoxy)phenyl)-4-methyl-3,4-dihydro-1H-benzo[4,5]imidazo[2,1-c][1,4]oxazine-8-carboxamide (compound T-111)**

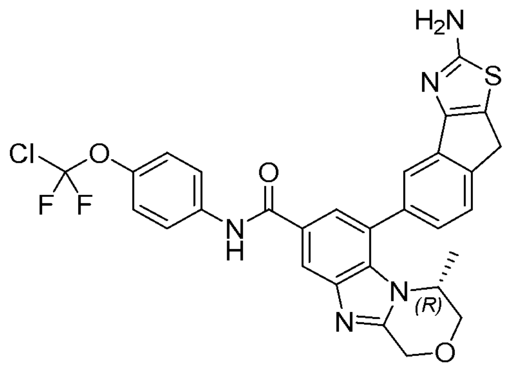

T-111

The following synthetic route was adopted:

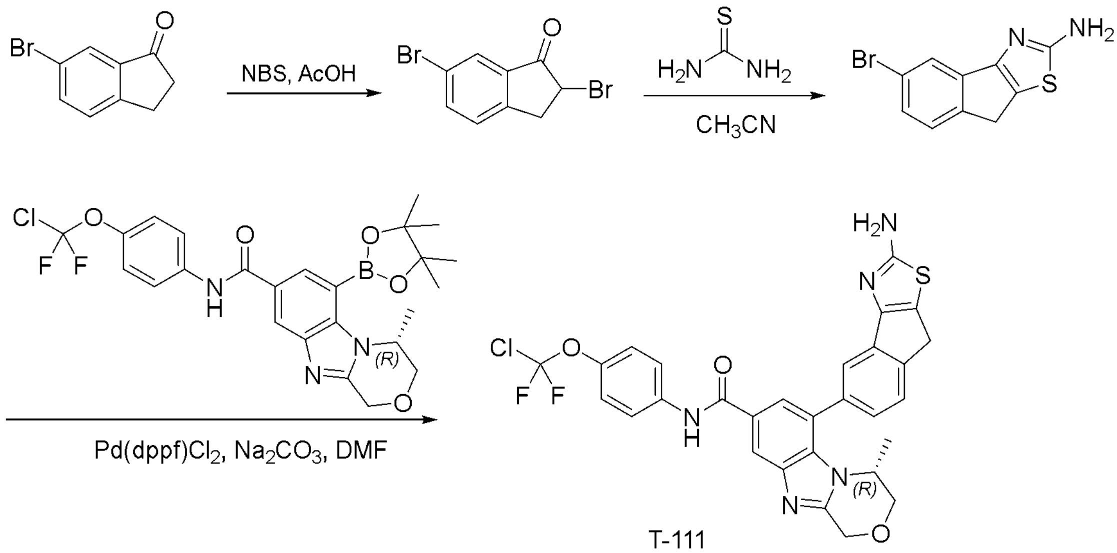


T-111

Step 1: Synthesis of compound 2,6-dibromo-2,3-dihydro-1H-inden-1-one

6-Bromo-2,3-dihydro-1H-inden-1-one (2.1 g, 10 mmol) was dissolved in 10 mL of acetic acid. NBS (1.87 g, 10.5 mmol) was added in batches in an ice bath. After the addition was completed, the mixture was warmed to room temperature, stirred and reacted for 2 to 4 hours. The reaction was monitored by TLC until it was completed. Water was added to quench the reaction. The resulting mixture was extracted with dichloromethane 3 to 4 times. The organic phases were combined, washed successively with saturated aqueous solution of sodium bicarbonate and saturated brine, concentrated, and then purified by silica gel column chromatography to give 1.81 g of a light yellow oily liquid, with a yield of 63%. LC-MS (APCI): m/z=289.3 $(M+1)^+$.

Step 2: Synthesis of compound 5-bromo-8H-indeno[1,2-d]thiazol-2-amine

2,6-Dibromo-2,3-dihydro-1H-inden-1-one (1.81 g, 6.3 mmol), thiourea (0.72 g, 9.4 mmol) and acetonitrile (10 mL) were added to a reaction flask. The mixture was heated to 70 °C, stirred and reacted overnight. The reaction was monitored by TLC until it was completed. The mixture was concentrated to remove the solvent. The residue was purified by silica gel column chromatography to give 637 mg of a yellow solid, with a yield of 38%. LC-MS (APCI): m/z=267.2 $(M+1)^+$.

Step 3: Synthesis of compound T-111

(R)-N-(4-(Chlorodifluoromethoxy)phenyl)-4-methyl-6-(4,4,5,5-tetramethyl-1,3,2-dioxaborolan-2-yl)-3,4-dihydro-1H-benzo[4,5]imidazo[2,1-c][1,4]oxazine-8-carboxamide (208 mg, 0.39 mmol), 5-bromo-8H-indeno[1,2-d]thiazol-2-amine (156 mg, 0.588 mmol), [1,1'-bis(diphenylphosphino)ferrocene]dichloropalladium(II) (32 mg, 0.02 mmol), sodium carbonate (125 mg, 1.18 mmol) and anhydrous DMF (5 mL) were added to a reaction flask. The mixture was purged with nitrogen for 5 minutes. The mixture was microwaved and heated to 90 °C, and reacted for 1 hour. The reaction was monitored by TLC until it was completed. The resulting mixture was concentrated, and purified by silica gel column chromatography to give 46 mg of product, with a yield of 20%. LC-MS (APCI): m/z=594.7 $(M+1)^+$. $^1H$ NMR (400 MHz, DMSO-$d_6$) δ 10.23 (s, 1H), 8.93 (s, 1H), 8.67 (s, 1H), 8.42 (s, 1H), 8.30-8.14 (m, 2H), 7.26 (d, J = 8.4 Hz, 2H), 6.82 (d, J = 8.4 Hz, 2H), 4.31 (s, 2H), 3.66 (s, 2H), 3.93-3.76 (m, 2H), 3.55 (m, 1H), 1.63 (d, J = 8.6 Hz, 3H).

**Example 112 Preparation of (R)-6-(2-acetamido-8H-indeno[1,2-d]thiazol-5-yl)-N-(4-(chlorodifluoromethoxy) phenyl)-4-methyl-3,4-dihydro-1H-benzo[4,5]imidazo[2,1-c][1,4]oxazine-8-carboxamide (compound T-112)**

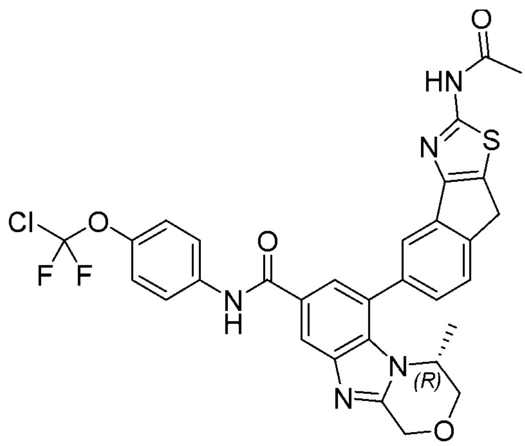

T-112

The following synthetic route was adopted:

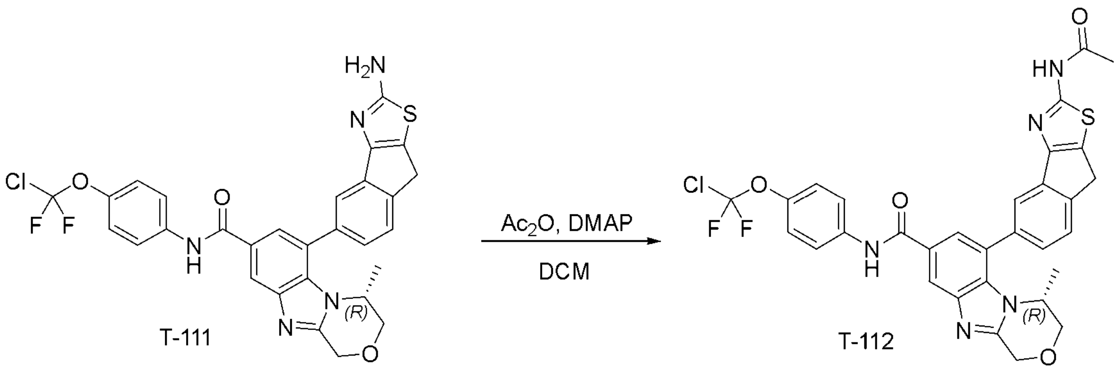


Compound T-111 (80 mg, 0.13 mmol) was dissolved in anhydrous dichloromethane (5 mL). Acetic anhydride (20 mg, 0.19 mmol) was added in an ice bath, and then a catalytic amount of DMAP was added. The mixture was stirred and reacted at room temperature for 2 to 4 hours. The reaction was monitored by TLC until it was completed. Water was added to quench the reaction. The organic phase was separated, concentrated, and then purified by silica gel column

chromatography to give 59 mg of a target product, with a yield of 72%. LC-MS (APCI): m/z=636.7 $(M+1)^+$. $^1H$ NMR (400 MHz, DMSO-$d_6$) δ 10.22 (s, 1H), 8.91 (s, 1H), 8.67 (s, 1H), 8.43 (s, 1H), 8.30-8.10 (m, 2H), 7.26 (d, J = 8.4 Hz, 2H), 6.82 (d, J = 8.4 Hz, 2H), 4.31 (s, 2H), 3.66 (s, 2H), 3.93-3.76 (m, 2H), 3.55 (m, 1H), 2.55 (s, 3H), 1.61 (d, J = 8.6 Hz, 3H).

**Example 113 Preparation of (R)-N-(4-(chlorodifluoromethoxy)phenyl)-6-(8H-indeno[1,2-d]thiazol-5-yl)-4-methyl-3,4-dihydro-1H-benzo[4,5]imidazo[2,1-c][1,4]oxazine-8-carboxamide (compound T-113)**

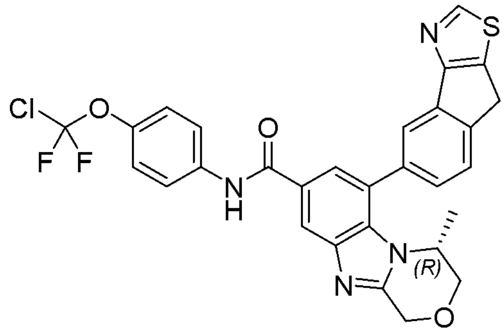

T-113

The following synthetic route was adopted:

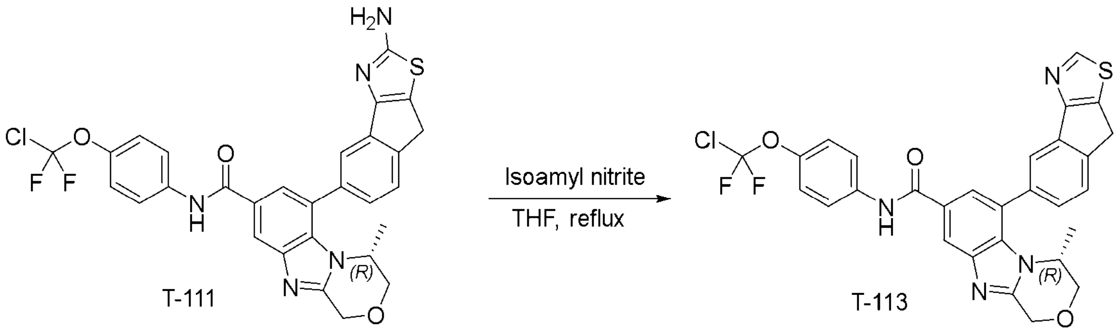


T-111

T-113

Compound T-111 (100 mg, 0.17 mmol) and isoamyl nitrite (40 mg, 0.34 mmol) were dissolved in 5 mL of anhydrous THF. The mixture was heated to reflux, stirred and reacted under nitrogen overnight. The reaction was monitored by TLC until it was completed. The mixture was concentrated to remove the solvent. The residue was purified by silica gel column chromatography to give 27.5 mg of a target product, with a yield of 28%. LC-MS (APCI): m/z=579.8 $(M+1)^+$. $^1H$ NMR (400 MHz, DMSO-$d_6$) δ 10.23 (s, 1H), 8.91 (s, 1H), 8.64 (s, 1H), 8.41 (s, 1H), 8.30-8.14 (m, 2H), 7.26 (d, J = 8.4 Hz, 2H), 6.82 (d, J = 8.4 Hz, 2H), 4.31 (s, 2H), 3.66 (s, 2H), 3.93-3.76 (m, 2H), 3.55 (m, 1H), 1.64 (d, J = 8.6 Hz, 3H).

**Example 114 Preparation of (R)-6-(2-amino-4H-thiazolo[4',5':3,4]cyclopenta[1,2-b]pyridin-7-yl)-N-(4-(chlorodifluoromethoxy)phenyl)-4-methyl-3,4-dihydro-1H-benzo[4,5]imidazo[2,1-c][1,4]oxazine-8-carboxamide (compound T-114)**

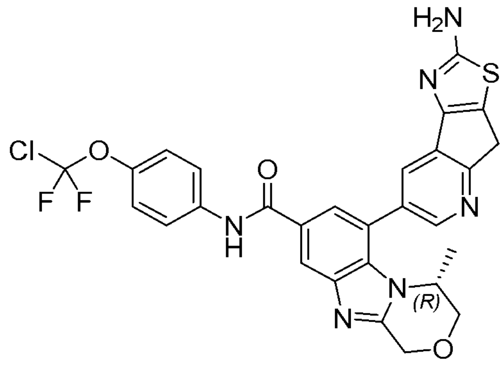

T-114

The following synthetic route was adopted:

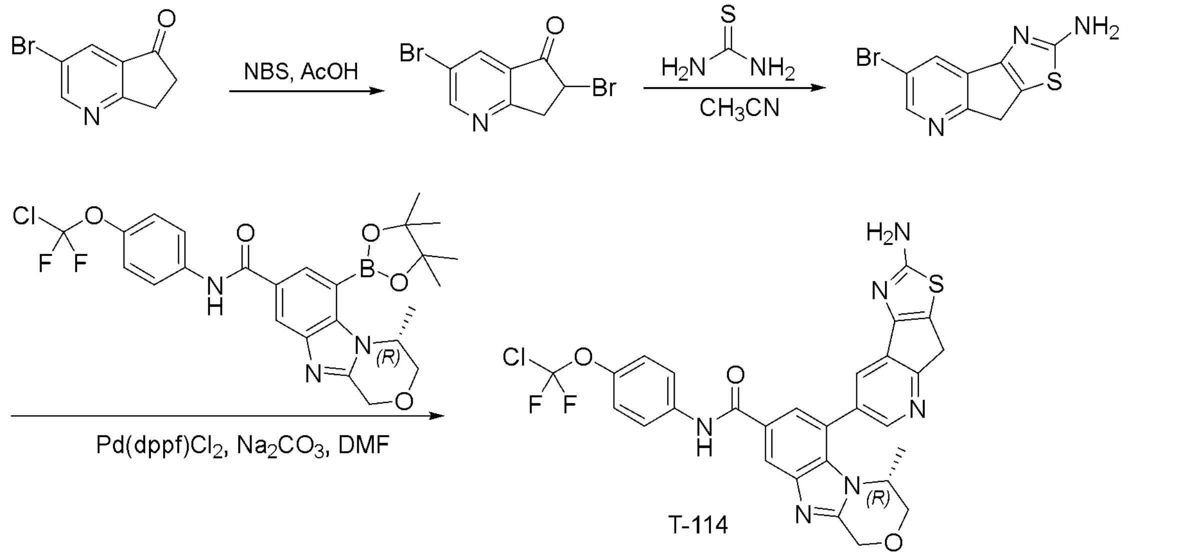


Compound T-114 was prepared using 3-bromo-6,7-dihydro-5H-cyclopenta[b]pyridin-5-one as the raw material via a three-step reaction according to the synthetic method of compound T-111, with an overall yield of 18%. LC-MS (APCI): m/z=595.1 $(M+1)^+$. $^1$H NMR (400 MHz, DMSO-$d_6$) δ 10.22 (s, 1H), 8.93 (s, 1H), 8.67 (s, 1H), 8.42 (s, 1H), 8.14 (s, 1H), 7.26 (d, J = 8.3 Hz, 2H), 6.82 (d, J = 8.4 Hz, 2H), 4.31 (s, 2H), 3.66 (s, 2H), 3.93-3.76 (m, 2H), 3.52 (m, 1H), 1.64 (d, J = 8.6 Hz, 3H).

**Example 115 Preparation of (R)-N-(4-(chlorodifluoromethoxy)phenyl)-4-methyl-6-(pyrazin-2-yl)-3,4-dihydro-1H-benzo[4,5]imidazo[2,1-c][1,4]oxazine-8-carboxamide (compound T-115)**

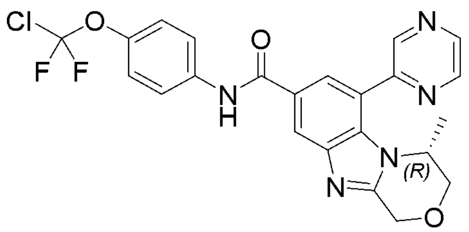

T-115

The following synthetic route was adopted:

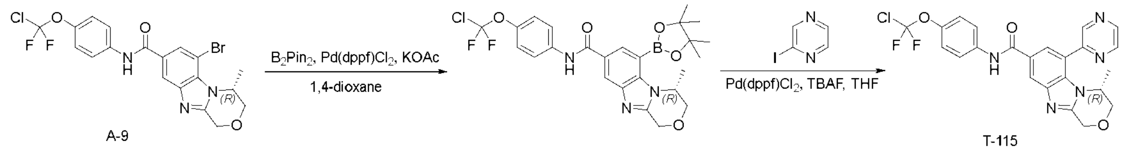


Step 1: Synthesis of compound (R)-N-(4-(chlorodifluoromethoxy)phenyl)-4-methyl-6-(4,4,5,5-tetramethyl-1,3,2-dioxaborolan-2-yl)-3,4-dihydro-1H-benzo[4,5]imidazo[2,1-c][1,4]oxazine-8-carboxamide

Intermediate compound A-9 (1.5 g, 3.1 mmol), bis(pinacolato)diboron (2.4 g, 9.3 mmol), [1,1'-bis(diphenylphosphino)ferrocene]dichloropalladium(II) (117 mg, 0.16 mmol) and potassium acetate (0.6 g, 6.2 mmol) were added to a reaction flask, and the mixture was dissolved in 10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 578 mg of product, with a yield of 35%. LC-MS (APCI): m/z=534.7 $(M+1)^+$.

Step 2: Synthesis of compound T-115

(R)-N-(4-(Chlorodifluoromethoxy)phenyl)-4-methyl-6-(4,4,5,5-tetramethyl-1,3,2-dioxaborolan-2-yl)-3,4-dihydro-1H-benzo[4,5]imidazo[2,1-c][1,4]oxazine-8-carboxamide (208 mg, 0.39 mmol), 2-iodopyrazine (92.9 mg, 0.588 mmol), [1,1'-bis(diphenylphosphino)ferrocene]dichloropalladium(II) (32 mg, 0.02 mmol), tetrabutylammonium fluoride (1.2 mL, 1.2 mmol) and 5 mL of tetrahydrofuran were added to a reaction flask. The mixture was purged with nitrogen for 5 minutes. The mixture was microwaved and heated to 70 °C, and reacted for 1 hour. The reaction was monitored by TLC until it was completed. The resulting mixture was concentrated, and purified by silica gel column chromatography to give 136 mg of product, with a yield of 72%. LC-MS (APCI): m/z=486.1 $(M+1)^+$. $^1$H NMR (400 MHz, DMSO-$d_6$) δ 10.21 (s, 1H), 8.86 - 8.79 (m, 2H), 8.74 - 8.71 (m, 1H), 8.63 (d, J = 2.5 Hz, 1H), 8.25 (d, J = 2.3 Hz, 1H), 7.86 (d, J = 9.0 Hz, 2H), 7.32 (d, J = 8.8 Hz, 2H), 4.94 (s, 2H), 3.52 (m, 1H), 3.22 (m, 2H), 1.86 (d, J = 8.6 Hz, 3H).

**Example 116 Preparation of (R)-N-(4-(chlorodifluoromethoxy)phenyl)-4-methyl-6-(pyrazin-2-yl)-3,4-dihydro-2H-benzo[4,5]imidazo[2,1-c][1,3]thiazine-8-carboxamide (compound T-116)**

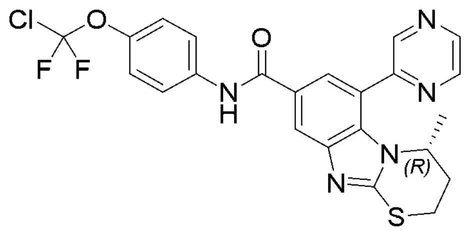

T-116

The following synthetic route was adopted:

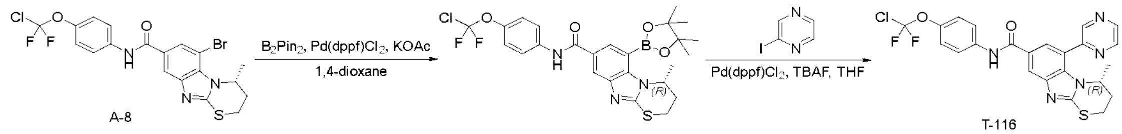


Step 1: Synthesis of compound (R)-N-(4-(chlorodifluoromethoxy)phenyl)-4-methyl-6-(4,4,5,5-tetramethyl-1,3,2-dioxaborolan-2-yl)-3,4-dihydro-2H-benzo[4,5]imidazo[2,1-b][1,3]thiazine-8-carboxamide

Intermediate compound A-8 (1.55 g, 3.1 mmol), bis(pinacolato)diboron (2.4 g, 9.3 mmol), [1,1'-bis(diphenylphosphino)ferrocene]dichloropalladium(II) (117 mg, 0.16 mmol) and potassium acetate (0.6 g, 6.2 mmol) were added to a reaction flask, and the mixture was dissolved in 10 mL of anhydrous 1,4-dioxane. The mixture was microwaved and heated to 100 °C, and reacted for 2 hours. The reaction was monitored by TLC until it was completed. The reaction solution was concentrated, and then purified by silica gel column chromatography to give 766 mg of product, with a yield of 45%. LC-MS (APCI): m/z=550.7 $(M+1)^+$.

Step 7: Synthesis of compound T-116

(R)-N-(4-(Chlorodifluoromethoxy)phenyl)-4-methyl-6-(4,4,5,5-tetramethyl-1,3,2-dioxaborolan-2-yl)-3,4-dihydro-2H-benzo[4,5]imidazo[2,1-b][1,3]thiazine-8-carboxamide (214 mg, 0.39 mmol), 2-iodopyrazine (92.9 mg, 0.588 mmol), [1,1'-bis(diphenylphosphino)ferrocene]dichloropalladium(II) (32 mg, 0.02 mmol), tetrabutylammonium fluoride (1.2 mL, 1.18 mmol) and 5 mL of tetrahydrofuran were added to a reaction flask. The mixture was purged with nitrogen for 5 minutes. The mixture was microwaved and heated to 70 °C, and reacted for 1 hour. The reaction was monitored by TLC until it was completed. The resulting mixture was concentrated, and then purified by silica gel column chromatography to give 115 mg of product, with a yield of 59%. LC-MS (APCI): m/z=502.4 $(M+1)^+$. $^1$H NMR (400 MHz, DMSO-$d_6$) δ 10.22 (s, 1H), 8.86 - 8.76 (m, 2H), 8.74 - 8.70 (m, 1H), 8.63 (d, J = 2.5 Hz, 1H), 8.25 (d, J = 2.3 Hz, 1H), 7.84 (d, J = 9.0 Hz, 2H), 7.31 (d, J = 8.8 Hz, 2H), 3.52 (m, 1H), 2.52 (m, 2H), 2.08 (t, J = 4.4 Hz, 2H), 1.81 (d, J = 8.5 Hz, 3H).

**Examples 117 and 118: Preparation of (4R)-N-(4-(chlorodifluoromethoxy)phenyl)-4-methyl-6-(pyrazin-2-yl)-3,4-dihydro-2H-benzo[4,5]imidazo[2,1-b][1,3]thiazine-8-carboxamide 1-oxide (compound T-117) and (R)-N-(4-(chlorodifluoromethoxy)phenyl)-4-methyl-6-(pyrazin-2-yl)-3,4-dihydro-2H-benzo[4,5]imidazo[2,1-b][1,3]thiazine-8-carboxamide 1,1-dioxide (compound T-118)**

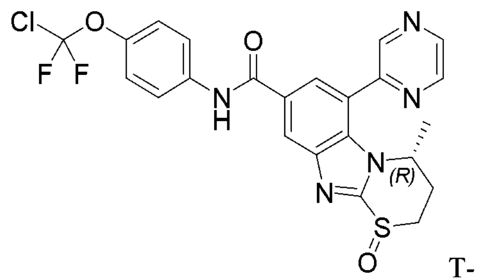

T-117 and

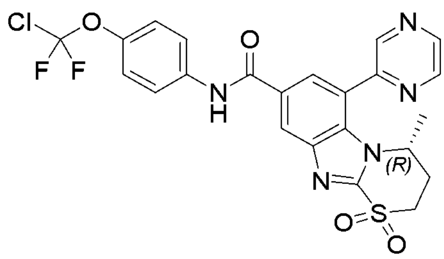

T-118

The following synthetic route was adopted:

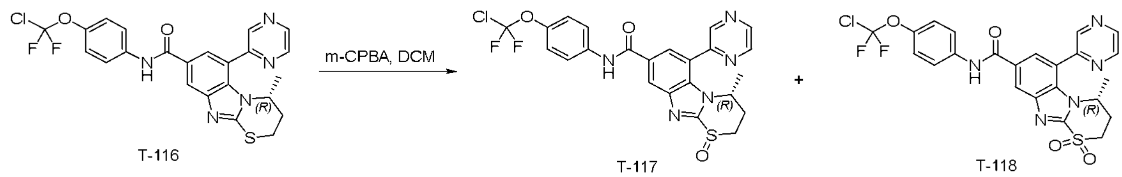


Compound T-116 (200 mg, 0.40 mmol) was dissolved in 10 mL of anhydrous dichloromethane. m-Chloroperoxybenzoic acid (103 mg, 0.6 mmol) was added in an ice bath. After the addition was completed, the mixture was stirred and reacted at room temperature under nitrogen overnight. The reaction was monitored by TLC until it was completed. Water was added to quench the reaction. The resulting mixture was extracted with dichloromethane 3 to 4 times. The organic phases were combined, washed with saturated brine, concentrated, and then purified by silica gel column chromatography to give 60 mg of product T-117, with a yield of 29%. LC-MS (APCI): m/z=518.2 $(M+1)^+$. $^1$H NMR (400 MHz, DMSO-$d_6$) δ 10.23 (s, 1H), 8.77-8.68 (m, 2H), 8.61 (s, 1H), 8.50 (d, J = 2.3 Hz, 1H), 8.22 (d, J = 2.3 Hz, 1H), 7.64 (d, J = 9.0 Hz, 2H), 7.30 (d, J = 8.8 Hz, 2H), 3.66 (m, 1H), 2.18 (t, J = 9.0 Hz, 2H), 2.04-1.87 (m, 2 H), 1.73 (d, J = 8.6 Hz, 3H).

75 mg of product T-118 was obtained, with a yield of 35%. LC-MS (APCI): m/z=534.6 $(M+1)^+$. $^1$H NMR (400 MHz, DMSO-$d_6$) δ 10.21 (s, 1H), 8.77-8.64 (m, 2H), 8.60 (s, 1H), 8.50 (d, J = 2.4 Hz, 1H), 8.22 (d, J = 2.3 Hz, 1H), 7.63 (d, J = 9.0 Hz, 2H), 7.30 (d, J = 8.8 Hz, 2H), 3.66 (m, 1H), 2.44 (t, J =6.6 Hz, 2H), 2.05-1.84 (m, 2 H), 1.71 (d, J = 8.4 Hz, 3H).

**Example 119 Preparation of N-(4-(chlorodifluoromethoxy)phenyl)-7-(2,4-dihvdropyrazolo[3',4':3,4]cyclopenta[1,2-b]pyridin-7-yl)-1-isopropyl-1H-benzo[d]imidazole-5-carboxamide (compound T-119)**

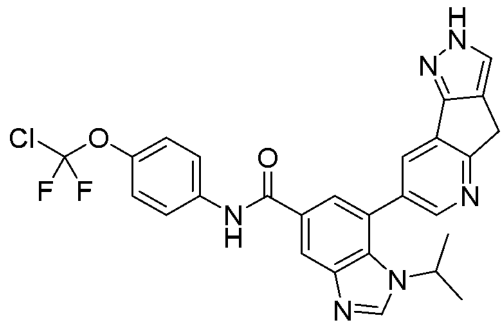

T-119

The following synthetic route was adopted:

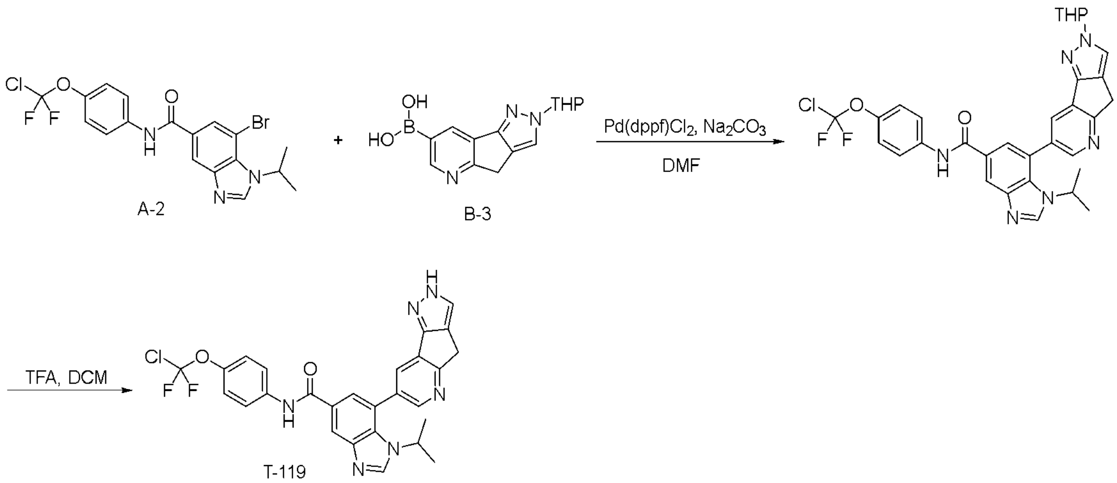


Step 1: Synthesis of compound N-(4-(chlorodifluoromethoxy)phenyl)-1-isopropyl-7-(2-(tetrahydro-2H-pyran-2-yl)-2,4-dihydropyrazolo[3',4':3,4]cyclopenta[1,2-b]pyridin-7-yl)-1H-benzo[d]imidazole-5-carboxamide

Intermediate compound A-2 (178 mg, 0.39 mmol), intermediate compound B-3 (167 mg, 0.588 mmol), [1,1'-bis(diphenylphosphino)ferrocene]dichloropalladium(II) (32 mg, 0.02 mmol) and sodium carbonate (125 mg, 1.18 mmol) were added to a reaction flask, and the mixture was dissolved in 5 mL of anhydrous DMF. The mixture was purged with nitrogen for 5 minutes. The mixture was microwaved and heated to 90 °C, and reacted for 1 hour. The reaction was monitored by TLC until it was completed. Then, the resulting mixture was concentrated, and purified by silica gel column chromatography to give 149 mg of product, with a yield of 62%. LC-MS (APCI): m/z=619.4 $(M+1)^+$.

Step 2: Synthesis of compound T-119

N-(4-(Chlorodifluoromethoxy)phenyl)-1-isopropyl-7-(2-(tetrahydro-2H-pyran-2-yl)-2,4-dihydropyrazolo [3',4':3,4]cyclopenta[1,2-b]pyridin-7-yl)-1H-benzo[d]imidazole-5-carboxamide (149 mg, 0.24 mmol) was dissolved in 5 mL of anhydrous dichloromethane, and 1 mL of trifluoroacetic acid was added. The mixture was stirred and reacted at room temperature for 1 to 2 hours. The reaction was monitored by TLC until it was completed. The mixture was concentrated to remove the solvent, and dichloromethane was added. The resulting mixture was washed with saturated aqueous solution of sodium bicarbonate three times. The organic phase was concentrated, and then purified by silica gel column chromatography to give 61 mg of a white solid, with a yield of 48%. LC-MS (APCI): m/z=535.3 $(M+1)^+$. $^1H$ NMR (400 MHz, DMSO-$d_6$) δ 10.23 (s, 1H), 9.33 (s, 1H), 8.66 (s, 1H), 8.41 (s, 1H), 8.21 (s, 1H), 7.69 (d, J = 11.2 Hz, 1H), 7.55 (s, 1H), 7.41 (d, J = 8.4 Hz, 2H), 6.82 (d, J = 8.4 Hz, 2H), 5.15 (s, 2H), 3.55 (m, 1H), 1.47 (d, J = 9.5 Hz, 6H).

**Example 120 Preparation of N-(4-(chlorodifluoromethoxy)phenyl)-1-isopropyl-7-(2-(pyrimidin-2-yl)-2,4-dihydropyrazolo[3',4':3,4]cyclopenta[1,2-b]pyridin-7-yl)-1H-benzo[d]imidazole-5-carboxamide (compound T-120)**

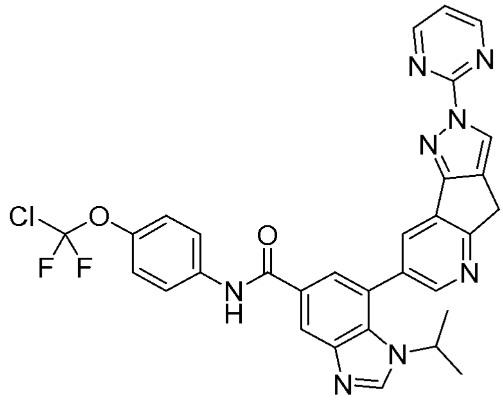

T-120

The following synthetic route was adopted:

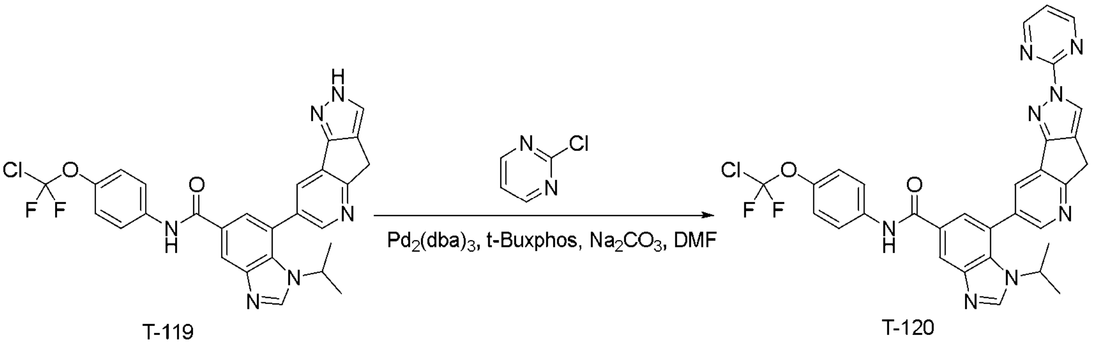


Compound T-120 was prepared using compound T-119 and 2-chloropyrimidine as the raw materials according to the method described in example 4, with a yield of 33%. LC-MS (APCI): m/z=613.5 $(M+1)^+$. $^1H$ NMR (400 MHz, DMSO-$d_6$) δ 10.22 (s, 1H), 9.33 (s, 1H), 8.66-8.51 (m, 3H), 8.41 (s, 1H), 8.21 (s, 1H), 7.78 (t, J = 9.6 Hz, 1H), 7.70 (d, J = 10.4 Hz, 1H), 7.56 (s, 1H), 7.45 (d, J = 8.4 Hz, 2H), 6.83 (d, J = 8.4 Hz, 2H), 5.18 (s, 2H), 3.32 (m, 1H), 1.48 (d, J = 9.5 Hz, 6H).

**Example 121 Preparation of N-(4-(chlorodifluoromethoxy)phenyl)-7-(2-(5-fluoropyrimidin-2-yl)-2,4-dihydropyrazolo[3',4':3,4]cyclopenta[1,2-b]pyridin-7-yl)-1-isopropyl-1H-benzo[d]imidazole-5-carboxamide (compound T-121)**

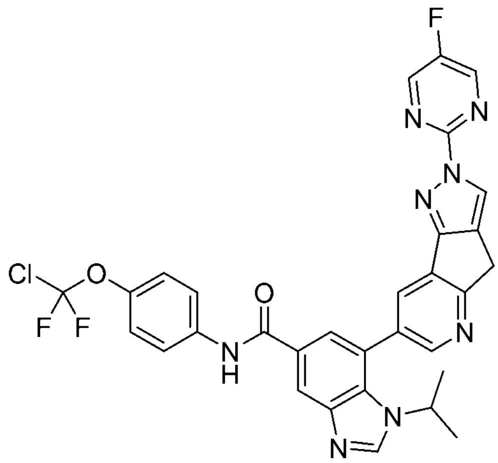

T-121

The following synthetic route was adopted:

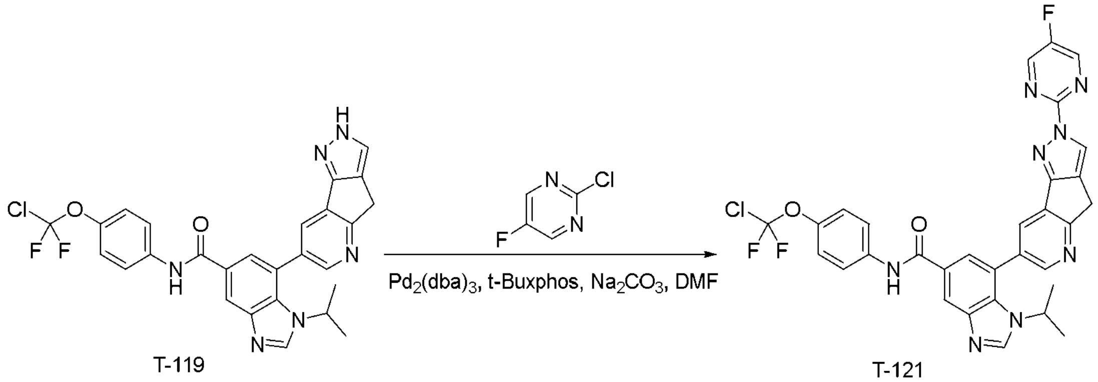


T-119 T-121

Compound T-121 was prepared using compound T-119 and 2-chloro-5-fluoropyrimidine as the raw materials according to the method described in example 4, with a yield of 41%. LC-MS (APCI): m/z=631.2 $(M+1)^+$. $^1$H NMR (400 MHz, DMSO-$d_6$) $\delta$ 10.23 (s, 1H), 9.33 (s, 1H), 8.68 (s, 1H), 8.49 (dd, J = 11.5 Hz, J = 8.4 Hz, 2H), 8.43 (s, 1H), 8.20 (s, 1H), 7.71 (d, J = 10.4 Hz, 1H), 7.53 (s, 1H), 7.42 (d, J = 8.4 Hz, 2H), 6.81 (d, J = 8.4 Hz, 2H), 5.18 (s, 2H), 3.32 (m, 1H), 1.47 (d, J = 9.5 Hz, 6H).

**Example 122 Preparation of N-(4-(chlorodifluoromethoxy)phenyl)-4-(2,4-dihydropyrazolo[3',4':3,4]cyclopenta[1,2-b]pyridin-7-yl)-3,3-dimethyl-2-oxoindoline-6-carboxamide (compound T-122)**

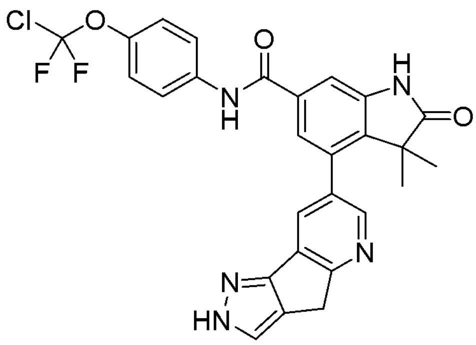

T-122

The following synthetic route was adopted:

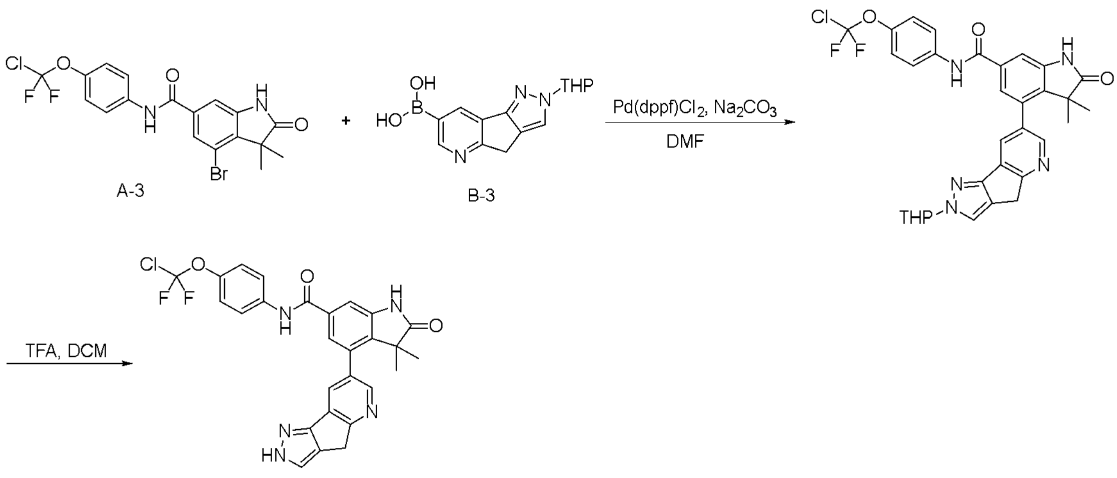


A-3 B-3 T-122

Step 1: Synthesis of compound N-(4-(chlorodifluoromethoxy)phenyl)-3,3-dimethyl-2-oxo-4-(2-(tetrahydro-2H-pyran-2-yl)-2,4-dihydropyrazolo[3',4':3,4]cyclopenta[1,2-b]pyridin-7-yl)indoline-6-carboxamide

Intermediate compound A-3 (179 mg, 0.39 mmol), intermediate compound B-3 (167 mg, 0.588 mmol), [1,1'-bis(diphenylphosphino)ferrocene]dichloropalladium(II) (32 mg, 0.02 mmol) and sodium carbonate (125 mg, 1.18 mmol) were added to a reaction flask. The mixture was dissolved in 5 mL of anhydrous DMF. The resulting mixture was purged with nitrogen for 5 minutes. The mixture was microwaved and heated to 90 °C, and reacted for 1 hour. The reaction was monitored by TLC until it was completed. Then, the resulting mixture was concentrated, and purified by silica gel column chromatography to give 193 mg of product, with a yield of 80%. LC-MS (APCI): m/z=620.3 $(M+1)^+$.

Step 2: Synthesis of compound T-122

N-(4-(Chlorodifluoromethoxy)phenyl)-3,3-dimethyl-2-oxo-4-(2-(tetrahydro-2H-pyran-2-yl)-2,4-dihydropyrazolo [3',4':3,4]cyclopenta[1,2-b]pyridin-7-yl)indoline-6-carboxamide (193 mg, 0.31 mmol) was dissolved in 5 mL of anhydrous dichloromethane, and 1 mL of trifluoroacetic acid was added. The mixture was stirred and reacted at room temperature for 1 to 2 hours. The reaction was monitored by TLC until it was completed. The mixture was concentrated to remove the solvent, and dichloromethane was added. The resulting mixture was washed with saturated aqueous solution of sodium bicarbonate three times. The organic phase was concentrated, and then purified by silica gel column chromatography to give 128 mg of a white solid, with a yield of 77%. LC-MS (APCI): m/z=536.7 $(M+1)^+$. $^1$H NMR (400 MHz, DMSO-$d_6$) δ 10.23 (s, 1H), 8.67 (s, 1H), 8.28 (s, 1H), 7.87 (d, J = 11.5 Hz, 1H), 7.48 (s, 1H), 7.40 (s, 1H), 7.35 (d, J = 8.4 Hz, 2H), 6.83 (d, J = 8.4 Hz, 2H), 5.04 (s, 2H), 1.85 (s, 6H).

**Example 123 Preparation of N-(4-(chlorodifluoromethoxy)phenyl)-3,3-dimethyl-2-oxo-4-(2-(pyrimidin-2-yl)-2,4-dihydropyrazolo[3',4':3,4]cyclopenta[1,2-b]pyridin-7-yl)indoline-6-carboxamide (compound T-123)**

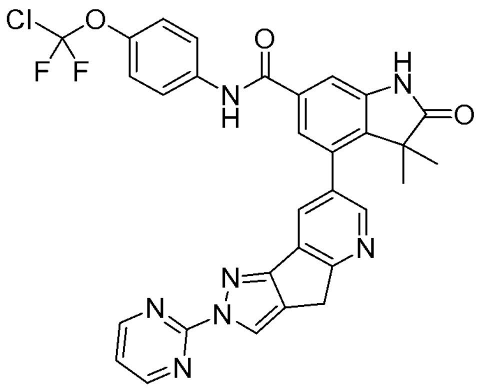

T-123

The following synthetic route was adopted:

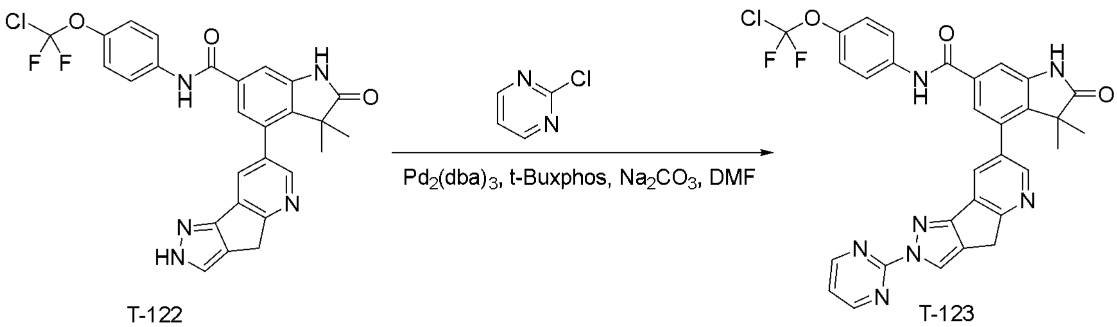


Compound T-123 was prepared using compound T-122 and 2-chloropyrimidine as the raw materials according to the method described in example 4, with a yield of 52%. LC-MS (APCI): m/z=614.8 $(M+1)^+$. $^1$H NMR (400 MHz, DMSO-$d_6$) δ 10.23 (s, 1H), 8.64 (s, 1H), 8.23-8.11 (m, 3H), 7.87 (d, J = 11.5 Hz, 1H), 7.48 (s, 1H), 7.40 (s, 1H), 7.38 (t, J = 9.6 Hz, 1H), 7.36 (d, J = 8.4 Hz, 2H), 6.82 (d, J = 8.4 Hz, 2H), 5.10 (s, 2H), 1.88 (s, 6H).

**Example 124 Preparation of N-(4-(chlorodifluoromethoxy)phenyl)-4-(2-(5-fluoropyrimidin-2-yl)-2,4-dihydropyrazolo[3',4':3,4]cyclopenta[1,2-b]pyridin-7-yl)-3,3-dimethyl-2-oxoindoline-6-carboxamide (compound T-124)**

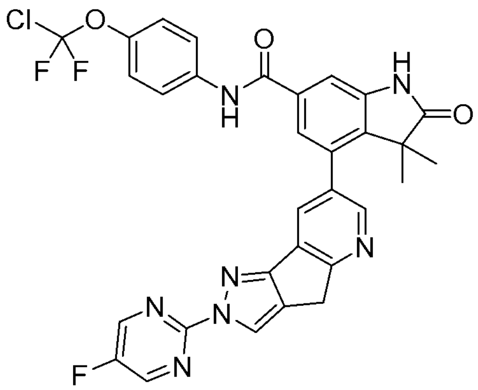

T-124

The following synthetic route was adopted:

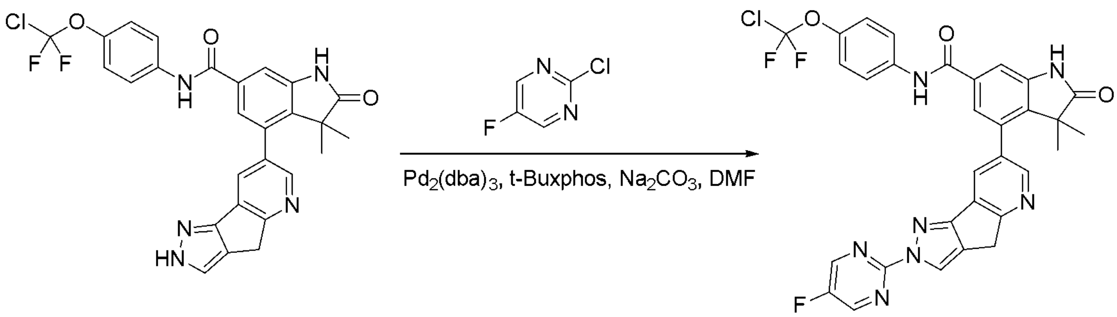


T-122 T-124

Compound T-124 was prepared using compound T-122 and 2-chloro-5-fluoropyrimidine as the raw materials according to the method described in example 4, with a yield of 29%. LC-MS (APCI): m/z=632.6 $(M+1)^+$. $^1H$ NMR (400 MHz, DMSO-$d_6$) δ 10.23 (s, 1H), 8.64 (s, 1H), 8.38-8.30 (m, 2H), 8.24 (s, 1H), 7.87 (d, J = 11.5 Hz, 1H), 7.46 (s, 1H), 7.42 (s, 1H), 7.36 (d, J = 8.4 Hz, 2H), 6.83 (d, J = 8.4 Hz, 2H), 5.06 (s, 2H), 1.78 (s, 6H).

**Example 125 Preparation of N-(4-(chlorodifluoromethoxy)phenyl)-4'-(2,4-dihydropyrazolo[3',4':3,4]cyclopenta[1,2-b]pyridin-7-yl)-2'-oxospiro[cyclohexane-1,3'-indoline]-6'-carboxamide (compound T-125)**

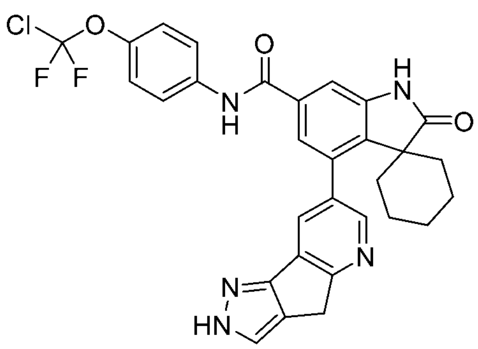

T-125

The following synthetic route was adopted:

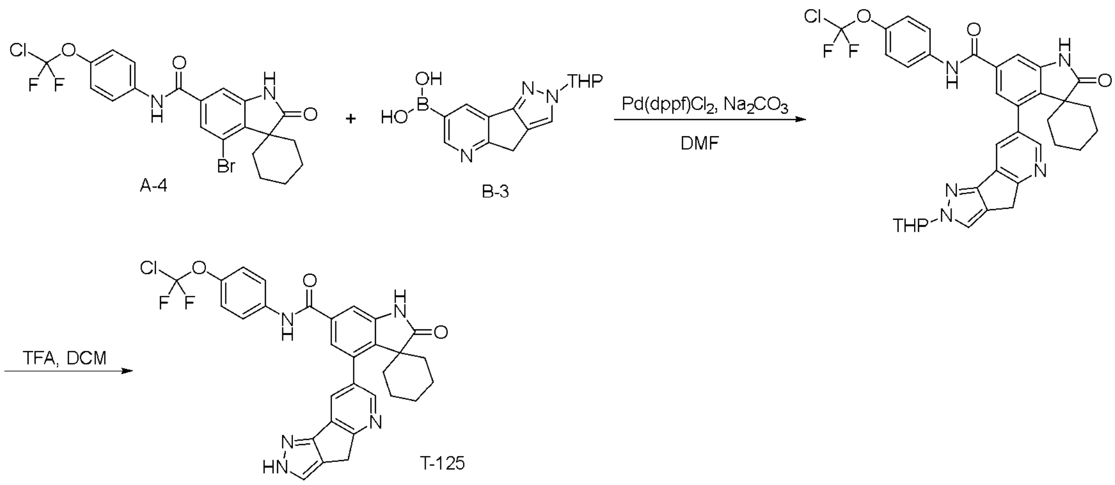


Step 1: Synthesis of compound N-(4-(chlorodifluoromethoxy)phenyl)-2'-oxo-4'-(2-(tetrahydro-2H-pyran-2-yl)-2,4-dihydropyrazolo[3',4':3,4]cyclopenta[1,2-b]pyridin-7-yl)spiro[cyclohexane-1,3'-indoline]-6'-carboxamide

Intermediate compound A-4 (194 mg, 0.39 mmol), intermediate compound B-3 (167 mg, 0.588 mmol), [1,1'-bis(diphenylphosphino)ferrocene]dichloropalladium(II) (32 mg, 0.02 mmol) and sodium carbonate (125 mg, 1.18 mmol) were added to a reaction flask, and the mixture was dissolved in 5 mL of anhydrous DMF. The mixture was purged with nitrogen for 5 minutes. The mixture was microwaved and heated to 90 °C, and reacted for 1 hour. The reaction was monitored by TLC until it was completed. Then, the resulting mixture was concentrated, and purified by silica gel column chromatography to give 105 mg of product, with a yield of 41%. LC-MS (APCI): m/z=660.2 $(M+1)^+$.

Step 2: Synthesis of compound T-125

N-(4-(Chlorodifluoromethoxy)phenyl)-2'-oxo-4'-(2-(tetrahydro-2H-pyran-2-yl)-2,4-dihydropyrazolo[3',4':3,4] cyclopenta[1,2-b]pyridin-7-yl)spiro[cyclohexane-1,3'-indoline]-6'-carboxamide (105 mg, 0.16 mmol) was dissolved in 5 mL of anhydrous dichloromethane, and 1 mL of trifluoroacetic acid was added. The mixture was stirred and reacted at room temperature for 1 to 2 hours. The reaction was monitored by TLC until it was completed. The mixture was concentrated to remove the solvent, and dichloromethane was added. The resulting mixture was washed with saturated aqueous solution of sodium bicarbonate three times. The organic phase was concentrated, and then purified by silica gel column chromatography to give 59 mg of a white solid, with a yield of 64%. LC-MS (APCI): m/z=576.4 $(M+1)^+$. $^1H$ NMR (400 MHz, DMSO-$d_6$) δ 10.23 (s, 1H), 8.65 (s, 1H), 8.25 (s, 1H), 7.83 (d, J = 11.5 Hz, 1H), 7.45 (s, 1H), 7.40 (s, 1H), 7.35 (d, J = 8.4 Hz, 2H), 6.83 (d, J = 8.4 Hz, 2H), 5.05 (s, 2H), 1.86 (m, 4H), 1.23-1.10 (m, 6H).

**Example 126 Preparation of N-(4-(chlorodifluoromethoxy)phenyl)-4'-(2-(pyrimidin-2-yl)-2,4-dihydropyrazolo [3',4':3,4]cyclopenta[1,2-b]pyridin-7-yl)-2'-oxospiro[cyclohexane-1,3'-indoline]-6'-carboxamide (compound T-126)**

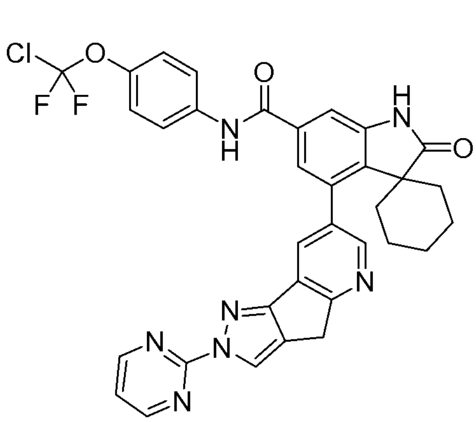

T-126

The following synthetic route was adopted:

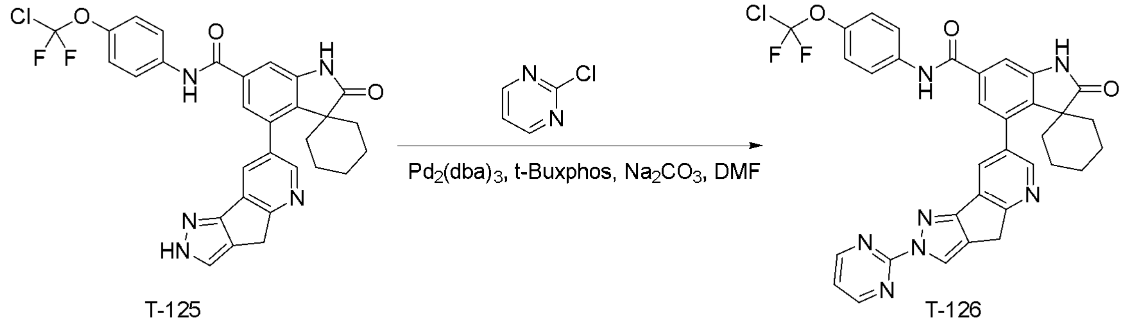


T-125 T-126

Compound T-126 was prepared using compound T-125 and 2-chloropyrimidine as the raw materials according to the method described in example 4, with a yield of 33%. LC-MS (APCI): m/z=654.7 $(M+1)^+$. $^1$H NMR (400 MHz, DMSO-$d_6$) δ 10.23 (s, 1H), 8.64 (s, 1H), 8.25-8.13 (m, 3H), 7.84 (d, J = 11.5 Hz, 1H), 7.43 (s, 1H), 7.40 (s, 1H), 7.38 (t, J = 9.6 Hz, 1H), 7.33 (d, J = 8.4 Hz, 2H), 6.84 (d, J = 8.4 Hz, 2H), 5.10 (s, 2H), 1.88 (m, 4H), 1.26-1.11 (m, 6H).

**<u>Example 127 Preparation of N-(4-(chlorodifluoromethoxy)phenyl)-4'-(2-(5-fluoropyrimidin-2-yl)-2,4-dihydropyrazolo[3',4':3,4]cyclopenta[1,2-b]pyridin-7-vl)-2'-oxospiro[cyclohexane-1,3'-indoline]-6'-carboxamide (compound T-127)</u>**

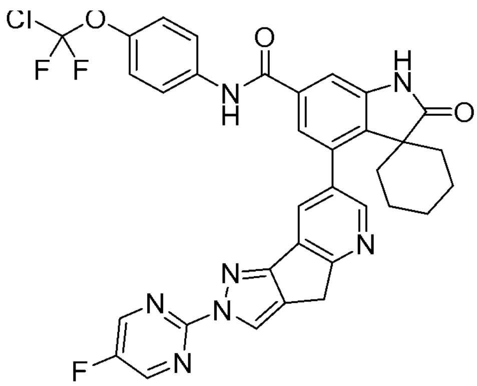

T-127

The following synthetic route was adopted:

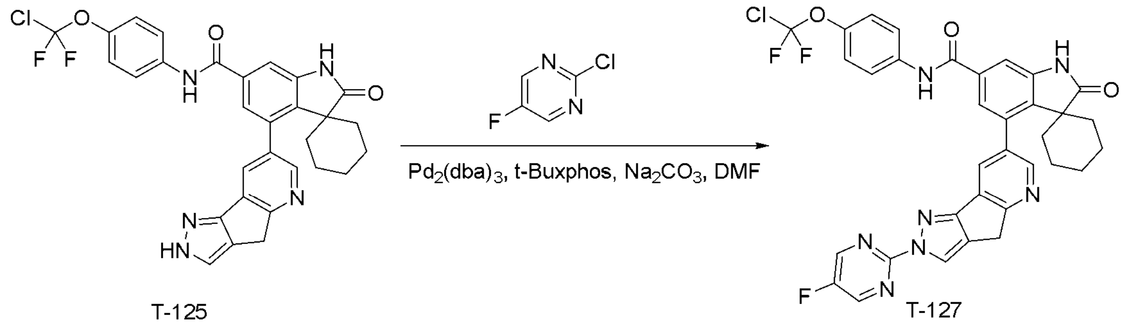


T-125 T-127

Compound T-127 was prepared using compound T-125 and 2-chloro-5-fluoropyrimidine as the raw materials according to the method described in example 4, with a yield of 28%. LC-MS (APCI): m/z=672.5 $(M+1)^+$. $^1$H NMR (400 MHz, DMSO-$d_6$) δ 10.22 (s, 1H), 8.64 (s, 1H), 8.39-8.31 (m, 2H), 8.24 (s, 1H), 7.85 (d, J = 11.5 Hz, 1H), 7.46 (s, 1H), 7.42 (s, 1H), 7.36 (d, J = 8.4 Hz, 2H), 6.83 (d, J = 8.4 Hz, 2H), 5.11 (s, 2H), 1.89 (m, 4H), 1.26-1.14 (m, 6H).

**Example 128 Preparation of (R)-N-(4-(chlorodifluoromethoxy)phenyl)-5-(2-(5-cyanopyrimidin-2-yl)-2,4-dihydropyrazolo[3',4':3,4]cyclopenta[1,2-b]pyridin-7-yl)-6-(3-fluoropyrrolidin-1-yl)nicotinamide (compound T-128)**

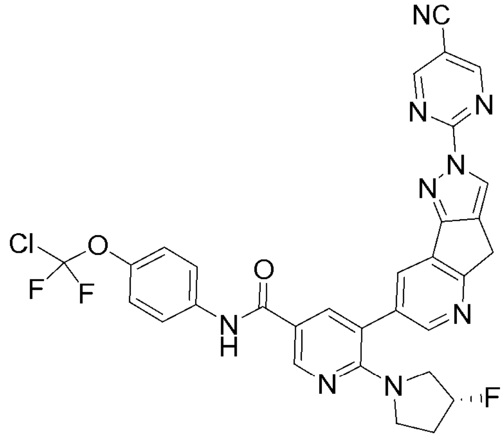

T-128

The following synthetic route was adopted:

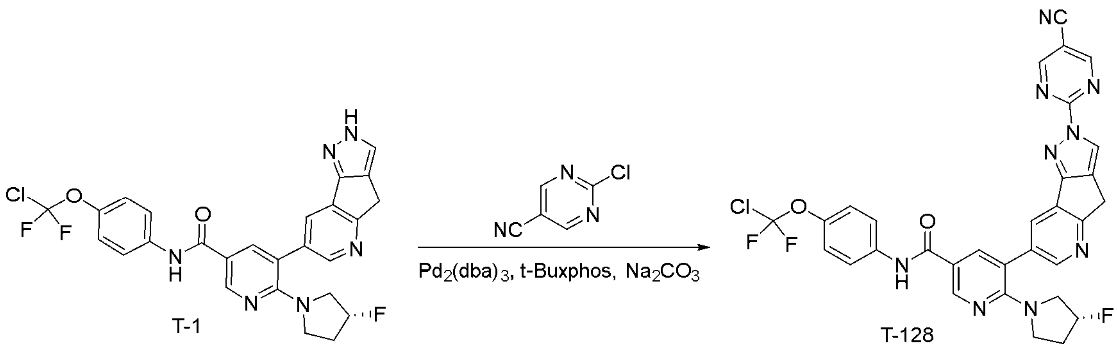


Compound T-128 was prepared using compound T-1 and 2-chloro-5-cyanopyrimidine as the raw materials according to the method described in example 4, with a yield of 12%. LC-MS (APCI): m/z=644.2 $(M+1)^+$. $^1H$ NMR (400 MHz, DMSO-$d_6$) δ 10.23 (s, 1H), 8.93 (s, 1H), 8.66 (s, 1H), 8.41 (s, 1H), 8.31 (s, 1H), 8.21 (s, 1H), 7.58 (d, J = 11.2 Hz, 2H), 7.24 (d, J = 8.4 Hz, 2H), 6.81 (d, J = 8.4 Hz, 2H), 5.27-5.15 (d, J = 22.3 Hz, 1H), 4.31 (s, 2H), 3.94 (m, 1H), 3.91-3.76 (m, 1H), 3.55 (d, J = 8.6 Hz, 2H), 2.14 (dd, J = 9.5, 3.8 Hz, 2H).

**Example 129 Preparation of (R)-N-(4-(chlorodifluoromethoxy)phenyl)-5-(2-(4-cyanopyrimidin-2-yl)-2,4-dihydropyrazolo[3',4':3,4]cyclopenta[1,2-b]pyridin-7-yl)-6-(3-fluoropyrrolidin-1-yl)nicotinamide (compound T-129)**

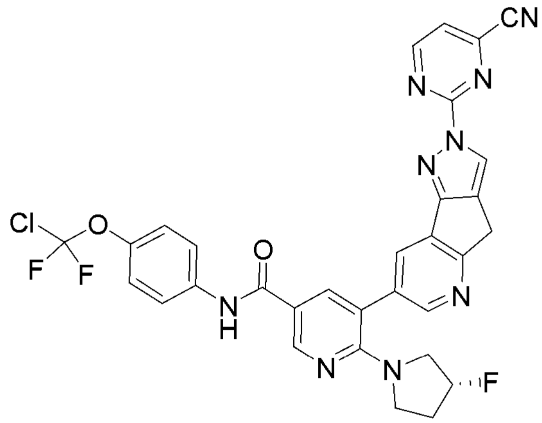

T-129

The following synthetic route was adopted:

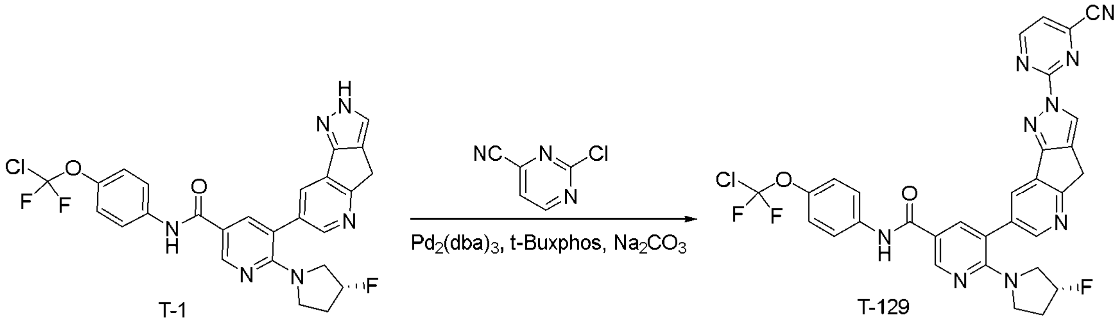


Compound T-129 was prepared using compound T-1 and 2-chloro-4-cyanopyrimidine as the raw materials according to the method described in example 4, with a yield of 22%. LC-MS (APCI): m/z=644.3 $(M+1)^+$. $^1H$ NMR (400 MHz, DMSO-$d_6$) δ 10.22 (s, 1H), 8.91 (s, 1H), 8.66 (s, 1H), 8.41 (s, 1H), 8.31 (s, 1H), 8.21 (s, 1H), 7.58 (d, J = 11.2 Hz, 2H), 7.25 (d, J = 8.4 Hz, 2H), 6.81 (d, J = 8.4 Hz, 2H), 5.27-5.15 (d, J = 21.6 Hz, 1H), 4.32 (s, 2H), 3.94 (m, 1H), 3.91-3.76 (m, 1H), 3.50 (d, J = 8.6 Hz, 2H), 2.13 (dd, J = 9.5, 3.8 Hz, 2H).

**Example 130 Preparation of N-(4-(chlorodifluoromethoxy)phenyl)-5-(4-fluoro-2-(5-fluoropyrimidin-2-yl)-2,4-dihydropyrazolo[3',4':3,4]cyclopenta[1,2-b]pyridin-7-yl)-6-((R)-3-fluoropyrrolidin-1-yl)nicotinamide (compound T-130)**

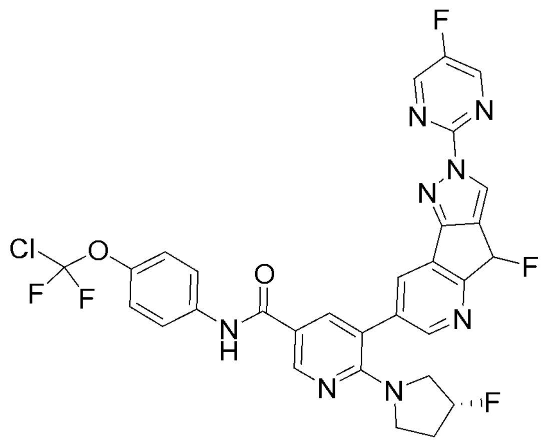

T-130

The following synthetic route was adopted:

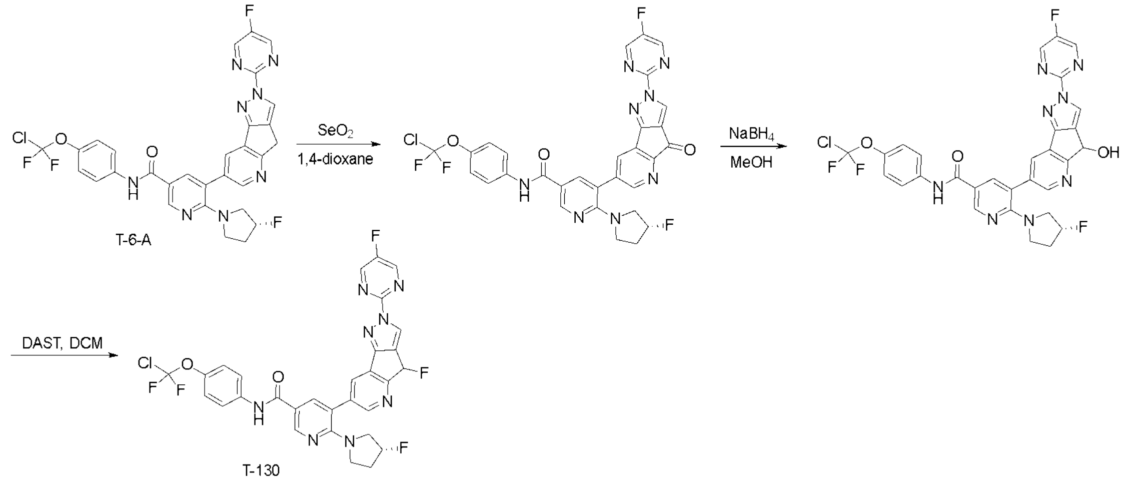


T-6-A

T-130

Step 1: Synthesis of compound (R)-N-(4-(chlorodifluoromethoxy)phenyl)-5-(2-(5-fluoropyrimidin-2-yl)-4-oxo-2,4-dihydropyrazolo[3',4':3,4]cyclopenta[1,2-b]pyridin-7-yl)-6-(3-fluoropyrrolidin-1-yl)nicotinamide

Compound T-6-A (200 mg, 0.31 mmol), 1,4-dioxane (5 mL) and selenium dioxide (52 mg, 0.47 mmol) were added to a reaction flask. After the addition was completed, the mixture was heated to 50 °C and reacted for 2 hours in an oil bath. The reaction was monitored by TLC until it was completed. The resulting mixture was concentrated, filtered with diatomaceous earth as an aid. The filtrate was concentrated, and then purified by silica gel column chromatography to give 177 mg of product, with a yield of 88%. LC-MS (APCI): m/z=651.2 $(M+1)^+$.

Step 2: Synthesis of compound N-(4-(chlorodifluoromethoxy)phenyl)-5-(2-(5-fluoropyrimidin-2-yl)-4-hydroxy-2,4-dihydropyrazolo[3',4':3,4]cyclopenta[1,2-b]pyridin-7-yl)-6-((R)-3-fluoropyrrolidin-1-yl)nicotinamide

(R)-N-(4-(Chlorodifluoromethoxy)phenyl)-5-(2-(5-fluoropyrimidin-2-yl)-4-oxo-2,4-dihydropyrazolo[3',4':3,4]cyclopenta[1,2-b]pyridin-7-yl)-6-(3-fluoropyrrolidin-1-yl)nicotinamide (177 mg, 0.27 mmol) and methanol (5 mL) were added to a reaction flask. Sodium borohydride (21 mg, 0.54 mmol) was added in an ice bath. The mixture was warmed to room temperature, stirred and reacted for 1 to 2 hours. The reaction was monitored by TLC until it was completed. The mixture was concentrated to remove the solvent, and dichloromethane was added. The resulting mixture was washed with saturated aqueous solution of sodium bicarbonate three times. The organic phase was concentrated, and then purified by silica gel column chromatography to give 148 mg of a white solid, with a yield of 84%. LC-MS (APCI): m/z=653.4 $(M+1)^+$.

Step 3: Synthesis of compound T-130

N-(4-(Chlorodifluoromethoxy)phenyl)-5-(2-(5-fluoropyrimidin-2-yl)-4-hydroxy-2,4-dihydropyrazolo[3',4':3,4]cyclopenta[1,2-b]pyridin-7-yl)-6-((R)-3-fluoropyrrolidin-1-yl)nicotinamide (148 mg, 0.23 mmol) was added to a reaction flask, and dissolved in 5 mL of added anhydrous dichloromethane under nitrogen. The mixture was cooled to -78 °C, and DAST (56 mg, 0.35 mmol) was added. The mixture was stirred and reacted at a low temperature for 1 to 2 hours. The reaction was monitored by TLC until it was completed. Water was added to quench the reaction. The resulting mixture was extracted with dichloromethane 3 to 4 times. The organic phases were combined, concentrated, and then purified by silica gel column chromatography to give 57 mg of a white solid, with a yield of 38%. LC-MS (APCI): m/z=655.6 $(M+1)^+$. $^1H$ NMR (400 MHz, DMSO-$d_6$) $\delta$ 9.91 (s, 1H), 8.89 (s, 2H), 8.65 (s, 1H), 8.52 (s, 1H), 8.19 (s, 1H), 8.11 (s, 1H), 7.76 (d, J = 8.6 Hz, 2H), 7.35-7.23 (m, 5H), 6.18 (d, J = 44.6 Hz, 1H), 5.25 (d, J = 53.8 Hz, 1H), 3.62-3.43 (m, 2H), 3.30-3.21 (m, 2H), 2.03 (d, J = 11.4 Hz, 2H).

**Example 131 Preparation of N-(4-(chlorodifluoromethoxy)phenyl)-5-(4-fluoro-1-(5-fluoropyrimidin-2-yl)-1,4-dihydrepyrazolo[3',4':3,4]cyclopenta[1,2-b]pyridin-7-yl)-6-((R)-3-fluoropyrrolidin-1-yl)nicotinamide (compound T-131)**

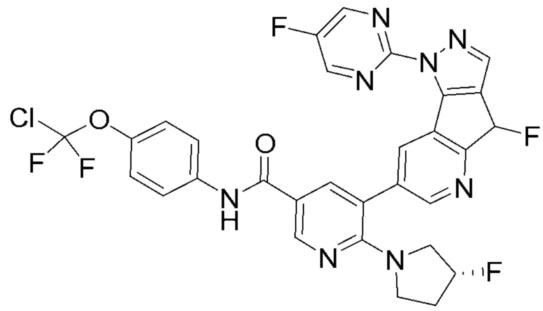

T-131

The following synthetic route was adopted:

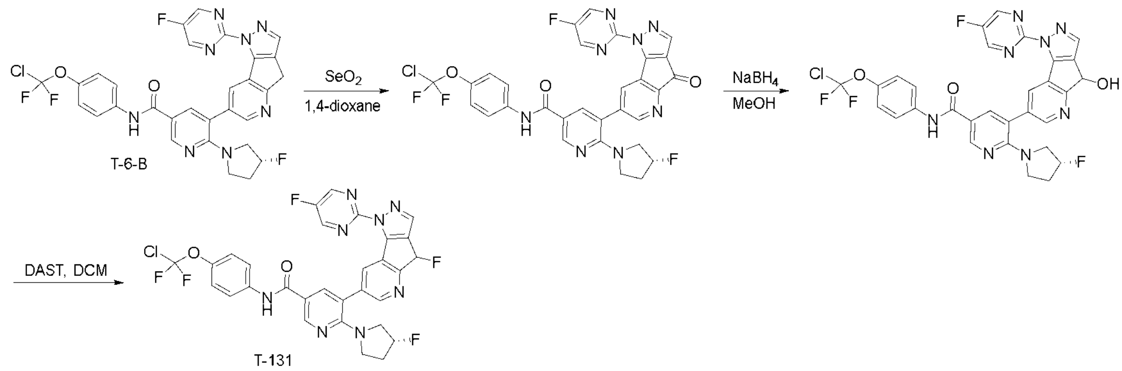


Compound T-131 was prepared using compound T-6-B as the raw material via a three-step reaction according to the method described in example T-130, with an overall yield of 21%. LC-MS (APCI): m/z=655.5 $(M+1)^+$. $^1H$ NMR (400 MHz, DMSO-$d_6$) $\delta$ 9.89 (s, 1H), 8.88 (s, 2H), 8.65 (s, 1H), 8.52 (s, 1H), 8.17 (s, 1H), 8.05 (s, 1H), 7.76 (d, J = 9.1 Hz, 2H), 7.35-7.23 (m, 5H), 6.18 (d, J = 44.6 Hz, 1H), 5.25 (d, J = 53.8 Hz, 1H), 3.62-3.43 (m, 2H), 3.30-3.19 (m, 2H), 2.04 (d, J = 11.4 Hz, 2H).

**Example 132 Preparation of (R)-2-amino-N-(4-(chlorodifluoromethoxy)phenyl)-5-(2-(5-fluoropyrimidin-2-yl)-2,4-dihydropyrazolo[3',4':3,4]cyclopenta[1,2-b]pyridin-7-yl)-6-(3-fluoropyrrolidin-1-yl)nicotinamide (compound T-132)**

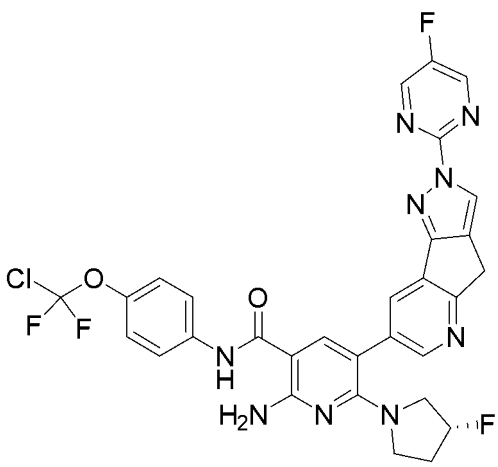

T-132

The following synthetic route was adopted:

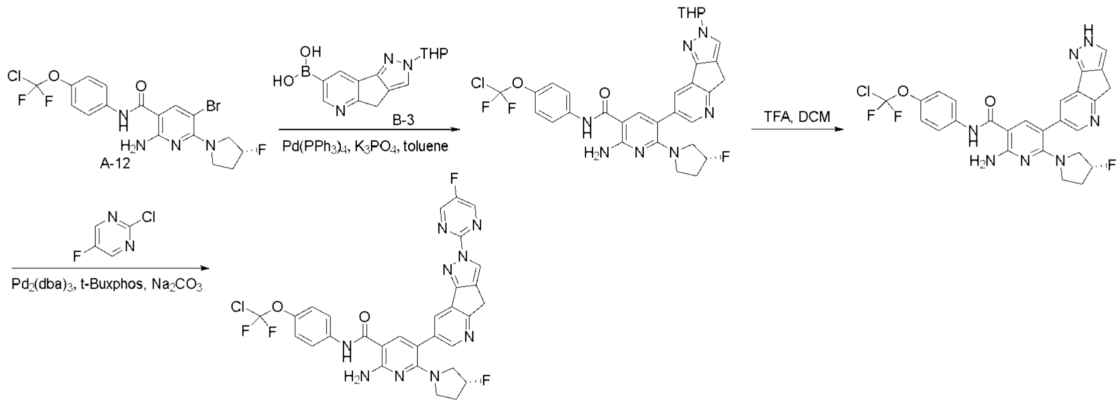


T-132

Step 1: Synthesis of compound 2-amino-N-(4-(chlorodifluoromethoxy)phenyl)-6-((R)-3-fluoropyrrolidin-1-yl)-5-(2-(tetrahydro-2H-pyran-2-yl)-2,4-dihydropyrazolo[3',4':3,4]cyclopenta[1,2-b]pyridin-7-yl)nicotinamide

Intermediate A-12 (110 mg, 0.23 mmol), intermediate compound B-3 (131 mg, 0.46 mmol), potassium phosphate (123 mg, 0.58 mmol), tetrakis(triphenylphosphine)palladium(0) (13 mg, 0.01 mmol) and toluene (3 mL) were added to a reaction flask. The mixture was purged with nitrogen for 5 minutes. The mixture was heated to 100 °C, stirred and reacted under nitrogen for 4 to 6 hours. The reaction was monitored by TLC until it was completed. The resulting mixture was concentrated, and then purified by silica gel column chromatography to give 94 mg of an off-white solid, with a yield of 64%. LC-MS (APCI): m/z=640.2 $(M+1)^+$.

Step 2: Synthesis of compound (R)-2-amino-N-(4-(chlorodifluoromethoxy)phenyl)-5-(2,4-dihydropyrazolo[3',4':3,4]cyclopenta[1,2-b]pyridin-7-yl)-6-(3-fluoropyrrolidin-1-yl)nicotinamide

2-Amino-N-(4-(chlorodifluoromethoxy)phenyl)-6-((R)-3-fluoropyrrolidin-1-yl)-5-(2-(tetrahydro-2H-pyran-2-yl)-2,4-dihydropyrazolo[3',4':3,4]cyclopenta[1,2-b]pyridin-7-yl)nicotinamide (94 mg, 0.15 mmol), dichloromethane (5 mL) and trifluoroacetic acid (1 mL) were added to a reaction flask. The mixture was stirred and reacted at room temperature for 2 hours. The reaction was monitored by TLC until it was completed. The mixture was concentrated to remove the solvent. The resulting mixture was extracted with dichloromethane 2 to 3 times, vacuum dried, and then directly added to the next step reaction. LC-MS (APCI): m/z=556.1 $(M+1)^+$.

Step 3: Synthesis of compound T-132

Compound T-133 was prepared using (R)-2-amino-N-(4-(chlorodifluoromethoxy)phenyl)-5-(2,4-dihydropyrazolo[3',4':3,4]cyclopenta[1,2-b]pyridin-7-yl)-6-(3-fluoropyrrolidin-1-yl)nicotinamide and 2-chloro-5-fluoropyrimidine as the raw materials according to the method described in example 4, with a yield of 34%. LC-MS (APCI): m/z=652.5 $(M+1)^+$. $^1$H NMR (400 MHz, DMSO-$d_6$) δ 9.89 (s, 1H), 8.97 (s, 2H), 8.64 (s, 1H), 8.56 (s, 1H), 8.17 (s, 1H), 8.05 (s, 1H), 7.76 (d, J = 9.1 Hz, 2H), 7.36-7.23 (m, 5H), 5.25 (d, J = 53.8 Hz, 1H), 3.92 (s, 2H), 3.62-3.43 (m, 2H), 3.30-3.16 (m, 2H), 2.04 (d, J = 11.8 Hz, 2H).

**Example 133 Preparation of (R)-2-amino-N-(4-(chlorodifluoromethoxy)phenyl)-5-(2-(4-cyanopyrimidin-2-yl)-2,4-dihydropyrazolo[3',4':3,4]cyclopenta[1,2-b]pyridin-7-yl)-6-(3-fluoropyrrolidin-1-yl)nicotinamide (compound T-133)**

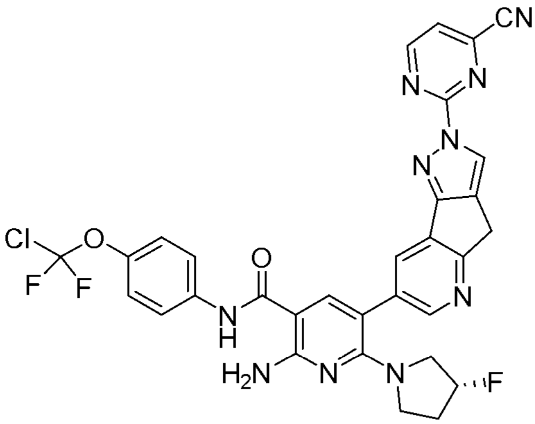

T-133

The following synthetic route was adopted:

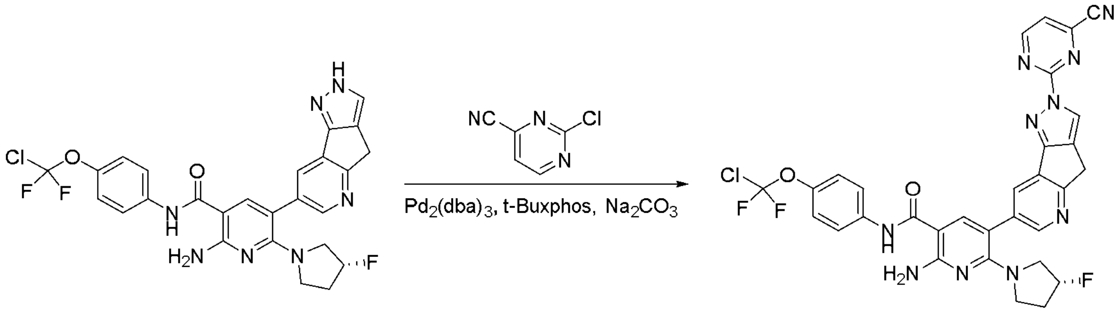


Compound T-134 was prepared using (R)-2-amino-N-(4-(chlorodifluoromethoxy)phenyl)-5-(2,4-dihydropyrazolo[3',4':3,4]cyclopenta[1,2-b]pyridin-7-yl)-6-(3-fluoropyrrolidin-1-yl)nicotinamide and 2-chloro-4-cyanopyrimidine as the raw materials according to the method described in example 4, with a yield of 41%. LC-MS (APCI): m/z=659.5 $(M+1)^+$. $^1H$ NMR (400 MHz, DMSO-$d_6$) δ 9.90 (s, 1H), 8.97 (s, 2H), 8.72 (d, J = 7.7 Hz, 1H), 8.59 (d, J = 7.7 Hz, 1H), 8.15 (s, 1H), 8.01 (s, 1H), 7.76 (d, J = 9.1 Hz, 2H), 7.33-7.23 (m, 5H), 5.26 (d, J = 53.6 Hz, 1H), 3.92 (s, 2H), 3.62-3.43 (m, 2H), 3.30-3.16 (m, 2H), 2.05 (d, J = 10.7 Hz, 2H).

**Example 134 Preparation of (R)-2-amino-N-(4-(chlorodifluoromethoxy)phenyl)-5-(2-(5-cyanopyrimidin-2-yl)-2,4-dihydropyrazolo[3',4':3,4]cyclooenta[1,2-b]pyridin-7-yl)-6-(3-fluoropyrrolidin-1-yl)nicotinamide (compound T-134)**

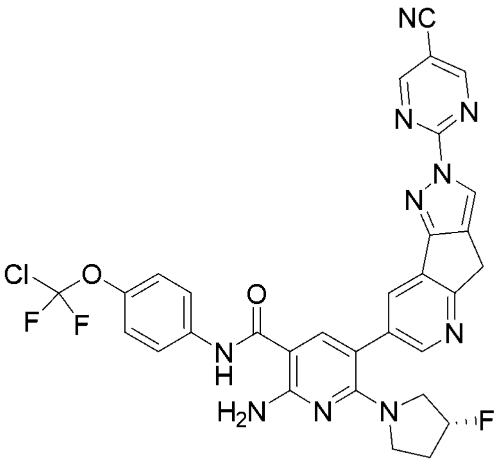

T-134

The following synthetic route was adopted:

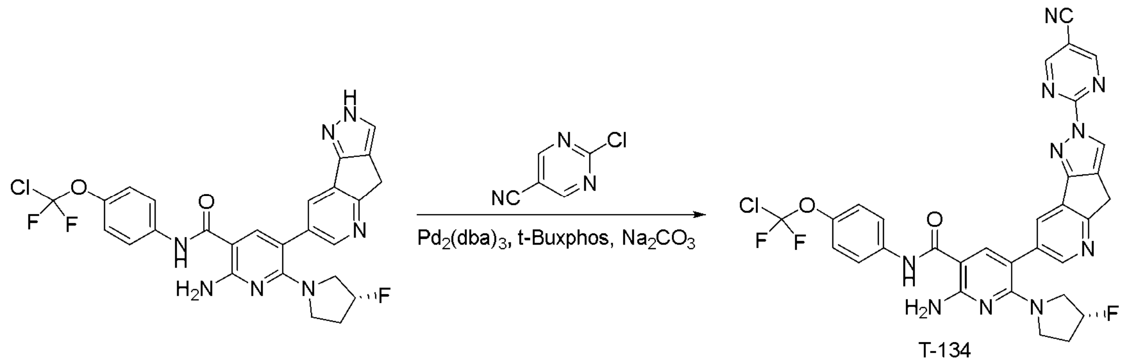

T-134

Compound T-135 was prepared using (R)-2-amino-N-(4-(chlorodifluoromethoxy)phenyl)-5-(2,4-dihydropyrazolo[3',4':3,4]cyclopenta[1,2-b]pyridin-7-yl)-6-(3-fluoropyrrolidin-1-yl)nicotinamide and 2-chloro-5-cyanopyrimidine as the raw materials according to the method described in example 4, with a yield of 17%. LC-MS (APCI): m/z=659.6 $(M+1)^+$. $^1$H NMR (400 MHz, DMSO-$d_6$) δ 9.93 (s, 1H), 9.21 (s, 2H), 8.65 (s, 1H), 8.56 (s, 1H), 8.17 (s, 1H), 8.05 (s, 1H), 7.76 (d, J = 9.1 Hz, 2H), 7.36-7.23 (m, 5H), 5.25 (d, J = 53.8 Hz, 1H), 3.93 (s, 2H), 3.62-3.43 (m, 2H), 3.30-3.14 (m, 2H), 2.04 (d, J = 11.8 Hz, 2H).

**Example 135 Preparation of N-(4-(chlorodifluoromethoxy)phenyl)-4'-(2-(4-(pyridin-3-yl)pyrimidin-2-yl)-2,4-dihydropyrazolo[3',4':3,4]cyclooenta[1,2-b]pyridin-7-yl)-2'-oxospiro[cyclohexane-1,3'-indoline]-6'-carboxamide (compound T-135)**

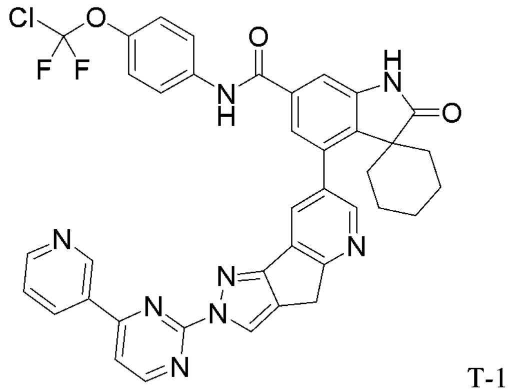
T-135

The following route was adopted for synthesis:

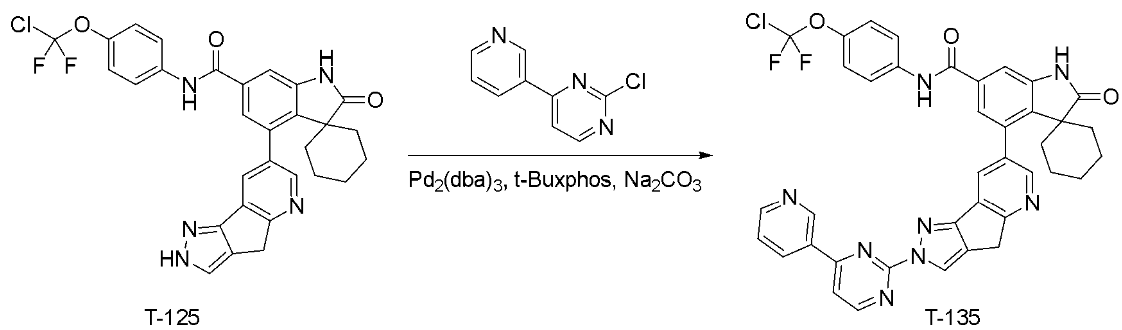

T-125 T-135

Compound T-135 was prepared using compound T-125 and 4-(3-pyridyl)-2-chloropyrimidine as the raw materials according to the preparation method in example 4, with a yield of 51%. LC-MS (APCI): m/z=731.2 $(M+1)^+$. $^1$H NMR (400 MHz, DMSO-$d_6$) δ 10.23 (s, 1H), 8.92 (d, J = 6.6 Hz, 2H), 8.64 (s, 1H), 8.27 - 8.13 (m, 3H), 7.85 - 7.71 (m, 2H),

7.44 (s, 1H), 7.40 (s, 1H), 7.39 (t, J = 9.6 Hz, 1H), 7.34 (d, J = 8.4 Hz, 2H), 6.84 (d, J = 8.4 Hz, 2H), 5.07 (s, 2H), 1.86 (m, 4H), 1.28 - 1.15 (m, 6H).

**Example 136 Preparation of N-(4-(chlorodifluoromethoxy)phenyl)-4'-(2-(4-(6-cyanopyridin-3-yl)pyrimidin-2-yl)-2,4-dihydropyrazolo[3',4':3,4]cyclopenta[1,2-b]pyridin-7-yl)-2'-oxospiro[cyclohexane-1,3'-indoline]-6'-carboxamide (compound T-136)**

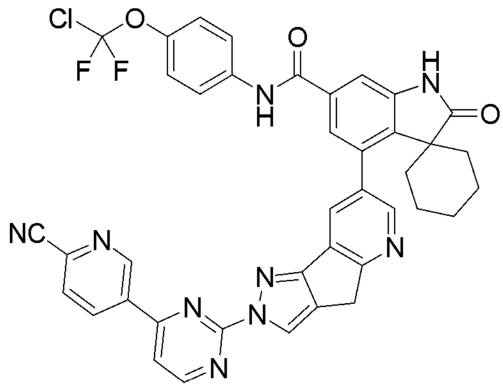

T-136

The following route was adopted for synthesis:

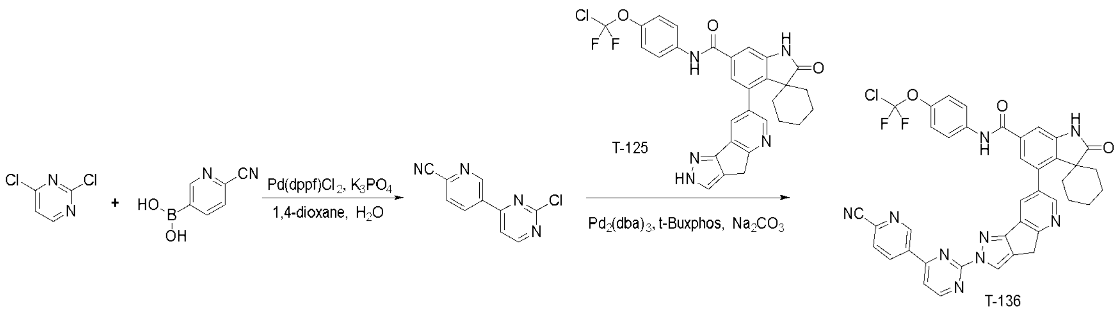


Step 1: Synthesis of compound 5-(2-chloropyrimidin-4-yl)-2-cyanopyridine

2,4-Dichloropyrimidine (1.2 g, 8.0 mmol), (6-cyanopyridin-3-yl)boronic acid (0.59 g, 4.0 mmol), potassium phosphate (2.57 g, 12.1 mmol), Pd(dppf)$Cl_2$ (0.29 g, 0.4 mmol), 15 ml of 1,4-dioxane and 3 ml of purified water were added to a reaction flask. The mixture was purged with nitrogen for 5 minutes. The mixture was heated to 100 °C, stirred and reacted under nitrogen for 6 to 8 hours. The reaction was monitored by TLC until it was completed. The mixture was concentrated to remove the solvent, and purified by silica gel column chromatography to give 430 mg of an off-white solid, with a yield of 50%. LC-MS(APCI): m/z=217.2 $(M+1)^+$.

Step 2: Synthesis of compound T-136

Compound T-136 was prepared using compound T-125 and compound 5-(2-chloropyrimidin-4-yl)-2-cyanopyridine as the raw materials according to the preparation method in example 4, with a yield of 63%. LC-MS (APCI): m/z=756.3 $(M+1)^+$. $^1$H NMR (400 MHz, DMSO-$d_6$) δ 10.23 (s, 1H), 9.22 (s, 1H), 8.60 (s, 1H), 8.28 - 8.14 (m, 3H), 7.84 (d, J = 9.8 Hz, 1H), 7.62 - 7.50 (m, 2H), 7.42 (s, 1H), 7.38 (t, J = 9.6 Hz, 1H), 7.33 (d, J = 8.4 Hz, 2H), 6.84 (d, J = 8.4 Hz, 2H), 5.04 (s, 2H), 1.83 (m, 4H), 1.26 - 1.11 (m, 6H).

**Example 137 Preparation of 4'-(2-([4,5'-bipyrimidin]-2-yl)-2,4-dihydropyrazolo[3',4':3,4]cyclopenta[1,2-b]pyridin-7-yl)-N-(4-(chlorodifluoromethoxy)phenyl)-2'-oxospiro[cyclohexane-1,3'-indoline]-6'-carboxamide (compound T-137)**

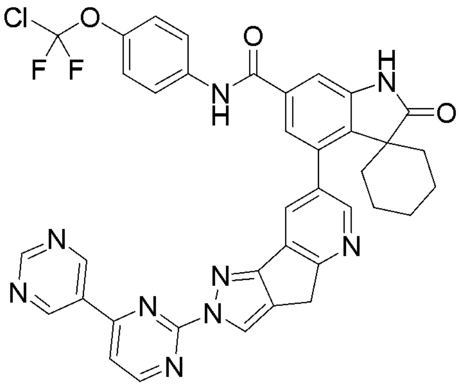

T-137

The following route was adopted for synthesis:

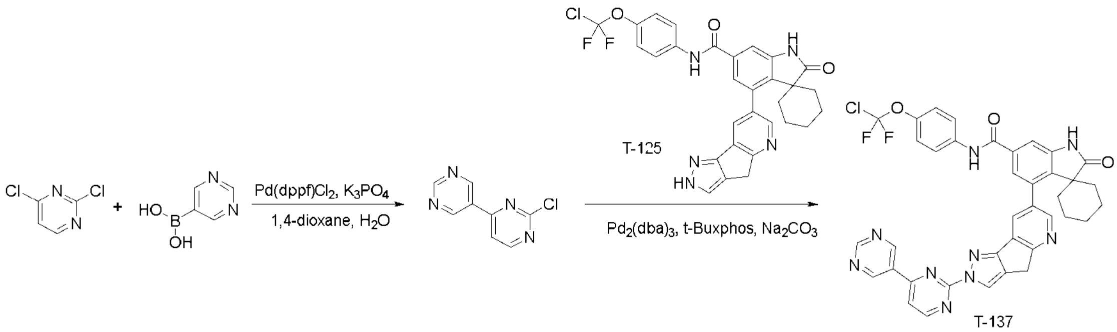


Step 1: Synthesis of compound 2-chloro-4,5'-bipyrimidine

2,4-Dichloropyrimidine (1.2 g, 8.0 mmol), pyrimidine-5-boronic acid (0.50 g, 4.0 mmol), potassium phosphate (2.57 g, 12.1 mmol), Pd(dppf)$Cl_2$ (0.29 g, 0.4 mmol), 15 ml of 1,4-dioxane and 3 ml of purified water were added to a reaction flask. The mixture was purged with nitrogen for 5 minutes. The mixture was heated to 100 °C, stirred and reacted under nitrogen for 6 to 8 hours. The reaction was monitored by TLC until it was completed. The resulting mixture was concentrated to remove the solvent, and purified by silica gel column chromatography to give 576 mg of an off-white solid, with a yield of 75%. LC-MS(APCI): m/z=193.6 $(M+1)^+$.

Step 2: Synthesis of compound T-137

Compound T-137 was prepared using compound T-125 and compound 2-chloro-4,5'-bipyrimidine as the raw materials according to the preparation method in example 4, with a yield of 37%. LC-MS (APCI): m/z=732.5 $(M+1)^+$. $^1H$ NMR (400 MHz, DMSO-$d_6$) $\delta$ 10.24 (s, 1H), 9.06 (s, 1H), 8.61 (s, 1H), 8.27 - 8.16 (m, 3H), 7.82 (d, J = 8.9 Hz, 1H), 7.68 - 7.43 (m, 2H), 7.41 (s, 1H), 7.38 (t, J = 9.6 Hz, 1H), 7.33 (d, J = 8.4 Hz, 2H), 6.84 (d, J = 8.4 Hz, 2H), 5.09 (s, 2H), 1.86 (m, 4H), 1.30 - 1.17 (m, 6H).

**<u>Example 138 Preparation of N-(4-(chlorodifluoromethoxy)phenyl)-4'-(2-(4-(1-methyl-1H-pyrazol-3-yl)pyrimidin-2-yl)-2,4-dihydropyrazolo[3',4':3,4]cyclopenta[1,2-b]pyridin-7-yl)-2'-oxospiro[cyclohexane-1,3'-indoline]-6'-carboxamide (compound T-138)</u>**

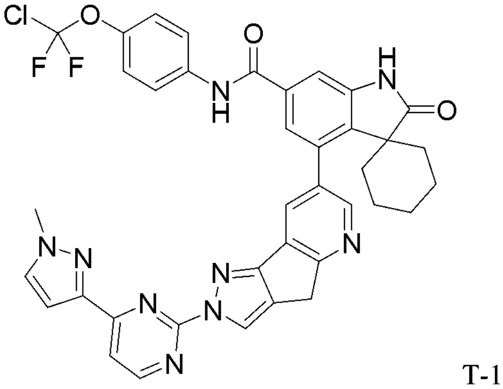

T-138

The following route was adopted for synthesis:

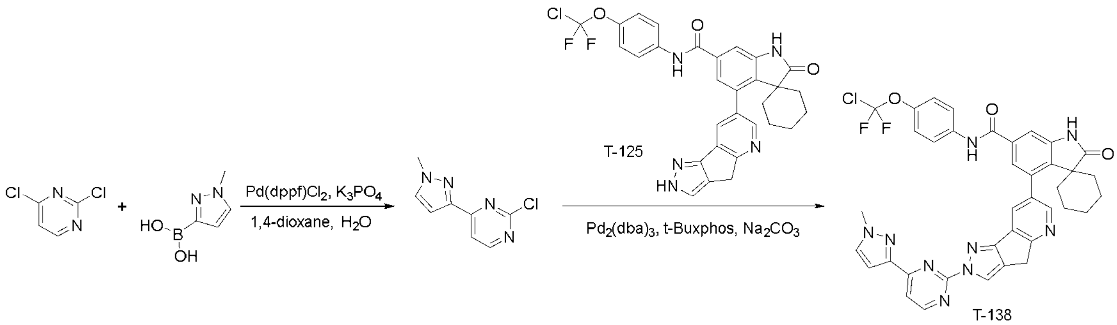


Step 1: Synthesis of compound 2-chloro-4-(1-methyl-1H-pyrazol-3-yl)pyrimidine

2,4-Dichloropyrimidine (1.2 g, 8.0 mmol), (1-methyl-1H-pyrazol-3-yl)boronic acid (0.50 g, 4.0 mmol), potassium phosphate (2.57 g, 12.1 mmol), Pd(dppf)$Cl_2$ (0.29 g, 0.4 mmol), 15 ml of 1,4-dioxane and 3 ml of purified water were added to a reaction flask. The mixture was purged with nitrogen for 5 minutes. The mixture was heated to 100 °C, stirred and reacted under nitrogen for 6 to 8 hours. The reaction was monitored by TLC until it was completed. The resulting mixture was concentrated to remove the solvent, and purified by silica gel column chromatography to give 248 mg of an off-white solid, with a yield of 32%. LC-MS(APCI): m/z=195.1 $(M+1)^+$.

Step 2: Synthesis of compound T-138

Compound T-138 was prepared using compound T-125 and compound 2-chloro-4-(1-methyl-1H-pyrazol-3-yl) pyrimidine as the raw materials according to the preparation method in example 4, with a yield of 29%. LC-MS (APCI): m/z=734.7 $(M+1)^+$. $^1$H NMR (400 MHz, DMSO-$d_6$) δ 10.23 (s, 1H), 8.98 (s, 1H), 8.64 (s, 1H), 8.27 - 8.11 (m, 3H), 7.84 (d, J = 11.5 Hz, 1H), 7.56 (m, 1H), 7.43 (s, 1H), 7.40 (s, 1H), 7.37 (t, J = 9.8 Hz, 1H), 7.35 (d, J = 8.6 Hz, 2H), 6.82 (d, J = 8.6 Hz, 2H), 5.11 (s, 2H), 1.84 (m, 4H), 1.26 - 1.10 (m, 6H).

**Example 139 Preparation of N-(4-(chlorodifluoromethoxy)phenyl)-4'-(2-(4-(6-fluoropyridin-3-yl)pyrimidin-2-yl)-2,4-dihydropyrazolo[3',4':3,4]cyclopenta[1,2-b]pyridin-7-yl)-2'-oxospiro[cyclohexane-1,3'-indoline]-6'-carboxamide (compound T-139)**

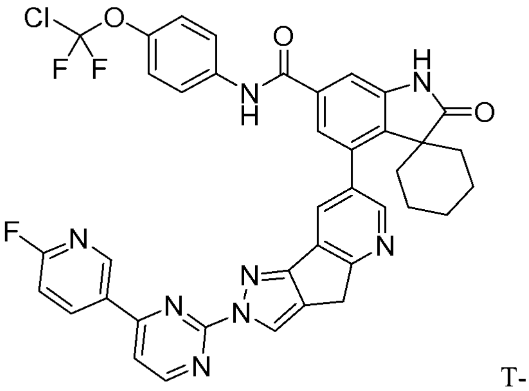

T-139

The following route was adopted for synthesis:

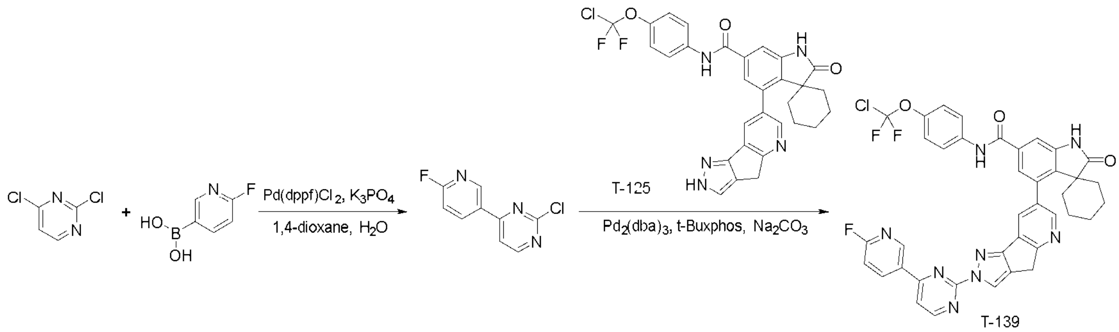


Step 1: Synthesis of compound 2-chloro-4-(6-fluoropyridin-3-yl)pyrimidine

2,4-Dichloropyrimidine (1.2 g, 8.0 mmol), (6-fluoropyridin-3-yl)boronic acid (0.56 g, 4.0 mmol), potassium phosphate (2.57 g, 12.1 mmol), Pd(dppf)$Cl_2$ (0.29 g, 0.4 mmol), 15 ml of 1,4-dioxane and 3 ml of purified water were added to a reaction flask. The mixture was purged with nitrogen for 5 minutes. The mixture was heated to 100 °C, stirred and reacted under nitrogen for 6 to 8 hours. The reaction was monitored by TLC until it was completed. The resulting mixture was concentrated to remove the solvent, and purified by silica gel column chromatography to give 443 mg of an off-white solid, with a yield of 53%. LC-MS(APCI): m/z=210.3 $(M+1)^+$.

Step 2: Synthesis of compound T-139

Compound T-139 was prepared using compound T-125 and compound 2-chloro-4-(6-fluoropyridin-3-yl)pyrimidine as the raw materials according to the preparation method in example 4, with a yield of 47%. LC-MS (APCI): m/z=749.6 $(M+1)^+$. $^1$H NMR (400 MHz, DMSO-$d_6$) δ 10.23 (s, 1H), 8.64 (s, 1H), 8.25 - 8.13 (m, 3H), 7.84 - 7.51 (m, 3H), 7.43 (s, 1H), 7.40 (s, 1H), 7.38 (t, J = 9.6 Hz, 1H), 7.33 (d, J = 8.7 Hz, 2H), 6.84 (d, J = 8.7 Hz, 2H), 5.10 (s, 2H), 1.88 (m, 4H), 1.28 - 1.11 (m, 6H).

**<u>Example 140 Preparation of N-(4-(chlorodifluoromethoxy)phenyl)-4'-(2-(4-(5-fluoropyridin-3-yl)pyrimidin-2-yl)-2,4-dihydropyrazolo[3',4':3,4]cyclopenta[1,2-b]pyridin-7-yl)-2'-oxospiro[cyclohexane-1,3'-indoline]-6'-carboxamide (compound T-140)</u>**

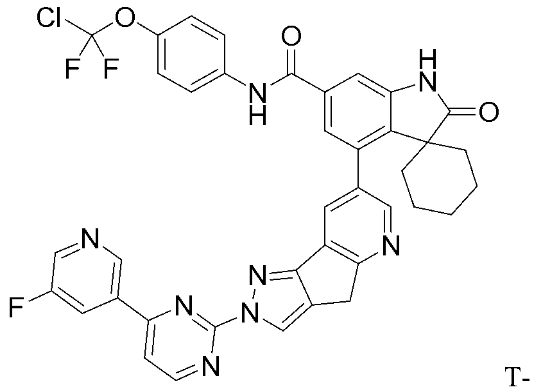

T-140

The following route was adopted for synthesis:

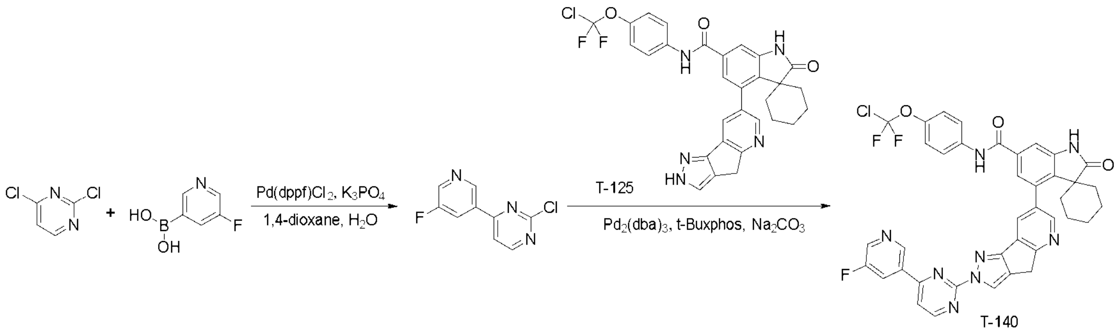


Step 1: Synthesis of compound 2-chloro-4-(5-fluoropyridin-3-yl)pyrimidine

2,4-Dichloropyrimidine (1.2 g, 8.0 mmol), (5-fluoropyridin-3-yl)boronic acid (0.56 g, 4.0 mmol), potassium phosphate (2.57 g, 12.1 mmol), $Pd(dppf)Cl_2$ (0.29 g, 0.4 mmol), 15 ml of 1,4-dioxane and 3 ml of purified water were added to a reaction flask. The mixture was purged with nitrogen for 5 minutes. The mixture was heated to 100 °C, stirred and reacted under nitrogen for 6 to 8 hours. The reaction was monitored by TLC until it was completed. The resulting mixture was concentrated to remove the solvent, and purified by silica gel column chromatography to give 343 mg of an off-white solid, with a yield of 41%. LC-MS(APCI): m/z=210.4 $(M+1)^+$.

Step 2: Synthesis of compound T-140

Compound T-140 was prepared using compound T-125 and compound 2-chloro-4-(5-fluoropyridin-3-yl)pyrimidine as the raw materials according to the preparation method in example 4, with a yield of 38%. LC-MS (APCI): m/z=749.5 $(M+1)^+$. $^1H$ NMR (400 MHz, DMSO-$d_6$) δ 10.22 (s, 1H), 8.64 (s, 1H), 8.39 - 8.31 (m, 2H), 8.24 - 8.02 (m, 3H), 7.85 (d, J = 11.5 Hz, 1H), 7.59 (s, 1H), 7.46 (s, 1H), 7.42 (s, 1H), 7.36 (d, J = 8.4 Hz, 2H), 6.83 (d, J = 8.4 Hz, 2H), 5.11 (s, 2H), 1.89 (m, 4H), 1.26 - 1.14 (m, 6H).

**Example 141 Preparation of (R)-N-(4-(chlorodifluoromethoxy)phenyl)-5-(2-(4-(6-fluoropyridin-3-yl)pyrimidin-2-yl)-2,4-dihydropyrazolo[3',4':3,4]cyclopenta[1,2-b]pyridin-7-yl)-6-(3--fluoropyrrolidin-1-yl)nicotinamide (compound T-141)**

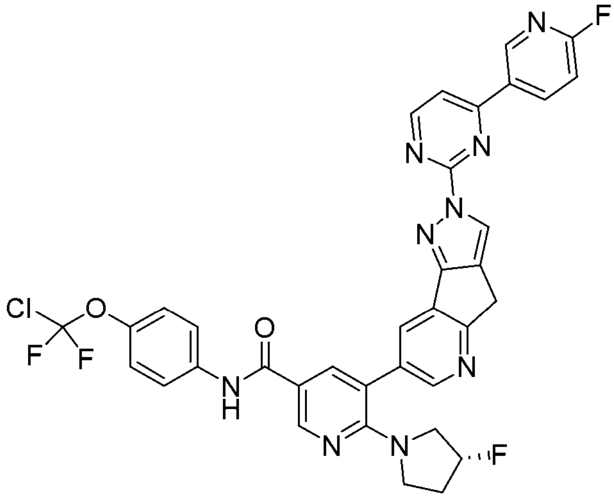

T-141

The following route was adopted for synthesis:

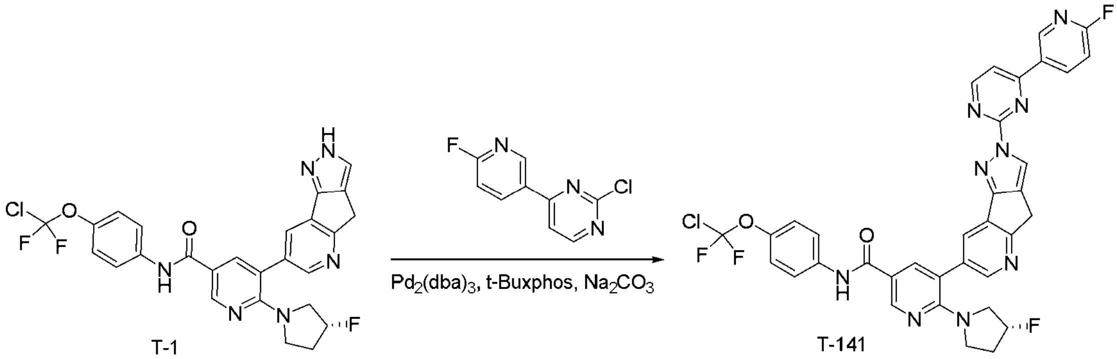


Compound T-141 was prepared using compound T-1 and compound 2-chloro-4-(6-fluoropyridin-3-yl)pyrimidine as the raw materials according to the preparation method in example 4, with a yield of 30%. LC-MS (APCI): m/z=714.3 $(M+1)^+$. $^1H$ NMR (400 MHz, DMSO-$d_6$) δ 10.22 (s, 1H), 8.93 (s, 1H), 8.68 (s, 1H), 8.42 (s, 1H), 8.31 (s, 1H), 8.20 - 8.11 (m, 2H), 7.58 (d, J = 11.2 Hz, 2H), 7.35 (d, J = 6.6 Hz, 1H), 7.27 (d, J = 6.6 Hz, 2H), 7.25 (d, J = 8.4 Hz, 2H), 6.81 (d, J = 8.4 Hz, 2H), 5.27 - 5.15 (d, J = 21.6 Hz, 1H), 4.33 (s, 2H), 3.96 (m, 1H), 3.98 - 3.74 (m, 1H), 3.53 (d, J = 8.8 Hz, 2H), 2.15 - 2.02 (m, 2H).

**Example 142 Preparation of (R)-N-(4-(chlorodifluoromethoxy)phenyl)-5-(2-(4-(5-fluoropyridin-3-yl)pyrimidin-2-yl)-2,4-dihydropyrazolo[3',4':3,4]cyclopenta[1,2-b]pyridin-7-yl)-6-(3-fluoropyrrolidin-1-yl)nicotinamide (compound T-142)**

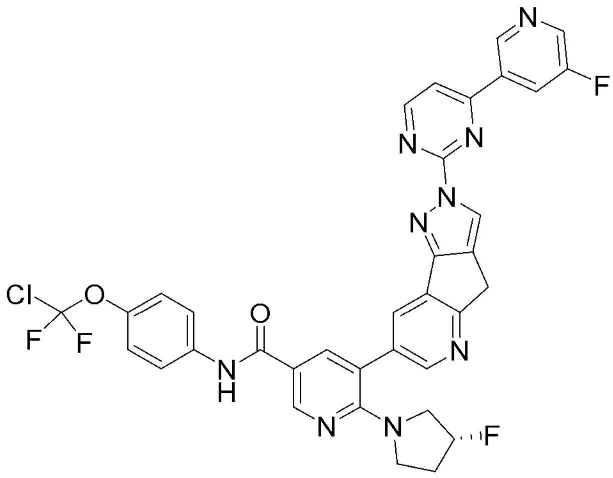

T-142

The following route was adopted for synthesis:

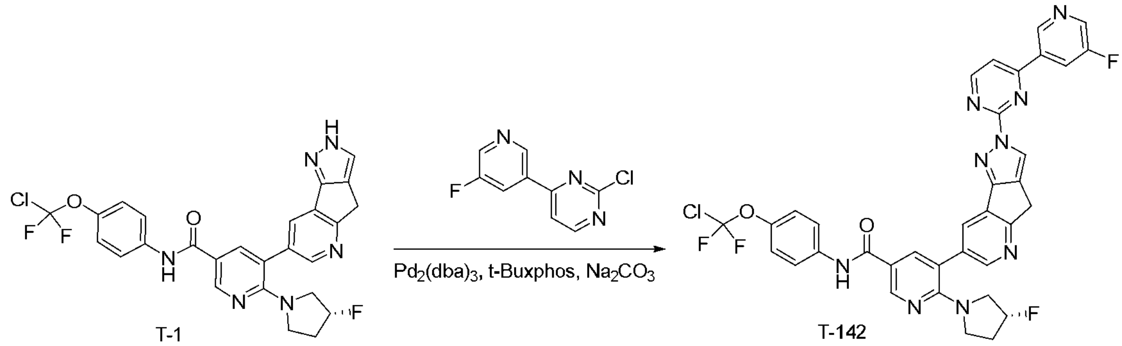


Compound T-142 was prepared using compound T-91 and compound 2-chloro-4-(5-fluoropyridin-3-yl)pyrimidine as the raw materials according to the preparation method in example 4, with a yield of 44%. LC-MS (APCI): m/z=714.7 $(M+1)^+$. $^1H$ NMR (400 MHz, DMSO-$d_6$) δ 10.24 (s, 1H), 8.91 (s, 1H), 8.66 (s, 1H), 8.41 (s, 1H), 8.31 (s, 1H), 8.22 - 8.08 (m, 3H), 7.58 (d, J = 11.2 Hz, 2H), 7.33 (s, 1H), 7.25 (d, J = 8.4 Hz, 2H), 6.81 (d, J = 8.4 Hz, 2H), 5.27 - 5.15 (d, J = 21.6 Hz, 1H), 4.32 (s, 2H), 3.94 (m, 1H), 3.91 - 3.76 (m, 1H), 3.50 (d, J = 8.6 Hz, 2H), 2.13 (dd, J = 9.5, 3.8 Hz, 2H).

**Biological activity assay**

(1) Cell growth inhibitory activity assay

In this assay, the Celltiter-Glo (CTG) kit provided by Promega was used, and it is a homogeneous cell viability assay method. The cell viability of cultured cells was measured by quantifying ATP. K562 cells were purchased from Nanjing Kebai, product number CBP60529; Ba/F3 parental cells were purchased from RIKEN BRC, product number RCB0805; other cells containing BCR-ABL1 WT, BCR-ABL1 T315I, BCR-ABL1 T315M, BCR-ABL1 Y253H/E255K, BCR-ABL1 E255V/T3151, BCR-ABL1 A424T, BCR-ABL1 A337V, BCR-ABL1 P465S, BCR-ABL1 V468F, and BCR-ABL1 I502L were all monoclonal engineered cell lines obtained by transducting Ba/F3 parental cells with the lentivirus plasmids containing WT or corresponding mutated BCR-ABL1.

The steps of cell viability assay are as follows:

a) Cells in logarithmic growth phase were collected. Cell suspension was prepared and the cells were counted. Concentration of the cells was calculated.
b) The cell suspension was diluted to the required concentration and then added to the middle wells of a 96-well plate, and PBS was added to the edge wells. The wells with no cells and only culture medium were used as blank controls.
c) In addition to the cell plate used for compound analysis, another 96-well plate was prepared as a T0 plate, and 3-6 blank wells and 3-6 cell wells were added. The next day, CTG was used to test the cell viability as the T0 viability value of the cells.
d) The cell plate was incubated at 37 °C in a 5% $CO_2$ incubator overnight.

e) The next day, each concentration of a compound subjected to a gradient dilution was added, and the cell plate was incubated at 37 °C in a 5% $CO_2$ incubator for 72 h. The wells with no compound and only 0.5% DMSO were used as positive controls for cell growth.
f) CTG reagent (CellTiter-Glo kit) was added according to the manufacturer's instructions to test the cell viability value.
g) The plate was read using Biotek cytation3.0 microplate reader and the data were exported.
h) $IC_{50}$ was calculated using GraphPad Prism 7.0 software.

The $IC_{50}$ (half-maximal inhibitory concentration) of the compound was obtained using the following nonlinear fitting formula:

$$Y=Bottom + (Top-Bottom)/(1+10^{\wedge}((LogIC_{50}-X)*HillSlope))$$

X: Log value of the compound concentration
Y: Inhibition rate (% inhibition)
Top: Maximum response
Bottom: Baseline response
HillSlope: Slope of the curve

The formula for calculating cell inhibition rate (inhibition rate %) was:

Inhibition rate % = 1- (Lum of drug to be tested - Lum of vehicle control)/(Lum of cell control - Lum of vehicle control) × 100%.

The compounds of the present disclosure were tested in the above assays, and it was found that the compounds of the present disclosure exhibiting potent activity on BCR-ABL1 WT, BCR-ABL1 T315I, BCR-ABL1 T315M, BCR-ABL1 Y253H/E255K, BCR-ABL1 E255V/T3151, BCR-ABL1 A424T, BCR-ABL1 A337V, BCR-ABL1 P465S, BCR-ABL1 V468F and BCR-ABL1 I502L cells. The above assay results of the representative example compounds are summarized in Table 1 and Table 2 below, wherein A indicates $IC_{50} \leq 5$ nM, B indicates $5 \leq IC_{50} < 50$ nM, C indicates $50 \leq IC_{50} < 1000$ nM, and D indicates $IC_{50} > 1000$ nM.

Table 1:

| Compound number T- | | Ba/F3 BCR-ABL1 | | | |
|---|---|---|---|---|---|
| | K562 | WT | T315I | T315M | Parental |
| 1 | A | A | A | | D |
| 2 | A | A | B | | D |
| 3 | A | A | A | C | D |
| 4 | A | A | A | D | D |
| 5 | A | A | A | | D |
| 6-A | A | A | A | | D |
| 6-B | A | A | B | | D |
| 7 | A | A | B | | D |
| 8 | A | A | B | | D |
| 9 | A | A | A | | D |
| 10-A | A | B | B | | D |
| 10-B | A | A | A | | D |
| 11 | A | A | C | D | D |
| 11-A | C | C | D | D | D |
| 11-B | A | A | B | C | D |

(continued)

| Compound number T- | | Ba/F3 BCR-ABL1 | | | |
|---|---|---|---|---|---|
| | K562 | WT | T315I | T315M | Parental |
| 12 | B | B | C | | D |
| 12-A | C | C | D | D | D |
| 12-B | A | A | B | C | D |
| 13 | A | A | A | | D |
| 14 | A | A | A | | D |
| 15 | A | A | B | | D |
| 16 | A | A | A | | D |
| 17 | A | A | A | | D |
| 18 | A | A | A | D | D |
| 19 | A | A | A | | D |
| 20 | A | A | B | D | D |
| 21 | A | A | B | D | D |
| 22 | A | A | A | | D |
| 23 | B | A | C | | D |
| 24 | A | B | C | | D |
| 25 | A | A | B | D | D |
| 26 | A | A | A | | D |
| 27 | A | B | C | D | D |
| 28 | A | B | B | C | D |
| 29 | A | A | B | | D |
| 30 | A | A | C | B | D |
| 31 | A | A | B | B | D |
| 32 | A | A | C | C | D |
| 33 | A | B | C | C | D |
| 34 | A | A | A | | D |
| 35 | A | A | B | B | D |
| 36 | A | A | D | | D |
| 37 | C | D | D | D | D |
| 38 | A | A | A | | D |
| 39 | A | A | C | D | D |
| 40 | A | A | D | | D |
| 42 | A | A | B | C | D |
| 43 | A | A | A | C | D |
| 44 | A | A | A | | D |
| 45 | A | A | A | | D |
| 46 | A | A | A | | D |
| 47 | A | A | A | | D |
| 48 | A | A | A | D | D |

(continued)

| Compound number T- | | Ba/F3 BCR-ABL1 | | | |
|---|---|---|---|---|---|
| | K562 | WT | T315I | T315M | Parental |
| 49 | A | A | A | C | D |
| 50 | A | A | B | | D |
| 51 | A | B | | | D |
| 52 | A | A | A | D | D |
| 53 | A | A | A | | D |
| 54 | A | A | A | D | D |
| 55 | A | A | B | | D |
| 56-A | B | C | D | | D |
| 56-B | A | A | D | | D |
| 57 | A | A | A | D | D |
| 58 | A | A | B | | D |
| 59 | A | A | C | | D |
| 60 | A | A | D | | D |
| 61 | A | A | B | | D |
| 62 | A | B | C | | D |
| 63 | A | A | D | | D |
| 64 | A | A | D | | D |
| 65 | A | A | A | A | D |
| 66 | A | A | B | D | D |
| 67 | A | A | A | | D |
| 68 | A | A | A | A | D |
| 69-A | A | A | B | C | D |
| 69-B | A | A | A | A | D |
| 70 | A | A | B | | D |
| 71 | A | A | A | B | D |
| 72 | A | A | A | | D |
| 73 | A | A | A | A | D |
| 74 | A | A | A | | D |
| 75 | A | A | A | | D |
| 76 | A | A | A | A | D |
| 77 | A | A | A | | D |
| 78 | A | A | A | | D |
| 79 | A | A | A | | D |
| 80 | A | A | A | | D |
| 81 | A | A | A | | D |
| 82-A | A | A | B | | D |
| 82-B | A | A | A | | D |
| 83 | A | A | A | | D |

(continued)

| Compound number T- | | Ba/F3 BCR-ABL1 | | | |
|---|---|---|---|---|---|
| | K562 | WT | T315I | T315M | Parental |
| 84 | A | A | A | | D |
| 85 | A | A | A | | D |
| 86 | A | A | A | | D |
| 87 | A | A | A | | D |
| 88 | A | A | A | | D |
| 89 | A | A | A | | D |
| 90 | A | A | A | | D |
| 91-A | A | B | C | | D |
| 91-B | A | A | A | | D |
| 92 | A | A | A | | D |
| 93 | A | A | A | | D |
| 94 | A | A | A | | D |
| 95 | A | A | A | | D |
| 96 | A | A | A | | D |
| 97 | A | A | A | | D |
| 98 | A | A | A | | D |
| 99 | A | A | A | | D |
| 100 | A | A | A | | D |
| 101 | A | A | A | | D |
| 102 | A | A | A | | D |
| 104-A | A | A | B | | D |
| 104-B | A | A | B | | D |
| 105 | A | A | A | | |
| 106 | A | A | A | | |
| 107 | A | A | A | | |
| 108 | A | A | D | | D |
| 109 | A | A | A | | D |
| 110 | A | A | B | | D |
| 111 | A | A | B | | D |
| 112 | A | A | B | | D |
| 113 | A | A | B | | D |
| 114 | A | A | B | D | D |
| 115 | A | A | B | D | D |
| 116 | A | A | B | | D |
| 117 | A | A | B | D | D |
| 118 | A | A | B | C | D |
| 119 | A | A | B | | D |
| 120 | A | A | A | | D |

(continued)

| Compound number T- | | Ba/F3 BCR-ABL1 | | | |
|---|---|---|---|---|---|
| | K562 | WT | T315I | T315M | Parental |
| 121 | A | A | A | | D |
| 122 | A | A | B | | D |
| 123 | A | A | A | | D |
| 124 | A | A | A | | D |
| 125 | A | A | B | | D |
| 126 | A | A | A | | D |
| 127 | A | A | A | | D |
| 128 | A | A | A | | D |
| 129 | A | A | A | | D |
| 130 | A | A | A | | D |
| 131 | B | C | C | | D |
| 132 | A | A | A | | C |
| 133 | A | A | A | | D |
| 134 | A | A | C | | C |
| 138 | A | A | A | | |
| 139 | A | A | A | | |
| 140 | A | A | A | | |
| 141 | A | A | A | | |
| 142 | A | A | A | | |

Table 2:

| Compound number T- | Ba/F3 BCR-ABL1 | | | | | | |
|---|---|---|---|---|---|---|---|
| | Y253H/E255K | E255V/T315I | A424T | A337V | P465S | V468F | I502L |
| 2 | | B | C | C | C | C | C |
| 3 | A | A | B | B | B | B | B |
| 4 | A | B | B | C | C | C | B |
| 7 | | B | D | D | D | C | C |
| 11 | C | C | C | | | D | |
| 18 | A | A | B | B | B | B | B |
| 20 | C | B | C | C | C | C | B |
| 21 | B | B | C | C | C | C | B |
| 25 | B | B | B | | | | |
| 31 | B | B | B | C | C | B | C |
| 42 | C | C | C | C | C | C | C |
| 48 | A | A | B | B | C | B | B |
| 49 | A | A | A | B | B | B | B |
| 52 | B | A | B | C | C | B | B |
| 54 | A | A | B | B | B | B | B |

(continued)

| Compound number T- | Ba/F3 BCR-ABL1 | | | | | | |
|---|---|---|---|---|---|---|---|
| | Y253H/E255K | E255V/T315I | A424T | A337V | P465S | V468F | I502L |
| 57 | B | A | B | B | C | B | B |
| 65 | A | A | A | A | B | B | A |
| 66 | C | C | B | C | C | C | C |
| 67 | A | | | | | | |
| 68 | | | A | B | B | B | A |
| 69-A | B | B | C | C | C | D | C |
| 69-B | B | A | B | C | C | C | A |
| 71 | A | A | B | C | C | C | B |
| 76 | A | A | A | C | C | C | B |
| 78 | A | A | | | | | |
| 114 | B | C | | | | | |
| 115 | B | B | C | C | C | C | C |

(2) Metabolic stability evaluation

Metabolic stability is generally used to describe the rate and degree of compounds being metabolized, and is one of the main factors affecting pharmacokinetic properties. Many compounds are substrates of CYP450 enzymes and other drug metabolizing enzymes, and liver microsomes are systems rich in CYP450. The purpose of this assay is to study the in vitro metabolic stability by incubating the compounds of the present disclosure with human and SD rat liver microsomes respectively and detecting the remaining proportion of the compounds by LC-MS/MS.

1) Preparation of solutions

Phosphate-buffered saline (PBS): 150mL of pre-prepared $K_2HPO_4$ (0.5 M) solution was mixed with 700 mL of $K_2HPO_4$ (0.5 M) solution; $K_2HPO_4$ (0.5 M) solution was then used to adjust the pH value of the mixed solution to 7.4. The mixture was used as 5-fold concentration of PBS, and stored at 4 °C for later use. The mixture was diluted 5 times with ultrapure water before use, and 3.3 mM magnesium chloride was added to give the phosphate-buffered saline PBS (100 mM).

NADPH regeneration system solution: A NADPH solution containing 6.5 mM NADP, 16.5 mM G-6-P, and 3U/mL G-6-P D was prepared with 5 mL of PBS.

Internal standard stop solution: 50 ng/mL propranolol hydrochloride and 200 ng/mL tolbutamide were prepared with acetonitrile as an internal standard working solution.

Human liver microsome solution: 0.31 mL of human liver microsomes (25mg/mL) were added to 0.961 mL of PBS (pH7.4) and mixed well to give a human liver microsome dilution with a protein concentration of 0.625 mg/mL.

SD rat liver microsome solution: 0.31 mL of SD rat liver microsomes (25 mg/mL) were added to 0.961 mL of PBS (pH7.4) and mixed well to give SD rat liver microsome dilution with a protein concentration of 0.625 mg/mL.

Sample working solution: The powder of a compound and a non-deuterated compound of the present disclosure, the positive control dextromethorphan powder and omeprazole powder were prepared with DMSO to 10 mM as the sample stock solution, and then diluted with 70% acetonitrile-water to give a 0.25 mM sample working solution.

2) Sample incubation

398 μL of human liver microsome dilution was added to a 96-well incubation plate (N = 2); 2 μL of 0.25 mM test compound and dextromethorphan were added respectively, and mixed well.

398 μL of SD rat liver microsome dilution was added to a 96-well incubation plate (N = 2); 2 μL of 0.25 mM test compound and omeprazole were added respectively, and mixed well.

300 μL of pre-cooled stop solution per well was added to a 96-well deep well plate as a stop plate and placed on ice.

The 96-well incubation plate and the NADPH regeneration system were placed in a 37 °C water bath, shaken at

100 rpm, and pre-incubated for 5 minutes. 80μL of incubation solution was taken out from each well of the incubation plate, added to the stop plate, and mixed well. 20μL of NADPH regeneration system solution was supplemented, as a 0 min sample. Then 80μL of NADPH regeneration system solution was added to each well of the incubation plate. The reaction was initiated, and timing was started. The reaction concentration of the compound to be tested was 1μM, and the protein concentration was 0.5 mg/mL.

At 10, 30, and 90 minutes of the reaction, respectively, 100 μL of the reaction solution was taken and added to the stop plate, and vortexed for 3 minutes to stop the reaction.

The stop plate was centrifuged at 5000rpm and 4 °C for 15 minutes. 200μL of the supernatant was added into a 96-well plate pre-added with 200μL of ultrapure water, and mixed well. LC-MS/MS was used for sample analysis, and 10 μL of a sample was injected.

3) Sample analysis method

In this assay, LC-MS/MS system was used to detect the peak areas of the compound to be tested, dextromethorphan, omeprazole and internal standard, and the ratio of the peak areas of the compound to the internal standard was calculated.

4) Data processing

The peak areas of the sample and internal standard were obtained by mass spectrometer and Analyst software. The substrate elimination rate constant K was obtained by plotting the residual amount of compound (R%) against time using the single exponential degradation model of Graphpad prism7.0 software.

$$Ct/C0= \exp(-K*t)$$

The half-life $T_{1/2}$ and intrinsic clearance rate $CL_{int}$ were calculated according to the following formula, where V/M is equal to 1/C (protein).

$$T_{1/2}= -\frac{0.693}{\text{Slope}},\ CL_{int} = \frac{0.693}{t_{1/2}} \cdot \frac{V}{M},\ t_{1/2}(\text{min});\ CL_{int}(\mu L/\text{min}/\text{mg}).$$

The assay results show that the compounds of the present disclosure have excellent metabolic stability.

(3) Pharmacokinetic assays in rats

For each test drug, six male Sprague-Dawley rats, 7-8 weeks old, weighing about 210g, were divided into two groups, with three rats in each group. A single dose of the compound (oral 10mg/kg) was administered intravenously or orally. The pharmacokinetic differences were compared.

Rats were fed on standard diet and given water. Fasting started 16 hours before the test. The drug was dissolved with PEG400 and dimethyl sulfoxide. Blood was collected from the orbit. The time points of blood collection were 0.083 hours, 0.25 hours, 0.5 hours, 1 hour, 2 hours, 4 hours, 6 hours, 8 hours, 12 hours and 24 hours after administration.

The rats were briefly anesthetized after inhaling ether, and a 300μL blood sample was collected from the orbit in the test tube. There was 30μL of 1% heparin salt solution in the test tube. Before use, the test tube was dried at 60 °C overnight. After the blood sample collection was completed at the last time point, the rats were sacrificed after ether anesthesia.

Immediately after the blood sample was collected, the test tube was gently inverted at least 5 times to ensure sufficient mixing and placed on ice. The blood sample was centrifuged at 4 °C at 5000 rpm for 5 minutes to separate the plasma from the red blood cells. A pipette was used to add 100 μL of the plasma into a clean plastic centrifuge tube. The name of the compound and time point were marked. The plasma was stored at -80 °C before analysis. The concentration of the compound of the present disclosure in the plasma was determined by LC-MS/MS. The pharmacokinetic parameters were calculated based on the plasma drug concentration of each animal at different time points.

Assay results show that, the compounds of the present disclosure have better pharmacokinetic properties in animals, and thus have better pharmacodynamics and therapeutic effects.

The above content is a further detailed description of the present disclosure in conjunction with specific alternative embodiments, and it cannot be considered that the specific implementation of the present disclosure is limited to these descriptions. For those skilled in the art to which the present disclosure belongs, some simple deductions or substitutions can be made without departing from the concept of the present disclosure, and should be regarded as belonging to the scope of protection of the present disclosure.

## Claims

**1.** A compound of formula (I), or a tautomer, a stereoisomer, a prodrug, a crystal form, a pharmaceutically acceptable salt, a hydrate or a solvate thereof:

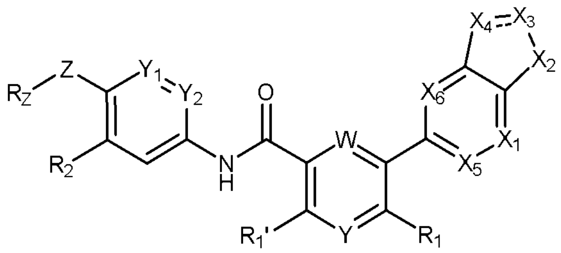

(I)

wherein

$X_1$ is N or $CR_{X1}$;
$X_2$ is $CR_{X2}R_{X2'}$ or C(O);
$X_3$ is $CR_{X3}R_{X3'}$, $CR_{X3}$ or $NR_{X3}$;
$X_4$ is $CR_{X4}R_{X4'}$, $CR_{X4}$, $NR_{X4}$ or C(O);
$X_5$ is N or $CR_{X5}$;
$X_6$ is N or $CR_{X6}$;
- - - - is single or double bonds;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with one or more $R_a$;
$R_{X4}$ and $R_{X4'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more $R_a$;
or, $R_{X3}$, $R_{X4}$ and the atoms to which they are attached are taken together to form $C_{3-8}$ cycloalkyl, 4- to 10-membered heterocyclyl, $C_{6-10}$ aryl or 5- to 10-membered heteroaryl; wherein the above groups are optionally substituted with one or more R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen;
Y is $CR_Y$ or N;
$R_1$ is 3- to 10-membered heterocyclyl, which is optionally substituted with one or more $R_b$;
Ry is H, D or halogen;
or, $R_1$, $R_Y$ and the carbon atoms to which they are attached are taken together to form $C_{3-8}$ cycloalkyl, 4- to 10-membered heterocyclyl, $C_{6-10}$ aryl or 5- to 10-membered heteroaryl, which is optionally substituted with one or more R*;
$R_1$' is H, D, halogen or $-NH_2$;
W is $CR_W$ or N;
$R_W$ is selected from H, D, halogen and $-NH_2$;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl; wherein the above groups are optionally substituted with one or more $R_c$;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
each of $R_a$, $R_b$ and $R_c$ is independently H, D, halogen, -CN, $-OR_A$, $-NR_BR_C$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl;

wherein the above groups are optionally substituted with one or more D, up to fully deuterated;
each R is independently H, D, halogen, -CN, $-OR_A$, $-C(O)R_A$, $-NR_BR_C$, $-NR_AC(O)R_B$, $-S(O)_2R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with one or more R';
each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with one or more R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with one or more R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more R";
or two adjacent R' and the atoms to which they are attached are taken together to form $C_{6-10}$ aryl or 5- to 10-membered heteroaryl; wherein the above groups are optionally substituted with one or more -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated;
with the proviso that, when $X_1$ is N, $X_2$ is $CH_2$, $X_3$ is $NR_{X3}$, $X_4$ is C(O), and $X_5$ is CH, Y is not N.

**2.** The compound according to claim 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$ or C(O);
$X_3$ is $CR_{X3}R_{X3'}$ or $NR_{X3}$;
$X_4$ is C(O);
$X_5$ is N or $CR_{X5}$;
$X_6$ is N or $CR_{X6}$, alternatively $CR_{X6}$;
- - - - is single bond;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with 1, 2, 3, 4, 5 or 6 $R_a$;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{3-8}$ cycloalkyl, 4- to 10-membered heterocyclyl, $C_{6-10}$ aryl or 5- to 10-membered heteroaryl, which is optionally substituted with 1, 2, 3, 4, 5 or 6 R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl; wherein the above groups are optionally substituted with one or more D, up to fully deuterated;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;

or -Z-$R_Z$ together represents -$SF_5$;
each $R_a$ is independently H, D, halogen, -CN or -$NR_BR_C$;
each R* is independently H, D, halogen, -CN, -OH, oxo, -$NR_AC(O)R_B$, $C_{1-6}$ alkyl or $C_{1-6}$ haloalkyl, or two R* and the atom(s) to which they are attached are taken together to form $C_{3-8}$ cycloalkyl or 4- to 10-membered heterocyclyl; wherein the above groups are optionally substituted with 1, 2, 3, 4, 5 or 6 R';
wherein $R_A$, $R_B$ and $R_C$ are independently H, D, $C_{1-6}$ alkyl or $C_{1-6}$ haloalkyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated.

**3.** The compound according to claim 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single or double bonds;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1, 2, 3, 4, 5 or 6 R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is $CR_Y$ or N;
$R_1$ is 3- to 10-membered heterocyclyl, which is optionally substituted with 1, 2, 3, 4, 5 or 6 $R_b$;
Ry is H, D or halogen;
or, $R_1$, $R_Y$ and the carbon atoms to which they are attached are taken together to form $C_{3-8}$ cycloalkyl, 4- to 10-membered heterocyclyl, $C_{6-10}$ aryl or 5- to 10-membered heteroaryl, which is optionally substituted with 1, 2, 3, 4, 5 or 6 R*;
$R_1$' is H, D, halogen or -$NH_2$, alternatively H or D;
W is $CR_W$ or N, alternatively $CR_W$;
$R_W$ is selected from H, D, halogen and -$NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, -$NO_2$, $C_{1-6}$ alkyl or $C_{1-6}$ haloalkyl; wherein the above groups are optionally substituted with one or more D, up to fully deuterated;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or -Z-$R_Z$ together represents -$SF_5$;
each $R_b$ is independently H, D, halogen, -CN, -$OR_A$, -$NR_BR_C$, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
each R is independently H, D, halogen, -CN, -$OR_A$, -$C(O)R_A$, -$NR_BR_C$, -$NR_AC(O)R_B$, -$S(O)_2R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
each R* is independently H, D, halogen, -CN, -OH, oxo, -$NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered

heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**4.** The compound according to claim 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$ or C(O);
$X_3$ is $CR_{X3}R_{X3'}$ or $NR_{X3}$;
$X_4$ is C(O);
$X_5$ is N or $CR_{X5}$;
$X_6$ is N or $CR_{X6}$, alternatively $CR_{X6}$;
- - - - is single bond;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with 1, 2, 3, 4, 5 or 6 $R_a$;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5- to 10-membered heteroaryl, which is optionally substituted with two or more R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
each $R_a$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
each R* is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**5.** The compound according to claim 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$ or C(O);
$X_3$ is $NR_{X3}$;
$X_4$ is C(O);
$X_5$ is N or $CR_{X5}$;
$X_6$ is N or $CR_{X6}$, alternatively $CR_{X6}$;
- - - - is single bond;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$ is H, $C_{1-6}$ alkyl, $C_{1-6}$ haloalkyl or $C_{3-6}$ cycloalkyl; wherein the above groups are optionally substituted with one or more D, up to fully deuterated;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5- to 10-membered heteroaryl, which is optionally substituted with two or more R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated.

**6.** The compound according to claim 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$;
$X_3$ is $NR_{X3}$;
$X_4$ is C(O);
$X_5$ is N or $CR_{X5}$;
$X_6$ is N or $CR_{X6}$, alternatively $CR_{X6}$;
- - - - is single bond;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$ is H, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;

Y is CRy;
and $R_1$, $R_Y$ and the carbon atoms to which they are attached are taken together to form $C_{6-10}$ aryl or 5- to 10-membered heteroaryl, which is optionally substituted with two or more R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated.

**7.** The compound according to claim 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single or double bonds;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3'}$ $R_{X4}$ and the atoms to which they are attached are taken together to form 5-membered heteroaryl; which is optionally substituted with 1, 2, 3, 4, 5 or 6 R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is $CR_Y$ or N;
$R_1$ is 3- to 10-membered heterocyclyl, which is optionally substituted with 1, 2, 3, 4, 5 or 6 $R_b$;
Ry is H, D or halogen;
or, $R_1$, $R_Y$ and the carbon atoms to which they are attached are taken together to form $C_{6-10}$ aryl or 5- to 10-membered heteroaryl, which is optionally substituted with 1, 2, 3, 4, 5 or 6 R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
each $R_b$ is independently H, D, halogen, -CN, -OH, $-NH_2$, $-NHC_{1-6}$ alkyl, $-N(C_{1-6}\ alkyl)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;

each R is independently H, D, halogen, -CN, -OH, -C(O)$R_A$, -N$R_B$$R_C$, -N$R_A$C(O)$R_B$, -S(O)$_2$$R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';

wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';

each R* is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1, 2, 3, 4, 5 or 6 R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";

each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**8.** The compound according to claim 1, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3'}$ $R_{X4}$ and the atoms to which they are attached are taken together to form 5-membered heteroaryl; which is optionally substituted with 1, 2, 3, 4, 5 or 6 R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is $CR_Y$ or N;
$R_1$ is 5- to 6-membered heterocyclyl, which is optionally substituted with 1, 2, 3, 4, 5 or 6 $R_b$;
$R_1$' is H, D, halogen or -$NH_2$, alternatively H or D;
W is $CR_W$ or N, alternatively $CR_W$;
$R_W$ is selected from H, D, halogen and -$NH_2$, alternatively H or D;
Ry is H, D or halogen;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, -$NO_2$, $C_{1-6}$ alkyl or $C_{1-6}$ haloalkyl;
Z is a chemical bond, O or S(O)$_{0-2}$;
$R_Z$ is H, D, $C_{1-6}$ alkyl or $C_{1-6}$ haloalkyl; wherein the above groups are optionally substituted with one or more D, up to fully deuterated;
or -Z-$R_Z$ together represents -$SF_5$;
each $R_b$ is independently H, D, halogen, -CN, -OH, -$NH_2$, -NH$C_{1-6}$ alkyl, -N($C_{1-6}$ alkyl)$_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
each R is independently H, D, halogen, -CN, -OH, -C(O)$R_A$, -N$R_B$$R_C$, -N$R_A$C(O)$R_B$, -S(O)$_2$$R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered

heteroaryl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**9.** The compound according to claim 1, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X2}$ and $R_{X2'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1, 2, 3, 4, 5 or 6 R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5- to 10-membered heteroaryl, which is optionally substituted with two or more R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
each R is independently H, D, halogen, -CN, $-C(O)R_A$, $C_{1-6}$ alkyl or $C_{1-6}$ haloalkyl; wherein the above groups are optionally substituted with one or more D, up to fully deuterated;
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated;
$R_A$ is H, D, $C_{1-6}$ alkyl, $C_{1-6}$ haloalkyl, $C_{3-6}$ cycloalkyl or 3- to 7-membered heterocyclyl.

**10.** The compound according to claim 1, or a tautomer, a stereoisomer, a prodrug, a crystal form, a pharmaceutically acceptable salt, a hydrate or a solvate thereof, wherein

$X_1$ is N or $CR_{X1}$N;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;

$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1, 2, 3, 4, 5 or 6 R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5- to 10-membered heteroaryl, which is optionally substituted with two or more R*;
$R_1$' is H, D, halogen or -$NH_2$, alternatively H or D;
W is $CR_W$ or N, alternatively $CR_W$;
$R_W$ is selected from H, D, halogen and -$NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, -$NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or -Z-$R_Z$ together represents -$SF_5$;
each R is independently H, D, -C(O)$R_A$, halogen, -CN, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R'; wherein the above groups are optionally substituted with one or more D, up to fully deuterated;
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated;
$R_A$ is H, D, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated.

**11.** The compound according to claim 1, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X2}$ and $R_{X2'}$ are independently H, D, OH, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1, 2, 3, 4, 5 or 6 R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5- to 10-membered heteroaryl, which is optionally substituted with two or more R*;
$R_1$' is H, D, halogen or -$NH_2$, alternatively H or D;
W is $CR_W$ or N, alternatively $CR_W$;
$R_W$ is selected from H, D, halogen and -$NH_2$, alternatively H or D;
$Y_1$ is $CR_{Y1}$ or N;

$Y_2$ is $CR_{Y2}$ or N;
$R_{Y1}$, $R_{Y2}$ and $R_2$ are independently H, D, halogen, -CN, -$NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or -Z-$R_Z$ together represents -$SF_5$;
wherein one R is pyrimidin-2-yl, pyrimidin-4-yl, pyrazin-2-yl, triazinyl or tetrazinyl, and the other R are independently H, D, halogen, -CN, $C_{1-6}$ alkyl or $C_{1-6}$ haloalkyl; wherein the above groups are optionally substituted with 1, 2, 3, 4, 5 or 6 R';
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated;
or two adjacent R' and the atoms to which they are attached are taken together to form $C_{6-10}$ aryl or 5- to 10-membered heteroaryl; wherein the above groups are optionally substituted with 1, 2, 3, 4, 5 or 6 -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

**12.** The compound according to claim 1, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X2}$ and $R_{X2'}$ are independently H, D, halogen, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1, 2, 3, 4, 5 or 6 R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5- to 10-membered heteroaryl, which is optionally substituted with two or more R*;
$R_1$' is H, D, halogen or -$NH_2$, alternatively H or D;
W is $CR_W$ or N, alternatively $CR_W$;
$R_W$ is selected from H, D, halogen and -$NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, -$NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or -Z-$R_Z$ together represents -$SF_5$;
wherein one R is pyridin-2-yl, pyrimidin-4-yl or pyrazin-2-yl, and the other R are independently H, D, halogen, -CN, $C_{1-6}$ alkyl or $C_{1-6}$ haloalkyl; wherein the above groups are optionally substituted with 1, 2, 3, 4, 5 or 6 R';
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated.

**13.** The compound according to claim 1, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$;
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X2}$ and $R_{X2'}$ are independently H, D, halogen, OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered heteroaryl; wherein the above groups are optionally substituted with 1, 2, 3, 4, 5 or 6 R;
$R_{X5}$ is H, D, halogen, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
Y is CRy;
and $R_1$, $R_Y$ and the carbon atoms to which they are attached are taken together to form $C_{6-10}$ aryl or 5- to 10-membered heteroaryl, which is optionally substituted with two or more R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
$Y_1$ is $CR_{Y1}$ or N;
$Y_2$ is $CR_{Y2}$ or N;
$R_{Y1}$, $R_{Y2}$ and $R_2$ are independently H, D, halogen, -CN, $-NO_2$, $C_{1-6}$ alkyl or $C_{1-6}$ haloalkyl;
Z is a chemical bond, O or $S(O)_{0-2}$;
$R_Z$ is H, D, $C_{1-6}$ alkyl or $C_{1-6}$ haloalkyl; wherein the above groups are optionally substituted with one or more D, up to fully deuterated;
or $-Z-R_Z$ together represents $-SF_5$;
wherein one R is pyrimidin-2-yl, and the other R are independently H, D, halogen, -CN, $C_{1-6}$ alkyl or $C_{1-6}$ haloalkyl; wherein the above groups are optionally substituted with 1, 2, 3, 4, 5 or 6 R';
wherein two adjacent R* and the atoms to which they are attached are taken together to form $C_{3-8}$ cycloalkyl or 4- to 10-membered heterocyclyl, and the other R* are independently H, D, halogen, -CN, -OH, $C_{1-6}$ alkyl or $C_{1-6}$ haloalkyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated;
or two adjacent R' and the atoms to which they are attached are taken together to form $C_{6-10}$ aryl or 5- to 10-membered heteroaryl; wherein the above groups are optionally substituted with 1, 2, 3, 4, 5 or 6 -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

**14.** The compound according to claim 1, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$ or C(O);
$X_3$ is $CR_{X3}$;
$X_4$ is $CR_{X4}$;
$X_5$ is N or $CR_{X5}$;
$X_6$ is N or $CR_{X6}$, alternatively $CR_{X6}$;
- - - - is single bond;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$, $R_{X4}$ and the atoms to which they are attached are taken together to form $C_{6-10}$ aryl or 5- to 10-membered

heteroaryl; wherein the above groups are optionally substituted with 1, 2, 3, 4, 5 or 6 R;

$R_{X5}$ is H, D, halogen, $C_{1-6}$ alkyl or $C_{1-6}$ haloalkyl; wherein the above groups are optionally substituted with one or more D, up to fully deuterated;

$R_{X6}$ is H, D or halogen, alternatively H or D;

Y is CRy;

and $R_1$, $R_Y$ and the carbon atoms to which they are attached are taken together to form $C_{3-8}$ cycloalkyl or 4- to 10-membered heterocyclyl, which is optionally substituted with two or more R*;

$R_1$' is H, D, halogen or $-NH_2$, alternatively H or D;

W is $CR_W$ or N, alternatively $CR_W$;

$R_W$ is selected from H, D, halogen and $-NH_2$, alternatively H or D;

$Y_1$ is $CR_{Y1}$ or N;

$Y_2$ is $CR_{Y2}$ or N;

$R_{Y1}$, $R_{Y2}$ and $R_2$ are independently H, D, halogen, -CN, $-NO_2$, $C_{1-6}$ alkyl or $C_{1-6}$ haloalkyl;

Z is a chemical bond, O or $S(O)_{0-2}$;

$R_Z$ is H, D, $C_{1-6}$ alkyl or $C_{1-6}$ haloalkyl; wherein the above groups are optionally substituted with one or more D, up to fully deuterated;

or $-Z-R_Z$ together represents $-SF_5$;

R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';

wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';

each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1, 2, 3, 4, 5 or 6 R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with one or more D, up to fully deuterated.

**15.** The compound according to any one of claims 1 to 14, or a tautomer, a stereoisomer, a prodrug, a crystal form, a pharmaceutically acceptable salt, a hydrate or a solvate thereof, wherein the compound is a compound of formula (II):

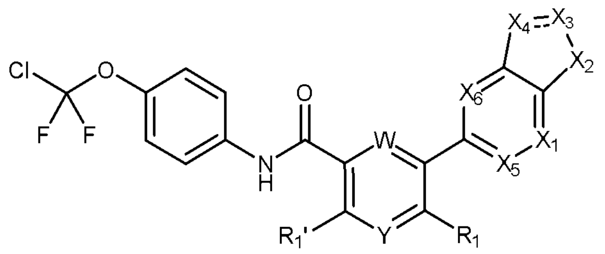

(II)

wherein $X_1$-$X_6$, Y, W, $R_1$ and $R_1$' are defined as in any one of claims 1 to 14.

**16.** The compound according to claim 15, or a tautomer, a stereoisomer, a prodrug, a crystal form, a pharmaceutically acceptable salt, a hydrate or a solvate thereof, wherein the compound is a compound of formula (III):

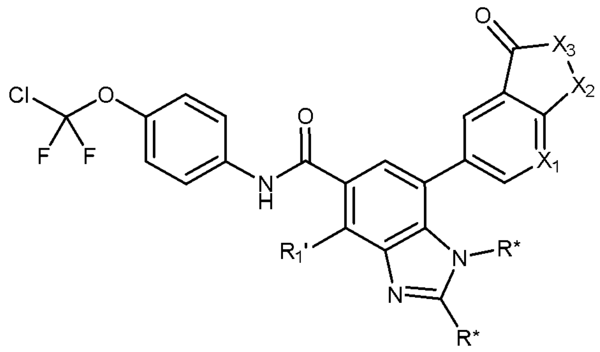

(III)

wherein

$X_1$ is N or $CR_{X1}$, alternatively N;
$X_2$ is $CR_{X2}R_{X2'}$ or C(O), alternatively $CR_{X2}R_{X2'}$;
$X_3$ is $CR_{X3}R_{X3'}$ or $NR_{X3}$;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with 1, 2, 3, 4, 5 or 6 $R_a$;
each $R_a$ is independently H, D, halogen, -CN, -OH, $-NH_2$, $-NHC_{1-6}$ alkyl, $-N(C_{1-6}\ alkyl)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
$R_1$' is H, D, halogen or $-NH_2$, alternatively H or D;
each R* is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atoms to which they are attached are taken together to form $C_{3-8}$ cycloalkyl or 4- to 10-membered heterocyclyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**17.** The compound according to claim 16, or a tautomer, a stereoisomer, a prodrug, a crystal form, a pharmaceutically acceptable salt, a hydrate or a solvate thereof, wherein

$X_1$ is N or CH;
$X_2$ is $CH_2$, CHD, $CD_2$ or C(O);
$X_3$ is $CR_{X3}R_{X3'}$ or $NR_{X3}$;
$R_{X3}$ and $R_{X3'}$ are independently H, D, halogen, $C_{1-6}$ alkyl, $C_{1-6}$ haloalkyl, $C_{3-6}$ cycloalkyl or 3- to 7-membered heterocyclyl, or $R_{X3}$, $R_{X3'}$ and the carbon atom to which they are attached are taken together to form $C_{1-6}$ alkylidene; wherein the above groups are optionally substituted with 1, 2, 3, 4, 5 or 6 $R_a$;
each $R_a$ is independently H, D, halogen, $-NH_2$, $-NHC_{1-6}$ alkyl or $-N(C_{1-6}\ alkyl)_2$; wherein the above groups are optionally substituted with one or more D, up to fully deuterated;
$R_1$' is H, D, halogen or $-NH_2$, alternatively H or D;
each R* is independently H, D, halogen, $C_{1-6}$ alkyl or $C_{1-6}$ haloalkyl, or two R* and the atoms to which they are attached are taken together to form 4- to 10-membered heterocyclyl; wherein the above groups are optionally substituted with 1, 2, 3, 4, 5 or 6 R';
each R' is independently H, D, halogen, -OH, $C_{1-6}$ alkyl or $C_{1-6}$ haloalkyl; wherein the above groups are optionally substituted with one or more D, up to fully deuterated.

**18.** The compound according to claim 16, or a tautomer, a stereoisomer, a prodrug, a crystal form, a pharmaceutically acceptable salt, a hydrate or a solvate thereof, wherein

$X_1$ is N or CH;
$X_2$ is $CH_2$ or $CD_2$;
$X_3$ is $CR_{X3}R_{X3'}$ or $NR_{X3}$;
$R_{X3}$ and $R_{X3'}$ are independently H, D, halogen, $C_{1-6}$ alkyl, $C_{1-6}$ haloalkyl or $C_{3-6}$ cycloalkyl; wherein the above groups are optionally substituted with one or more D, up to fully deuterated;
$R_1$' is H, D, halogen or $-NH_2$, alternatively H or D;
each R* is independently H, D, halogen, $C_{1-6}$ alkyl or $C_{1-6}$ haloalkyl, or two R* and the atoms to which they are attached are taken together to form 4- to 10-membered heterocyclyl; wherein the above groups are optionally substituted with 1, 2, 3, 4, 5 or 6 R';
each R' is independently H, D, halogen, -OH, $C_{1-6}$ alkyl or $C_{1-6}$ haloalkyl; wherein the above groups are optionally substituted with one or more D, up to fully deuterated.

**19.** The compound according to claim 16, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$;
$X_3$ is $CR_{X3}R_{X3'}$ or $NR_{X3}$;
$R_{X2}$ and $R_{X2'}$ are independently H, D, $C_{1-6}$ alkyl or $C_{1-6}$ haloalkyl; wherein the above groups are optionally substituted with one or more D, up to fully deuterated;
$R_{X3}$ and $R_{X3'}$ are independently H, D, halogen, $C_{1-6}$ alkyl or $C_{1-6}$ haloalkyl, or $R_{X3}$, $R_{X3'}$ and the carbon atom to which they are attached are taken together to form $C_{1-6}$ alkylidene; wherein the above groups are optionally substituted with 1, 2, 3, 4, 5 or 6 $R_a$;
each $R_a$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy or $C_{1-6}$ haloalkoxy; wherein the above groups are optionally substituted with one or more D, up to fully deuterated;
$R_1$' is H, D, halogen or $-NH_2$, alternatively H or D;
each R* is independently H, D, halogen, -CN, -OH, $C_{1-6}$ alkyl or $C_{1-6}$ haloalkyl, or two R* and the atoms to which they are attached are taken together to form $C_{3-8}$ cycloalkyl or 4- to 10-membered heterocyclyl; wherein the above groups are optionally substituted with 1, 2, 3, 4, 5 or 6 R';
each R' is independently H, D, halogen, -CN, -OH, $C_{1-6}$ alkyl or $C_{1-6}$ haloalkyl.

**20.** The compound according to claim 15, or a tautomer, a stereoisomer, a prodrug, a crystal form, a pharmaceutically acceptable salt, a hydrate or a solvate thereof, wherein the compound is a compound of formula (III-1):

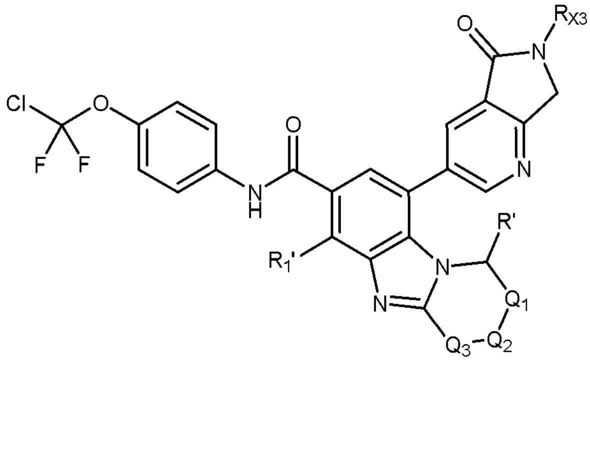

(III-1)

wherein

$R_{X3}$ is H, $C_{1-6}$ alkyl, $C_{1-6}$ haloalkyl or $C_{3-6}$ cycloalkyl; wherein the above groups are optionally substituted with one or more D, up to fully deuterated;
$R_1$' is H, D, halogen or $-NH_2$, alternatively H or D;
$Q_1$ is $C(R')_2$ or $C(R')_2C(R')_2$;
$Q_2$ is $C(R')_2$, O, NR', S, S(O) or $S(O)_2$;

$Q_3$ is $C(R')_2$, $C(R')_2C(R')_2$, O, NR', S, S(O) or $S(O)_2$;
each R' is independently H, D, halogen, -OH, $C_{1-6}$ alkyl or $C_{1-6}$ haloalkyl; wherein the above groups are optionally substituted with one or more D, up to fully deuterated.

**21.** The compound according to claim 15, or a tautomer, a stereoisomer, a prodrug, a crystal form, a pharmaceutically acceptable salt, a hydrate or a solvate thereof, wherein the compound is a compound of formula (III-2):

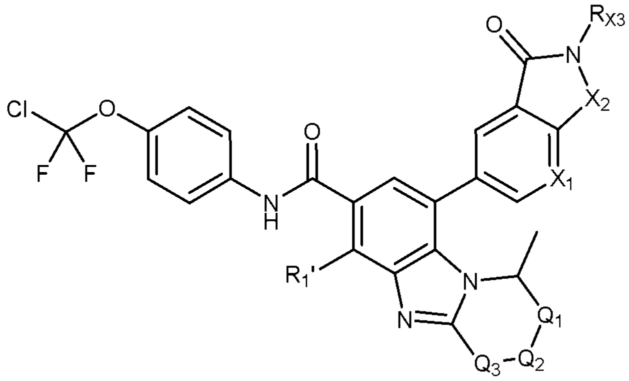

(III-2)

wherein

$X_1$ is N or $CR_{X1}$, alternatively N;
$X_2$ is $CR_{X2}R_{X2'}$ or C(O), alternatively $CR_{X2}R_{X2'}$;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
$R_{X3}$ is H, $C_{1-6}$ alkyl, $C_{1-6}$ haloalkyl or $C_{3-6}$ cycloalkyl, alternatively $C_{1-6}$ alkyl or $C_{1-6}$ haloalkyl; wherein the above groups are optionally substituted with one or more D, up to fully deuterated;
$R_1$' is H, D, halogen or $-NH_2$, alternatively H or D;
$Q_1$ is $C_{1-2}$ alkylene or $C_{1-2}$ haloalkylene; wherein the above groups are optionally substituted with one or more D, up to fully deuterated;
$Q_2$ and $Q_3$ are independently O, S, S(O), $S(O)_2$, NR', $C_{1-2}$ alkylene or $C_{1-2}$ haloalkylene, alternatively O, S, $C_{1-2}$ alkylene or $C_{1-2}$ haloalkylene; wherein the above groups are optionally substituted with 1, 2, 3 or 4 halogen or OH; wherein the above groups are also optionally substituted with one or more D, up to fully deuterated;
each R' is independently H, D, halogen, -OH, $C_{1-6}$ alkyl or $C_{1-6}$ haloalkyl.

**22.** The compound according to claim 21, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
$R_{X3}$ is Me;
$R_1$' is H, D, halogen or $-NH_2$, alternatively H or D;
$Q_1$ is $CH_2$, CHD or $CD_2$;
$Q_2$ is O, S, $CH_2$, CHD or $CD_2$;
$Q_3$ is O, S, $CH_2$, CHD or $CD_2$.

**23.** The compound according to claim 15, or a tautomer, a stereoisomer, a prodrug, a crystal form, a pharmaceutically acceptable salt, a hydrate or a solvate thereof, wherein the compound is a compound of formula (III-2a):

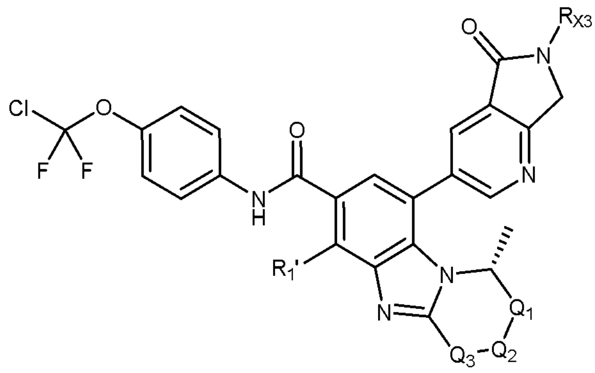

(III-2a)

wherein

$R_{X3}$ is independently H, $C_{1-6}$ alkyl, $C_{1-6}$ haloalkyl or $C_{3-6}$ cycloalkyl; wherein the above groups are optionally substituted with one or more D, up to fully deuterated;
$R_1$' is H, D, halogen or $-NH_2$, alternatively H or D;
$Q_1$ is $C(R')_2$ or $C(R')_2C(R')_2$;
$Q_2$ is $C(R')_2$, O, S, S(O) or $S(O)_2$;
$Q_3$ is $C(R')_2$, $C(R')_2C(R')_2$, O, S, S(O) or $S(O)_2$;
each R' is independently H, D, halogen, -OH, $C_{1-6}$ alkyl or $C_{1-6}$ haloalkyl; wherein the above groups are optionally substituted with one or more D, up to fully deuterated.

**24.** The compound according to claim 23, or a tautomer, a stereoisomer, a prodrug, a crystal form, a pharmaceutically acceptable salt, a hydrate or a solvate thereof, wherein

$R_{X3}$ is independently H, $C_{1-6}$ alkyl, $C_{1-6}$ haloalkyl or $C_{3-6}$ cycloalkyl; wherein the above groups are optionally substituted with one or more D, up to fully deuterated;
$R_1$' is H, D, halogen or $-NH_2$;
$Q_1$ is $CH_2$, $(CH_2)_2$, CHD, $(CHD)_2$, $CD_2$ or $(CD_2)_2$;
$Q_2$ is $CH_2$, CHD, $CD_2$, O, S, S(O) or $S(O)_2$;
$Q_3$ is $C(R')_2$, S or $S(O)_2$;
each R' is independently H, D, F or -OH.

**25.** The compound according to claim 15, or a tautomer, a stereoisomer, a prodrug, a crystal form, a pharmaceutically acceptable salt, a hydrate or a solvate thereof, wherein the compound is a compound of formula (III-3):

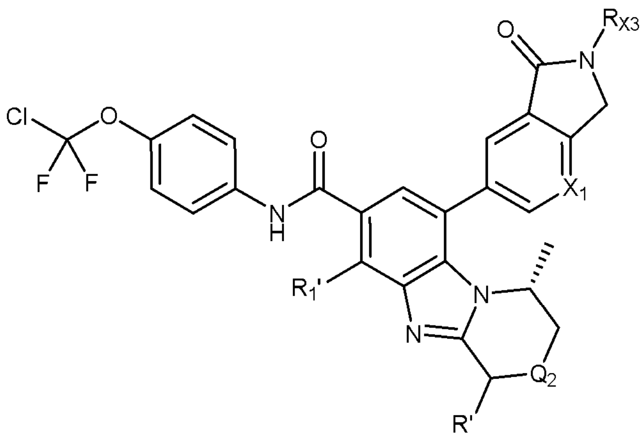

(III-3)

wherein

$X_1$ is N or $CR_{X1}$, alternatively N;
$R_{X1}$ is H, D, halogen, $C_{1-6}$ alkyl or $C_{1-6}$ haloalkyl; wherein the above groups are optionally substituted with one or

more D, up to fully deuterated;
$R_{X3}$ is H, $C_{1-6}$ alkyl, $C_{1-6}$ haloalkyl, $C_{3-6}$ cycloalkyl or 3- to 7-membered heterocyclyl, alternatively $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_1$' is H, D, halogen or $-NH_2$, alternatively H or D;
$Q_2$ is O, S or NR', alternatively O or S;
R' is H, D, halogen, OH, $C_{1-6}$ alkyl or $C_{1-6}$ haloalkyl, alternatively H, D, $C_{1-6}$ alkyl or $C_{1-6}$ haloalkyl; wherein the above groups are optionally substituted with one or more D, up to fully deuterated.

**26.** The compound according to claim 25, or a tautomer, a stereoisomer, a prodrug, a crystal form, a pharmaceutically acceptable salt, a hydrate or a solvate thereof, wherein

$X_1$ is N;
$R_{X3}$ is Me, $CD_3$ or cyclopropyl;
$R_1$' is H, D, halogen or $-NH_2$, alternatively H or D;
$Q_2$ is O;
R' is H or D.

**27.** The compound according to claim 15, or a tautomer, a stereoisomer, a prodrug, a crystal form, a pharmaceutically acceptable salt, a hydrate or a solvate thereof, wherein the compound is a compound of formula (III-3a):

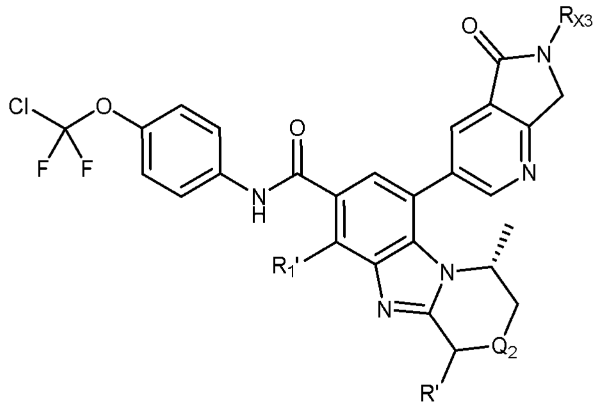

(III-3a)

wherein

$R_{X3}$ is $C_{1-6}$ alkyl, $C_{1-6}$ haloalkyl or $C_{3-6}$ cycloalkyl; wherein the above groups are optionally substituted with one or more D, up to fully deuterated;
$R_1$' is H, D, halogen or $-NH_2$, alternatively H or D;
$Q_2$ is $CH_2$, $CD_2$ or O;
R' is H, D or F.

**28.** The compound according to claim 15, or a tautomer, a stereoisomer, a prodrug, a crystal form, a pharmaceutically acceptable salt, a hydrate or a solvate thereof, wherein the compound is a compound of formula (IV):

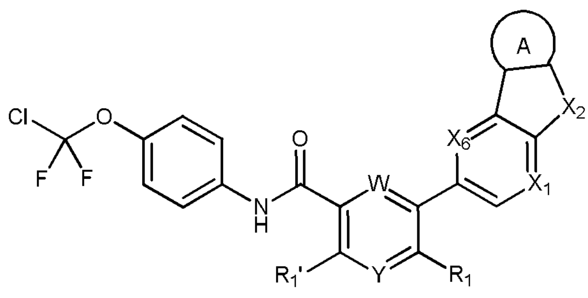

(IV)

wherein

$X_1$ is N or $CR_{X1}$;
$X_2$ is $CR_{X2}R_{X2'}$ or C(O), alternatively $CR_{X2}R_{X2'}$;
$X_6$ is N or $CR_{X6}$, alternatively $CR_{X6}$;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1, 2, 3 or 4 R;
Y is CRy or N, alternatively CRy;
$R_1$ is 3- to 10-membered heterocyclyl, which is optionally substituted with 1, 2, 3, 4, 5 or 6 $R_b$;
Ry is H, D or halogen;
or, $R_1$, $R_Y$ and the carbon atoms to which they are attached are taken together to form $C_{6-10}$ aryl or 5- to 10-membered heteroaryl, which is optionally substituted with 1, 2, 3, 4, 5 or 6 R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
each $R_b$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
each R is independently H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R* is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**29.** The compound according to claim 28, or a tautomer, a stereoisomer, a prodrug, a crystal form, a pharmaceutically acceptable salt, a hydrate or a solvate thereof, wherein ring A is

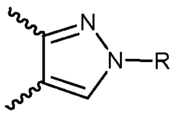
,

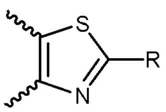 , 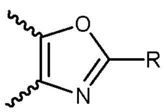 , 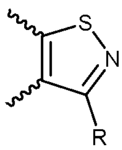 , 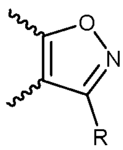 , 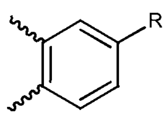 , 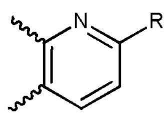 ,

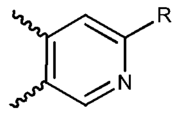

or

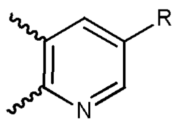

.

**30.** The compound according to claim 28, or a tautomer, a stereoisomer, a prodrug, a crystal form, a pharmaceutically acceptable salt, a hydrate or a solvate thereof, wherein

$X_1$ is N or $CR_{X1}$;
$X_2$ is $CR_{X2}R_{X2'}$;
$X_6$ is N or $CR_{X6}$, alternatively $CR_{X6}$;
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, $C_{1-6}$ alkyl or $C_{1-6}$ haloalkyl; wherein the above groups are optionally substituted with one or more D, up to fully deuterated;
$R_{X6}$ is H, D or halogen, alternatively H or D;
ring A is 5-membered heteroaryl, which is optionally substituted with 1, 2, 3 or 4 R;
Y is CRy;
$R_1$, $R_Y$ and the carbon atoms to which they are attached are taken together to form $C_{6-10}$ aryl or 5- to 6-membered heteroaryl, which is optionally substituted with 1, 2, 3, 4, 5 or 6 R*;
$R_1$' is H, D, halogen or $-NH_2$, alternatively H or D;
W is $CR_W$ or N, alternatively $CR_W$;
$R_W$ is selected from H, D, halogen and $-NH_2$, alternatively H or D;
each R is independently H, D, halogen, -CN, -OH, $-C(O)R_A$, $-NR_BR_C$, $-NR_AC(O)R_B$, $C_{1-6}$ alkyl, $C_{1-6}$ haloalkyl, $C_{1-6}$ alkoxy, $C_{1-6}$ haloalkoxy, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl or $C_{1-6}$ haloalkyl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R* is independently H, D, halogen, -CN, -OH, $C_{1-6}$ alkyl, $C_{1-6}$ haloalkyl, $C_{1-6}$ alkoxy or $C_{1-6}$ haloalkoxy, or two R* and the atom(s) to which they are attached are taken together to form $C_{3-8}$ cycloalkyl or 4- to 10-membered heterocyclyl; wherein the above groups are optionally substituted with 1, 2, 3, 4, 5 or 6 R';
each R' is independently H, D, halogen, -CN, -OH, $C_{1-6}$ alkyl or $C_{1-6}$ haloalkyl.

**31.** The compound according to claim 15, or a tautomer, a stereoisomer, a prodrug, a crystal form, a pharmaceutically acceptable salt, a hydrate or a solvate thereof, wherein the compound is a compound of formula (IV-1):

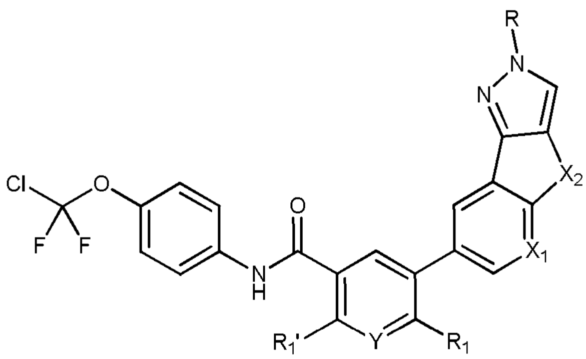

(IV-1)

wherein

$X_1$ is N or $CR_{X1}$;
$X_2$ is $CR_{X2}R_{X2'}$ or C(O);
$R_{X1}$ is H, D, halogen, $C_{1-6}$ alkyl or $C_{1-6}$ haloalkyl; wherein the above groups are optionally substituted with one or more D, up to fully deuterated;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
Y is CRy or N;
$R_1$ is 3- to 10-membered heterocyclyl, which is optionally substituted with 1, 2, 3, 4, 5 or 6 $R_b$;
Ry is H, D or halogen;
or, $R_1$, $R_Y$ and the carbon atoms to which they are attached are taken together to form $C_{6-10}$ aryl or 5- to 10-membered heteroaryl, which is optionally substituted with 1, 2, 3, 4, 5 or 6 R*;
$R_1$' is H, D, halogen or $-NH_2$, alternatively H or D;
each $R_b$ is independently H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R* is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**32.** The compound according to any one of claims 28 to 31, or a tautomer, a stereoisomer, a prodrug, a crystal form, a pharmaceutically acceptable salt, a hydrate or a solvate thereof, wherein $R_1$ is 4- to 10-membered heterocyclyl, which is optionally substituted with 1, 2, 3, 4, 5 or 6 $R_b$;
alternatively, $R_1$ is

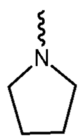, 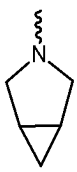, 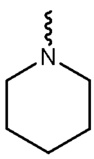, 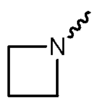, 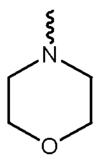, 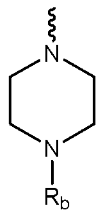, 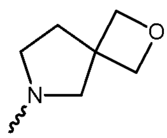,

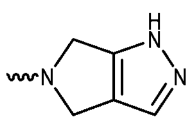, 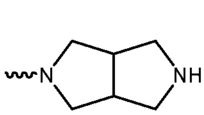, 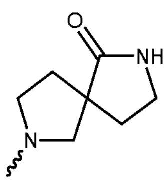

or

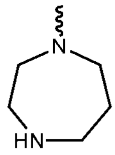

,

wherein the above groups are optionally substituted with 1, 2, 3, 4, 5 or 6 $R_b$.

**33.** The compound according to any one of claims 28 to 32, or a tautomer, a stereoisomer, a prodrug, a crystal form, a pharmaceutically acceptable salt, a hydrate or a solvate thereof, wherein $R_1$, $R_Y$ and the carbon atoms to which they are attached are taken together to form 5-membered heterocyclyl or 5-membered heteroaryl, which is optionally substituted with 1, 2, 3, 4, 5 or 6 R*;
alternatively, $R_1$, $R_Y$ and the carbon atoms to which they are attached are taken together to form

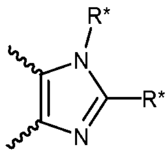

,

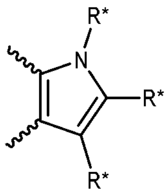

,

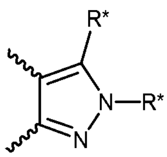

or

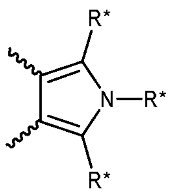

.

**34.** The compound according to claim 15, or a tautomer, a stereoisomer, a prodrug, a crystal form, a pharmaceutically acceptable salt, a hydrate or a solvate thereof, wherein the compound is a compound of formula (IV-2):

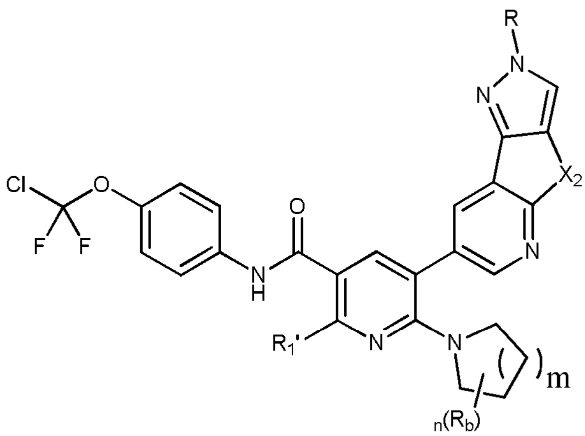

(IV-2)

wherein

$R_1$' is H, D, halogen or -$NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
m is 0, 1, 2 or 3;
n is 0, 1, 2, 3, 4 or 5;
each $R_b$ is independently H, D, halogen, -CN, -OH, -$NH_2$, -NH$C_{1-6}$ alkyl, -N($C_{1-6}$ alkyl)$_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
R is H, D, halogen, -CN, -OH, -C(O)$R_A$, -N$R_B$$R_C$, -N$R_A$C(O)$R_B$, -S(O)$_2$$R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**35.** The compound according to claim 15, or a tautomer, a stereoisomer, a prodrug, a crystal form, a pharmaceutically acceptable salt, a hydrate or a solvate thereof, wherein the compound is a compound of formula (IV-2a):

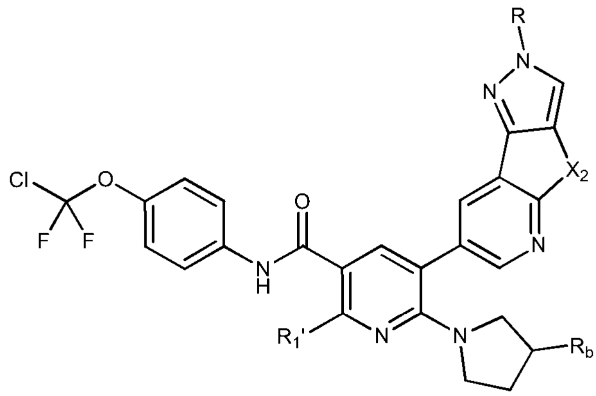

(IV-2a)

wherein

$R_1$' is H, D, halogen or -$NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_b$ is H, D, halogen, -CN, -OH, -$NH_2$, -NH$C_{1-6}$ alkyl, -N($C_{1-6}$ alkyl)$_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl; wherein the above groups are optionally substituted with one or more D, up to fully deuterated;
R is H, D, halogen, -CN, -OH, -C(O)$R_A$, -N$R_B$$R_C$, -N$R_A$C(O)$R_B$, -S(O)$_2$$R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';

each R’ is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**36.** The compound according to claim 35, or a tautomer, a stereoisomer, a prodrug, a crystal form, a pharmaceutically acceptable salt, a hydrate or a solvate thereof, wherein

$R_1$’ is H, D, halogen or $-NH_2$, alternatively H or D;
$R_b$ is H, D, halogen, -OH, $-NH_2$, $-NHC_{1-6}$ alkyl or $-N(C_{1-6}$ alkyl$)_2$; wherein the above groups are optionally substituted with one or more D, up to fully deuterated;
alternatively,
$R_b$ is halogen or -OH.

**37.** The compound according to claim 15, or a tautomer, a stereoisomer, a prodrug, a crystal form, a pharmaceutically acceptable salt, a hydrate or a solvate thereof, wherein the compound is a compound of formula (IV-2a):

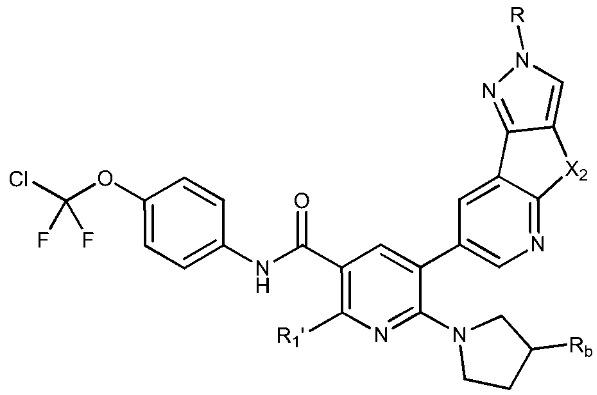
(IV-2a)

wherein

$R_1$’ is H, D, halogen or $-NH_2$, alternatively H or D;
$X_2$ is $CR_{X2}R_{X2'}$ or C(O), alternatively $CR_{X2}R_{X2'}$;
$R_{X2}$ and $R_{X2'}$ are independently H, D, halogen, -OH, $C_{1-6}$ alkyl or $C_{1-6}$ haloalkyl, alternatively H, D, $C_{1-6}$ alkyl or $C_{1-6}$ haloalkyl; wherein the above groups are optionally substituted with one or more D, up to fully deuterated;
$R_b$ is H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, alternatively H, D, halogen, -CN, -OH, $-NH_2$, $-NHC_{1-6}$ alkyl, $-N(C_{1-6}$ alkyl$)_2$, $C_{1-6}$ alkyl, $C_{1-6}$ haloalkyl, $C_{1-6}$ alkoxy or $C_{1-6}$ haloalkoxy; wherein the above groups are optionally substituted with one or more D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, $C_{1-6}$ alkyl, $C_{1-6}$ haloalkyl, $C_{6-10}$ aryl or 5- to 10-membered heteroaryl; wherein the above groups are optionally substituted with 1, 2, 3, 4, 5 or 6 R’;
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R’;
each R’ is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**38.** The compound according to claim 15, or a tautomer, a stereoisomer, a prodrug, a crystal form, a pharmaceutically acceptable salt, a hydrate or a solvate thereof, wherein the compound is a compound of formula (IV-2b):

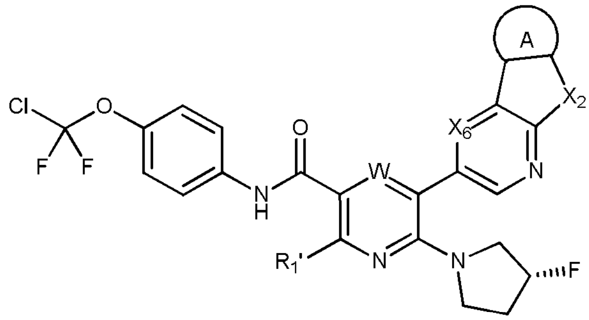

(IV-2b)

wherein

W is $CR_W$ or N;
$R_W$ is selected from H, D, halogen and $-NH_2$, alternatively H or D;
$R_1'$ is H, D, halogen or $-NH_2$, alternatively H or D;
$X_2$ is $CR_{X2}R_{X2'}$ or C(O);
$X_6$ is N or $CR_{X6}$, alternatively $CR_{X6}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1, 2, 3 or 4 R;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**39.** The compound according to claim 38, or a tautomer, a stereoisomer, a prodrug, a crystal form, a pharmaceutically acceptable salt, a hydrate or a solvate thereof, wherein ring A is

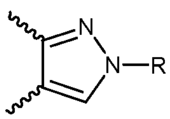
,

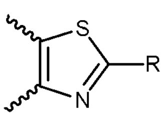
,
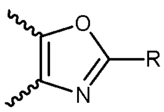
,
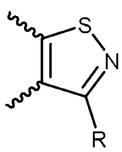
,
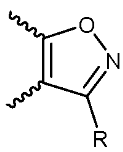
,
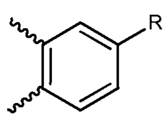
,
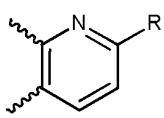
,

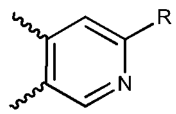

or

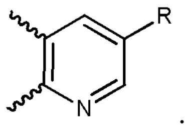

.

**40.** The compound according to claim 38, or a tautomer, a stereoisomer, a prodrug, a crystal form, a pharmaceutically acceptable salt, a hydrate or a solvate thereof, wherein one R is pyrimidin-2-yl, pyrimidin-4-yl, pyrazin-2-yl, triazinyl or tetrazinyl, which is optionally substituted with 1, 2 or 3 R'.

**41.** The compound according to claim 15, or a tautomer, a stereoisomer, a prodrug, a crystal form, a pharmaceutically acceptable salt, a hydrate or a solvate thereof, wherein the compound is a compound of formula (IV-2c):

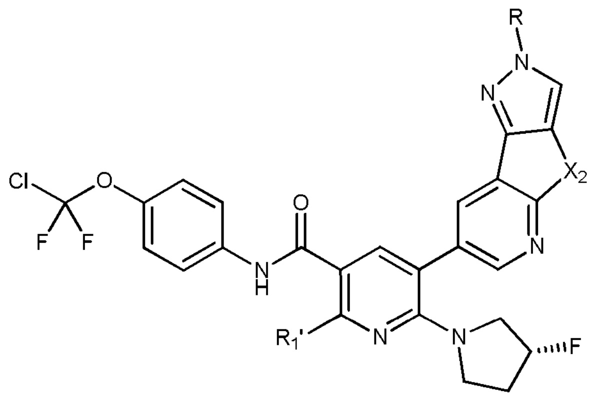

(IV-2c)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**42.** The compound according to claim 41, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;

$X_2$ is $CR_{X2}R_{X2'}$;
$R_{X2}$ and $R_{X2'}$ are independently H, D, halogen or -OH;
R is H, D, -C(O)$R_A$, -S(O)$_2$$R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each $R_A$ is independently $C_{1-6}$ alkyl, $C_{1-6}$ haloalkyl, $C_{3-6}$ cycloalkyl or 3- to 7-membered heterocyclyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy; wherein the above groups are optionally substituted with one or more D, up to fully deuterated.

**43.** The compound according to claim 42, or a tautomer, a stereoisomer, a prodrug, a crystal form, a pharmaceutically acceptable salt, a hydrate or a solvate thereof, wherein

$R_1$' is H, D, halogen or -$NH_2$, alternatively H or D;
$X_2$ is $CR_{X2}R_{X2'}$;
$R_{X2}$ and $R_{X2'}$ are independently H, D, halogen or -OH;
R is H, D, -C(O)$R_A$, -S(O)$_2$$R_A$, $C_{1-6}$ alkyl, $C_{1-6}$ haloalkyl, $C_{3-6}$ cycloalkyl, 3- to 7-membered heterocyclyl or 5-to 10-membered heteroaryl; wherein the above groups are optionally substituted with 1, 2, 3, 4, 5 or 6 R';
wherein each $R_A$ is independently $C_{1-6}$ alkyl, $C_{1-6}$ haloalkyl or $C_{3-6}$ cycloalkyl; wherein the above groups are optionally substituted with 1, 2, 3, 4, 5 or 6 R';
each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1, 2, 3, 4, 5 or 6 R";
each R" is independently H, D, halogen, -CN, -OH, $C_{1-3}$ alkyl, $C_{1-3}$ haloalkyl, $C_{1-3}$ alkoxy$C_{1-3}$ haloalkoxy; wherein the above groups are optionally substituted with one or more D, up to fully deuterated.

**44.** The compound according to claim 43, or a tautomer, a stereoisomer, a prodrug, a crystal form, a pharmaceutically acceptable salt, a hydrate or a solvate thereof, wherein

$R_1$' is H, D, halogen or -$NH_2$, alternatively H or D;
$X_2$ is $CR_{X2}R_{X2'}$;
$R_{X2}$ and $R_{X2'}$ are independently H, D, halogen or -OH;
R is H, D, $C_{1-6}$ alkyl, $C_{1-6}$ haloalkyl, $C_{3-6}$ cycloalkyl, 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1, 2, 3, 4, 5 or 6 R";
each R" is independently H, D, halogen, -CN, -OH, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**45.** The compound according to claim 15, or a tautomer, a stereoisomer, a prodrug, a crystal form, a pharmaceutically acceptable salt, a hydrate or a solvate thereof, wherein the compound is a compound of formula (IV-2d):

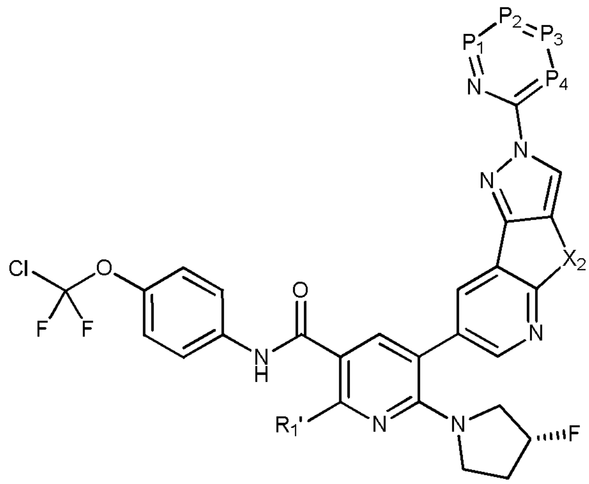

(IV-2d)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**46.** The compound according to claim 45, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**47.** The compound according to claim 15, or a tautomer, a stereoisomer, a prodrug, a crystal form, a pharmaceutically acceptable salt, a hydrate or a solvate thereof, wherein the compound is a compound of formula (IV-2e):

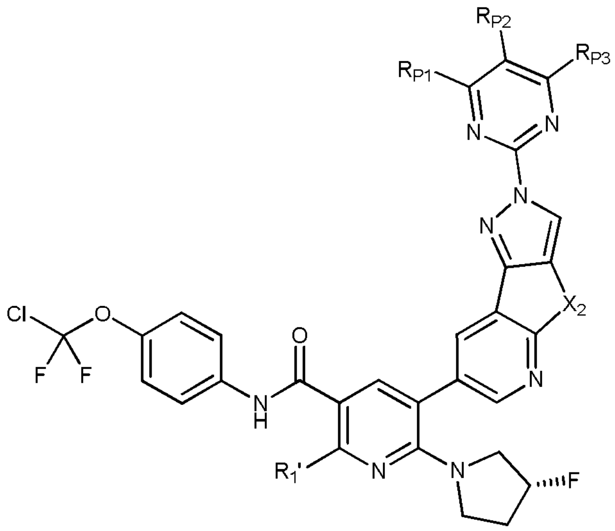

(IV-2e)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{P1}$, $R_{P2}$ and $R_{P3}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**48.** The compound according to claim 47, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$, $R_{P2}$ and $R_{P3}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**49.** The compound according to claim 47, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ is H, D, halogen, -CN, $C_{1-3}$ alkyl, $C_{1-3}$ haloalkyl, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
$R_{P2}$ is H;
$R_{P3}$ is H, $C_{1-3}$ alkyl or $C_{1-3}$ haloalkyl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**50.** The compound according to claim 47, or a tautomer, a stereoisomer, a prodrug, a crystal form, a pharmaceutically

acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**51.** The compound according to claim 50, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, 3- to 7-membered heterocyclyl or phenyl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**52.** The compound according to claim 51, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy or phenyl; wherein the above groups are optionally substituted with one or more D, up to fully deuterated.

**53.** The compound according to claim 15, or a tautomer, a stereoisomer, a prodrug, a crystal form, a pharmaceutically acceptable salt, a hydrate or a solvate thereof, wherein the compound is a compound of formula (V):

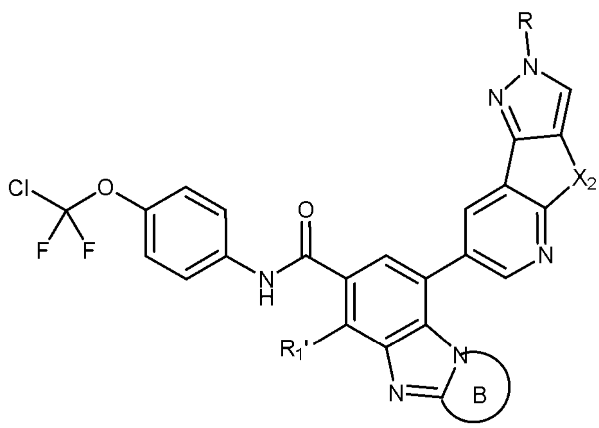

(V)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;

ring B is 4- to 10-membered heterocyclyl, which is optionally substituted with 1, 2, 3, 4, 5 or 6 R';

R is H, D, halogen, -CN, -OH, -C(O)$R_A$, -N$R_B$$R_C$, -N$R_A$C(O)$R_B$, -S(O)$_2$$R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';

wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";

each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**54.** The compound according to claim 15, or a tautomer, a stereoisomer, a prodrug, a crystal form, a pharmaceutically acceptable salt, a hydrate or a solvate thereof, wherein the compound is a compound of formula (V-1):

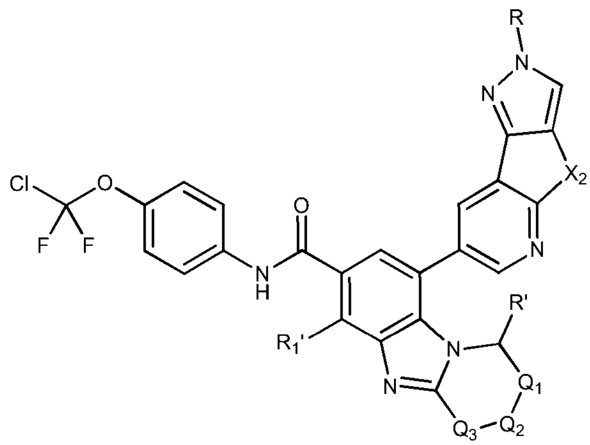

(V-1)

wherein

$R_1$' is H, D, halogen or -N$H_2$, alternatively H or D;

$X_2$ is C$R_{X2}$$R_{X2'}$ or C(O);

$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;

$Q_1$ is C(R')$_2$ or C(R')$_2$C(R')$_2$;

$Q_2$ is C(R')$_2$, O, NR', S, S(O) or S(O)$_2$;

$Q_3$ is C(R')$_2$, C(R')$_2$C(R')$_2$, O, NR', S, S(O) or S(O)$_2$;

R is H, D, halogen, -CN, -OH, -C(O)$R_A$, -N$R_B$$R_C$, -N$R_A$C(O)$R_B$, -S(O)$_2$$R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';

wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';

each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";

each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**55.** The compound according to claim 54, or a tautomer, a stereoisomer, a prodrug, a crystal form, a pharmaceutically

acceptable salt, a hydrate or a solvate thereof, wherein

$R_1$' is H, D, halogen or $-NH_2$, alternatively H or D;
$Q_1$ is $CH_2$, $(CH_2)_2$, CHD, $(CHD)_2$, $CD_2$ or $(CD_2)_2$;
$Q_2$ is $CH_2$, CHD, $CD_2$, O, S, S(O) or $S(O)_2$;
$Q_3$ is $CH_2$, CHD, $CD_2$, CHF, CDF, CH(OH), CD(OH), S or $S(O)_2$;
alternatively,
$R_b$ is halogen or -OH;
$Q_1$ is $CH_2$ or $CD_2$;
$Q_2$ is $CH_2$, CHD, $CD_2$, O or S;
$Q_3$ is $CH_2$, CHD, $CD_2$, CHF, CDF, CH(OH) or CD(OH).

**56.** The compound according to claim 15, or a tautomer, a stereoisomer, a prodrug, a crystal form, a pharmaceutically acceptable salt, a hydrate or a solvate thereof, wherein the compound is a compound of formula (V-2):

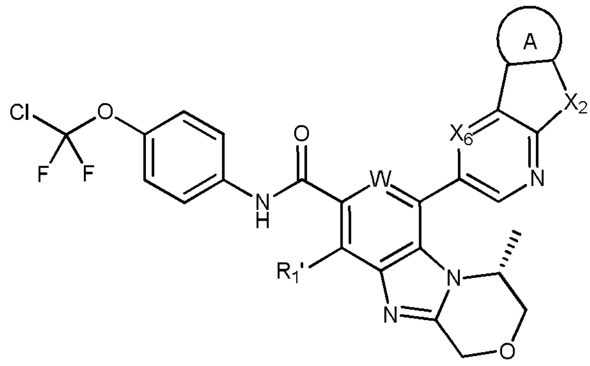

(V-2)

wherein

W is $CR_W$ or N;
$R_W$ is selected from H, D, halogen and $-NH_2$, alternatively H or D;
$R_1$' is H, D, halogen or $-NH_2$, alternatively H or D;
$X_2$ is $CR_{X2}R_{X2'}$ or C(O);
$X_6$ is N or $CR_{X6}$, alternatively $CR_{X6}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1, 2, 3 or 4 R;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**57.** The compound according to claim 56, or a tautomer, a stereoisomer, a prodrug, a crystal form, a pharmaceutically acceptable salt, a hydrate or a solvate thereof, wherein ring A is

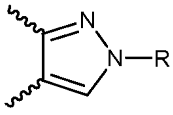

,

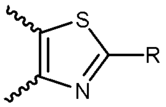 , 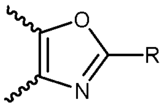 , 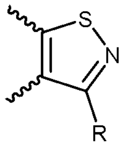 , 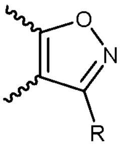 , 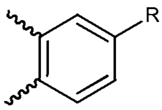 , 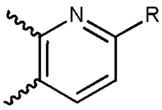 ,

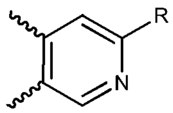

or

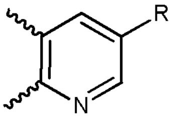

.

**58.** The compound according to claim 56, or a tautomer, a stereoisomer, a prodrug, a crystal form, a pharmaceutically acceptable salt, a hydrate or a solvate thereof, wherein one R is pyrimidin-2-yl, pyrimidin-4-yl, pyrazin-2-yl, triazinyl or tetrazinyl, which is optionally substituted with 1, 2 or 3 R'.

**59.** The compound according to claim 15, or a tautomer, a stereoisomer, a prodrug, a crystal form, a pharmaceutically acceptable salt, a hydrate or a solvate thereof, wherein the compound is a compound of formula (V-3):

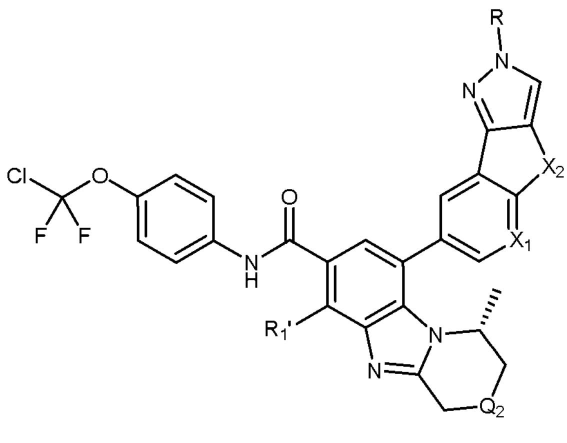

(V-3)

wherein

$X_1$ is N or $CR_{X1}$, alternatively N;
$R_{X1}$ is H, D, halogen, $C_{1-6}$ alkyl or $C_{1-6}$ haloalkyl; wherein the above groups are optionally substituted with 1, 2, 3, 4, 5, 6 or more D, up to fully deuterated;
$X_2$ is $CR_{X2}R_{X2'}$ or C(O), alternatively $CR_{X2}R_{X2'}$;
$R_{X2}$ and $R_{X2'}$ are independently H, D, halogen, -OH, $C_{1-6}$ alkyl or $C_{1-6}$ haloalkyl, alternatively H, D, halogen, $C_{1-6}$ alkyl or $C_{1-6}$ haloalkyl; wherein the above groups are optionally substituted with one or more D, up to fully deuterated;
$R_1$' is H, D, halogen or $-NH_2$, alternatively H or D;
$Q_2$ is O, S or NR', alternatively O or S, still alternatively O;
R' is H, D, -CN, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered

heterocyclyl, $C_{6-10}$ aryl or 5- to 10-membered heteroaryl, alternatively H, D, -CN or halogen; wherein the above groups are optionally substituted with 1, 2, 3, 4, 5 or 6 -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
R is H, D, -C(O)$R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl, alternatively H, D, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R'; wherein the above groups are also optionally substituted with one or more D, up to fully deuterated;
$R_A$ is H, D, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated.

**60.** The compound according to claim 59, or a tautomer, a stereoisomer, a prodrug, a crystal form, a pharmaceutically acceptable salt, a hydrate or a solvate thereof, wherein

$X_1$ is N;
$X_2$ is $CR_{X2}R_{X2'}$;
$R_{X2}$ and $R_{X2'}$ are independently H or D;
$R_1$' is H, D, halogen or -$NH_2$, alternatively H or D;
$Q_2$ is O;
R is H, D, Et, $CH_2CF_3$, isopropyl, $CD(CD_3)_2$, cyclopropyl, oxetanyl, pyrazin-2-yl, pyridin-2-yl or pyrimidin-5-yl.

**61.** The compound according to claim 15, or a tautomer, a stereoisomer, a prodrug, a crystal form, a pharmaceutically acceptable salt, a hydrate or a solvate thereof, wherein the compound is a compound of formula (V-4):

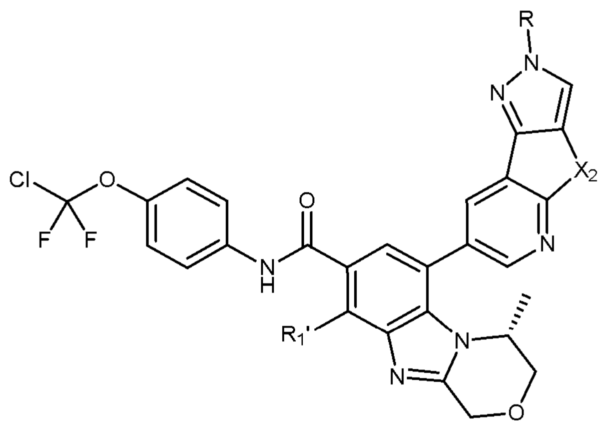

(V-4)

wherein

$R_1$' is H, D, halogen or -$NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
R is H, D, halogen, -CN, -OH, -C(O)$R_A$, -$NR_BR_C$, -$NR_A$C(O)$R_B$, -S(O)$_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**62.** The compound according to claim 61, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
R is H, D, $-C(O)R_A$, $-S(O)_2R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each $R_A$ is independently $C_{1-6}$ alkyl, $C_{1-6}$ haloalkyl, $C_{3-6}$ cycloalkyl or 3- to 7-membered heterocyclyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy; wherein the above groups are optionally substituted with one or more D, up to fully deuterated.

**63.** The compound according to claim 62, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
R is H, D, $-C(O)R_A$, $-S(O)_2R_A$, $C_{1-6}$ alkyl, $C_{1-6}$ haloalkyl, $C_{3-6}$ cycloalkyl, 3- to 7-membered heterocyclyl or 5-to 10-membered heteroaryl; wherein the above groups are optionally substituted with 1, 2, 3, 4, 5 or 6 R';
wherein each $R_A$ is independently $C_{1-6}$ alkyl, $C_{1-6}$ haloalkyl or $C_{3-6}$ cycloalkyl; wherein the above groups are optionally substituted with 1, 2, 3, 4, 5 or 6 R';
each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1, 2, 3, 4, 5 or 6 R";
each R" is independently H, D, halogen, -CN, -OH, $C_{1-3}$ alkyl, $C_{1-3}$ haloalkyl, $C_{1-3}$ alkoxy$C_{1-3}$ haloalkoxy; wherein the above groups are optionally substituted with one or more D, up to fully deuterated.

**64.** The compound according to claim 63, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
R is H, D, $C_{1-6}$ alkyl, $C_{1-6}$ haloalkyl, $C_{3-6}$ cycloalkyl, 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1, 2, 3, 4, 5 or 6 R";
each R" is independently H, D, halogen, -CN, -OH, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**65.** The compound according to claim 15, or a tautomer, a stereoisomer, a prodrug, a crystal form, a pharmaceutically acceptable salt, a hydrate or a solvate thereof, wherein the compound is a compound of formula (V-4):

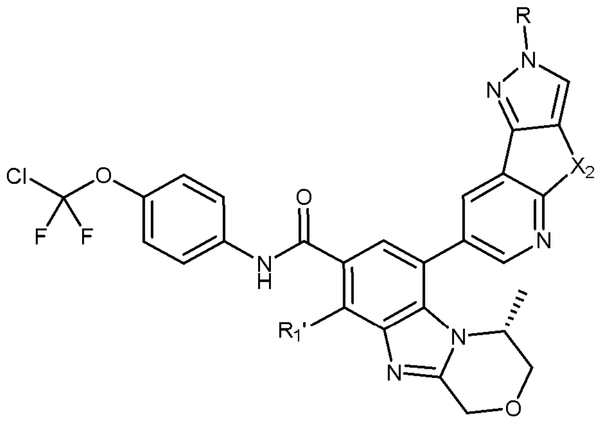

(V-4)

wherein

$R_1$' is H, D, halogen or $-NH_2$, alternatively H or D;
$X_2$ is $CR_{X2}R_{X2'}$ or C(O), alternatively $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
R is H, D, $-C(O)R_A$, $C_{1-6}$ alkyl or $C_{1-6}$ haloalkyl; wherein the above groups are optionally substituted with one or more D, up to fully deuterated;
$R_A$ is H, D, $C_{1-6}$ alkyl, $C_{1-6}$ haloalkyl, $C_{3-6}$ cycloalkyl or 3- to 7-membered heterocyclyl.

**66.** The compound according to claim 65, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H or D;
R is H, D, Et, $CH_2CF_3$, isopropyl or $CD(CD_3)_2$.

**67.** The compound according to claim 15, or a tautomer, a stereoisomer, a prodrug, a crystal form, a pharmaceutically acceptable salt, a hydrate or a solvate thereof, wherein the compound is a compound of formula (V-5):

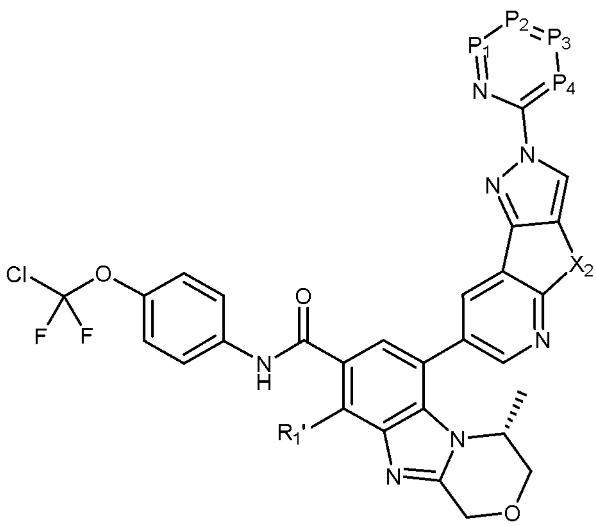

(V-5)

wherein

$R_1$' is H, D, halogen or $-NH_2$, alternatively H or D;

$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**68.** The compound according to claim 67, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**69.** The compound according to claim 15, or a tautomer, a stereoisomer, a prodrug, a crystal form, a pharmaceutically acceptable salt, a hydrate or a solvate thereof, wherein the compound is a compound of formula (V-5):

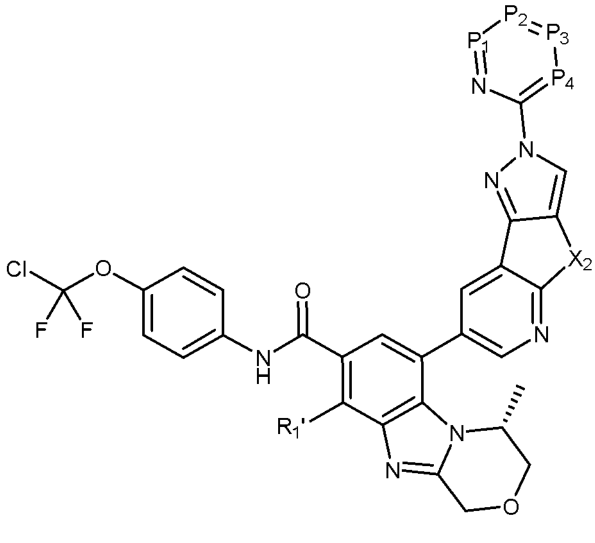

(V-5)

wherein

$R_1$' is H, D, halogen or $-NH_2$, alternatively H or D;
$X_2$ is $CR_{X2}R_{X2'}$;
$R_{X2}$ and $R_{X2'}$ are independently H, D, OH, halogen, $C_{1-6}$ alkyl or $C_{1-6}$ haloalkyl, alternatively H, D, halogen, $C_{1-6}$ alkyl or $C_{1-6}$ haloalkyl; wherein the above groups are optionally substituted with one or more D, up to fully deuterated;

$P_1$ is $CR_{P1}$;
$P_2$ is N or $CR_{P2}$;
$P_3$ is N or $CR_{P3}$;
$P_4$ is N or $CR_{P4}$;
and alternatively, at least one of $P_3$ and $P_4$ is N;
$R_{P1}$, $R_{P2}$, $R_{P3}$ and $R_{P4}$ are independently H, D, halogen, -CN, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, halogen, -CN, $C_{1-6}$ alkyl or $C_{1-6}$ haloalkyl; wherein the above groups are optionally substituted with 1, 2, 3, 4, 5 or 6 -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
or $R_{P1}$ and $R_{P2}$, $R_{P2}$ and $R_{P3}$, or $R_{P3}$ and $R_{P4}$, and the atoms to which they are attached are taken together to form $C_{6-10}$ aryl or 5- to 10-membered heteroaryl; wherein the above groups are optionally substituted with 1, 2, 3, 4, 5 or 6 -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

**70.** The compound according to claim 69, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
$P_1$ is $CR_{P1}$;
$P_2$ is $CR_{P2}$;
$P_3$ is N or $CR_{P3}$;
$P_4$ is N or $CR_{P4}$;
and at least one of $P_3$ and $P_4$ is N;
$R_{P1}$ is H, D, halogen, -CN, $C_{1-6}$ alkyl or $C_{1-6}$ haloalkyl; wherein the above groups are optionally substituted with one or more D, up to fully deuterated;
$R_{P2}$ is H, D, halogen or -CN;
$R_{P3}$ is H, D, $C_{1-6}$ alkyl or $C_{1-6}$ haloalkyl; wherein the above groups are optionally substituted with one or more D, up to fully deuterated;
$R_{P4}$ is H or D.

**71.** The compound according to claim 69, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H or D;
$P_1$ is $CR_{P1}$;
$P_2$ is $CR_{P2}$;
$P_3$ is N or $CR_{P3}$;
$P_4$ is N or $CR_{P4}$;
and at least one of $P_3$ and $P_4$ is N;
$R_{P1}$ is H, D, -CN or Me;
$R_{P2}$ is H, D, F or -CN;
$R_{P3}$ is H, D or Me;
$R_{P4}$ is H or D.

**72.** The compound according to claim 15, or a tautomer, a stereoisomer, a prodrug, a crystal form, a pharmaceutically acceptable salt, a hydrate or a solvate thereof, wherein the compound is a compound of formula (V-5):

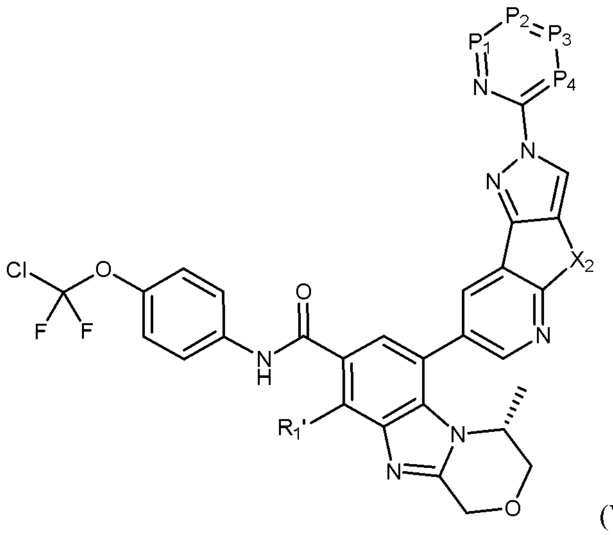

(V-5)

wherein

$R_1$' is H, D, halogen or -$NH_2$, alternatively H or D;
$X_2$ is $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
$P_1$ is $CR_{P1}$;
$P_2$ is N or $CR_{P2}$;
$P_3$ is N or $CR_{P3}$;
$P_4$ is $CR_{P4}$;
$R_{P1}$, $R_{P2}$, $R_{P3}$ and $R_{P4}$ are independently H, D, halogen, -CN, $C_{1-6}$ alkyl or $C_{1-6}$ haloalkyl; wherein the above groups are optionally substituted with one or more D, up to fully deuterated.

**73.** The compound according to claim 72, or a tautomer, a stereoisomer, a prodrug, a crystal form, a pharmaceutically acceptable salt, a hydrate or a solvate thereof, wherein

$R_1$' is H, D, halogen or -$NH_2$, alternatively H or D;
$X_2$ is $CR_{X2}R_{X2'}$;
$R_{X2}$ and $R_{X2'}$ are independently H or D;
$P_1$ is $CR_{P1}$;
$P_2$ is N or $CR_{P2}$;
$P_3$ is N or $CR_{P3}$;
$P_4$ is $CR_{P4}$;
$R_{P1}$ is H or D;
$R_{P2}$ is H or D;
$R_{P3}$ is H or D;
$R_{P4}$ is H or D.

**74.** The compound according to claim 15, or a tautomer, a stereoisomer, a prodrug, a crystal form, a pharmaceutically acceptable salt, a hydrate or a solvate thereof, wherein the compound is a compound of formula (V-6):

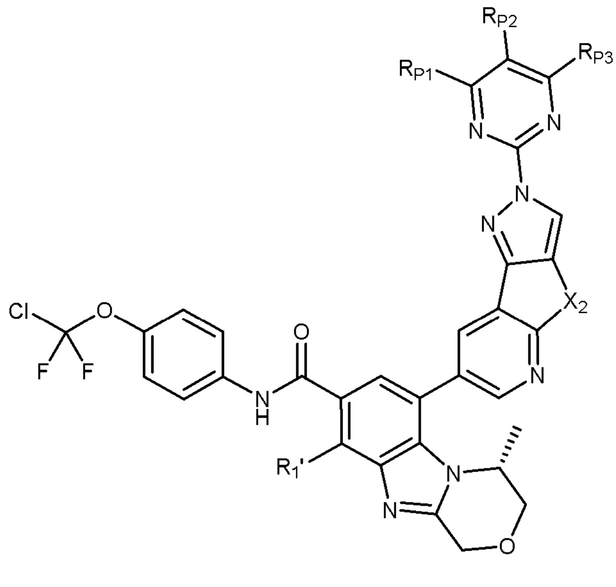

(V-6)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{P1}$, $R_{P2}$ and $R_{P3}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**75.** The compound according to claim 74, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$, $R_{P2}$ and $R_{P3}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**76.** The compound according to claim 74, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ is H, D, halogen, -CN, $C_{1-3}$ alkyl, $C_{1-3}$ haloalkyl, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
$R_{P2}$ is H;
$R_{P3}$ is H, $C_{1-3}$ alkyl or $C_{1-3}$ haloalkyl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**77.** The compound according to claim 74, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**78.** The compound according to claim 77, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, 3- to 7-membered heterocyclyl or phenyl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**79.** The compound according to claim 78, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy or phenyl; wherein the above groups are optionally substituted with one or more D, up to fully deuterated.

**80.** The compound according to claim 15, or a tautomer, a stereoisomer, a prodrug, a crystal form, a pharmaceutically acceptable salt, a hydrate or a solvate thereof, wherein the compound is a compound of formula (V-6):

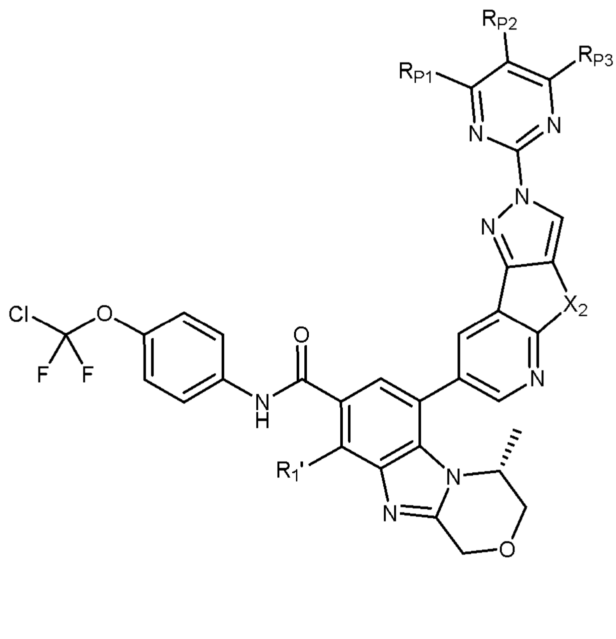

(V-6)

wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen, OH, $C_{1-6}$ alkyl or $C_{1-6}$ haloalkyl, alternatively H, D, halogen, $C_{1-6}$ alkyl or $C_{1-6}$ haloalkyl; wherein the above groups are optionally substituted with one or more D, up to fully deuterated;
$R_{P1}$, $R_{P2}$ and $R_{P3}$ are independently H, D, halogen, -CN, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, halogen, -CN, $C_{1-6}$ alkyl or $C_{1-6}$ haloalkyl; wherein the above groups are optionally substituted with 1, 2, 3, 4, 5 or 6 -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
or $R_{P1}$ and $R_{P2}$, or $R_{P2}$ and $R_{P3}$ and the atoms to which they are attached are taken together to form $C_{6-10}$ aryl or 5- to 10-membered heteroaryl; wherein the above groups are optionally substituted with 1, 2, 3, 4, 5 or 6 -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

**81.** The compound according to claim 80, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
$R_{P1}$ is H, D, halogen, -CN, $C_{1-6}$ alkyl or $C_{1-6}$ haloalkyl; wherein the above groups are optionally substituted with one or more D, up to fully deuterated;
$R_{P2}$ is H, D, halogen or -CN;
$R_{P3}$ is H, D, $C_{1-6}$ alkyl or $C_{1-6}$ haloalkyl; wherein the above groups are optionally substituted with one or more D, up to fully deuterated.

**82.** The compound according to claim 80, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H or D;
$R_{P1}$ is H, D, -CN or Me;
$R_{P2}$ is H, D, F or -CN;
$R_{P3}$ is H, D or Me.

**83.** The compound according to claim 15, or a tautomer, a stereoisomer, a prodrug, a crystal form, a pharmaceutically acceptable salt, a hydrate or a solvate thereof, wherein the compound is a compound of formula (VI):

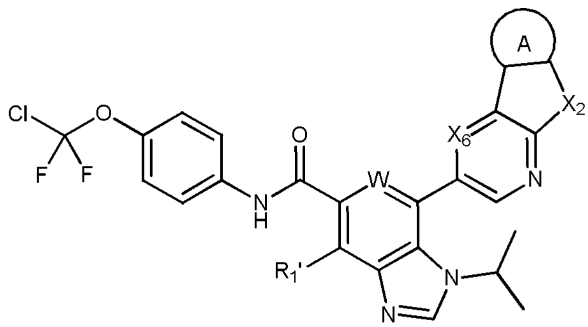
(VI)

wherein

W is $CR_W$ or N;

$R_W$ is selected from H, D, halogen and $-NH_2$, alternatively H or D;
$R_1$' is H, D, halogen or $-NH_2$, alternatively H or D;
$X_2$ is $CR_{X2}R_{X2'}$ or C(O);
$X_6$ is N or $CR_{X6}$, alternatively $CR_{X6}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1, 2, 3 or 4 R;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**84.** The compound according to claim 83, or a tautomer, a stereoisomer, a prodrug, a crystal form, a pharmaceutically acceptable salt, a hydrate or a solvate thereof, wherein ring A is

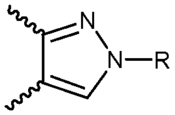,

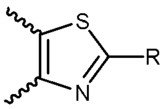, 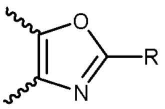, 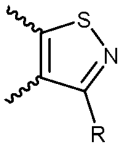, 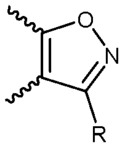, 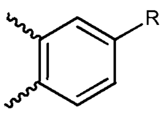, 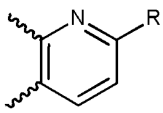,

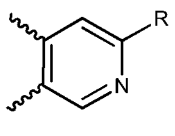

or

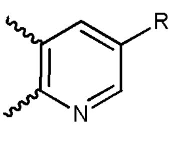.

**85.** The compound according to claim 83, or a tautomer, a stereoisomer, a prodrug, a crystal form, a pharmaceutically acceptable salt, a hydrate or a solvate thereof, wherein one R is pyrimidin-2-yl, pyrimidin-4-yl, pyrazin-2-yl, triazinyl or tetrazinyl, which is optionally substituted with 1, 2 or 3 R'.

**86.** The compound according to claim 15, or a tautomer, a stereoisomer, a prodrug, a crystal form, a pharmaceutically acceptable salt, a hydrate or a solvate thereof, wherein the compound is a compound of formula (VI-1):

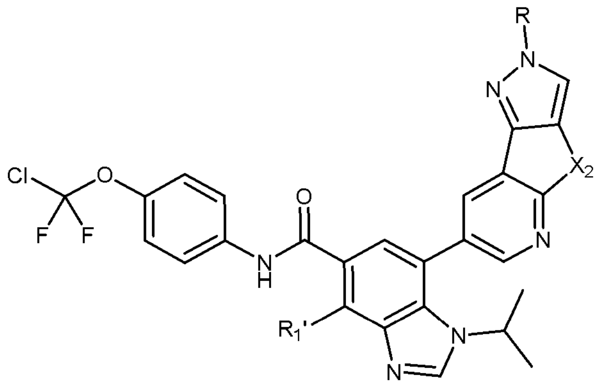

(VI-1)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**87.** The compound according to claim 86, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
R is H, D, $-C(O)R_A$, $-S(O)_2R_A$, $C_{1-6}$ alkyl, $C_{1-6}$ haloalkyl, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each $R_A$ is independently $C_{1-6}$ alkyl, $C_{1-6}$ haloalkyl, $C_{3-6}$ cycloalkyl or 3- to 7-membered heterocyclyl; wherein the above groups are optionally substituted with one or more R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy; wherein the above groups are optionally substituted with one or more D, up to fully deuterated.

**88.** The compound according to claim 87, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
R is H, D, $-C(O)R_A$, $-S(O)_2R_A$, $C_{1-6}$ alkyl, $C_{1-6}$ haloalkyl, $C_{3-6}$ cycloalkyl, 3- to 7-membered heterocyclyl or 5-to 10-membered heteroaryl; wherein the above groups are optionally substituted with 1, 2, 3, 4, 5 or 6 R';

wherein each $R_A$ is independently $C_{1-6}$ alkyl, $C_{1-6}$ haloalkyl or $C_{3-6}$ cycloalkyl; wherein the above groups are optionally substituted with 1, 2, 3, 4, 5 or 6 R';

each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1, 2, 3, 4, 5 or 6 R";

each R" is independently H, D, halogen, -CN, -OH, $C_{1-3}$ alkyl, $C_{1-3}$ haloalkyl, $C_{1-3}$ alkoxy$C_{1-3}$ haloalkoxy; wherein the above groups are optionally substituted with one or more D, up to fully deuterated.

**89.** The compound according to claim 88, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;

$X_2$ is $CR_{X2}R_{X2'}$;

$R_{X2}$ and $R_{X2'}$ are independently H, D, halogen or -OH;

R is H, D, $C_{1-6}$ alkyl, $C_{1-6}$ haloalkyl, $C_{3-6}$ cycloalkyl, 3- to 7-membered heterocyclyl or 5- to 10-membered heteroaryl; wherein the above groups are optionally substituted with 1, 2, 3, 4, 5 or 6 R';

each R' is independently H, D, halogen, -CN, -OH, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- to 10-membered heteroaryl; wherein the above groups are optionally substituted with 1, 2, 3, 4, 5 or 6 R";

each R" is independently H, D, halogen, -CN, -OH, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**90.** The compound according to claim 15, or a tautomer, a stereoisomer, a prodrug, a crystal form, a pharmaceutically acceptable salt, a hydrate or a solvate thereof, wherein the compound is a compound of formula (VI-2):

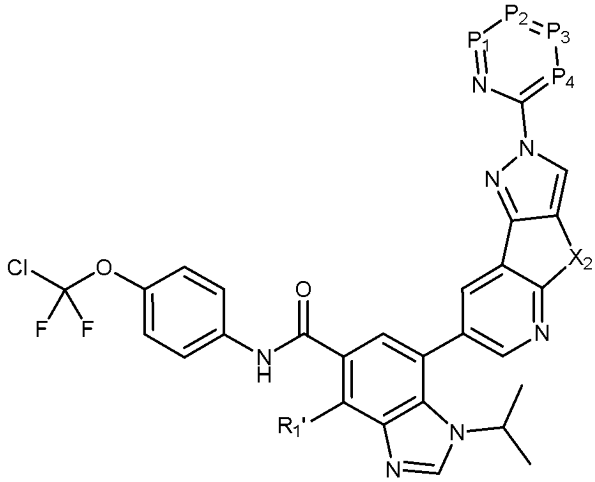

(VI-2)

wherein

$R_1$' is H, D, halogen or $-NH_2$, alternatively H or D;

$X_2$ is $CR_{X2}R_{X2'}$ or C(O);

$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;

$P_1$ is N or $CR_{P1}$;

$P_2$ is N or $CR_{P2}$;

$P_3$ is N or $CR_{P3}$;

$P_4$ is N or $CR_{P4}$;

$R_{P1}$, $R_{P2}$, $R_{P3}$ and $R_{P4}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";

each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**91.** The compound according to claim 90, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$P_1$ is N or $CR_{P1}$;
$P_2$ is N or $CR_{P2}$;
$P_3$ is N or $CR_{P3}$;
$P_4$ is N or $CR_{P4}$;
$R_{P1}$, $R_{P2}$, $R_{P3}$ and $R_{P4}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**92.** The compound according to claim 15, or a tautomer, a stereoisomer, a prodrug, a crystal form, a pharmaceutically acceptable salt, a hydrate or a solvate thereof, wherein the compound is a compound of formula (VI-3):

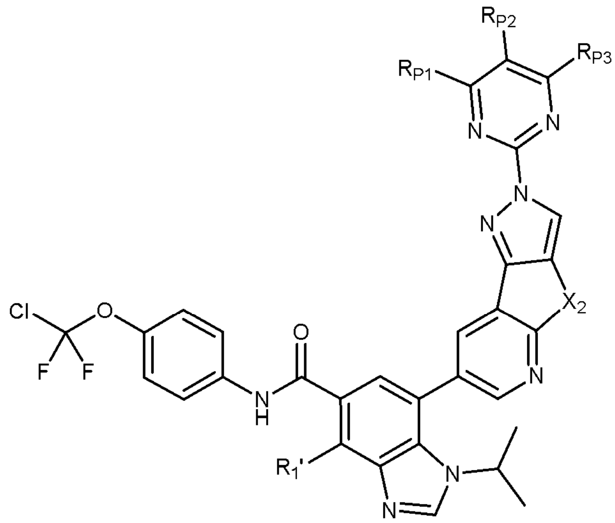
(VI-3)

wherein

$R_1$' is H, D, halogen or $-NH_2$, alternatively H or D;
$X_2$ is $CR_{X2}R_{X2'}$ or C(O);
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{P1}$, $R_{P2}$ and $R_{P3}$ are independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**93.** The compound according to claim 92, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$, $R_{P2}$ and $R_{P3}$ are independently H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups

are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**94.** The compound according to claim 92, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ is H, D, halogen, -CN, $C_{1-3}$ alkyl, $C_{1-3}$ haloalkyl, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
$R_{P2}$ is H;
$R_{P3}$ is H, $C_{1-3}$ alkyl or $C_{1-3}$ haloalkyl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with 1, 2, 3, 4, 5, 6 or more D, up to fully deuterated.

**95.** The compound according to claim 92, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, $C_{3-6}$ cycloalkyl, 3- to 7-membered heterocyclyl, phenyl or 5- or 6-membered heteroaryl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**96.** The compound according to claim 95, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy, 3- to 7-membered heterocyclyl or phenyl; wherein the above groups are optionally substituted with 1, 2, 3, 4, 5 or 6 R";
each R" is independently H, D, halogen, -CN, $C_{1-3}$ alkyl, $C_{1-3}$ haloalkyl, $C_{1-3}$ alkoxy or $C_{1-3}$ haloalkoxy; wherein the above groups are optionally substituted with one or more D, up to fully deuterated.

**97.** The compound according to claim 96, or a tautomer, a stereoisomer, a prodrug, a crystal form, a pharmaceutically acceptable salt, a hydrate or a solvate thereof, wherein

$R_1$' is H, D, halogen or $-NH_2$, alternatively H or D;
$X_2$ is $CR_{X2}R_{X2'}$;
$R_{X2}$ and $R_{X2'}$ are independently H, D, halogen or -OH;
$R_{P1}$ and $R_{P3}$ are H;
$R_{P2}$ is H, D, halogen, -CN, $C_{1-3}$ alkyl, $C_{1-3}$ haloalkyl, $C_{1-3}$ alkoxy, $C_{1-3}$ haloalkoxy or phenyl; wherein the above groups are optionally substituted with one or more D, up to fully deuterated.

**98.** The compound according to claim 15, or a tautomer, a stereoisomer, a prodrug, a crystal form, a pharmaceutically acceptable salt, a hydrate or a solvate thereof, wherein the compound is a compound of formula (VII):

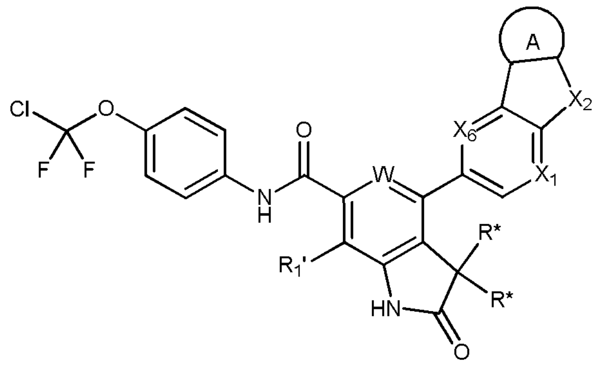

(VII)

wherein

W is $CR_W$ or N;
$R_W$ is selected from H, D, halogen and $-NH_2$, alternatively H or D;
$R_1'$ is H, D, halogen or $-NH_2$, alternatively H or D;
$X_1$ is N or $CR_{X1}$, alternatively N;
$X_6$ is N or $CR_{X6}$, alternatively $CR_{X6}$;
$R_{X1}$ is H, D, halogen, $C_{1-6}$ alkyl or $C_{1-6}$ haloalkyl; wherein the above groups are optionally substituted with 1, 2, 3, 4, 5, 6 or more D, up to fully deuterated;
$X_2$ is $CR_{X2}R_{X2'}$ or C(O), alternatively $CR_{X2}R_{X2'}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1, 2, 3 or 4 R;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R* and the atom(s) to which they are attached are taken together to form $C_{3-8}$ cycloalkyl or 4- to 10-membered heterocyclyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**99.** The compound according to claim 98, or a tautomer, a stereoisomer, a prodrug, a crystal form, a pharmaceutically acceptable salt, a hydrate or a solvate thereof, wherein ring A is

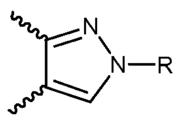

,

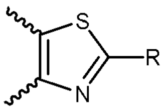, 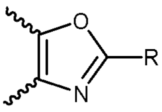, 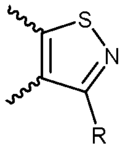, 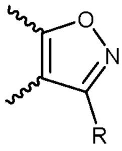, 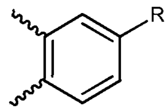, 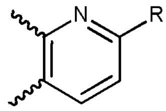,

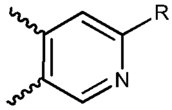

or

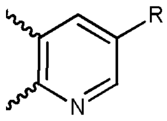.

**100.**
The compound according to claim 98, or a tautomer, a stereoisomer, a prodrug, a crystal form, a pharmaceutically acceptable salt, a hydrate or a solvate thereof, wherein one R is pyrimidin-2-yl, pyrimidin-4-yl, pyrazin-2-yl, triazinyl or tetrazinyl, which is optionally substituted with 1, 2 or 3 R'.

**101.**
The compound according to claim 15, or a tautomer, a stereoisomer, a prodrug, a crystal form, a pharmaceutically acceptable salt, a hydrate or a solvate thereof, wherein the compound is a compound of formula (VII-1):

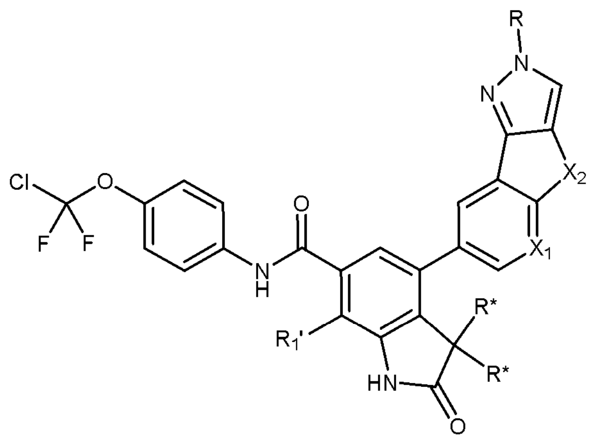

(VII-1)

wherein

$R_1$' is H, D, halogen or $-NH_2$, alternatively H or D;
$X_1$ is N or $CR_{X1}$, alternatively N;
$R_{X1}$ is H, D, halogen, $C_{1-6}$ alkyl or $C_{1-6}$ haloalkyl; wherein the above groups are optionally substituted with 1, 2, 3, 4, 5, 6 or more D, up to fully deuterated;
$X_2$ is $CR_{X2}R_{X2'}$ or C(O), alternatively $CR_{X2}R_{X2'}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, $C_{1-6}$ alkyl, $C_{1-6}$ haloalkyl, $C_{6-10}$ aryl or 5- to 10-membered heteroaryl; wherein the above groups are optionally substituted with 1, 2, 3, 4, 5 or 6 R';
each R* is independently H, D, halogen, -CN, -OH, oxo, $-NR_AC(O)R_B$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, or two R*

and the atom to which they are attached are taken together to form $C_{3-8}$ cycloalkyl or 4- to 10-membered heterocyclyl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl;
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R'.

**102.**
The compound according to claim 15, or a tautomer, a stereoisomer, a prodrug, a crystal form, a pharmaceutically acceptable salt, a hydrate or a solvate thereof, wherein the compound is a compound of formula (VII-2):

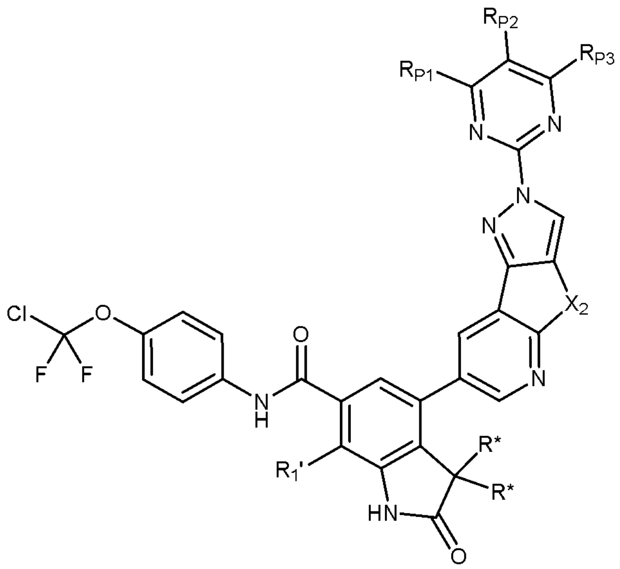

(VII-2)

wherein

$R_1$' is H, D, halogen or $-NH_2$, alternatively H or D;
$X_2$ is $CR_{X2}R_{X2'}$ or C(O), alternatively $CR_{X2}R_{X2'}$;
$R_{X2}$ and $R_{X2'}$ are independently H, D, halogen, $C_{1-6}$ alkyl or $C_{1-6}$ haloalkyl; wherein the above groups are optionally substituted with one or more D, up to fully deuterated;
each R* is independently H, D, halogen, $C_{1-6}$ alkyl or $C_{1-6}$ haloalkyl, or two R* and the atom to which they are attached are taken together to form $C_{3-8}$ cycloalkyl or 4- to 10-membered heterocyclyl; wherein the above groups are optionally substituted with 1, 2, 3, 4, 5 or 6 R';
each R' is independently H, D, halogen, -CN, -OH, $C_{1-6}$ alkyl or $C_{1-6}$ haloalkyl;
$R_{P1}$, $R_{P2}$ and $R_{P3}$ are independently H, D, halogen, -CN, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl, alternatively H, D, halogen, -CN, $C_{1-6}$ alkyl or $C_{1-6}$ haloalkyl; wherein the above groups are optionally substituted with 1, 2, 3, 4, 5 or 6 -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated;
or $R_{P1}$ and $R_{P2}$, or $R_{P2}$ and $R_{P3}$ and the atoms to which they are attached are taken together to form $C_{6-10}$ aryl or 5- to 10-membered heteroaryl; wherein the above groups are optionally substituted with 1, 2, 3, 4, 5 or 6 -CN, halogen, $C_{1-6}$ alkyl, $C_{1-6}$ haloalkyl, $C_{1-6}$ alkoxy or $C_{1-6}$ haloalkoxy; wherein the above groups are also optionally substituted with one or more D, up to fully deuterated.

**103.**
The compound according to claim 15, or a tautomer, a stereoisomer, a prodrug, a crystal form, a pharmaceutically acceptable salt, a hydrate or a solvate thereof, wherein the compound is a compound of formula (VIII):

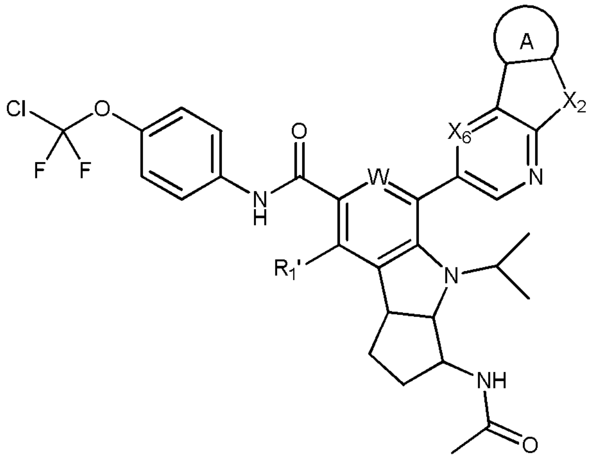

(VIII)

wherein

W is $CR_W$ or N;
$R_W$ is selected from H, D, halogen and $-NH_2$, alternatively H or D;
$R_1$' is H, D, halogen or $-NH_2$, alternatively H or D;
$X_2$ is $CR_{X2}R_{X2'}$ or C(O);
$X_6$ is N or $CR_{X6}$, alternatively $CR_{X6}$;
$R_{X2}$ and $R_{X2'}$ are independently H, D, halogen, -OH, $C_{1-6}$ alkyl, $C_{1-6}$ haloalkyl, $C_{3-6}$ cycloalkyl or 3- to 7-membered heterocyclyl; wherein the above groups are optionally substituted with one or more D, up to fully deuterated;
$R_{X6}$ is H, D or halogen, alternatively H or D;
ring A is phenyl or 5- to 6-membered heteroaryl, which is optionally substituted with 1, 2, 3 or 4 R;
R is H, D, halogen, -CN, -OH, $-C(O)R_A$, $-NR_BR_C$, $-NR_AC(O)R_B$, $-S(O)_2R_A$, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R';
wherein each of $R_A$, $R_B$ and $R_C$ is independently H, D, $C_{1-6}$ alkyl, $C_{1-6}$ haloalkyl, $C_{3-6}$ cycloalkyl, 3- to 7-membered heterocyclyl, $C_{2-6}$ alkenyl, $C_{2-6}$ alkynyl, $C_{6-10}$ aryl or 5- to 10-membered heteroaryl, or $R_B$, $R_C$ and the N atom to which they are attached are taken together to form 3- to 7-membered heterocyclyl or 5- to 10-membered heteroaryl; wherein the above groups are optionally substituted with 1, 2, 3, 4, 5 or 6 R';
each R' is independently H, D, halogen, -CN, -OH, $C_{1-6}$ alkyl, $C_{1-6}$ haloalkyl, $C_{1-6}$ alkoxy, $C_{1-6}$ haloalkoxy, $C_{3-6}$ cycloalkyl, 3- to 7-membered heterocyclyl, $C_{6-10}$ aryl or 5- to 10-membered heteroaryl; wherein the above groups are optionally substituted with 1, 2, 3, 4, 5 or 6 R";
each R" is independently H, D, halogen, -CN, -OH, $C_{1-6}$ alkyl, $C_{1-6}$ haloalkyl, $C_{1-6}$ alkoxy$C_{1-6}$ haloalkoxy, $C_{3-6}$ cycloalkyl or 3- to 7-membered heterocyclyl; wherein the above groups are optionally substituted with one or more D, up to fully deuterated.

**104.**

The compound according to claim 103, or a tautomer, a stereoisomer, a prodrug, a crystal form, a pharmaceutically acceptable salt, a hydrate or a solvate thereof, wherein ring A is

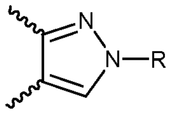

,

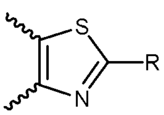

,

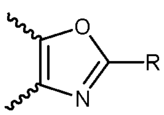

,

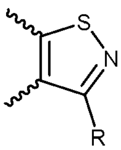

,

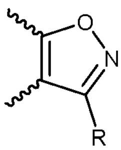

,

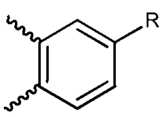

,

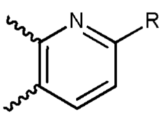

,

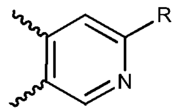

or

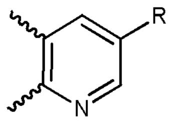

.

**105.**

The compound according to claim 103, or a tautomer, a stereoisomer, a prodrug, a crystal form, a pharmaceutically acceptable salt, a hydrate or a solvate thereof, wherein one R is pyrimidin-2-yl, pyrimidin-4-yl, pyrazin-2-yl, triazinyl or tetrazinyl, which is optionally substituted with 1, 2 or 3 R'.

**106.**

A compound, or a tautomer, a stereoisomer, a prodrug, a crystal form, a pharmaceutically acceptable salt, a hydrate or a solvate thereof, wherein the compound is selected from:

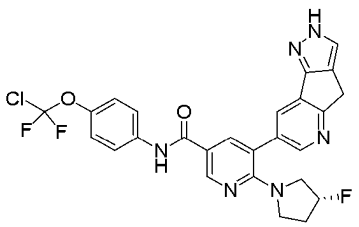

,

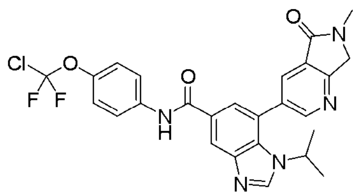

,

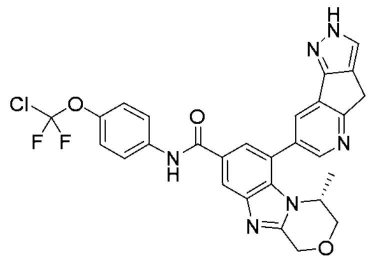

,

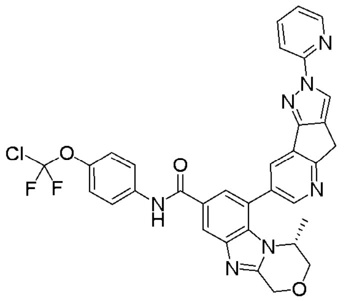

,

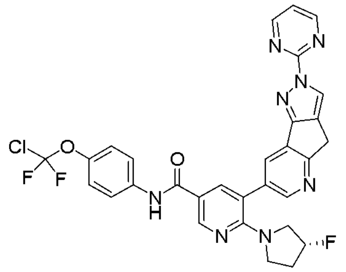

,

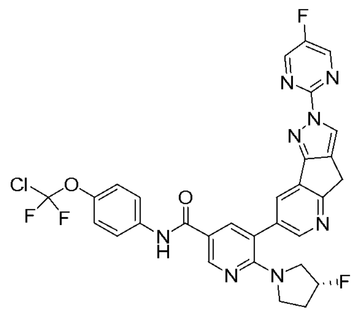

,

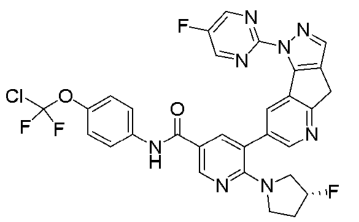

,

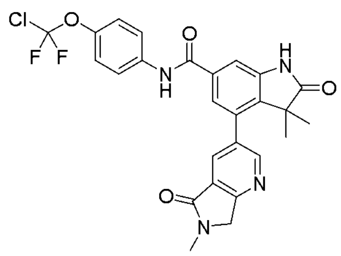

,

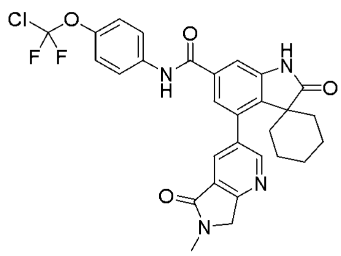

,

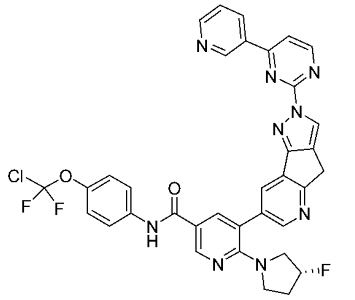,
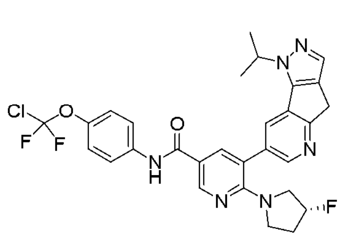,
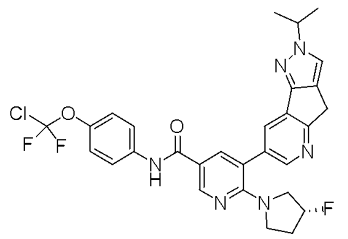,

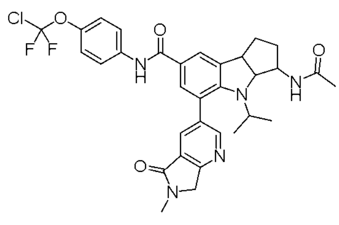,
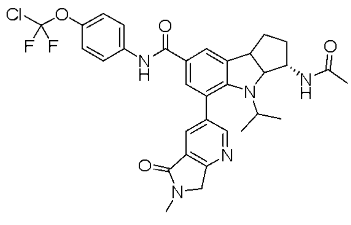,
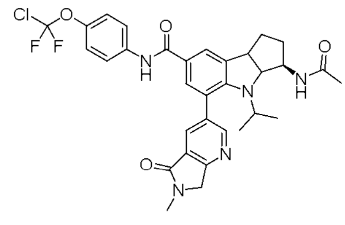,

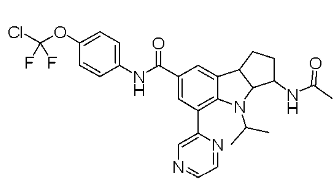,
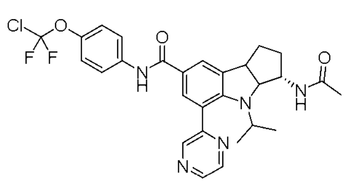,
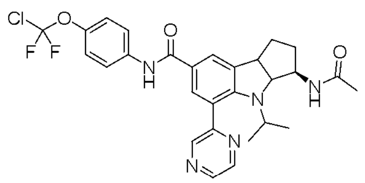,

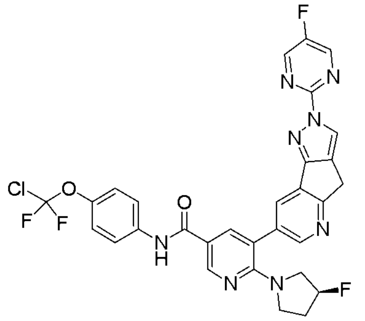,
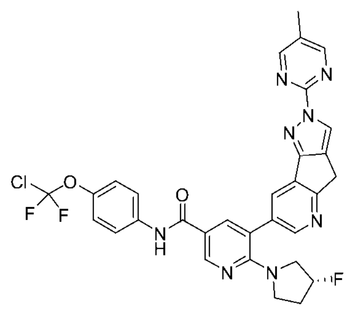,
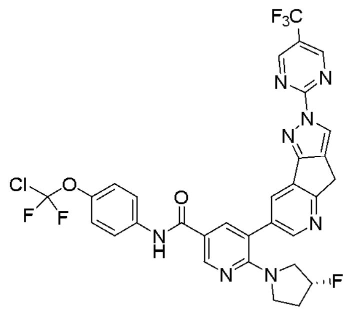,

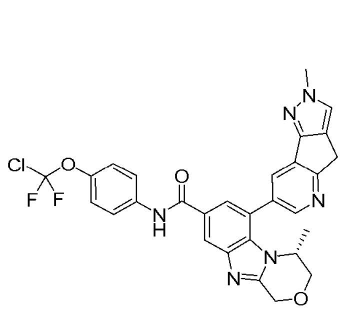,
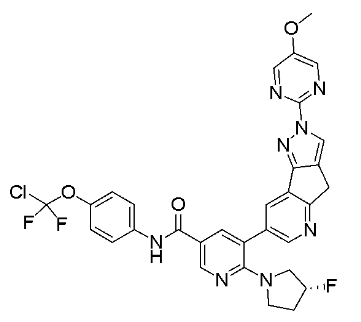,
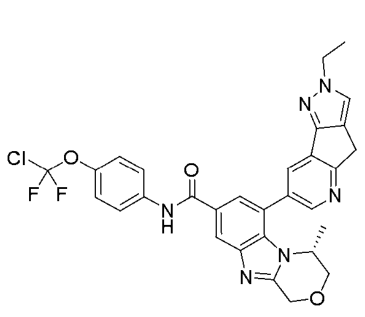,

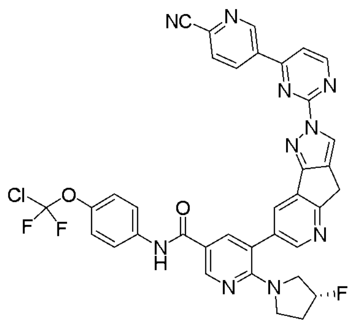

,

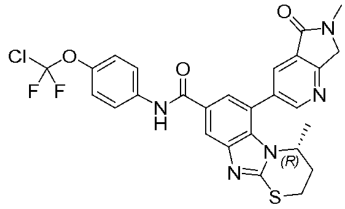

,

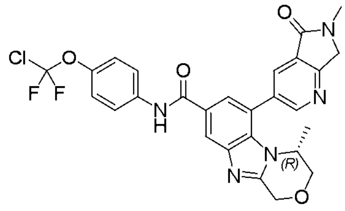

,

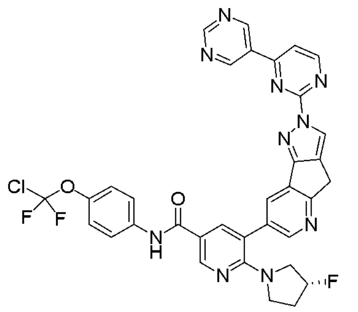

,

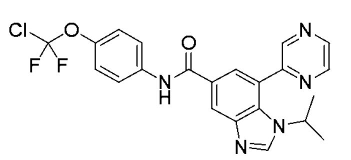

,

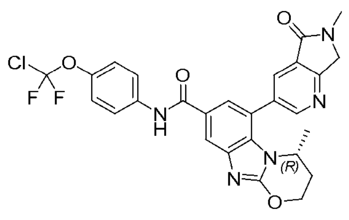

,

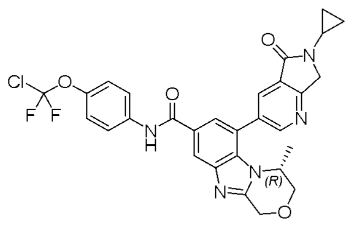

,

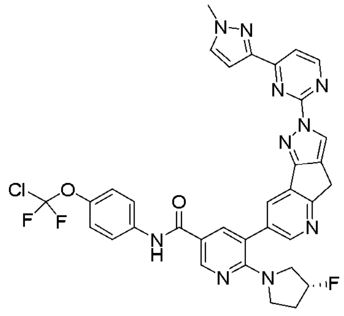

,

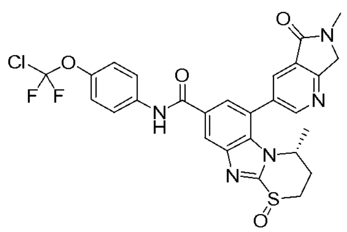

,

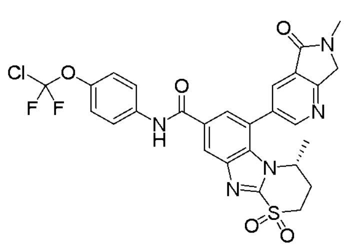

,

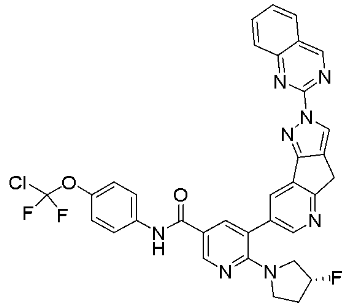

,

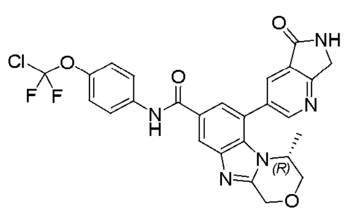

,

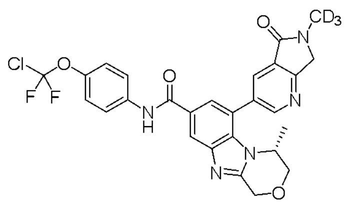

,

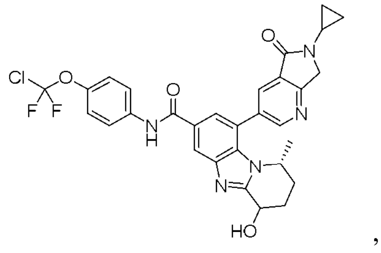

,

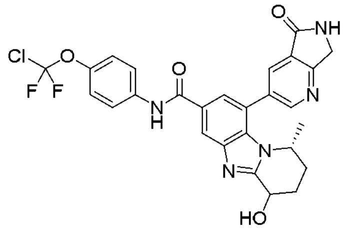

,

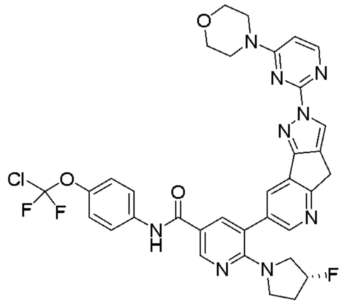 , 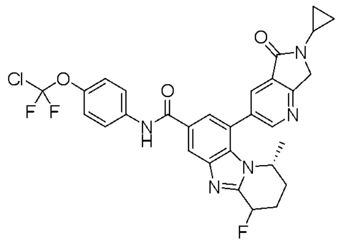 , 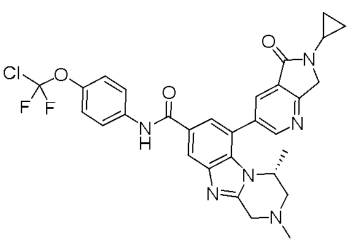 ,

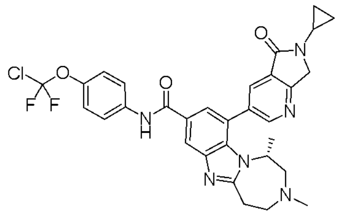 , 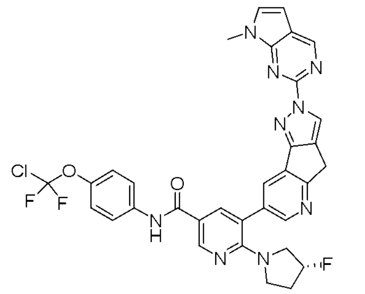 , 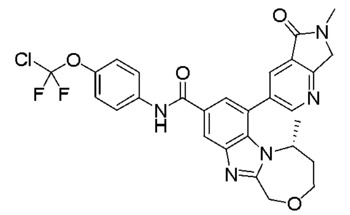 ,

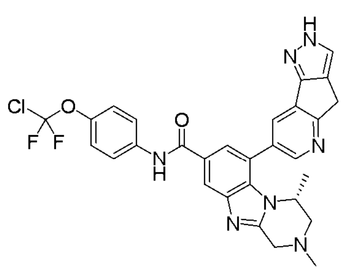 , 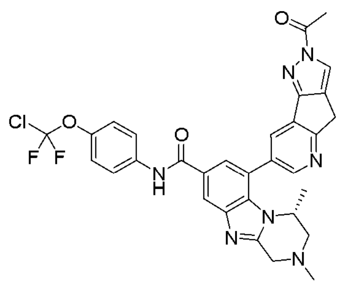 , 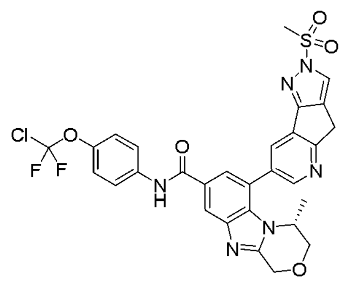 ,

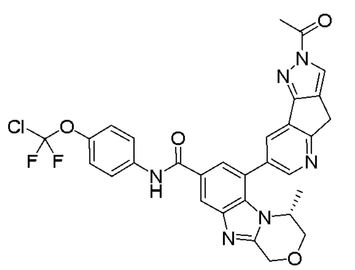 , 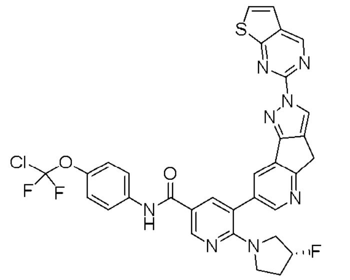 , 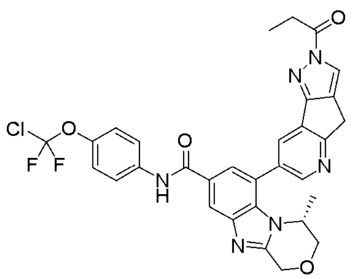 ,

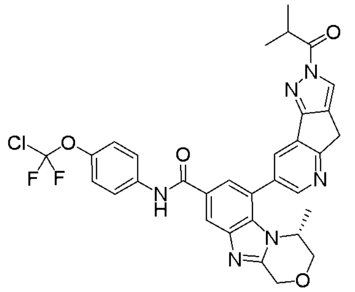 , 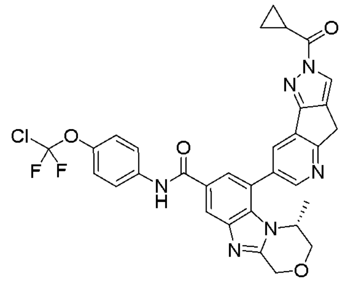 , 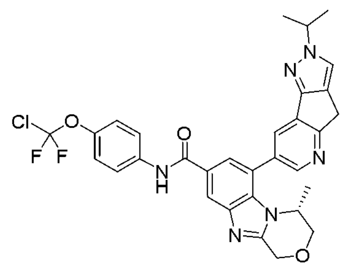 ,

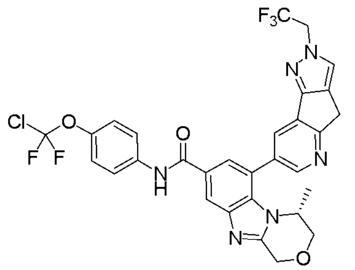
,
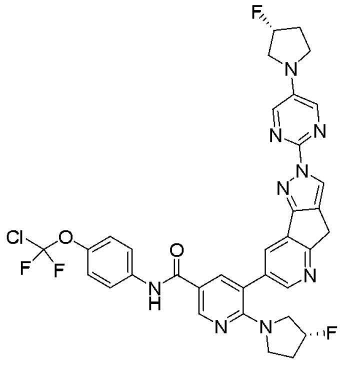
,
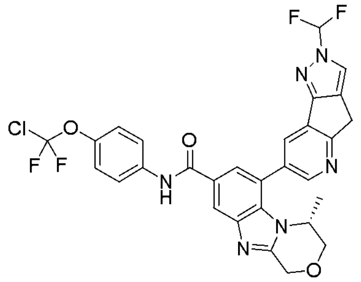
,

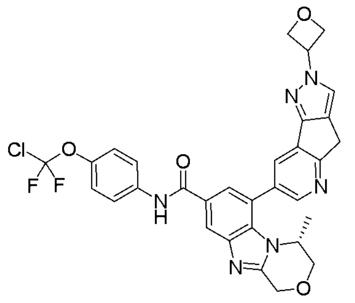
,
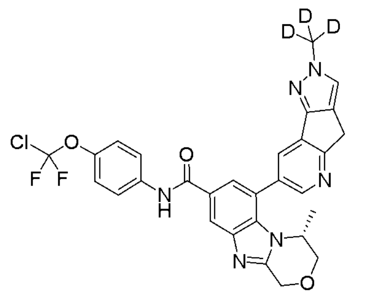
,
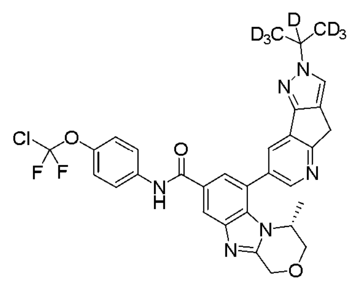
,

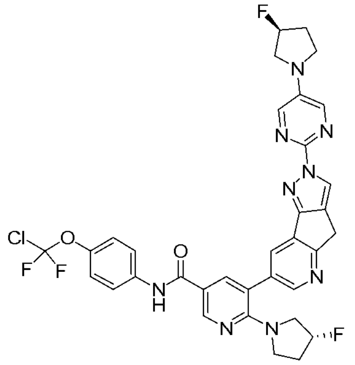
,
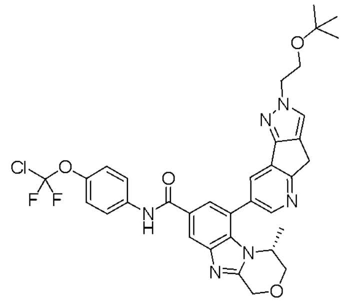
,
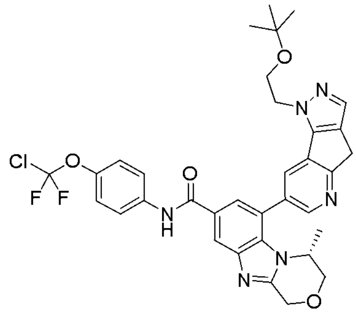
,

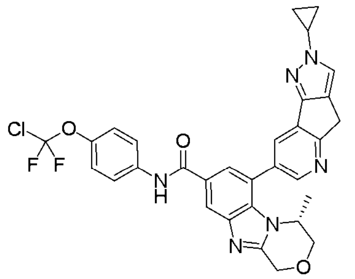
,
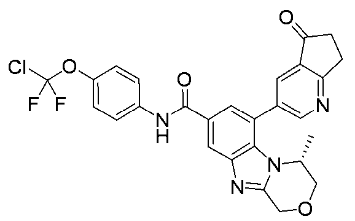
,
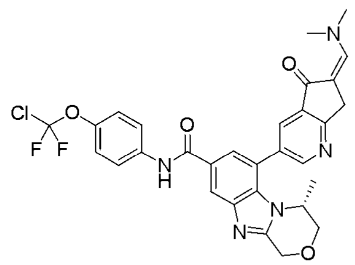
,

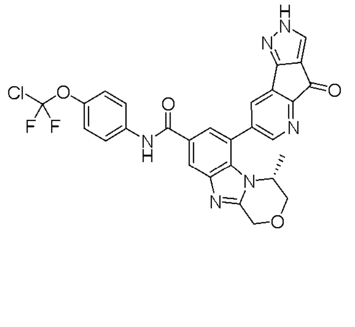
,
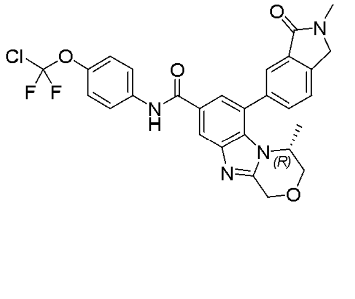
,
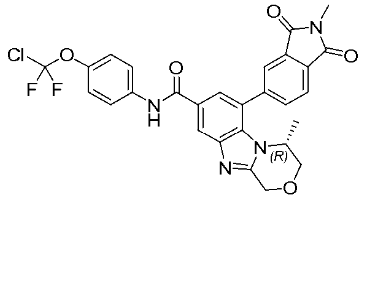
,

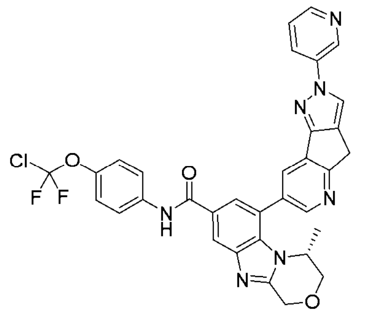 , 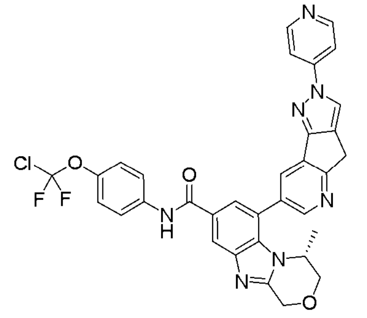 , 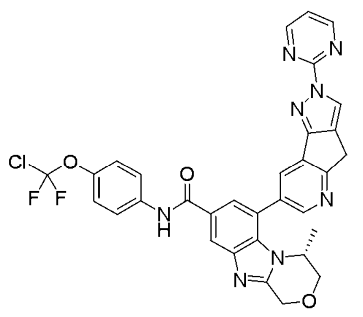 ,

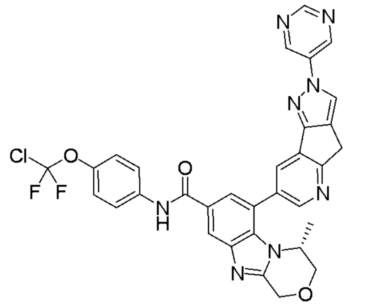 , 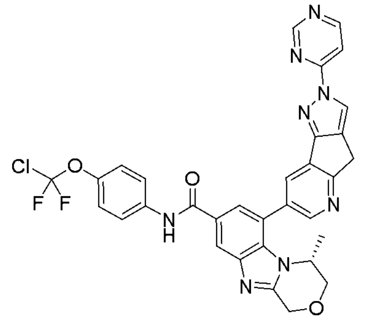 , 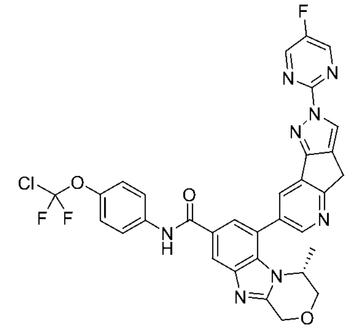 ,

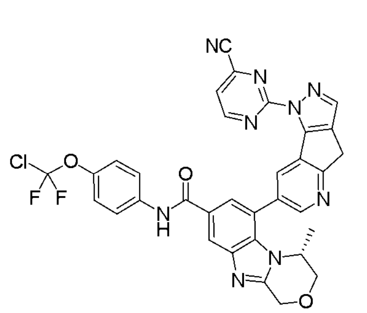 , 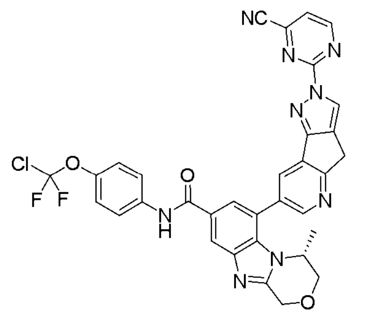 , 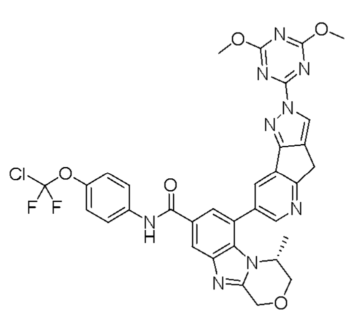 ,

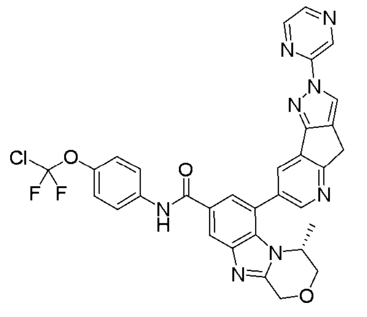 , 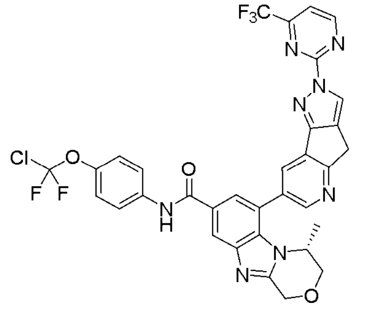 , 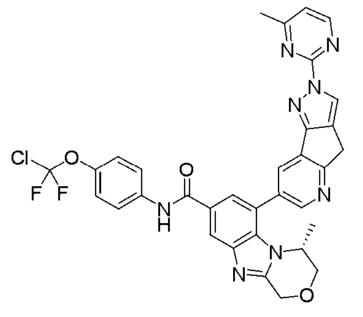 ,

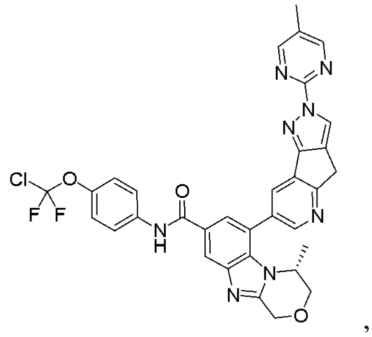 , 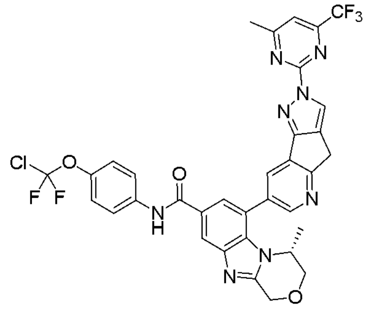 , 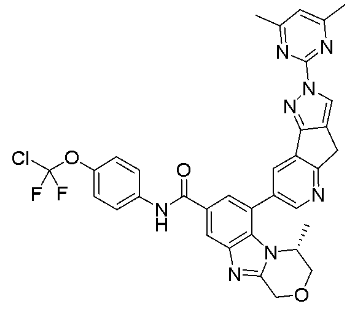 ,

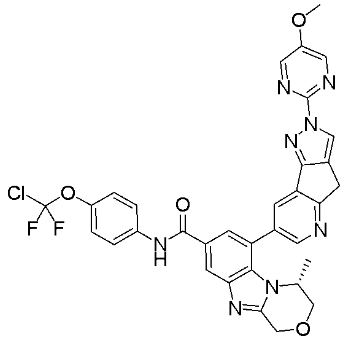,
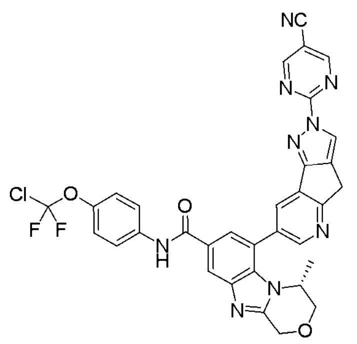,
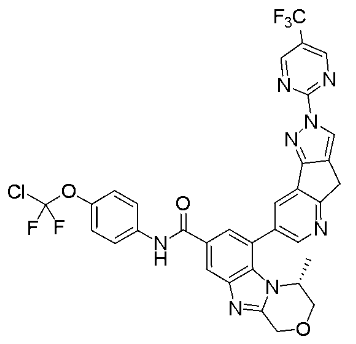,

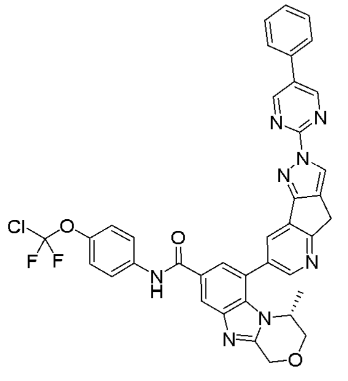,
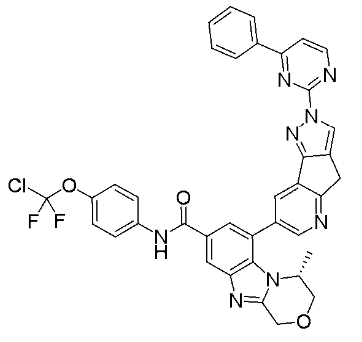,
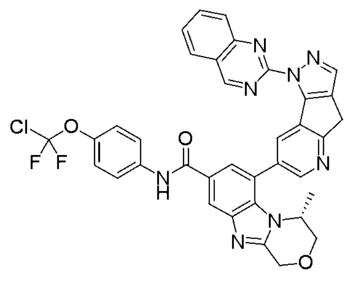,

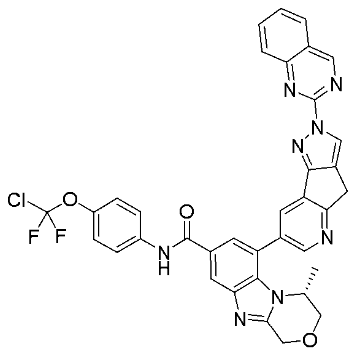,
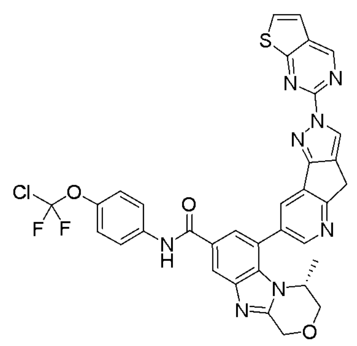,
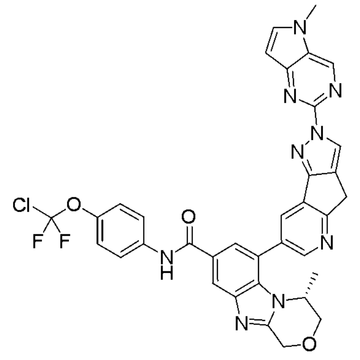,

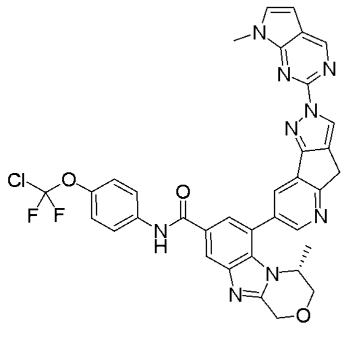,
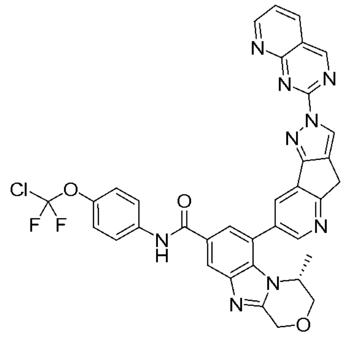,
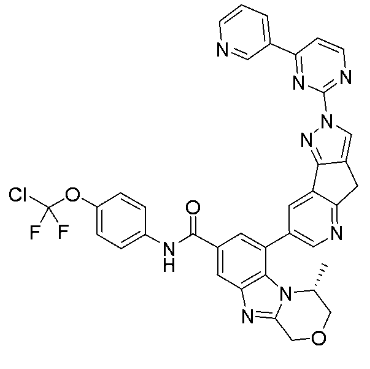,

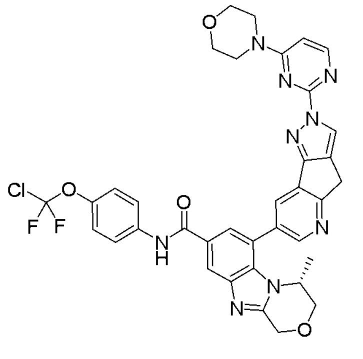
,
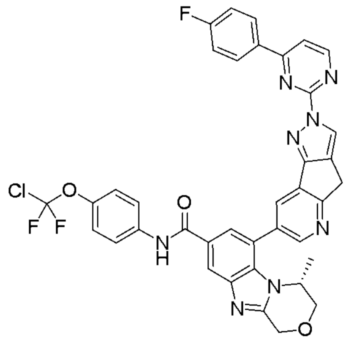
,
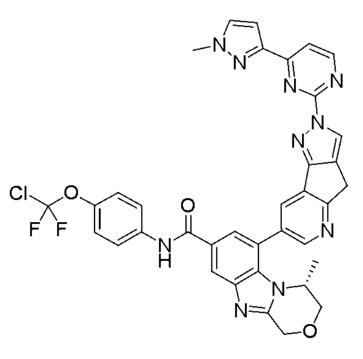
,

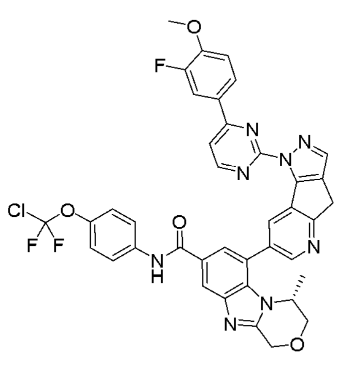
,
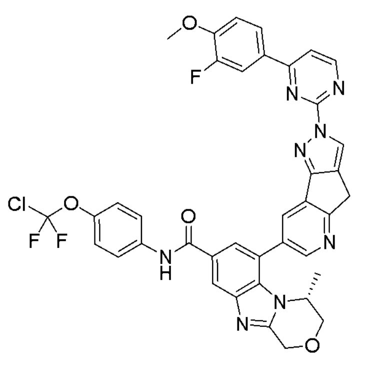
,
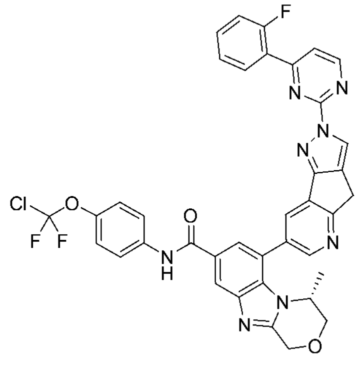
,

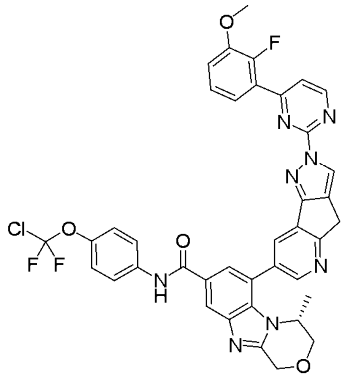
,
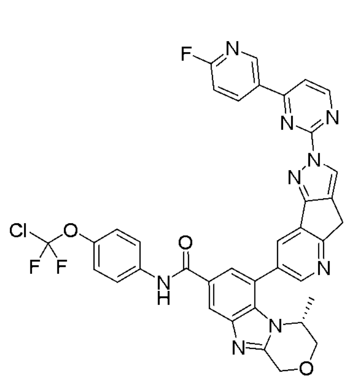
,
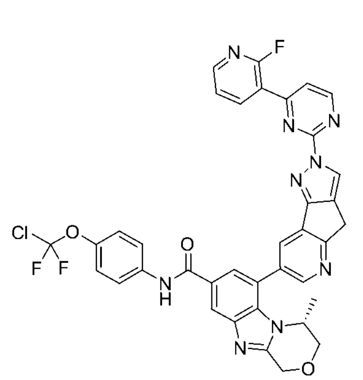
,

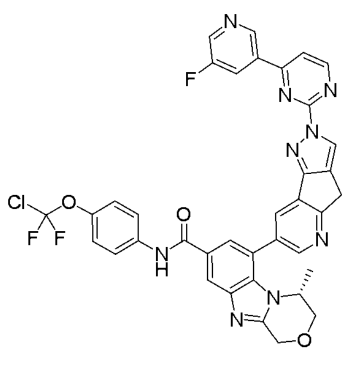
,
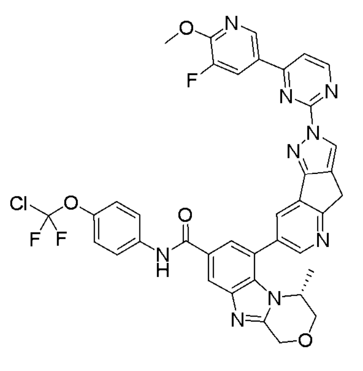
,
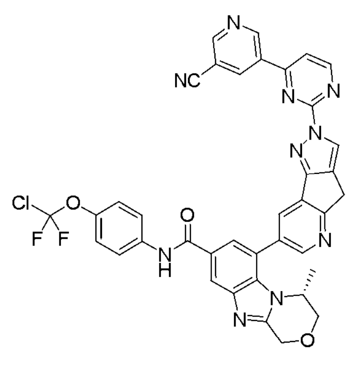
,

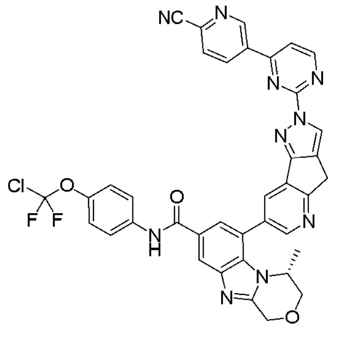,
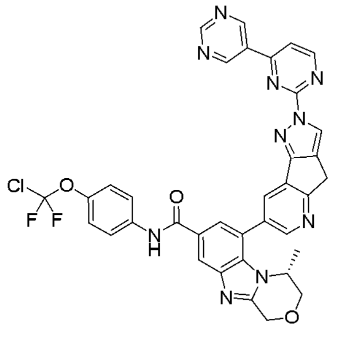,
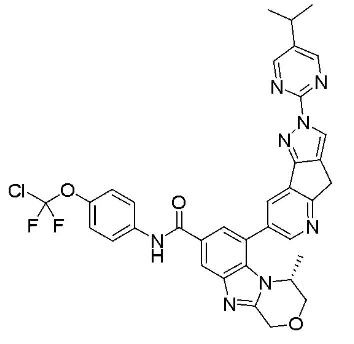,

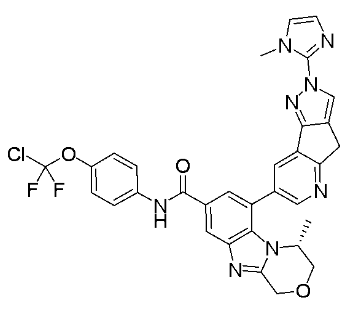,
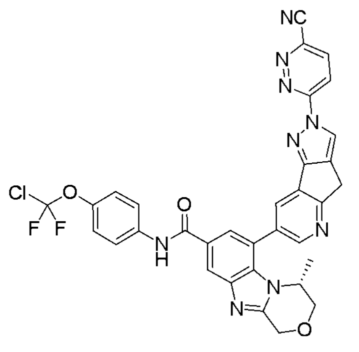,
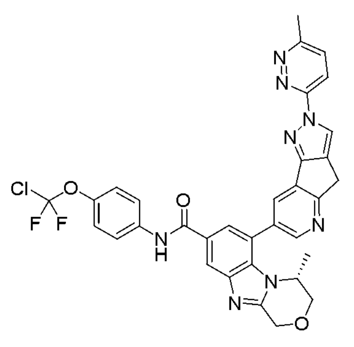,

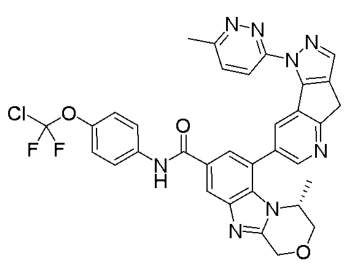,
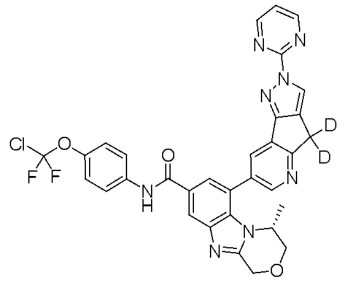,
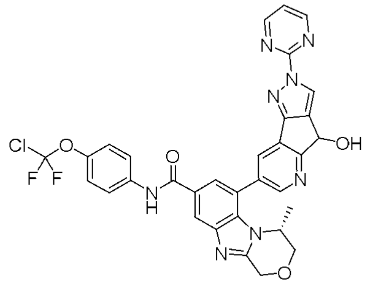,

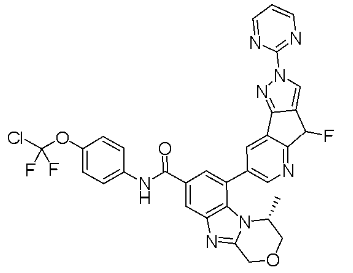,
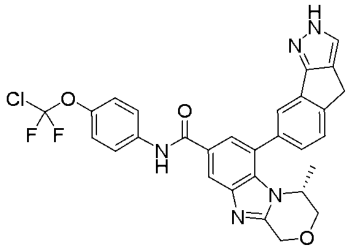,
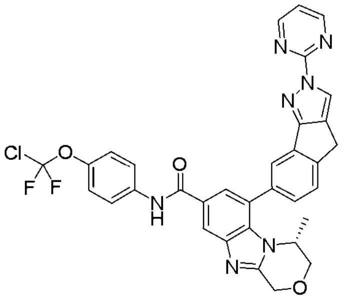,

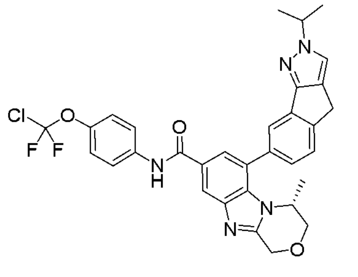,
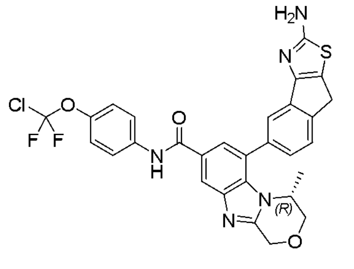,
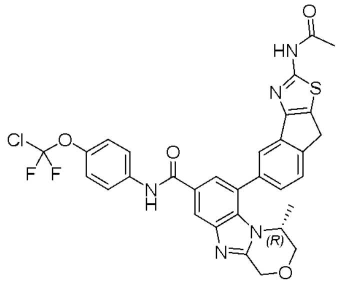,

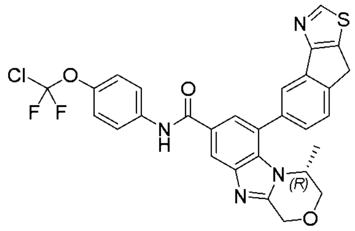 , 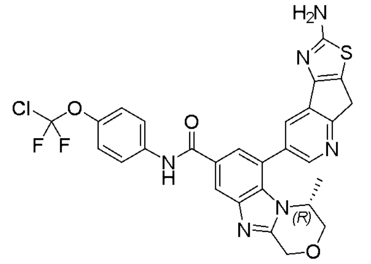 , 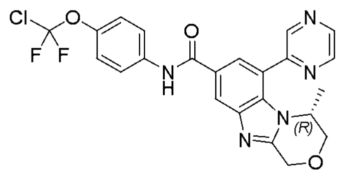 ,

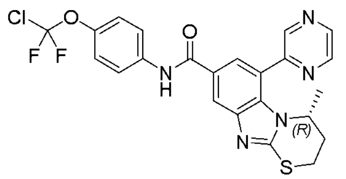 , 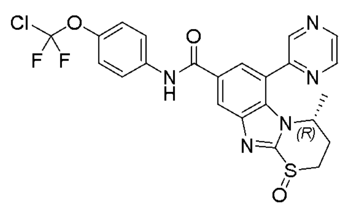 , 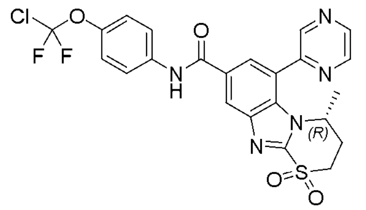 ,

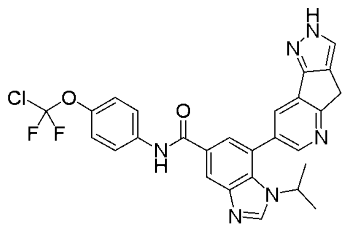 , 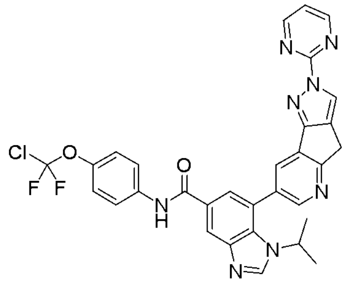 , 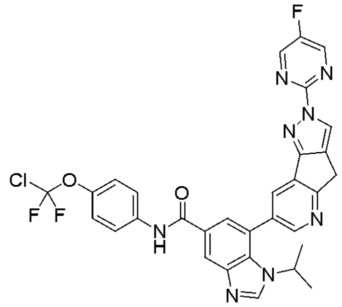 ,

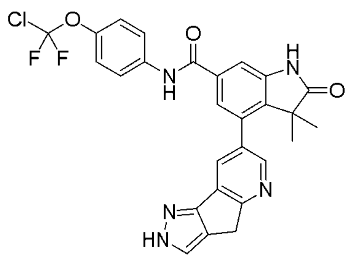 , 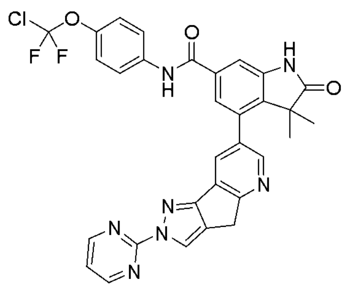 , 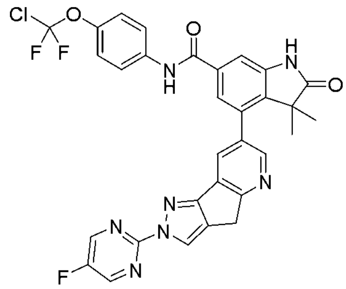 ,

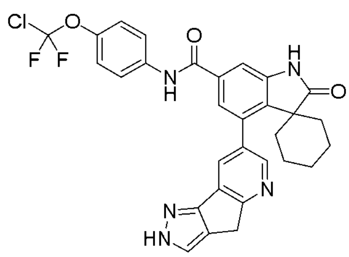 , 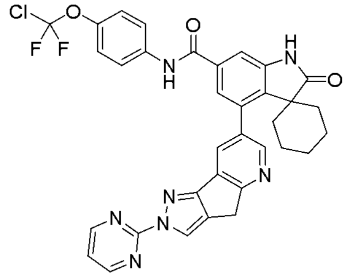 , 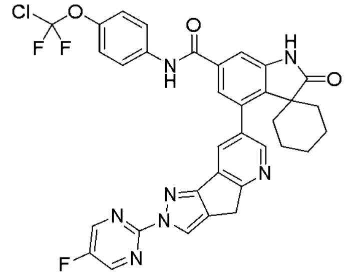 ,

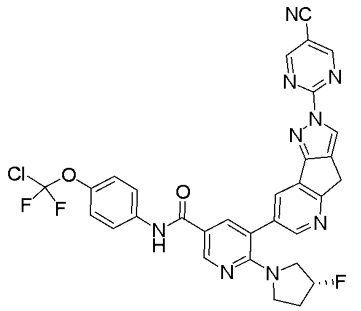
,
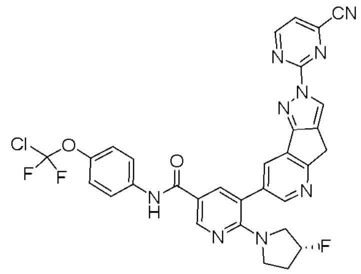
,
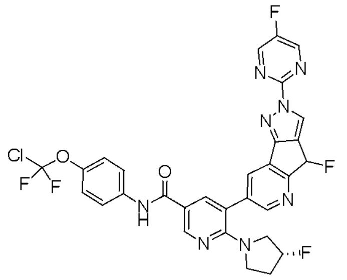
,

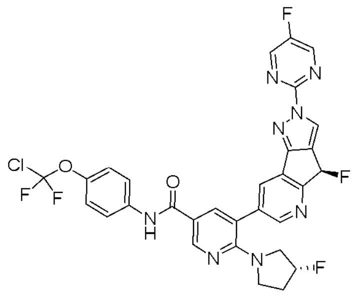
,
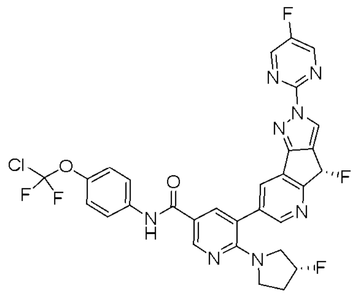
,
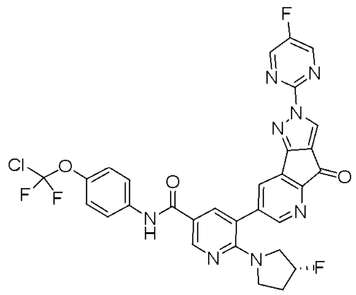
,

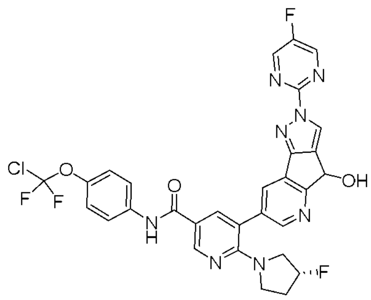
,
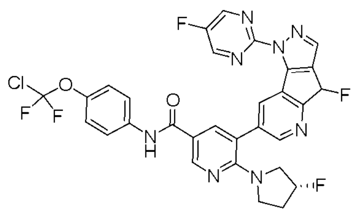
,
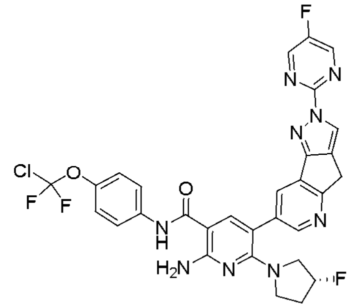
,

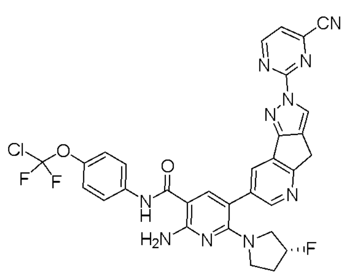
,
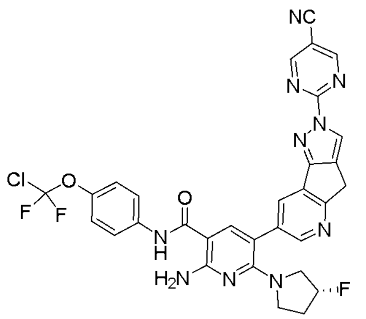
,
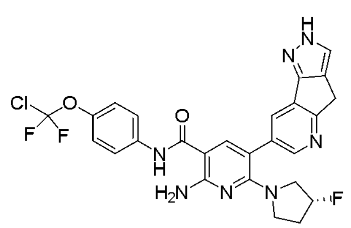
,

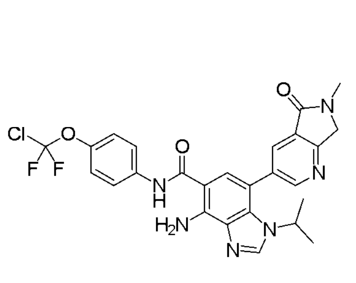
,
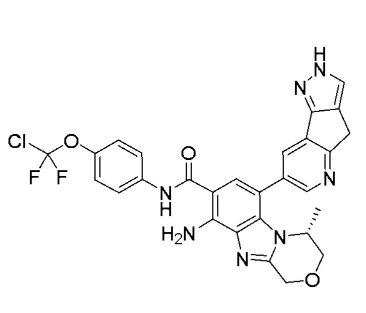
,
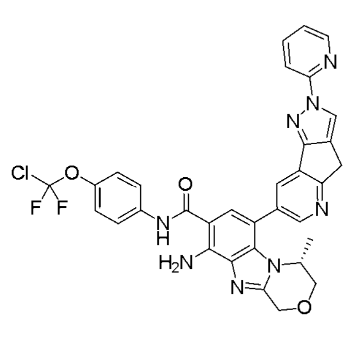
,

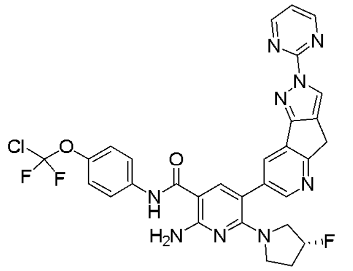,

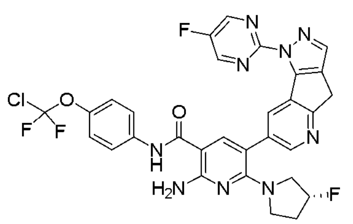,

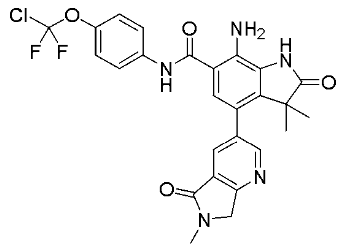,

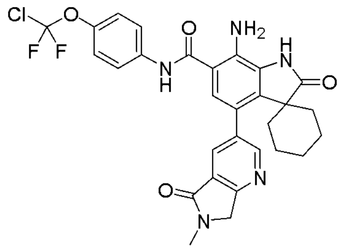,

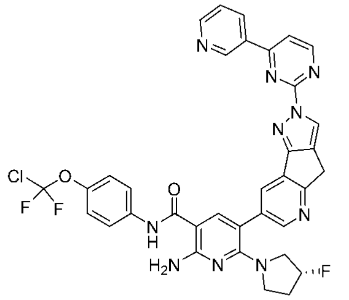,

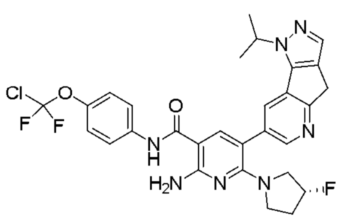,

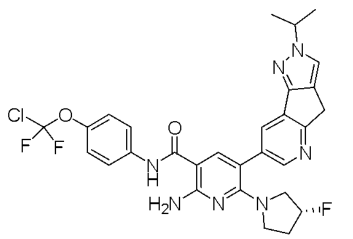,

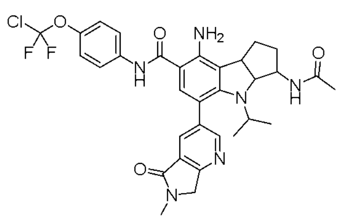,

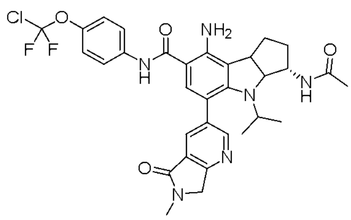,

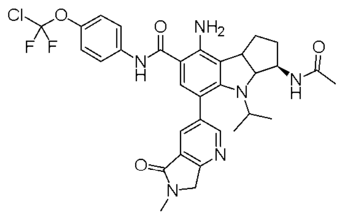,

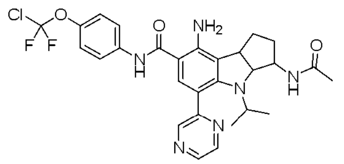,

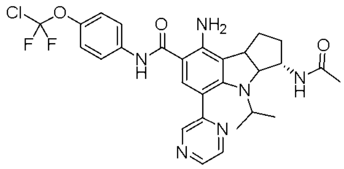,

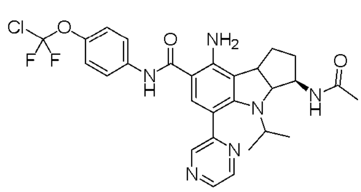,

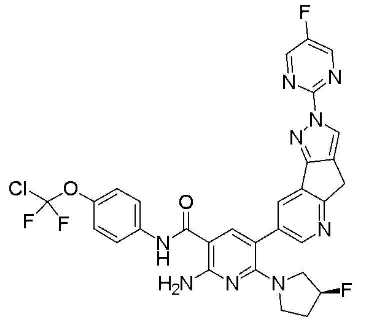,

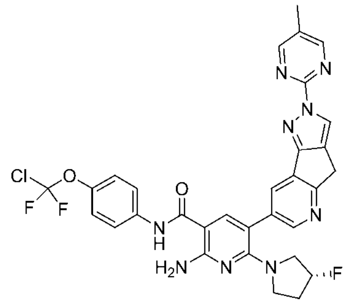,

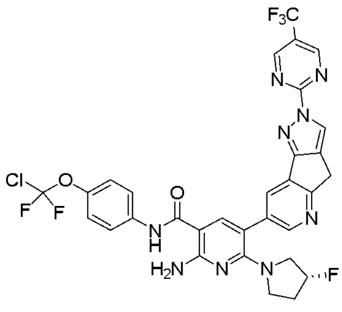,
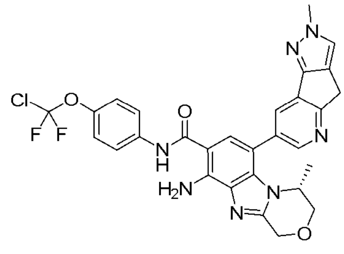,
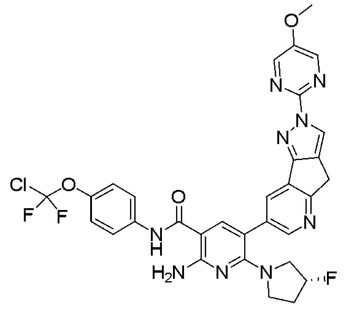,

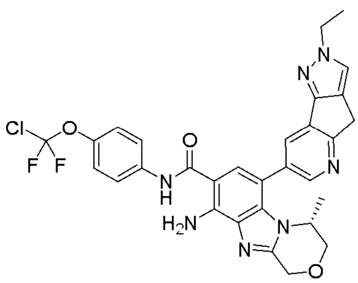,
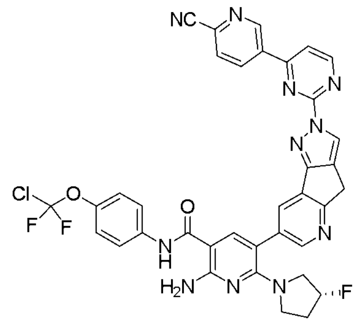,
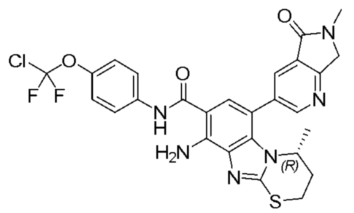,

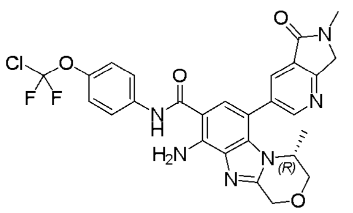,
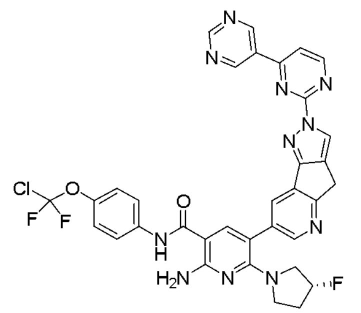,
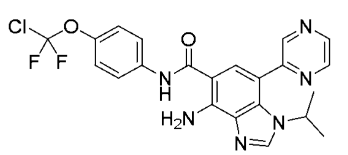,

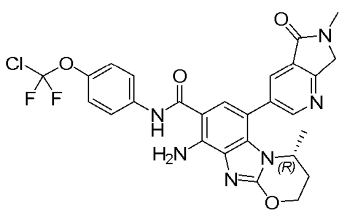,
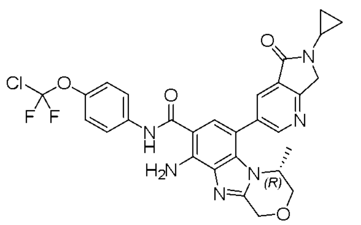,
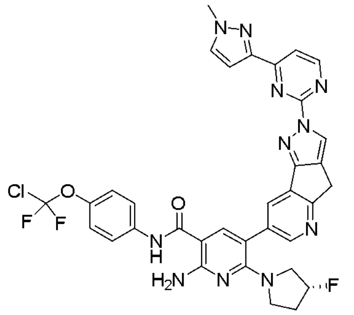,

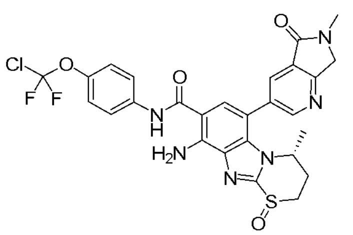,
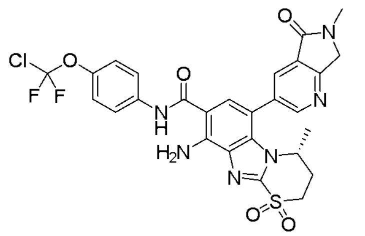,
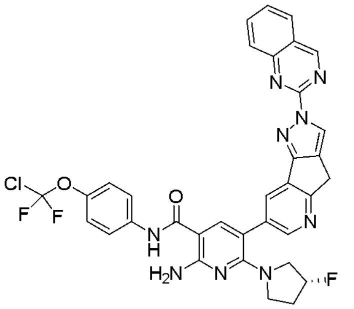,

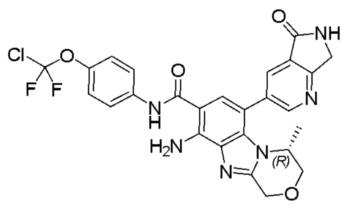,

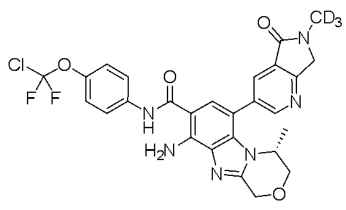,

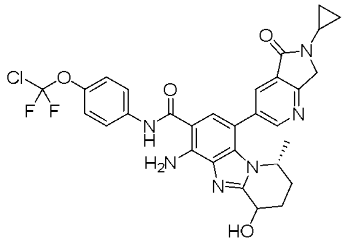,

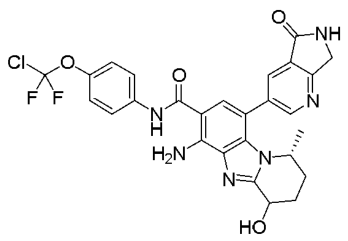,

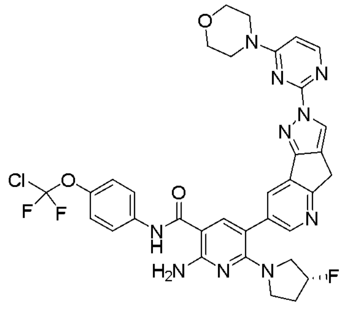,

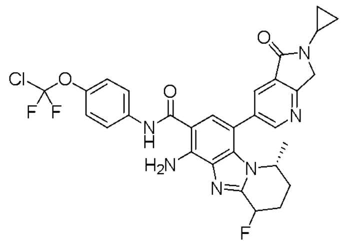,

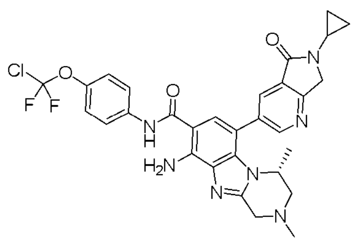,

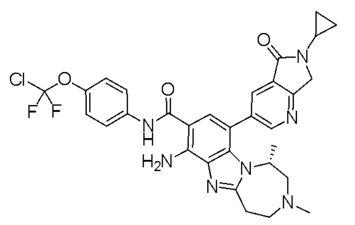,

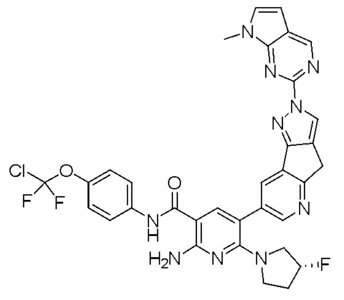,

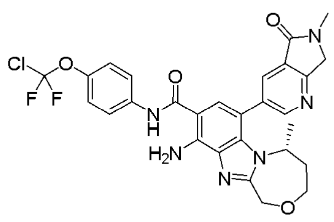,

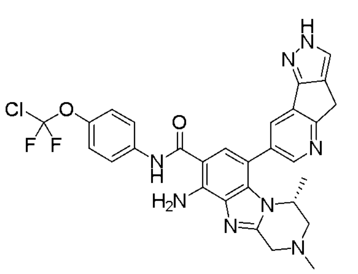,

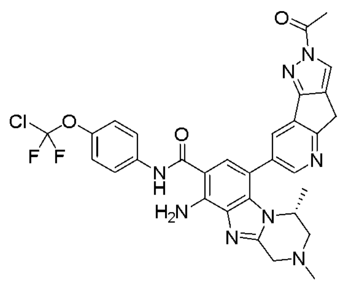,

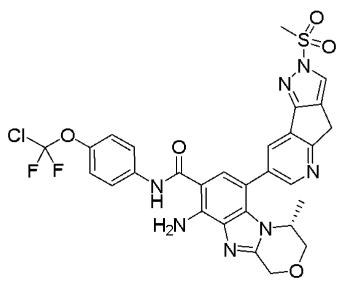,

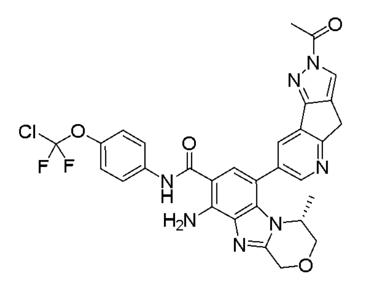,

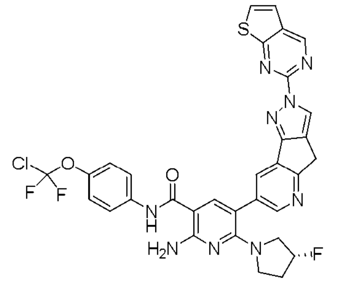,

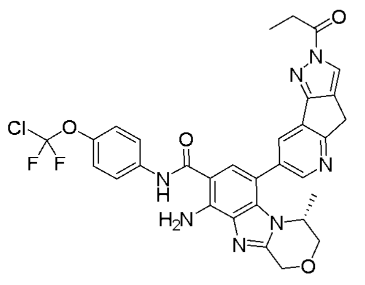 , 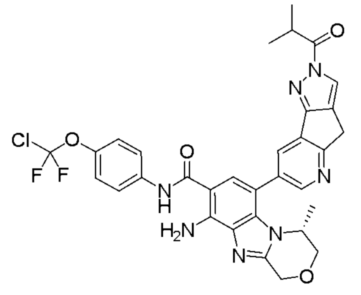 , 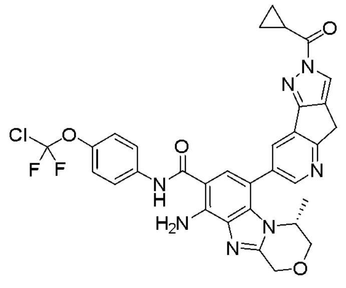 ,

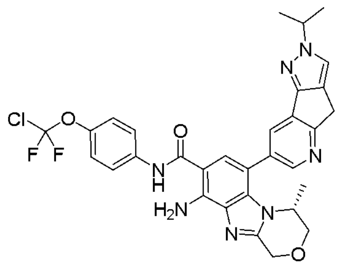 , 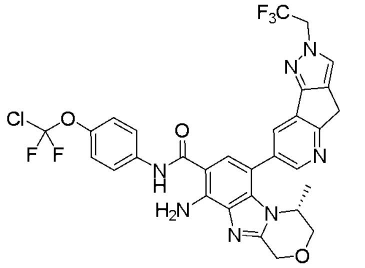 , 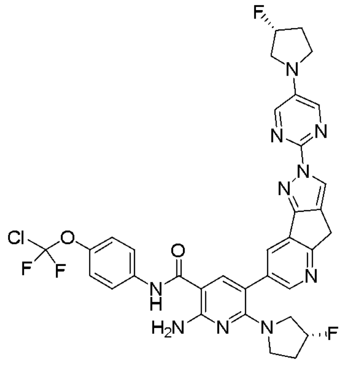 ,

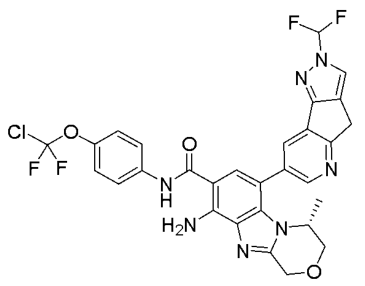 , 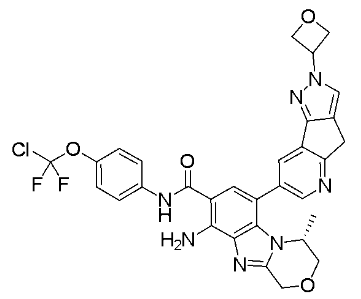 , 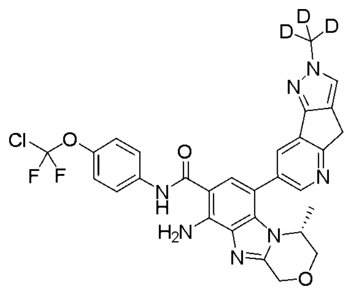 ,

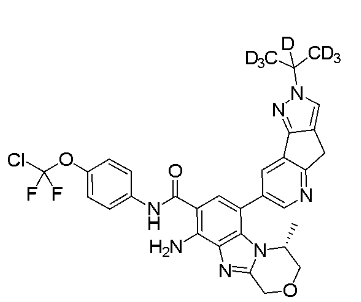 , 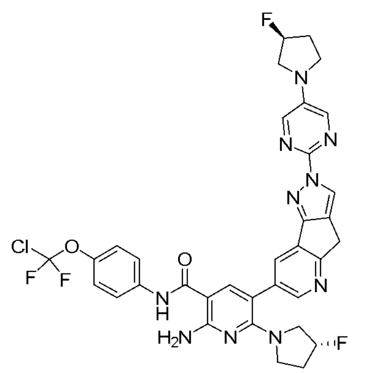 , 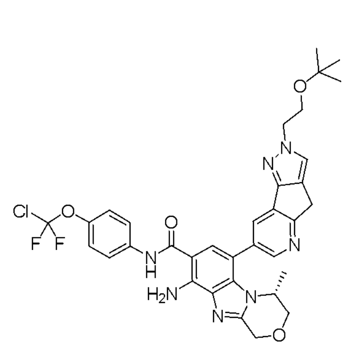 ,

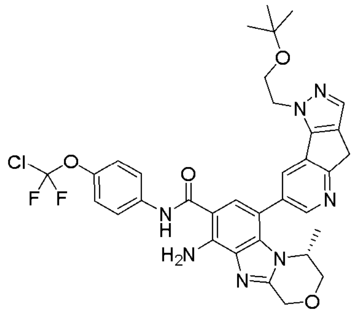
,
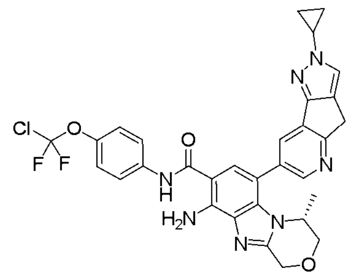
,
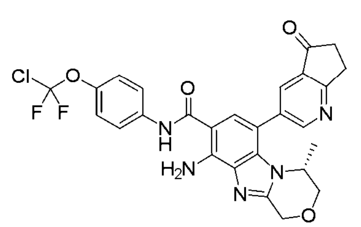
,

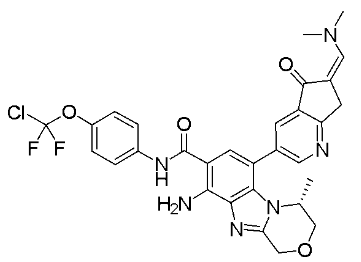
,
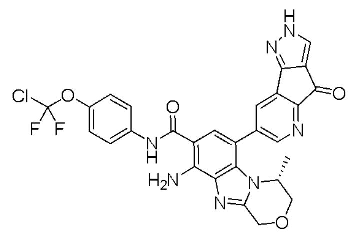
,
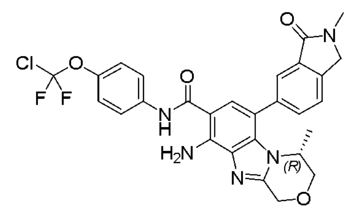
,

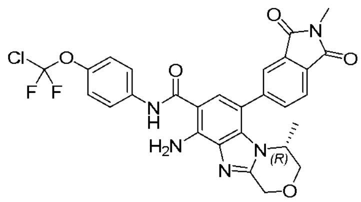
,
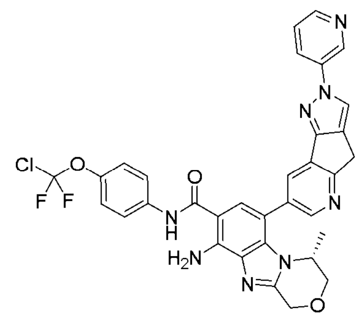
,
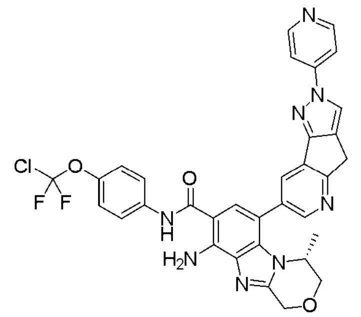
,

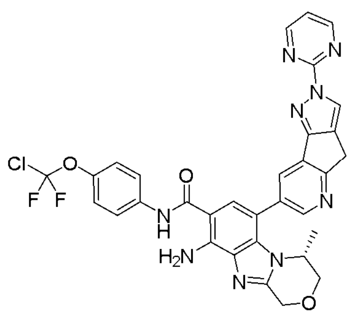
,
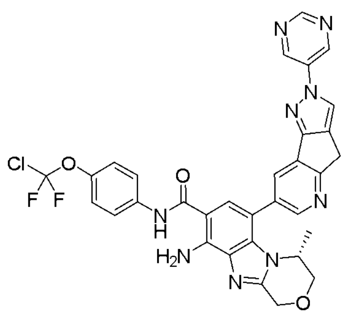
,
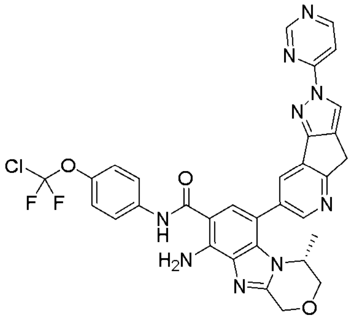
,

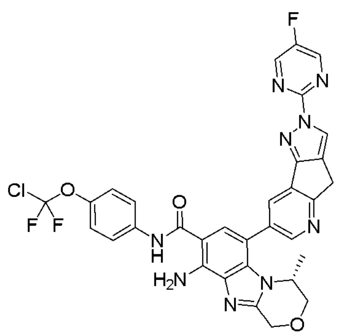
,
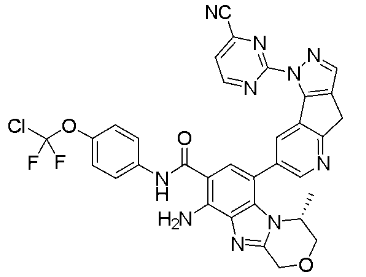
,
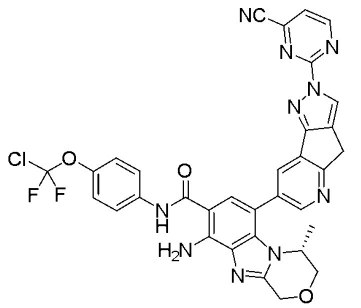
,

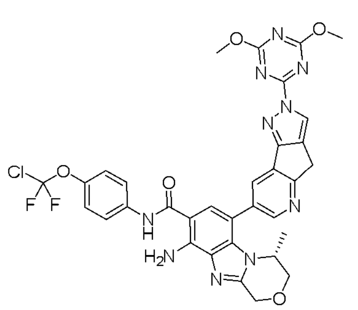,

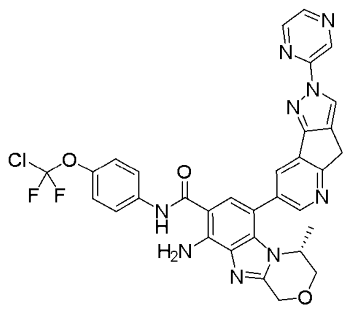,

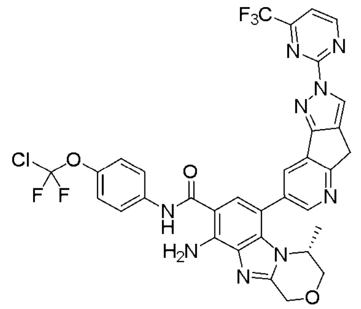,

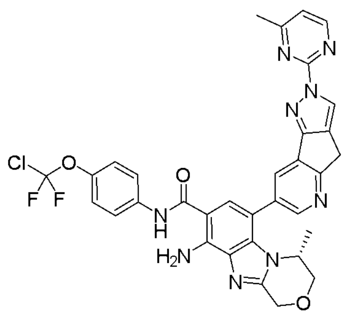,

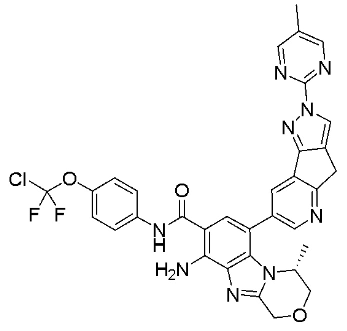,

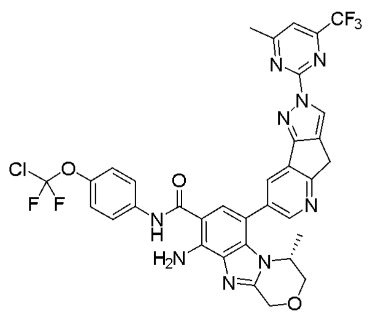,

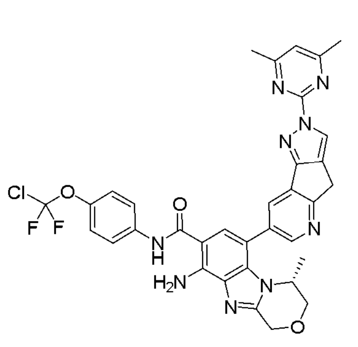,

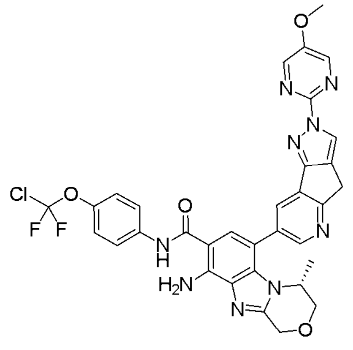,

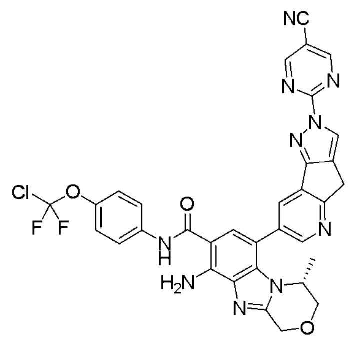,

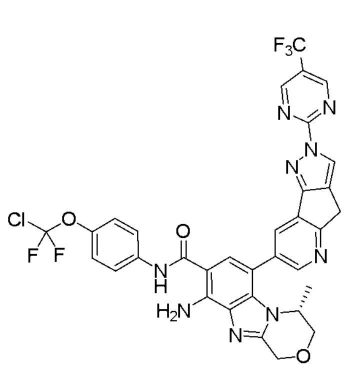,

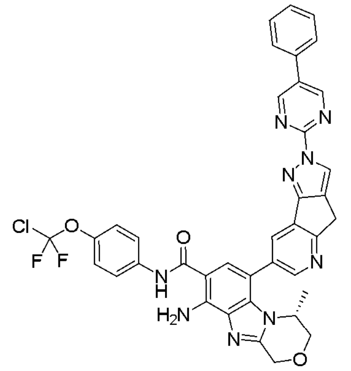,

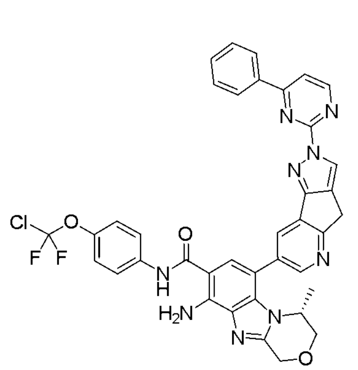,

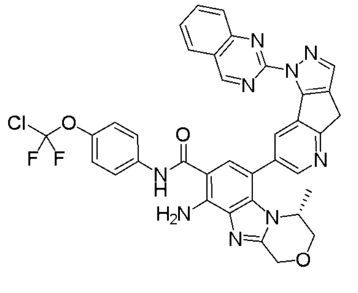 ,

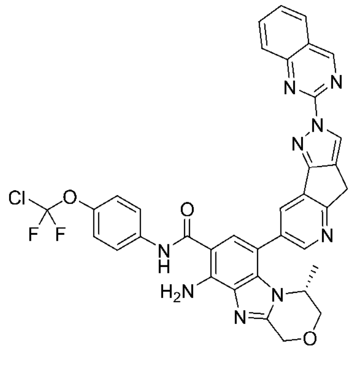 ,

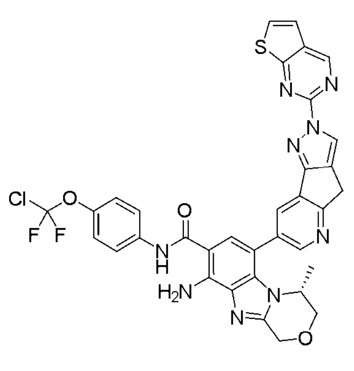 ,

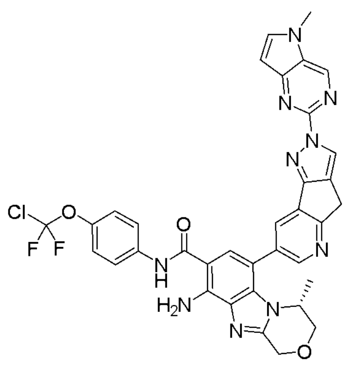 ,

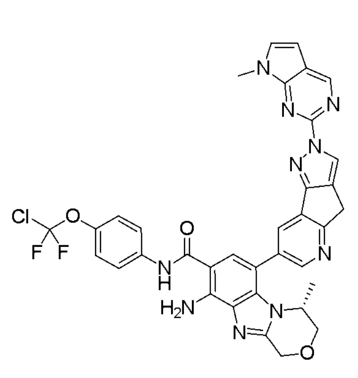 ,

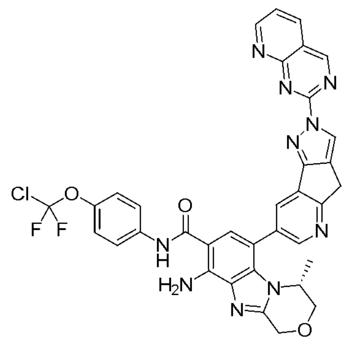 ,

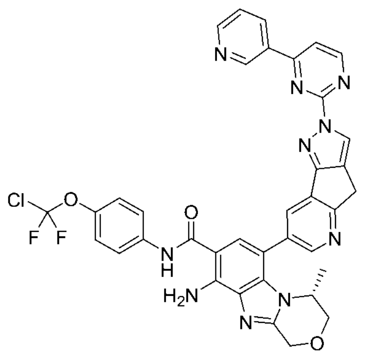 ,

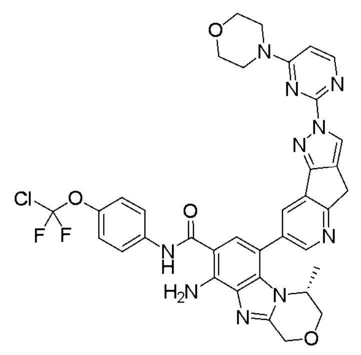 ,

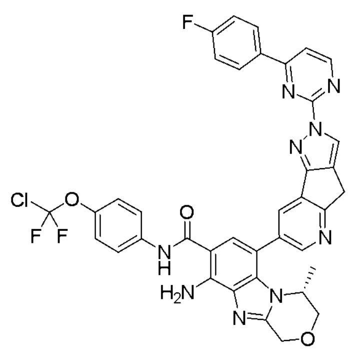 ,

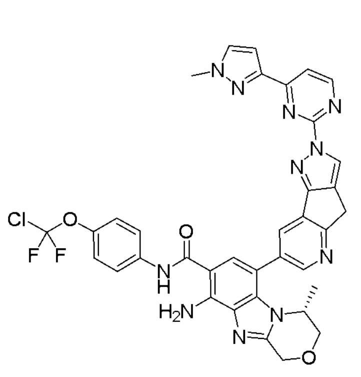 ,

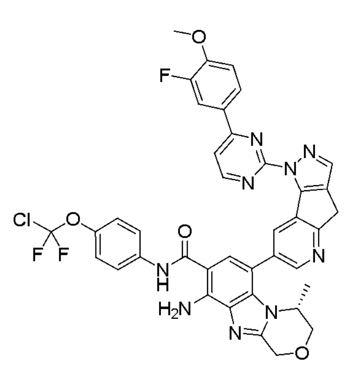 ,

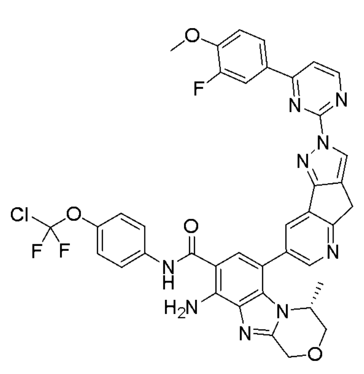 ,

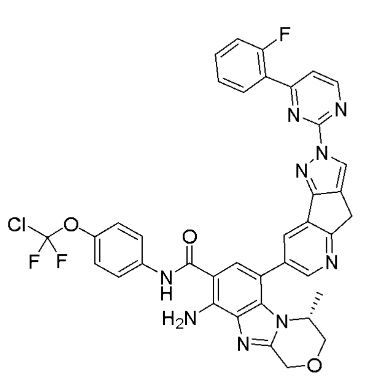,
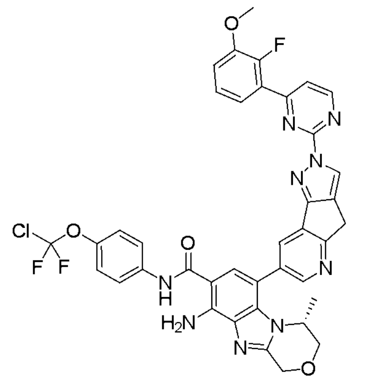,
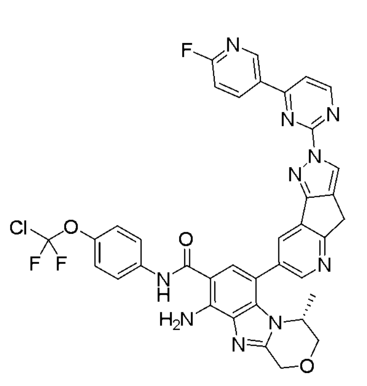,

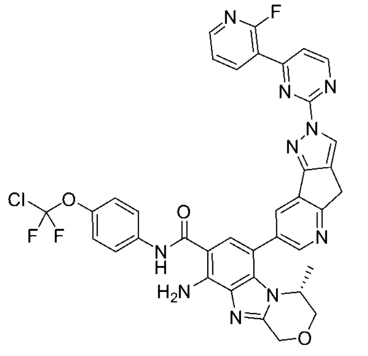,
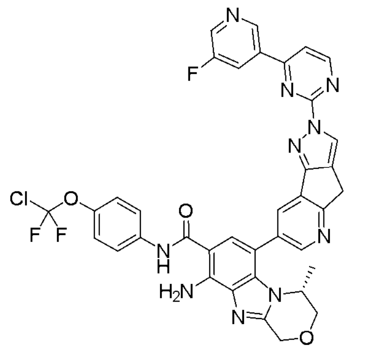,
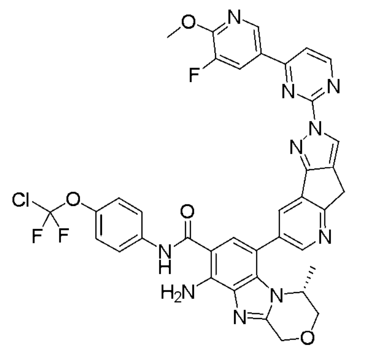,

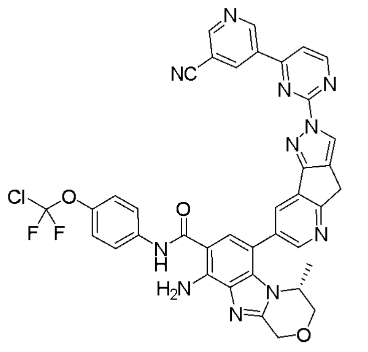,
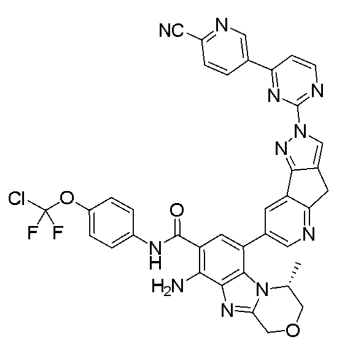,
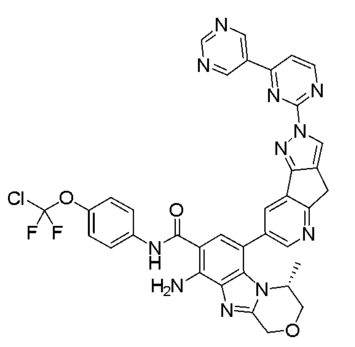,

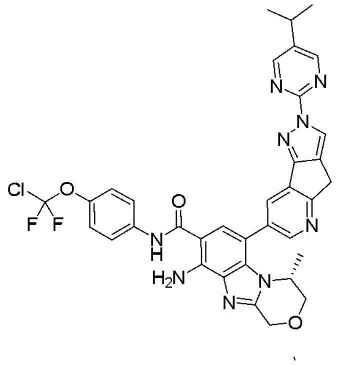,
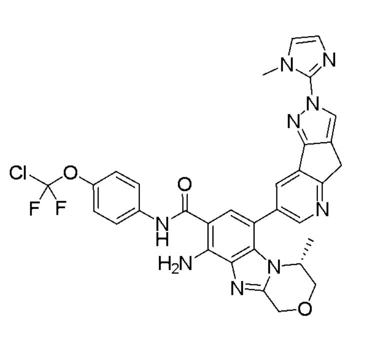,
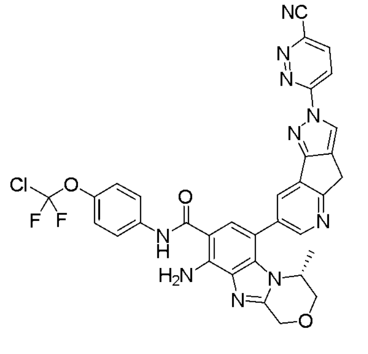,

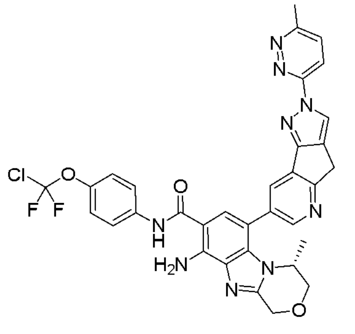,
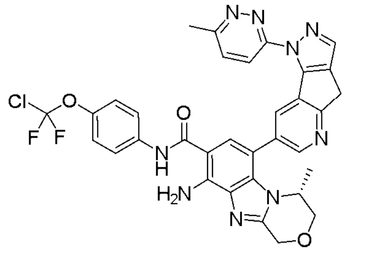,
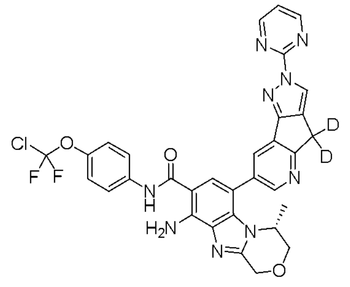,

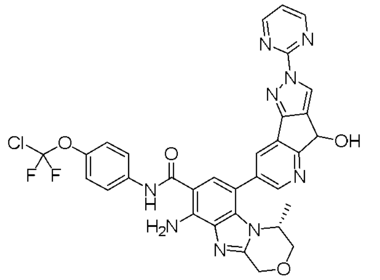,
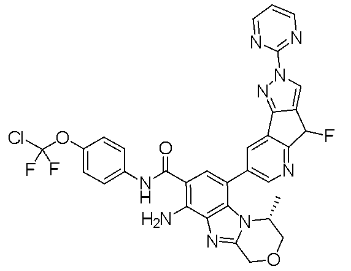,
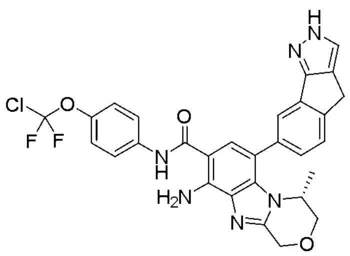,

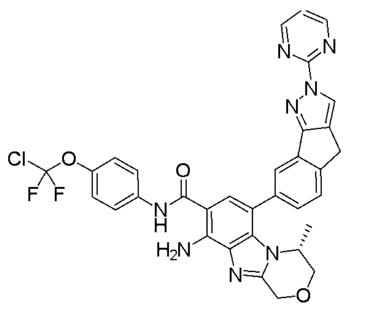,
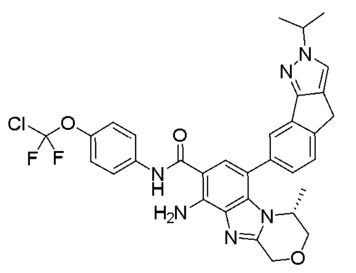,
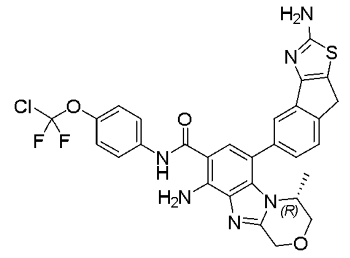,

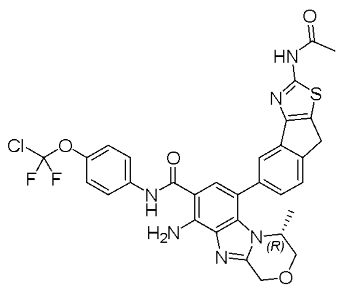,
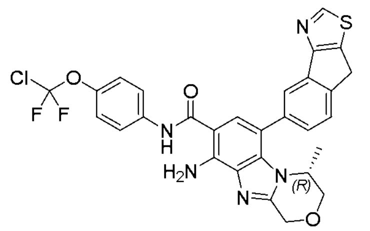,
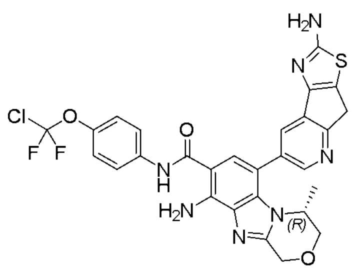,

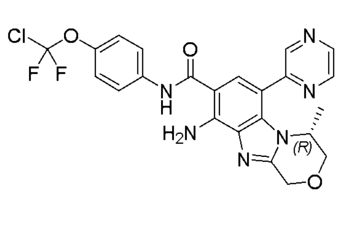,
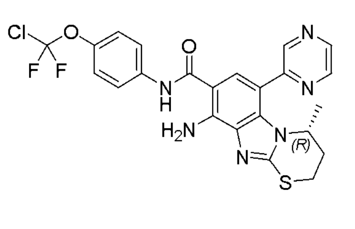,
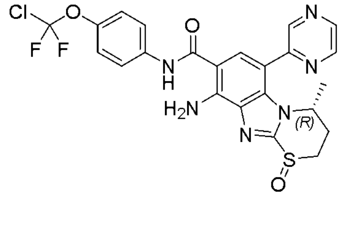,

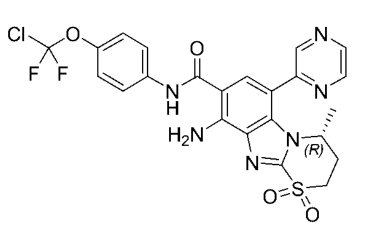,
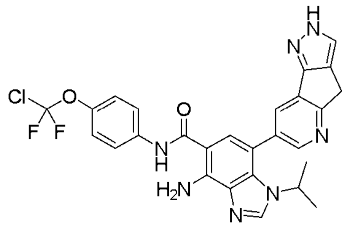,
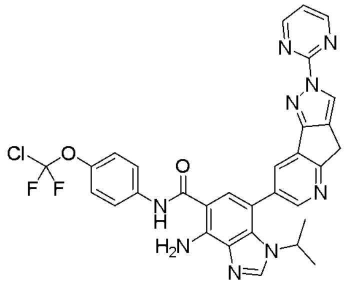,

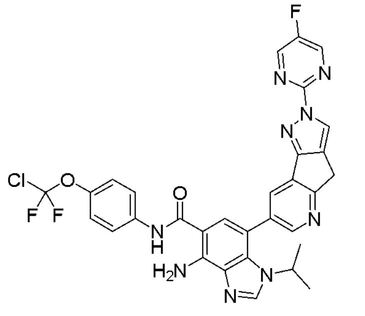,
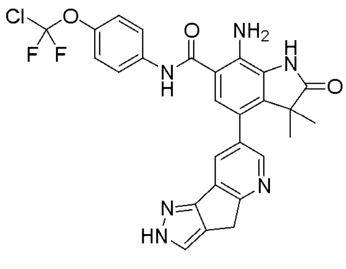,
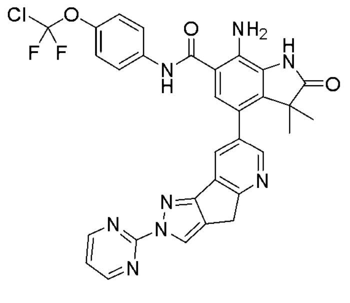,

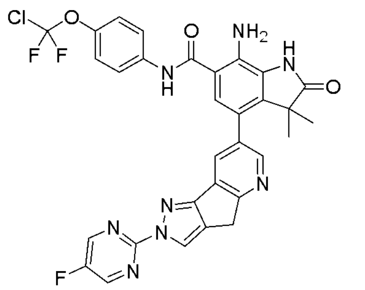,
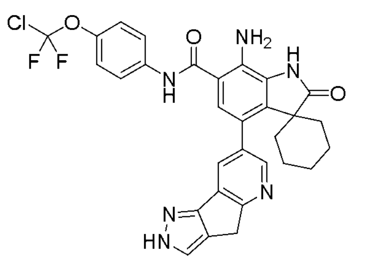,
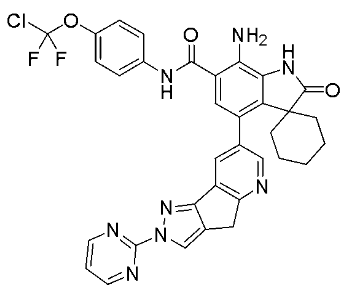,

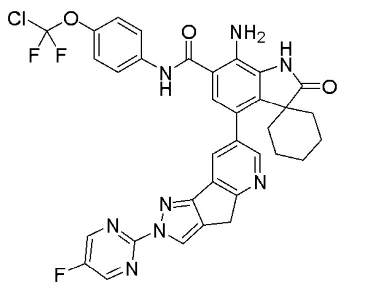,
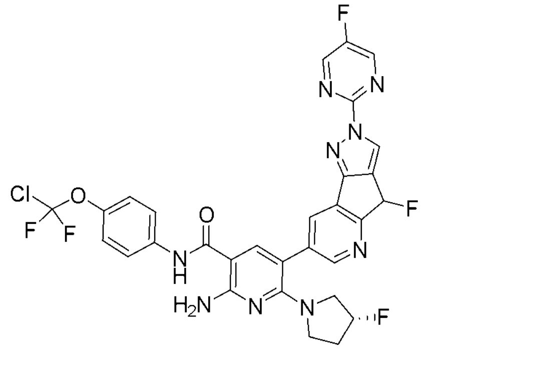,

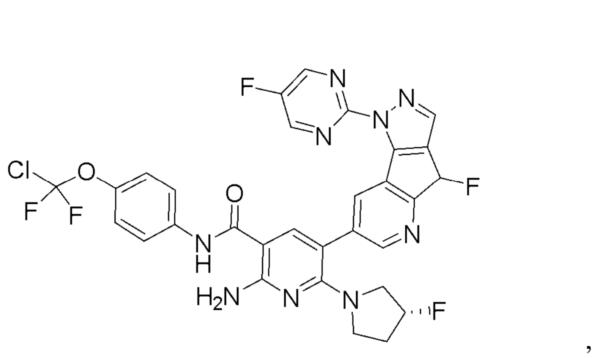,
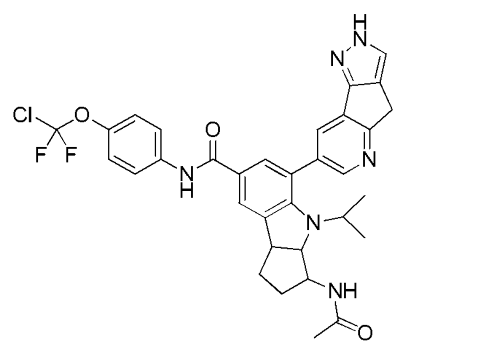,

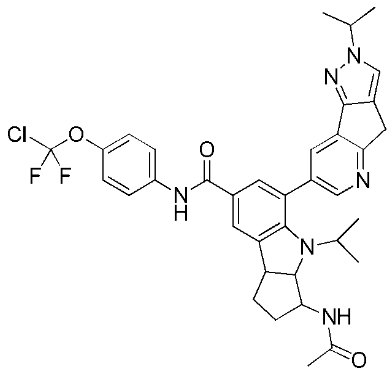
,
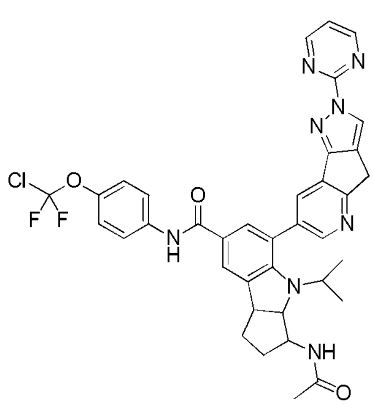
,
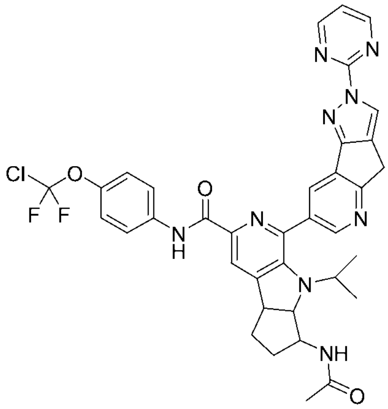
,

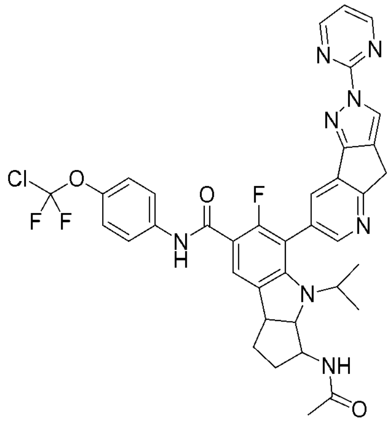
,
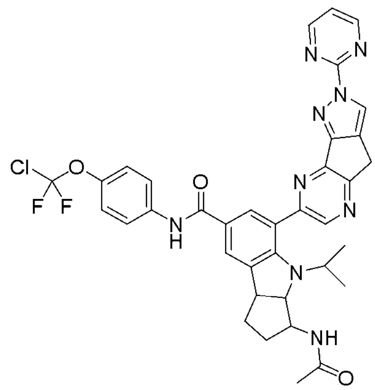
,
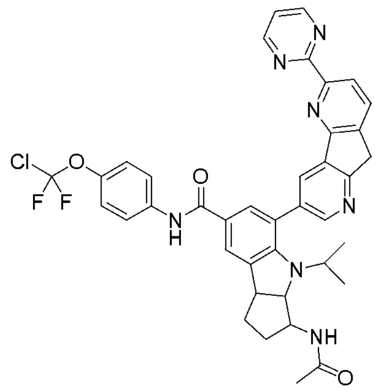
,

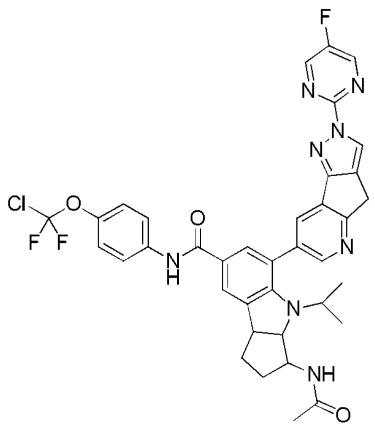
,
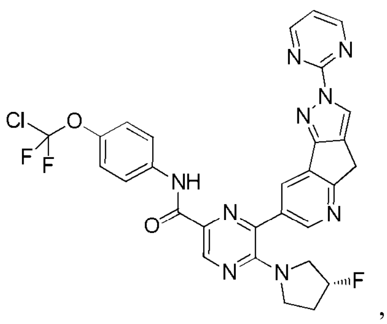
,
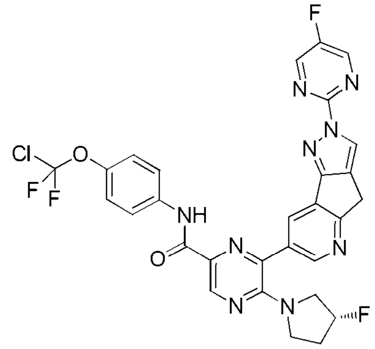
,

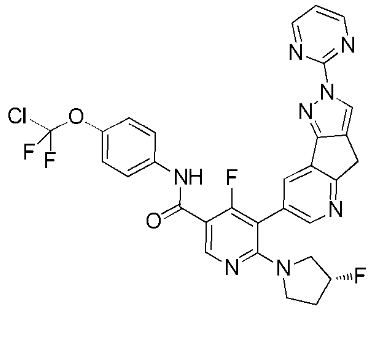
,
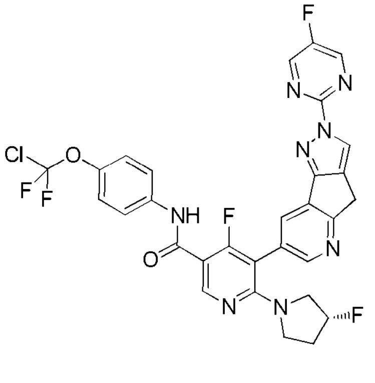
,
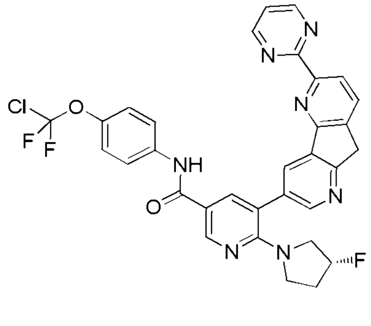
,

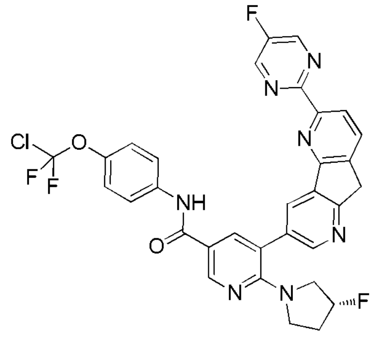
,
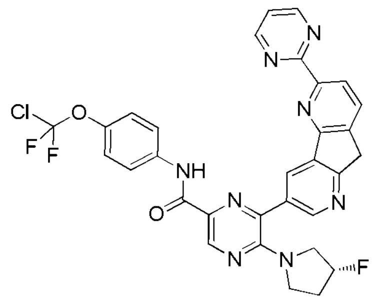
,
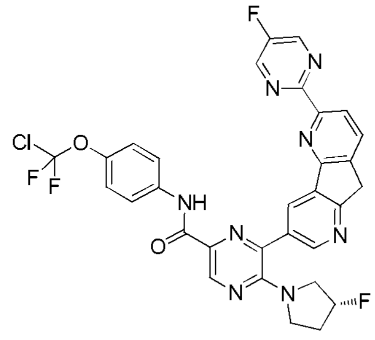
,

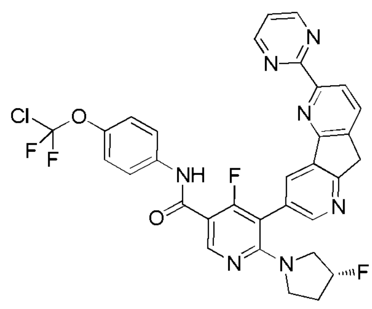
,
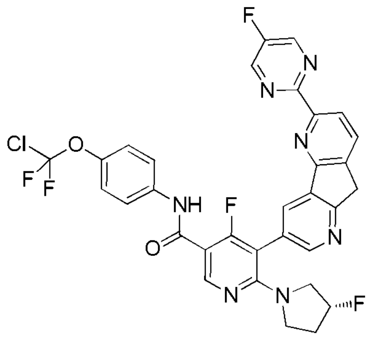
,
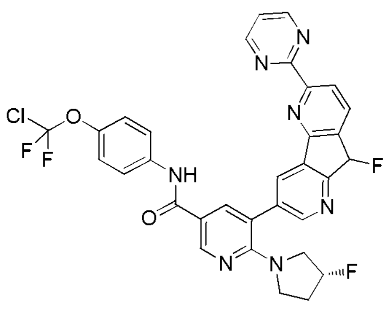
,

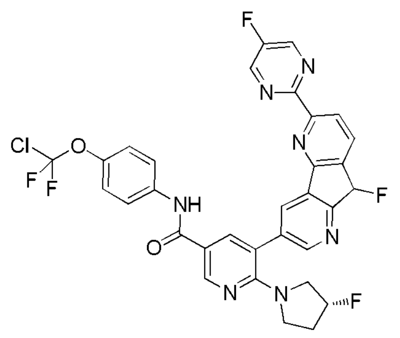
,
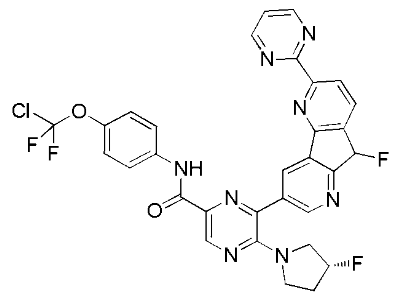
,

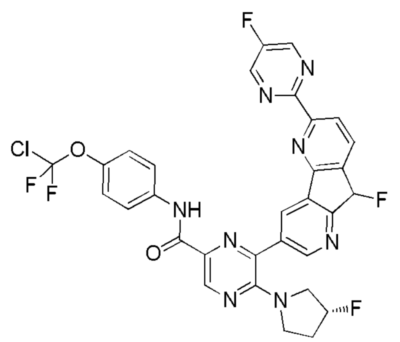
,
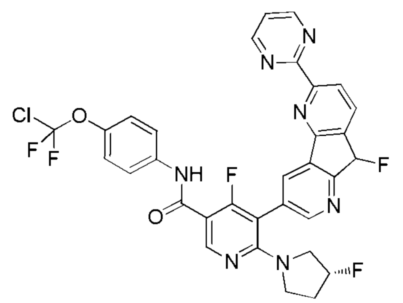
,

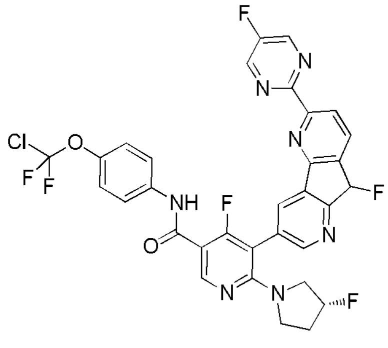,
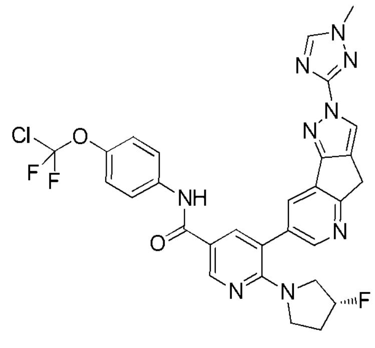,
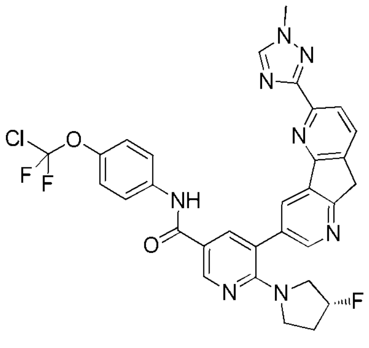,

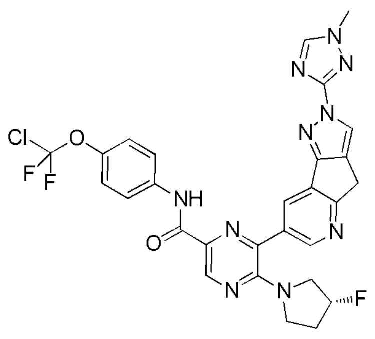,
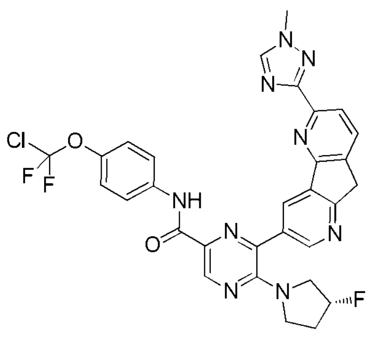,
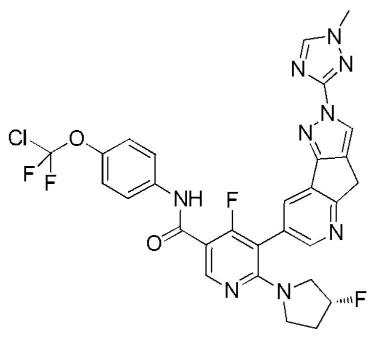,

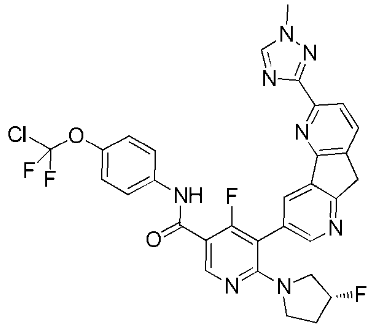,
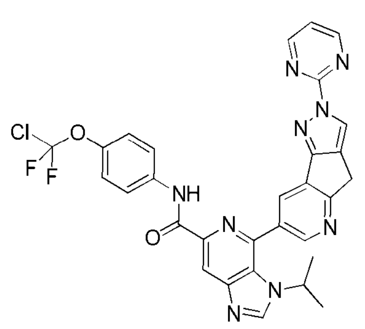,
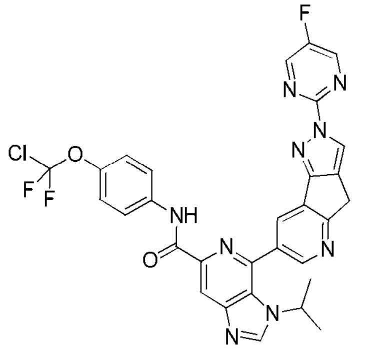,

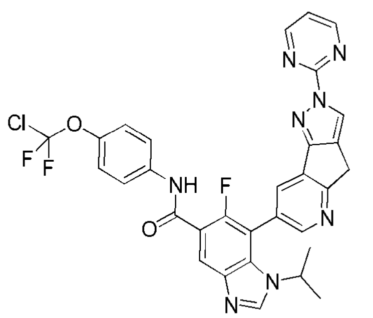,
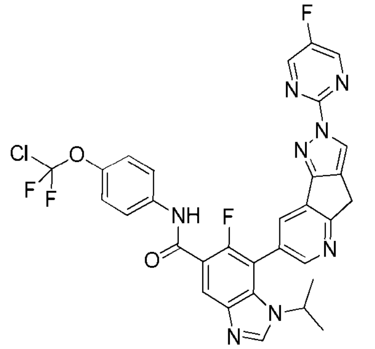,
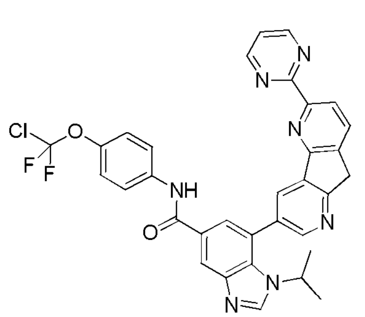,

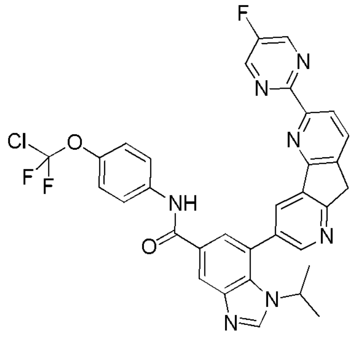,
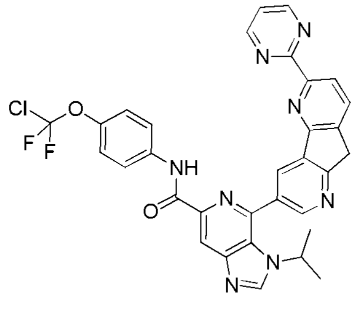,
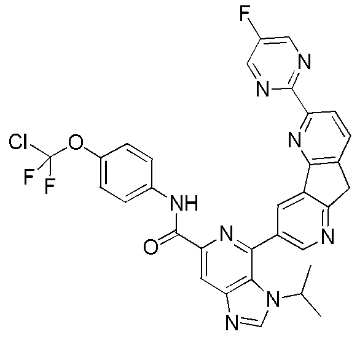,
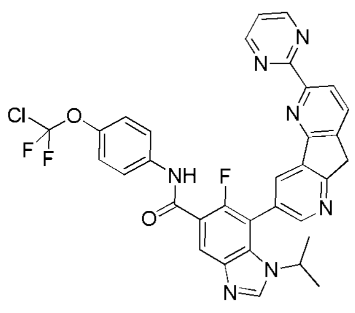,
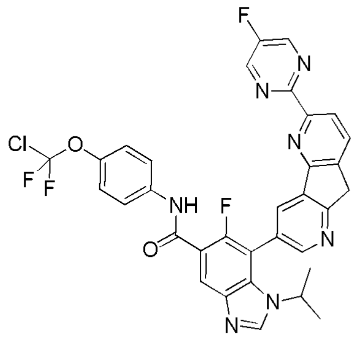,
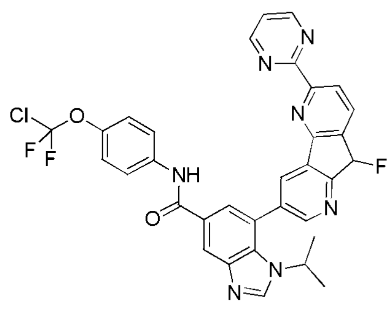,
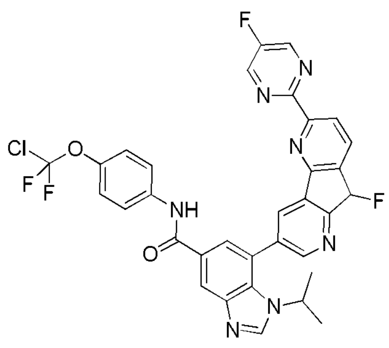,
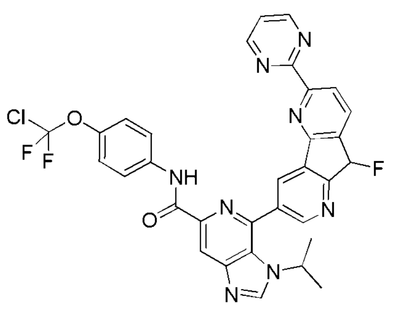,
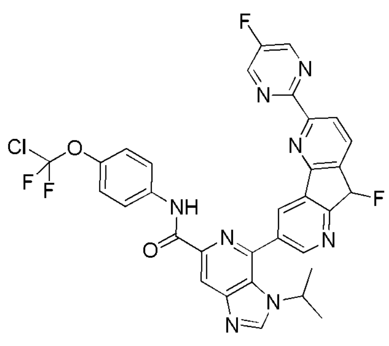,
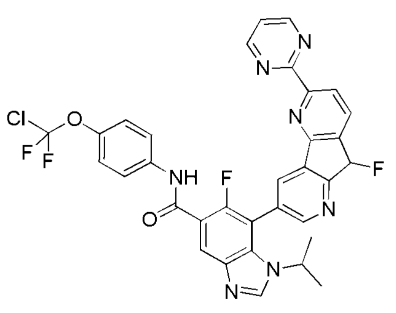,

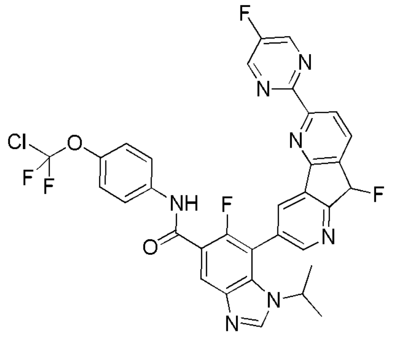 , 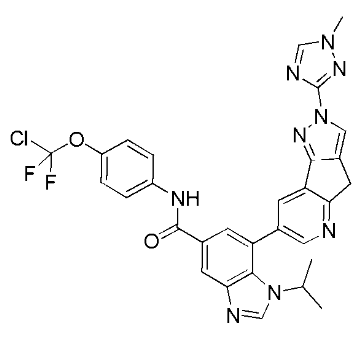 , 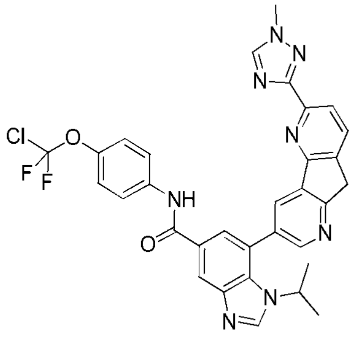 ,

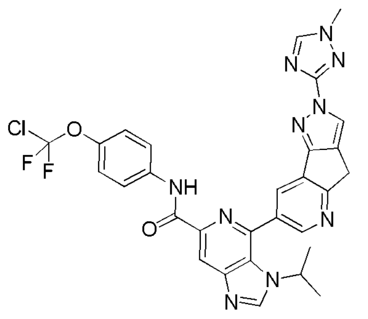 , 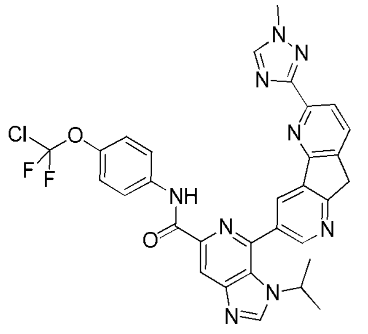 , 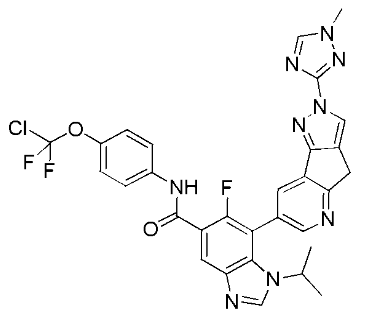 ,

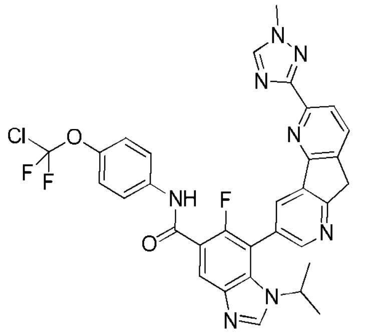 , 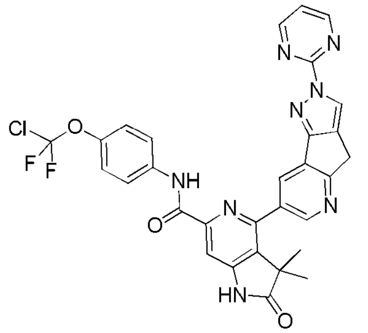 , 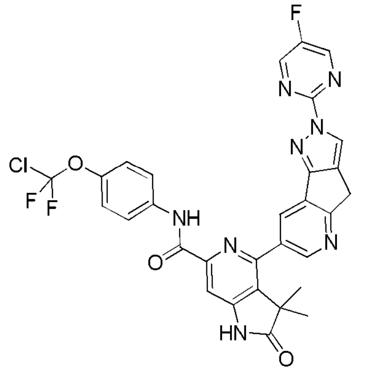 ,

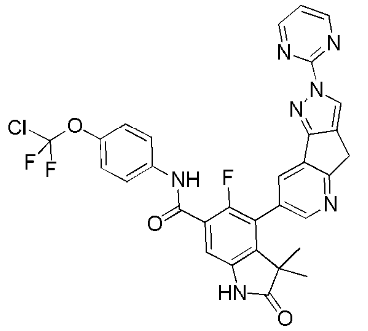 , 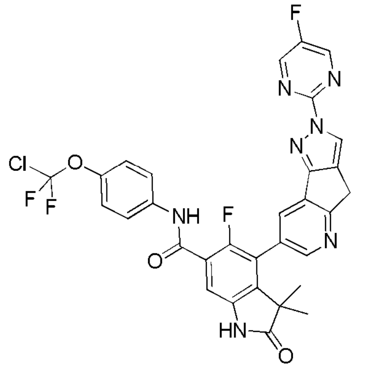 , 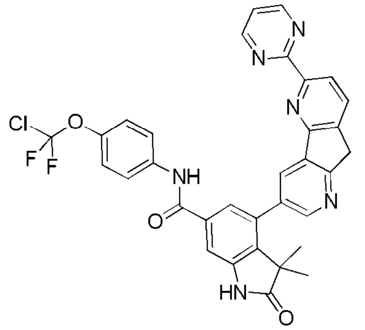 ,

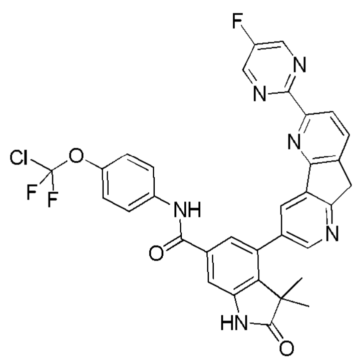
,
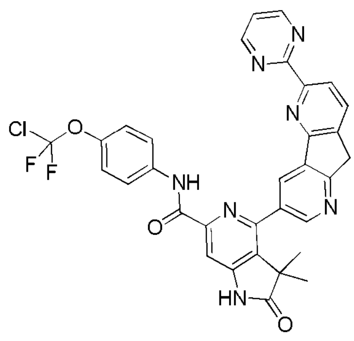
,
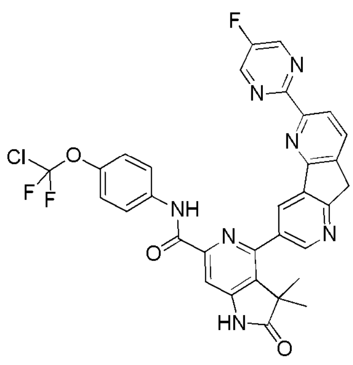
,
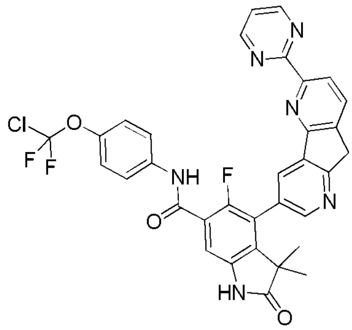
,
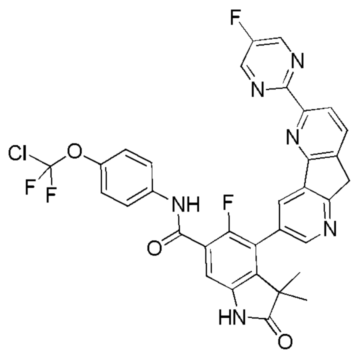
,
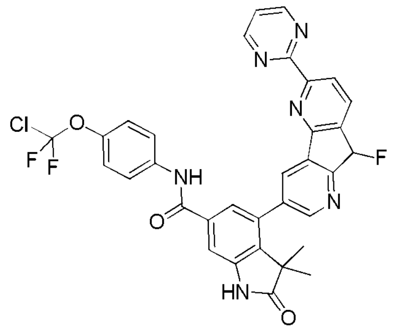
,
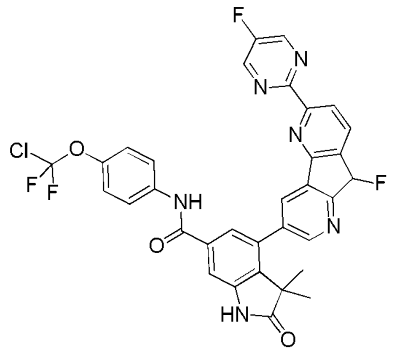
,
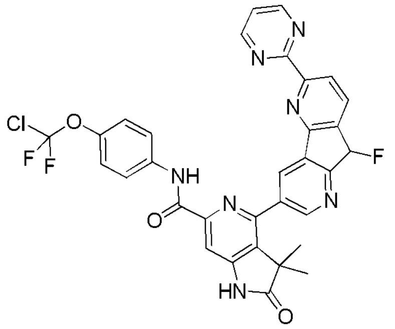
,
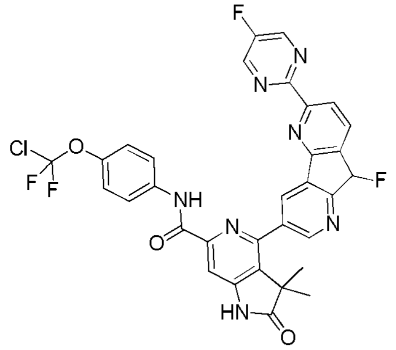
,
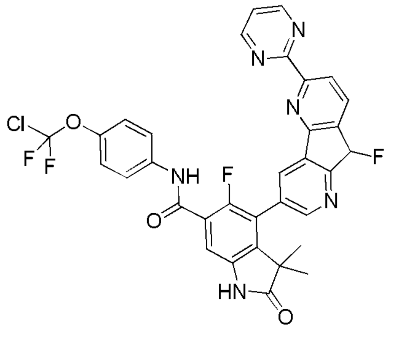
,

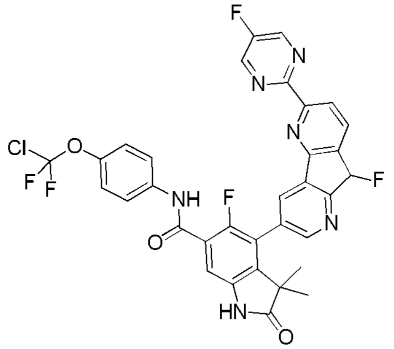
,
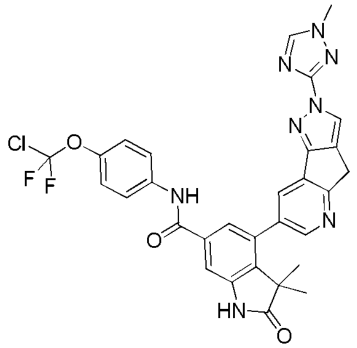
,
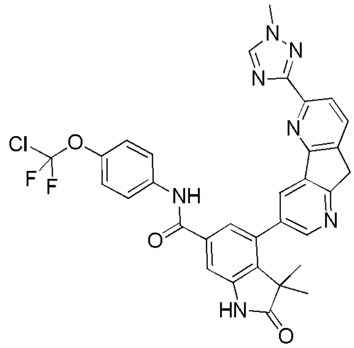
,

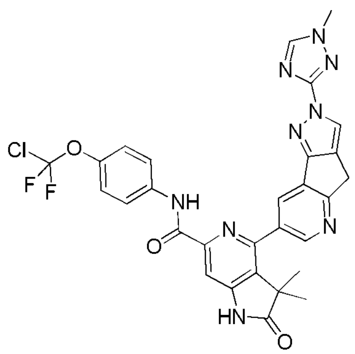
,
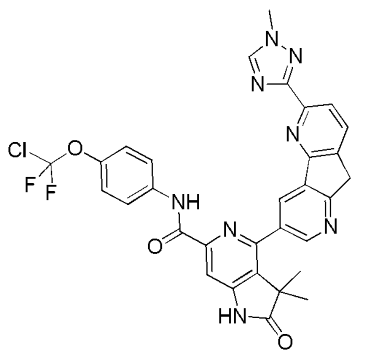
,
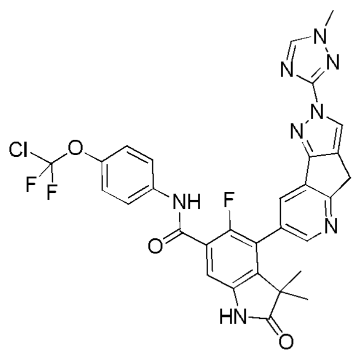
,

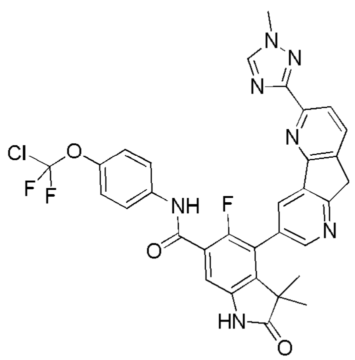
,
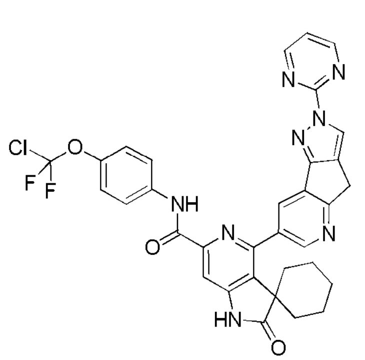
,
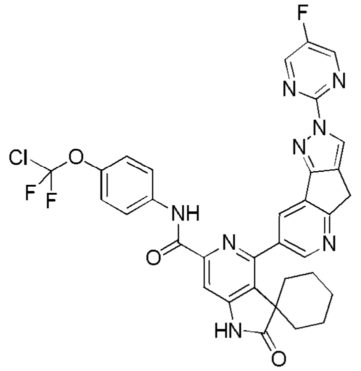
,

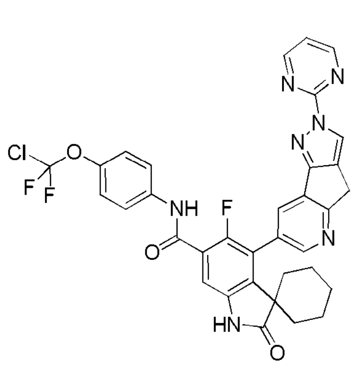
,
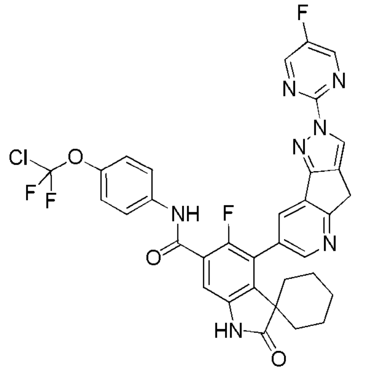
,
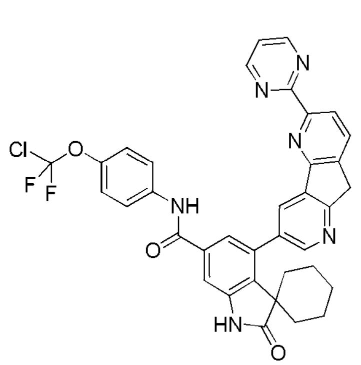
,

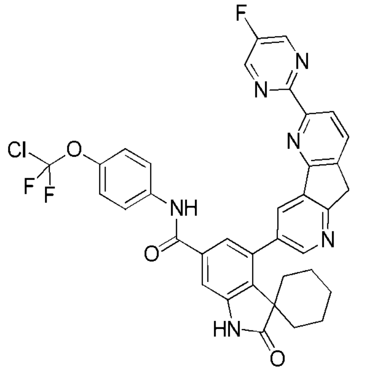,
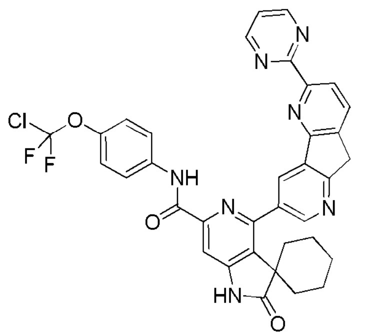,
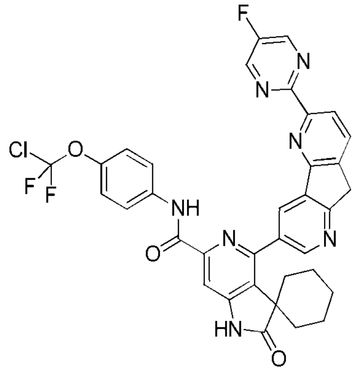,

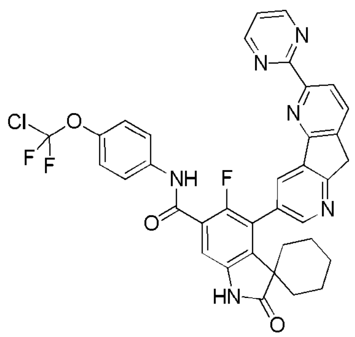,
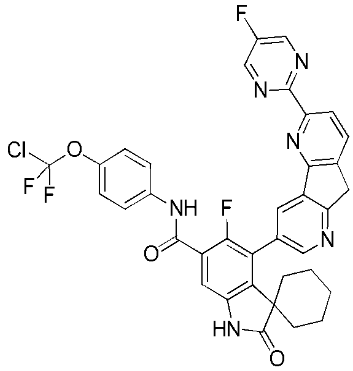,
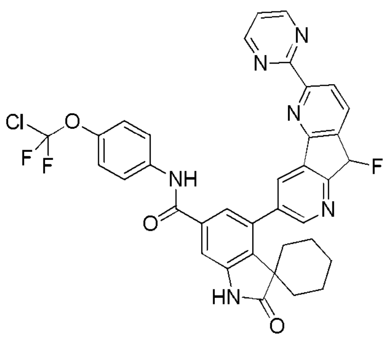,

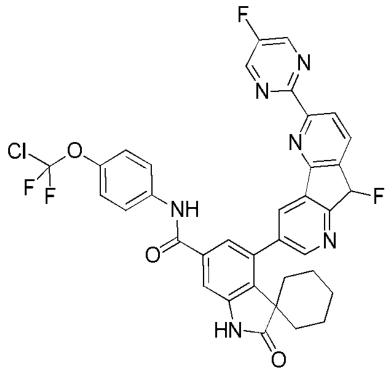,
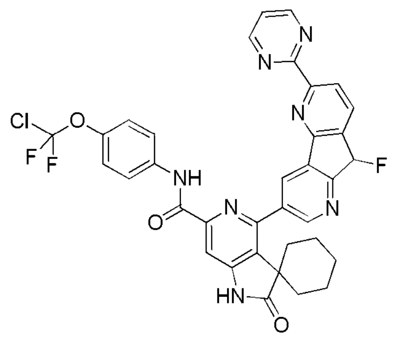,

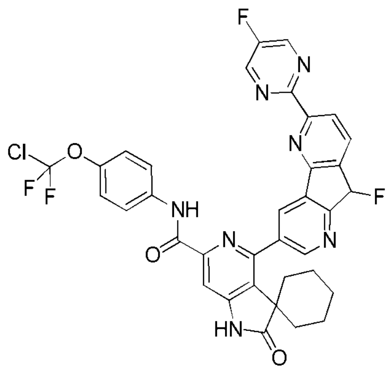,
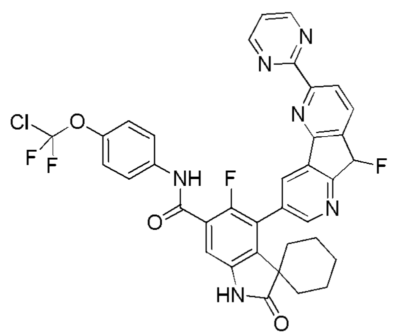,

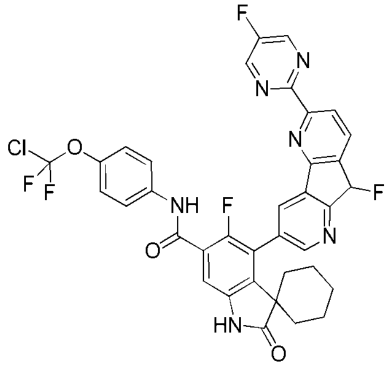,

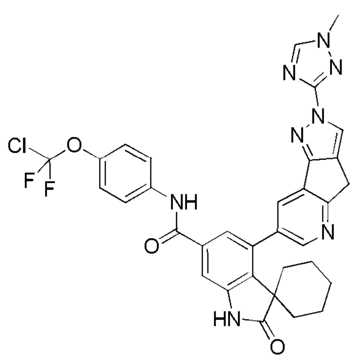,

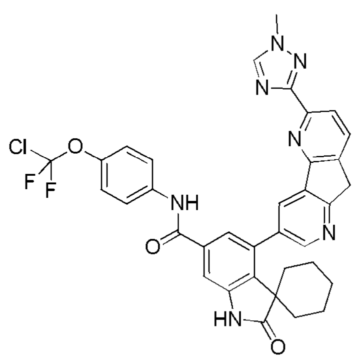,

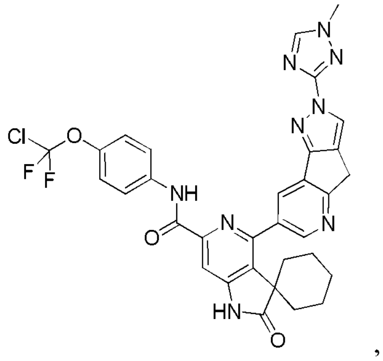,

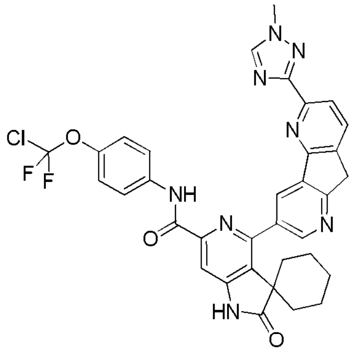,

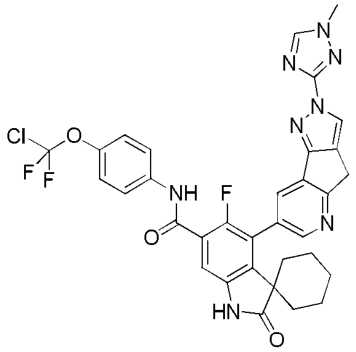,

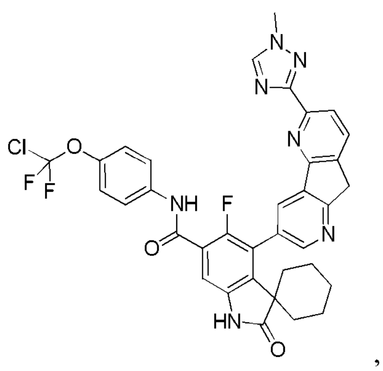,

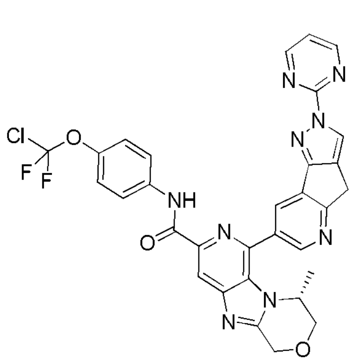,

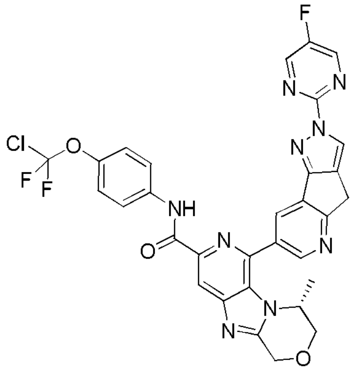,

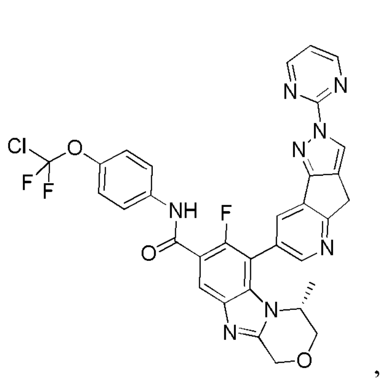,

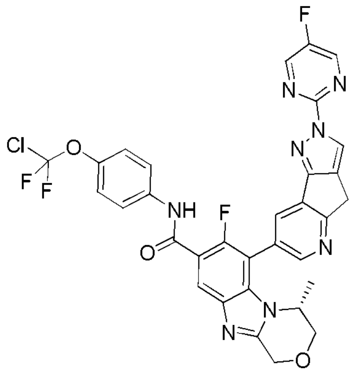,

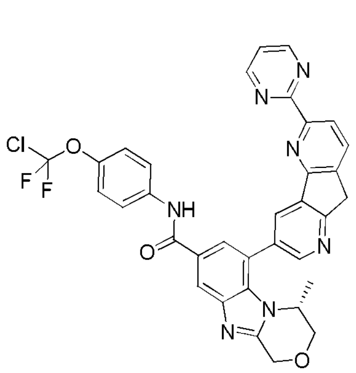,

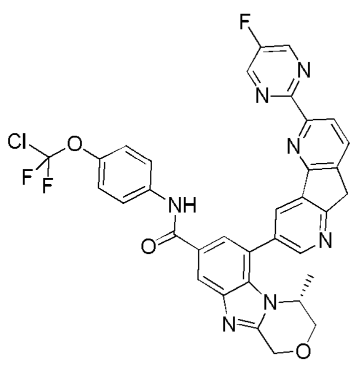,
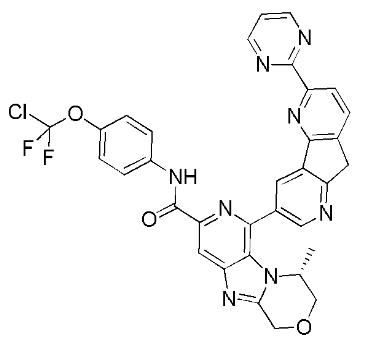,
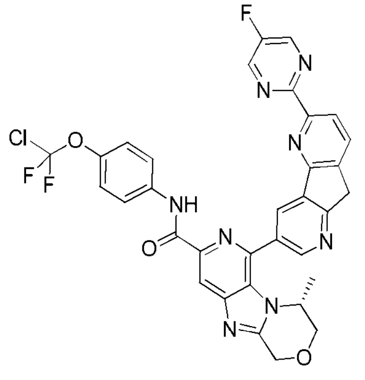,

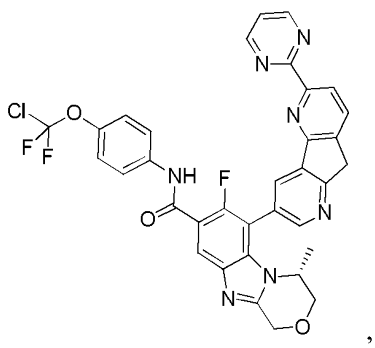,
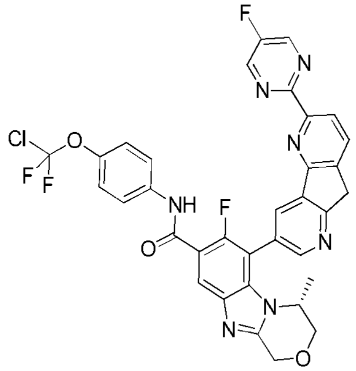,
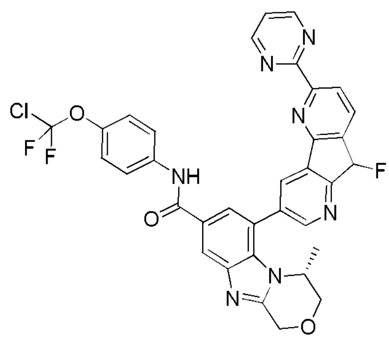,

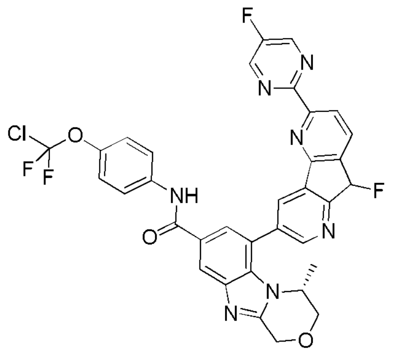,
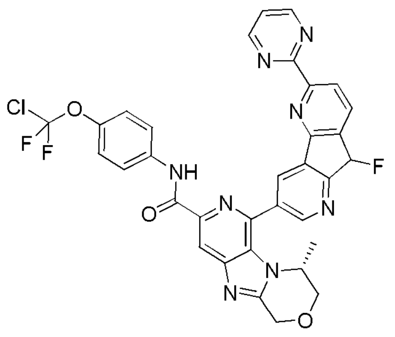,

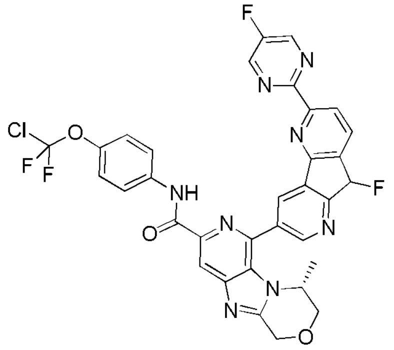,
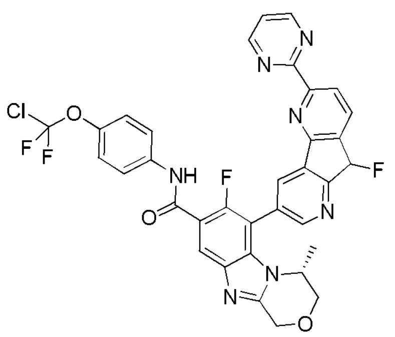,

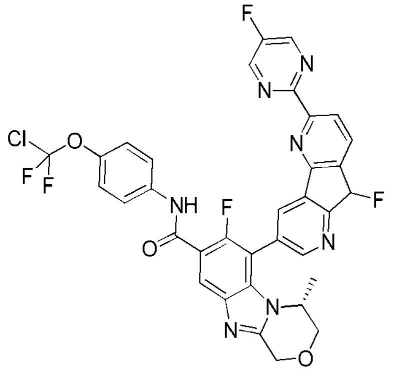 , 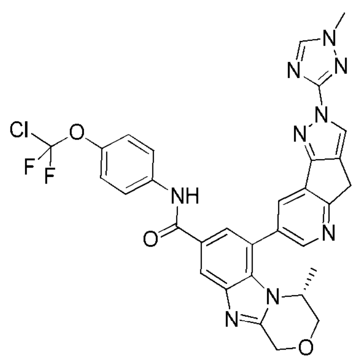 , 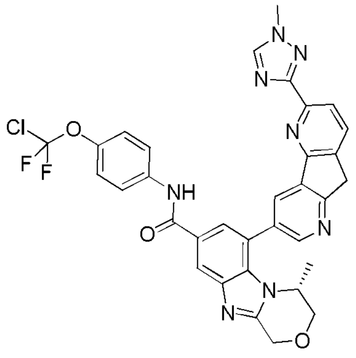 ,

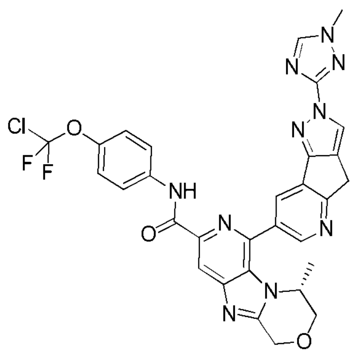 , 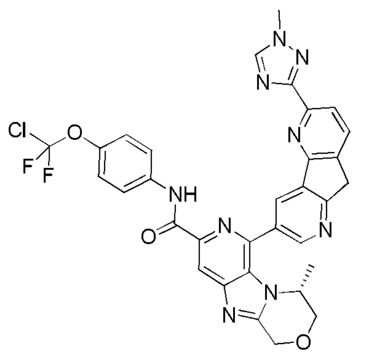 , 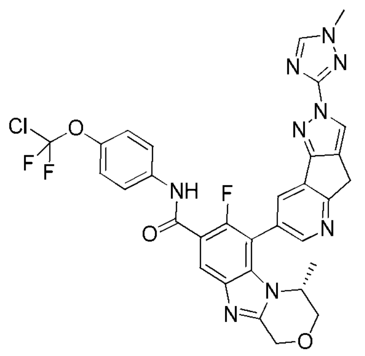 ,

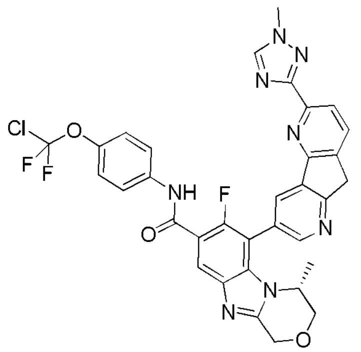 , 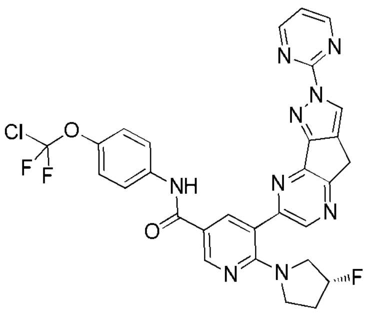 , 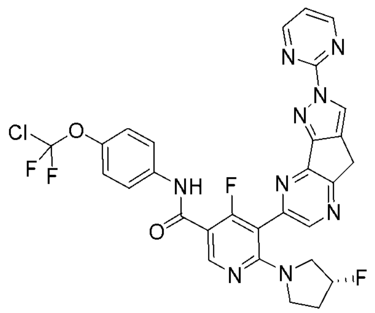 ,

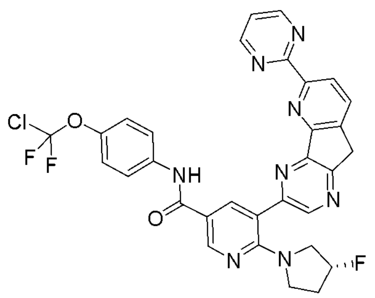 , 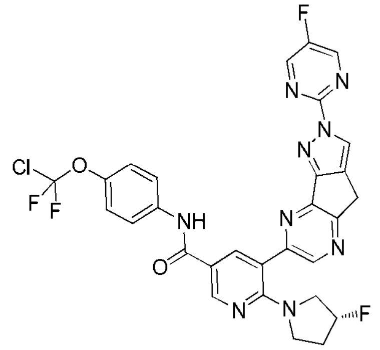 , 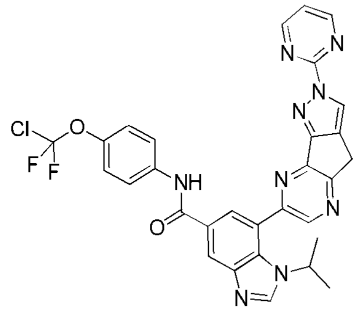 ,

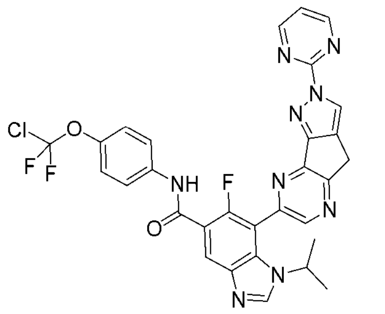,

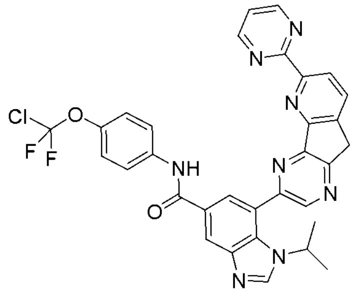

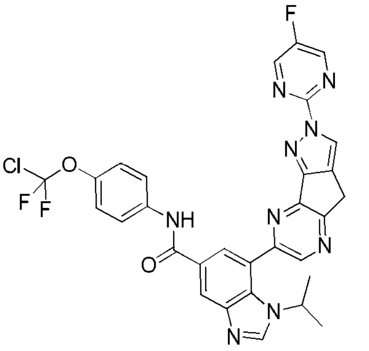,

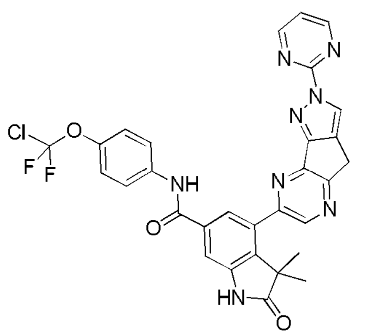,

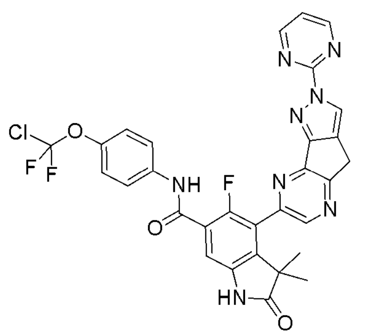,

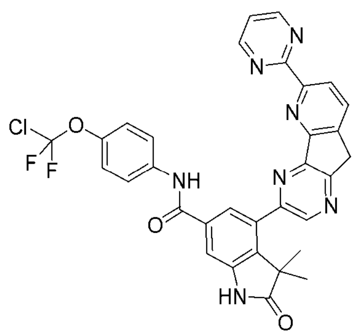,

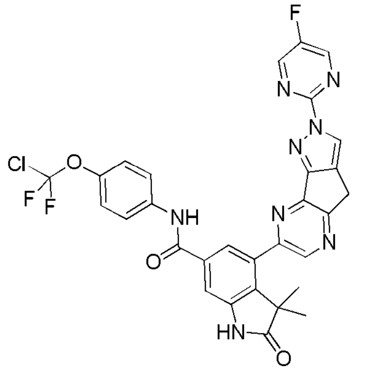,

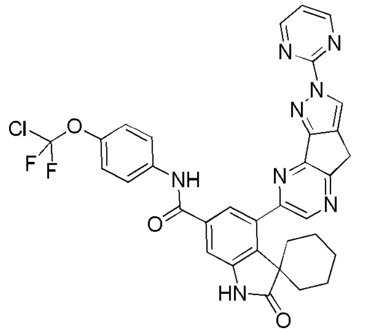,

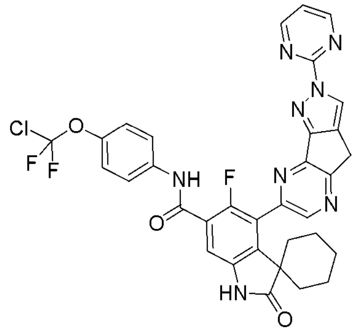,

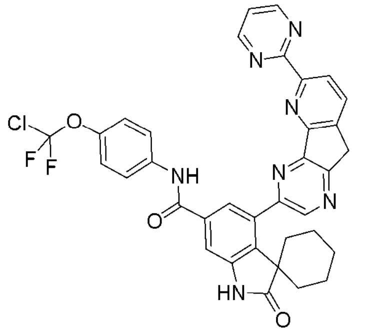,

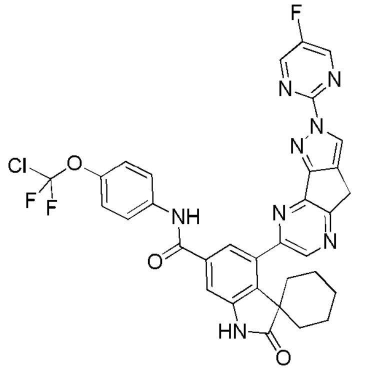,

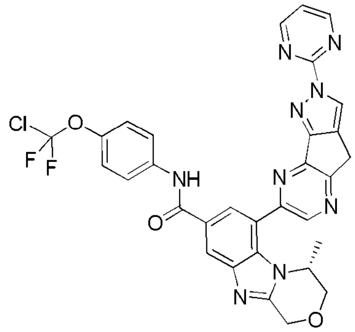,

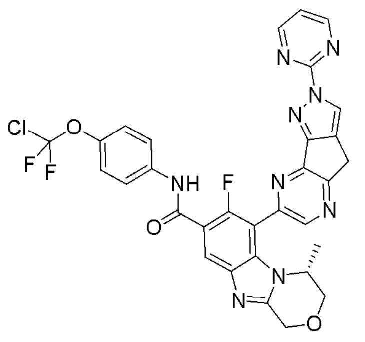
,
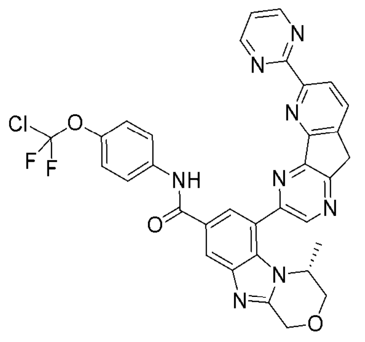
,
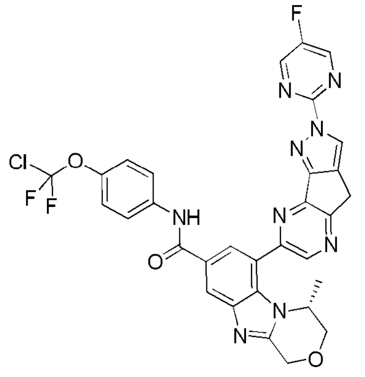
,

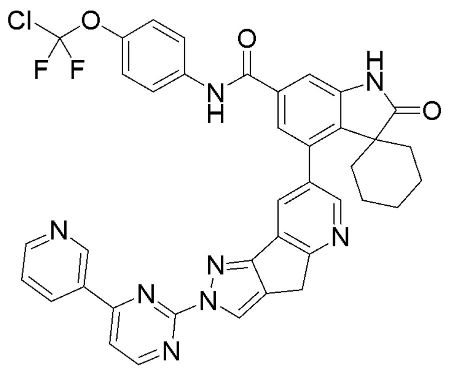
,
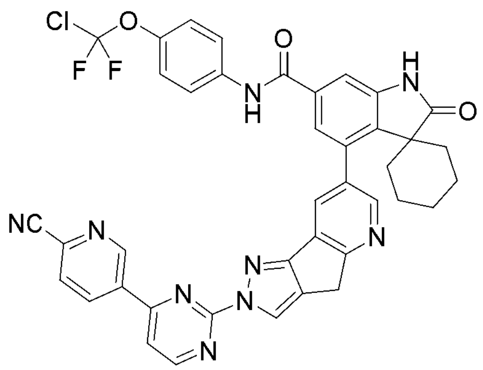
,

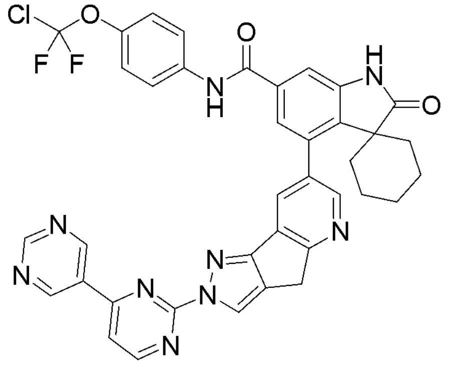
,
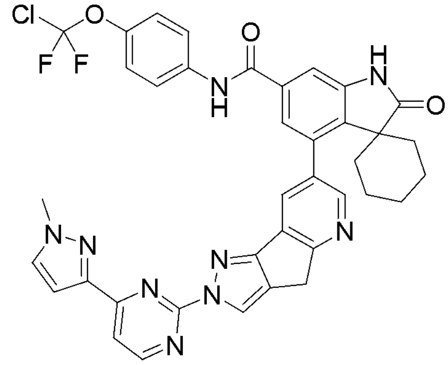
,

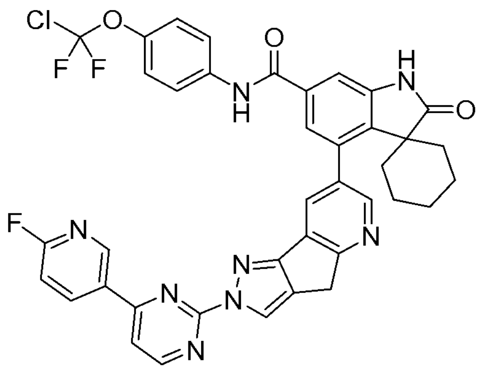
,
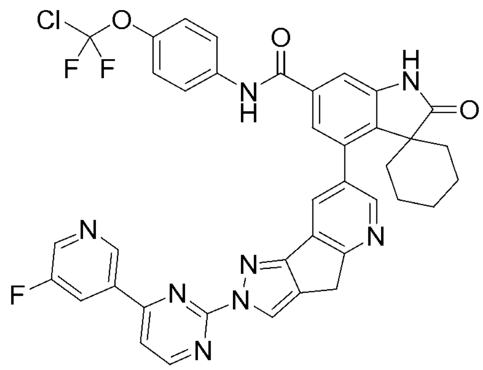
,

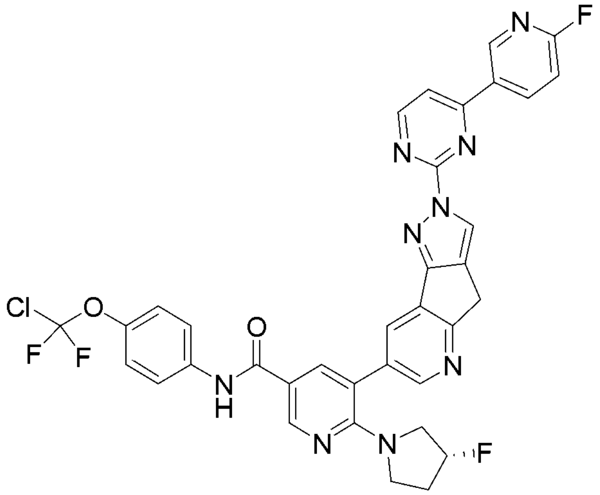

and

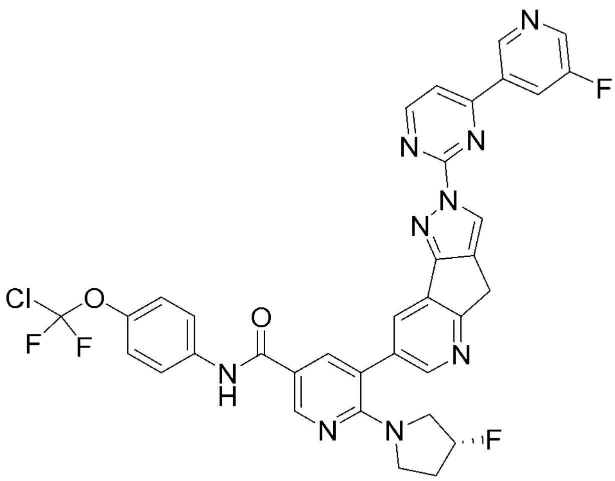

.

**107.**

A pharmaceutical composition, comprising the compound according to any one of claims 1 to 106, or a tautomer, a stereoisomer, a prodrug, a crystal form, a pharmaceutically acceptable salt, a hydrate or a solvate thereof, and a pharmaceutically acceptable excipient, and optionally, other therapeutic agent(s).

**108.**

A unit dosage form, comprising the pharmaceutical composition according to claim 107.

**109.**

Use of the compound according to any one of claims 1 to 106, or a tautomer, a stereoisomer, a prodrug, a crystal form, a pharmaceutically acceptable salt, a hydrate or a solvate thereof, or the pharmaceutical composition according to claim 107, or the unit dosage form according to claim 108, in the manufacture of a medicament for the treatment and/or prevention of a wild-type and/or mutated Bcr-Abl1 kinase-mediated disease; optionally, wherein the mutation of the mutated Ber-Abl1 kinase is selected from: T3151, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V and A424T;

alternatively, wherein the mutation of the mutated Ber-Abl1 kinase is selected from: T3151 and T315M;
alternatively, wherein the mutation of the mutated Ber-Abl1 kinase is T315I;
alternatively, wherein the mutation of the mutated Ber-Abl1 kinase is selected from: P465S, V468F, I502L, A337V and A424T.

**110.**

A method of treating and/or preventing a wild-type and/or mutated Ber-Abl1 kinase-mediated disease in a subject, comprising administering to the subject the compound according to any one of claims 1 to 106 or a pharmaceutically acceptable salt, a stereoisomer, a solvate, a hydrate, a polymorph, a prodrug or an isotopic variant thereof, or the pharmaceutical composition according to claim 107, or the unit dosage form according to claim 108;

optionally, wherein the mutation of the mutated Ber-Abl1 kinase is selected from: T3151, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V and A424T;
alternatively, wherein the mutation of the mutated Ber-Abl1 kinase is selected from: T3151 and T315M;
alternatively, wherein the mutation of the mutated Ber-Abl1 kinase is T315I;
alternatively, wherein the mutation of the mutated Ber-Abl1 kinase is selected from: P465S, V468F, I502L, A337V and A424T.

**111.** The compound according to any one of claims 1 to 106, or a tautomer, a stereoisomer, a prodrug, a crystal form, a pharmaceutically acceptable salt, a hydrate or a solvate thereof, or the pharmaceutical composition according to claim 107, or the unit dosage form according to claim 108, for use in the treatment and/or prevention of a wild-type and/or mutated Ber-Abl1 kinase-mediated disease;

optionally, wherein the mutation of the mutated Ber-Abl1 kinase is selected from: T3151, T315M, F317L, E355G, V299L, M244V, G250E, Q252H, Y253H, E255K, E255V, H396P, H396R, H396A, A397P, P223S, K294E, M351T, F359V, E459K, P465S, V468F, I502L, A337V and A424T;
alternatively, wherein the mutation of the mutated Ber-Abl1 kinase is selected from: T3151 and T315M;
alternatively, wherein the mutation of the mutated Ber-Abl1 kinase is T315I;
alternatively, wherein the mutation of the mutated Ber-Abl1 kinase is selected from: P465S, V468F, I502L, A337V and A424T.

**112.**

The use according to claim 109 or the method according to claim 110 or the compound or composition for use according to claim 111, wherein the wild-type and/or mutated Ber-Abl1 kinase-mediated disease is selected from: leukemia, non-Hodgkin's lymphoma, Hodgkin's lymphoma, myeloma, neurodegenerative diseases, solid tumors, immune diseases and fibrosis.

**113.**

The use according to claim 109 or the method according to claim 110 or the compound or composition for use according to claim 111, wherein the wild-type and/or mutated Ber-Abl1 kinase-mediated disease is selected from acute lymphoblastic leukemia, acute myeloid leukemia, acute promyelocytic leukemia, chronic lymphocytic lymphoma, chronic myeloid leukemia, chronic myelomonocytic leukemia, chronic neutrophilic leukemia, acute undifferentiated leukemia, anaplastic large cell lymphoma, prolymphocytic leukemia, juvenile myelomonocytic leukemia, adult T-cell leukemia, acute myeloid leukemia with trilineage myelodysplasia, mixed lineage leukemia, myelodysplastic syndrome, amyotrophic lateral sclerosis, Parkinson's disease and Alzheimer's disease; alternatively, the wild-type and/or mutated Ber-Abl1 kinase-mediated disease is selected from acute lymphoblastic leukemia and chronic myeloid leukemia.

**114.**

A method of preparing the compound according to any one of claims 1 to 106.

**115.**

An intermediate compound or a salt thereof, wherein the intermediate compound is one of the following compounds:

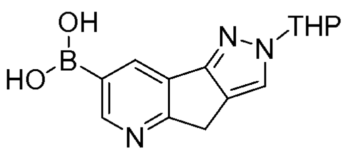
,
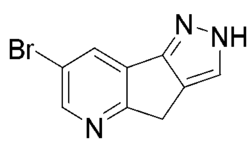
,
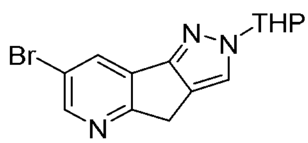
,

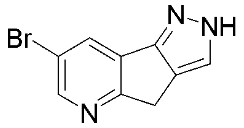 , 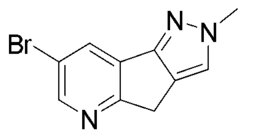 , 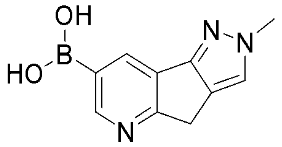 , 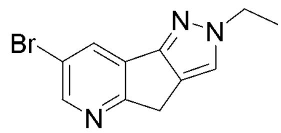 ,

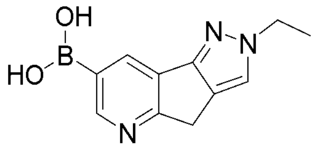 , 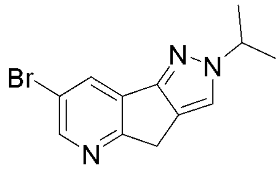 , 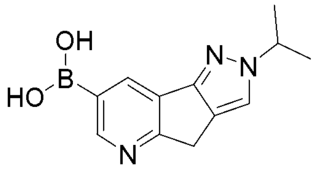 ,

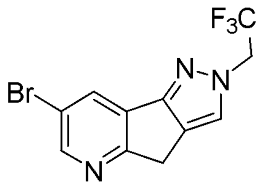 , 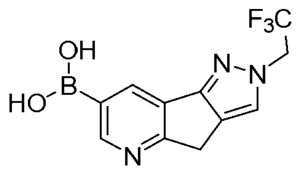 , 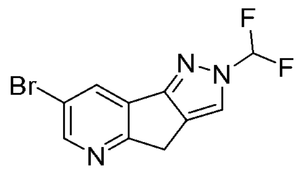 ,

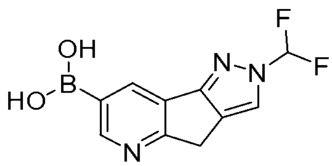 , 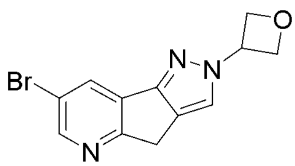 , 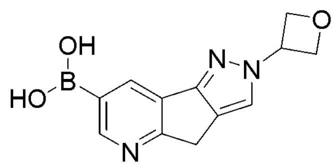 ,

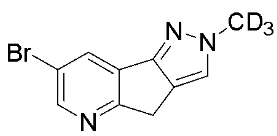 , 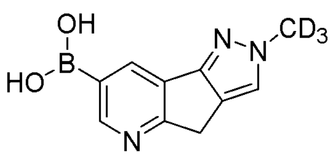 , 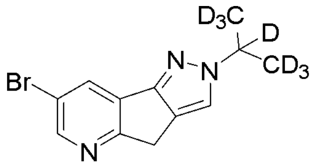 ,

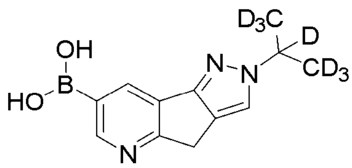 , 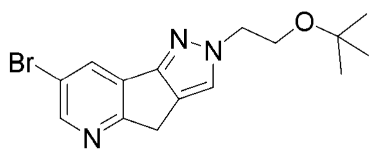 , 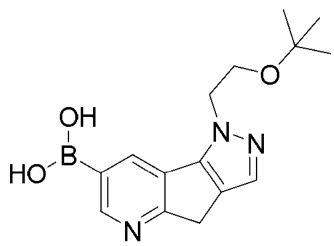 ,

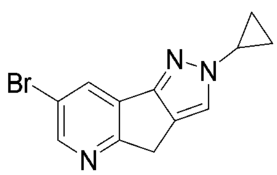 , 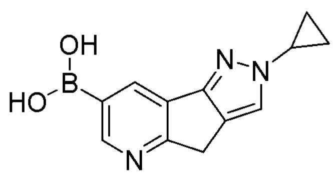 , 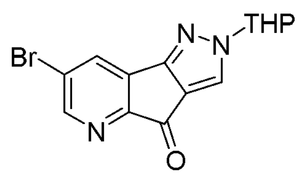 ,

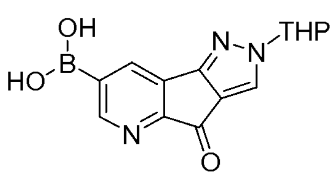 , 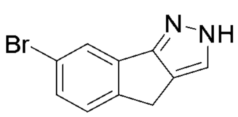 , 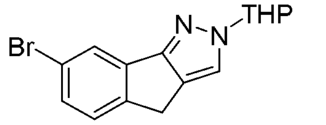 ,

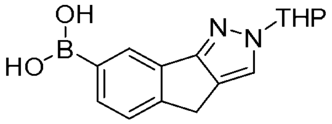
,
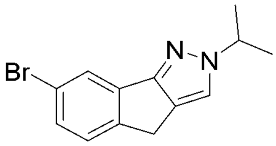
,
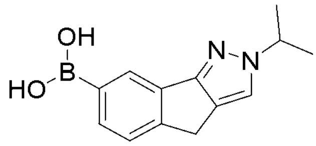
,
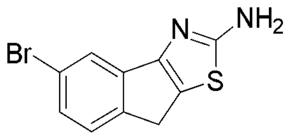
or
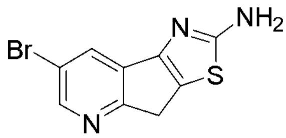
.

## INTERNATIONAL SEARCH REPORT

International application No.
**PCT/CN2024/121848**

**A. CLASSIFICATION OF SUBJECT MATTER**

C07D471/04(2006.01)i； C07D401/14(2006.01)i； C07D403/10(2006.01)i； C07D413/14(2006.01)i； A61K31/505(2006.01)i； A61K31/444(2006.01)i； A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXTC, REGISTRY (STN), CAPLUS (STN), CNKI: 稠环, 吡啶, 吡嗪, 吡咯烷酮, 激酶抑制剂, fused ring, pyridine, pyrazine, pyrrolidone, kinase inhibitors, bcr-abl, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| A | WO 2021143927 A1 (CHIA TAI TIANQING PHARMACEUTICAL GROUP CO., LTD.) 22 July 2021 (2021-07-22)<br>claims 1 and 12-15 | 1-115 |
| A | CN 1798734 A (IRM LLC) 05 July 2006 (2006-07-05)<br>claims 11-16, and description, page 36, tables | 1-115 |
| A | CN 102695547 A (NOVARTIS AG et al.) 26 September 2012 (2012-09-26)<br>description, paragraphs 0028-0198 | 1-115 |
| A | CN 104334529 A (NOVARTIS AG) 04 February 2015 (2015-02-04)<br>claims 5, 8 and 10-26 | 1-115 |
| X | Registry. "RN 896156-35-7 compound"<br>*STN*, 27 July 2006 (2006-07-27),<br>pages 1-2 | 115 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 January 2025** | **10 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/121848**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **110-113**
because they relate to subject matter not required to be searched by this Authority, namely:

The subject matter of claims 110-113 relates to a method for treating diseases in a human body (PCT Rule 39.1(iv)). The search is carried out on the basis of the use of the compound or pharmaceutical composition in the preparation of a drug for treating corresponding diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/121848**

| Patent document cited in search report | Publication date (day/month/year) | Patent family member(s) | Publication date (day/month/year) |
|---|---|---|---|
| WO 2021143927 A1 | 22 July 2021 | None | |
| CN 1798734 A | 05 July 2006 | None | |
| CN 102695547 A | 26 September 2012 | BR 112012003278 A2 | 01 March 2016 |
| | | ZA 201200434 B | 31 October 2012 |
| | | IL 217764 A0 | 29 March 2012 |
| | | CL 2012000350 A1 | 12 October 2012 |
| | | CA 2769300 A1 | 17 February 2011 |
| | | MX 2012001846 A | 22 May 2012 |
| | | WO 2011019798 A1 | 17 February 2011 |
| | | US 2011039850 A1 | 17 February 2011 |
| | | MA 33555 B1 | 01 September 2012 |
| | | RU 2012108930 A | 20 September 2013 |
| | | SG 178082 A1 | 29 March 2012 |
| | | US 2012329798 A1 | 27 December 2012 |
| | | KR 20120089844 A | 14 August 2012 |
| | | TN 2012000029 A1 | 19 September 2013 |
| | | AU 2010282547 A1 | 16 February 2012 |
| | | US 2015209365 A1 | 30 July 2015 |
| | | NZ 597864 A | 31 January 2014 |
| | | SG 10201407435 WA | 30 December 2014 |
| | | EP 2464423 A1 | 20 June 2012 |
| | | JP 2013501798 A | 17 January 2013 |
| | | PH 12014500538 A1 | 07 September 2015 |
| CN 104334529 A | 04 February 2015 | JP 2015520157 A | 16 July 2015 |
| | | JP 6080947 B2 | 15 February 2017 |
| | | WO 2013171641 A1 | 21 November 2013 |
| | | KR 20150014452 A | 06 February 2015 |
| | | KR 102190848 B1 | 15 December 2020 |
| | | EP 2900637 A1 | 05 August 2015 |
| | | EP 2900637 B1 | 09 August 2017 |
| | | ES 2646777 T3 | 15 December 2017 |
| | | CA 2870339 A1 | 21 November 2013 |
| | | CA 2870339 C | 02 June 2020 |
| | | BR 112014027584 A2 | 27 June 2017 |
| | | BR 112014027584 B1 | 24 January 2023 |
| | | PT 2900637 T | 15 November 2017 |
| | | US 2015141427 A1 | 21 May 2015 |
| | | US 9315489 B2 | 19 April 2016 |
| | | PL 2900637 T3 | 31 January 2018 |
| | | AU 2013261129 A1 | 23 October 2014 |
| | | AU 2013261129 B2 | 12 May 2016 |
| | | MX 2014013375 A | 14 August 2015 |
| | | MX 357305 B | 04 July 2018 |
| | | EA 201492092 A1 | 31 March 2015 |
| | | EA 026559 B1 | 28 April 2017 |
| | | US 2016185733 A1 | 30 June 2016 |
| | | US 9458112 B2 | 04 October 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5376645 A


### Non-patent literature cited in the description


- *J. Pharmaceutical Sciences*, 1977, vol. 66, 1-19
- Bioreversible Carriers in Drug Design. **T. HIGUCHI** ; **V. STELLA**. Prodrugs as Novel Delivery Systems, A.C.S. Symposium Series. American Pharmaceutical Association and Pergamon Press, 1987, vol. 14
- **D. FLEISHER** ; **S. RAMON** ; **H. BARBRA**. Improved oral drug delivery: solubility limitations overcome by the use of prodrugs. *Advanced Drug Delivery Reviews*, 1996, vol. 19 (2), 115-130
- Remington's Pharmaceutical Sciences. Mack Publishing Company, 1985